# EUROPEAN PATENT SPECIFICATION

(11) **EP 4 444 305 B1**
(45) Date of publication and mention of the grant of the patent: **25.11.2026**
(21) Application number: 23705256.8
(22) Date of filing: 14.02.2023
(51) Int. Cl.: A61K 31/4545, A61K 31/506, A61K 31/517, A61K 31/519, A61P 35/00, C07D 223/02, C07D 239/00, C07D 239/72, C07D 241/06, C07D 241/40

(54) **PHARMACEUTICAL COMPOUND**
PHARMAZEUTISCHE VERBINDUNG
COMPOSÉ PHARMACEUTIQUE

(30) Priority: 16.02.2022 GB 202202070; 06.09.2022 GB 202212967
(43) Date of publication of application: 16.10.2024
(60) Divisional application: 25217560.9 / 4 674 841
(73) Proprietor: Duke Street Bio Limited, London W1U 3EH (GB)
(72) Inventor: COWLEY, Phillip Martin, London Greater London W1U 3EH (GB); WISE, Alan, London Greater London W1U 3EH (GB); HILL, Christopher, London Greater London W1U 3EH (GB)
(74) Representative: Page White Farrer
(86) International application number: PCT/EP2023/053638
(87) International publication number: WO 2023/156386

(56) References cited:
- EP-A1- 1 396 488
- EP-A1- 1 604 981
- WO-A1-02/080928
- WO-A1-03/099808
- WO-A1-2004/099161
- WO-A1-2005/030726
- WO-A1-2006/077496
- WO-A1-2008/023239
- WO-A1-2008/139293
- WO-A1-2009/086264
- WO-A1-2010/056567
- WO-A1-2011/019090
- WO-A1-2013/066729
- WO-A1-2014/165143
- WO-A1-2015/091939
- WO-A1-2017/162510
- WO-A1-2020/103884
- WO-A1-2020/118133
- WO-A1-2020/132071
- WO-A1-2021/028854
- WO-A1-2022/242696
- WO-A2-2008/108957
- WO-A2-2015/021358
- WO-A2-97/38665
- CN-A- 110 357 888
- JINHUI HU ET AL, ACS CHEMICAL NEUROSCIENCE, vol. 10, no. 1, 25 September 2018 (2018-09-25), US, pages 537 - 551, XP055631836, ISSN: 1948-7193, DOI: 10.1021/acschemneuro.8b00376
- RABAL OBDULIA ET AL: "Multitarget Approach for the Treatment of Alzheimer's Disease: Inhibition of Phosphodiesterase 9 (PDE9) and Histone Deacetylases (HDACs) Covering Diverse Selectivity Profiles", ACS CHEMICAL NEUROSCIENCE, vol. 10, no. 9, 23 August 2019 (2019-08-23), US, pages 4076 - 4101, XP093042086, ISSN: 1948-7193, DOI: 10.1021/acschemneuro.9b00303

## Description

The present invention relates to PARP1 inhibitor compounds, and in particular to PARP1 inhibitor compounds for use in medicine. The inhibitors of the invention may be used in pharmaceutical compositions, and in particular pharmaceutical compositions for treating a cancer. The invention also relates to methods of manufacture of such inhibitors The scope of the invention is defined by the claims. Any embodiment not falling under the scope of the claims is provided for information purposes only.

### Background to the Invention

The family of poly(ADP-ribose) polymerases (PARPs) consists of 17 PARP proteins that catalyse the transfer of ADP-ribose to target proteins, a posttranslational process termed PARylation. Target protein modification by PARylation causes significant changes to function and as such PARPs play an important role in many cellular processes such as chromatin remodelling, transcription, replication, recombination, cell cycle progression and DNA damage repair (Kamaletdinova, T. et al. Cell. 2019; 8: 1625). PARP1 and 2 are the most widely studied PARP enzymes, primarily due to their role in DNA damage repair, in particular in the base excision repair (BER) process of DNA single-strand breaks (Ngoi, YL. et al. Cancer J. 2021; 27: 521-528). PARP1 is activated by DNA damage breaks, and the subsequent PARylation of target proteins leads to recruitment of additional factors that initiate repair of DNA lesions. Auto-PARylation of PARP triggers the release of bound PARP from the DNA allowing other DNA repair proteins access to complete lesion repair. This highlights the critical role PARP plays in enabling a cancer cell to repair DNA damage caused by exogenous agents such as radiation therapy and chemotherapeutic agents. Hence, inhibition of PARP enzymes has been utilised as a strategy to selectively kill cancer cells that harbour genetic defects in complementary DNA damage repair pathways (Farmer, H. et al. Nature. 2005; 434: 917-921). This synthetic lethality approach has been demonstrated successfully in tumours with epigenetic modifications or deleterious mutations in BRCA1 and BRCA2, two functionally redundant tumour suppressor proteins involved in DNA double-strand break (DSB) repair by homologous recombination (HR) (Lord, CJ. and Ashworth, A. Science. 2017; 355: 1152-1158). Such tumours with HR deficiency (HRD) are dependent on PARP function for survival - following PARP inhibition in these tumours, DSB breaks will be processed by alternative error-prone repair pathways leading to genomic instability and cancer cell death.

The inhibition of PARP can trap the inactivated PARP at the sites of DNA damage. This leads to replication fork stalling and subsequent collapse in S-phase when the fork reaches the site of the trapped PARP, resulting in the generation of genotoxic DNA double-strand breaks. It is believed that this PARP1-DNA trapping can lead to the selective death of cancer cells harbouring HRD (Farmer, H. et al. Nature. 2005; 434: 917-921.

This strategy has led to the successful approval of several PARP inhibitors for the treatment of cancers with HRD, such as in BRCA1/2-mutated breast, ovarian and prostate cancer, as well as in ovarian and prostate cancer harbouring genomic consequences of HRD, and ovarian cancer in the maintenance setting where platinum sensitivity acts as a surrogate for HRD (Fong, PC. et al. N. Engl. J. Med. 2009; 361: 123-134).

It has recently been shown that genomic instability, in the form of unrepaired DNA double-strand breaks or micronuclei disruption can trigger innate immune system activation via the cytosolic DNA sensor cyclic GMP-AMP synthase (cGAS), leading to generation of cyclic guanosine monophosphate-adenosine monophosphate (cGAMP) and induction of dimerization of Stimulator of interferon genes (STING). STING subsequently translocates from the endoplasmic reticulum to the Golgi where it recruits and activates TANK-binding kinase 1 (TBK1). TBK1 phosphorylates interferon regulatory transcription factor 3 (IRF3) which drives the production of type I interferons and supports the induction of an adaptive immune response (Zhu, Y. et al. Mol. Cancer. 2019, 18: 152).

For example, PARP inhibitor-induced STING pathway activation and anti-tumour immune responses have been demonstrated in multiple tumour models, providing rationale for exploiting combinations of PARP inhibitors with immunotherapies for improved therapeutic efficacy (Sen, T. et al. Cancer Discov. 2019; 9: 646-661). For example, the PARP inhibitor Olaparib was also recently shown to induce synthetic lethal effects in combination with a synthetic cyclic dinucleotide STING agonist in DNA damage repair deficient cancer cells and a BRCA-deficient breast cancer model (Pantelidou, C. et al. 2021: bioRxiv).

Overall, modulation of nucleic acid sensing pathways via multiple mechanisms has been shown to promote anti-tumour efficacy in a variety of cell and animal models thus demonstrating therapeutic potential for augmenting efficacy of immunotherapies and overcoming resistance to immune checkpoint blockade through use of PARP inhibitors. There are numerous clinical trials ongoing combining PARP inhibitors with immunotherapies (reviewed in Chabanon, RM, et al. Nat. Rev. Cancer. 2021; 21: 701-717).

Recently, PARP1 has also been shown to bind the Epstein Barr Virus (EBV) genome and that PARP1 inhibition can alter EBV chromatin structure and latent gene expression (Morgan, SM. et al. Nat. Commun. 2022; 13: 187). Hence, PARP1 inhibitors may play a role in cancers where EBV plays a contributing role such as Burkitt's lymphoma, Hodgkin's lymphoma, nasopharyngeal and gastrointestinal cancers. Interestingly, EBV has also been shown to be a causative factor in multiple sclerosis (MS) whereby EBV infection greatly increases the risk of subsequent MS (Bjornevik, K. et al. Science (2021); 375: 296-301).

First-generation PARP inhibitors generally demonstrate non-selective activity at PARP1 and 2. Haematological toxicities such as anaemia, neutropenia and thrombocytopenia are associated with clinical use of these molecules which restricts their use in combination with cytotoxic chemotherapies and other targeted agents due to dose-limiting cytopenias (LaFargue, CJ. et al. Lancet Oncol. 2019, 20, e15-e28). Evidence from pre-clinical mouse studies strongly suggests that PARP2 inhibition is a major driver of these haematological toxicities, with PARP2 being particularly linked to erythrogenesis in mice (Farrés, J. et al. Blood. 2013; 122: 44-54). In addition, PARP2 function has been shown to be dispensable for anti-tumour activity in HRD mouse cancer models (Ronson, G E. et al. Nat. Commun. 2018, 9: 746). Taken together, these data suggest an unmet medical need for the development of inhibitors with improved selectivity for PARP1 over PARP2 and other PARPs, thus providing expanded therapeutic utility (1) as single agents and (2) in combination with other anti-cancer agents.

To date, two PARP1-selective inhibitors, AZD5305 and AZD9574, have entered clinical development. AZD5305 was described as a potent PARP1 inhibitor and trapper with 500-fold selectivity over PARP2 and less off-target activity against secondary pharmacology targets than first-generation PARP inhibitors (Johannes, JW. et al. J. Med. Chem. 2021; 64: 14498-14512). Importantly, significantly less haematotoxicity was observed for AZD5305 in rodent models than with first-generation PARP inhibitors, confirming the reported pathogenic role of PARP2 in haematologic toxicity (Illuzzi, G. et al. Clin. Cancer Res. 2022; CCR-22-0301).

Having regard to the above, it is an aim of the present invention to provide PARP1 inhibitors, and in particular PARP1 inhibitors for use in medicine. It is a further aim to provide pharmaceutical compositions comprising such inhibitors, and in particular to provide compounds and pharmaceutical compositions for treating a cancer. It is also an aim to provide methods of synthesis of the compounds.

WO 2011/019090 A1 discloses compounds for use in preventive and/or therapeutic treatment of a disease caused by abnormal activity of tau protein kinase 1, such as a neurodegenerative diseases (e.g. Alzheimer's disease).

WO 2006/077496 A1 discloses substituted triazoles for use in the treatment of sexual dysfunction.

WO 2017/162510 A1 discloses substituted quinazolinone compounds for the treatment of proliferative diseases.

WO 2013/066729 A1 discloses aminopyrimidinone compounds that are inhibitors of Interleukin receptor-associated kinases.

WO 2008/139293 A1 discloses PDE9-inhibiting compounds which are amino-heterocyclic compounds.

CN 110 357 888 A discloses a heterocyclic phosphodiesterase inhibitor compound, for use in treatment or prevention of diseases associated with PDE9 kinase.

WO 2009/086264 A1 discloses benzofuropyrimidinones as protein kinase inhibitors.

WO 2015/021358 A2 discloses non-NHE3-binding agents having activity as phosphate transport/uptake inhibitors in the gastrointestinal tract.

WO 2020/118133 A1 discloses compounds for treating conditions associated with TREX1.

WO 2008/023239 A1 discloses pyrimidone derivatives I having activity as GSK-3 inhibitors.

Hu et al (ACS Chem. Neurosci. 2019, 10, 537-551) discloses dual-target inhibitors of acetylcholinesterase and phosphodiesterase 9A for the treatment of Alzheimer's disease.

Obdulia et al (ACS Chem. Neurosci. 2019, 10, 4076-4101) discloses dual-target phosphodiesterase 9 and histone deacetylase inhibitors for the treatment of Alzheimer's disease.

WO 2020/132071 A1 discloses compositions for treating cancers, inflammatory diseases, rasopathies, and fibrotic disease involving aberrant Ras superfamily signaling through the binding of compounds to the GTP binding domain of Ras superfamily proteins.

WO 2021/028854 A1 discloses compounds for treating a disease or condition mediated by nuclear SET domain-containing protein 2.

WO 97/38665 A2 discloses compounds which inhibit farnesyl-protein transferase and the farnesylation of the oncogene protein Ras.

EP 1 396 488 A1 discloses a fused heterocyclic compound which shows poly(ADP-ribose) synthase inhibitory action and is useful as a therapeutic drug for cerebral infarction.

WO 03/099808 A1 discloses a substituted heterocyclic compound for the prophylaxis or treatment of various disorders, such as inflammation.

EP 1 604 981 A1 discloses compounds for regulating the CCR5 receptor.

WO 2005/030726 A1 discloses isoquinolinone compounds useful as potassium channel inhibitors to treat cardiac arrhythmias and the like.

WO 2008/108957 A2 discloses piperidine derivatives for treating or preventing pain, diabetes, a diabetic complication, impaired glucose tolerance or impaired fasting glucose in a patient.

WO 2010/056567 A1 discloses imidazoisoindole compounds which are antagonists of neuropeptide S receptors, and which are useful in the treatment or prevention of neurological and psychiatric disorders and diseases in which the neuropeptide S receptor is involved. WO 2014/165143 A1 discloses dihydro-pyrrolopyridinone bromodomain inhibitors that are useful as agents in the treatment of diseases and conditions, including inflammatory diseases, cancer, and AIDS.

WO 2015/091939 A1 discloses piperidine compounds having dual pharmacological activity towards both the sigma receptor and the mu-opioid receptor.

WO 2004/099161 A1 discloses substituted pyrazinone derivatives that are CRF1 receptor antagonists.

WO 02/080928 A1 discloses N-substituted nonaryl-heterocyclo amidyl compounds as NMDA NR2B antagonists useful for relieving pain.

### Summary of the Invention

In one aspect, the present invention provides a PARP1 inhibitor compound for use in medicine, according to claim 1.

In another aspect, the present invention provides a pharmaceutical composition comprising a compound, according to claim 23.

In still another aspect, the present invention provides a pharmaceutical kit according to claim 28.

A further aspect of the invention provides a method of synthesis of a PARP1 inhibitor compound, according to claim 30.

Certain more specific aspects of the invention are set out in the dependent claims.

### Detailed Description

The present disclosure provides a PARP1 inhibitor compound for use in medicine, which compound comprises the following structure, which is more general than the structure defined in present claim 1: 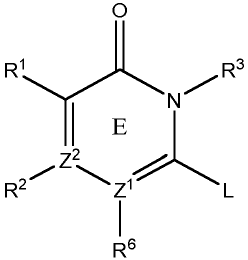
wherein R¹ is selected from H and a substituted or unsubstituted organic group; R² may be present or absent and is independently selected from H and a substituted or unsubstituted organic group; R¹ and R² may together form a ring; R³ is independently selected from H and a substituted or unsubstituted organic group; R⁶ may be present or absent and is independently selected from H and a substituted or unsubstituted organic group; Z¹ and Z² are independently selected from C and N; and L comprises a group having the following structure: 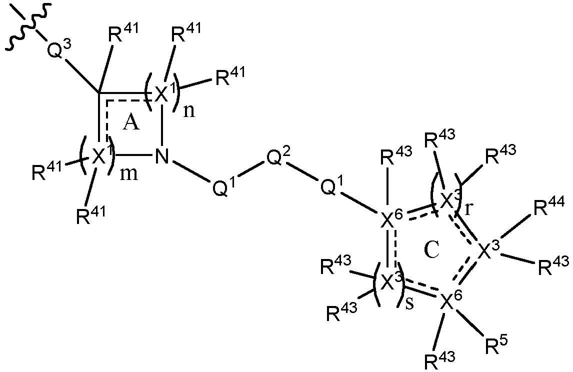
wherein, n is a number independently selected from 0, 1, 2, 3, 4, 5 and 6; m is a number independently selected from 0, 1, 2, 3, 4, 5 and 6; and m + n is a number selected from 2, 3, 4, 5, and 6; preferably wherein both n and m are at least 1;
wherein, r is a number independently selected from 0, 1, 2, 3, 4, and 5; s is a number independently selected from 0, 1, 2, 3, 4, and 5, preferably from 1, 2, 3, 4 and 5; and r + s is a number selected from 1, 2, 3, 4, and 5; preferably wherein both r and s are at least 1;
and wherein, each X¹ may be the same or different and is independently selected from C, N, O and S; each X³ may be the same or different and is independently selected from C, N, O and S, preferably wherein at least one X³ is independently N; each X⁶ may be the same or different and is independently selected from C and N; each R⁴¹ may be the same or different, and may be present or absent, and is selected from H and a substituted or unsubstituted organic group; each R⁴³ may be the same or different, and may be present or absent, and is selected from H and a substituted or unsubstituted organic group; and R⁴⁴ may be present or absent, and is selected from H and a substituted or unsubstituted organic group;
and wherein, the dotted lines indicate that: the ring A may comprise single bonds, or a combination of both single and double bonds, and may be aliphatic or aromatic; and independently the ring C may comprise single bonds, or a combination of both single and double bonds, and may be aliphatic or aromatic;
and wherein, R⁵ is selected from H and a substituted or unsubstituted organic group and is preferably a substituted or unsubstituted organic group;
and wherein each Q¹ may be the same or different, and may be present or absent, and comprises a group independently selected from the following structures: 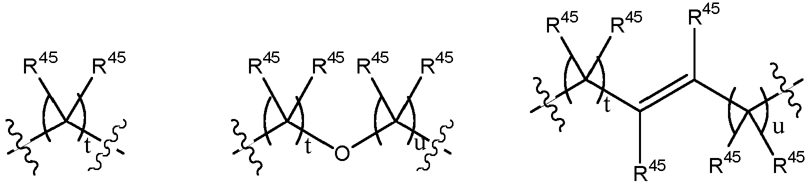 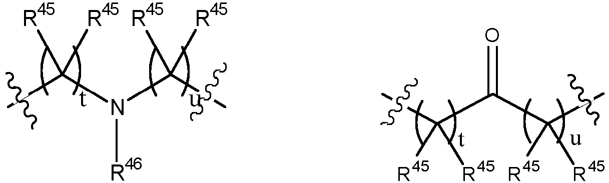
wherein t is independently a number selected from 0, 1, 2, 3, 4 and 5; u is independently a number selected from 0, 1, 2, 3, 4 and 5; and t + u is a number selected from 0, 1, 2, 3, 4, 5 and 6 (preferably a number selected from 0, 1, 2, and 3); each R⁴⁵ may be the same or different and is independently selected from H and a substituted or unsubstituted organic group; and R⁴⁶ is selected from H and a substituted or unsubstituted organic group;
and wherein Q² may be present or absent and is a group having the following structure: 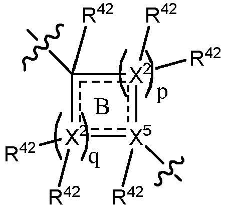
wherein, p is a number independently selected from 0, 1, 2, 3, 4, 5 and 6; q is a number independently selected from 0, 1, 2, 3, 4, 5 and 6; and p + q is a number selected from 2, 3, 4, 5, and 6;
and wherein, each X² may be the same or different and is independently selected from C, N, O and S; X⁵ is independently selected from C and N; each R⁴² may be the same or different, and may be present or absent, and is selected from H and a substituted or unsubstituted organic group;
and wherein, the dotted lines indicate that: the ring B may comprise single bonds, or a combination of both single and double bonds, and may be aliphatic or aromatic;
and wherein Q³ may be present or absent and comprises a group independently selected from the following structures: 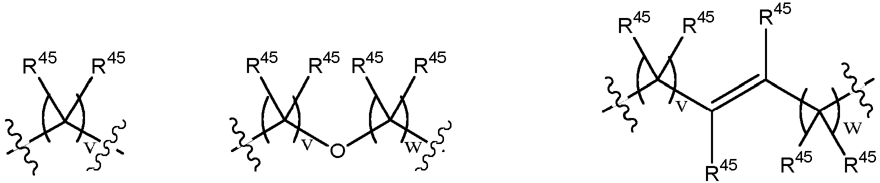 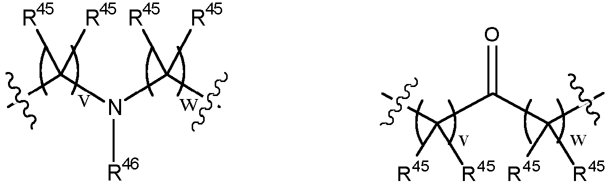
wherein v is independently a number selected from 0, 1, 2, 3, 4 and 5; w is independently a number selected from 0, 1, 2, 3, 4 and 5; and v + w is a number selected from 0, 1, 2, 3, 4, 5 and 6 (preferably a number selected from 0, 1, and 2).

In the context of the present disclosure, the Z¹, Z², X¹, X², X³, X⁴, X⁵ and X⁶ atoms maintain their normal valency and the number of substituents attached to these will depend on their nature and the number of other bonds they possess. Maintaining the valency means ensuring that an atom has its normal (typically most common) valency in organic compounds (i.e. 2 for oxygen and divalent sulphur, 3 for nitrogen and 4 for carbon). Nitrogen atoms may, in some instances, have 4 bonds, but in such cases they are typically positively charged such that the compound may have a counter-ion. Such compounds are also considered to be part of the invention, and in these cases, due to the positive charge, it will be clear that the nitrogen atom still maintains its normal valency of 3. For the avoidance of doubt, where the number of R groups may vary according to the choice of X group, it may vary as follows.

R⁶ is absent when Z¹ is N. R² is absent when Z² is N. Each R⁴¹ may be the same or different, provided that for each X¹: R⁴¹ is absent when X¹ is O or divalent S; R⁴¹ is absent when X¹ is N and is double bonded to an adjacent atom; one R⁴¹ is present when X¹ is N and is not double bonded to an adjacent atom; one R⁴¹ is present when X¹ is C and is double bonded to an adjacent atom; and two R⁴¹ are present when X¹ is C and is not double bonded to an adjacent atom. Each R⁴³ may be the same or different, provided that for each X³: R⁴³ is absent when X³ is O or divalent S; R⁴³ is absent when X³ is N and is double bonded to an adjacent atom; one R⁴³ is present when X³ is N and is not double bonded to an adjacent atom; one R⁴³ is present when X³ is C and is double bonded to an adjacent atom; and two R⁴³ are present when X³ is C and is not double bonded to an adjacent atom. For each X⁶: R⁴³ is absent when X⁶ is N or is C and is double bonded to an adjacent atom; and one R⁴³ is present when X⁶ is C and not double bonded to an adjacent atom. For the X³ with which it is associated: R⁴⁴ is absent when X³ is O or divalent S; R⁴⁴ is absent when X³ is N and is double bonded to an adjacent atom; one R⁴⁴ is present when X³ is N and is not double bonded to an adjacent atom; one R⁴⁴ is present when X³ is C and is double bonded to an adjacent atom; and one R⁴⁴ is present when X³ is C and the C is attached to R⁴³ and not double bonded to an adjacent atom. Each R⁴² may be the same or different, provided that for each X²: R⁴² is absent when X² is O or divalent S; R⁴² is absent when X² is N and is double bonded to an adjacent atom; one R⁴² is present when X² is N and is not double bonded to an adjacent atom; one R⁴² is present when X² is C and is double bonded to an adjacent atom; and two R⁴² are present when X² is C and is not double bonded to an adjacent atom. For X⁵: R⁴² is absent when X⁵ is N or is C and is double bonded to an adjacent atom; and one R⁴² is present when X⁵ is C and not double bonded to an adjacent atom. Each R¹¹ may be the same or different, provided that for each X⁴: R¹¹ is absent when X⁴ is O or divalent S; R¹¹ is absent when X⁴ is N and is double bonded to an adjacent atom; one R¹¹ is present when X⁴ is N and is not double bonded to an adjacent atom; one R¹¹ is present when X⁴ is C and is double bonded to an adjacent atom; and two R¹¹ are present when X⁴ is C and is not double bonded to an adjacent atom. Each R¹⁴ bonded to N may be the same or different, provided that R¹⁴ is absent when the N is double bonded to an adjacent atom.

In these compounds, and elsewhere herein, in some embodiments any R group may form a ring with any other R group on an adjacent and/or proximal atom, although in most embodiments this is not preferred, except where explicitly stated. Thus, in some embodiments the following substituents may together form a ring: R⁵ with R⁴⁴; R¹¹ with another R¹¹; R¹³ with another R¹³; R⁴¹ with another R⁴¹; R⁴² with another R⁴²; R⁴³ with another R⁴³; R⁴⁵ with another R⁴⁵. In the context of the present invention, an adjacent and/or proximal atom may mean another atom directly bonded to an atom (adjacent) or may be two atoms with only a single atom in between (proximal), or may mean two atoms close enough sterically to be capable of forming a ring (proximal). Preferably R groups attached to the same atom do not together form a ring, although this is not excluded.

In the present context the disclosure includes compounds in which a single R group on an atom, or two R groups on the same atom, form a group which is double bonded to that atom. Accordingly, an R group, or two R groups attached to the same atom, may together form a =O group, or a =C(R')₂ group (wherein each R' group is the same or different and is H or an organic group, preferably H or a straight or branched C₁-C₆ alkyl group). This is more typical in cases where the R groups are attached to a C atom, such that together they form a C=O group or a C=C(R')₂ group. Thus is some cases a C ring atom in a ring may comprise a =O group, as may an X, and/or one or more of R¹¹, R⁴¹, R⁴², R⁴³, R⁴⁴ and R⁴⁵.

In the present context a part of any structure present in brackets may be repeated the number of times given by the numbers next to the brackets (whether regular brackets or square brackets). For example, in the case of (C(R))_{0,1,2} or [C(R)]_{0,1,2} the C-R group may be absent, present once i.e. -C(R)-; or present twice i.e. -C(R)-C(R)-.

In the context of the present disclosure, a compound is considered to be a PARP1 inhibitor if its presence is capable of preventing or reducing the ability of immobilised PARP1 to undergo auto-poly-ADP ribosylation (AutoPARylation) following incubation with biotinylated-NAD+ as compared to the same process in its absence. Typically, the compound is considered to be a PARP1 inhibitor if it has an IC₅₀ < 10 µM in a suitable assay. A suitable assay may be conducted using 2 nM PARP1, 2 µM biotin-NAD⁺ assay solution in 20 mM HEPES (pH 7.5), 100 mM NaCl, 2 mM DTT, 0.1 % BSA (w/v), 0.02 % Tween (v/v) assay buffer. PARylation may take place for 2 h at room temperature and may be detected using a dissociation-enhanced lanthanide fluorescence immunoassay (DELFIA) readout. A particularly suitable assay is described in the Examples below. Preferably, the compound has an IC₅₀ < 1 µM, more preferably < 100 nM and most preferably < 10 nM in the PARP1 inhibitor assay.

A compound is also considered to be a selective PARP1 inhibitor if its presence is capable of displacing or reducing the ability of a high affinity Cy5 fluorescent dye-labelled chemical probe to bind to PARP1 whilst displacing the same chemical probe at PARP2 with at least 10-fold weaker activity. Typically, the compound is considered to be a selective PARP1 inhibitor if it has an IC₅₀ < 10 µM in this assay at PARP1 with at least 10-fold selectivity preference over PARP2. A suitable such assay may be conducted for 1 h at room temperature using 10 nM PARP1 or PARP2, Tb-cryptate antibody and PARP1/2 binding probe in 20 mM HEPES (pH 7.5), 100 mM NaCl, 2 mM DTT, 0.1 % BSA (w/v), 0.02 % Tween (v/v) assay buffer. Probe binding displacement may be detected using homogeneous time-resolved fluorescence. A particularly suitable assay is described in the Examples below. Preferably the selectivity preference of PARP1 over PARP2 is at least 50-fold, more preferably at least 100-fold.

The compound is also considered to be a selective PARP1 inhibitor if it has an IC₅₀ < 10 µM at PARP1 with at least 10-fold selectivity preference over PARP2 in NanoBRET assays demonstrating cellular target engagement. These assays are based on bioluminescence resonance energy transfer (BRET) between a Nano-luc-tagged protein (eg PARP1 or PARP2) and a fluorescent group on a high affinity NAD⁺ competitive binding probe. Such cellular probe displacement assays can be utilised to measure inhibitor affinities and selectivity ratios at PARP1 and 2. A particularly suitable assay is described in the Examples below. Preferably the selectivity preference of PARP1 over PARP2 is at least 50-fold, more preferably at least 100-fold.

In the compounds of this disclosure (both above and below herein), the substituents (each of the R groups) are not especially limited, provided that they do not prevent the PARP1 inhibitory function from occurring. In all of the compounds mentioned in connection with this disclosure, both above and in the following, the substituents are selected from H and an organic group. Thus, both above and in the following, the terms 'substituent' and 'organic group' are not especially limited and may be any functional group or any atom, especially any functional group or atom common in organic chemistry. Thus, 'substituent' and 'organic group' may have any of the following meanings.

The organic group may comprise any one or more atoms from any of groups IIIA, IVA, VA, VIA or VIIA of the Periodic Table, such as a B, Si, N, P, O, or S atom (e.g. OH, OR, NH₂, NHR, NR₂, SH, SR, SO₂R, SO₃H, PO₄H₂) or a halogen atom (e.g. F, Cl, Br or I) where R is a linear or branched lower hydrocarbon (1-6 C atoms) or a linear or branched higher hydrocarbon (7 C atoms or more, e.g. 7-40 C atoms).

The organic group preferably comprises a hydrocarbon group. The hydrocarbon group may comprise a straight chain, a branched chain or a cyclic group. Independently, the hydrocarbon group may comprise an aliphatic or an aromatic group. Also independently, the hydrocarbon group may comprise a saturated or unsaturated group.

When the hydrocarbon comprises an unsaturated group, it may comprise one or more alkene functionalities and/or one or more alkyne functionalities. When the hydrocarbon comprises a straight or branched chain group, it may comprise one or more primary, secondary and/or tertiary alkyl groups.

When the hydrocarbon comprises a cyclic group it may comprise an aromatic ring, a non-aromatic ring, an aliphatic ring, a heterocyclic group, and/or fused ring derivatives of these groups. The ring may be fully saturated, partially saturated, or fully unsaturated. The cyclic group may thus comprise a benzene, naphthalene, anthracene, phenanthrene, phenalene, biphenylene, pentalene, indene, as-indacene, s-indacene, acenaphthylene, fluorene, fluoranthene, acephenanthrylene, azulene, heptalene, pyrrole, pyrazole, imidazole, 1,2,3-triazole, 1,2,4-triazole, tetrazole, pyrrolidine, furan, oxetane, tetrahydrofuran, 2-aza-tetrahydrofuran, 3-aza-tetrahydrofuran, oxazole, isoxazole, furazan, 1,2,4-oxadiazol, 1,3,4-oxadiazole, thiophene, isothiazole, thiazole, thiolane, pyridine, pyridazine, pyrimidine, pyrazine, piperidine, 2-azapiperidine, 3-azapiperidine, piperazine, pyran, tetrahydropyran, 2-azapyran, 3-azapyran, 4-azapyran, 2-aza-tetrahydropyran, 3-aza-tetrahydropyran, morpholine, thiopyran, 2-azathiopyran, 3-azathiopyran, 4-azathiopyran, thiane, indole, indazole, benzimidazole, 4-azaindole, 5-azaindole, 6-azaindole, 7-azaindole, isoindole, 4-azaisoindole, 5-azaisoindole, 6-azaisoindole, 7-azaisoindole, indolizine, 1-azaindolizine, 2-azaindolizine, 3-azaindolizine, 5-azaindolizine, 6-azaindolizine, 7-azaindolizine, 8-azaindolizine, 9-azaindolizine, purine, carbazole, carboline, benzofuran, isobenzofuran, benzothiophene, isobenzothiophene, quinoline, cinnoline, quinazoline, quinoxaline, 5-azaquinoline, 6-azaquinoline, 7-azaquinoline, isoquinoline, phthalazine, 6-azaisoquinoline, 7-azaisoquinoline, pteridine, chromene, isochromene, acridine, phenanthridine, perimidine, phenanthroline, phenoxazine, xanthene, phenoxanthiin, and/or thianthrene, as well as regioisomers of the above groups. These groups may generally be attached at any point in the group, and also may be attached at a hetero-atom or at a carbon atom. In some instances particular attachment points are preferred, such as at 1-yl, 2-yl and the like, and these are specified explicitly where appropriate. All tautomeric ring forms are included in these definitions. For example pyrrole is intended to include 1*H*-pyrrole, 2*H*-pyrrole and 3*H*-pyrrole.

The number of carbon atoms in the hydrocarbon group is not especially limited, but preferably the hydrocarbon group comprises from 1-40 C atoms. The hydrocarbon group may thus be a lower hydrocarbon (1-6 C atoms) or a higher hydrocarbon (7 C atoms or more, e.g. 7-40 C atoms). The lower hydrocarbon group may be a methyl, ethyl, propyl, butyl, pentyl or hexyl group or regioisomers of these, such as isopropyl, isobutyl, tert-butyl, etc. The number of atoms in the ring of the cyclic group is not especially limited, but preferably the ring of the cyclic group comprises from 3-10 atoms, such as 3, 4, 5, 6, 7, 8, 9 or 10 atoms.

The groups comprising heteroatoms described above, as well as any of the other groups defined above, may comprise one or more heteroatoms from any of groups IIIA, IVA, VA, VIA or VIIA of the Periodic Table, such as a B, Si, N, P, O, or S atom or a halogen atom (e.g. F, Cl, Br or I). Thus, the substituent may comprise one or more of any of the common functional groups in organic chemistry, such as hydroxy groups, carboxylic acid groups, ester groups, ether groups, aldehyde groups, ketone groups, amine groups, amide groups, imine groups, thiol groups, thioether groups, sulphate groups, sulphonic acid groups, sulphonyl groups, and phosphate groups etc. The substituent may also comprise derivatives of these groups, such as carboxylic acid anhydrides and carboxylic acid halides.

In addition, any substituent may comprise a combination of two or more of the substituents and/or functional groups defined above.

The rings A, B (when present) and C of the compounds of the present disclosure form a bicyclic or tricyclic ring structure (which may comprise further fused rings when the substituents on either ring themselves form a ring). Each of rings A, B, C and D are not necessarily limited, provided that they do not prevent the PARP1 inhibitory function from occurring. Rings A, B, C and D may independently be comprised of an aromatic ring, a non-aromatic ring, an aliphatic ring, and/or a heterocyclic group. The rings may be fully saturated, partially saturated, or fully unsaturated. Each ring may thus independently comprise a benzene, naphthalene, anthracene, phenanthrene, phenalene, biphenylene, pentalene, indene, as-indacene, s-indacene, acenaphthylene, fluorene, fluoranthene, acephenanthrylene, azulene, heptalene, pyrrole, pyrazole, imidazole, 1,2,3-triazole, 1,2,4-triazole, tetrazole, pyrrolidine, oxetane, furan, tetrahydrofuran, 2-aza-tetrahydrofuran, 3-aza-tetrahydrofuran, oxazole, isoxazole, furazan, 1,2,4-oxadiazol, 1,3,4-oxadiazole, thiophene, isothiazole, thiazole, thiolane, pyridine, pyridazine, pyrimidine, pyrazine, piperidine, 2-azapiperidine, 3-azapiperidine, piperazine, pyran, tetrahydropyran, 2-azapyran, 3-azapyran, 4-azapyran, 2-aza-tetrahydropyran, 3-aza-tetrahydropyran, morpholine, thiopyran, 2-azathiopyran, 3-azathiopyran, 4-azathiopyran, thiane, indole, indazole, benzimidazole, 4-azaindole, 5-azaindole, 6-azaindole, 7-azaindole, isoindole, 4-azaisoindole, 5-azaisoindole, 6-azaisoindole, 7-azaisoindole, indolizine, 1-azaindolizine, 2-azaindolizine, 3-azaindolizine, 5-azaindolizine, 6-azaindolizine, 7-azaindolizine, 8-azaindolizine, 9-azaindolizine, purine, carbazole, carboline, benzofuran, isobenzofuran, benzothiophene, isobenzothiophene, quinoline, cinnoline, quinazoline, quinoxaline, 5-azaquinoline, 6-azaquinoline, 7-azaquinoline, isoquinoline, phthalazine, 6-azaisoquinoline, 7-azaisoquinoline, pteridine, chromene, isochromene, acridine, phenanthridine, perimidine, phenanthroline, phenoxazine, xanthene, phenoxanthiin, and/or thianthrene, as well as regioisomers of the above groups. These rings may generally be substituted at any point in the group, and also may be substituted at a hetero-atom or at a carbon atom. All tautomeric ring forms are included in these definitions. For example, pyrrole is intended to include 1H-pyrrole, 2H-pyrrole and 3H-pyrrole.

In typical examples, the disclosure provides a compound as defined above, wherein Q³ is absent or is a group independently selected from the following structures: 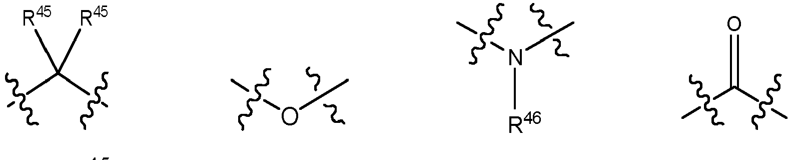 wherein each R⁴⁵ may be the same or different and is independently selected from H and a substituted or unsubstituted organic group; and R⁴⁶ is selected from H and a substituted or unsubstituted organic group. It is preferred that Q³ is absent or is 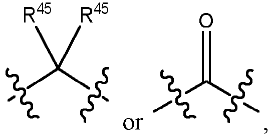 such as a -CH₂- group.

In typical examples, the disclosure provides a compound as defined above, wherein Q² is present and each Q¹ is independently absent or is a group independently selected from the following structures: 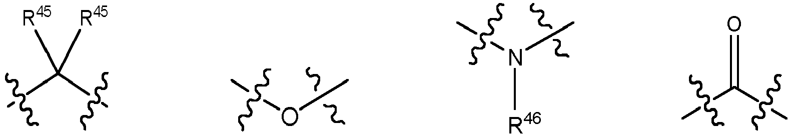 wherein each R⁴⁵ may be the same or different and is independently selected from H and a substituted or unsubstituted organic group; and R⁴⁶ is selected from H and a substituted or unsubstituted organic group. It is preferred that Q¹ is absent or is 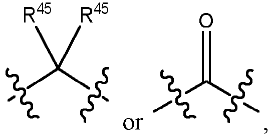 such as a -CH₂- group, particularly when Q² is present.

The Q¹ group bound to ring A is preferably 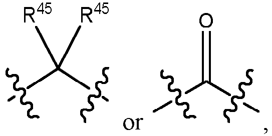 such as a -CH₂- group.

In some examples, the disclosure provides a compound as defined above, wherein the group L comprises a group having the following structure: 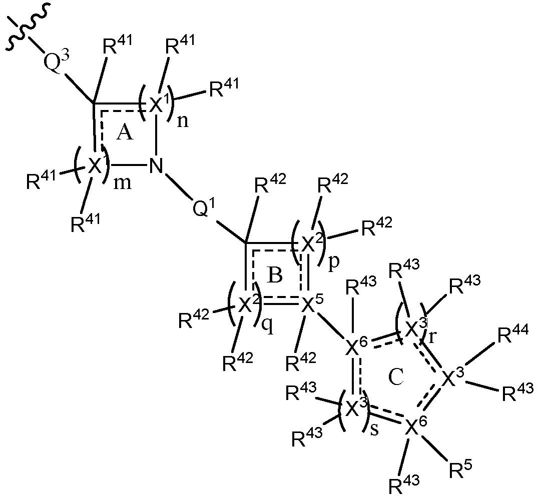 wherein Q¹ is absent or is a group as defined above, Q³ is absent or is a group as defined above, and n, m, p, q, r, s, X¹, X², X³, X⁵, X⁶, R⁴¹, R⁴², R⁴³, R⁴⁴, R⁵, and rings A, B, and C are as defined above. It is preferred that X⁵ is N with no R⁴² substituent.

In some of the disclosed compounds, the group L comprises a group having the following structure: 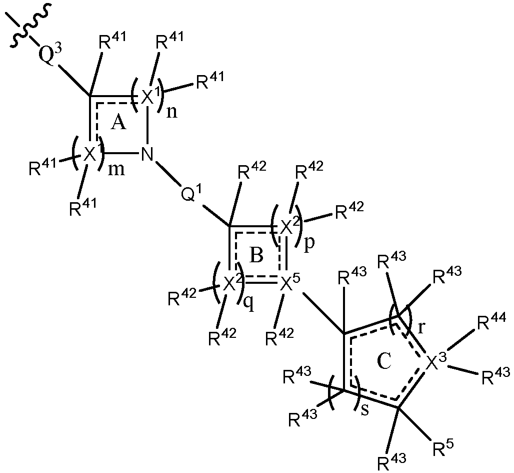 wherein n, m, p, q, r, s, X¹, X², X³, X⁵, R⁴¹, R⁴², R⁴³, R⁴⁴, R⁵, Q¹ , Q³ and rings A, B, and C are as defined above. It is preferred that X⁵ is N with no R⁴² substituent.

In some of the disclosed compounds, the group L comprises a group having the following structure: 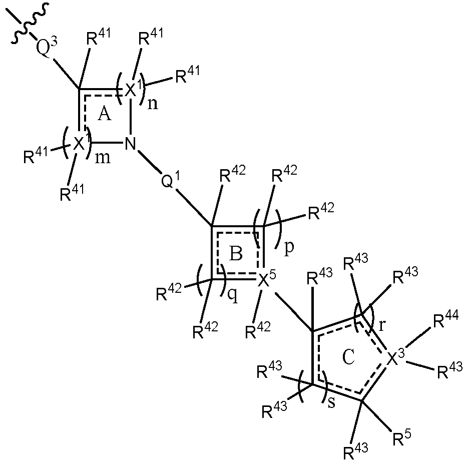 wherein n, m, p, q, r, s, X¹, X³, X⁵, R⁴¹, R⁴², R⁴³, R⁴⁴, R⁵, Q¹, Q³ and rings A, B, and C are as defined above. It is preferred that X⁵ is N with no R⁴² substituent.

In some of the disclosed compounds, the group L comprises a group having the following structure: 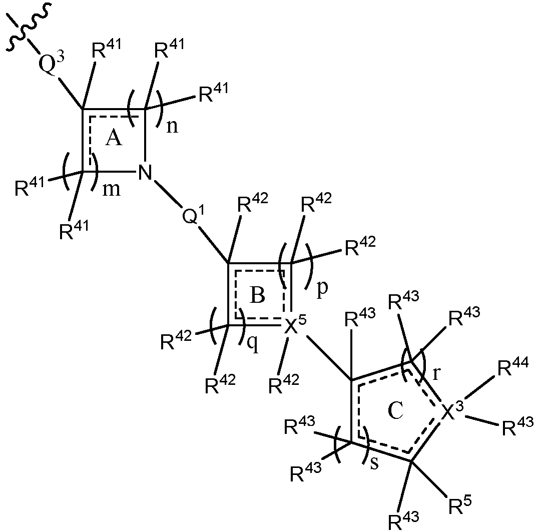 wherein n, m, p, q, r, s, X³, X⁵, R⁴¹, R⁴², R⁴³, R⁴⁴, R⁵, and rings A, B, and C are as defined above. It is preferred that X⁵ is N with no R⁴² substituent.

In compounds of the invention, the group L comprises a group having the following structure: 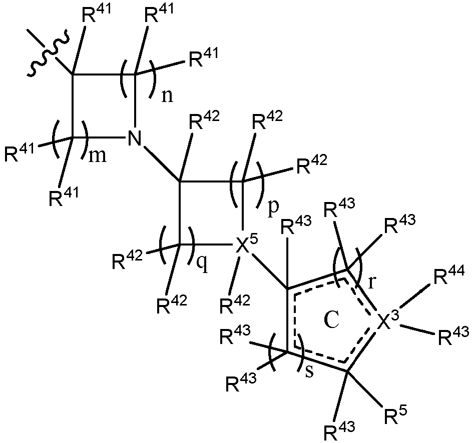 wherein n, m, p, q, r, s, X³, X⁵, R⁴¹, R⁴², R⁴³, R⁴⁴, R⁵, and ring C, are as defined in the claims. It is preferred that X⁵ is N with no R⁴² substituent.

In some of the disclosed compounds, the group L comprises a group having the following structure: 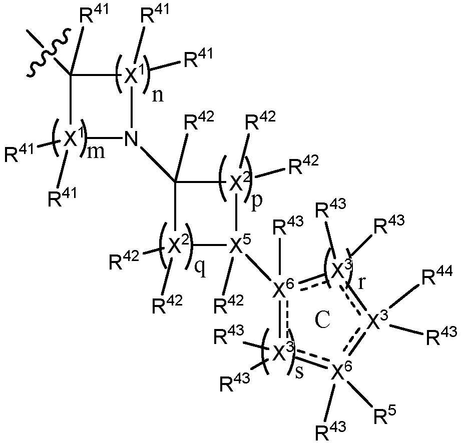 wherein n, m, p, q, r, s, X¹, X², X³, X⁵, X⁶, R⁴¹, R⁴², R⁴³, R⁴⁴, R⁵, and ring C, are as defined above. It is preferred that X⁵ is N with no R⁴² substituent.

In some of the disclosed compounds, the group L comprises a group selected from the following structures: 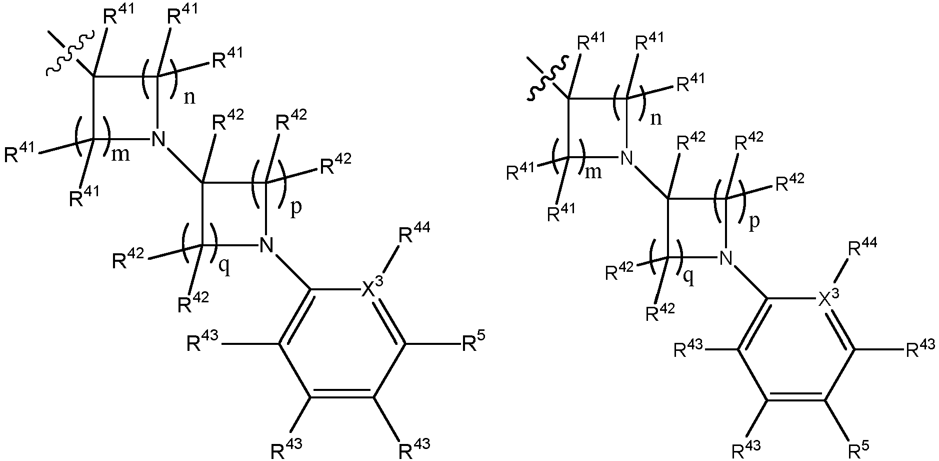 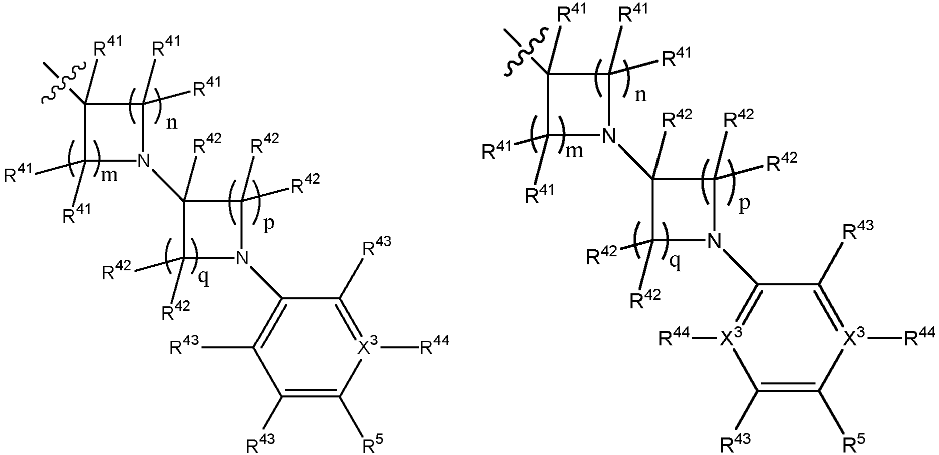 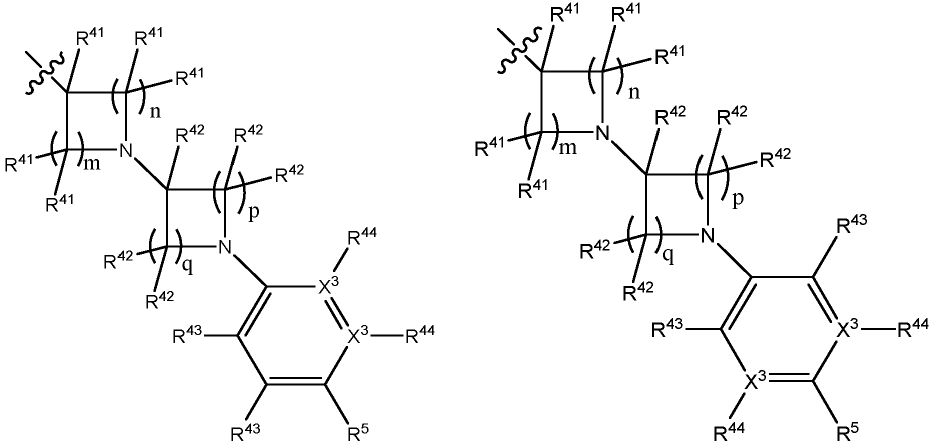 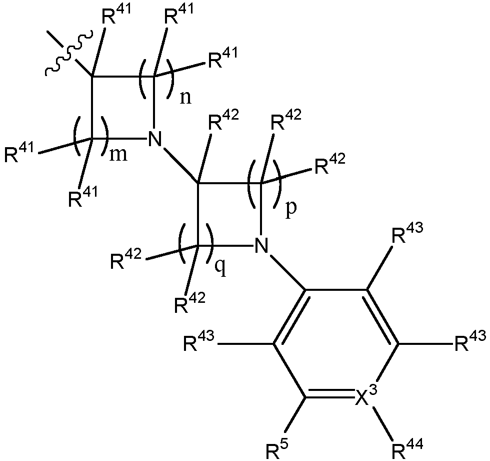 wherein n, m, p, q, X³, R⁴¹, R⁴², R⁴³, R⁴⁴, and R⁵, are as defined above.

In these structures for the L group it is preferred that m is selected from 1 or 2, n is selected from 2 or 3, p is selected from 1, 2 or 3 (more preferably for 2 or 3) and q is selected from 1 or 2.

In some embodiments of the invention, group L comprises a group having any the following structures: 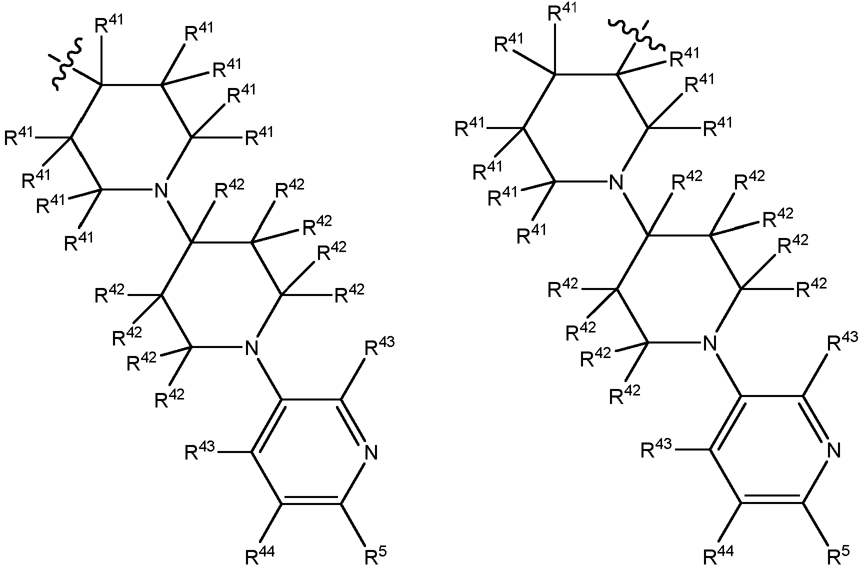 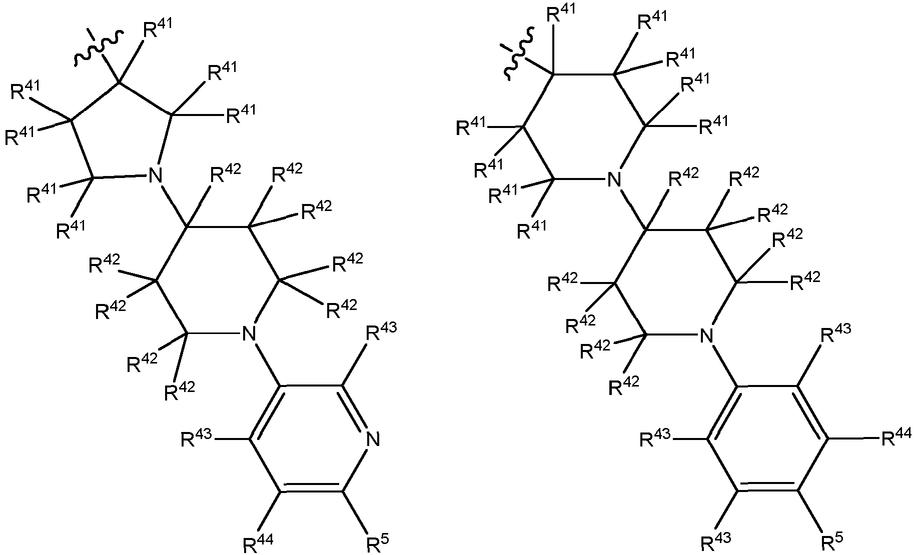 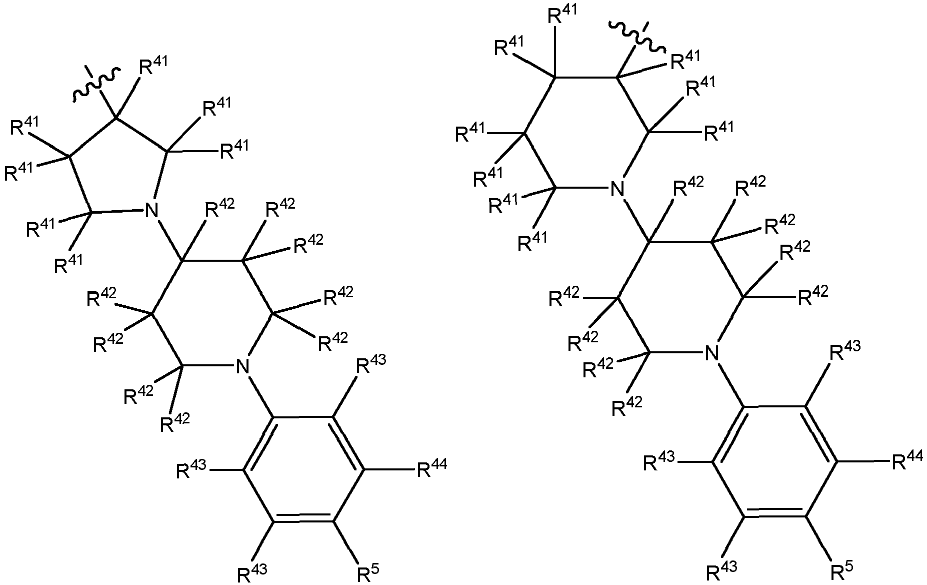 wherein, R⁴¹, R⁴², R⁴³, R⁴⁴, and R⁵ are as defined in the claims.

Typically, in the disclosed compounds, R⁴¹, R⁴², R⁴³, R⁴⁴ and R⁴⁵ are each independently selected from H and a group selected from the following groups (the following list being more general than the list defined in claim 1):
- deuterium;
- a halogen (such as -F, -Cl, -Br and -I);
- a nitrile group;
- a substituted or unsubstituted linear or branched C₁-C₆ alkyl group (such as Me, Et, Pr, i-Pr, n-Bu, i-Bu, t-Bu, pentyl and hexyl);
- a substituted or unsubstituted linear or branched C₁-C₆ alkyl-aryl group (such as -CH₂Ph,-CH₂(2,3 or 4)F-Ph, -CH₂(2,3 or 4)Cl-Ph, -CH₂(2,3 or 4)Br-Ph, -CH₂(2,3 or 4)I-Ph,-CH₂CH₂Ph, -CH₂CH₂CH₂Ph, -CH₂CH₂CH₂CH₂Ph, -CH₂CH₂CH₂CH₂CH₂Ph, and -CH₂CH₂CH₂CH₂CH₂CH₂Ph);
- a substituted or unsubstituted linear or branched C₁-C₆ halogenated alkyl group (such as -CH₂F, -CH₂Cl, -CH₂Br, -CH₂I, -CF₃, -CCl₃ -CBr₃, -CI₃, -CH₂CF₃, -CH₂CCl₃, -CH₂CBr₃, and -CH₂CI₃);
- -NH₂ or a substituted or unsubstituted linear or branched primary secondary or tertiary C₁-C₆ amine group (such as -NMeH, -NMe₂, -NEtH, -NEtMe, -NEt₂, -NPrH, -NPrMe, -NPrEt, -NPr₂, -NBuH, -NBuMe, -NBuEt, -CH₂-NH₂, -CH₂-NMeH, -CH₂-NMe₂, -CH₂-NEtH, -CH₂-NEtMe, -CH₂-NEt₂, -CH₂-NPrH, -CH₂-NPrMe, and -CH₂-NPrEt);
- a substituted or unsubstituted amino-aryl group (such as -NH-Ph, -NH-(2,3 or 4)F-Ph, -NH-(2,3 or 4)Cl-Ph, -NH-(2,3 or 4)Br-Ph, -NH-(2,3 or 4)I-Ph, -NH-(2,3 or 4)Me-Ph, -NH-(2,3 or 4)Et-Ph, -NH-(2,3 or 4)Pr-Ph, -NH-(2,3 or 4)Bu-Ph, NH-(2,3 or 4)OMe-Ph, -NH-(2,3 or 4)OEt-Ph, -NH-(2,3 or 4)OPr-Ph, -NH-(2,3 or 4)OBu-Ph, -NH-2,(3,4,5 or 6)F₂-Ph, -NH-2,(3,4,5 or 6)Cl₂-Ph, -NH-2,(3,4,5 or 6)Br₂-Ph, -NH-2,(3,4,5 or 6)I₂-Ph, -NH-2,(3,4,5 or 6)Me₂-Ph, -NH-2,(3,4,5 or 6)Et₂-Ph, -NH-2,(3,4,5, or 6)Pr₂-Ph, -NH-2,(3,4,5 or 6)Bu₂-Ph,
- a substituted or unsubstituted cyclic amine or amido group (such as pyrrolidin-1-yl, pyrrolidin-2-yl, pyrrolidin-3-yl, piperidin-1-yl, piperidin-2-yl, piperidin-3-yl, piperidin-4-yl, morpholin-2-yl, morpholin-3-yl, morpholin-4-yl, 2-keto-pyrrolidinyl, 3-keto-pyrrolidinyl, 2-keto-piperidinyl, 3-keto-piperidinyl, and 4-keto-piperidinyl);
- a substituted or unsubstituted cyclic C₃-C₈ alkyl group (such as cyclopropyl, cyclobutyl, cyclopentyl, cyclohexyl, cycloheptyl and cyclooctyl);
- an -OH group or a substituted or unsubstituted linear or branched C₁-C₆ alcohol group (such as -CH₂OH, -CH₂CH₂OH, -CH(CH₃)CH₂OH, -C(CH₃)₂OH, -CH₂CH₂CH₂OH,-CH₂CH₂CH₂CH₂OH, -CH(CH₃)CH₂CH₂OH, -CH(CH₃)CH(CH₃)OH, -CH(CH₂CH₃)CH₂OH, -C(CH₃)₂CH₂OH, -CH₂CH₂CH₂CH₂CH₂OH, and -CH₂CH₂CH₂CH₂CH₂CH₂OH); - a substituted or unsubstituted linear or branched C₁-C₆ carboxylic acid group (such as -COOH, -CH₂COOH, -CH₂CH₂COOH, -CH₂CH₂CH₂COOH, -CH₂CH₂CH₂CH₂COOH, and -CH₂CH₂CH₂CH₂CH₂COOH);
- a substituted or unsubstituted linear or branched carbonyl group (such as -(CO)Me, -(CO)Et, -(CO)Pr, -(CO)iPr, -(CO)nBu, -(CO)iBu, -(CO)tBu, -(CO)Ph, -(CO)CH₂Ph, -(CO)CH₂OH, -(CO)CH₂OCH₃, -(CO)CH₂NH₂, -(CO)CH₂NHMe, -(CO)CH₂NMe₂, -(CO)-cyclopropyl, -(CO)-1,3-epoxypropan-2-yl; -(CO)NH₂, -(CO)NHMe, -(CO)NMe₂, -(CO)NHEt, -(CO)NEt₂, -(CO)-pyrollidine-N-yl, -(CO)-morpholine-N-yl, -(CO)-piperazine-N-yl, -(CO)-N-methyl-piperazine-N-yl, -(CO)NHCH₂CH₂OH, -(CO)NHCH₂CH₂OMe, -(CO)NHCH₂CH₂NH₂, -(CO)NHCH₂CH₂NHMe, and -(CO)NHCH₂CH₂NMe₂;
- a substituted or unsubstituted linear or branched C₁-C₆ carboxylic acid ester group (such as -COOMe, -COOEt, -COOPr, -COO-i-Pr, -COO-n-Bu, -COO-i-Bu, -COO-t-Bu, -CH₂COOMe, -CH₂CH₂COOMe, -CH₂CH₂CH₂COOMe, and -CH₂CH₂CH₂CH₂COOMe);
- a substituted or unsubstituted linear or branched C₁-C₆ amide group (such as -CO-NH₂,-CO-NMeH, -CO-NMe₂, -CO-NEtH, -CO-NEtMe, -CO-NEt₂, -CO-NPrH, -CO-NPrMe, and-CO-NPrEt);
- a substituted or unsubstituted linear or branched C₁-C₇ amino carbonyl group (such as -NH-CO-Me, -NH-CO-Et, -NH-CO-Pr, -NH-CO-Bu, -NH-CO-pentyl, -NH-CO-hexyl, -NH-CO-Ph, -NMe-CO-Me, -NMe-CO-Et, -NMe-CO-Pr, -NMe-CO-Bu, -NMe-CO-pentyl, -NMe-CO-hexyl, -NMe-CO-Ph;
- a substituted or unsubstituted linear or branched C₁-C₇ alkoxy or aryloxy group (such as-OMe, -OEt, -OPr, -O-i-Pr, -O-n-Bu, -O-i-Bu, -O-t-Bu, -O-pentyl, -O-hexyl, -OCH₂F, -OCHF₂, -OCF₃, -OCH₂Cl, -OCHCl₂, -OCCl₃, -O-Ph, -O-CH₂-Ph, -O-CH₂-(2,3 or 4)-F-Ph, -O-CH₂-(2,3 or 4)-Cl-Ph, -CH₂OMe, -CH₂OEt, -CH₂OPr, -CH₂OBu, -CH₂CH₂OMe, -CH₂CH₂CH₂OMe, -CH₂CH₂CH₂CH₂OMe, and -CH₂CH₂CH₂CH₂CH₂OMe);
- a substituted or unsubstituted linear or branched aminoalkoxy group (such as-OCH₂NH₂, -OCH₂NHMe, -OCH₂NMe₂, -OCH₂NHEt, -OCH₂NEt₂, -OCH₂CH₂NH₂, -OCH₂C H₂NHMe, -OCH₂CH₂NMe₂, -OCH₂CH₂NHEt, and -OCH₂CH₂NEt₂;
- a substituted or unsubstituted sulphonyl group (such as -SO₂Me, -SO₂Et, -SO₂Pr, -SO₂iPr,-SO₂Ph, -SO₂-(2,3 or 4)-F-Ph, -SO₂-cyclopropyl, -SO₂CH₂CH₂OCH₃), -SO₂NH₂, -SO₂NHMe, -SO₂NMe₂, -SO₂NHEt, -SO₂NEt₂, -SO2-pyrrolidine-N-yl, -SO₂-morpholine-N-yl, -SO₂NHCH₂OMe, and -SO₂NHCH₂CH₂OMe;
- a substituted or unsubstituted aminosulphonyl group (such as -NHSO₂Me, - NHSO₂Et,-NHSO₂Pr, - NHSO₂iPr, - NHSO₂Ph, - NHSO₂-(2,3 or 4)-F-Ph, - NHSO₂-cyclopropyl, - NHSO₂CH₂CH₂OCH₃);
- a substituted or unsubstituted aromatic group (such as Ph-, 2-F-Ph-, 3-F-Ph-, 4-F-Ph-, 2-Cl-Ph-, 3-Cl-Ph-, 4-Cl-Ph-, 2-Br-Ph-, 3-Br-Ph-, 4-Br-Ph-, 2-I-Ph-, 3-I-Ph, 4-I-Ph-, 2,(3,4,5 or 6)-F₂-Ph-, 2,(3,4,5 or 6)-Cl₂-Ph-, 2,(3,4,5 or 6)-Br₂-Ph-, 2,(3,4,5 or 6)-I₂-Ph-, 2,(3,4,5 or 6)-Me₂-Ph-, 2,(3,4,5 or 6)-Et₂-Ph-, 2,(3,4,5 or 6)-Pr₂-Ph-, 2,(3,4,5 or 6)-Bu₂-Ph-, 2,(3,4,5 or 6)-(CN)₂-Ph-, 2,(3,4,5 or 6)-(NO₂)₂-Ph-, 2,(3,4,5 or 6)-(NH₂)₂-Ph-, 2,(3,4,5 or 6)-(MeO)₂-Ph-, 2,(3,4,5 or 6)-(CF₃)₂-Ph-, 3,(4 or 5)-F₂-Ph-, 3,(4 or 5)-Cl₂-Ph-, 3,(4 or 5)-Br₂-Ph-, 3,(4 or 5)-I₂-Ph-, 3,(4 or 5)-Me₂-Ph-, 3,(4 or 5)-Et₂-Ph-, 3,(4 or 5)-Pr₂-Ph-, 3,(4 or 5)-Bu₂-Ph-, 3,(4 or 5)-(CN)₂-Ph-, 3,(4 or 5)-(NO₂)₂-Ph-, 3,(4 or 5)-(NH₂)₂-Ph-, 3,(4 or 5)-(MeO)₂-Ph-, 3,(4 or 5)-(CF₃)₂-Ph-, 2-Me-Ph-, 3-Me-Ph-, 4-Me-Ph-, 2-Et-Ph-, 3-Et-Ph-, 4-Et-Ph-, 2-Pr-Ph-, 3-Pr-Ph-, 4-Pr-Ph-, 2-Bu-Ph-, 3-Bu-Ph-, 4-Bu-Ph-, 2-(CN)-Ph-, 3-(CN)-Ph-, 4-(CN)-Ph-, 2-(NO₂)-Ph-, 3-(NO₂)-Ph-, 4-(NO₂)-Ph-, 2-(NH₂)-Ph-, 3-(NH₂)-Ph-, 4-(NH₂)-Ph-, 2-MeO-Ph-, 3-MeO-Ph-, 4-MeO-Ph-, 2-(NH₂-CO)-Ph-, 3-(NH₂-CO)-Ph-, 4-(NH₂-CO)-Ph-, 2-CF₃-Ph-, 3-CF₃-Ph-, 4-CF₃-Ph-, 2-CF₃O-Ph-, 3-CF₃O-Ph-, and 4-CF₃O-Ph-);
- a saturated or unsaturated, substituted or unsubstituted, heterocyclic group including an aromatic heterocyclic group and/or a non-aromatic heterocyclic group (such as pyrrole-1-yl, pyrrole-2-yl, pyrrole-3-yl, pyrazole-1-yl, pyrazole-3-yl, pyrazole-4-yl, pyrazole-5-yl, imidazole-1-yl, imidazole-2-yl, imidazole-4-yl, imidazole-5-yl, 1,2,3-triazole-1-yl, 1,2,3-triazole-4-yl, 1,2,3-triazole-5-yl, 1,2,4-triazole-1-yl, 1,2,4-triazole-3-yl, 1,2,4-triazole-5-yl, pyridin-2-yl, pyridin-3-yl, pyridin-4-yl, pyridazine-3-yl, pyridazine-4-yl, pyrimidin-2-yl, pyrimidin-4-yl, pyrimidin-5-yl, pyrimidin-6-yl, pyrazine-2-yl, pyrrolidine-1-yl, pyrrolidine-2-yl, pyrrolidine-3-yl, piperidine-1-yl, piperidine-2-yl, piperidine-3-yl, piperidine-4-yl, 2-azapiperidine-1-yl, 2-azapiperidine-3-yl, 2-azapiperidine-4-yl, 3-azapiperidine-1-yl, 3-azapiperidine-2-yl, 3-azapiperidine-4-yl, 3-azapiperidine-5-yl, piperazine-1-yl, piperazine-2-yl, furan-2-yl, furan-3-yl, pyran-2-yl, pyran-3-yl, pyran-4-yl, 2-azapyran-2-yl, 2-azapyran-3-yl, 2-azapyran-4-yl, 2-azapyran-5-yl, 2-azapyran-6-yl, 3-azapyran-2-yl, 3-azapyran-4-yl, 3-azapyran-5-yl, 3-azapyran-6-yl, 4-azapyran-2-yl, 4-azapyran-3-yl, 4-azapyran-4-yl, 4-azapyran-5-yl, 4-azapyran-6-yl, oxetan-2-yl, oxetan-3-yl, tetrahydrofuran-2-yl, tetrahydrofuran-3-yl, 2-aza-tetrahydrofuran-2-yl, 2-aza-tetrahydrofuran-3-yl, 2-aza-tetrahydrofuran-4-yl, 2-aza-tetrahydrofuran-5-yl, 3-aza-tetrahydrofuran-2-yl, 3-aza-tetrahydrofuran-3-yl, 3-aza-tetrahydrofuran-4-yl, 3-aza-tetrahydrofuran-5-yl, tetrahydropyran-2-yl, tetrahydropyran-3-yl, tetrahydropyran-4-yl, 2-aza-tetrahydropyran-2-yl, 2-aza-tetrahydropyran-3-yl, 2-aza-tetrahydropyran-4-yl, 2-aza-tetrahydropyran-5-yl, 2-aza-tetrahydropyran-6-yl, 3-aza-tetrahydropyran-2-yl, 3-aza-tetrahydropyran-3-yl, 3-aza-tetrahydropyran-4-yl, 3-aza-tetrahydropyran-5-yl, 3-aza-tetrahydropyran-6-yl, morpholine-2-yl, morpholine-3-yl, morpholine-4-yl, thiophen-2-yl, thiophen-3-yl, isothiazole-3-yl, isothiazole-4-yl, isothiazole-5-yl, thiazole-2-yl, thiazole-4-yl, thiazole-5-yl, thiopyran-2-yl, thiopyran-3-yl, thiopyran-4-yl, 2-azathiopyran-2-yl, 2-azathiopyran-3-yl, 2-azathiopyran-4-yl, 2-azathiopyran-5-yl, 2-azathiopyran-6-yl, 3-azathiopyran-2-yl, 3-azathiopyran-4-yl, 3-azathiopyran-5-yl, 3-azathiopyran-6-yl, 4-azathiopyran-2-yl, 4-azathiopyran-3-yl, 4-azathiopyran-4-yl, 4-azathiopyran-5-yl, 4-azathiopyran-6-yl, thiolane-2-yl, thiolane-3-yl, thiane-2-yl, thiane-3-yl, thiane-4-yl, oxazol-2-yl, oxazol-4-yl, oxazol-5-yl, isoxazol-3-yl, isoxazol-4-yl, isoxazol-5-yl, furazan-3-yl, (1,3,4-oxadiazol)-2-yl, (1,3,4-oxadiazol)-5-yl, (1,2,4-oxadiazol)-3-yl, (1,2,4-oxadiazol)-5-yl; and tetrazole-1-yl, tetrazole-2-yl, tetrazole-5-yl);
- where there are two R groups attached to the same atom, they may together form a group which is double bonded to that atom, (such as a carbonyl group (=O) or an alkene group (=C(R')₂) wherein each R' group is the same or different and is H or an organic group, preferably H or a straight or branched C₁-C₆ alkyl group).

In some arrangements, a pair of R⁴¹ groups attached to different atoms may together form a ring with ring A atoms, and/or a pair of R⁴² groups attached to different atoms may together form a ring with ring B atoms, optionally wherein each of the pair of R⁴¹ groups and/or pair of R⁴² groups independently comprises (X⁷)_{1 or 2}, wherein each X⁷ may be the same or different and is independently selected from C, N, O and S; and wherein each X⁷ is independently unsubstituted or (i) independently substituted with H or an organic group selected from a C₁-C₆ alkyl group, a C₁-C₆ halogenated alkyl group, a halogen such as F, or hydroxyl, when X⁷ is C; and (ii) independently substituted with H or an organic group selected from a C₁-C₆ alkyl group or a C₁-C₆ amide group, when X⁷ is N.

In some arrangements, groups R⁵ and R⁴⁴ may together form a ring with atoms X⁶ and X³ of ring C to which they are attached, optionally wherein the R⁵ and R⁴⁴ groups together comprise (X⁸)_{3, 4 or 5}, wherein each X⁸ may be the same or different and is independently selected from C, N, O and S; and wherein each X⁸ is independently unsubstituted or (i) independently substituted with H or an organic group selected from a C₁-C₆ alkyl group, a C₁-C₆ halogenated alkyl group, a halogen such as F, or hydroxyl, when X⁸ is C; and (ii) independently substituted with H or an organic group selected from a C₁-C₆ alkyl group, a C₁-C₆ halogenated alkyl group, or a C₁-C₆ amide group, when X⁸ is N.

In the compounds of the invention, R⁴¹, R⁴², R⁴³ and R⁴⁴ are each independently selected from H, deuterium, a halogen (such as -F, -Cl, -Br, and -I, preferably F or Cl), a nitrile group, a substituted or unsubstituted C₁-C₆ alkyl group, a substituted or unsubstituted linear or branched C₁-C₆ halogenated alkyl group, (preferably CF₃ or CHF₂), a cyclopropyl group, an - OH group or a substituted or unsubstituted linear or branched C₁-C₆ alcohol group, a substituted or unsubstituted linear or branched C₁-C₇ amino carbonyl group (such as -NH-CO-Me), an -NH₂ group or a substituted or unsubstituted C₁-C₆ amino group and a substituted or unsubstituted C₁-C₆ alkoxy group; wherein, when a pair of R⁴¹ groups attached to different atoms together forms a ring with ring A atoms, and/or a pair of R⁴² groups attached to different atoms together forms a ring with ring B atoms, each of the pair of R⁴¹ groups and/or pair of R⁴² groups independently comprises -CH₂- or -CH₂CH₂-; and wherein, when groups R⁵ and R⁴⁴ together form a ring with atoms of ring C, R⁵ and R⁴⁴ together comprise -CH=CH-CH=CH- or -NH-CO-NH-.

It is preferred that R⁴⁵ is selected from H, a halogen (such as -F, -Cl, -Br, and -I, preferably-F), a substituted or unsubstituted C₁-C₆ alkyl group, a substituted or unsubstituted linear or branched C₁-C₆ halogenated alkyl group (preferably CF₃), an -NH₂ group or a substituted or unsubstituted C₁-C₆ amino group, an -OH group or a substituted or unsubstituted linear or branched C₁-C₆ alcohol group and a substituted or unsubstituted C₁-C₆ alkoxy group.

Typically, R⁴⁶ is selected from H and a group selected from the following groups:
- a substituted or unsubstituted linear or branched C₁-C₆ alkyl group (such as Me, Et, Pr, i-Pr, n-Bu, i-Bu, t-Bu, pentyl and hexyl);
- a substituted or unsubstituted linear or branched C₁-C₆ alkyl-aryl group (such as -CH₂Ph,-CH₂(2,3 or 4)F-Ph, -CH₂(2,3 or 4)Cl-Ph, -CH₂(2,3 or 4)Br-Ph, -CH₂(2,3 or 4)I-Ph,-CH₂CH₂Ph, -CH₂CH₂CH₂Ph, -CH₂CH₂CH₂CH₂Ph, -CH₂CH₂CH₂CH₂CH₂Ph, and -CH₂CH₂CH₂CH₂CH₂CH₂Ph);

- a substituted or unsubstituted linear or branched C₁-C₆ halogenated alkyl group (such as -CH₂F, and -CH₂CF₃);
- a substituted or unsubstituted cyclic amine or amido group (such as pyrrolidin-3-yl, piperidin-3-yl, piperidin-4-yl, 2-keto-pyrrolidinyl, 3-keto-pyrrolidinyl, 2-keto-piperidinyl, 3-keto-piperidinyl, and 4-keto-piperidinyl);
- a substituted or unsubstituted cyclic C₃-C₈ alkyl group (such as cyclopropyl, cyclobutyl, cyclopentyl, cyclohexyl, cycloheptyl and cyclooctyl);
- a substituted or unsubstituted linear or branched C₂-C₆ alcohol group (such as -CH₂CH₂OH, -CH(CH₃)CH₂OH, -C(CH₃)₂OH, -CH₂CH₂CH₂OH,-CH₂CH₂CH₂CH₂OH, -CH(CH₃)CH₂CH₂OH, -CH(CH₃)CH(CH₃)OH, -CH(CH₂CH₃)CH₂OH, -C(CH₃)₂CH₂OH, -CH₂CH₂CH₂CH₂CH₂OH, and -CH₂CH₂CH₂CH₂CH₂CH₂OH);
- a substituted or unsubstituted linear or branched C₂-C₆ carboxylic acid group (such as-CH₂COOH, -CH₂CH₂COOH, -CH₂CH₂CH₂COOH, -CH₂CH₂CH₂CH₂COOH, and -CH₂CH₂CH₂CH₂CH₂COOH);
- a substituted or unsubstituted linear or branched carbonyl group (such as -(CO)Me, -(CO)Et, -(CO)Pr, -(CO)-i_Pr, -(CO)-n-Bu, -(CO)-i-Bu, -(CO)-t-Bu, -(CO)Ph, -( CO)CH₂Ph, -(CO)CH₂OH, -(CO)CH₂OCH₃, -(CO)CH₂NH₂, -(CO)CH₂NHMe, -(CO)CH₂NMe₂, -(CO)-cyclopropyl, -(CO)-1,3-epoxypropan-2-yl; -(CO)NH₂, -(CO)NHMe, -(CO)NMe₂, -(CO)NHEt, -(CO)NEt₂, -(CO)-pyrollidine-N-yl, -(CO)-morpholine-N-yl, -(CO)-piperazine-N-yl, -(CO)-N-methyl-piperazine-N-yl, -(CO)NHCH₂CH₂OH, -(CO)NHCH₂CH₂OMe, -(CO)NHCH₂CH₂NH₂, -(CO)NHCH₂CH₂NHMe, and -(CO)NHCH₂CH₂NMe₂;
- a substituted or unsubstituted linear or branched C₁-C₆ carboxylic acid ester group (such as -COOMe, -COOEt, -COOPr, -COO-i-Pr, -COO-n-Bu, -COO-i-Bu, -COO-t-Bu, -CH₂COOMe, -CH₂CH₂COOMe, -CH₂CH₂CH₂COOMe, and -CH₂CH₂CH₂CH₂COOMe);
- a substituted or unsubstituted linear or branched C₁-C₆ amide group (such as -CO-NH₂,-CO-NMeH, -CO-NMe₂, -CO-NEtH, -CO-NEtMe, -CO-NEt₂, -CO-NPrH, -CO-NPrMe, and-CO-NPrEt);
- a substituted or unsubstituted sulphonyl group (such as -SO₂Me, -SO₂Et, -SO₂Pr, -SO₂iPr,-SO₂Ph, -SO₂-(2,3 or 4)-F-Ph, -SO₂-cyclopropyl, -SO₂CH₂CH₂OCH₃), -SO₂NH₂, -SO₂NHMe, -SO₂NMe₂,
- SO₂NHEt, -SO₂NEt₂, -SO₂-pyrrolidine-N-yl, -SO₂-morpholine-N-yl, -SO₂NHCH₂OMe, and -SO₂NHCH₂CH₂OMe;
- a substituted or unsubstituted aromatic group (such as Ph-, 2-F-Ph-, 3-F-Ph-, 4-F-Ph-, 2-Cl-Ph-, 3-Cl-Ph-, 4-Cl-Ph-, 2-Br-Ph-, 3-Br-Ph-, 4-Br-Ph-, 2-I-Ph-, 3-I-Ph, 4-I-Ph-, 2,(3,4,5 or 6)-F₂-Ph-, 2,(3,4,5 or 6)-Cl₂-Ph-, 2,(3,4,5 or 6)-Br₂-Ph-, 2,(3,4,5 or 6)-I₂-Ph-, 2,(3,4,5 or 6)-Me₂-Ph-, 2,(3,4,5 or 6)-Et₂-Ph-, 2,(3,4,5 or 6)-Pr₂-Ph-, 2,(3,4,5 or 6)-Bu₂-Ph-, 2,(3,4,5 or 6)-(CN)₂-Ph-, 2,(3,4,5 or 6)-(NO₂)₂-Ph-, 2,(3,4,5 or 6)-(NH₂)₂-Ph-, 2,(3,4,5 or 6)-(MeO)₂-Ph-, 2,(3,4,5 or 6)-(CF₃)₂-Ph-, 3,(4 or 5)-F₂-Ph-, 3,(4 or 5)-Cl₂-Ph-, 3,(4 or 5)-Br₂-Ph-, 3,(4 or 5)-I₂-Ph-, 3,(4 or 5)-Me₂-Ph-, 3,(4 or 5)-Et₂-Ph-, 3,(4 or 5)-Pr₂-Ph-, 3,(4 or 5)-Bu₂-Ph-, 3,(4 or 5)-(CN)₂-Ph-, 3,(4 or 5)-(NO₂)₂-Ph-, 3,(4 or 5)-(NH₂)₂-Ph-, 3,(4 or 5)-(MeO)₂-Ph-, 3,(4 or 5)-(CF₃)₂-Ph-, 2-Me-Ph-, 3-Me-Ph-, 4-Me-Ph-, 2-Et-Ph-, 3-Et-Ph-, 4-Et-Ph-, 2-Pr-Ph-, 3-Pr-Ph-, 4-Pr-Ph-, 2-Bu-Ph-, 3-Bu-Ph-, 4-Bu-Ph-, 2-(CN)-Ph-, 3-(CN)-Ph-, 4-(CN)-Ph-, 2-(NO₂)-Ph-, 3-(NO₂)-Ph-, 4-(NO₂)-Ph-, 2-(NH₂)-Ph-, 3-(NH₂)-Ph-, 4-(NH₂)-Ph-, 2-MeO-Ph-, 3-MeO-Ph-, 4-MeO-Ph-, 2-(NH₂-CO)-Ph-, 3-(NH₂-CO)-Ph-, 4-(NH₂-CO)-Ph-, 2-CF₃-Ph-, 3-CF₃-Ph-, 4-CF₃-Ph-, 2-CF₃O-Ph-, 3-CF₃O-Ph-, and 4-CF₃O-Ph-); and
- a substituted or unsubstituted saturated or unsaturated, substituted or unsubstituted, heterocyclic group including an aromatic heterocyclic group and/or a non-aromatic heterocyclic group (such as pyrrole-2-yl, pyrrole-3-yl, pyrazole-3-yl, pyrazole-4-yl, pyrazole-5-yl, imidazole-2-yl, imidazole-4-yl, imidazole-5-yl, 1,2,3-triazole-4-yl, 1,2,3-triazole-5-yl, 1,2,4-triazole-3-yl, 1,2,4-triazole-5-yl, pyridin-2-yl, pyridin-3-yl, pyridin-4-yl, pyridazine-3-yl, pyridazine-4-yl, pyrimidin-2-yl, pyrimidin-4-yl, pyrimidin-5-yl, pyrimidin-6-yl, pyrazine-2-yl, pyrrolidine-2-yl, pyrrolidine-3-yl, piperidine-2-yl, piperidine-3-yl, piperidine-4-yl, 2-azapiperidine-3-yl, 2-azapiperidine-4-yl, 3-azapiperidine-2-yl, 3-azapiperidine-4-yl, 3-azapiperidine-5-yl, piperazine-2-yl, furan-2-yl, furan-3-yl, pyran-2-yl, pyran-3-yl, pyran-4-yl, 2-azapyran-3-yl, 2-azapyran-4-yl, 2-azapyran-5-yl, 2-azapyran-6-yl, 3-azapyran-2-yl, 3-azapyran-4-yl, 3-azapyran-5-yl, 3-azapyran-6-yl, 4-azapyran-2-yl, 4-azapyran-3-yl, 4-azapyran-5-yl, 4-azapyran-6-yl, oxetan-3-yl, tetrahydrofuran-2-yl, tetrahydrofuran-3-yl, 2-aza-tetrahydrofuran-3-yl, 2-aza-tetrahydrofuran-4-yl, 2-aza-tetrahydrofuran-5-yl, 3-aza-tetrahydrofuran-2-yl, 3-aza-tetrahydrofuran-4-yl, 3-aza-tetrahydrofuran-5-yl, tetrahydropyran-2-yl, tetrahydropyran-3-yl, tetrahydropyran-4-yl, 2-aza-tetrahydropyran-3-yl, 2-aza-tetrahydropyran-4-yl, 2-aza-tetrahydropyran-5-yl, 2-aza-tetrahydropyran-6-yl, 3-aza-tetrahydropyran-2-yl, 3-aza-tetrahydropyran-4-yl, 3-aza-tetrahydropyran-5-yl, 3-aza-tetrahydropyran-6-yl, morpholine-2-yl, morpholine-3-yl, thiophen-2-yl, thiophen-3-yl, isothiazole-3-yl, isothiazole-4-yl, isothiazole-5-yl, thiazole-2-yl, thiazole-4-yl, thiazole-5-yl, thiopyran-2-yl, thiopyran-3-yl, thiopyran-4-yl, 2-azathiopyran-3-yl, 2-azathiopyran-4-yl, 2-azathiopyran-5-yl, 2-azathiopyran-6-yl, 3-azathiopyran-2-yl, 3-azathiopyran-4-yl, 3-azathiopyran-5-yl, 3-azathiopyran-6-yl, 4-azathiopyran-2-yl, 4-azathiopyran-3-yl, 4-azathiopyran-5-yl, 4-azathiopyran-6-yl, thiolane-2-yl, thiolane-3-yl, thiane-2-yl, thiane-3-yl, thiane-4-yl, oxazol-2-yl, oxazol-4-yl, oxazol-5-yl, isoxazol-3-yl, isoxazol-4-yl, isoxazol-5-yl, furazan-3-yl, (1,3,4-oxadiazol)-2-yl, (1,3,4-oxadiazol)-5-yl, (1,2,4-oxadiazol)-3-yl, (1,2,4-oxadiazol)-5-yl; and tetrazole-5-yl).

It is preferred that R⁴⁶ is selected from H, a substituted or unsubstituted C₁-C₆ alkyl group or a substituted or unsubstituted linear or branched C₁-C₆ halogenated alkyl group.

Ring A of the group L preferably comprises any of the following structures: 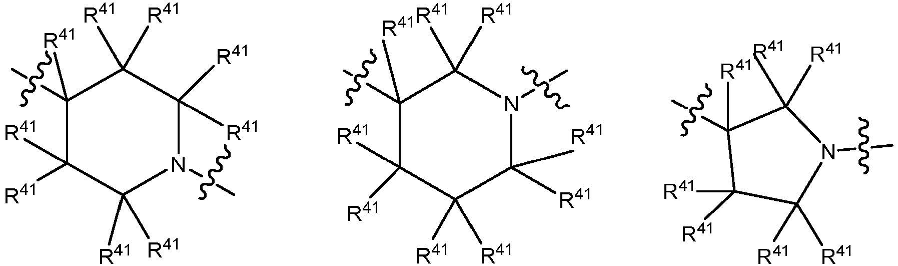 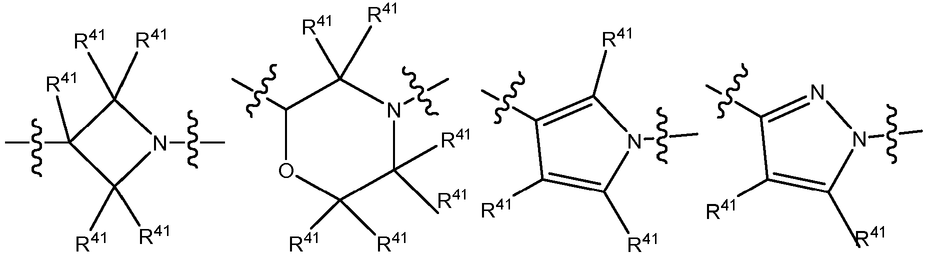 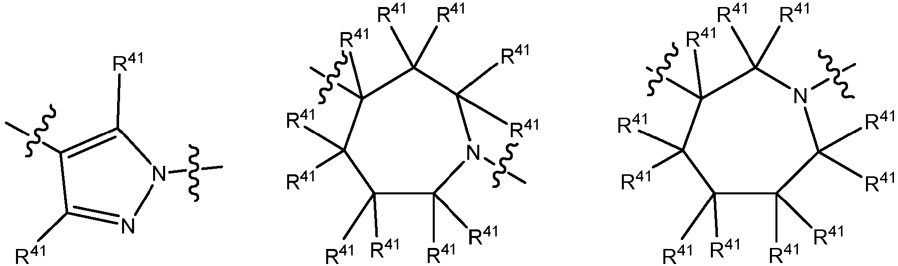 wherein R⁴¹ is as defined herein.

More preferably, ring A of the group L comprises

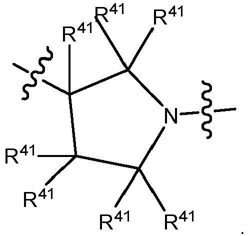

Still more preferably, ring A of the group L comprises any of the following structures:

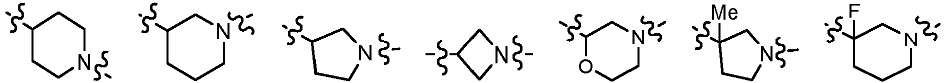

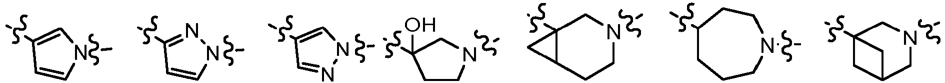

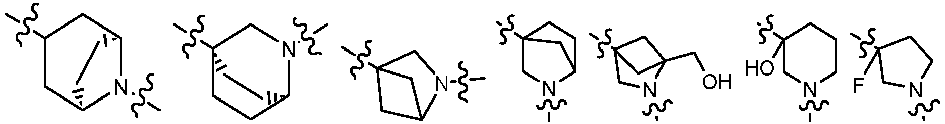

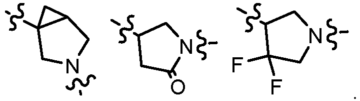

Particularly preferably, ring A of the group L comprises

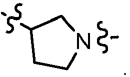

In one arrangement, Q² of the group L is present and comprises any of the following structures: 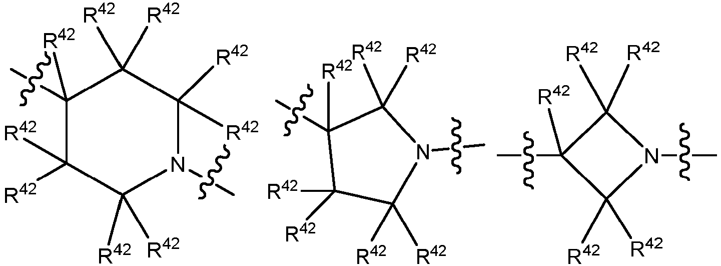 wherein R⁴² is as defined herein.

More preferably, Q² of the group L comprises any of the following structures:

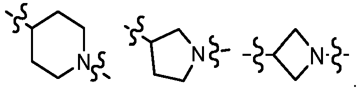

Alternatively, group Q² of the group L is absent and only one Q¹ group is present. In this arrangement the Q¹ group typically separates ring A from ring C by 3 or 4 atoms in the linear direction and may comprise any of the following structures: 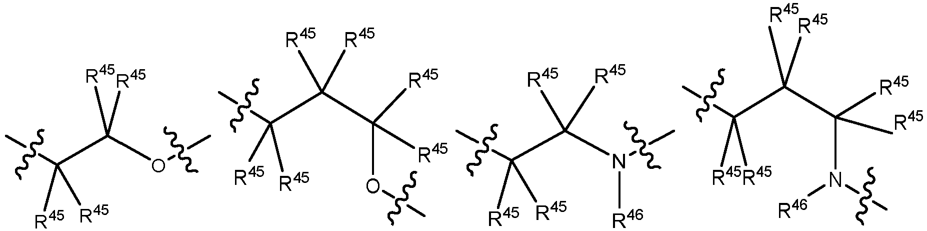 wherein R⁴⁵ and R⁴⁶ are as defined herein.

In this arrangement, the Q¹ group preferably comprises any of the following structures:

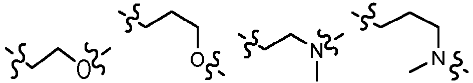

Preferably, ring C of the group L comprises any of the following structures: 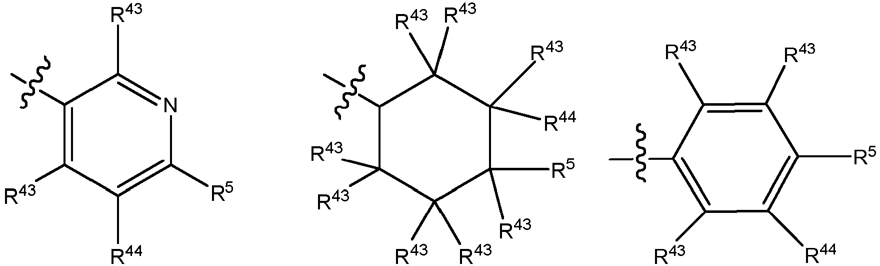 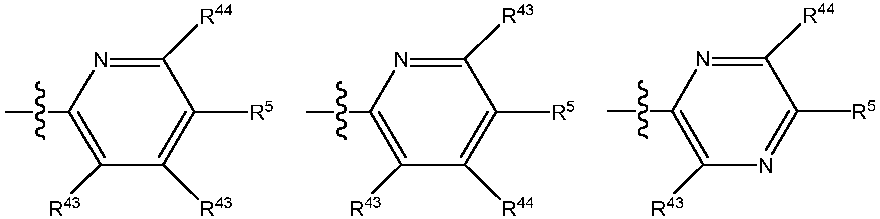 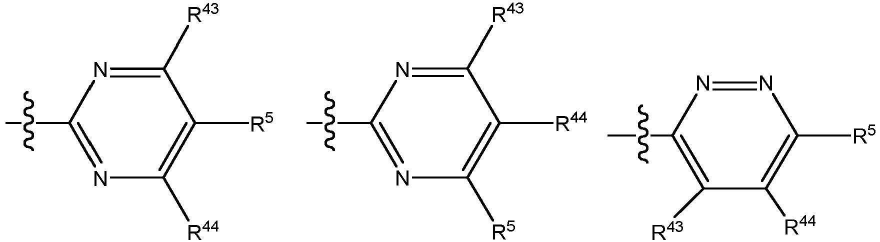 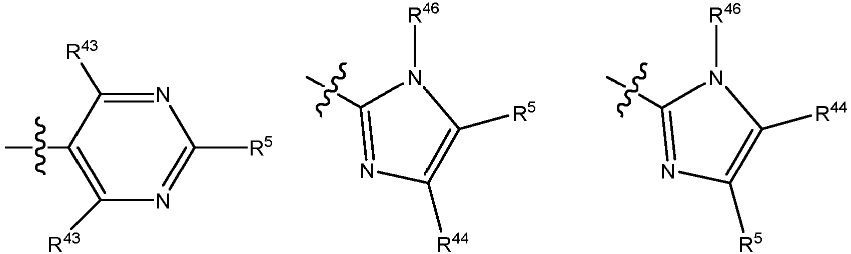 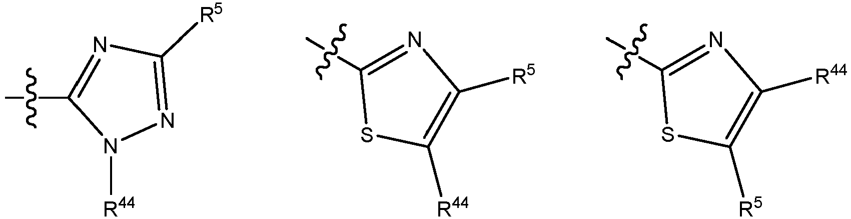 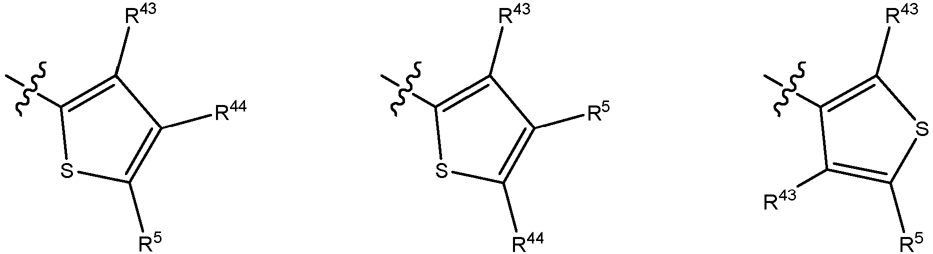 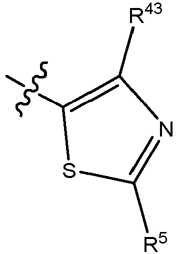 wherein R⁵, R⁴³, R⁴⁴ and R⁴⁶ are as defined herein.

More preferably, ring C of the group L comprises any of the following structures: 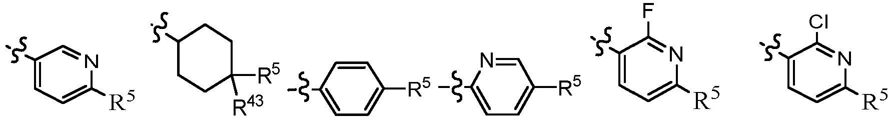 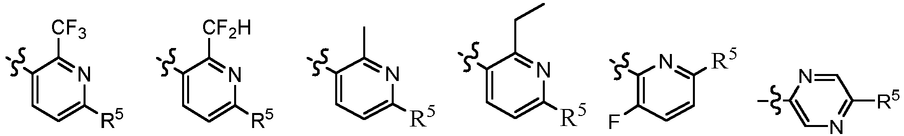 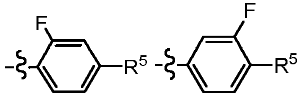 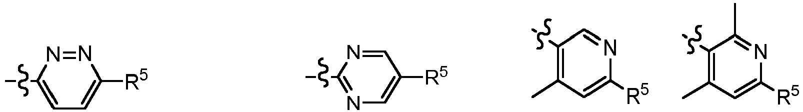 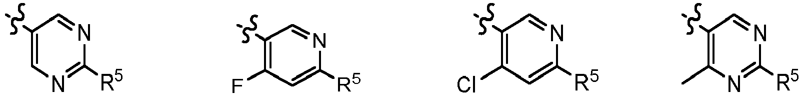 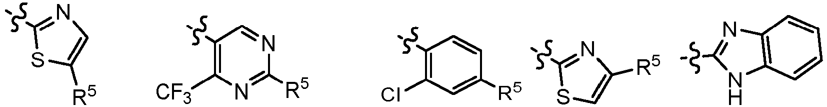 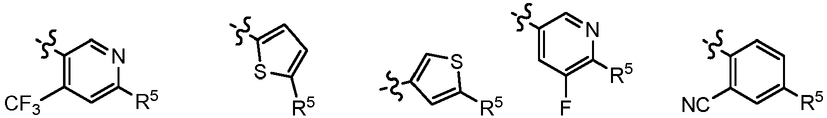 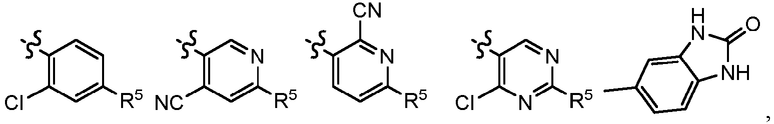 wherein R⁵ and R⁴³ are as defined herein.

In some arrangements, R⁵ is a substituted or unsubstituted organic group.

Preferably, the group R⁵ is not MeO, and is more preferably selected from H, -F, -Cl, -Br, -I,-CN, -CONR⁵¹R⁵¹, -NR⁵¹COR⁵², -SO₂NR⁵¹R⁵¹, -NR⁵¹SO₂R⁵³, -O-CR⁵²R⁵²R⁵²,-CR⁵²R⁵²NR⁵¹R⁵¹ and any of the following structures: 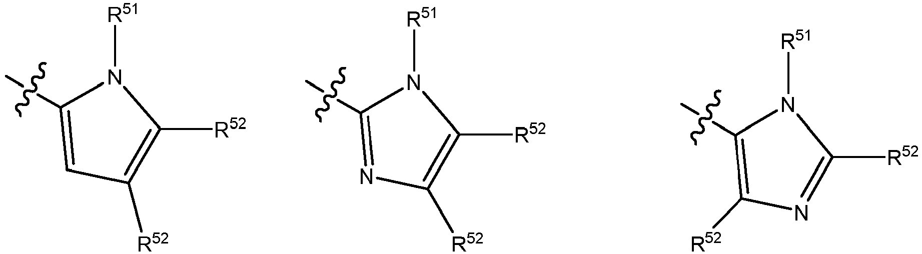 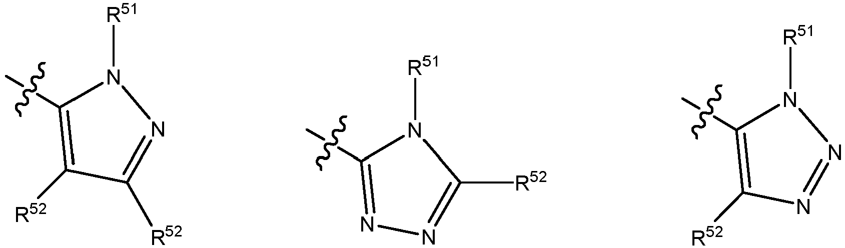 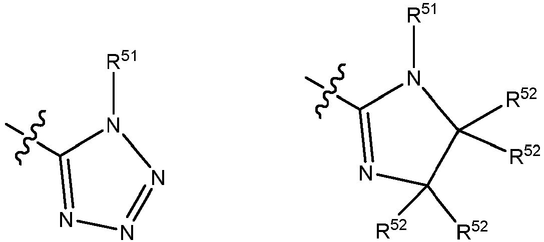 wherein, each ofR⁵¹, R⁵² and R⁵³ may be the same or different and are independently selected from H and a substituted or unsubstituted organic group. More preferably, the group R⁵ is - CONR⁵¹R⁵¹.

Still more preferably, the group R⁵ is selected from -F, -Cl, -CN, -CONH₂, -CONHMe,-CONHEt, -CONMe₂, -CONHCOMe, -CONHCH₂-CH₂OMe, -CONH-CH₂-CH₂F, -CONH-CH₂-CF₃, -CONH-CH₂-CHF₂, -OCHF₂, -NHCOMe, -NHSO₂Me, -SO₂NHMe,-CONHSO₂Me,

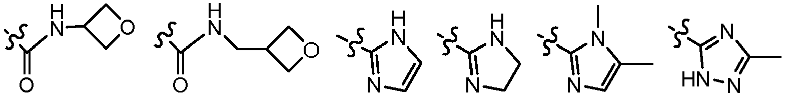

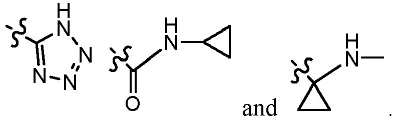

A particularly preferred group R⁵ is -CONHMe, especially where ring C of the group L comprises 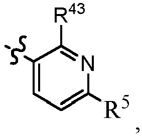 preferably

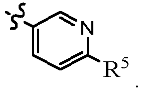

In some of the disclosed compounds, the group L of the compound comprises a group having any the following structures:

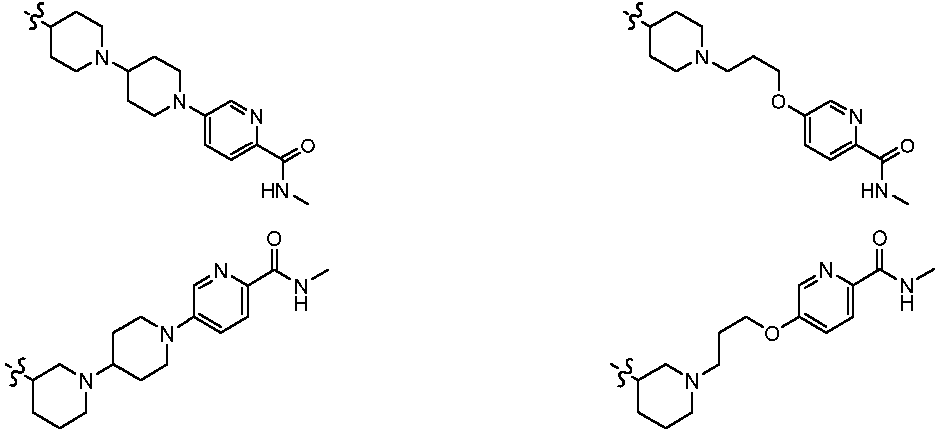

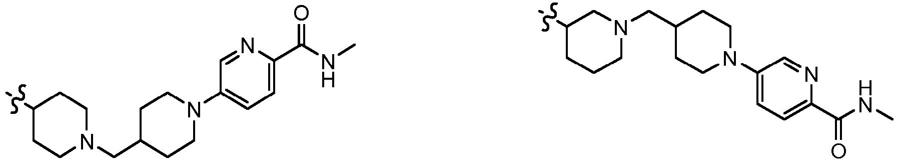

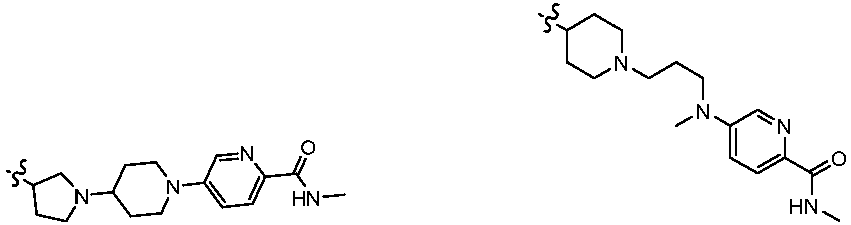

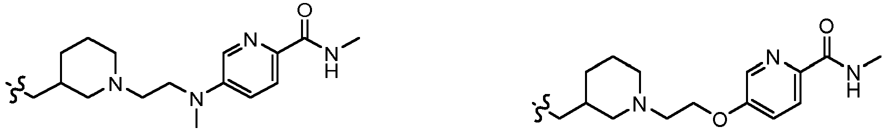

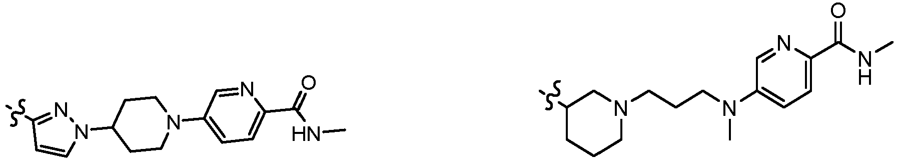

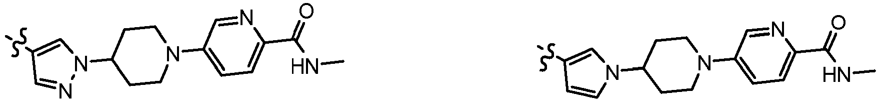

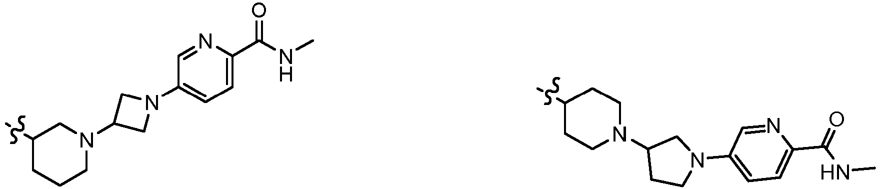

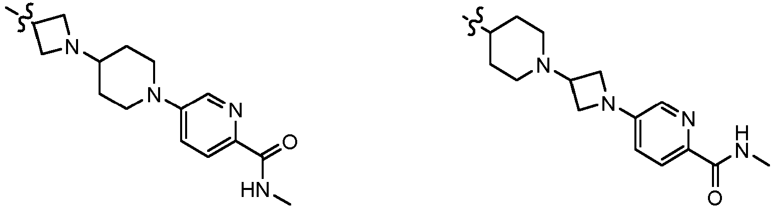

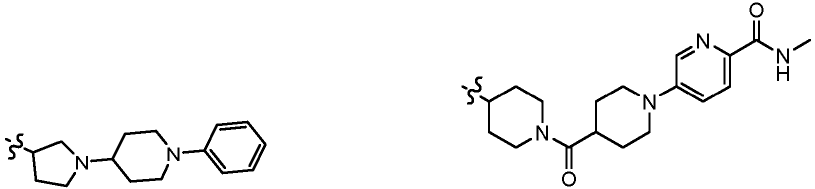

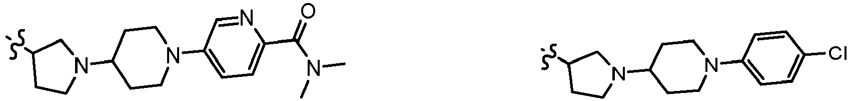

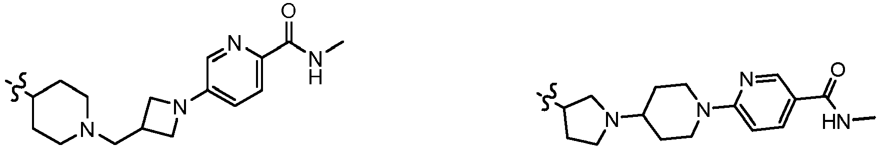

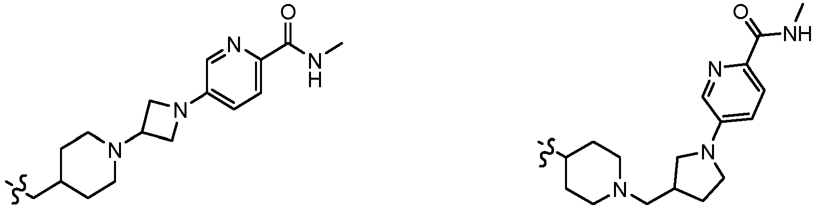

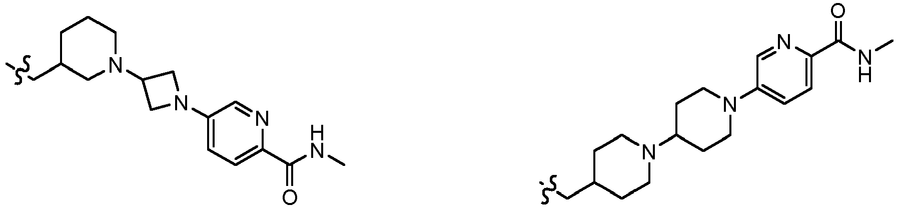

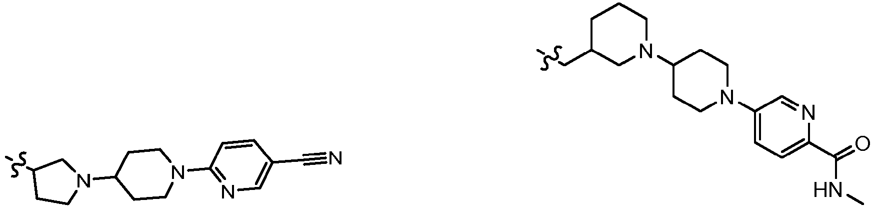

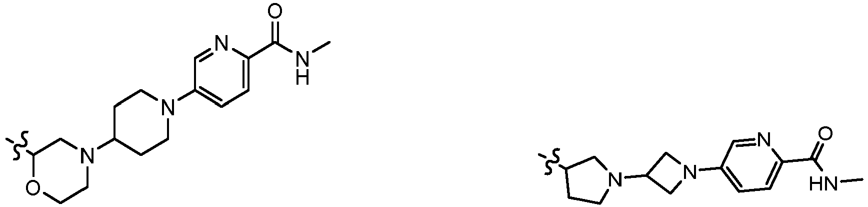

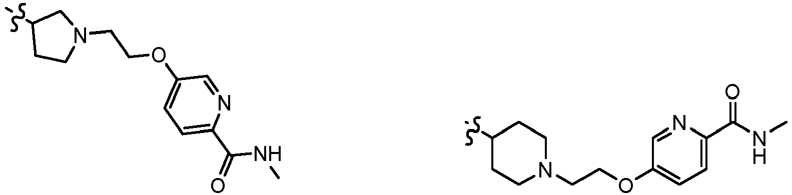

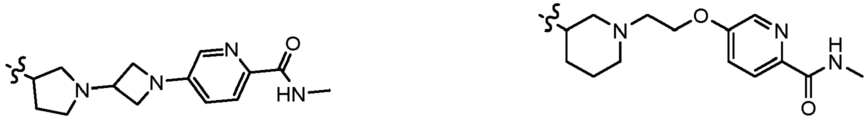

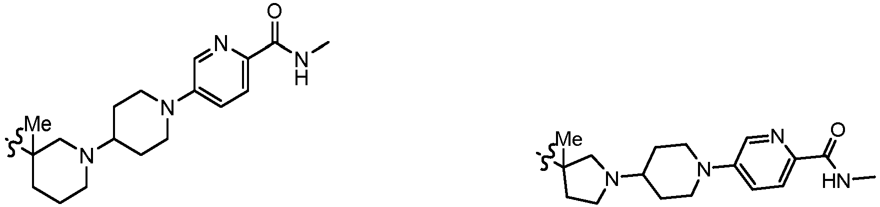

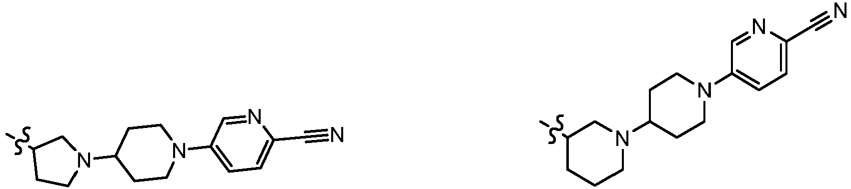

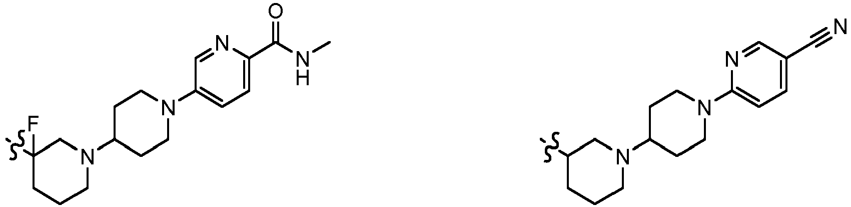

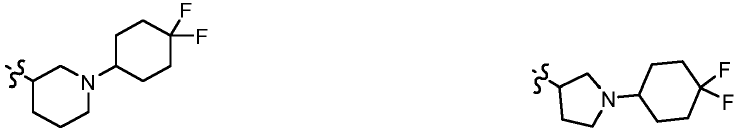

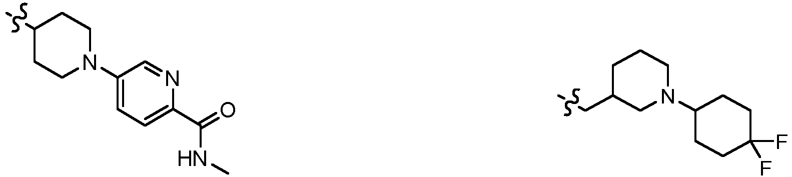

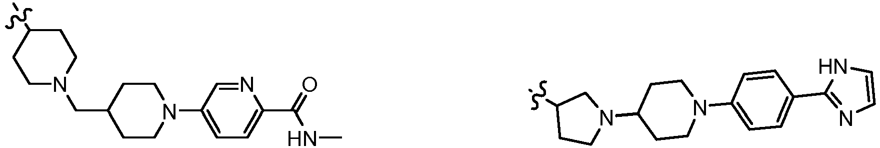

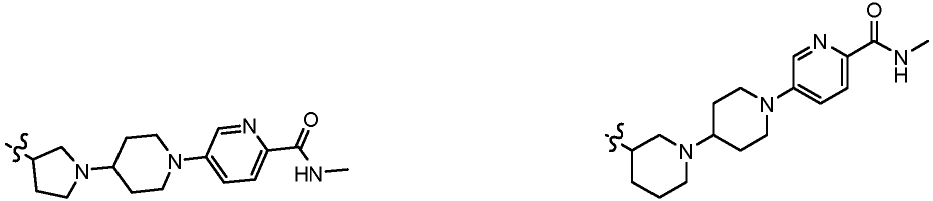

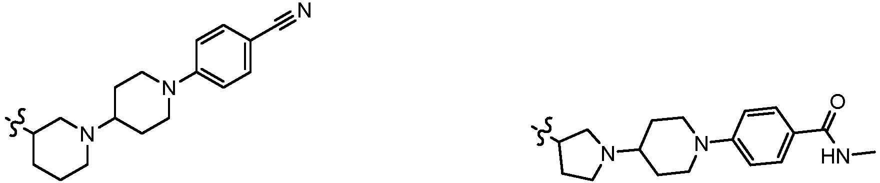

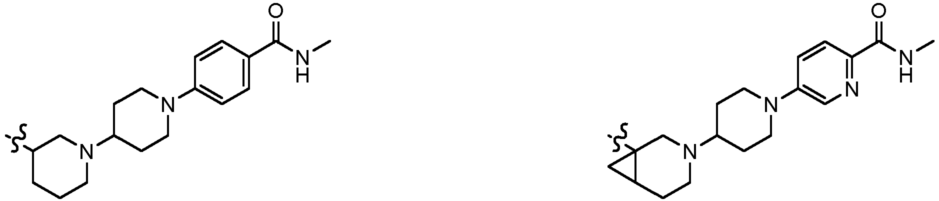

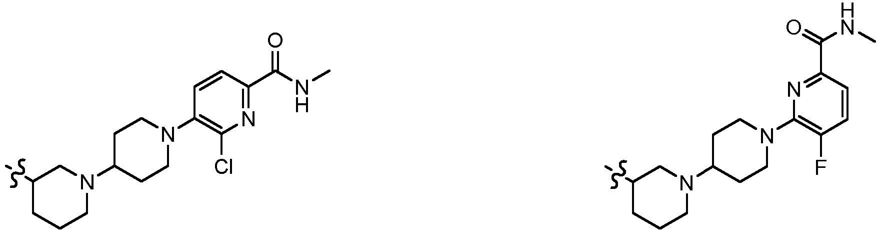

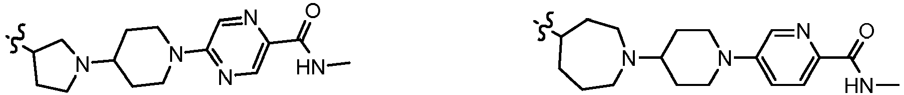

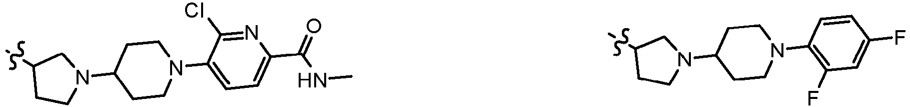

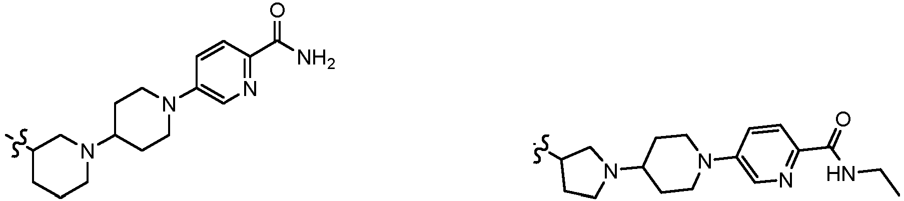

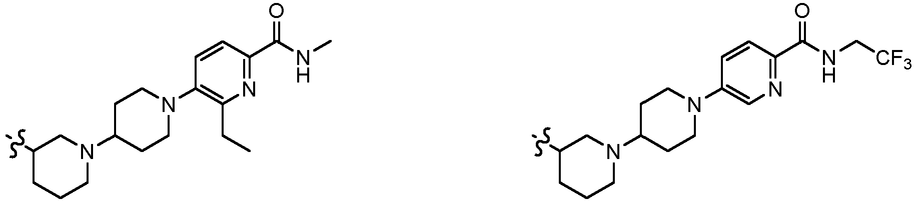

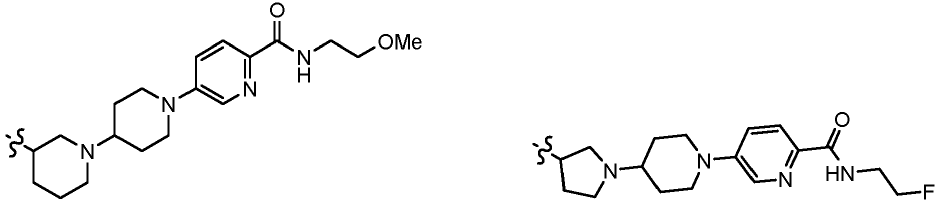

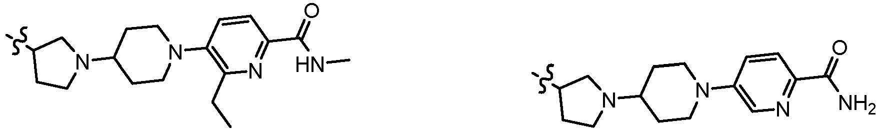

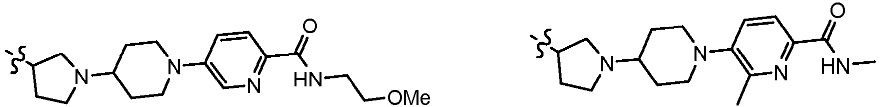

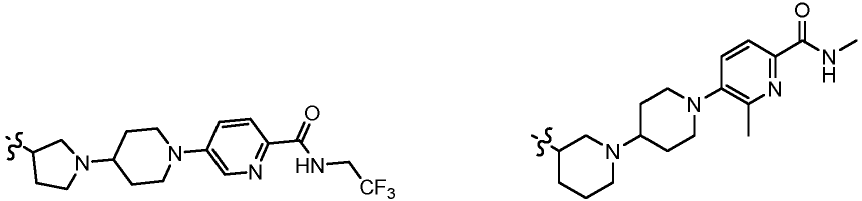

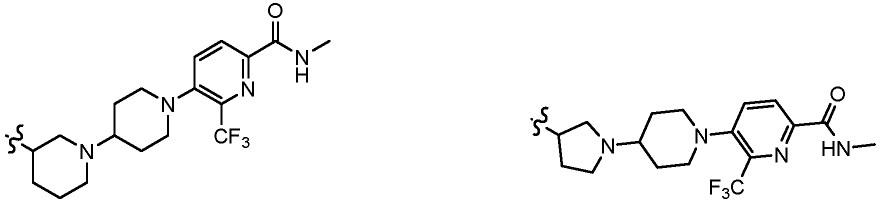

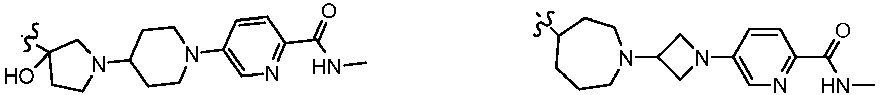

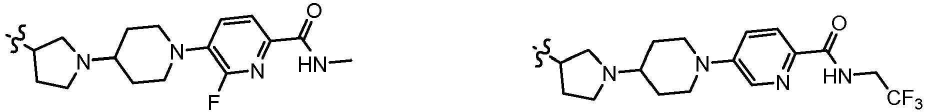

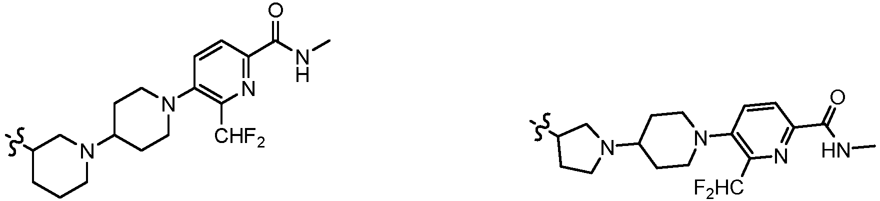

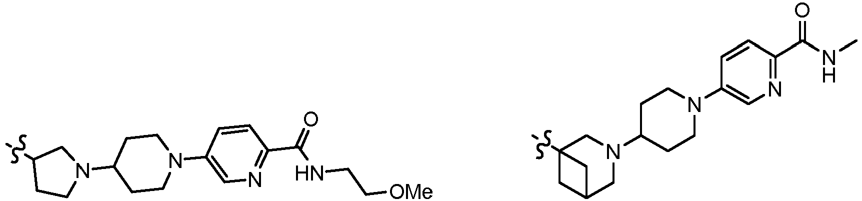

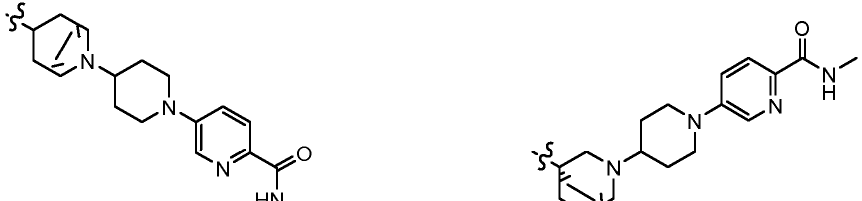

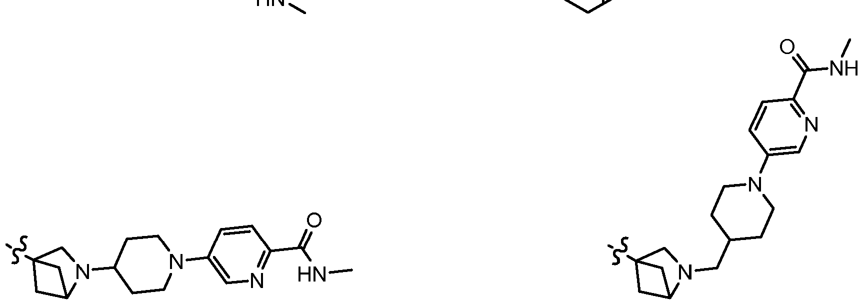

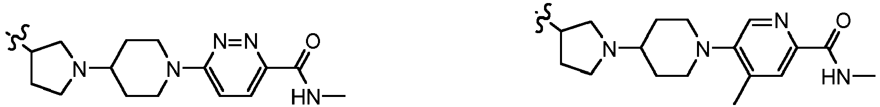

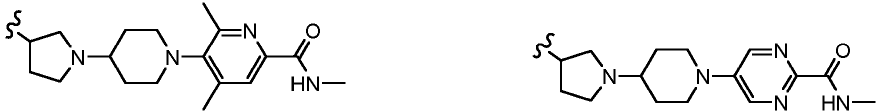

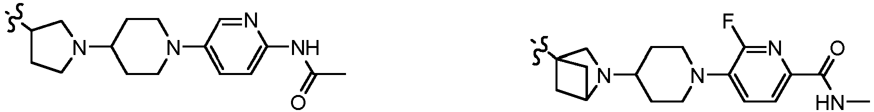

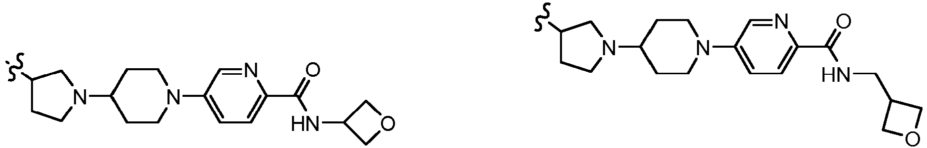

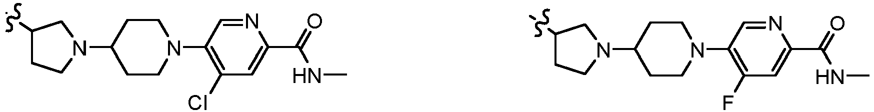

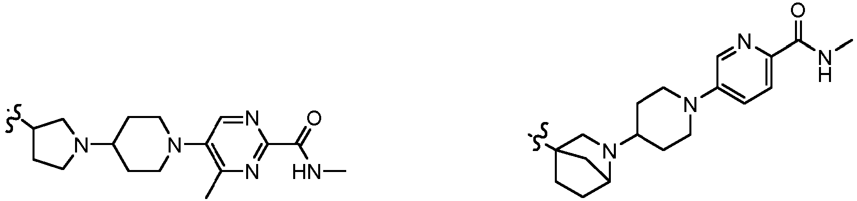

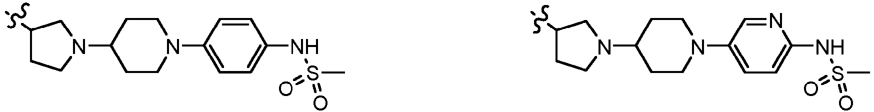

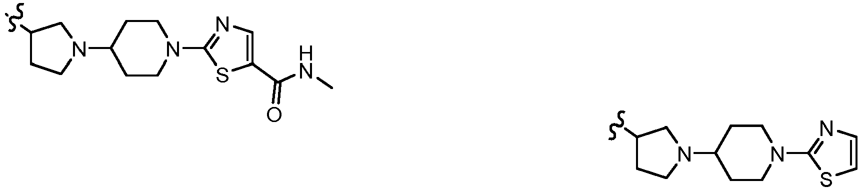

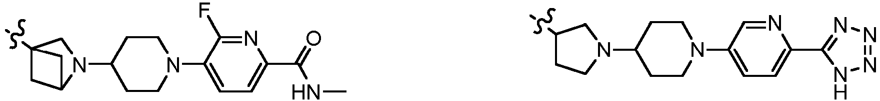

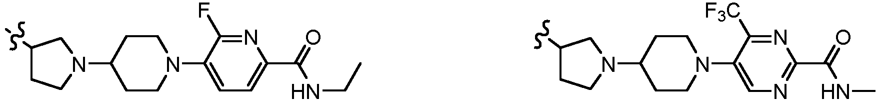

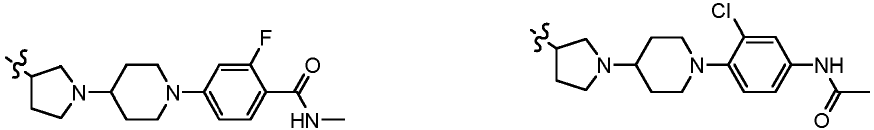

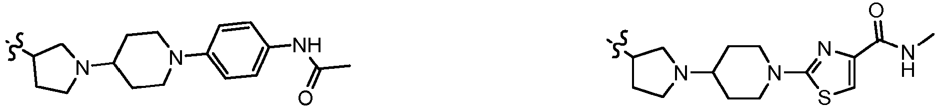

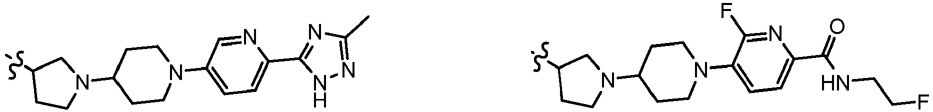

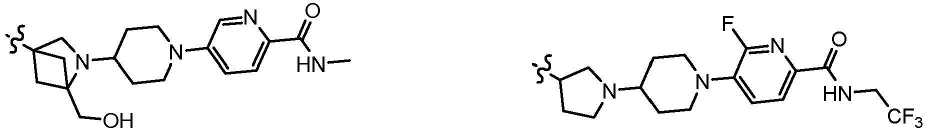

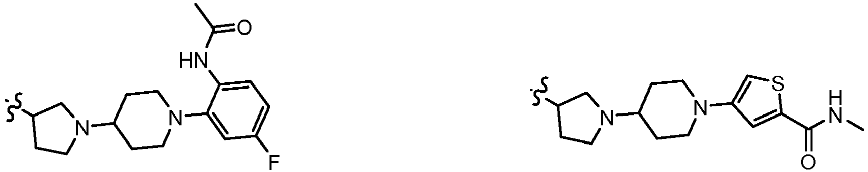

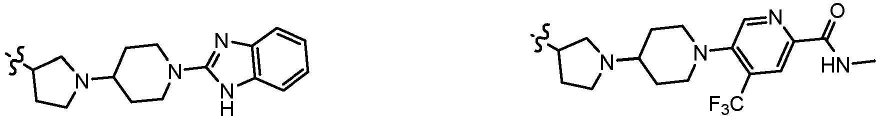

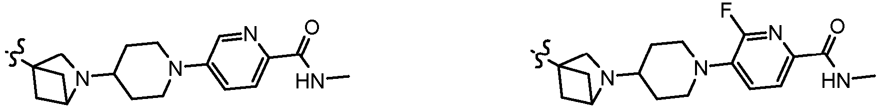

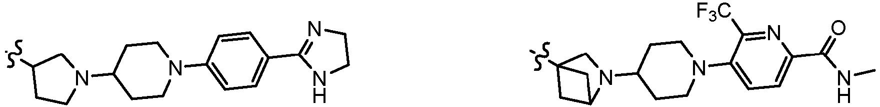

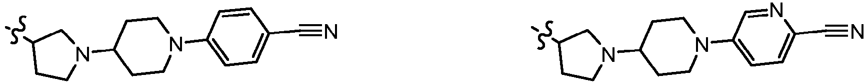

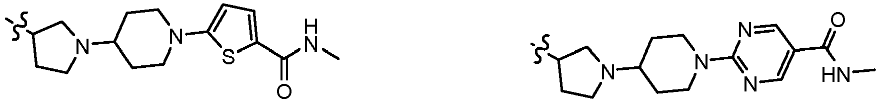

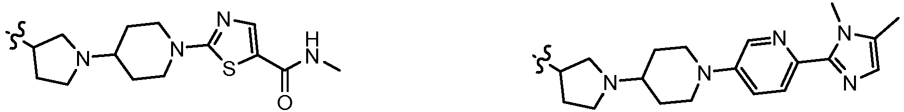

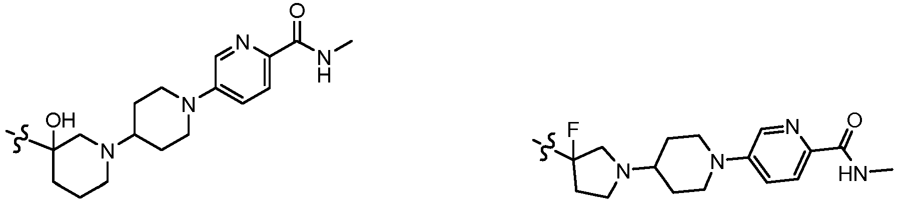

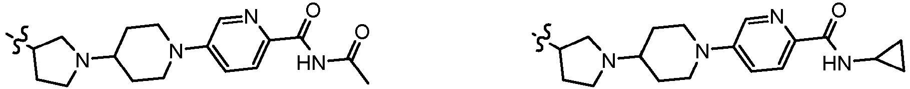

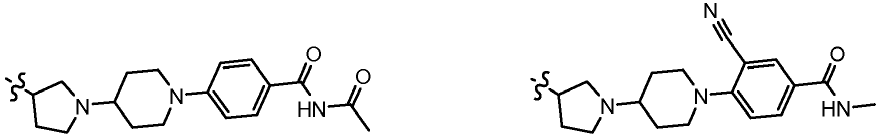

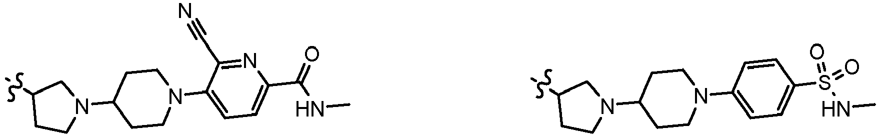

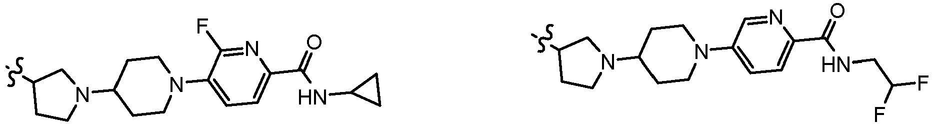

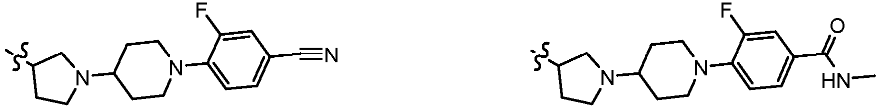

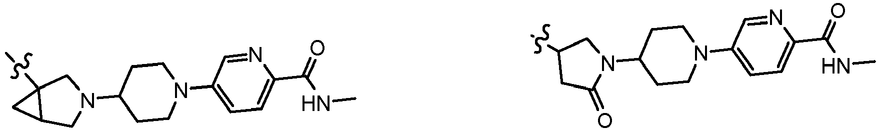

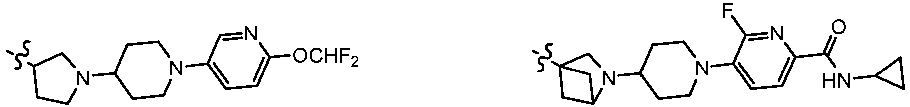

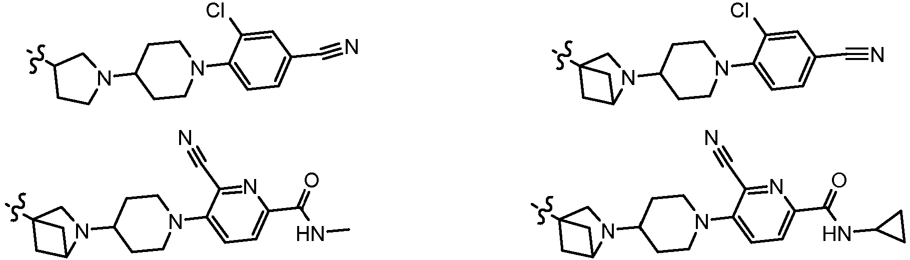

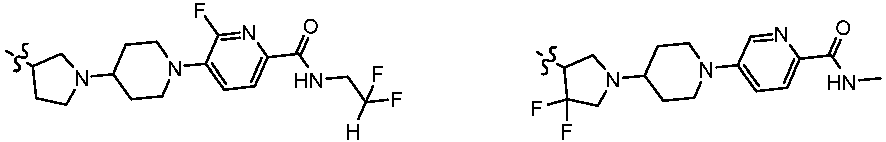

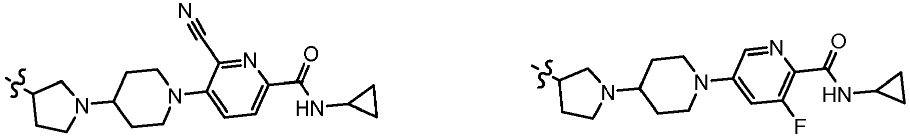

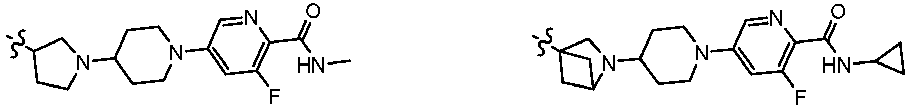

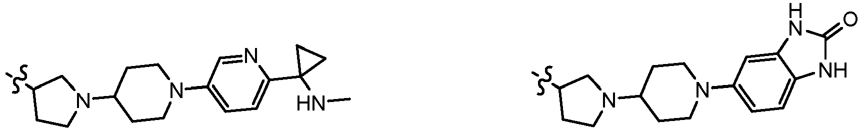

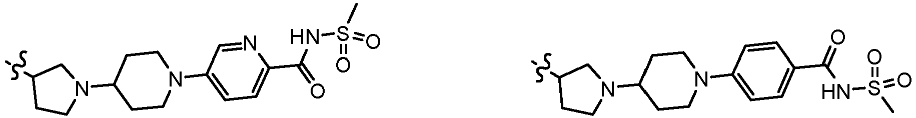

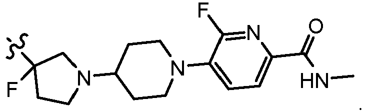

The disclosure provides a compound which comprises any of the following structures: 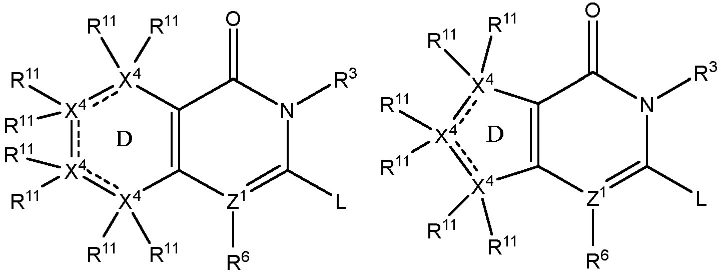 wherein, the dotted lines indicate that the ring D may comprise single bonds, or a combination of both single and double bonds, and may be aliphatic or aromatic; each X⁴ may be the same or different and is independently selected from C, N, O and S; each R¹¹ may be the same or different and is independently selected from H and a substituted or unsubstituted organic group; and wherein Z¹, R³, R⁶, and L are as defined herein. In some of the disclosed compounds, one X⁴ of the ring D is N. It is preferred that at least one X⁴ is C.

Preferably, the compound comprises any of the following structures: 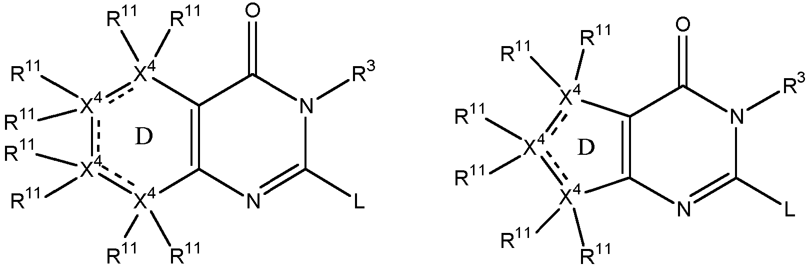 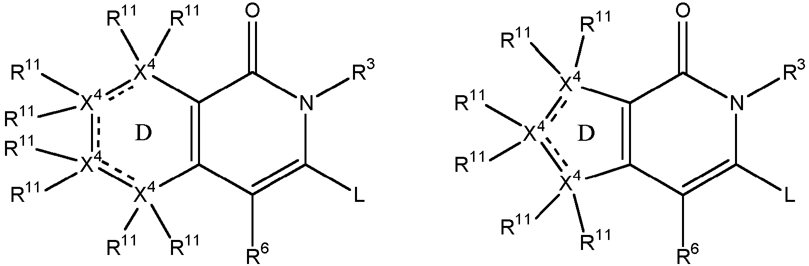 wherein the ring D, X⁴, R¹¹, R³, R⁶, and L are as defined herein.

More preferably, the compound comprises any of the following structures: 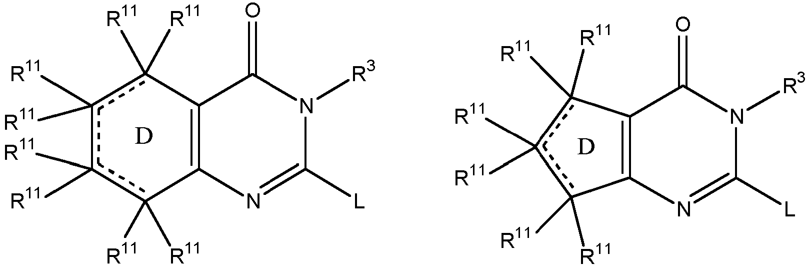 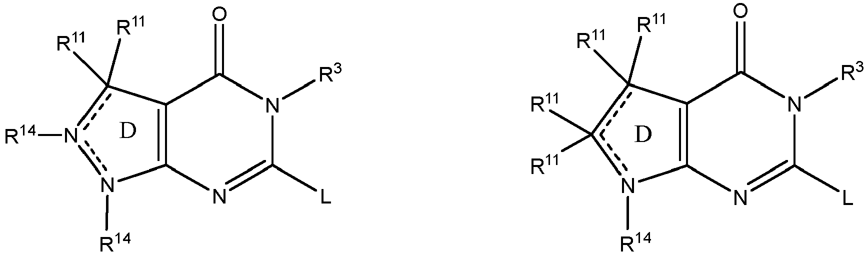 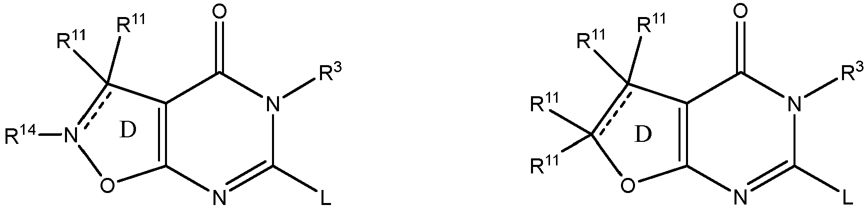 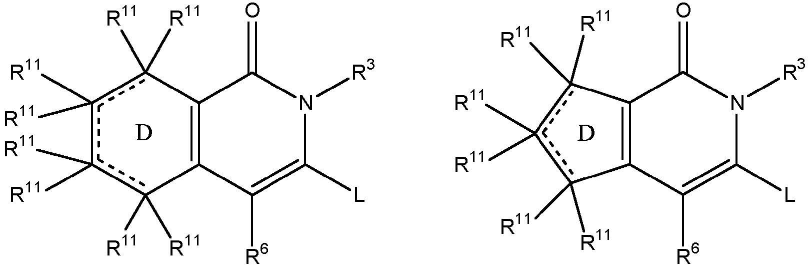 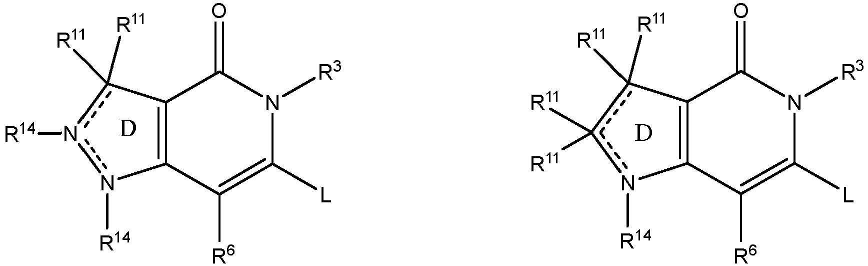 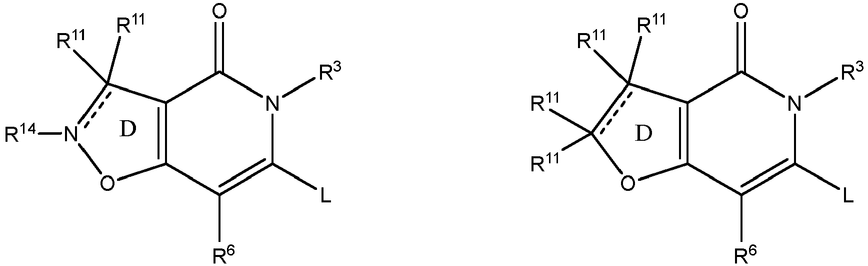 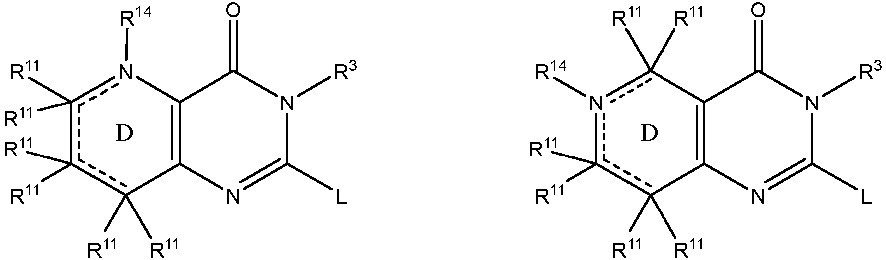 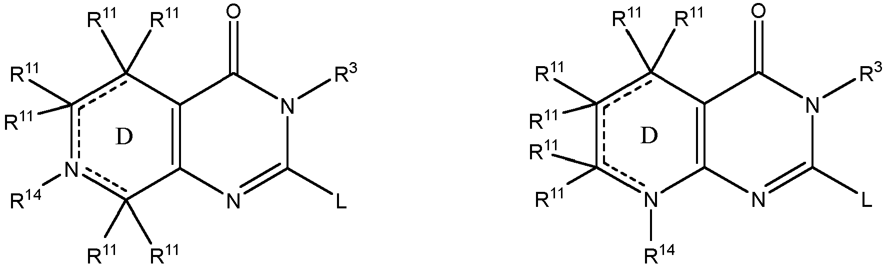 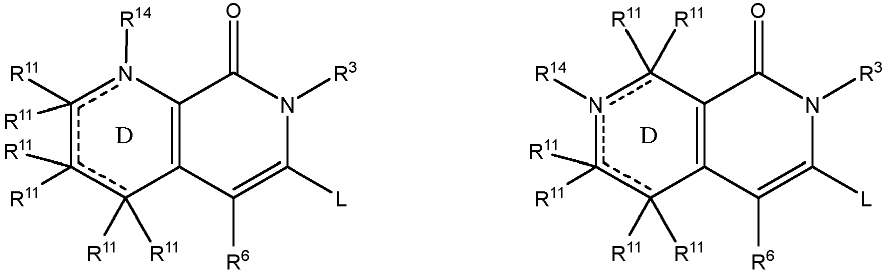 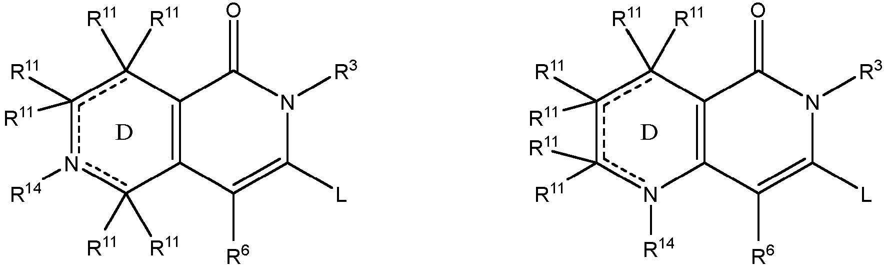 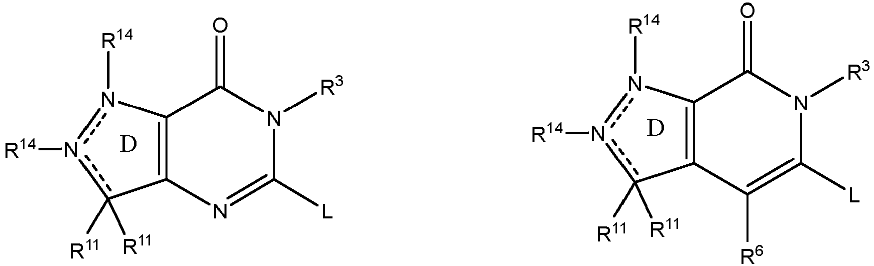 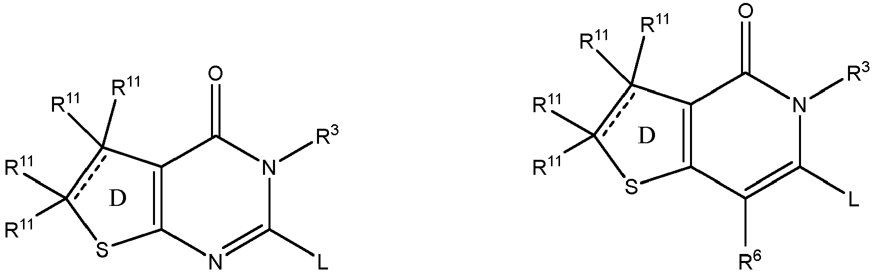 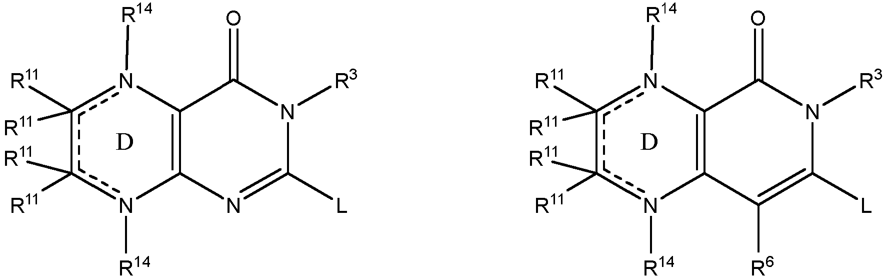 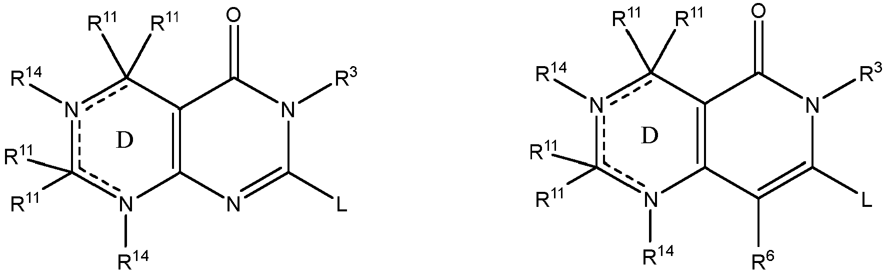 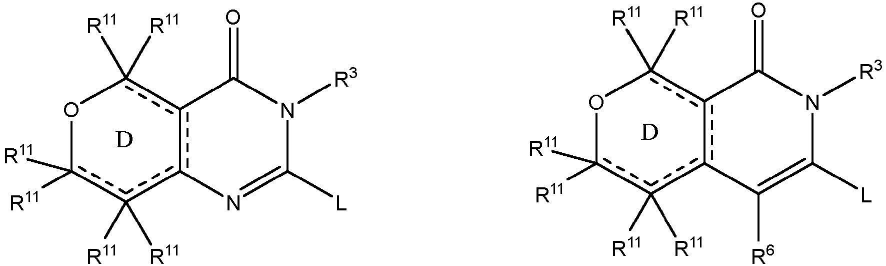 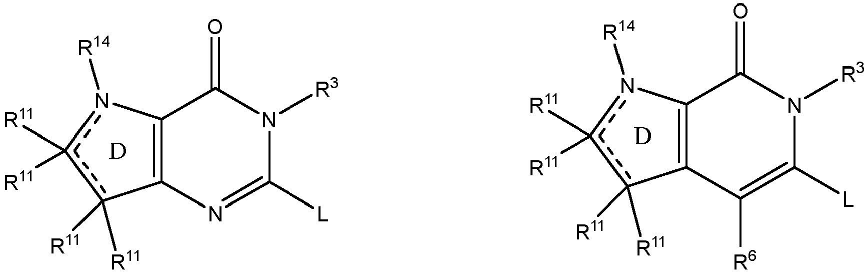 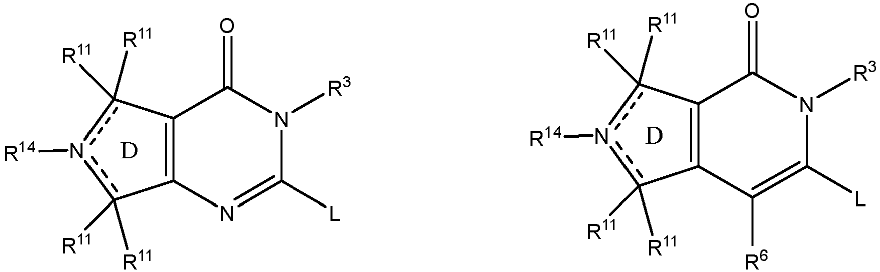 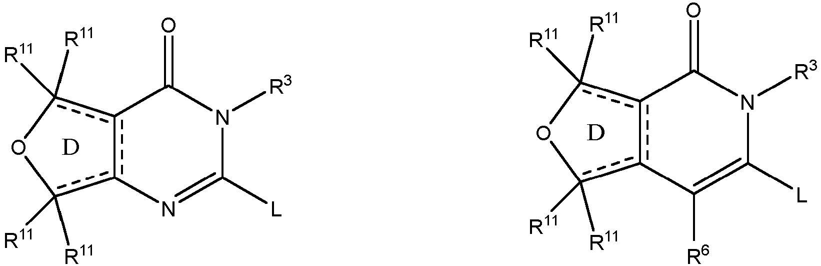 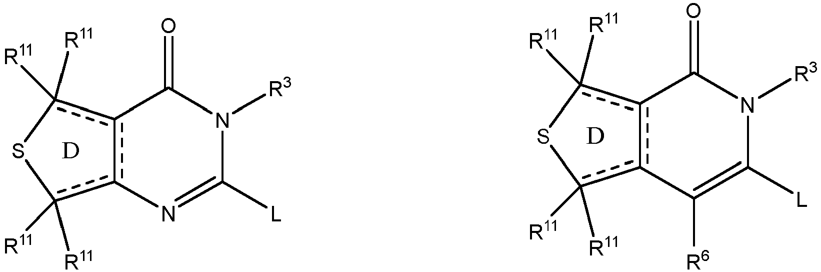 wherein R¹⁴ is selected from H, C₁ to C₃ alkyl or C₁ to C₃ fluoroalkyl; and wherein R¹¹, ring D, R³, R⁶, and L are as defined herein.

Still more preferably, the compound comprises any of the following structures: 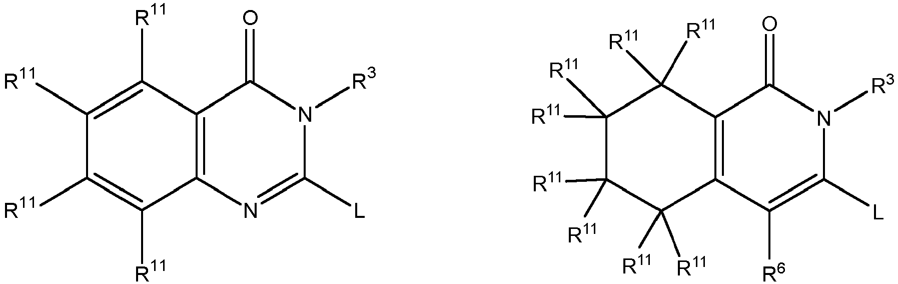 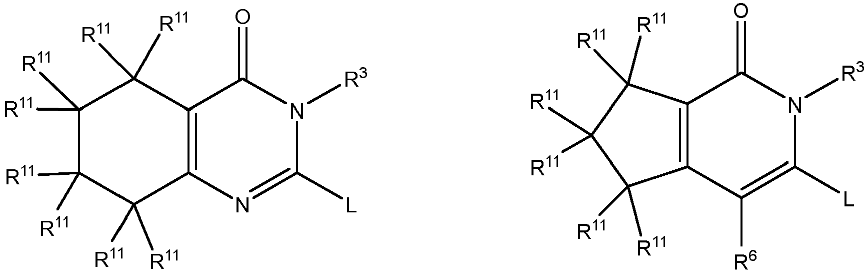 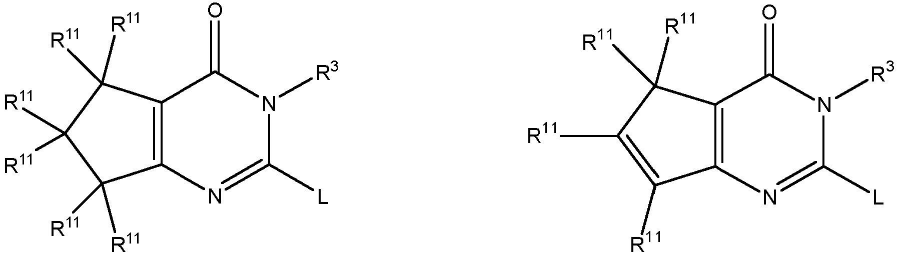 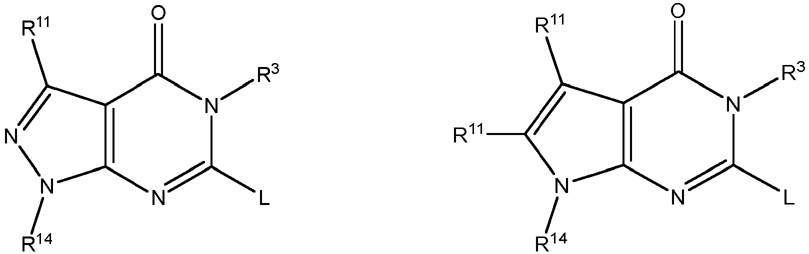 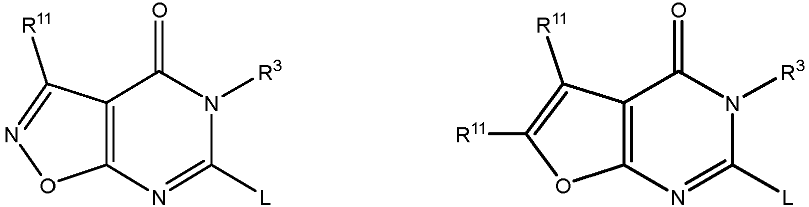 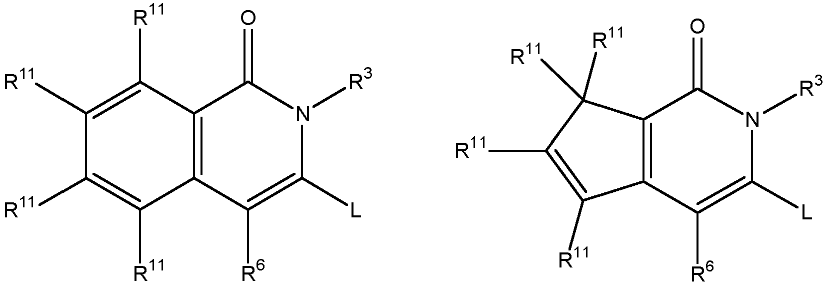 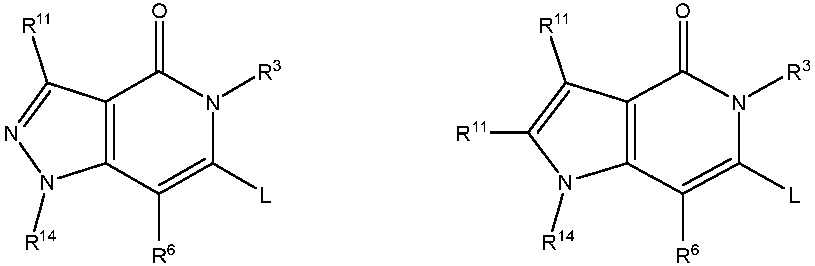 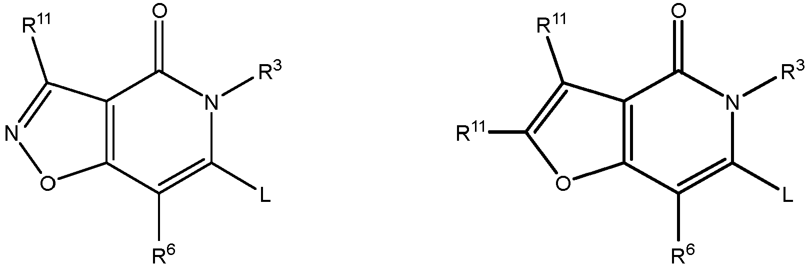 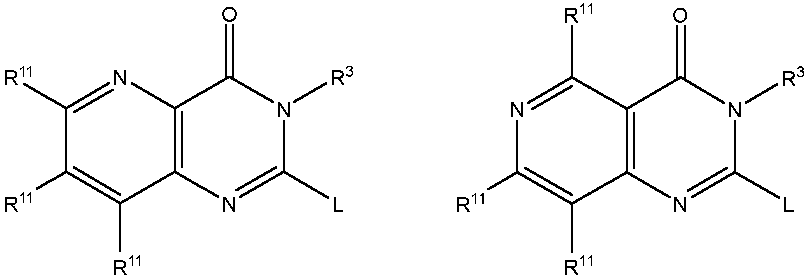 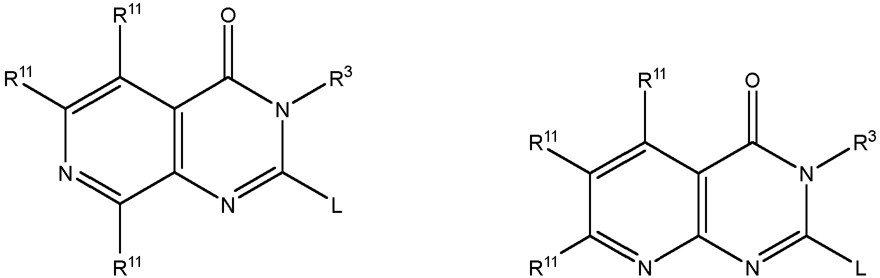 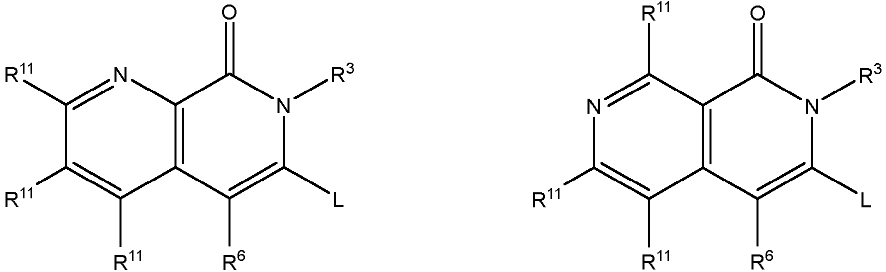 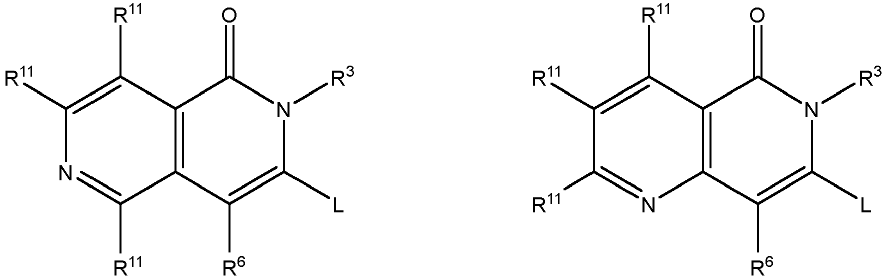 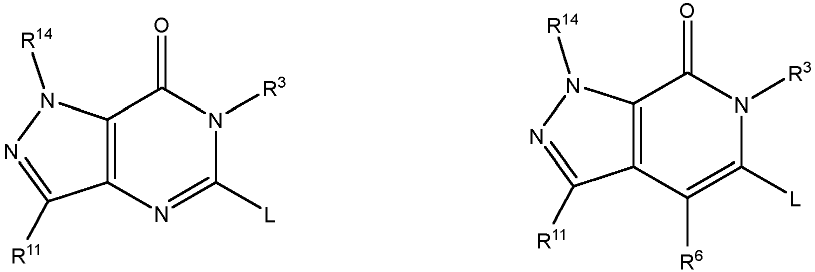 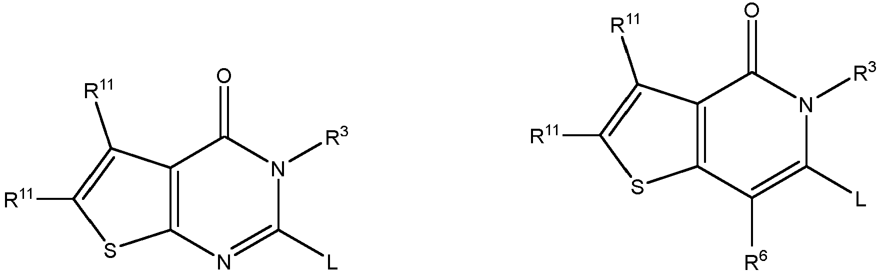 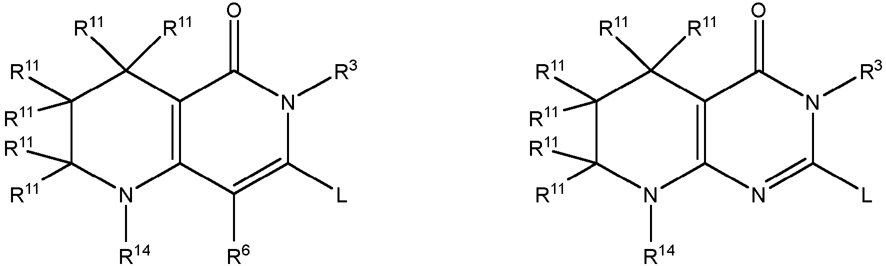 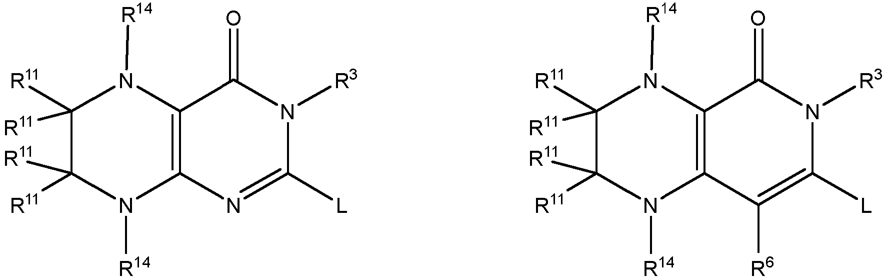 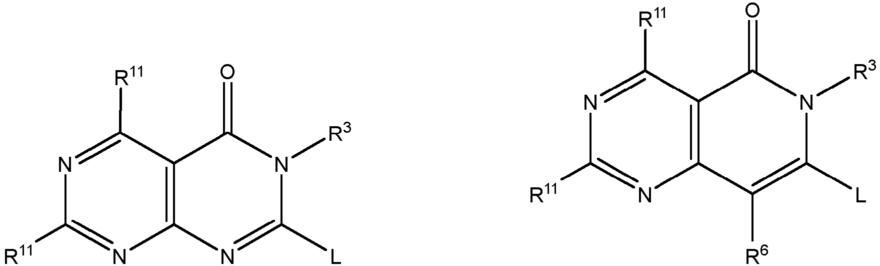 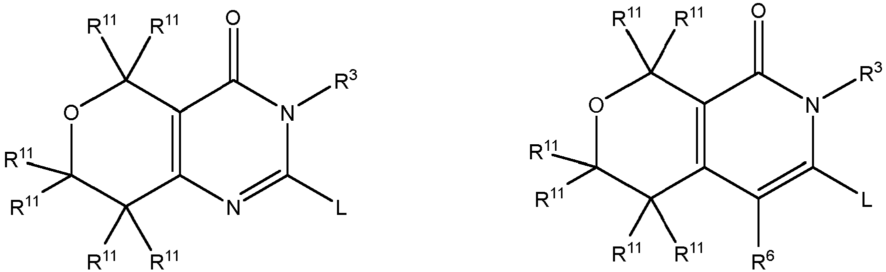 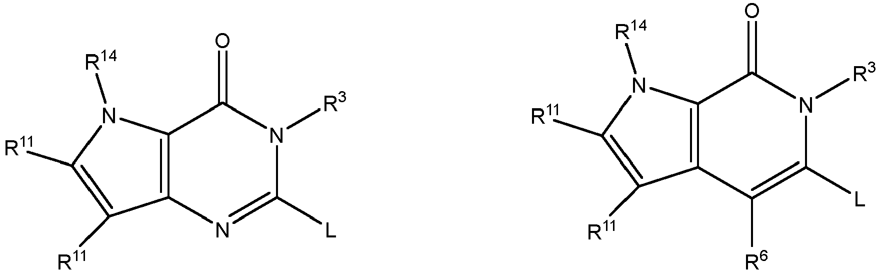 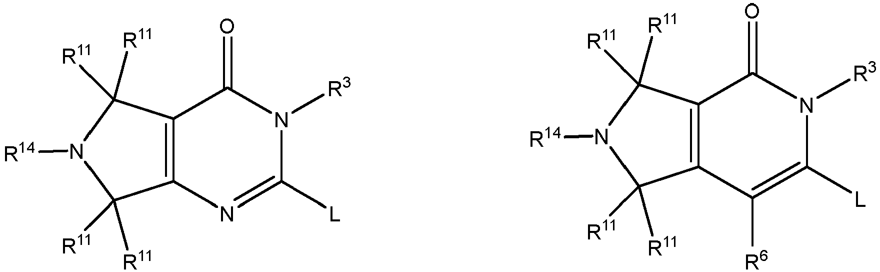 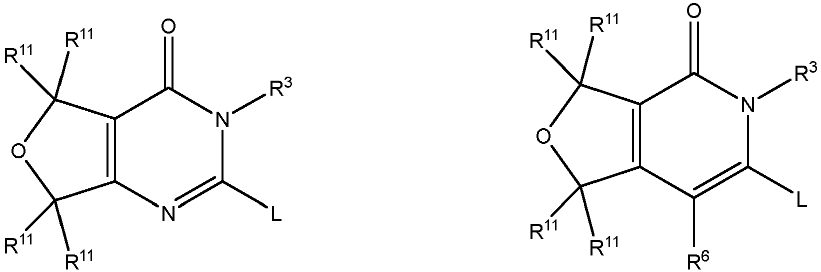 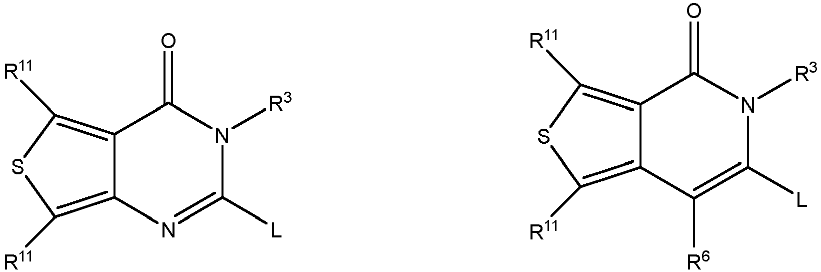 wherein R³, R¹¹, R¹⁴, R⁶, and L are as defined herein.

In the compounds of the invention, each R¹¹ is independently be selected from H, a halogen, a nitrile group, a linear or branched C₁-C₃ alkyl group, a linear or branched C₁-C₃ halogenated alkyl group (preferably fluoroalkyl), an -OH group, a linear or branched C₁-C₃ alcohol group, a halogenated (preferably fluoro-) linear or branched C₁-C₃ alcohol group, -NH₂, a linear or branched primary secondary or tertiary C₁-C₃ amine group, a halogenated (preferably fluoro-) linear or branched primary, secondary or tertiary C₁-C₃ amine group, a linear or branched C₁-C₃ alkoxy group, a linear or branched C₁-C₃ halogenated alkoxy group (preferably fluoroalkoxy), and/or a pair of R¹¹ groups attached to the same atom forming =O; and independently, and/or when a pair of R¹¹ groups attached to different atoms together forms a ring with ring D atoms, the pair of R¹¹ groups comprises -CH₂CH₂CH₂-.

It is preferred that each R¹¹ is independently selected from H, Cl, F, CHF₂, CF₃, CH₃, OH, CH₃O, and NH₂, and/or a pair of R¹¹ groups attached to the same atom forming =O; and independently, when a pair of R¹¹ groups attached to different atoms together forms a ring with ring D atoms, the pair of R¹¹ groups comprises -CH₂CH₂CH₂-.

A compound is provided which comprises any of the following structures: 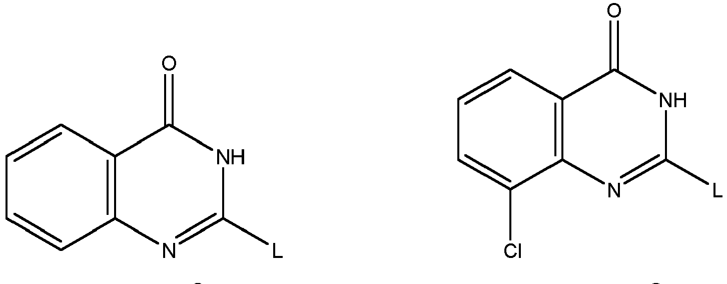 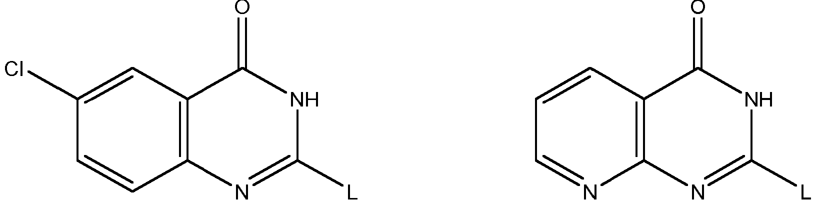 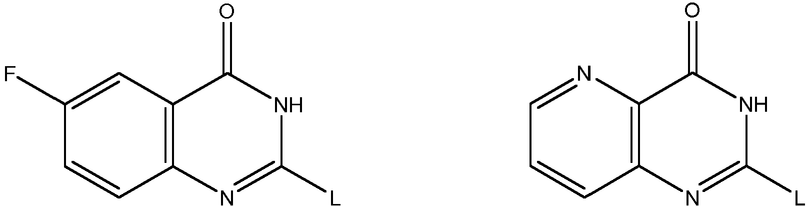 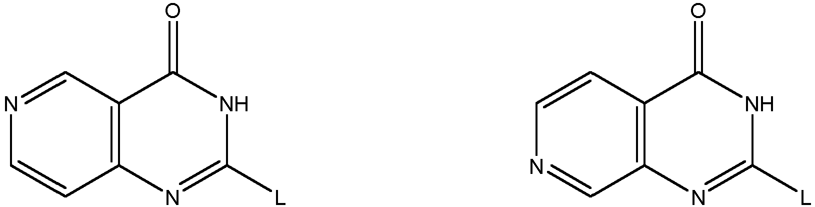 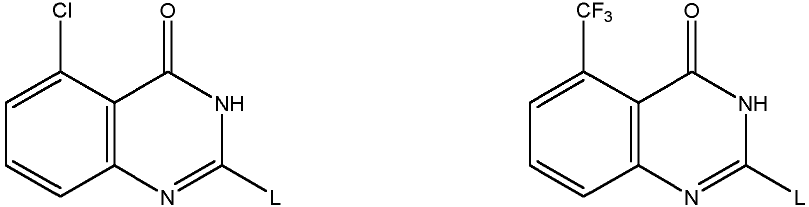 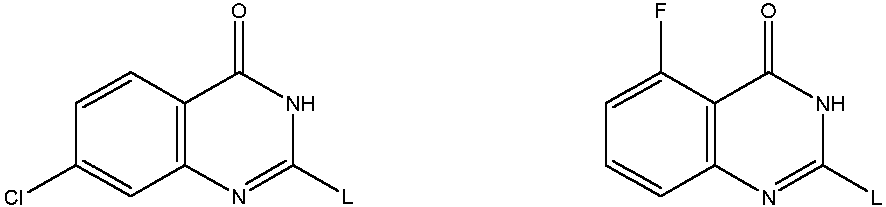 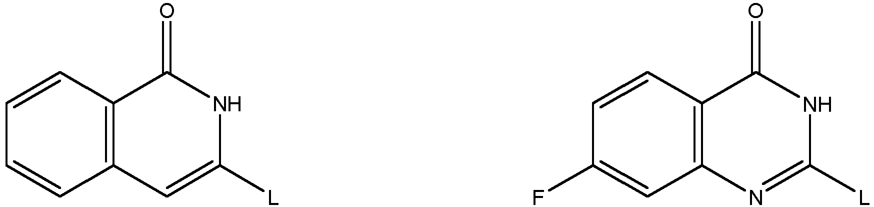 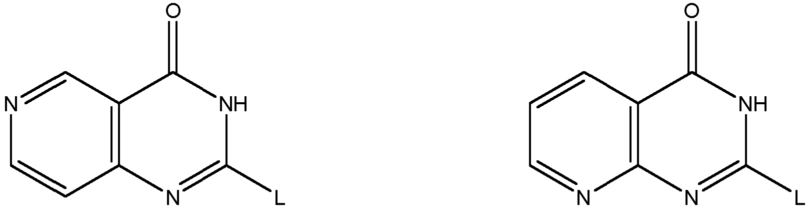 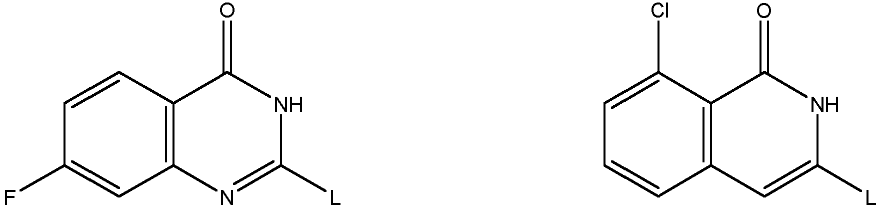 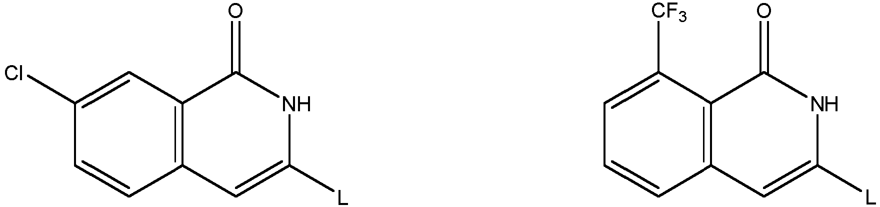 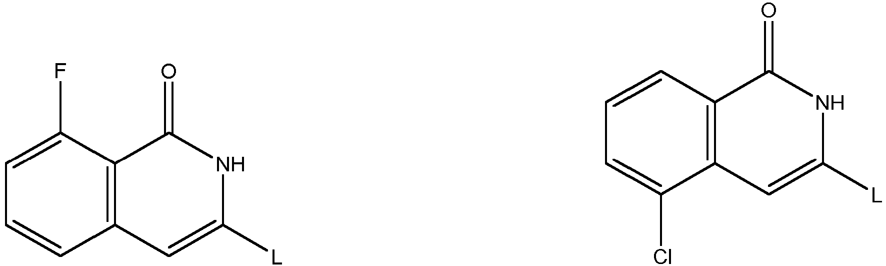 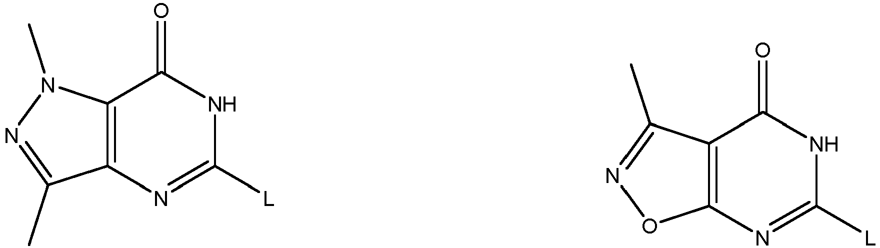 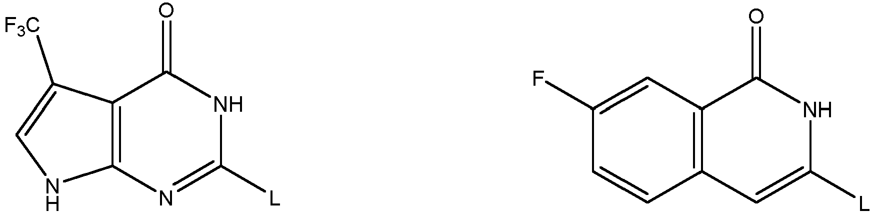 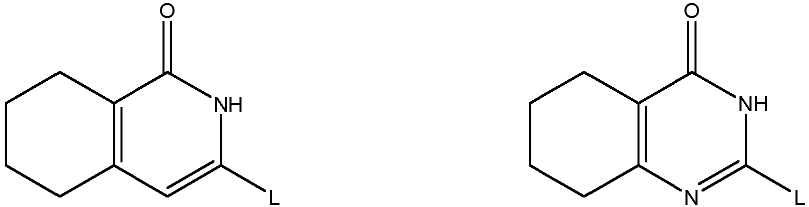 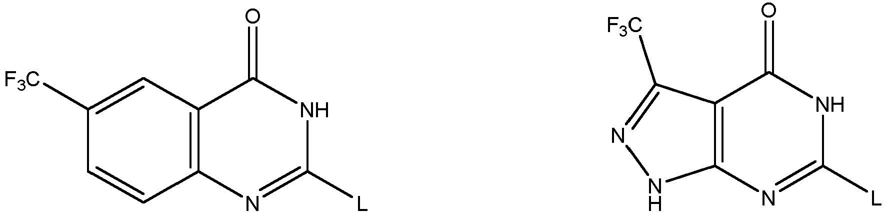 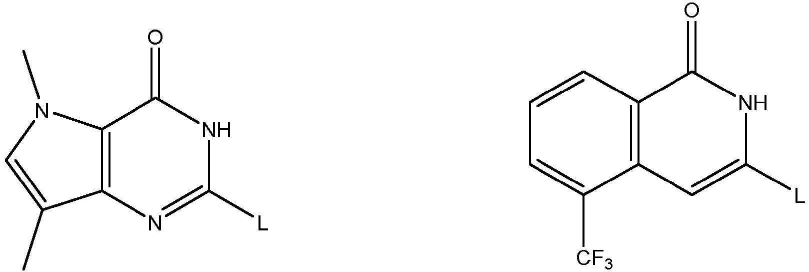 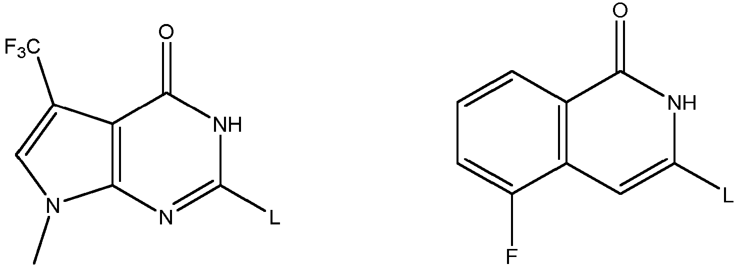 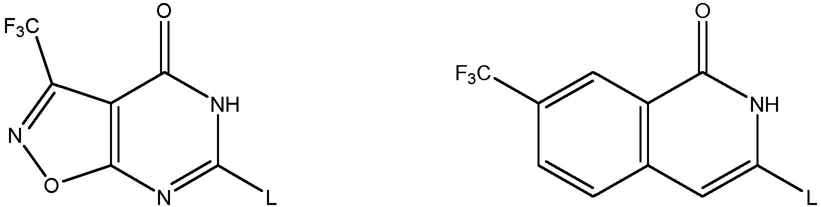 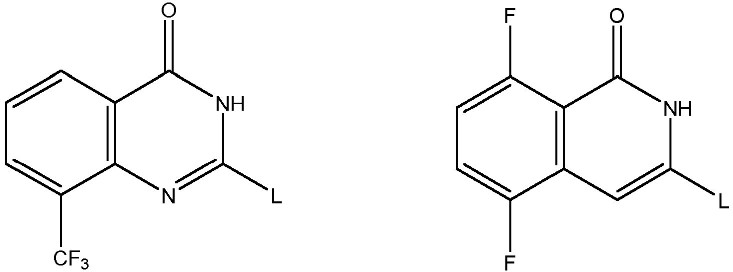 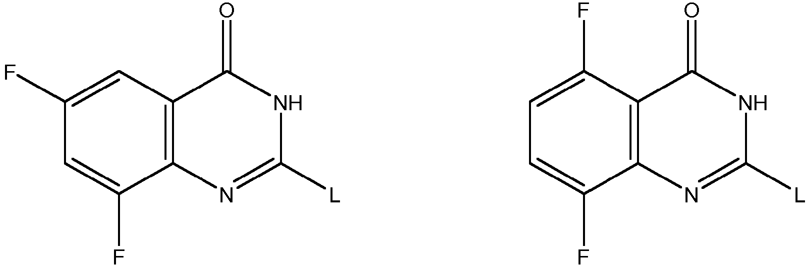 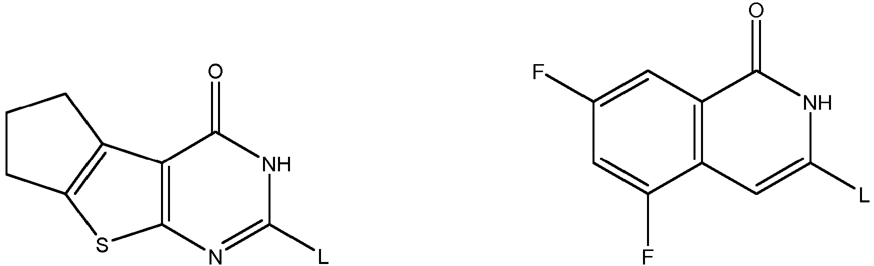 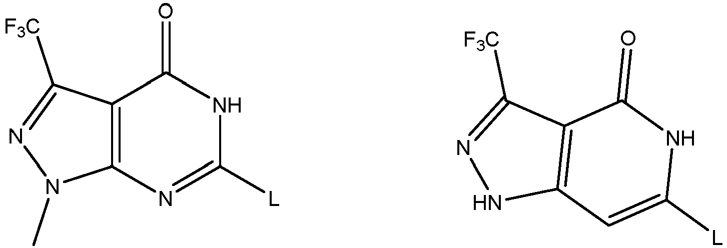 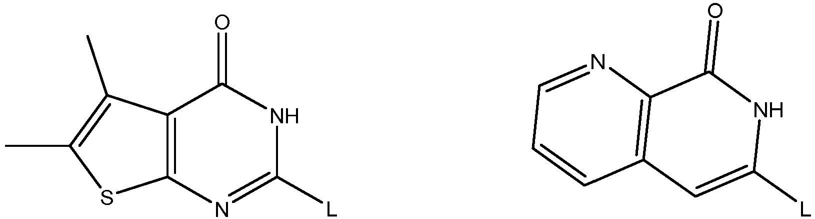 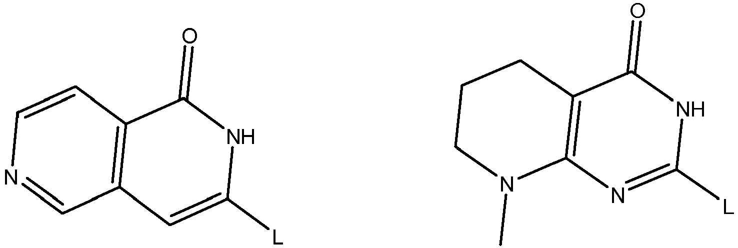 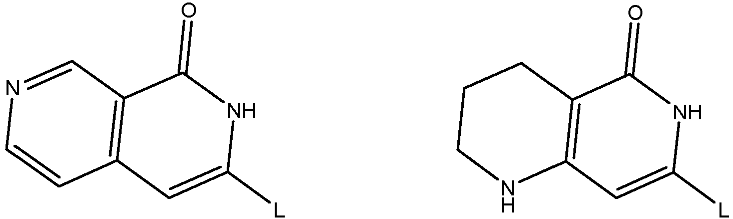 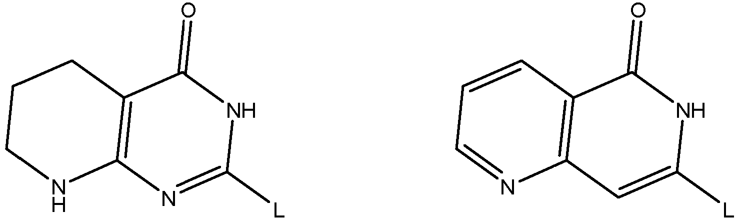 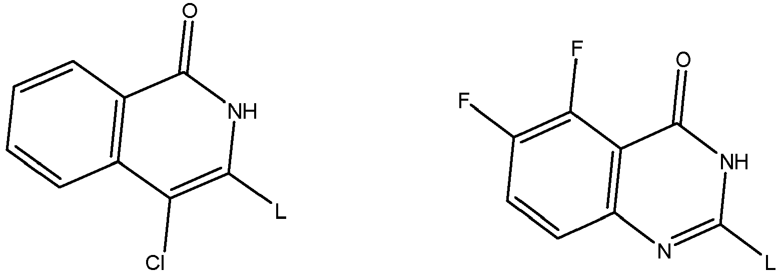 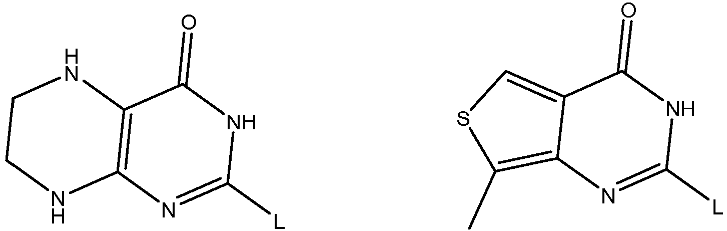 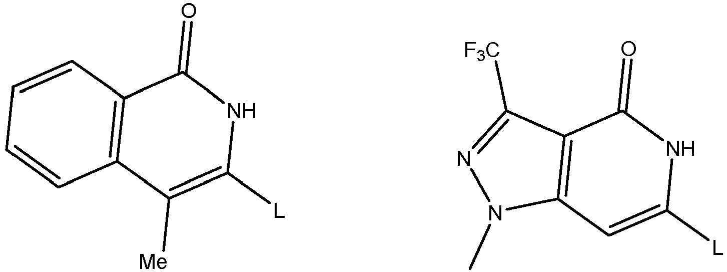 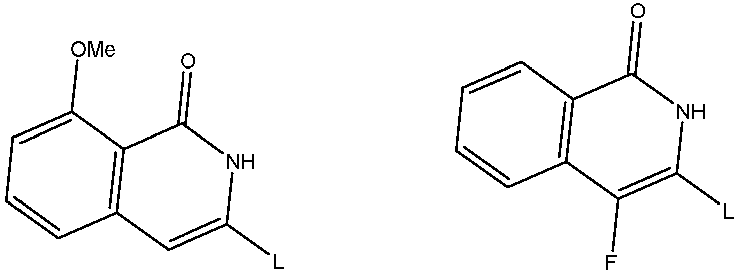 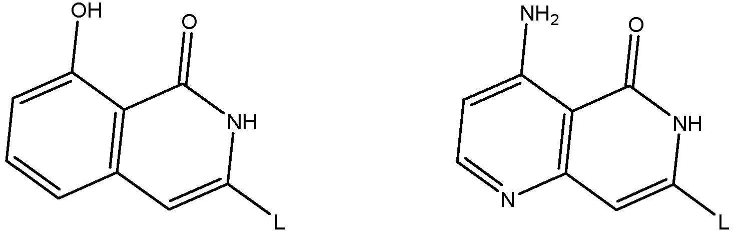 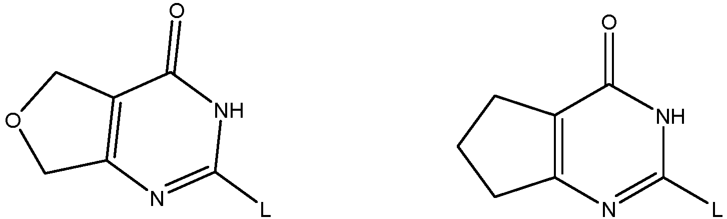 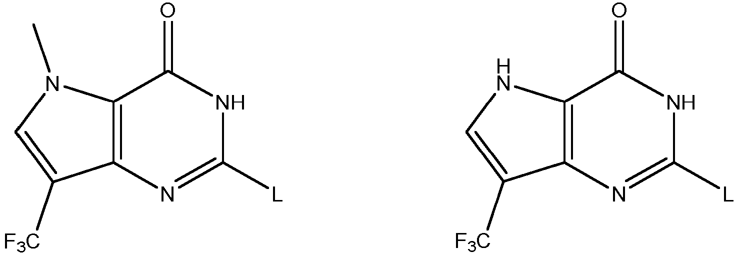 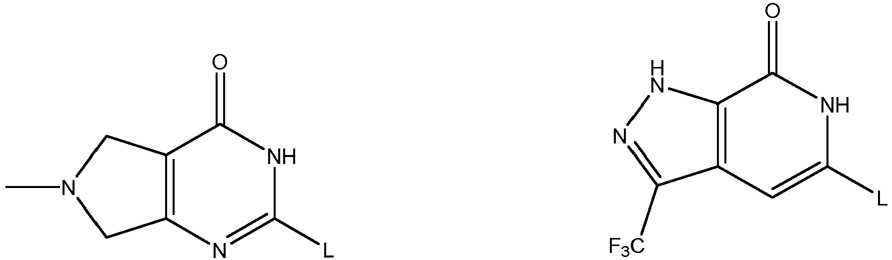 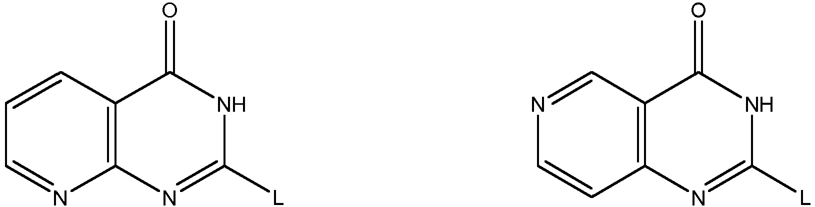 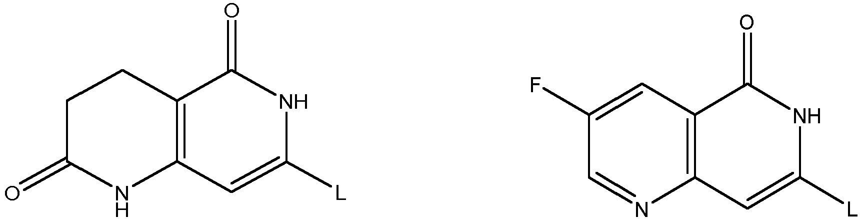 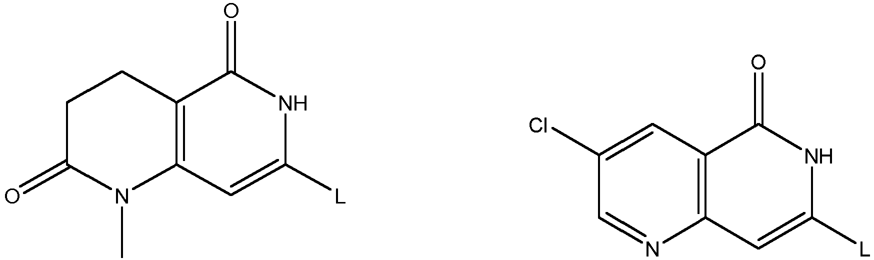 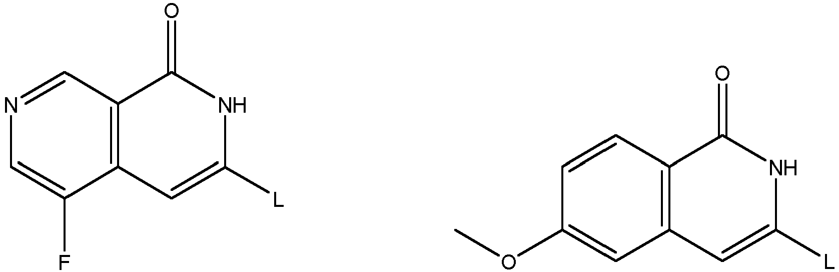 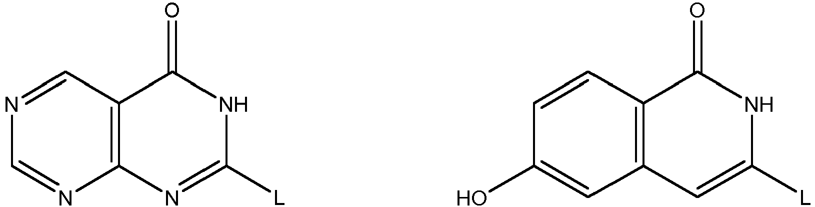 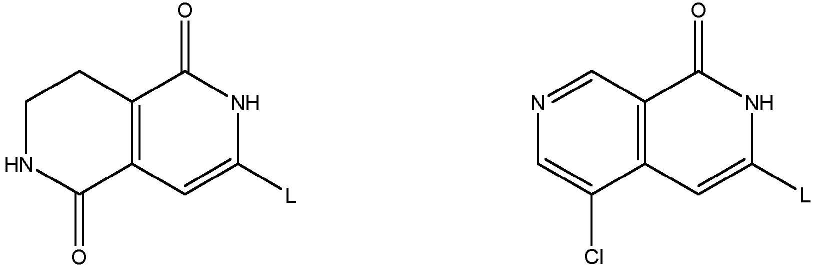 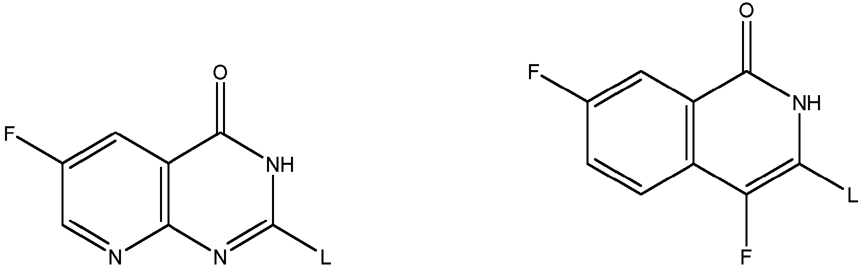 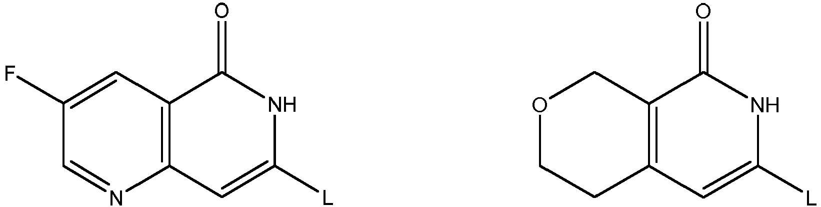 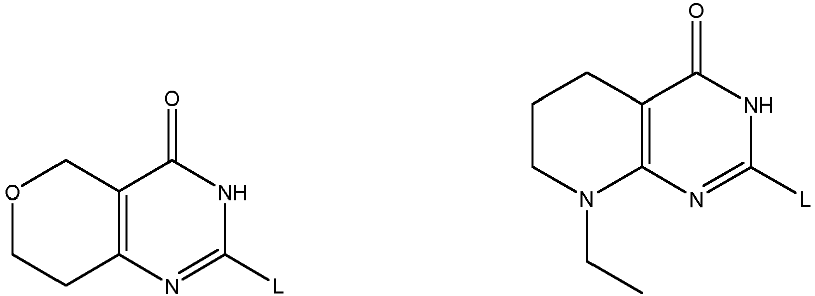 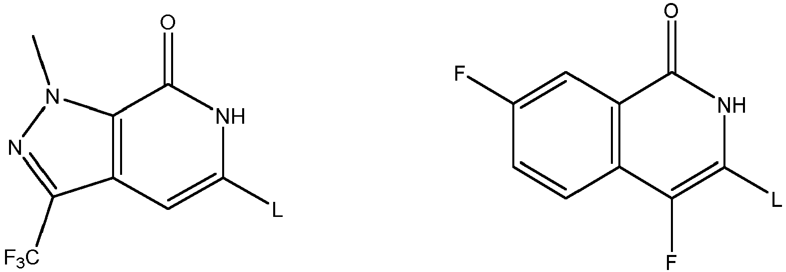 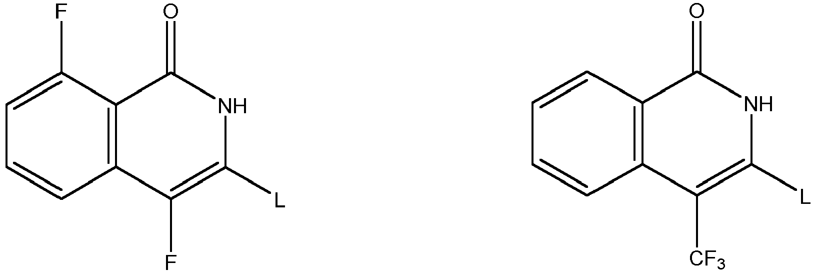 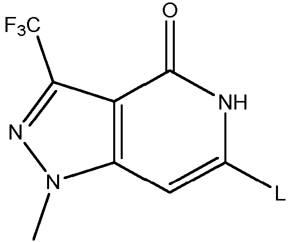 wherein L is as defined herein.

In some reference examples, not in accordance with the present claims, a compound is provided which comprises any of the following structures: 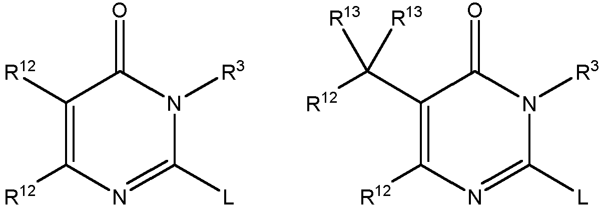 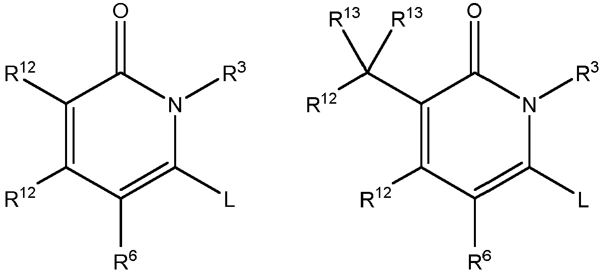 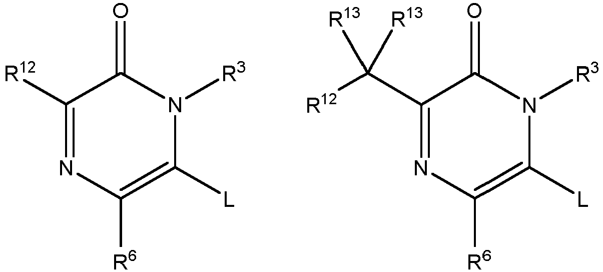 wherein each R¹² is independently selected from H and a substituted or unsubstituted organic group, preferably a lower (C₁ to C₆) alkyl, alkoxy or haloalkyl group, a substituted or unsubstituted C₃ to C₆ cycloalkyl or heterocyclic group, and a halogen group, wherein at least one R¹² is not H; and wherein each R¹³ may be the same or different and is independently selected from H and a substituted or unsubstituted organic group; and wherein R³, R⁶, and L are as defined herein.

At least one R¹² is preferably selected from -CH₃, -CH₂CH₃, -CH₂CH₂CH₃, -CH₂Cl,-CHCl₂, -CCl₃, -CH₂F, -CHF₂, -CF₃, -Cl, -F, -Cl, -CH₂CF₃, -CH₂CH₂F, -CH₂CH₂OH, methoxy, methoxymethyl, methoxyethyl, isopropyl, cyclopropyl or cyclopropylmethyl.

R¹³ is preferably selected from H, F, C₁ to C₃ alkyl or C₁ to C₃ fluoroalkyl.

R³ is preferably H.

R⁶ is preferably selected from H, halogen, C₁ to C₃ alkyl, C₁ to C₃ haloalkyl, C₁ to C₃ alcohol or C₁ to C₃ aminooalkyl.

In some reference examples, a compound is provided which comprises any of the following structures: 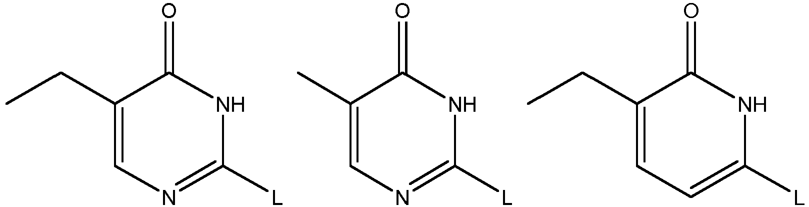 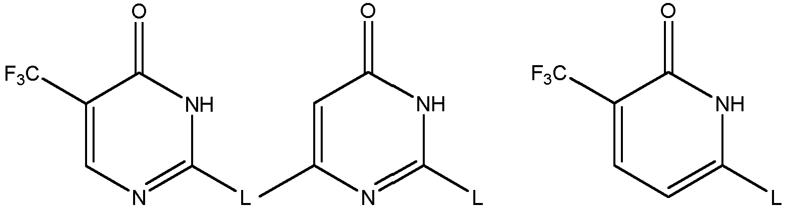 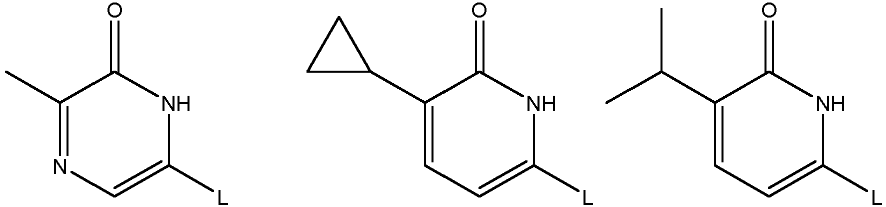 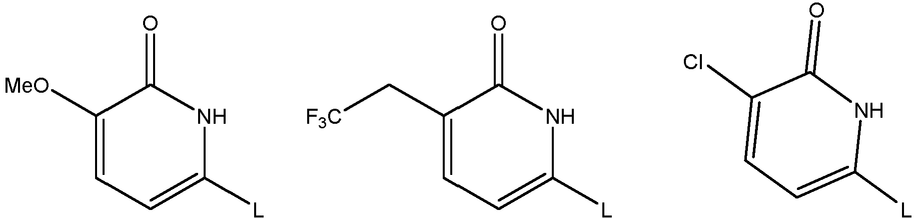 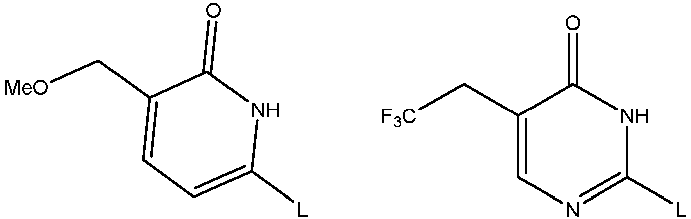 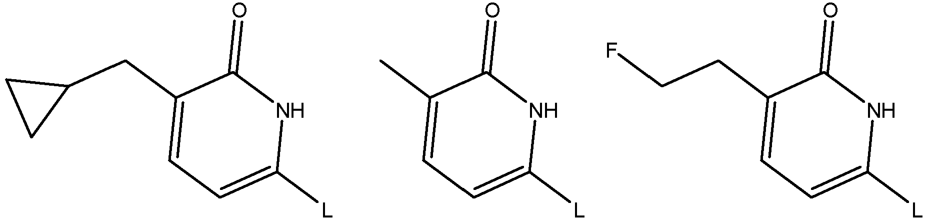 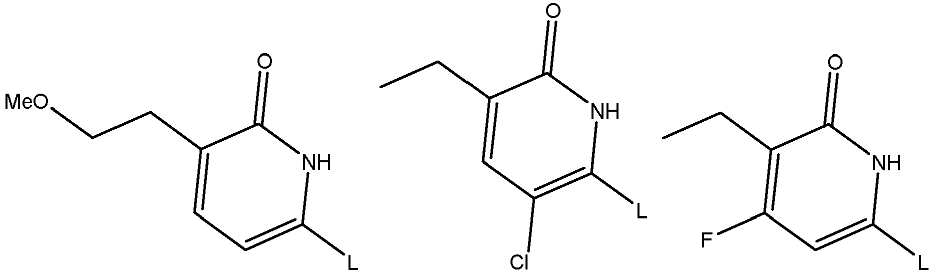 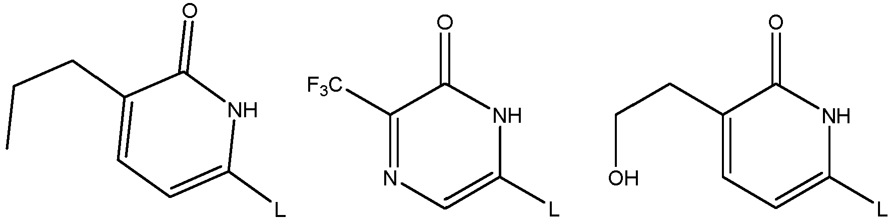 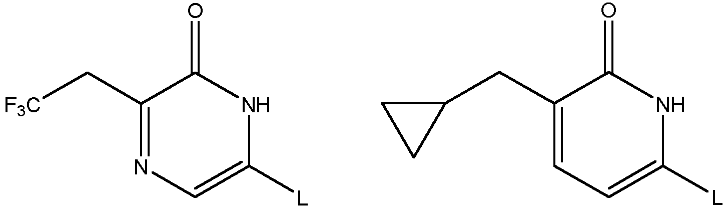 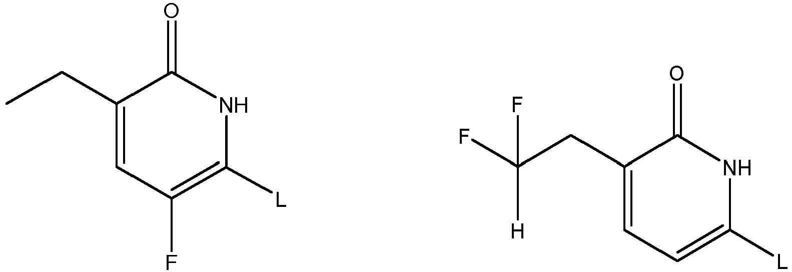 wherein L is as defined herein.

The present disclosure provides a PARP1 inhibitor compound which comprises a formula selected from one of the following:

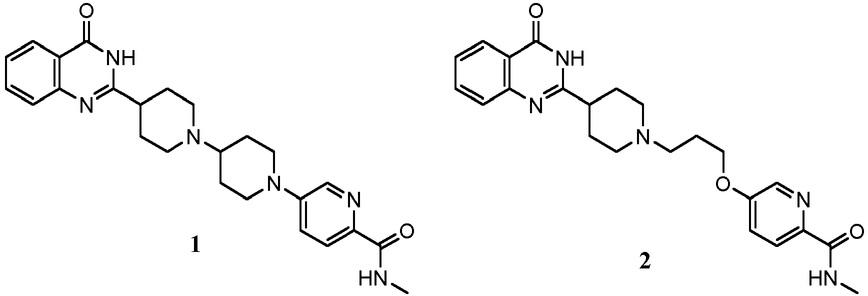

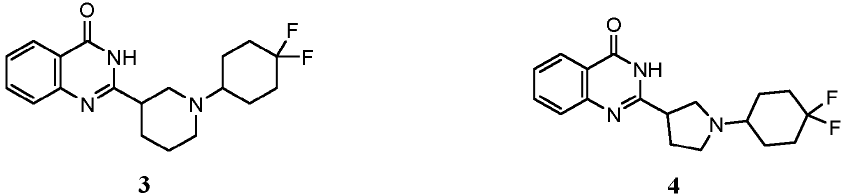

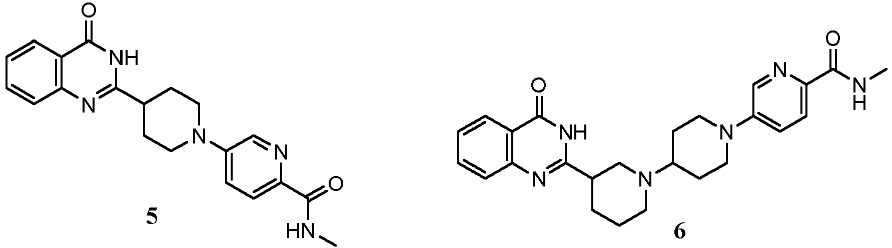

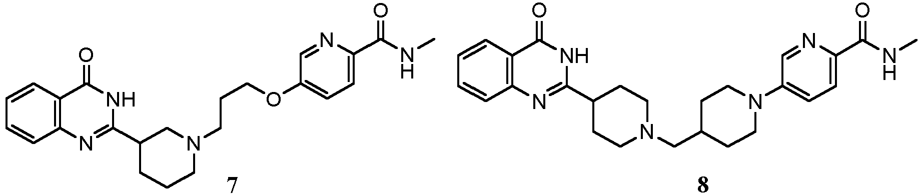

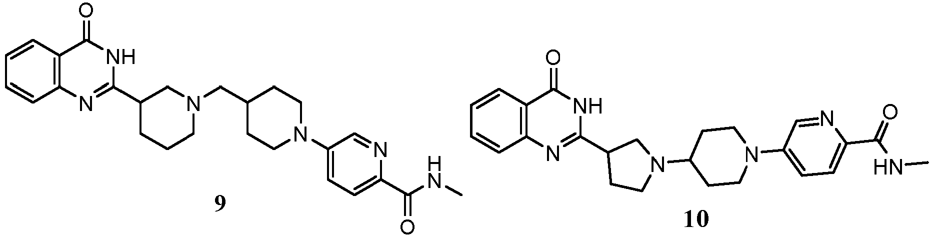

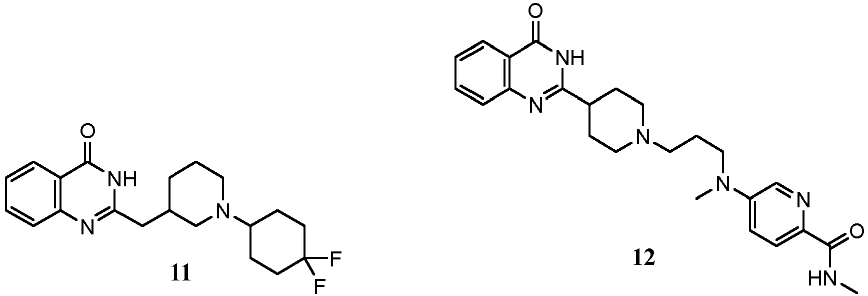

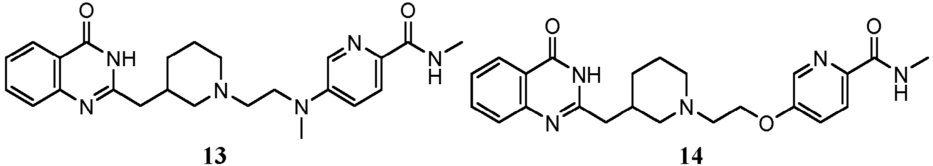

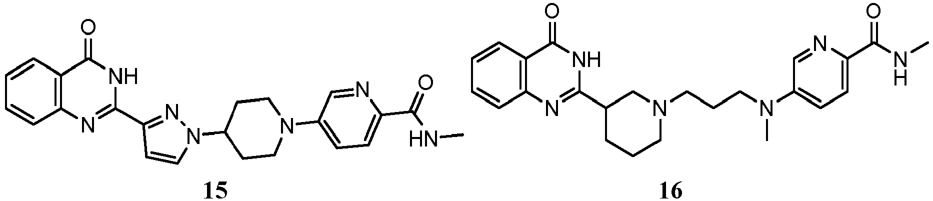

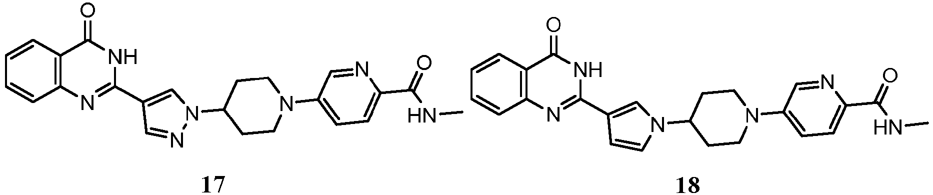

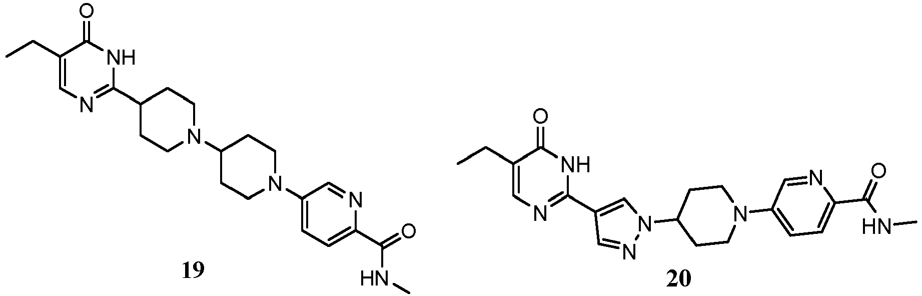

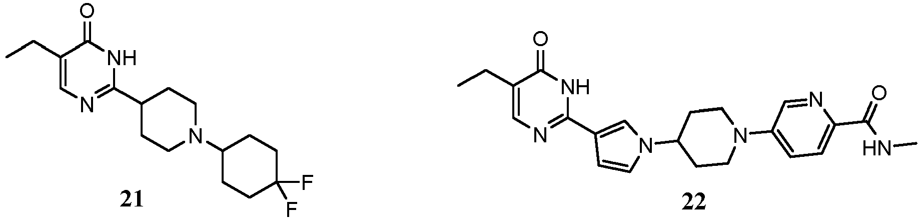

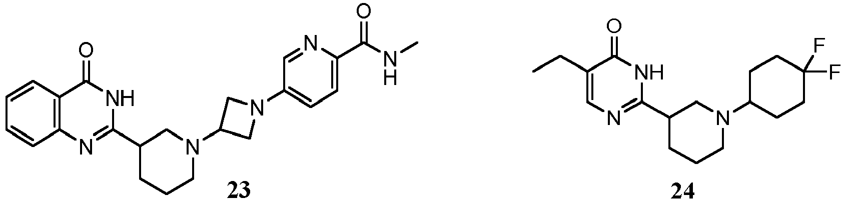

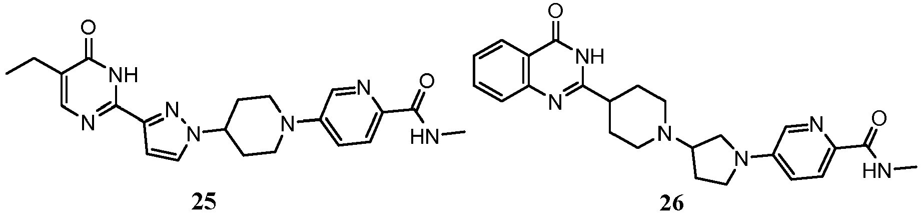

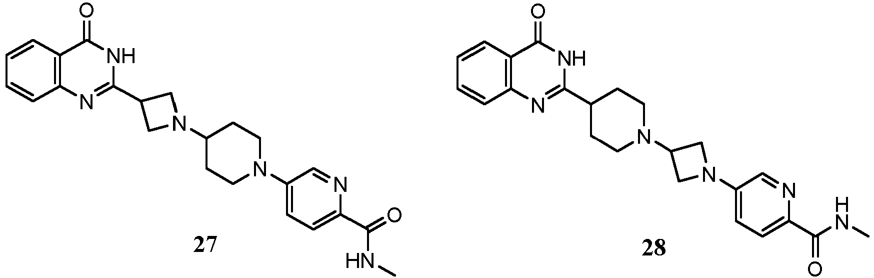

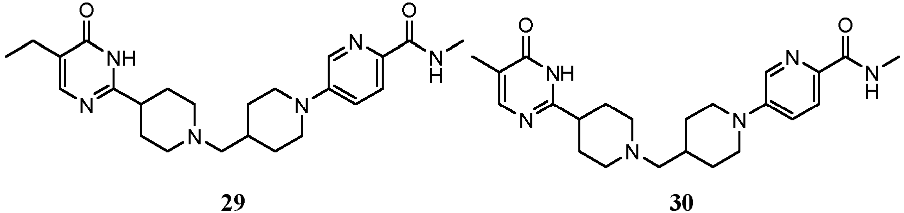

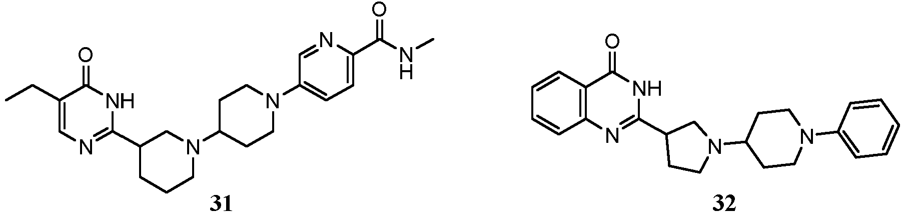

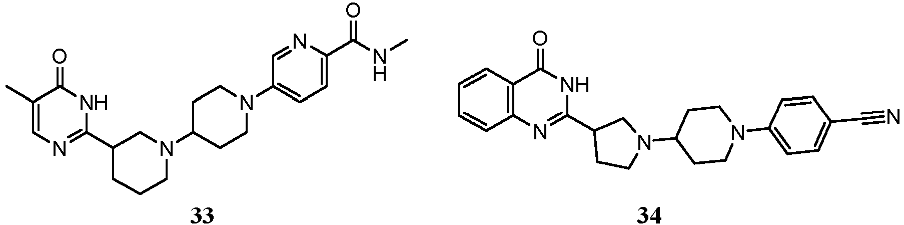

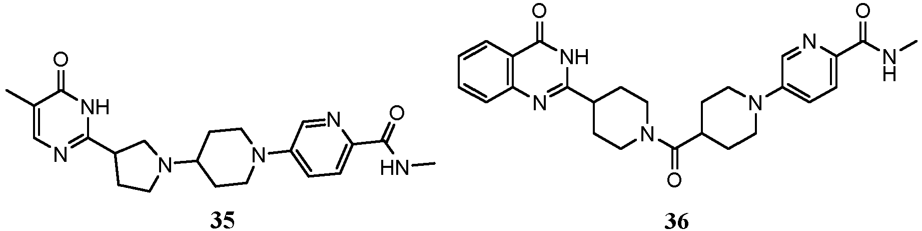

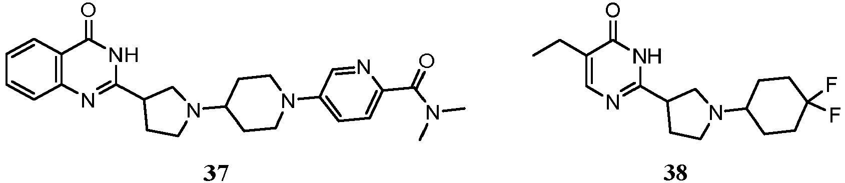

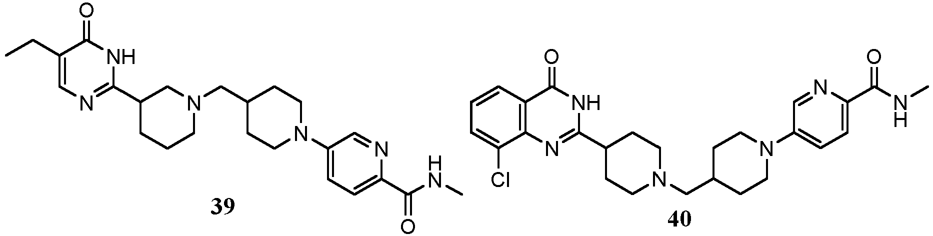

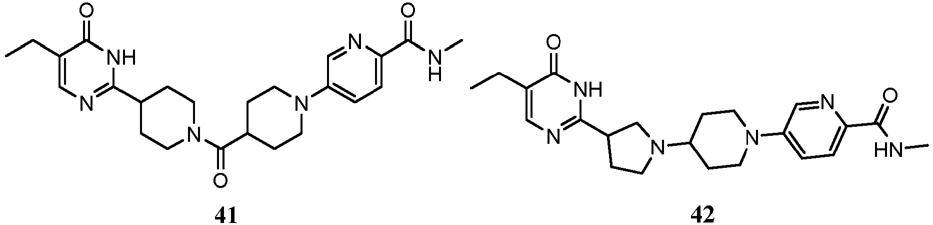

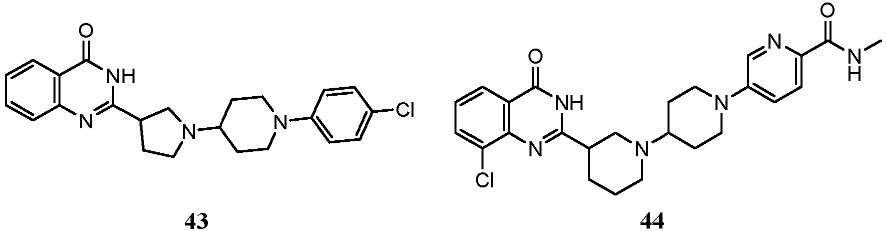

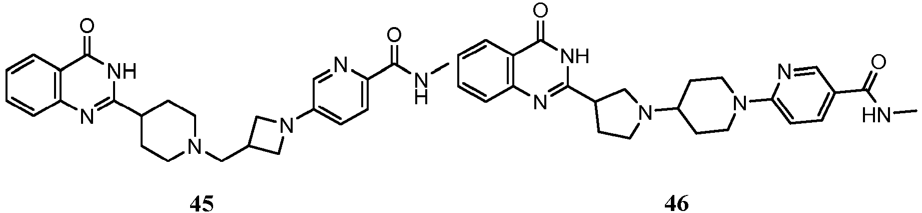

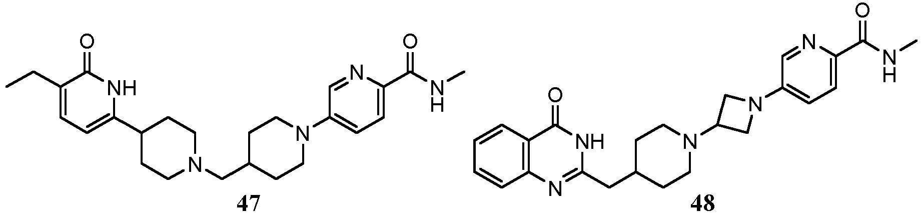

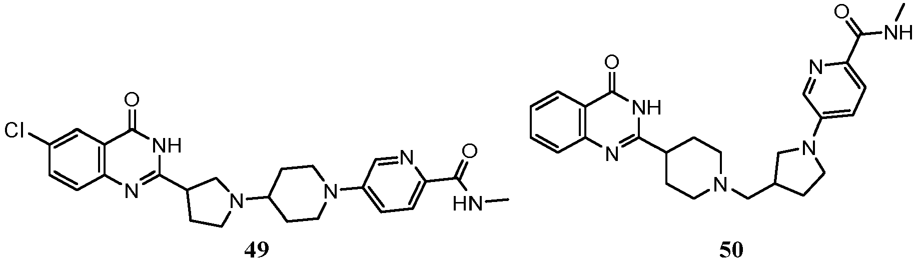

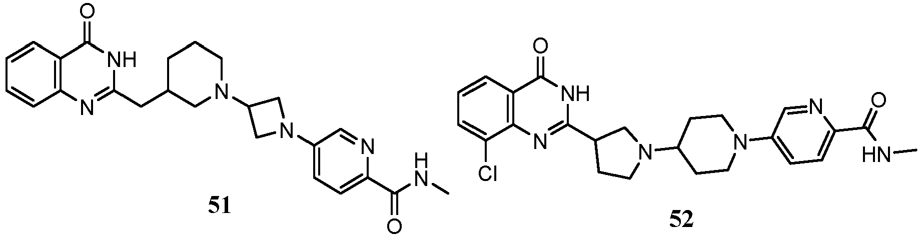

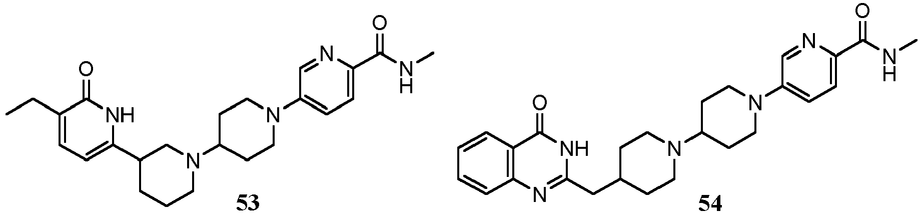

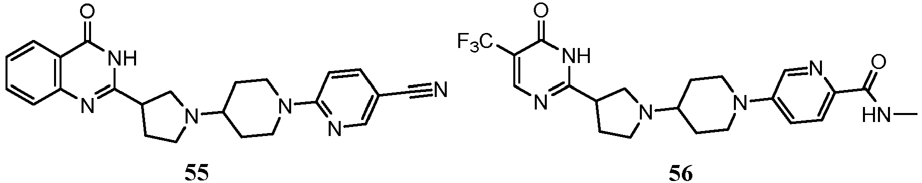

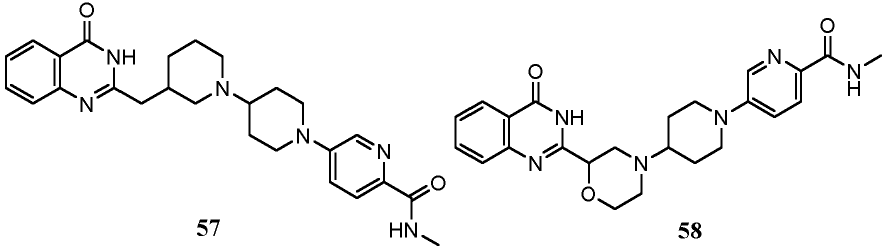

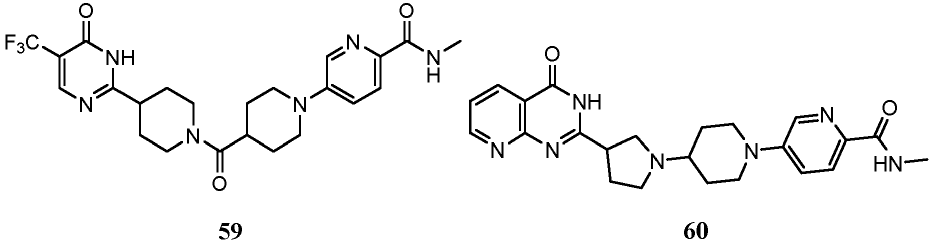

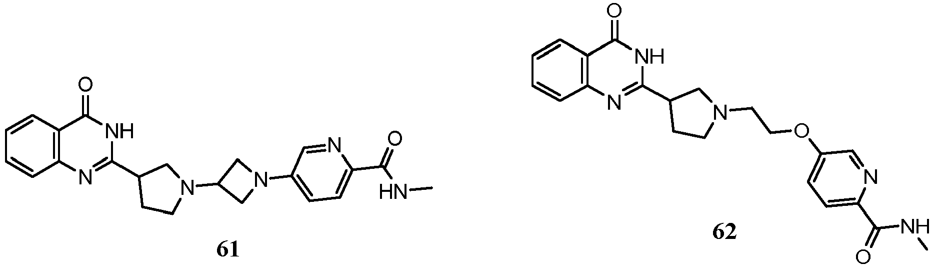

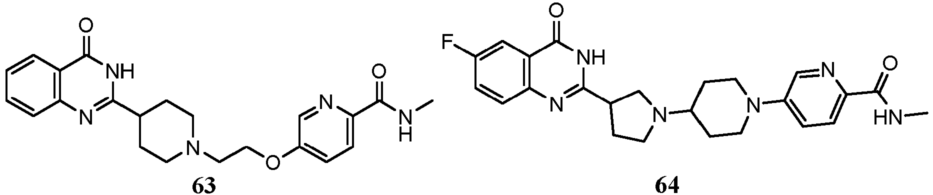

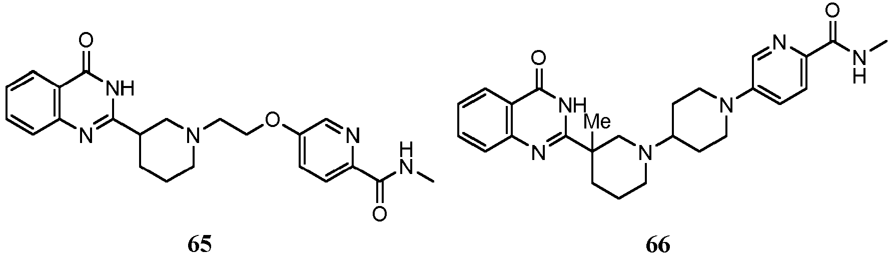

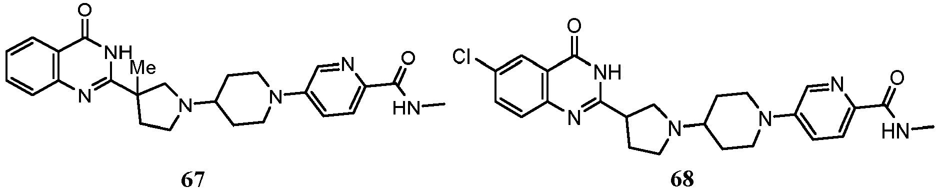

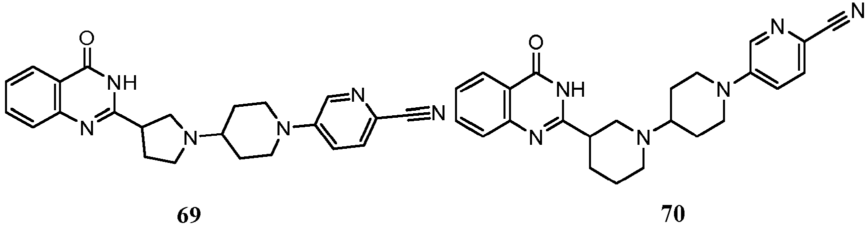

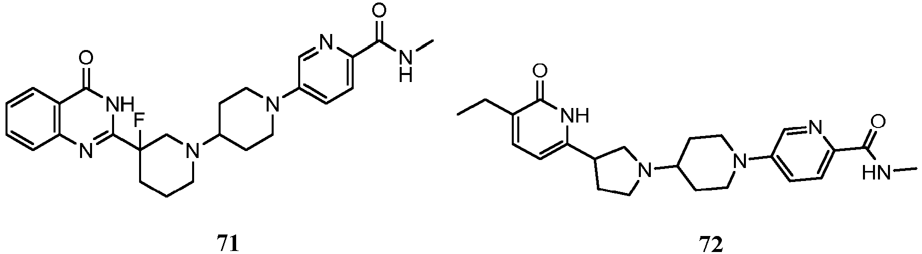

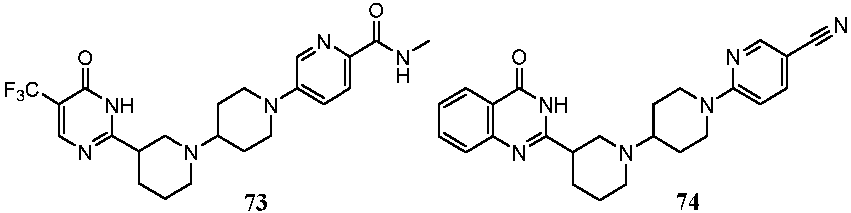

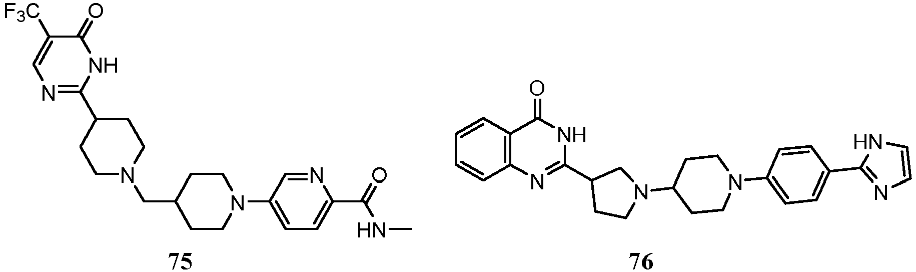

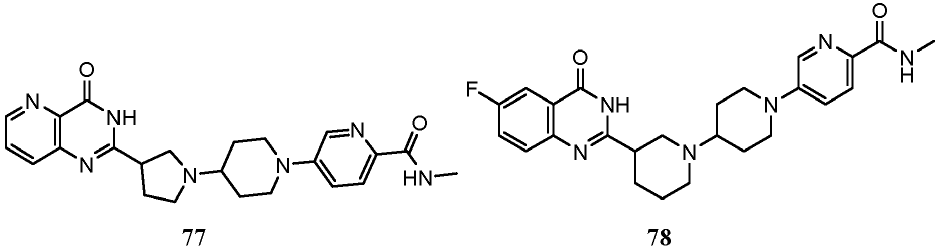

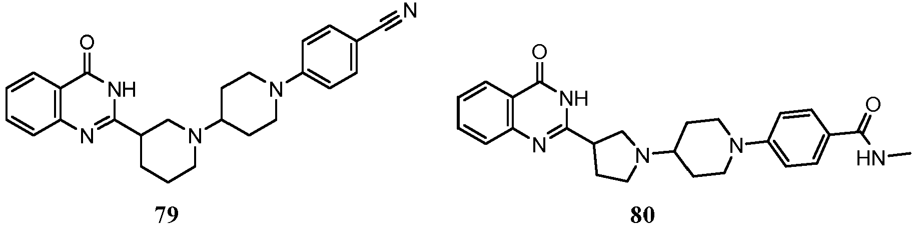

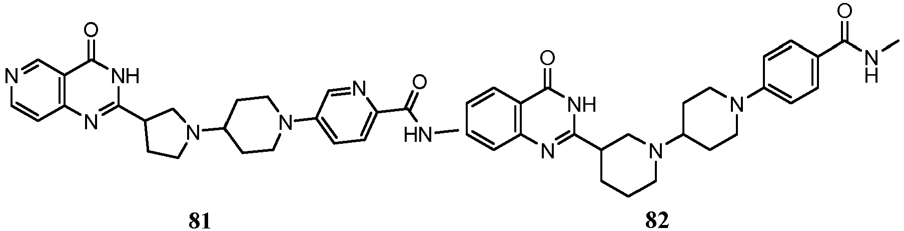

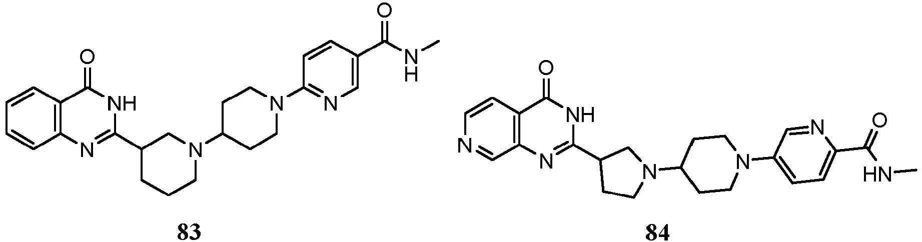

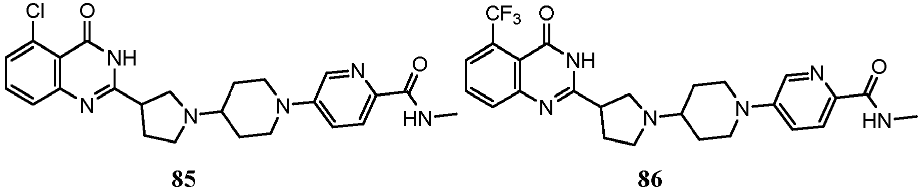

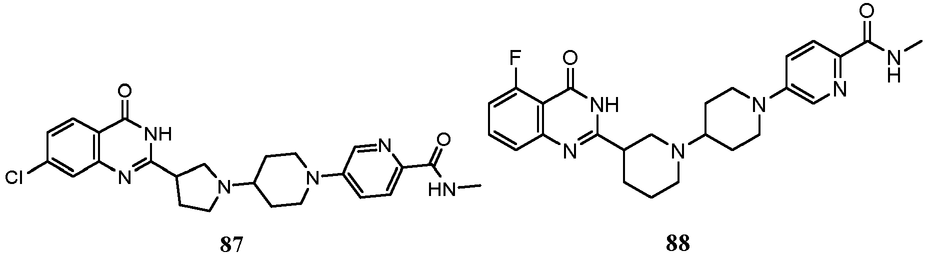

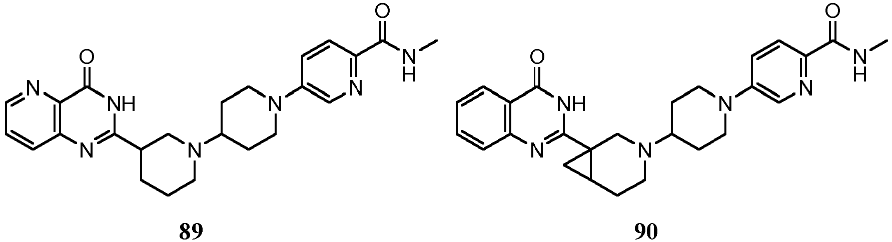

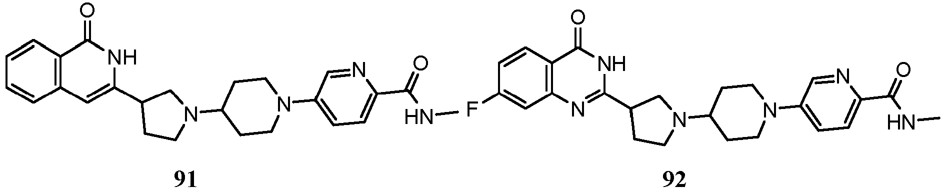

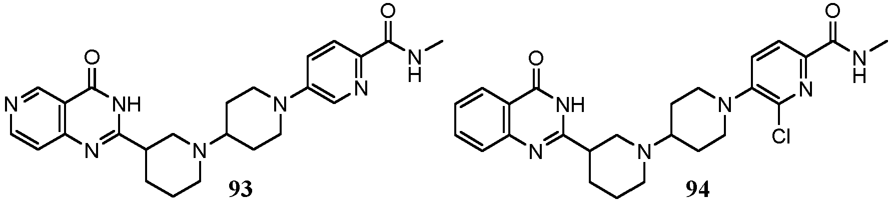

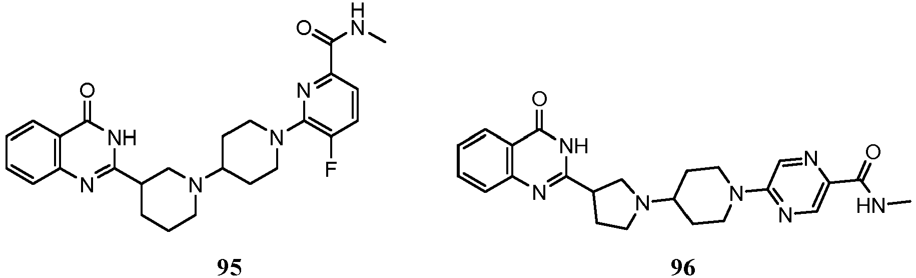

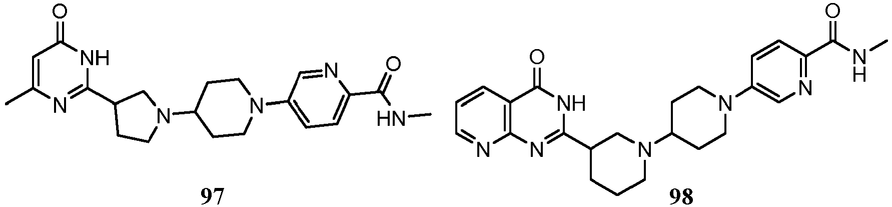

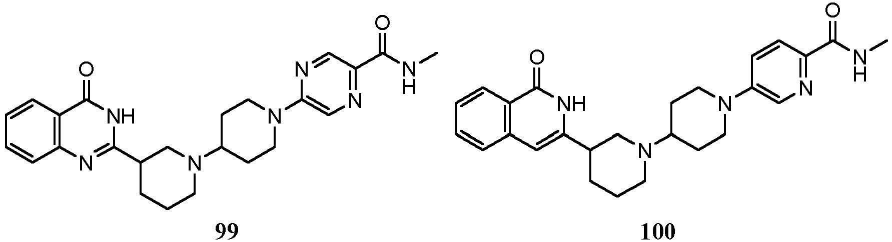

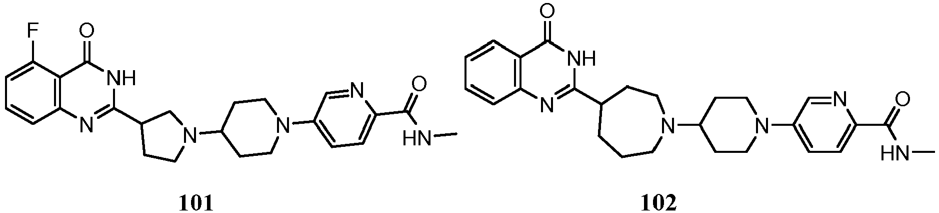

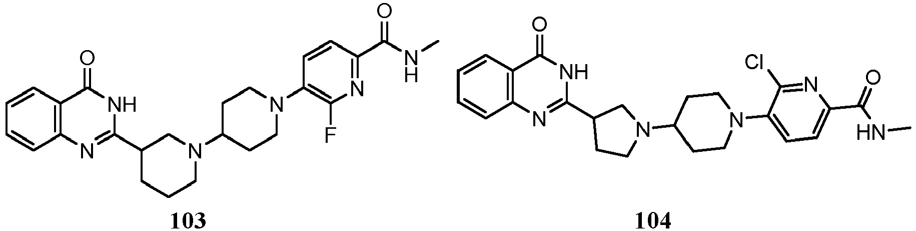

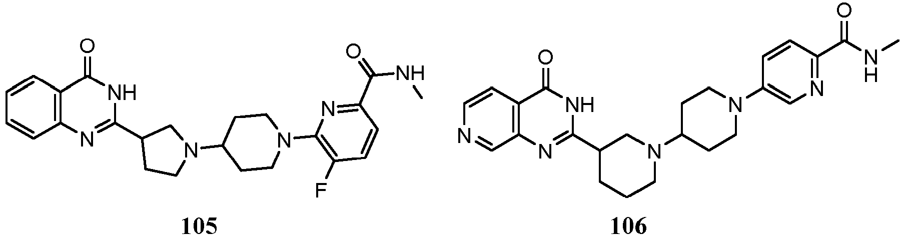

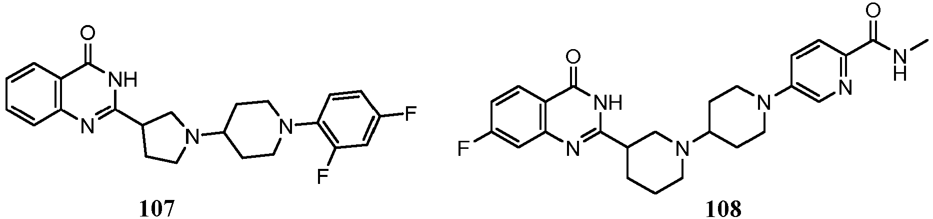

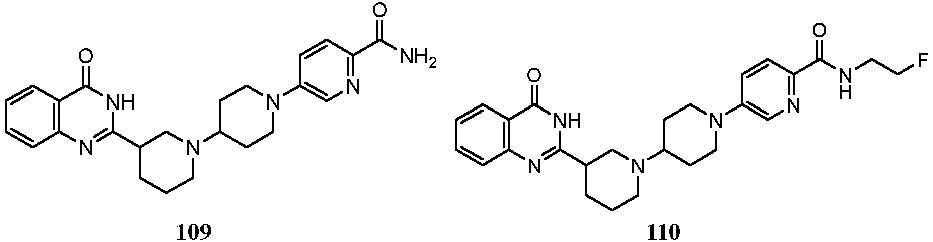

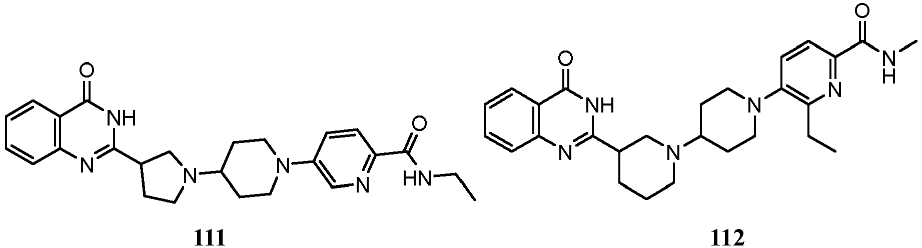

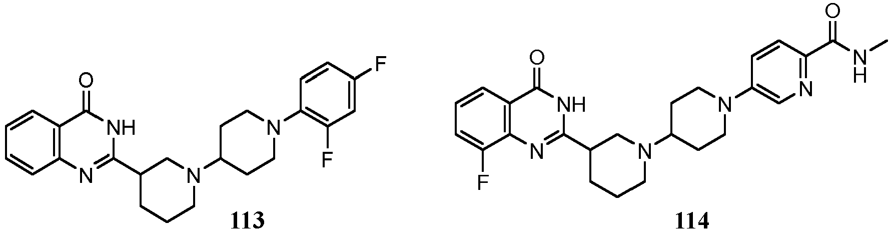

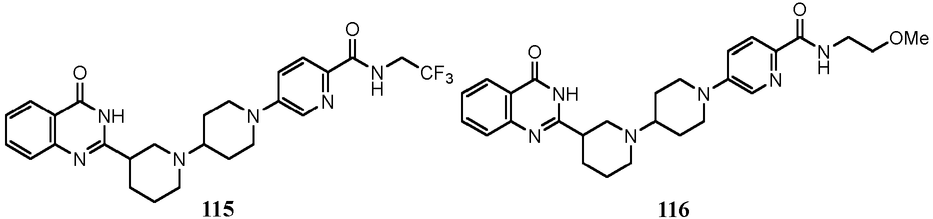

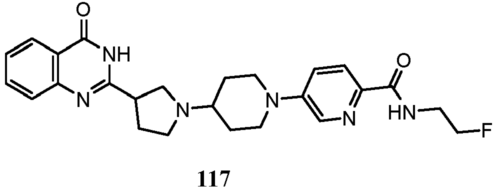

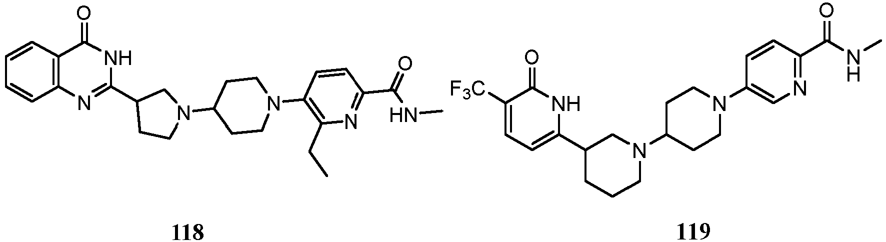

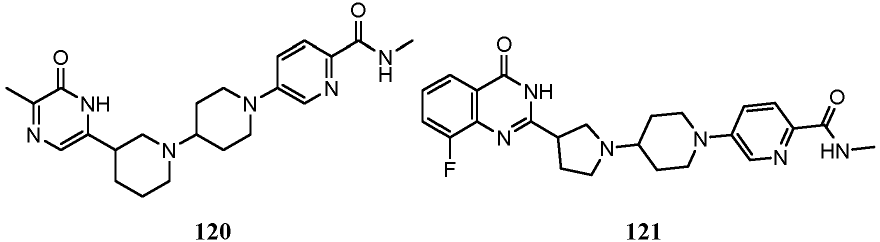

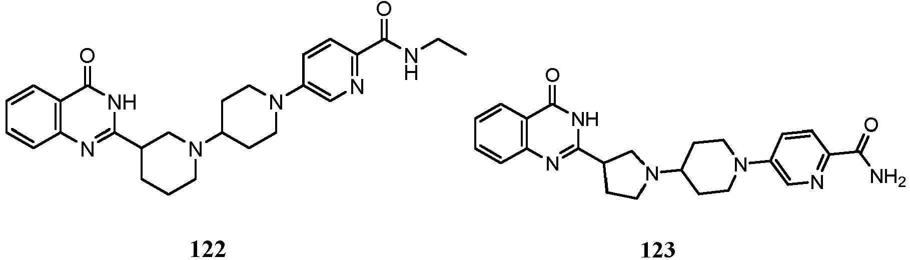

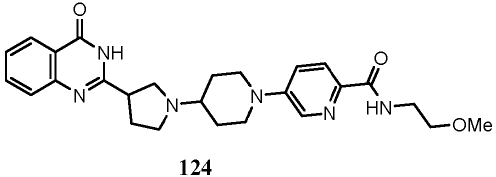

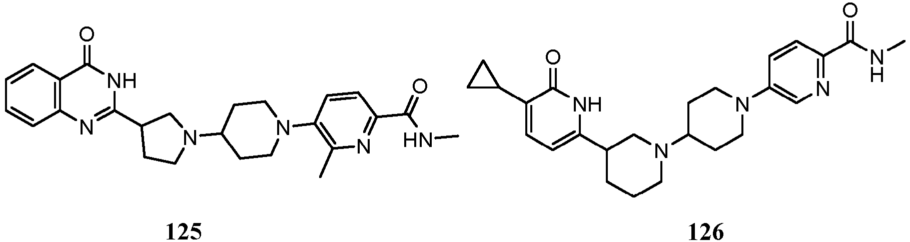

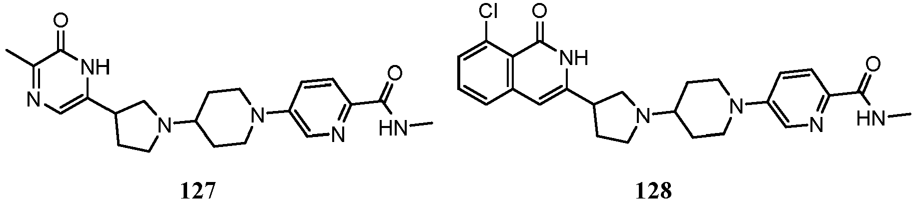

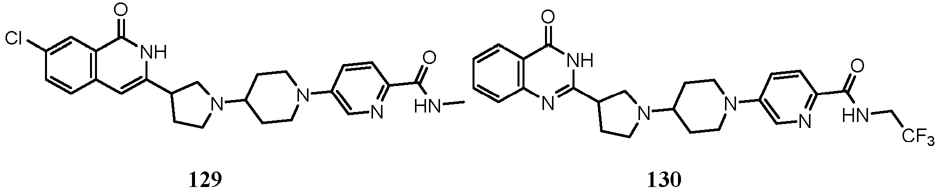

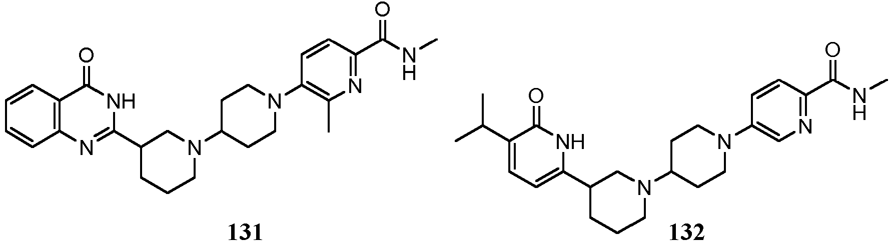

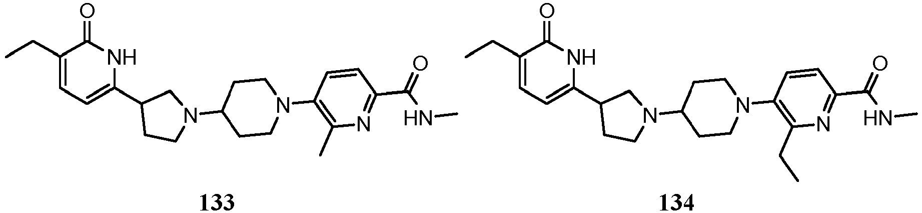

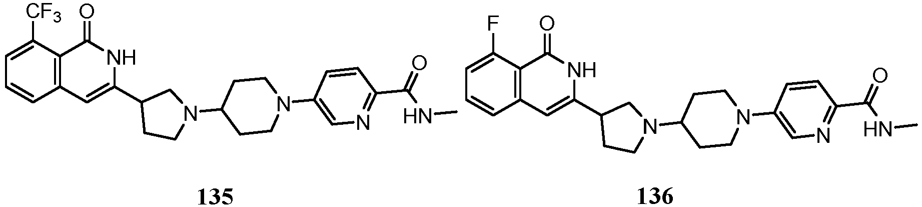

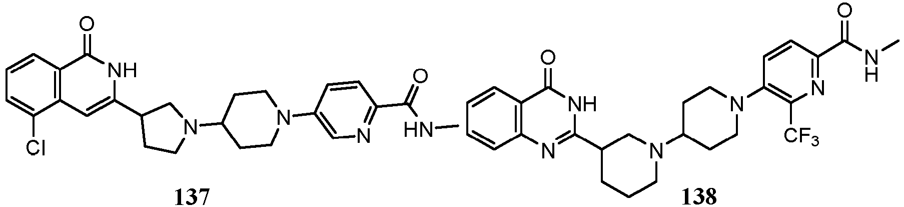

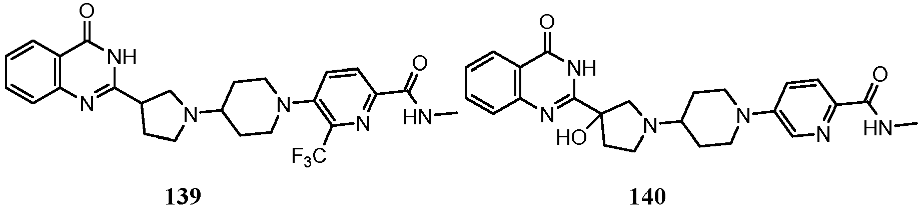

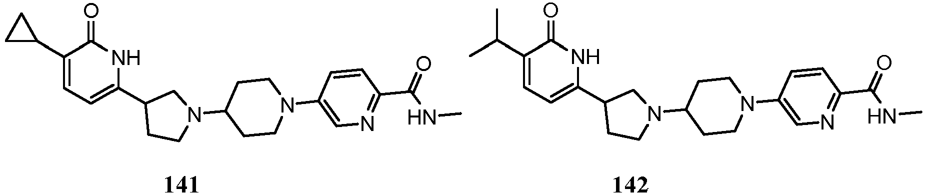

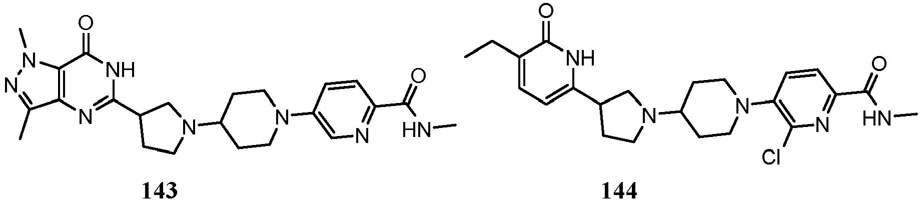

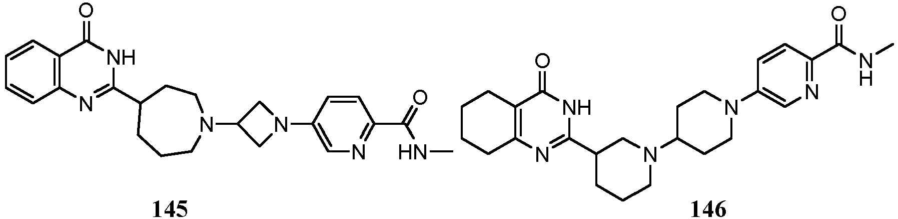

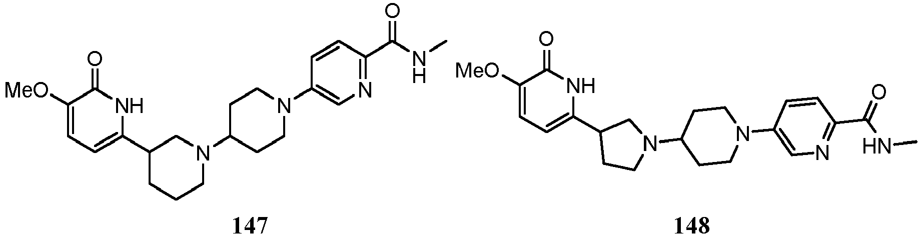

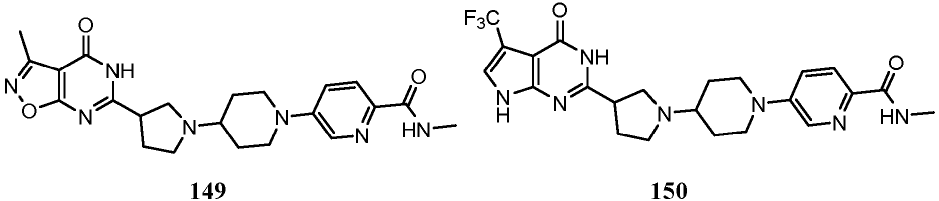

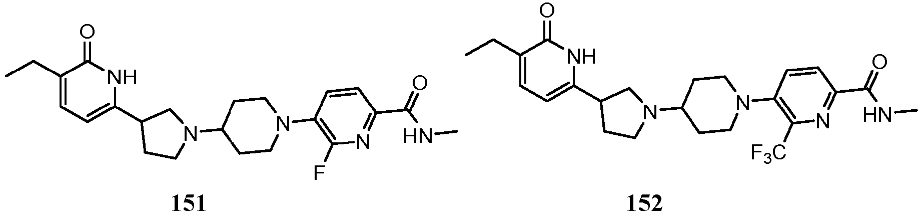

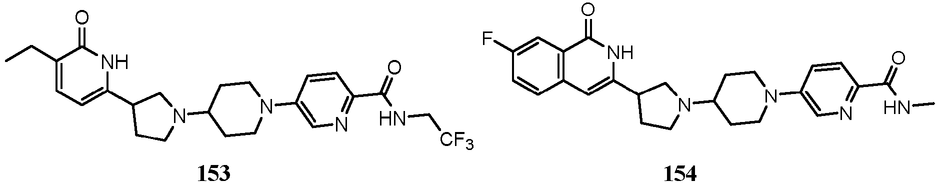

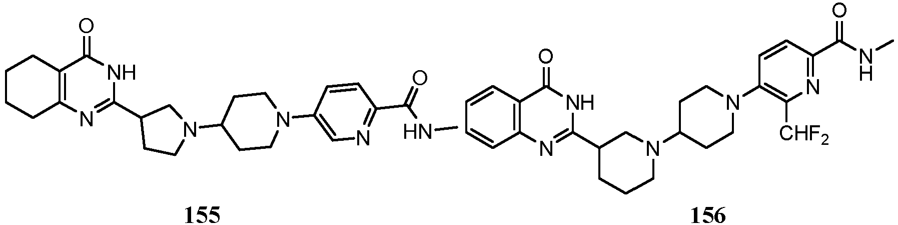

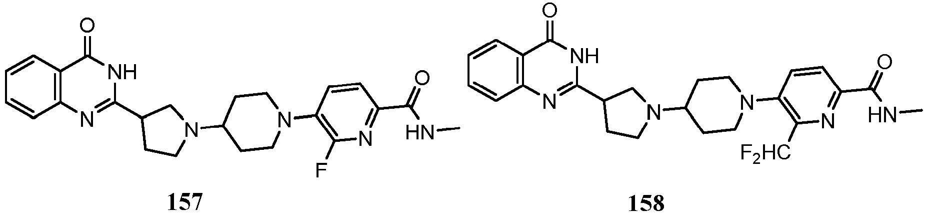

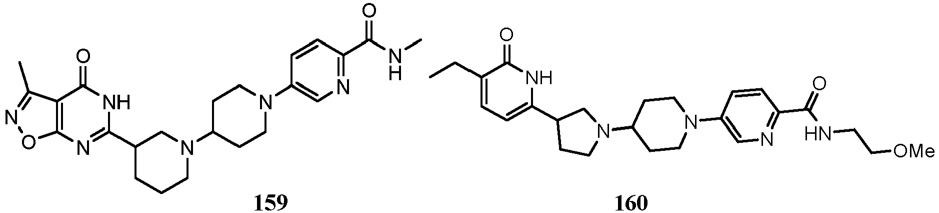

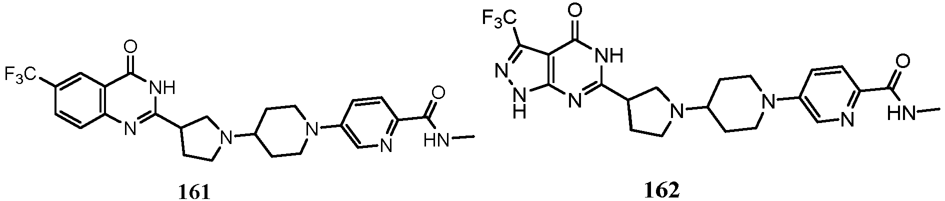

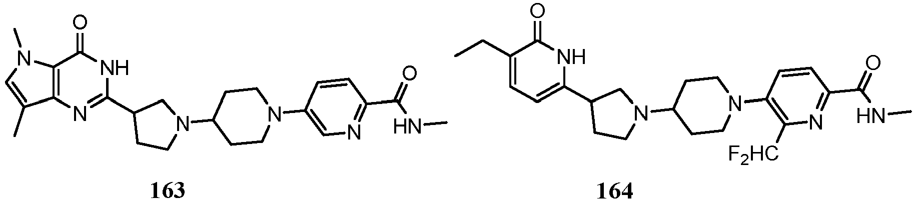

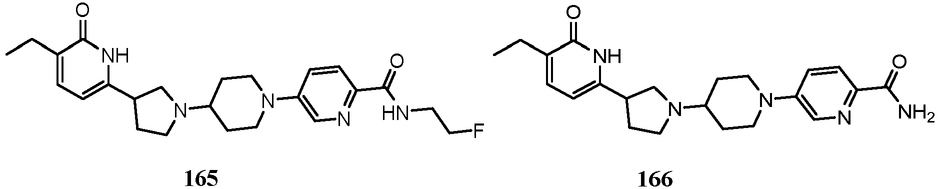

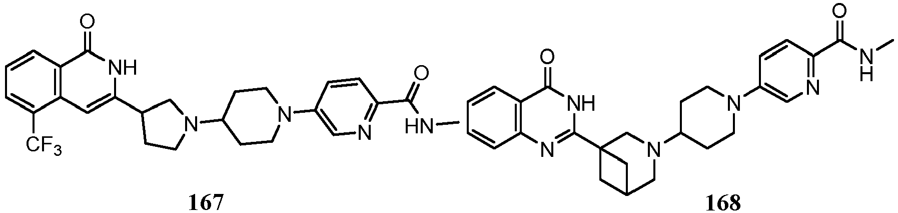

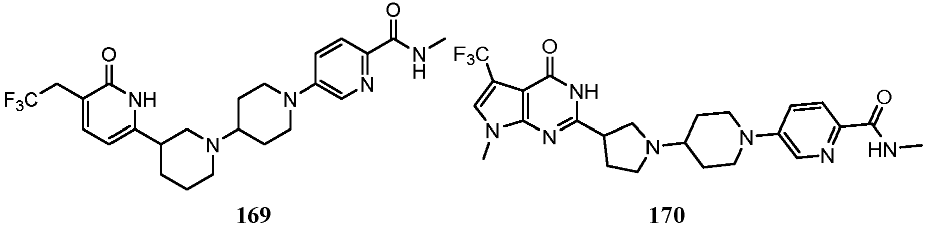

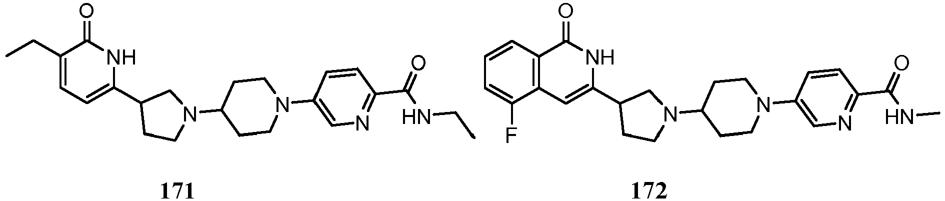

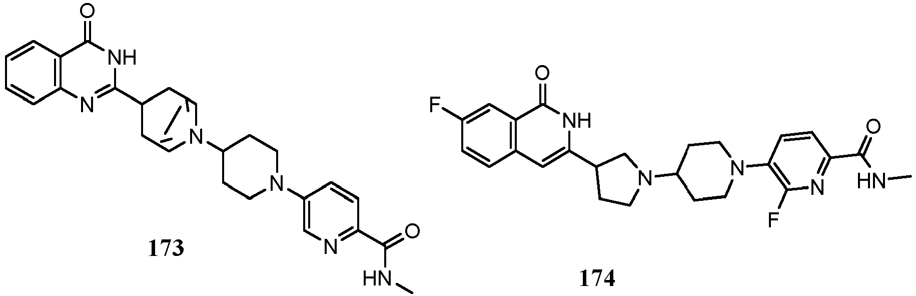

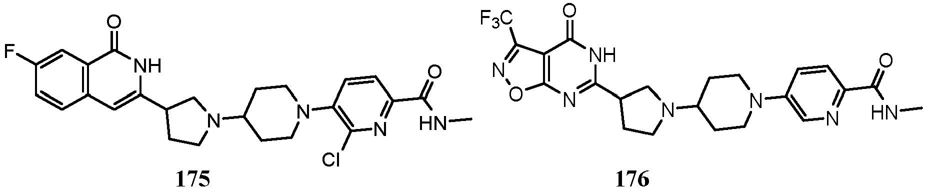

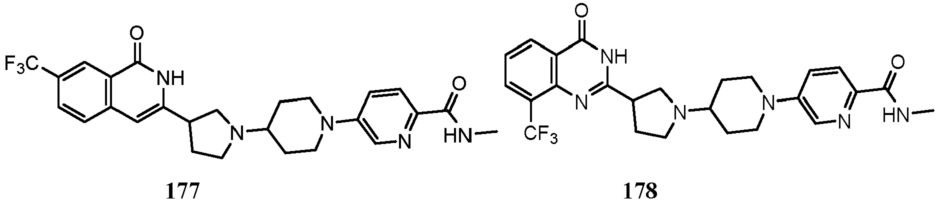

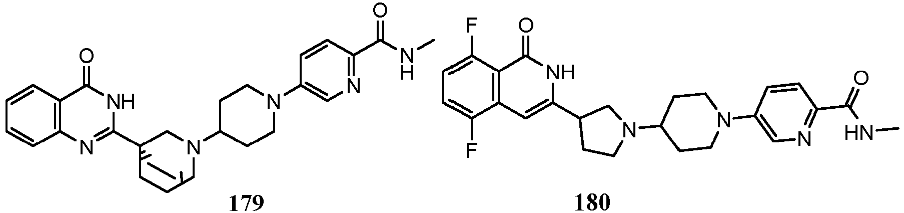

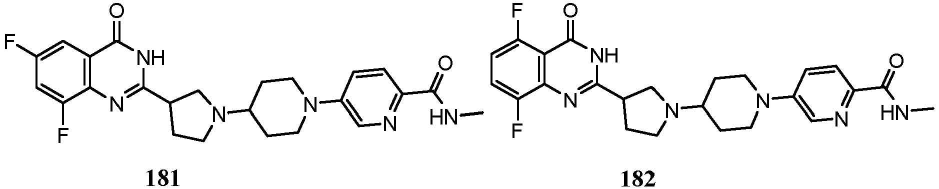

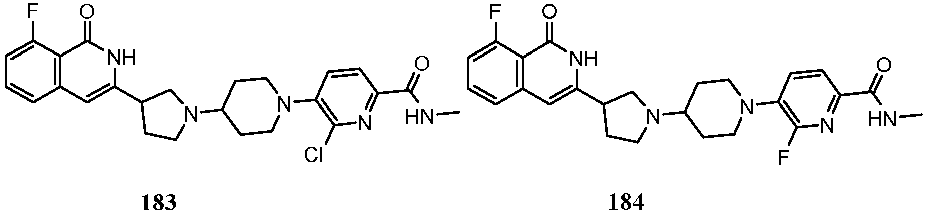

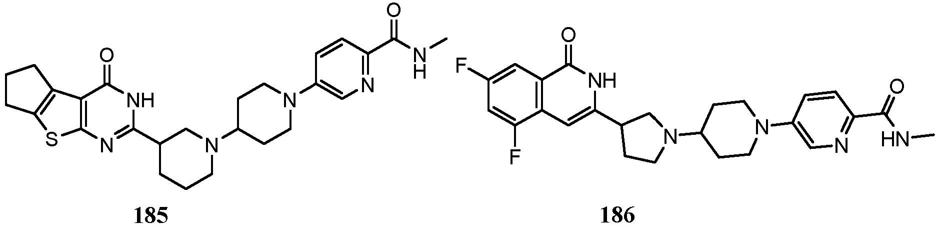

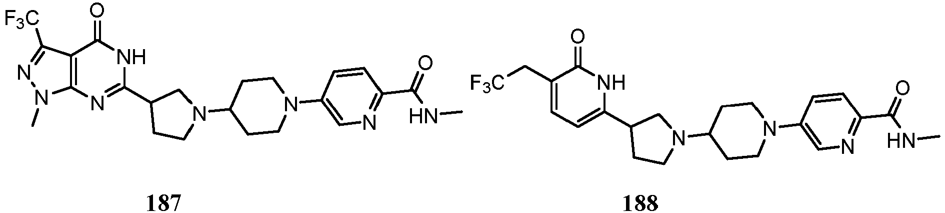

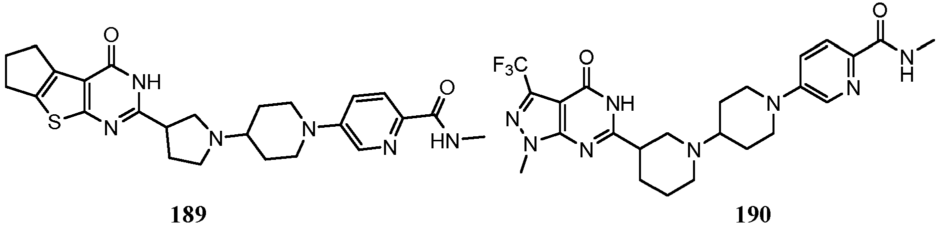

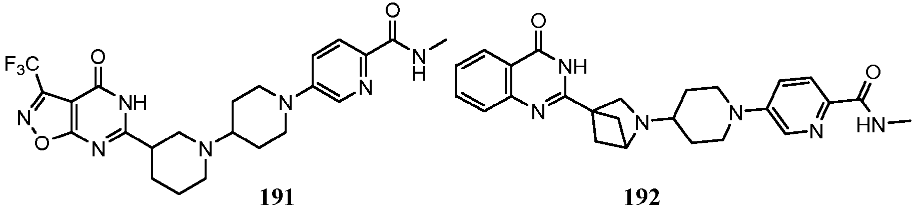

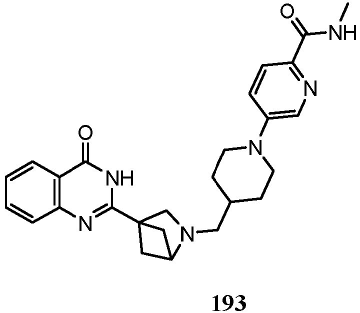

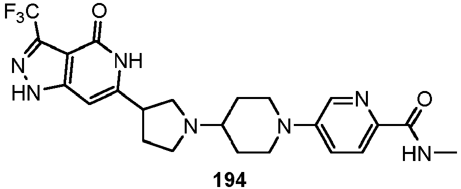

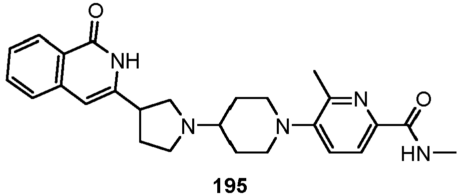

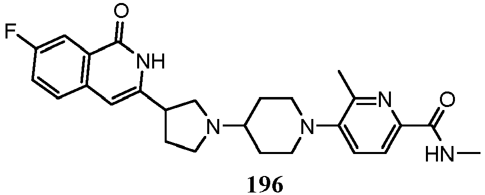

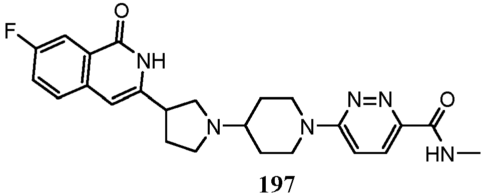

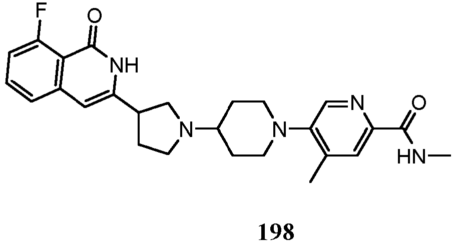

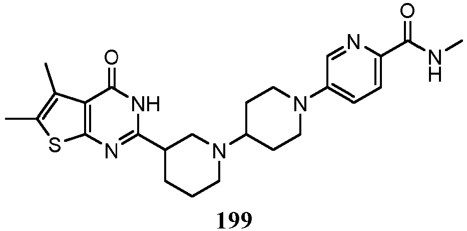

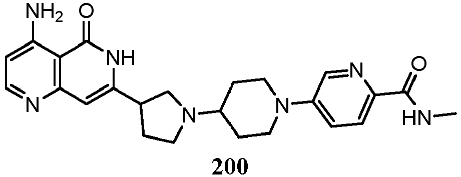

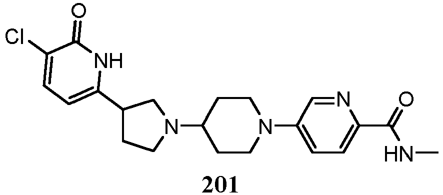

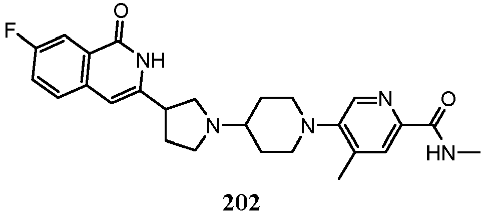

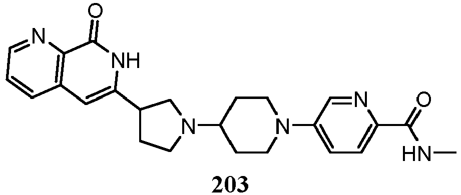

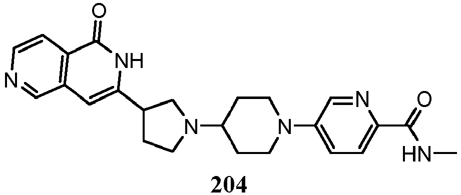

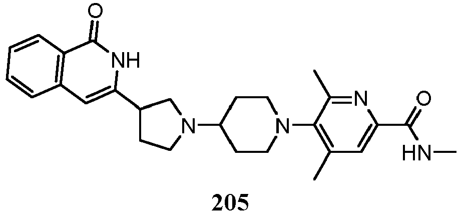

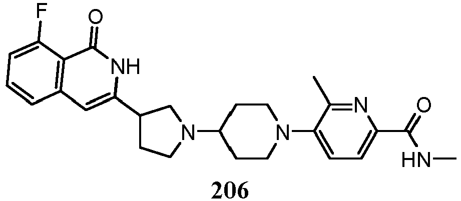

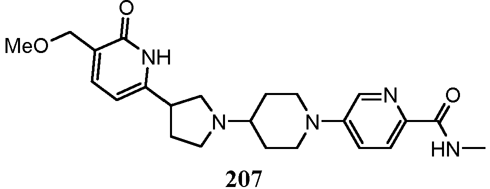

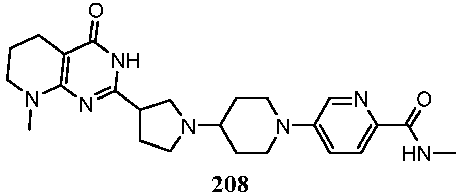

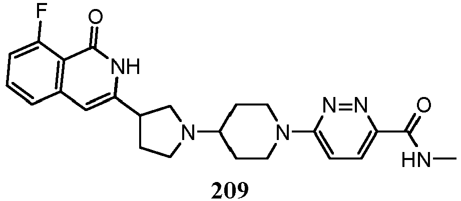

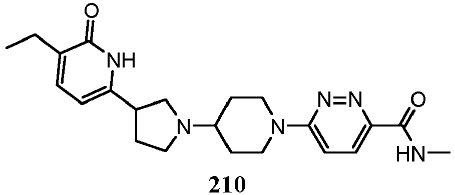

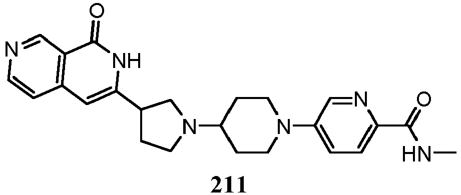

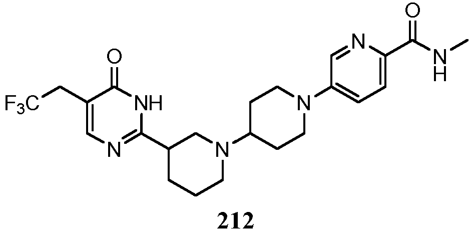

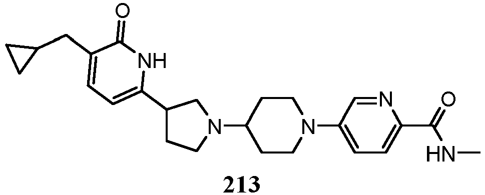

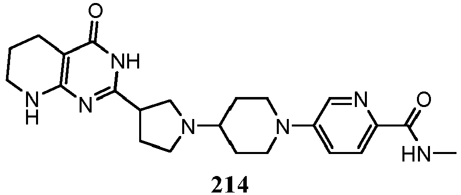

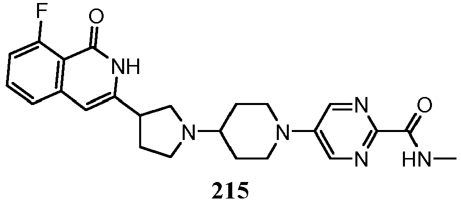

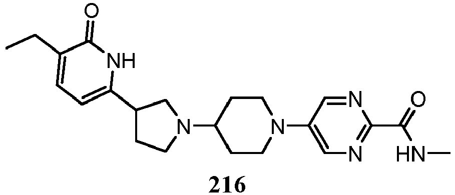

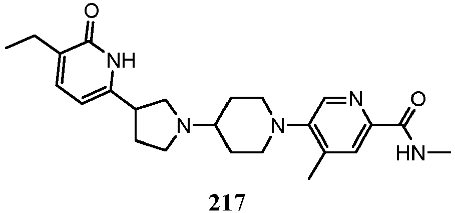

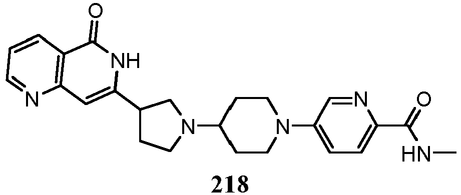

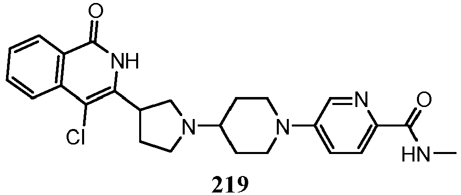

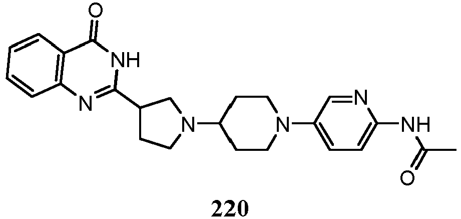

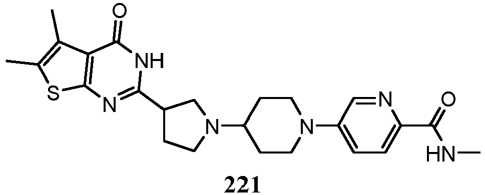

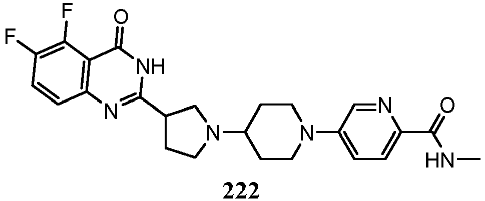

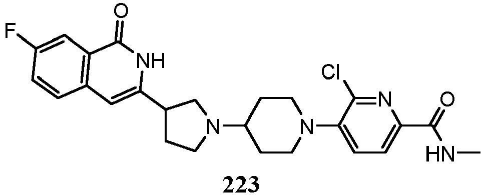

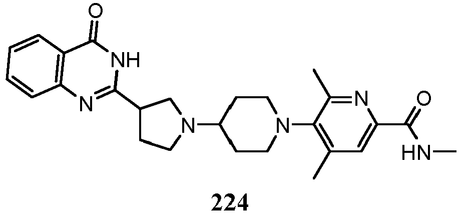

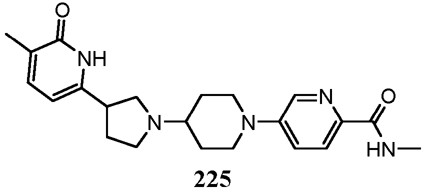

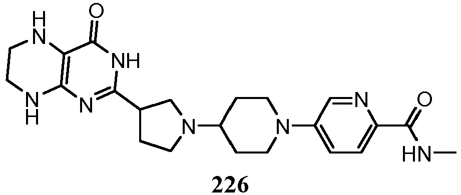

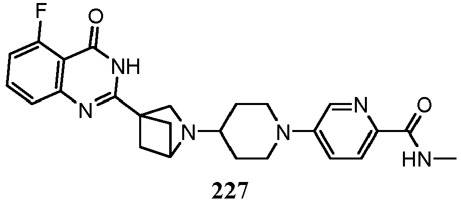

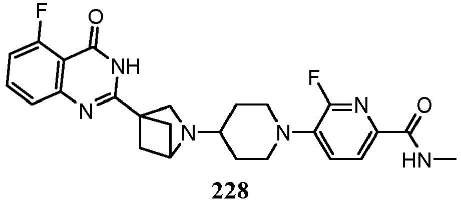

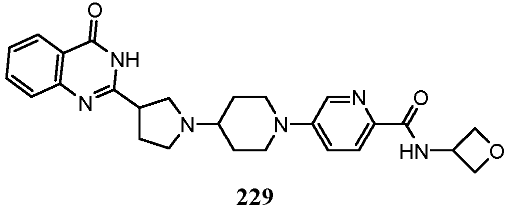

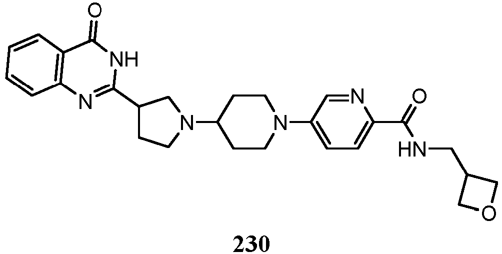

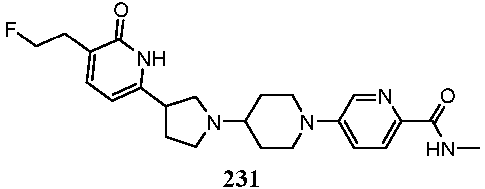

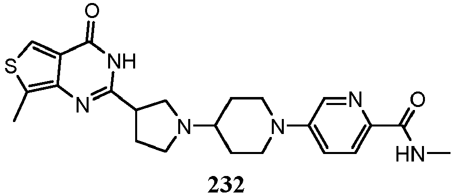

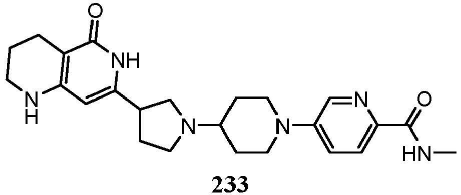

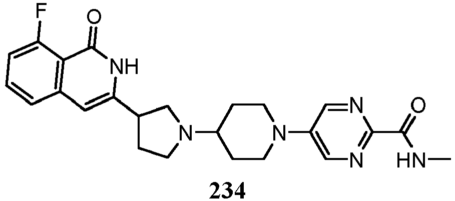

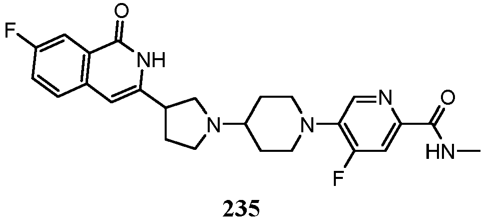

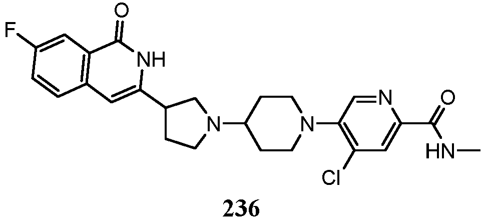

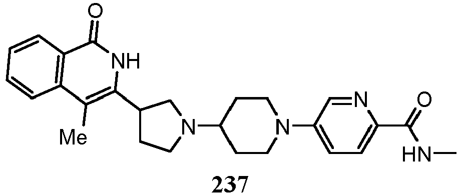

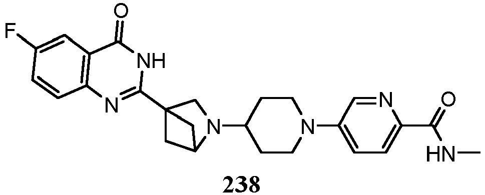

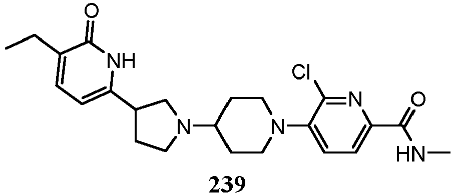

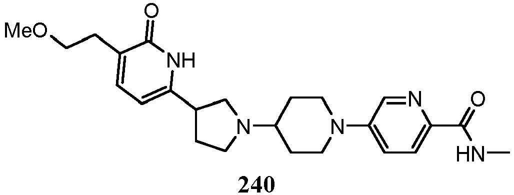

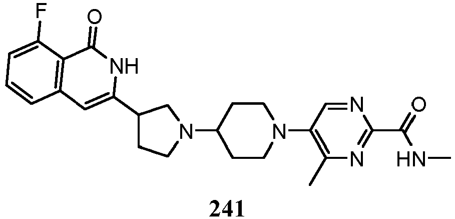

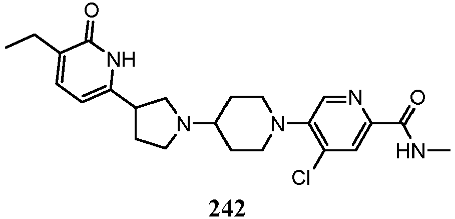

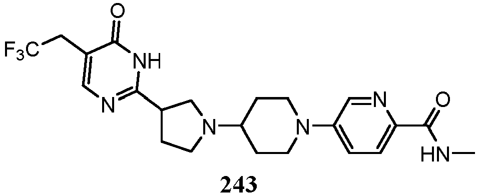

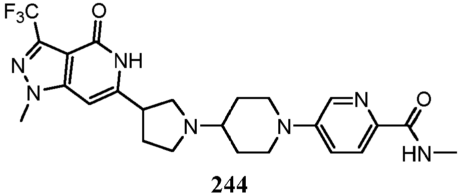

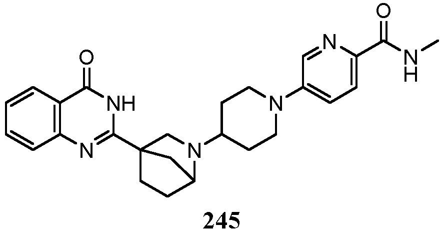

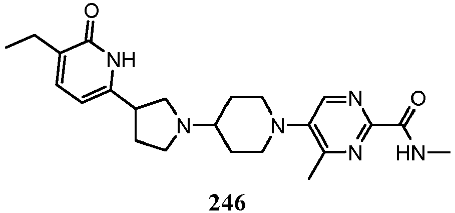

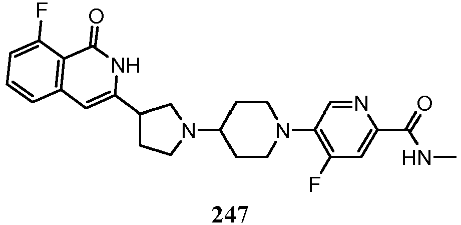

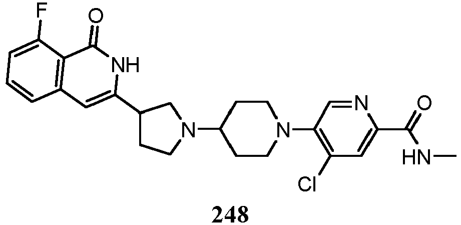

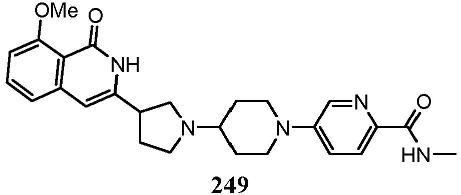

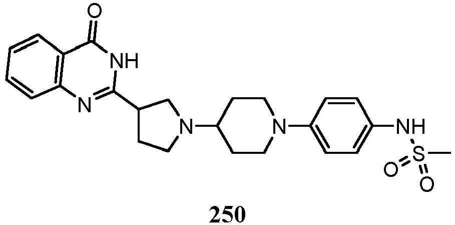

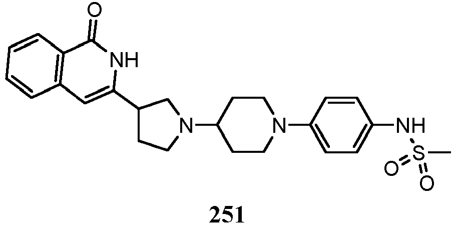

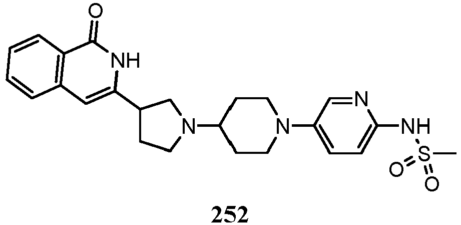

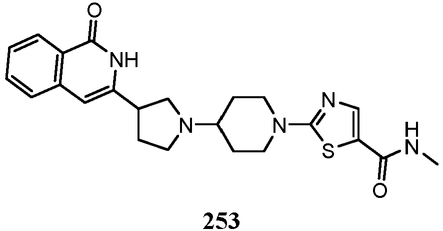

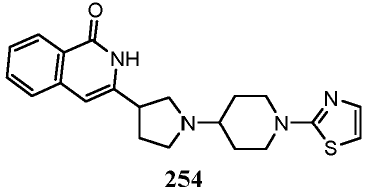

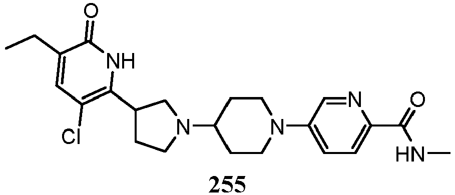

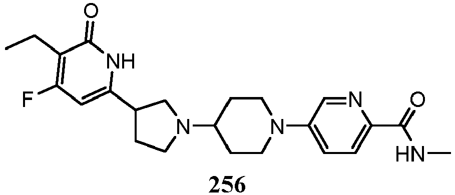

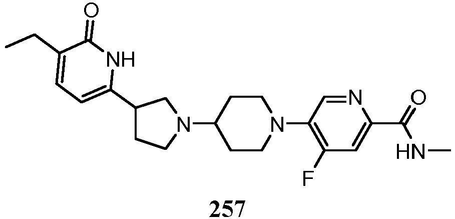

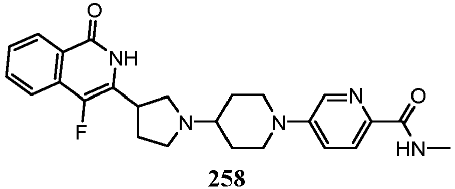

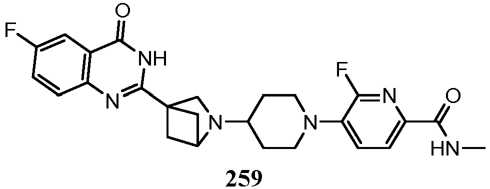

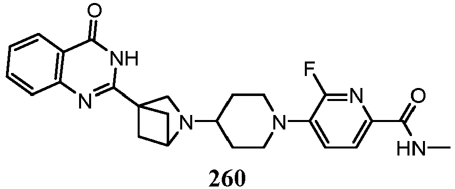

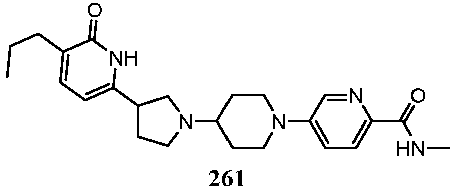

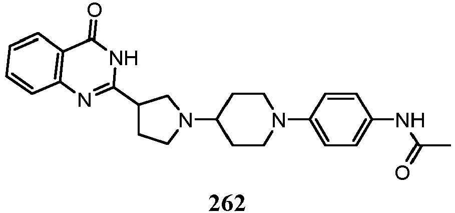

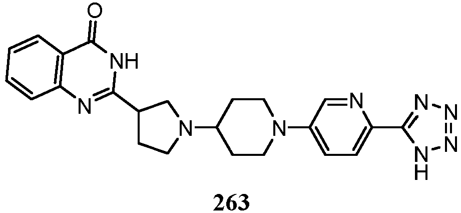

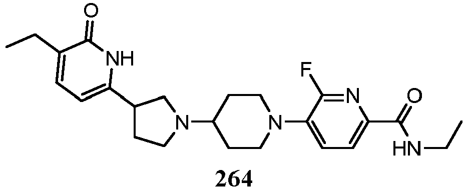

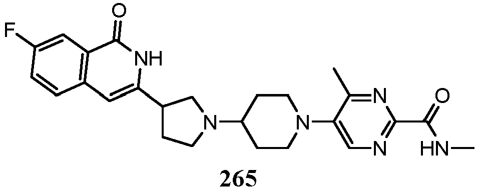

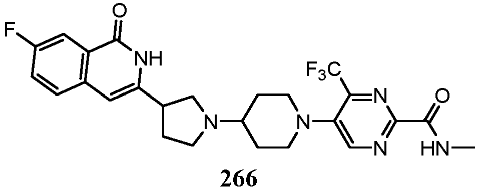

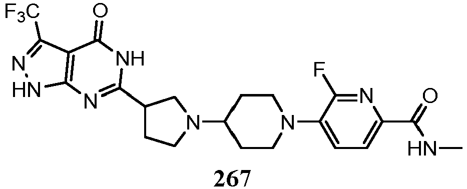

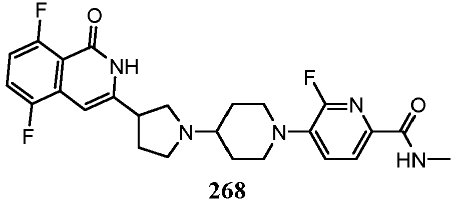

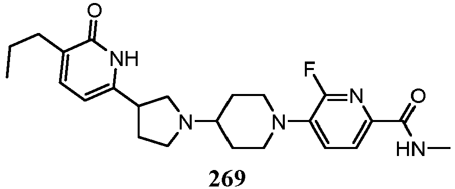

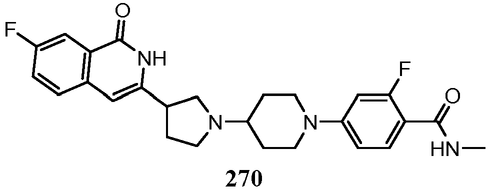

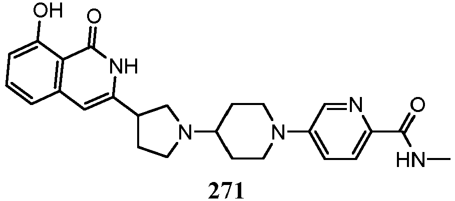

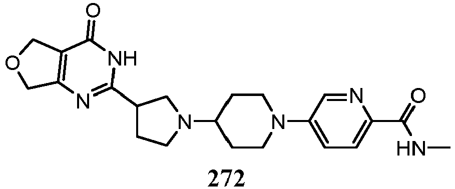

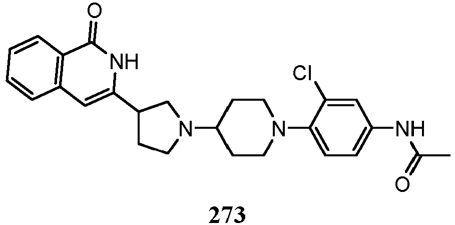

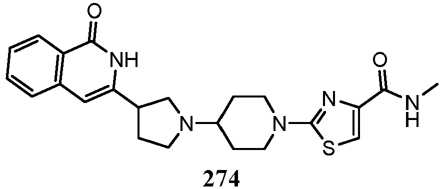

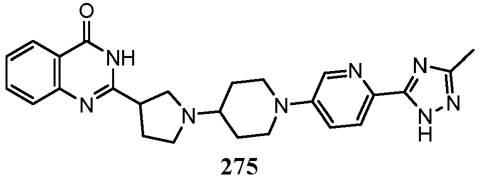

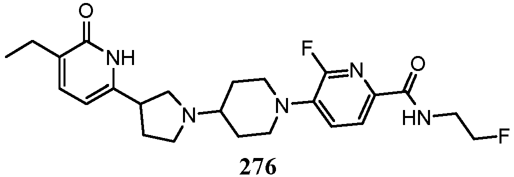

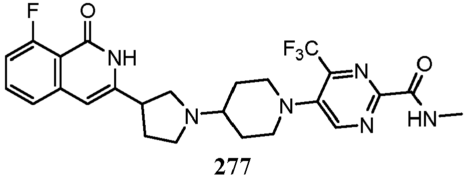

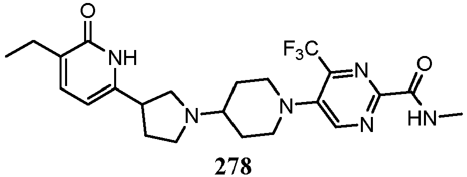

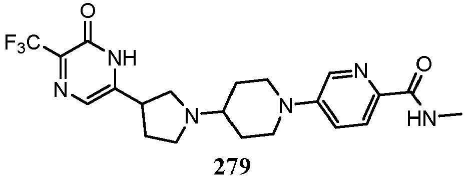

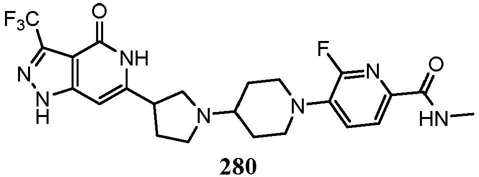

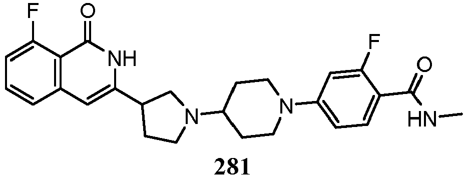

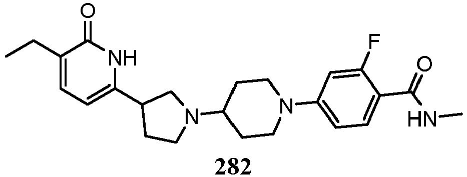

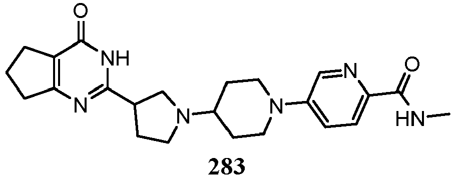

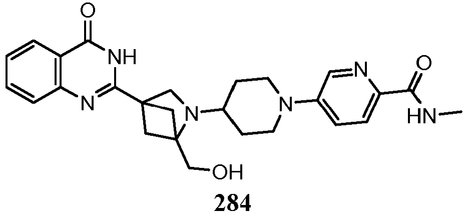

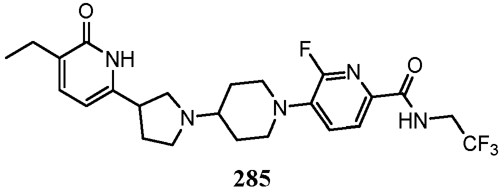

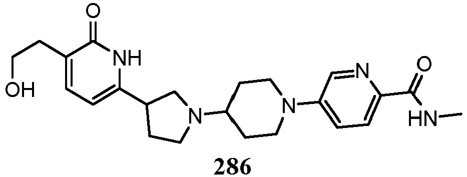

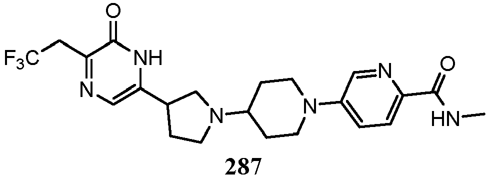

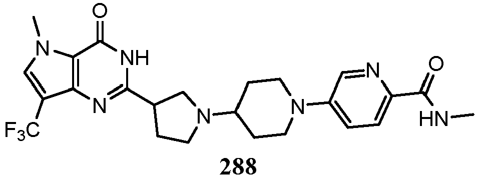

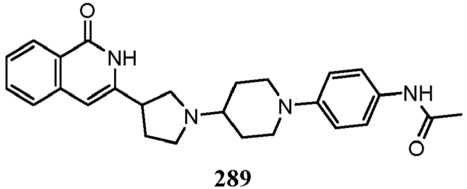

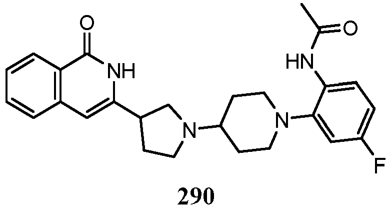

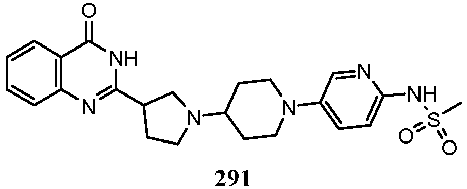

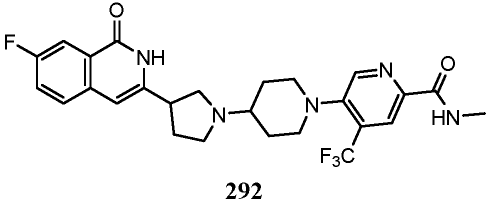

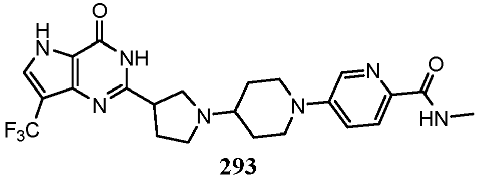

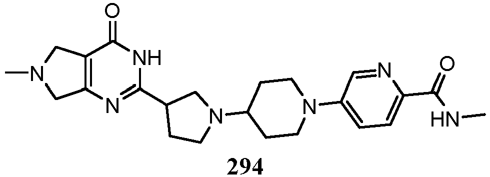

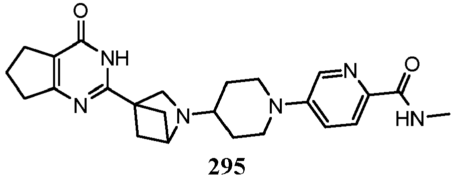

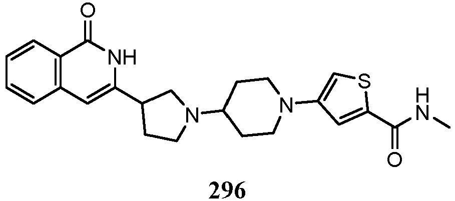

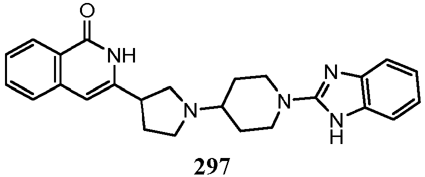

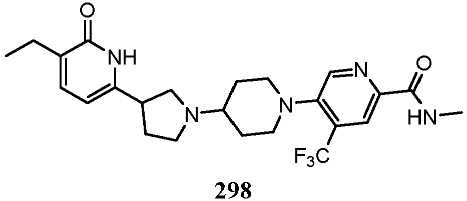

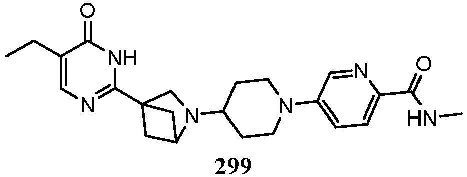

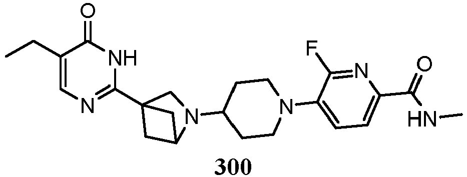

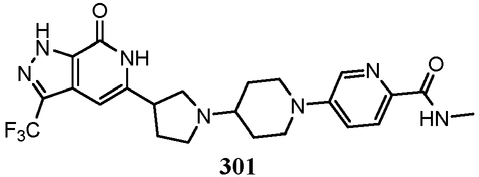

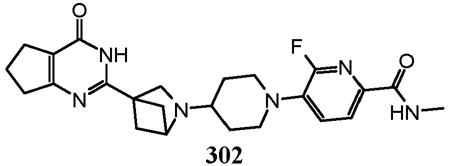

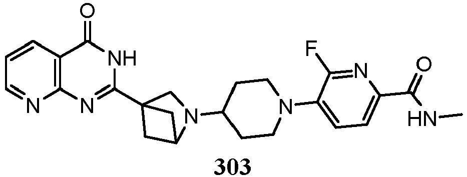

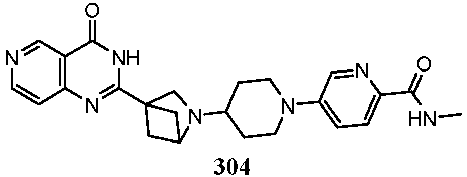

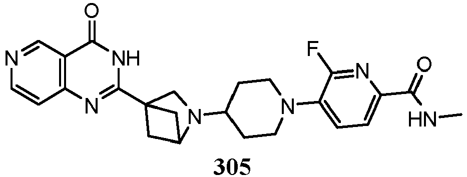

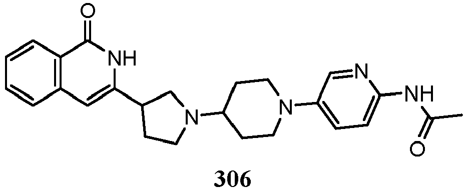

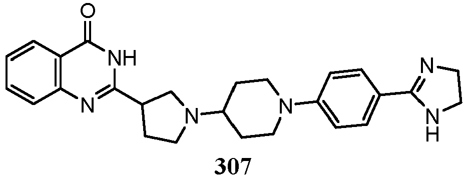

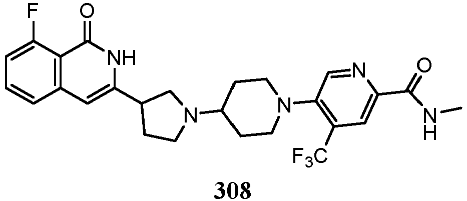

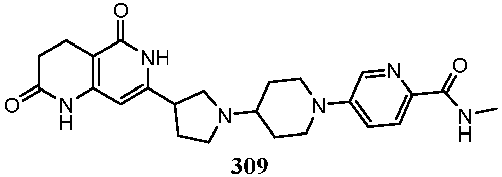

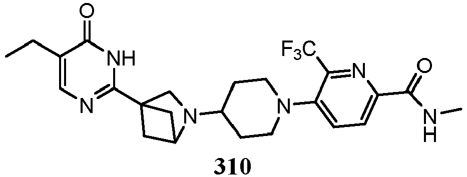

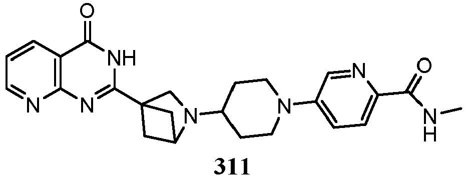

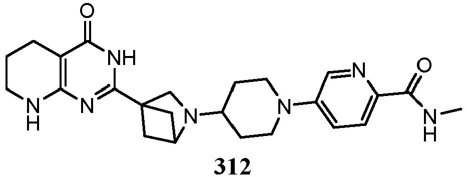

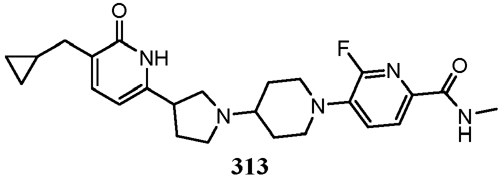

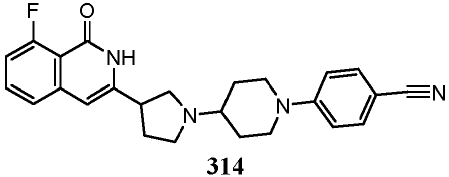

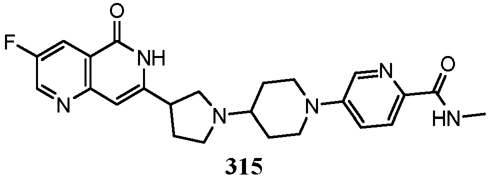

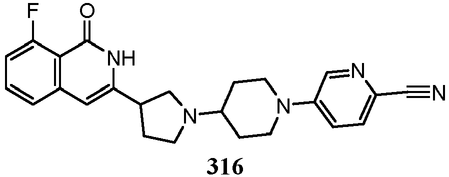

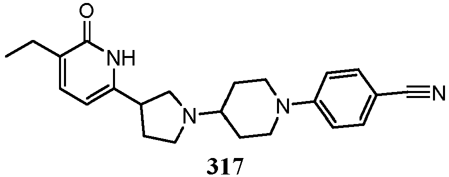

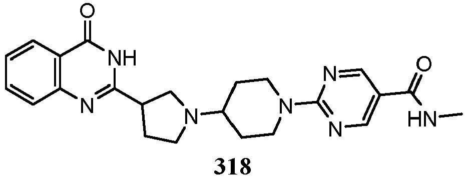

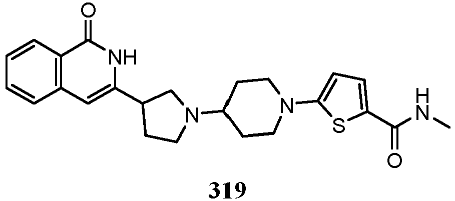

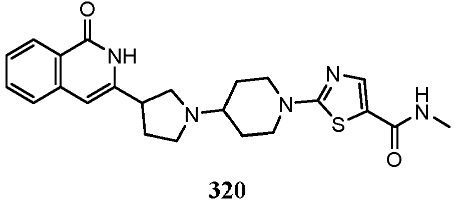

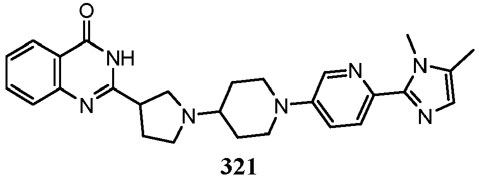

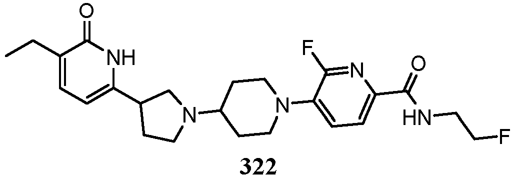

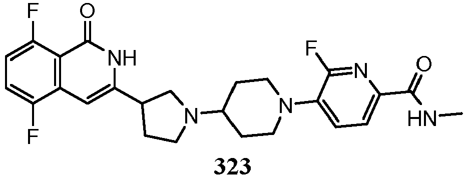

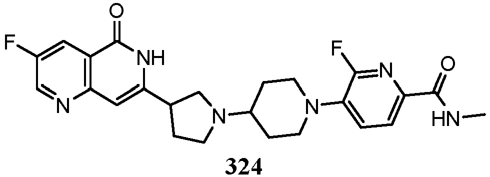

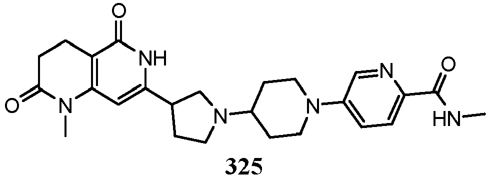

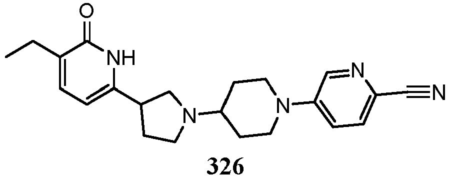

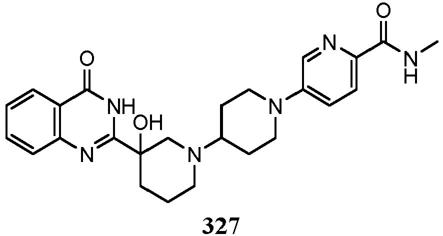

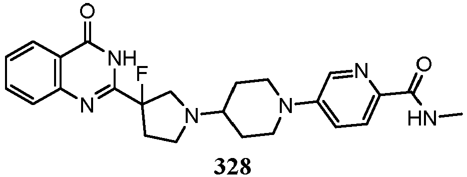

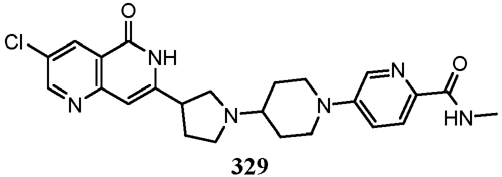

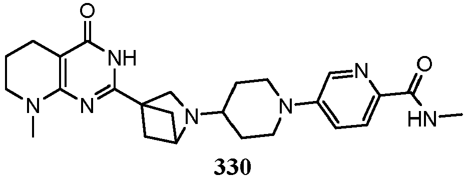

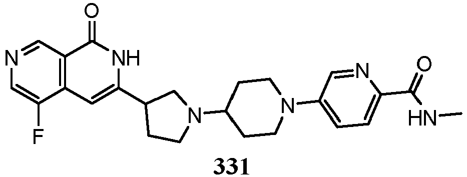

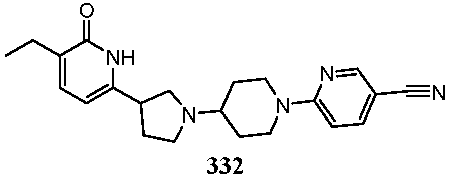

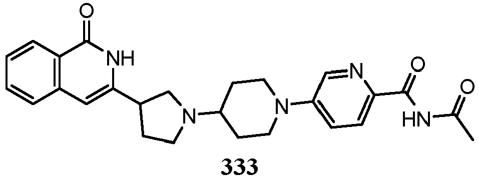

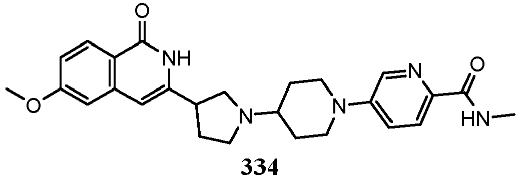

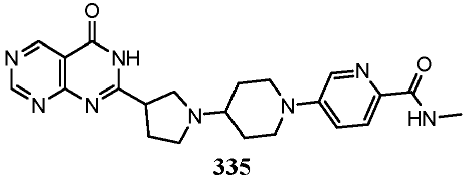

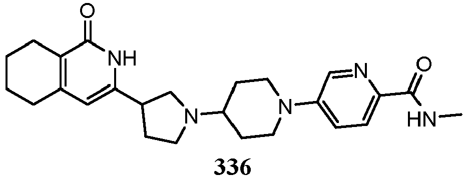

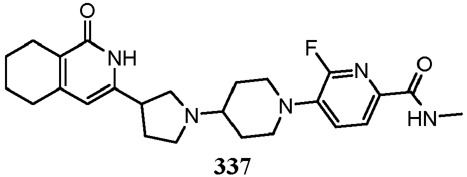

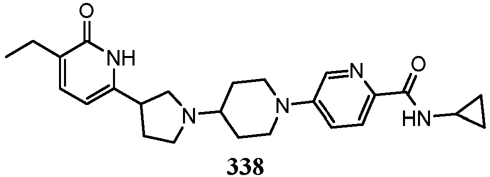

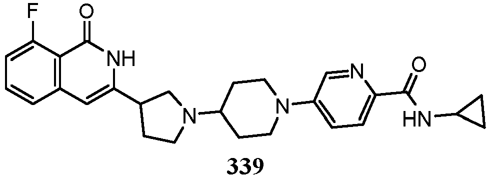

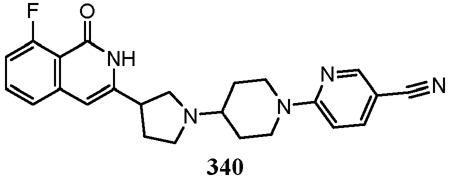

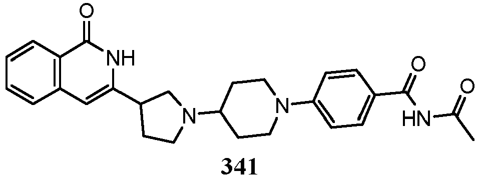

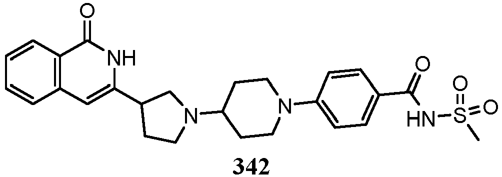

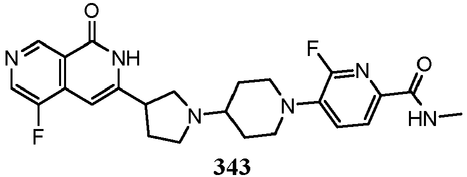

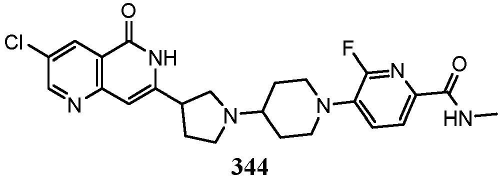

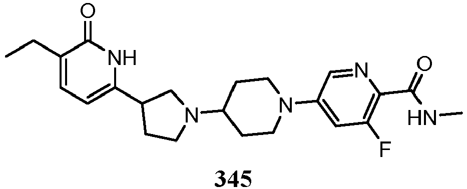

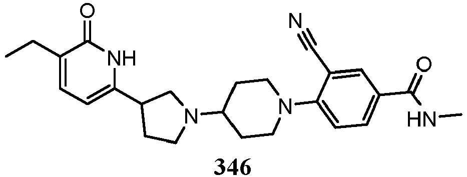

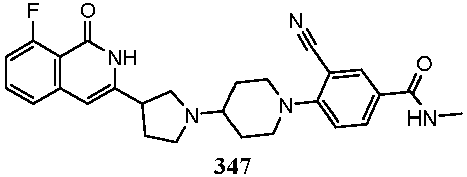

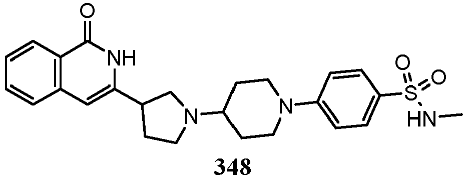

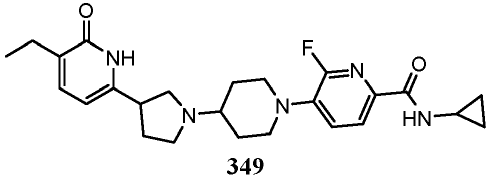

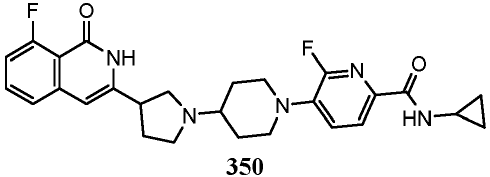

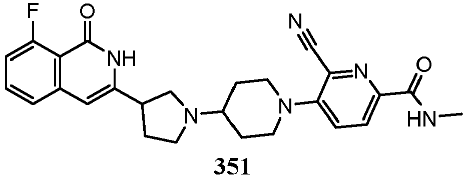

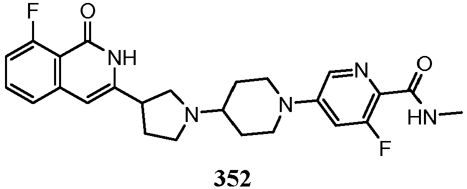

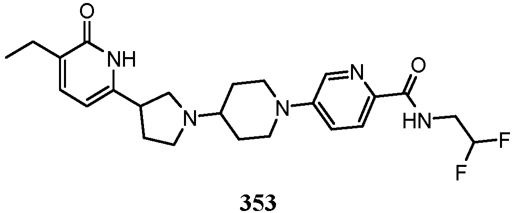

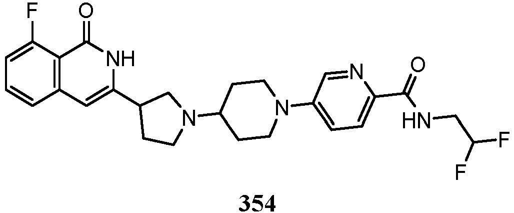

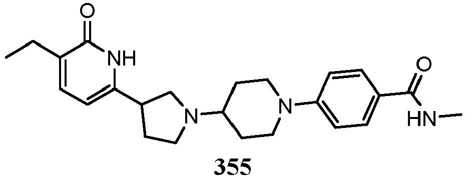

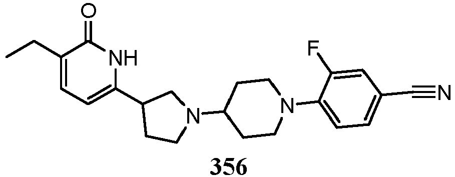

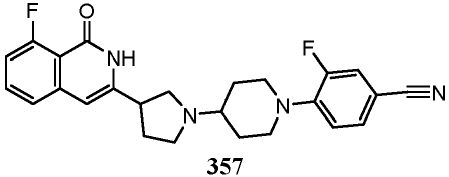

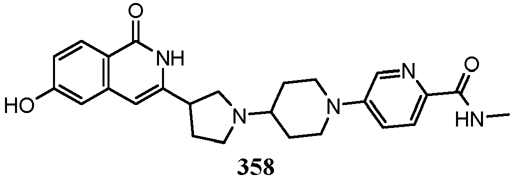

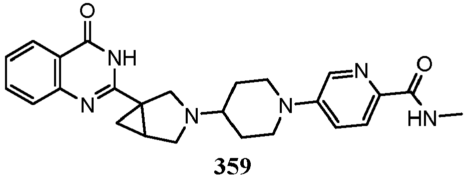

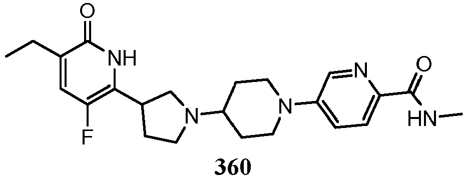

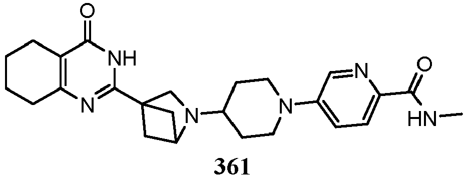

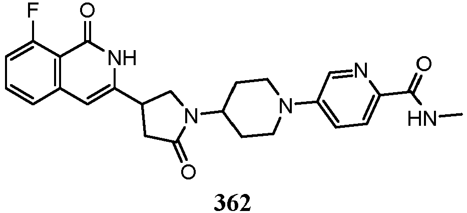

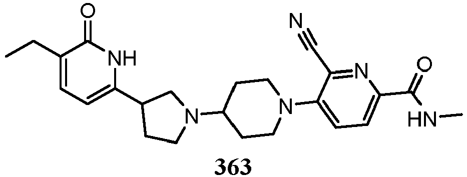

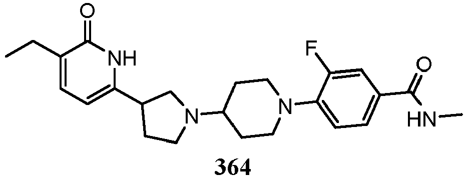

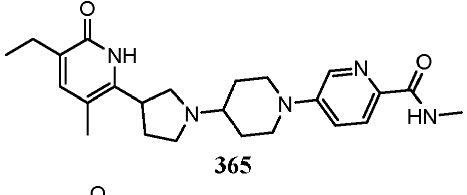

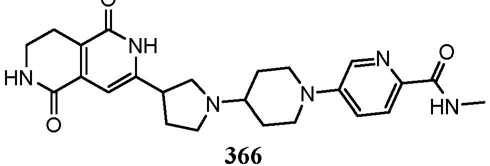

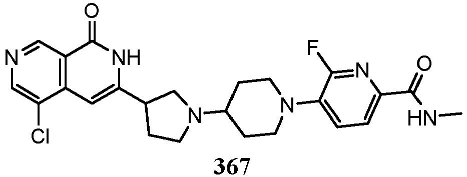

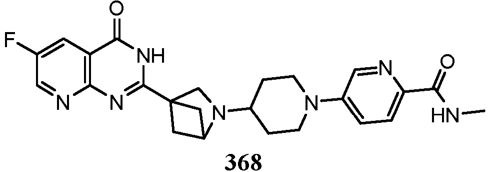

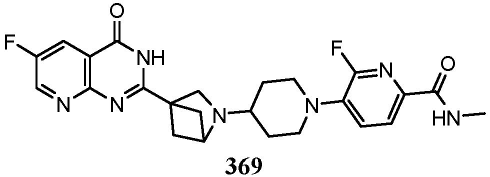

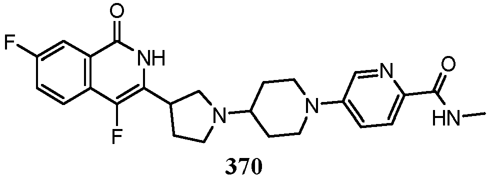

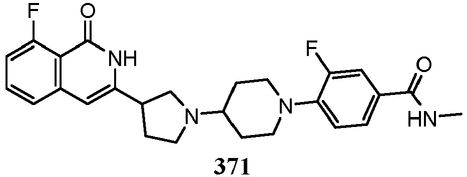

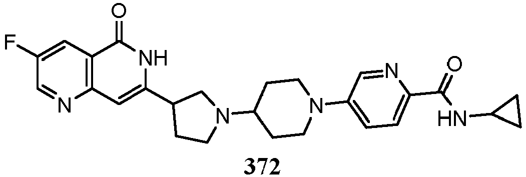

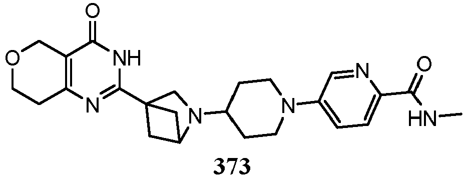

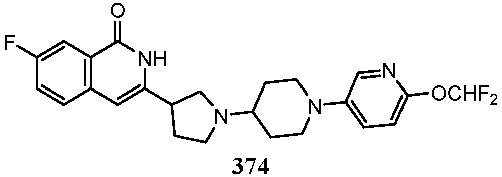

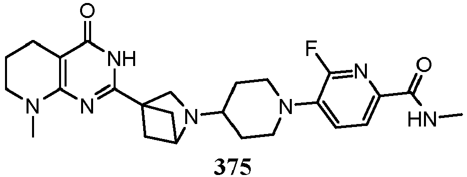

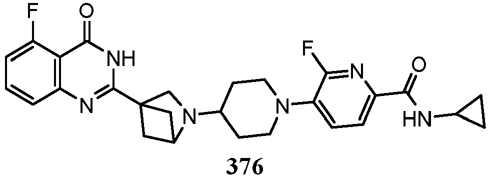

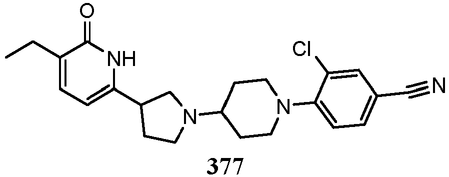

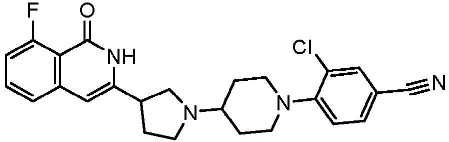

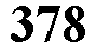

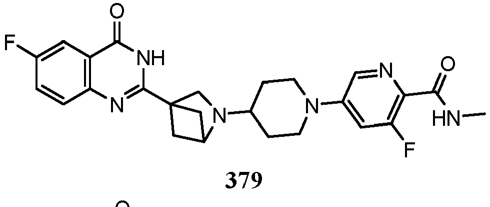

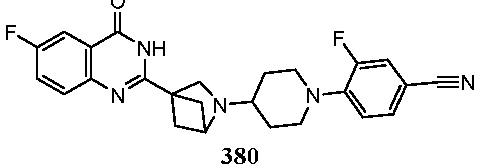

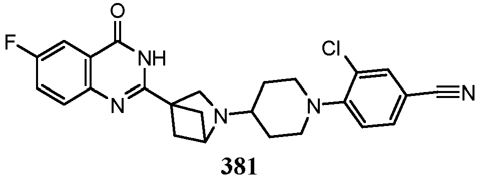

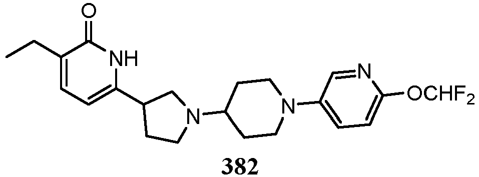

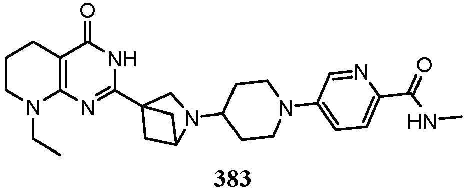

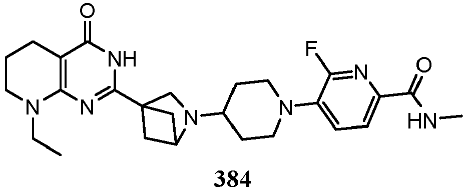

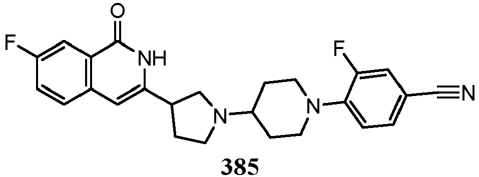

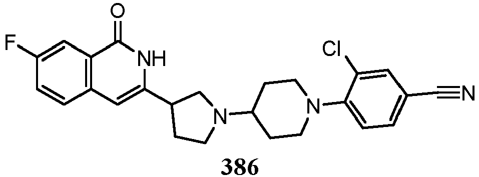

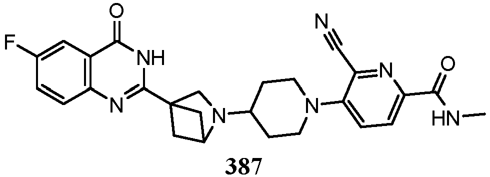

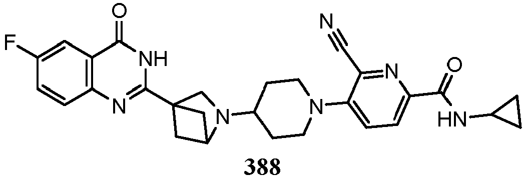

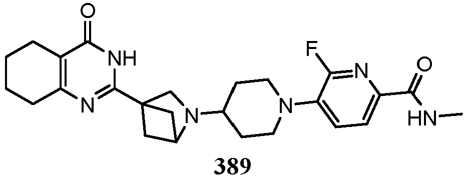

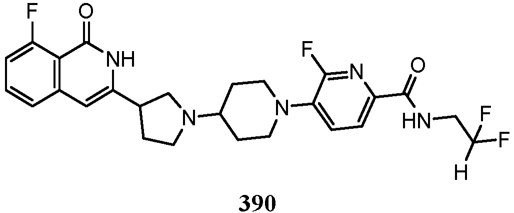

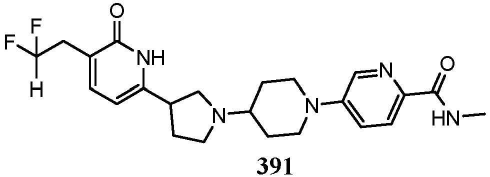

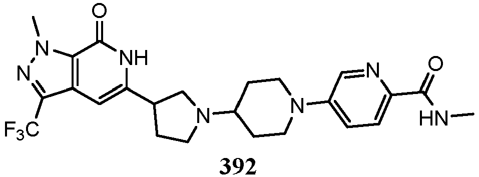

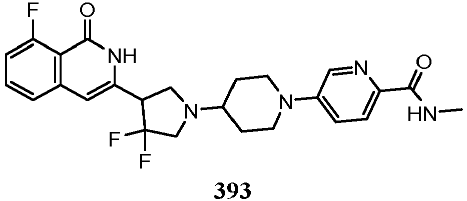

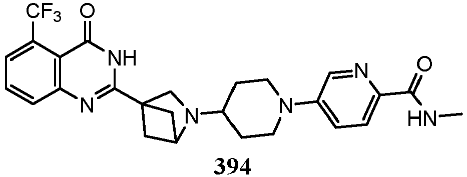

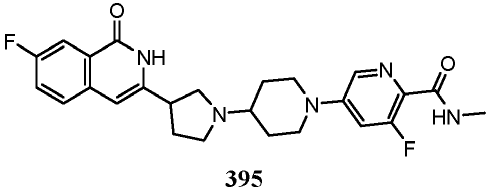

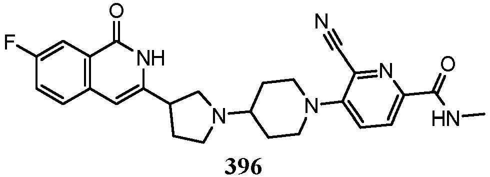

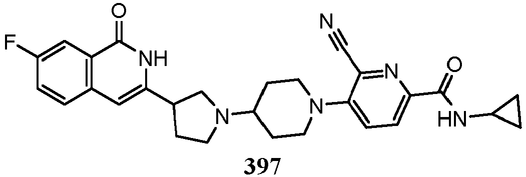

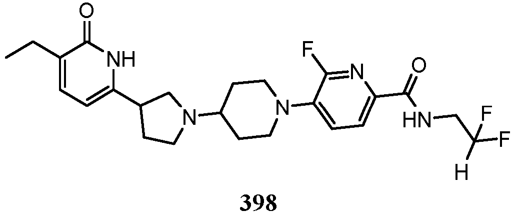

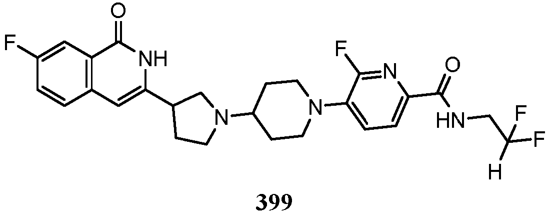

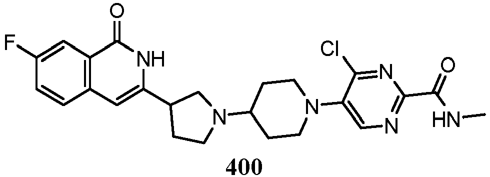

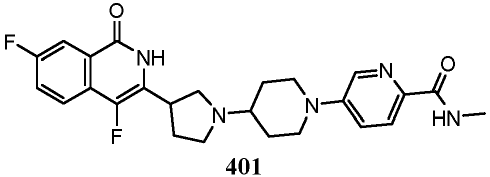

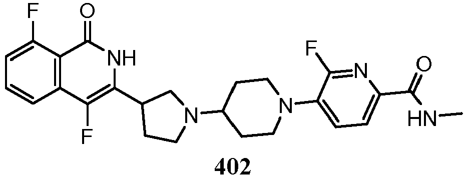

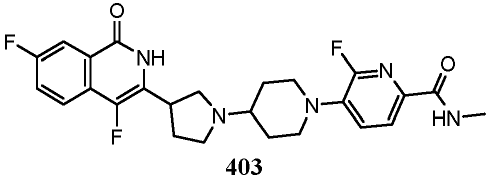

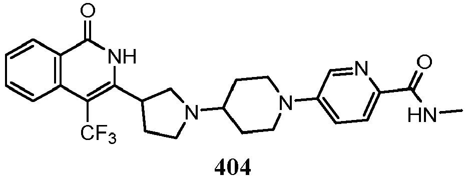

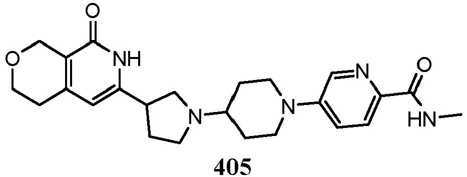

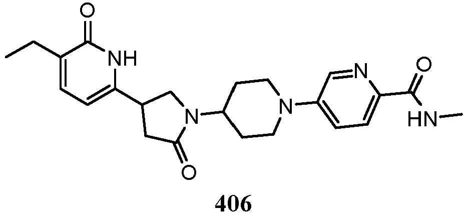

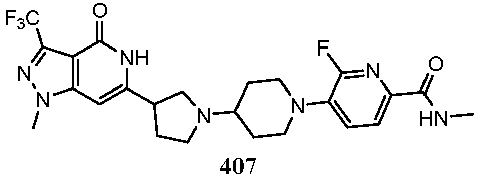

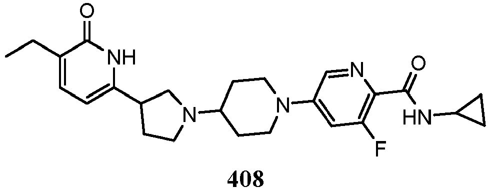

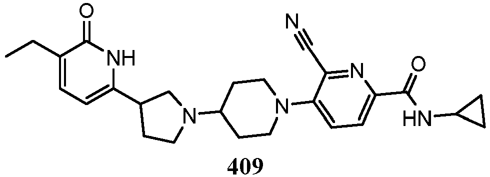

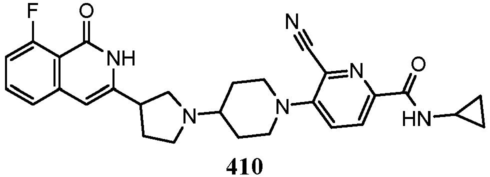

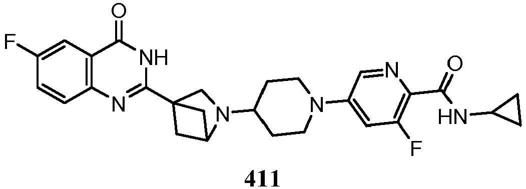

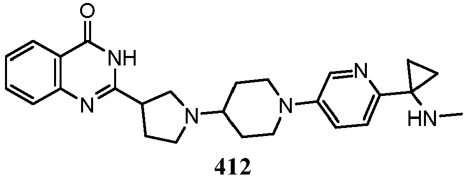

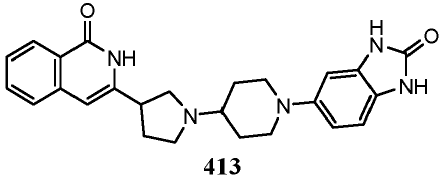

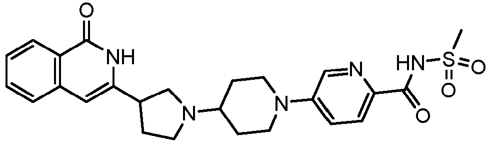

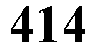

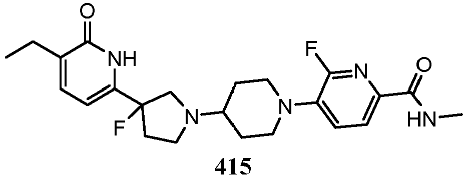

The compounds of the present disclosure have been described in detail above in terms of their structures. For the avoidance of doubt, any compounds of the present disclosure may comprise compounds or compositions in accordance with their structure as follows:
- an isolated enantiomer, or
- a mixture of two or more enantiomers, or
- a mixture of two or more diastereomers, and/or epimers, or
- a racemic mixture, or
- one or more tautomers;
of each structure.

For the above numbered compounds, compounds 1, 2, 5, 8, 12, 15, 17 to 22, 25, 27 to 30, 36, 40, 41, 45, 47, 48, 54, 59, 63, 75, 168, 173, 179, 192, 193, 227, 228, 238, 259, 260, 284, 295, 299, 300, 302-305, 310-312, 330, 361, 368, 369, 373, 375, 376, 379-381, 383, 384, 387-389, 394 and 411 are achiral. The remaining compounds represent more than one enantiomeric structure which may have PARP1 inhibitory activity as a racemic mixture and/or as a separated enantiomer(s). In the examples below, a compound with a suffix "a" (eg 10a) represents an enantiomer eluted as a first fraction when a racemic mixture of the two enantiomers is applied to a Daicel CHIRALPAK chiral chromatography column. In the examples below, a compound with a suffix "b" (eg 10b) represents an enantiomer eluted as a second fraction when a racemic mixture of the two enantiomers is applied to a Daicel CHIRALPAK chiral chromatography column. In the examples below, a compound with no suffix represents either an achiral compound or a racemic mixture of the enantiomers. In the examples below, a compound which bears a suffix "rac" represents a racemic mixture of the enantiomers.

The compounds described herein may be provided for use in medicine. In the context of the present disclosure, the medicinal use is not especially limited, provided that it is a use which is facilitated by the PARP1 inhibitory effect of the compound. Thus, the compounds of the invention may be for use in any disease, condition or disorder that may be prevented, ameliorated or treated using a PARP1 inhibitor. Typically, this comprises a disease condition and/or a disorder selected from: a cancer, it is not especially limited, provided that the cancer is one which may be treated, prevented or ameliorated by using a PARP1 inhibitor. Thus the cancer may be a cancer selected from: a solid or liquid tumour including cancer of the eye, brain (such as gliomas, glioblastomas, medullablastomas, craniopharyngioma, ependymoma, and astrocytoma), spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer, an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes (PHTS) (such as Lhermitte-Duclos disease, Cowden syndrome, Proteus syndrome, and Proteus-like syndrome), leukaemias and lymphomas (such as acute lymphoblastic leukaemia, chronic lymphocytic leukaemia, acute myelogenous leukaemia, chronic myelogenous leukaemia, hairy cell leukaemia, T-cell prolymphocytic leukemia (T-PLL), large granular lymphocytic leukemia, adult T-cell leukemia, juvenile myelomonocytic leukaemia, Hodgkin lymphoma, non-Hodgkin lymphoma, mantle lymphoma, follicular lymphoma, primary effusion lymphoma, AIDS-related lymphoma, Hodgkin lymphoma, diffuse B cell lymphoma, Burkitt lymphoma, and cutaneous T-cell lymphoma). In addition, the compounds described herein may be of use in cancers where EBV plays a contributing role such as Burkitt's lymphoma, Hodgkin's lymphoma, nasopharyngeal and gastrointestinal cancers.

The compounds described herein may be provided for use in for treating a cancer which is deficient in DNA damage response repair pathways, in particular in Homologous Recombination dependent DNA DSB DNA repair activity. Components of HR dependent DNA DSB repair pathways and other DNA damage response pathways include but are not limited to the following proteins: ATM, ATR, ERCC1, XRCC1, XRCC2, XRCC3, RAD51, RAD51L1, RAD51C, RAD51D, RAD51L3, DMC1, RAD52, RAD54L, RAD54B, RAD50, MRE11A, NBS1, BRCA1, BRCA2, FANCP (SLX4), FEN1, PALB2, PBRM1, SMARCA4, ARID1A, ARID1B, FANCD2, BLM. Other components involved in HR dependent DNA DSB repair include regulatory factors such as ESMY (Hughes-Davies, L. et al. Cell. 2003; 115: 523-535). A cancer which is deficient in HR-dependent DNA DSB repair typically becomes dependent on alternative DSB pathway repair mechanisms. Such cancers include but are not limited to cancers of the ovary, prostate, breast, lung, gastrointestine, blood and pancreas.

In some instances, the cancer cells may have a BRCA1 and/or BRCA2 deficient phenotype, i.e. they may be deficient in BRCA1 and/or 2 expression and function by means of mutation, polymorphism or epigenetic silencing in the encoding nucleic acids or by means of amplification, polymorphism, mutation in a gene encoding a regulatory factor eg, the ESMY gene which encodes a BRCA2 regulatory factor (Hughes-Davies, L. et al. Cell. 2003; 115: 523-535). Amplification of the ESMY gene is associated with breast and ovarian cancer. Carriers of mutations in the tumour suppressor BRCA1 and/or BRCA2 genes are known to have an elevated risk of developing certain cancers including ovarian, prostate and breast. Wild-type alleles of BRCA1 and/or BRCA2 are frequently lost in tumours of heterozygous carriers (Jasin, M. et al. Oncogene. 2002; 21: 8981-93) and their detection, as a means of patient selection, is well known in the art (Radice, PJ. et al. Exp. Clin. Cancer. Res. 2002; 21: 9-12; Chappnis, PO and Foulkes WO. Cancer Treat Res. 2002; 107: 29-59).

In some instances, the compounds described herein are selective PARP1 inhibitors, as defined above. Selective inhibition of PARP1 over PARP2 reduces PARP2 associated side effects including one or more haematological toxicities such as anaemia, neutropenia and thrombocytopenia. This enables treatment of cancer patients with reduced haematological side effects. This also enables higher doses of PARP1 inhibitors to be administered to patients and for such inhibitors to be administered in combination with chemotherapeutic agents.

The present disclosure also provides a pharmaceutical composition comprising a compound as defined above. Whilst the pharmaceutical composition is not especially limited, typically the composition further comprises a pharmaceutically acceptable additive and/or excipient. In the pharmaceutical composition, the compound as defined above may be present in the form described above, but may alternatively be in a form suitable for improving bioavailability, solubility, and/or activity, and/or may be in a form suitable for improving formulation. Thus, the compound may be in the form of a pharmaceutically acceptable salt, hydrate, acid, ester, or other alternative suitable form. Typically, the composition is for treating a disease, condition or disorder as defined above. In some instances, the compound may be present in the composition as a pharmaceutically acceptable salt, or other alternative form of the compound, in order to ameliorate pharmaceutical formulation.

In some instances the pharmaceutical composition is a composition for treating a cancer, further comprising a further agent for treating cancer. The further agent for treating cancer is not especially limited, provided that it affords some utility for cancer treatment. However, typically the further agent for treating cancer is selected from ionising radiation, chemotherapeutic agents such as anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, immunotherapeutic agents, hormone-deprivation therapies, proapoptotic agents and cell cycle signalling inhibitors. An immunotherapeutic agent may consist of but is not limited to an anti-tumour vaccine, an oncolytic virus, an immune stimulatory antibody such as anti-CTLA4, anti-PD1, anti-PDL-1, anti-OX40, anti-41BB, anti-CD27, anti-CD40, anti-LAG3, anti-TIM3, and anti-GITR, a pattern recognition receptor agonist such as a STING, TLR-9 or RIG-I Helicase agonist, an IDO or TDO inhibitor, a novel adjuvant, a peptide, a cytokine, a chimeric antigen receptor T cell therapy (CAR-T), a small molecule immune modulator, tumour microenvironment modulators, and anti-angiogenic agents.

The present disclosure further provides a pharmaceutical kit for treating a cancer, which pharmaceutical kit comprises:
(a) a compound as defined above; and
(b) a further agent for treating cancer; preferably wherein the further agent for treating cancer is selected from ionising radiation, chemotherapeutic agents such as anti-microtubule agents, platinum coordination complexes, alkylating agents, antibiotic agents, topoisomerase II inhibitors, antimetabolites, topoisomerase I inhibitors, senolytic agents, hormones and hormone analogues, hormone-deprivation therapies, signal transduction pathway inhibitors, other DNA damage repair pathway inhibitors, non-receptor tyrosine kinase angiogenesis inhibitors, antibody-drug conjugates, immunotherapeutic agents, proapoptotic agents and cell cycle signalling inhibitors;
wherein the compound and the further agent are suitable for administration simultaneously, sequentially or separately.

Further provided is a method of treating a disease and/or a condition and/or a disorder, which method comprises administering to a patient (or subject) a compound, or a composition, or a kit as defined above. The method is typically a method for treating any disease condition or disorder mentioned herein. In typical implementations, the method is a method for treating a cancer. Preferably such a method comprises administering to a patient (or subject) a compound or a composition as defined above and a further agent for treating cancer as defined above. The compound or composition and the further agent may be administered simultaneously, sequentially or separately, depending upon the agents and patients involved, and the type of cancer indicated.

Typically, the patient (or subject) is an animal, typically a mammal, including canines, equines and felines, and more typically a human.

Further provided by the present disclosure is a method of synthesis of a compound as defined above, which method comprises conducting a reaction between (i) a first reactant comprising ring E bearing a portion of substituent group L and (ii) a second reactant comprising the remainder of substituent group L, so as to form the PARP1 inhibitor compound.

Typically, in one method of synthesis, the first reactant comprises ring E and ring A, and the second reactant comprises a Q1 or Q2 precursor bearing a reactive group, which method comprises joining the N atom of ring A to the Q1 or Q2 precursor. In this method, the reactive group of the Q1 or Q2 precursor may comprise a carbonyl group, an alkyl halide or an alkyl sulfonate. Typically, the reaction comprises alkylation, reductive amination or amide formation so as to form group L.

Typically, in another method of synthesis, the first reactant comprises ring E, ring A, and at least one of Q1 and Q2, and the second reactant comprises a ring C derivative bearing a leaving group such as a halide or sulfonate. In this method, the reaction comprises a nucleophilic substitution reaction, such as a nucleophilic aromatic substitution reaction, so as to form group L.

The skilled person may select the reaction conditions in these methods with reference to known synthesis techniques depending on the appropriate starting materials. In some implementations, the method comprises one or more additional steps. Exemplary synthesis methodology is shown in the Examples herein

Typically, the above formulae (and all formulae herein) are shown in non-stereoisomeric form. For the avoidance of doubt, throughout the present disclosure a single formula is intended to represent all possible stereoisomers of a particular structure, including all possible isolated enantiomers corresponding to the formula, all possible mixtures of enantiomers corresponding to the formula, all possible mixtures of diastereomers corresponding to the formula, all possible mixtures of epimers corresponding to the formula and all possible racemic mixtures corresponding to the formula. In addition to this, the above formulae (and all formulae herein) are intended to represent all tautomeric forms equivalent to the corresponding formula.

The term "comprises" as used throughout the description and claims herein means "includes or consists of". The term denotes the inclusion of at least the features following the term and does not exclude the inclusion of other features which have not been explicitly mentioned. The term may also denote an entity which consists only of the features following the term.

The invention will now be described in more detail, by way of example only, with reference to the following specific embodiments.

### EXAMPLES

### Exemplary syntheses of compounds of the disclosure

The compounds of the present disclosure may be synthesised using readily available starting materials and known reactions. Exemplary syntheses of three compounds are shown below:

### Example 1 - Synthesis of Compound 42 (a reference example, not in accordance with the present claims)

### INT-4 Synthesis

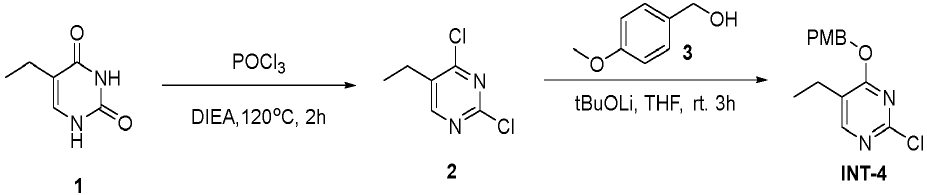

### Preparation of 2,4-dichloro-5-ethylpyrimidine (2)

To a solution of 5-ethyl-1,3-dihydropyrimidine-2,4-dione **1** (10.00 g, 0.07 mol) in POCl₃ (55.00 g, 0.36 mol) at 0 °C and added DIEA (23.00 g, 0.18 mol). Then the mixture was stirred for 2 h at 120 °C. The hot reaction mixture was poured into ice water and the aqueous layer was extracted with ethyl acetate (50 mL × 3). The combined organic layers were dried over Na₂SO₄. The reaction mixture was concentrated under reduced pressure. The residue was purified by flash chromatography (eluting with PE/EA = 100 : 0 to 92 : 8) to give 2,4-dichloro-5-ethylpyrimidine **2** (4.80 g, 38 % yield ) as a white solid.

LCMS (ESI) calcd for C₆H₆Cl₂N₂ [M + H] ⁺ m/z 176.99, found 177.00

### Preparation of 2-chloro-5-ethyl-4-(2-methoxy-5-methylphenoxy)pyrimidine (INT-4)

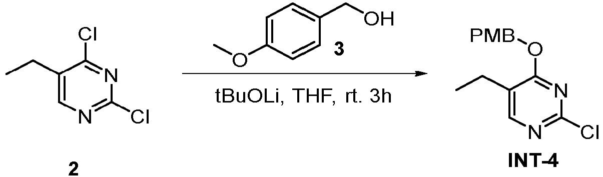

To a solution of (4-methoxyphenyl) methanol **3** (4.54 g, 32.90 mmol) in THF (20 mL) was added tBuOLi (2.30 g, 28.75 mmol) at 70 °C for 15 min. Then 2,4-dichloro-5-ethylpyrimidine **2** (4.80 g, 27.27 mmol) was added to the mixture at 0 °C. The mixture was stirred for 3 h at room temperature. The reaction mixture was concentrated under reduced pressure. The residue was purified by flash chromatography (eluting with DCM/MeOH = 100 : 0 to 93 : 7) to give 2-chloro-5-ethyl-4-(2-methoxy-5-methylphenoxy)pyrimidine **INT-4** (4.00 g, 53 % yield ) as a white solid.

LCMS (ESI) calcd for C₁₄H₁₅ClN₂O₂ [M + H] ⁺ m/z 279.08, found 279.15.

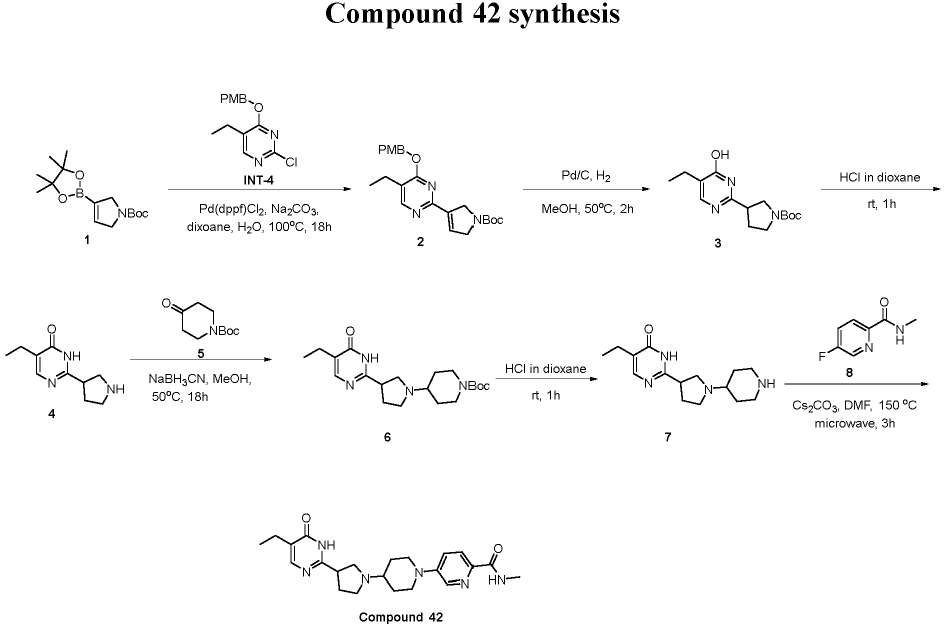

### Preparation of tert-butyl 3-(5-ethyl-4-((4-methoxybenzyl)oxy)pyrimidin-2-yl)-2,5-dihydro-1H-pyrrole-1-carboxylate (2)

To a solution tert-butyl 3-(4,4,5,5-tetramethyl-1,3,2-dioxaborolan-2-yl)-2,5-dihydropyrrole-1-carboxylate 1 (500 mg, 1.69 mmol) in 1,4-dioxane/H₂O=4:1 (15 mL) was added 2-chloro-5-ethyl-4-(2-methoxy-5-methylphenoxy)pyrimidine **INT-4** (377.72 mg, 1.76 mmol) and Na₂CO₃ (189.11 mg, 3.01 mmol), then Pd(dppf)Cl₂ (247.89 mg, 0.34 mmol) was added at room temperature. The reaction mixture was refluxed under nitrogen at 100 °C and stirred for 18 h. After cooling to room temperature, the reaction mixture was concentrated under reduced pressure. The residue was purified by flash chromatography (eluting with petroleum ether/ethyl acetate = 100 : 0 to 85 : 15) to give tert-butyl 3-(5-ethyl-4-((4-methoxybenzyl)oxy)pyrimidin-2-yl)-2,5-dihydro-1H-pyrrole-1-carboxylate 2 (250 mg, 31 % yield ) as a colorless oil.

LCMS (ESI) calcd for C₂₃H₂₉N₃O₄ [M + H] ⁺ m/z 412.22, found 412.28.

### Preparation of tert-butyl 3-(5-ethyl-4-hydroxypyrimidin-2-yl)pyrrolidine-1-carboxylate (3)

To a solution 3-(5-ethyl-4-((4-methoxybenzyl)oxy)pyrimidin-2-yl)-2,5-dihydro-1H-pyrrole-1-carboxylate **2** (250 mg, 0.61 mmol) in MeOH (15 mL) was added Pd/C (97 mg, 0.91 mmol) at room temperature. The reaction mixture was stirred for 2h at 50 °C under hydrogen. After cooling to room temperature, the mixture was filtered through a Celite pad and the filtrate was concentrated to give tert-butyl 3-(5-ethyl-4-hydroxypyrimidin-2-yl)pyrrolidine-1-carboxylate 3 (150 mg, 80 % yield ) as a colourless oil.

LCMS (ESI) calcd for C₁₅H₂₃N₃O₃ [M + H] ⁺ m/z 294.17, found 294.19.

### Preparation of 5-ethyl-2-(pyrrolidin-3-yl)-3H-pyrimidin-4-one (4)

To a solution 3-(5-ethyl-4-hydroxypyrimidin-2-yl)pyrrolidine-1-carboxylate **3** (150 mg, 0.51 mmol) was added HCl in dioxane (4 M, 10 mL) at room temperature. The reaction mixture was stirred for 1 h at room temperature. The reaction mixture was concentrated under reduced pressure to give 5-ethyl-2-(pyrrolidin-3-yl)-3H-pyrimidin-4-one **4** (100 mg, 94 % yield) as a white solid.

LCMS (ESI) calcd for C₁₀H₁₃N₃O [M + H] ⁺ m/z 194.12, found 194.19.

### Preparation of tert-butyl 4-[3-(5-ethyl-4-oxo-3H-pyrimidin-2-yl)pyrrolidin-1-yl]piperidine-1-carboxylate (6)

To a solution 5-ethyl-2-(pyrrolidin-3-yl)-3H-pyrimidin-4-one **4** (100 mg, 0.52 mmol) in MeOH (15 mL) was added tert-butyl 4-oxopiperidine-1-carboxylate **5** (124.48 mg, 0.61 mmol) and NaBH₃CN (145 mg, 3.11 mmol). The reaction mixture was refluxed under nitrogen at 50 °C and stirred for 18 h. After cooling to room temperature, the reaction mixture was concentrated under reduced pressure. The residue was purified by flash chromatography (eluting with DCM/MeOH = 100 : 0 to 95 : 5) to give tert-butyl 4-[3-(5-ethyl-4-oxo-3H-pyrimidin-2-yl)pyrrolidin-1-yl]piperidine-1-carboxylate **6** (120 mg, 55 % yield ) as a white solid.

LCMS (ESI) calcd for C₂₀H₃₂N₄O₃ [M + H] ⁺ m/z 377.25, found 377.30.

### Preparation of 5-ethyl-2-[1-(piperidin-4-yl)pyrrolidin-3-yl]-3H-pyrimidin-4-one (7)

Tert-butyl 4-[3-(5-ethyl-4-oxo-3H-pyrimidin-2-yl)pyrrolidin-1-yl]piperidine-1-carboxylate **6** (120 mg, 0.32 mmol) was added to HCl in dioxane (4 M, 10 mL) at room temperature. The reaction mixture was stirred for 1 h at room temperature, then concentrated under reduced pressure to give 5-ethyl-2-[1-(piperidin-4-yl)pyrrolidin-3-yl]-3H-pyrimidin-4-one **7** (90 mg, 92 % yield) as a white solid.

LCMS (ESI) calcd for C₁₅H₂₄N₄O [M + H] ⁺ m/z 277.20, found 277.24.

### Preparation of 5-{4-[3-(5-ethyl-4-oxo-3H-pyrimidin-2-yl)pyrrolidin-1-yl]piperidin-1-yl}-N-methylpyridine-2-carboxamide (Compound 42)

To a solution 5-ethyl-2-[1-(piperidin-4-yl)pyrrolidin-3-yl]-3H-pyrimidin-4-one **7** (90 mg, 0.33 mmol) in DMF (10 mL) was added 5-fluoro-N-methylpyridine-2-carboxamide **8** (104 mg, 0.68 mmol) and Cs₂CO₃ (884 mg, 2.71 mmol). The reaction mixture was irradiated in a microwave reactor at 150 °C for 3 h. The reaction mixture was concentrated under reduced pressure. The residue was purified by flash chromatography (eluting with DCM/MeOH = 100 : 0 to 95 : 5) to give 5-{4-[3-(5-ethyl-4-oxo-3H-pyrimidin-2-yl)pyrrolidin-1-yl]piperidin-1-yl}-N-methylpyridine-2-carboxamide **Compound 42** (20 mg, 95 % purity, 20 % yield ) as a white solid.

¹H NMR (400 MHz, DMSO) δ 12.16 (s, 1 H), 8.42-8.38 (m, 1 H), 8.28 (d, *J =* 2.4 Hz, 1 H), 7.83 (d, *J=* 8.8 Hz, 1 H), 7.73 (s, 1 H), 7.42-7.39 (dd, *J= 8.8,* 2.8 Hz, 1 H), 3.87 (d, *J =* 12.8 Hz, 2 H), 3.29-3.25 (m, 2 H), 3.17-3.13 (m, 1 H), 2.94-2.74 (m, 8 H), 2.34 (q, *J=* 7.2 Hz, 2 H), 2.18-1.94 (m, 4 H), 1.56-1.47 (m, 2 H), 1.08 (t, *J =* 7.6 Hz, 3 H).

LCMS (ESI) calcd for C₂₂H₃₀N₆O₂ [M + H] ⁺ m/z 411.24, found 411.33.

### Example 2 - Synthesis of Compound 53 (a reference example, not in accordance with the present claims)

### Synthesis of intermediate INT:

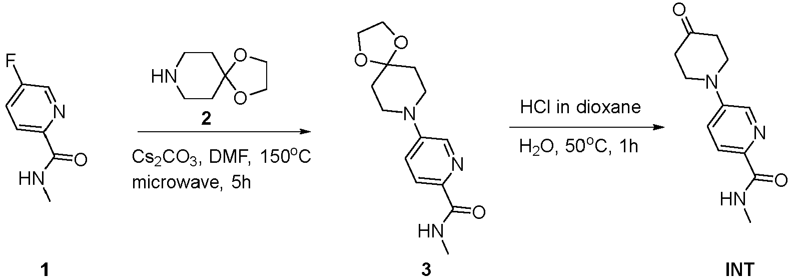

### Preparation of 5-{1,4-dioxa-8-azaspiro[4.5]decan-8-yl}-N-methylpyridine-2-carboxamide (3)

To a solution of 5-fluoro-N-methylpyridine-2-carboxamide **1** (1.00 g, 6.50 mmol) in DMF (15 mL) was added 1,4-dioxa-8-azaspiro[4.5]decane **2** (1.40 g, 9.75 mmol), then Cs₂CO₃ (2.12 g, 6.50 mmol) was added at room temperature. The reaction mixture was stirred at 150 °C using microwave for 5 h. After cooling to room temperature, the reaction mixture was concentrated under reduced pressure. The residue was purified by flash chromatography (eluting with DCM/MeOH = 100 : 0 to 97 : 3) to give 5-{1,4-dioxa-8-azaspiro[4.5]decan-8-yl}-N-methylpyridine-2-carboxamide **3** (1.50 g, 76 % yield ) as a white solid.

LCMS (ESI) calcd for C₁₄H₁₉N₃O₃ [M + H] ⁺ m/z 278.14, found 278.14.

### Preparation of N-methyl-5-(4-oxopiperidin-1-yl)picolinamide (INT)

To a solution of 5-{1,4-dioxa-8-azaspiro[4.5]decan-8-yl}-N-methylpyridine-2-carboxamide **3** (1.50 g, 5.40 mmol) in H₂O (10 mL) was added HCl in 1,4-dioxane (4 M, 20 mL) at room temperature. The reaction mixture was stirred at 50 °C for 1 h. After cooling to room temperature, the reaction mixture was concentrated under reduced pressure. The residue was adjusted pH > 7 using NaHCO₃ solution, then extracted with EtOAc (50 mL x 3), the organic phase dried by Na₂SO₄ and concentrated to give N-methyl-5-(4-oxopiperidin-1-yl)picolinamide **INT** (1.50 g, 76 % yield ) as a yellow solid.

LCMS (ESI) calcd for C₁₂H₁₅N₃O₂ [M + H] ⁺ m/z 234.12, found 234.18.

### Synthesis of Compound 53

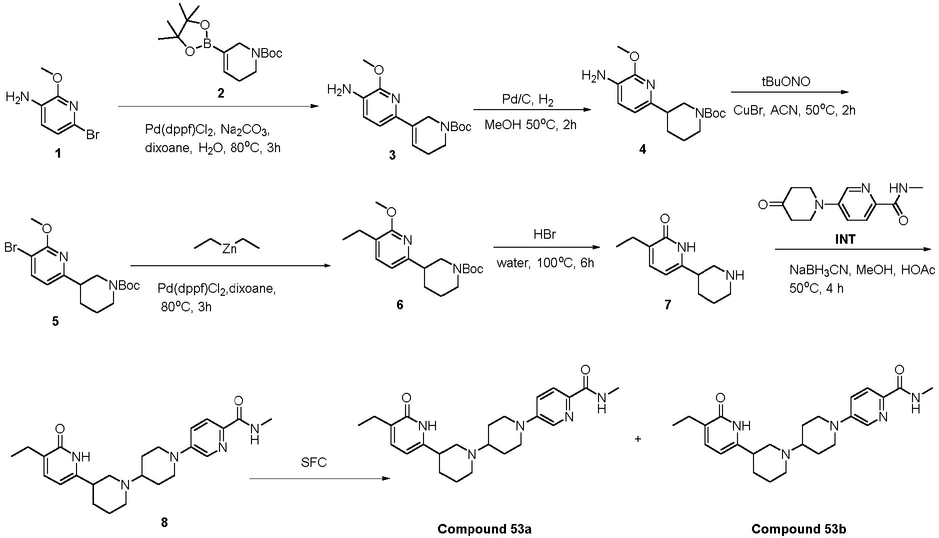

### Preparation of tert-butyl 3-(5-amino-6-methoxypyridin-2-yl)-5,6-dihydro-2H-pyridine-1-carboxylate (3)

To a solution of 6-bromo-2-methoxypyridin-3-amine 1 (2.00 g, 9.90 mmol) in 1,4-dioxane/H2O=4:1 (45 mL) was added tert-butyl 3-(4,4,5,5-tetramethyl-1,3,2-dioxaborolan-2-yl)-5,6-dihydro-2H-pyridine-1-carboxylate **2** (3.98 g, 12.87 mmol), then Pd(dppf)Cl₂ (0.72 g, 0.99 mmol) and Na₂CO₃ (3.12 g, 99.00 mmol) were added at room temperature. The reaction mixture was stirred under nitrogen at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-amino-6-methoxypyridin-2-yl)-5,6-dihydro-2H-pyridine-1-carboxylate **3** (3.00 g, 89 % yield ) as a white solid.

LCMS (ESI) calcd for C₁₆H₂₃N₃O₃ [M + H] ⁺ m/z 306.17, found 306.19.

### Preparation of tert-butyl 3-(5-amino-6-methoxypyridin-2-yl)piperidine-1-carboxylate (4)

To a solution of tert-butyl 3-(5-amino-6-methoxypyridin-2-yl)-5,6-dihydro-2H-pyridine-1-carboxylate 3 (2.80 g, 9.20 mmol) in MeOH (50 mL) was added Pd/C (0.78 g, 7.36 mmol) at room temperature. The reaction mixture was stirred under hydrogen at 50 °C for 2 h. After cooling to room temperature, the reaction mixture was filtered, the solution was concentrated under reduced pressure to give product of tert-butyl 3-(5-amino-6-methoxypyridin-2-yl)piperidine-1-carboxylate **4** (2.80 g, 89 % yield) as a white solid.

LCMS (ESI) calcd for C₁₆H₂₅N₃O₃ [M + H] ⁺ m/z 308.19, found 308.19.

### Preparation of tert-butyl 3-(5-bromo-6-methoxypyridin-2-yl)piperidine-1-carboxylate (5)

To a solution of tert-butyl 3-(5-amino-6-methoxypyridin-2-yl)piperidine-1-carboxylate **4** (2.80 g, 9.10 mmol) in ACN (50 mL) was added tert-Butyl nitrite (2.82 g, 27.30 mmol), stirred for 15 minutes, then CuBr (5.22 g, 36.40 mmol) was added at room temperature. The reaction mixture was stirred at 50 °C for 2 h. After cooling to room temperature, the reaction mixture was concentrated under reduced pressure. The residue was purified by flash chromatography (eluting with PE/EA = 100 : 0 to 85 : 15) to give tert-butyl tert-butyl 3-(5-bromo-6-methoxypyridin-2-yl)piperidine-1-carboxylate **5** (1.10 g, 30 % yield ) as a white solid.

LCMS (ESI) calcd for C₁₆H₂₃BrN₂O₃ [M + H] ⁺ m/z 371.09, found 371.15.

### Preparation of tert-butyl 3-(5-ethyl-6-methoxypyridin-2-yl)piperidine-1-carboxylate (6)

To a solution of tert-butyl 3-(5-bromo-6-methoxypyridin-2-yl)piperidine-1-carboxylate **5** (1.10 g, 2.96 mmol) in 1,4-dioxane (65 mL) was added Pd(dppf)Cl₂ (219 mg, 0.30 mmol). Then Et₂Zn (1M, 11.84 mL, 11.84 mml) was added at room temperature. The reaction mixture under nitrogen was stirred at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-ethyl-6-methoxypyridin-2-yl)piperidine-1-carboxylate 6 (700 mg, 67 % yield ) as a white solid.

LCMS (ESI) calcd for C₁₈H₂₈N₂O₃ [M + H] ⁺ m/z 321.21, found 321.30.

### Preparation of 3-ethyl-6-(piperidin-3-yl)-1H-pyridin-2-one (7)

To a solution of tert-butyl 3-(5-ethyl-6-methoxypyridin-2-yl)piperidine-1-carboxylate **6** (200 mg, 0.62 mmol) in HBr in water (48 %, 6 mL). The reaction mixture was stirred at 100 °C for 6 h. After cooling to room temperature, the reaction mixture was concentrated under reduced pressure to give 3-ethyl-6-(piperidin-3-yl)-1H-pyridin-2-one **7** (80 mg, 56 % yield) as a yellow solid.

LCMS (ESI) calcd for C₁₂H₁₈N₂O [M + H] ⁺ m/z 207.14, found 206.95.

### Preparation of 5-{4-[3-(5-ethyl-6-oxo-1H-pyridin-2-yl)piperidin-1-yl]piperidin-1-yl}-N-methylpyridine-2-carboxamide (8)

To a solution of 3-ethyl-6-(piperidin-3-yl)-1H-pyridin-2-one **7** (80 mg, 0.38 mmol) in MeOH (10 mL) was added N-methyl-5-(4-oxopiperidin-1-yl)pyridine-2-carboxamide **INT** (89 mg, 0.38 mmol). Then two drops of acetic acid and NaBH₃CN (24 mg, 0.38mml) was added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 95 : 5) to give 5-{ 4-[3-(5-ethyl-6-oxo-1H-pyridin-2-yl)piperidin-1-yl]piperidin-1-yl}-N-methylpyridine-2-carboxamide 8 (48 mg, 31 % yield) as a white solid.

LCMS (ESI) calcd for C₂₄H₃₃N₅O₂ [M + H] ⁺ m/z 424.26, found 424.37.

### Preparation of 5-{4-[3-(5-ethyl-6-oxo-1H-pyridin-2-yl)piperidin-1-yl]piperidin-1-yl}-N-methylpyridine-2-carboxamide (Compound 53a and Compound 53b)

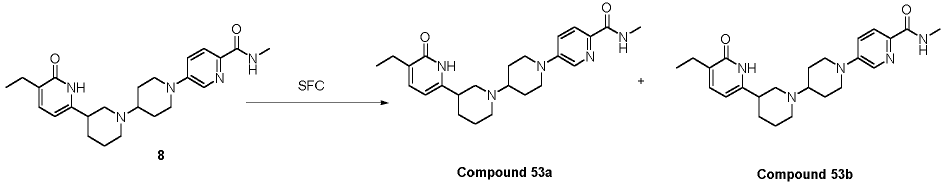

5-{4-[3-(5-ethyl-6-oxo-1H-pyridin-2-yl)piperidin-1-yl]piperidin-1-yl}-N-methylpyridine-2-carboxamide **8** was separated by SFC (Column: Daicel CHIRALPAK AD-H 250 mm × 20 mm I.D., 5 µmm; Mobile phase: CO₂/MeOH[0.1 %(NH₃)] = 75/25) and concentrated under reduced pressure to afford the first fraction as **Compound 53a** (7.16 mg, 95% purity, ee%: 100, white solid) and the second fraction as **Compound 53b** (6.57 mg, 99 % purity, ee%: 100, white solid)

### Compound 53a

¹H NMR (400 MHz, DMSO) δ 11.48 (s, 1 H), 8.39 (q, *J=* 4.8 Hz, 1 H), 8.26 (d, *J=* 2.8 Hz, 1 H), 7.81 (d, *J=* 8.8 Hz, 1 H), 7.40 (dd, *J=* 8.8, 2.8 Hz, 1 H), 7.17 (d, *J=* 6.8 Hz, 1 H), 5.98 (d, *J=* 7.2 Hz, 1 H), 3.97 (d, *J=* 12.4 Hz, 2 H), 2.91-2.77 (m, 7 H), 2.67-2.54 (m, 2 H), 2.36-2.28 (m, 4 H), 1.83-1.81 (m, 3 H), 1.68-1.65 (m,1 H), 1.59-1.40(m, 4 H), 1.05 (t, *J=* 7.6 Hz, 3 H).

### Compound 53b

¹H NMR (400 MHz, DMSO) δ 11.48 (s, 1 H), 8.39 (q, *J* = 4.4 Hz, 1 H), 8.26 (d, *J* = 2.8 Hz, 1 H), 7.81 (d, *J = 8.8* Hz, 1 H), 7.40 (dd, *J=* 9.2, 3.2 Hz, 1 H), 7.17 (d, *J = 7.2* Hz, 1 H), 5.98 (d, *J=* 6.8 Hz, 1 H), 3.97 (d, *J =* 12.4 Hz, 2 H), 2.91-2.77 (m, 7 H), 2.66-2.54 (m, 2 H), 2.36-2.25 (m, 4 H), 1.83-1.80 (m, 3 H), 1.67-1.64 (m, 1 H), 1.59-1.36 (m, 4 H), 1.05 (t, *J =* 7.6 Hz, 3 H).

### Example 3 - Synthesis of Compound 2 (a reference example, not in accordance with the present claims)

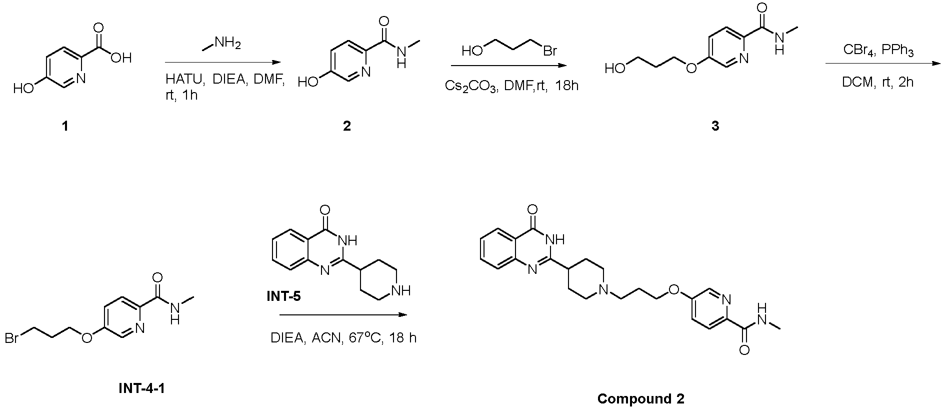

### Preparation of 5-hydroxy-N-methylpicolinamide (2)

To a solution of 5-hydroxypicolinic acid 1 (4.00 g, 28.72 mmol) in DMF (15 mL) was added HATU (12.55 g, 33.01 mmol) and DIEA (9.28 g, 71.80 mmol). Then methanamine (2M, 15 mL, 30.00 mmol) was added at room temperature. Then the mixture was stirred for 1 h at room temperature. The reaction mixture was concentrated under reduced pressure. The residue was purified by flash chromatography (eluting with DCM/MeOH = 100 : 0 to 93 : 7) to give ***5-hydroxy-N-methylpicolinamide* 2** (2.00 g, 46 % yield) as a white solid.

LCMS (ESI) calcd for C₇H₈N₂O₂ [M + H] ⁺ m/z 153.06, found 153.10

### Preparation of 5-(3-hydroxypropoxy)-N-methylpicolinamide (3)

To a solution of 5-hydroxy-N-methylpicolinamide **2** (1.00 g, 6.61 mmol) in DMF (10 mL) was added 3-bromopropan-1-ol (2.75 g, 19.58 mmol) and Cs₂CO₃ (3.23 g, 9.93 mmol) at room temperature. Then the mixture was stirred for 18 h at room temperature. The reaction mixture was filtered to give 5-(3-hydroxypropoxy)-N-methylpicolinamide **3** (1.00 g, 65 % yield) as an oil.

LCMS (ESI) calcd for C₁₀H₁₄N₂O₃ [M + H] ⁺ m/z 211.10, found 211.05.

### Preparation of 5-(3-bromopropoxy)-N-methylpicolinamide (INT-4-1)

To a solution of 5-(3-hydroxypropoxy)-N-methylpicolinamide **3** (250.00 mg, 1.19 mmol) in DCM (15 mL) was added CBr₄ (788.74 mg, 2.38 mmol). Then PPh₃ (623.83 mg, 2.38 mmol) was added at room temperature. Then the mixture was stirred for 2 h at room temperature. The reaction mixture was concentrated under reduced pressure. The residue was purified by flash chromatography (eluting with DCM/MeOH = 100 : 0 to 95 : 5) to give 5-(3-bromopropoxy)-N-methylpicolinamide **INT-4-1** (300 mg, 69 % yield) as a white sold.

LCMS (ESI) calcd for C₁₀H₁₃BrN₂O₂ [M + H] ⁺ m/z 273.02, found 274.85.

### Preparation of N-methyl-5-(3-(4-(4-oxo-3,4-dihydroquinazolin-2-yl)piperidin-1-yl)propoxy)picolinamide (Compound 2)

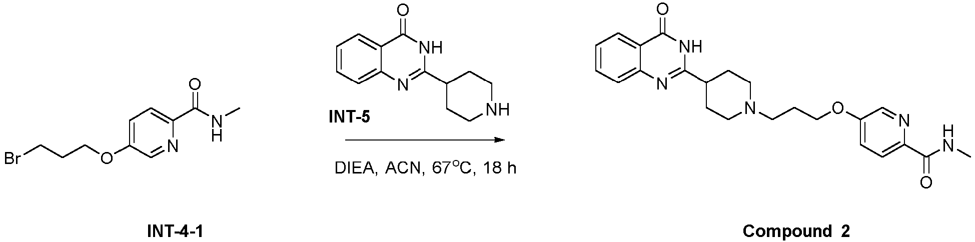

To a solution of 5-(3-bromopropoxy)-N-methylpicolinamide **INT-4-1** (150.00 mg, 0.55 mmol) in ACN (10mL) was added 2-(piperidin-4-yl)-3H-quinazolin-4-one **INT-5** (151.10 mg, 0.66 mmol). Then DIEA (212.94 mg, 1.65 mmol) was added at room temperature. Then the mixture was stirred for 18 h at 67 °C. The reaction mixture was concentrated under reduced pressure. The residue was purified by flash chromatography (eluting with DCM/MeOH = 100 : 0 to 92 : 8) to give N-methyl-5-(3-(4-(4-oxo-3,4-dihydroquinazolin-2-yl)piperidin-1-yl)propoxy)picolinamide **Compound 2** (100 mg, 78% yield) as a white sold.

1H NMR (400 MHz, DMSO) δ 12.15 (s, 1 H), 8.57-8.54 (m, 1 H), 8.29 (d, *J =* 2.8 Hz, 1 H), 8.08 (d, *J =* 8.0 Hz, 1 H), 7.98 (d, *J =* 8.8 Hz, 1 H), 7.78 (t, *J =* 7.6 Hz,1 H), 7.60 (d, *J =* 8.0 Hz, 1 H), 7.54 (dd, *J = 8.8,* 2.8 Hz, 1 H), 7.47 (t, *J =* 7.6 Hz, 1 H), 4.18 (t, *J =* 5.6 Hz, 2 H), 3.10-2.86 (m, 2 H), 2.79 (d, *J =* 4.8 Hz, 3 H), 2.65-2.53 (m, 1 H), 2.49-2.41 (m, 2 H), 1.94 (s, 8 H).

LCMS (ESI) calcd for C₂₃H₂₇N₅O₃ [M + H] ⁺ m/z 422.21, found 422.30.

### Example 4 - Synthesis of Compound 6 (an embodiment of the invention)

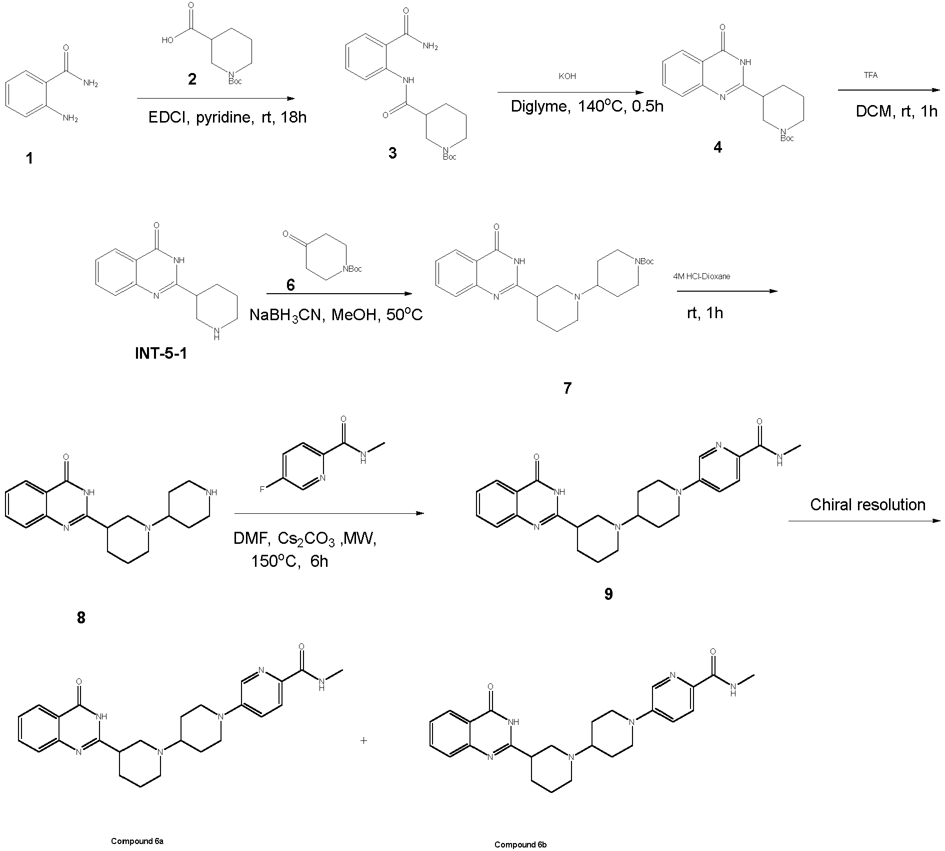

### Preparation of tert-butyl 3-((2-carbamoylphenyl)carbamoyl)piperidine-1-carboxylate (3)

To a solution of 2-aminobenzamide 1 (6.00 g, 44.10 mmol) and 1-(tert-butoxycarbonyl)piperidine-3-carboxylic acid 2 (10.11 g, 44.10 mmol) in pyridine (50 mL) was added EDCI (8.45 g, 44.10 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over Na₂SO₄, concentrated under reduced pressure to give product of tert-butyl 3-((2-carbamoylphenyl)carbamoyl)piperidine-1-carboxylate **3** (12.00 g, 79 % yield) as a white solid.

LCMS (ESI) calcd for C₁₈H₂₅N₃O₄ [M - H] ⁻ m/z 346.18, found 346.20.

### Preparation of tert-butyl 3-(4-oxo-3,4-dihydroquinazolin-2-yl)piperidine-1-carboxylate (4)

To the solution of tert-butyl 3-((2-carbamoylphenyl)carbamoyl)piperidine-1-carboxylate **3** (12.00 g, 34.50 mmol) in Diglyme (80 mL) was added KOH (2.13 g, 37.95 mmol), heated to 140 °C stirred for 2 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 3-(4-oxo-3,4-dihydroquinazolin-2-yl)piperidine-1-carboxylate **4** (11.00 g, 97 % yield) as a white solid.

LCMS (ESI) calcd for C₁₈H₂₃N₃O₃ [M + H] ⁺ m/z 330.17, found 330.20.

### Preparation of 2-(piperidin-3-yl)quinazolin-4(3H)-one (INT-5-1)

To the solution of tert-butyl 3-(4-oxo-3,4-dihydroquinazolin-2-yl)piperidine-1-carboxylate **4** (3.30 g, 10.00 mmol) in DCM (10 mL) were added TFA (10 mL), stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-3-yl)quinazolin-4(3H)-one **INT-5-1** (1.50 g, 66 % yield) as a white solid.

LCMS (ESI) calcd for C₁₃H₁₅N₃O [M + H] ⁺ m/z 230.12, found 230.00.

### Preparation of tert-butyl 3-(4-oxo-3,4-dihydroquinazolin-2-yl)-[1,4'-bipiperidine]-1'-carboxylate (7)

To a solution of 2-(piperidin-3-yl)quinazolin-4(3H)-one **INT-5-1** (460 mg, 2.01 mmol) in MeOH (15 mL) was added tert-butyl 4-oxopiperidine-1-carboxylate **6** (8.45 g, 44.10 mmol), then two drops of acetic acid and NaBH₃CN (189.11 mg, 3.01 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 93 : 7) to give tert-butyl 3-(4-oxo-3,4-dihydroquinazolin-2-yl)-[1,4'-bipiperidine]-1'-carboxylate 7 (300 mg, 36 % yield) as a white solid.

¹H NMR (400 MHz, DMSO) δ 12.40 (s, 1 H), 8.11 (d, *J=* 7.2 Hz, 1 H), 7.81 (s, 1 H), 7.62 (d, *J= 7.8* Hz, 1 H), 7.51 (s, 1 H), 4.10-3.99 (m, 2 H), 3.53-3.44 (m, 1H), 3.16 -3.04 (m, 1 H), 2.94-2.62 (m, 4 H), 1.99 (d, *J =* 2.4 Hz, 4 H), 1.91 (d, *J =* 2.4 Hz, 2 H), 1.82-1.53 (m, 4 H), 1.41 (s, 9 H).

### Preparation of 2-([1,4'-bipiperidin]-3-yl)quinazolin-4(3H)-one (8)

Tert-butyl 3-(4-oxo-3,4-dihydroquinazolin-2-yl)-[1,4'-bipiperidine]-1'-carboxylate **7** (300 mg, 0.65 mmol) was added to HCl in dioxane (4 M, 10 mL), stirred for 1 h at room temperature, the reaction mixture was concentrated under reduced pressure to give 2-([1,4'-bipiperidin]-3-yl)quinazolin-4(3H)-one 8 (200 mg, 88 % yield) as a white solid.

LCMS (ESI) calcd for C₁₈H₂₄N₄O [M + H] ⁺ m/z 313.20, found 313.15.

### Preparation of N-methyl-5-(3-(4-oxo-3,4-dihydroquinazolin-2-yl)-[1,4'-bipiperidin]-1'-yl)picolinamide (9)

To a solution 2-([1,4'-bipiperidin]-3-yl)quinazolin-4(3H)-one **8** (200.00 mg, 0.64 mmol) in DMF (10 mL) was added Cs₂CO₃ (1042.94 mg, 3.20 mmol) and 5-fluoro-N-methylpicolinamide (148.02 mg, 0.96 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to N-methyl-5-(3-(4-oxo-3,4-dihydroquinazolin-2-yl)-[1,4'-bipiperidin]-1'-yl)picolinamide **9** (100 mg, 95 % purity, 33 % yield) as a yellow solid.

LCMS (ESI) calcd for C₂₅H₃₀N₆O₂ [M + H] ⁺ m/z 447.24, found 447.20

### Chiral resolution of N-methyl-5-(3-(4-oxo-3,4-dihydroquinazolin-2-yl)-[1,4'-bipiperidin]-1'-yl)picolinamide (Compound 6a & Compound 6b)

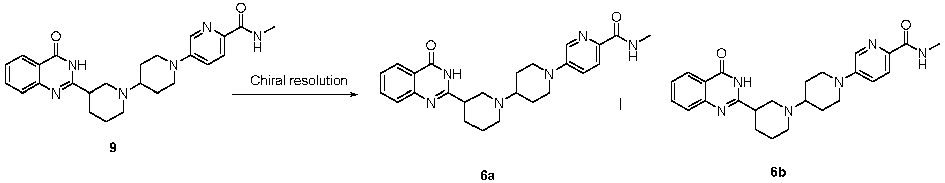

Compound 9 was separated by SFC (Column: Daicel CHIRALPAK OJ -H 250 mm × 20 mm I.D., 5 µmm; Mobile phase: CO₂/MeOH[0.1%(NH₃)] = 70/30) and concentrated under reduced pressure to afford the first fraction as **Compound 6a** (40.6 mg, 100 % purity, ee%: 100, white solid) and the second fraction as **Compound 6b** (39.5 mg, 99 % purity, ee%: 100, white solid)

### Compound 6a

¹H NMR (400 MHz, DMSO) δ 12.25 (s, 1 H), 8.40 (q, *J =* 4.8 Hz, 1 H), 8.29 (d, *J* = 2.8 Hz, 1 H), 8.10 (dd, *J =* 7.6, 1.6 Hz, 1 H), 7.85-7.78 (m, 2 H), 7.63 (d, *J =* 8.0 Hz, 1 H), 7.49 (t, *J =* 7.4 Hz, 1 H), 7.42 (dd, *J=* 8.8, 2.8 Hz, 1 H), 3.99 (d, *J =* 12.8 Hz, 2 H), 3.08 (d, *J* = 8.8 Hz, 1 H), 2.87-2.81 (m, 4 H), 2.80 (d, *J=* 4.8 Hz, 3 H), 2.61-2.55 (m, 2 H), 2.32 (t, *J=* 9.8 Hz, 1 H), 1.98-1.96 (m, 1 H), 1.87 (d, *J =* 12.4 Hz, 2 H), 1.78-1.75 (m, 1 H), 1.69-1.55 (m, 4 H).

LCMS (ESI) calcd for C₂₅H₃₀N₆O₂ [M + H] ⁺ m/z 447.24, found 447.

### Compound 6b

¹H NMR (400 MHz, DMSO) δ 12.25 (s, 1 H), 8.39 (q, *J= 4.8* Hz, 1 H), 8.29 (d, *J= 2.8* Hz, 1 H), 8.10 (dd, *J=* 7.6, 1.6 Hz, 1 H), 7.84-7.78 (m, 2 H), 7.63 (d, *J= 8.0* Hz, 1 H), 7.50- 7.47 (m, 1 H), 7.42 (dd, *J* = 8.8, 2.8 Hz, 1 H), 3.99 (d, *J* = 12.8 Hz, 2 H), 3.08 (d, *J* = 8.8 Hz, 1 H), 2.87-2.81 (m, 4 H), 2.80 (d, *J =* 4.8 Hz, 3 H), 2.61-2.55 (m, 2 H), 2.31 (t, *J =* 9.6 Hz, 1 H), 1.98-1.96 (m, 1 H), 1.87 (d, *J =* 12.4 Hz, 2 H), 1.78-1.75 (m, 1 H), 1.63-1.55 (m, 4 H).

LCMS (ESI) calcd for C₂₅H₃₀N₆O₂ [M + H] ⁺ m/z 447.24, found 447.

### Example 5 - Synthesis of Compound 8 (a reference example, not in accordance with the present claims)

### Synthesis of INT-5

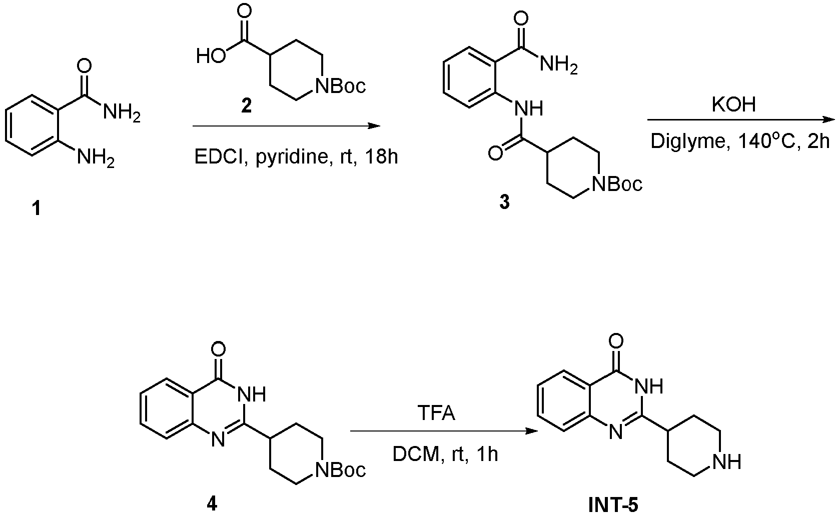

### Preparation of tert-butyl 4-((2-carbamoylphenyl)carbamoyl)piperidine-1-carboxylate (3)

To a solution of 2-aminobenzamide 1 (6.00 g, 44.10 mmol) and 1-(tert-butoxycarbonyl)piperidine-4-carboxylic acid 2 (10.11 g, 44.10 mmol) in pyridine (50 mL) was added EDCI (8.45 g, 44.10 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over Na₂SO₄, concentrated under reduced pressure to give product of tert-butyl 4-((2-carbamoylphenyl)carbamoyl)piperidine-1-carboxylate **3** (12.00 g, 79 % yield) as a white solid.

LCMS (ESI) calcd for C₁₈H₂₅N₃O₄ [M + Na] ⁺ m/z 370.42, found 370.15.

### Preparation of tert-butyl 4-(4-oxo-3,4-dihydroquinazolin-2-yl)piperidine-1-carboxylate (4)

To the solution of tert-butyl 4-((2-carbamoylphenyl)carbamoyl)piperidine-1-carboxylate **3** (12.00 g, 34.50 mmol) in Diglyme (80 mL) was added KOH (2.13 g, 37.95 mmol), heated to 140 °C stirred for 2 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 4-(4-oxo-3,4-dihydroquinazolin-2-yl)piperidine-1-carboxylate **4** (11.00 g, 97 % yield) as a white solid.

LCMS (ESI) calcd for C₁₈H₂₃N₃O₃ [M + H] ⁺ m/z 330.17, found 330.20.

### Preparation of 2-(piperidin-4-yl)quinazolin-4(3H)-one (INT-5)

To the solution of tert-butyl 4-(4-oxo-3,4-dihydroquinazolin-2-yl)piperidine-1-carboxylate **4** (3.30 g, 10.00 mmol) in DCM (10 mL) were added TFA (10 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-4-yl)quinazolin-4(3H)-one **INT-5** (1.50 g, 66% yield) as a white solid.

LCMS (ESI) calcd for C₁₃H₁₅N₃O [M + H] ⁺ m/z 230.12, found 230.20.

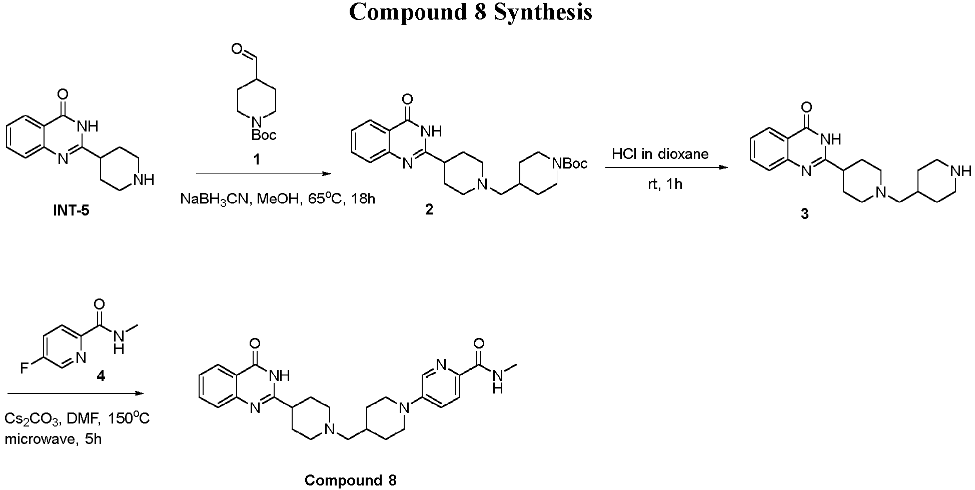

### Preparation of tert-butyl 4-formylpiperidine-1-carboxylate (2)

To a solution of tert-butyl 4-formylpiperidine-1-carboxylate 1 (837.00 mg, 3.85 mmol) in MeOH (50 mL) was added 2-(piperidin-4-yl) quinazolin-4(3H)-one **INT-5** (300.00 mg, 1.31 mmol). Then two drops of acetic acid and NaBH₃CN (400.00 mg, 6.37 mmol) were added at room temperature. Then the mixture was stirred for 18 h at 65 °C. After cooling to room temperature, the reaction mixture was concentrated under reduced pressure. The residue was purified by flash chromatography (eluting with DCM/MeOH = 100 : 0 to 92 : 8) to give tert-butyl 4-((4-(4-oxo-3,4-dihydroquinazolin-2-yl)piperidin-1-yl)methyl)piperidine-1-carboxylate **2** (500.00 mg, 90 % yield ) as a white solid.

LCMS (ESI) calcd for C₂₄H₃₄N₄O₃ [M + H] ⁺ m/z 427.26, found 427.30.

### Preparation of tert-butyl 4-((4-(4-oxo-3,4-dihydroquinazolin-2-yl)piperidin-1-yl) methyl)piperidine-1-carboxylate (3)

To a solution of tert-butyl 4-((4-(4-oxo-3,4-dihydroquinazolin-2-yl)piperidin-1-yl)methyl)piperidine-1-carboxylate **2** (500.00 mg, 1.17 mmol) in MeOH (5 mL) was added 4M HCl-Dioxane (10 mL) at room temperature. The reaction mixture stirred at room temperature for 1 h. The reaction mixture was concentrated under reduced pressure to give 2-(1-(piperidin-4-ylmethyl) piperidin-4-yl) quinazolin-4(3H)-one **3** (350.00 mg, 87 % yield) as a white solid.

LCMS (ESI) calcd for C₁₉H₂₆N₄O [M + H] ⁺ m/z 327.21, found 327.15.

### Preparation of N-methyl-5-(4-((4-(4-oxo-3,4-dihydroquinazolin-2-yl)piperidin-1-yl)methyl)piperidin-1-yl)picolinamide (Compound 8)

To a solution of 2-(1-(piperidin-4-ylmethyl)piperidin-4-yl)quinazolin-4(3H)-one **3** (300.00 mg, 0.92 mmol) in DMF (5 mL) was added 5-fluoro-N-methylpicolinamide **4** (354.14 mg, 2.30 mmol). Then Cs₂CO₃ (2994.20 mg, 9.19 mmol) was added at room temperature. The reaction mixture was irradiated in a microwave reactor at 150 °C for 5 h. the reaction mixture was concentrated under reduced pressure. The residue was purified by prep-HPLC (columns: Gemini 5 um C18 150 × 21.2 mm, mobile phase: ACN - H₂O (0.1% FA), gradient: 15-25) to give N-methyl-5-(4-((4-(4-oxo-3,4-dihydroquinazolin-2-yl)piperidin-1-yl)methyl)piperidin-1-yl)picolinamide **Compound 8** (100 mg, 98 % purity, 24 % yield) as a white sold.

LCMS (ESI) calcd for C₂₆H₃₂N₆O₂ [M + H] ⁺ m/z 461.26, found 461.20.

1H NMR (400 MHz, DMSO) δ 12.13 (s, 1 H), 8.37 (d, *J= 4.8* Hz, 1 H), 8.26 (d, *J= 2.8* Hz, 1 H), 8.08 (d, *J* = 7.6 Hz, 1 H), 7.82-7.76 (m, 2 H), 7.60 (d, *J=* 8.0 Hz, 1 H), 7.50-7.35 (m, 2 H), 3.91 (d, *J=* 12.4 Hz, 2 H), 2.95 (d, *J=* 10.8 Hz, 2 H), 2.89-2.75 (m, 5 H), 2.62-2.53 (m, 1 H), 2.18 (d, *J=* 6.4 Hz, 2 H), 1.98-1.73 (m, 9 H), 1.23-1.17 (m, 2 H).

### Example 6 - Synthesis of Compound 10 (an embodiment of the invention)

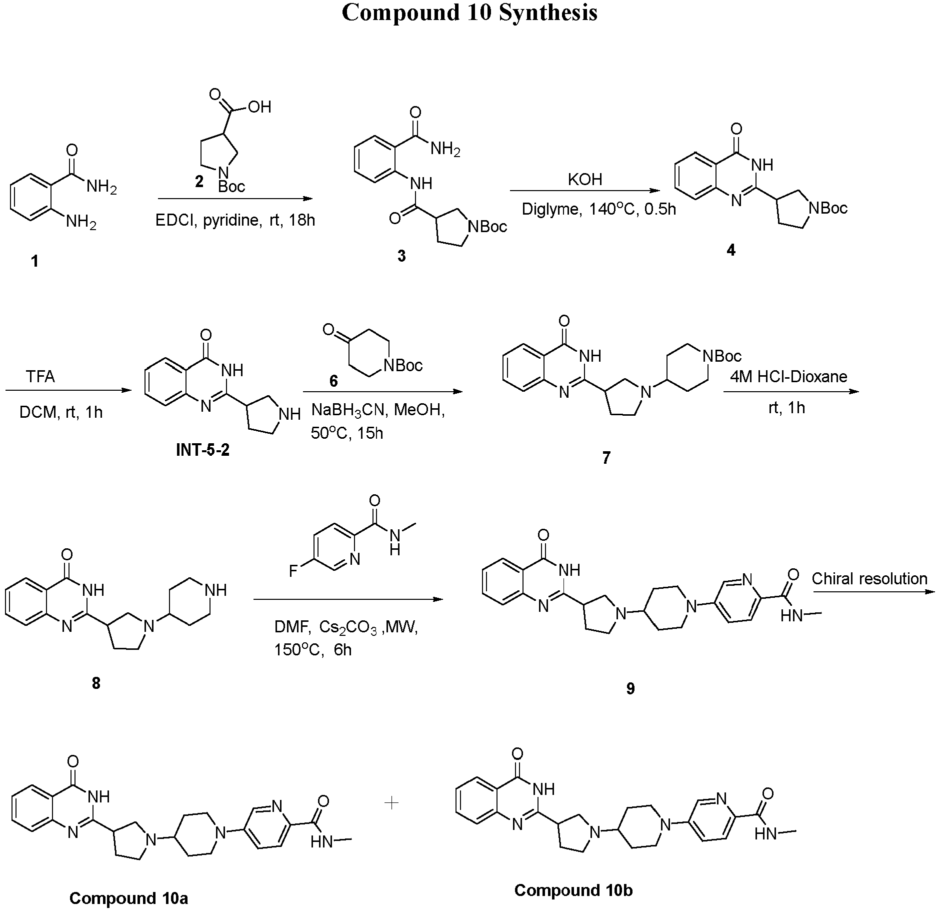

### Preparation of tert-butyl 3-((2-carbamoylphenyl)carbamoyl)pyrrolidine-1-carboxylate (3)

To a solution of 2-aminobenzamide 1 (6.00 g, 44.10 mmol) and 1-(tert-butoxycarbonyl)pyrrolidine-3-carboxylic acid 2 (9.49 g, 44.10 mmol) in pyridine (50 mL) was added EDCI (8.45 g, 44.10 mmol). then the mixture was stirred for 18 h at room temperature. The reaction mixture was quenched with water and the aqueous layer was extracted with EtOAc (50 mL x 3). The combined organic layers was washed by 1M HCl solution and brine, dried over Na₂SO₄, concentrated under reduced pressure to give tert-butyl 3-((2-carbamoylphenyl)carbamoyl)pyrrolidine-1-carboxylate 3 (12.00 g, 77 % yield) as a white solid LCMS (ESI) calcd for C₁₇H₂₃N₃O₄ [M + Na] ⁺ m/z 356.17, found 356.00.

### Preparation of tert-butyl 3-(4-oxo-3,4-dihydroquinazolin-2-yl)pyrrolidine-1-carboxylate (4)

To the solution of tert-butyl 3-((2-carbamoylphenyl)carbamoyl)pyrrolidine-1-carboxylate **3** (12.00 g, 36.04 mmol) in Diglyme (80 mL) was added KOH (2.42 g, 43.25 mmol), heated to 140 °C stirred for 0.5 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 3-(4-oxo-3,4-dihydroquinazolin-2-yl)pyrrolidine-1-carboxylate **4** (10.00 g, 83% yield) as a white solid .

LCMS (ESI) calcd for C₁₇H₂₁N₃O₃ [M + H] ⁺ m/z 316.16, found 316.05.

### Preparation of 2-(pyrrolidin-3-yl)quinazolin-4(3H)-one (INT-5-2)

To the solution of tert-butyl 3-(4-oxo-3,4-dihydroquinazolin-2-yl)pyrrolidine-1-carboxylate **4** (10.00 g, 31.75 mmol) in DCM (20 mL) was added TFA (20 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(pyrrolidin-3-yl)quinazolin-4(3H)-one **INT-5-2** (8.00 g, 80 % yield) as a white solid.

1H NMR (400 MHz, DMSO-d₆, ppm) δ 8.14-8.07 (m, 1 H), 7.87-7.76 (m, 1 H), 7.64 (d, *J* = 8.0 Hz, 1 H), 7.57-7.44 (m, 1 H), 3.76-3.61 (m, 1 H), 3.61-3.49 (m, 2 H),3.43-3.33 (m, 1 H), 3.29 (dt, *J=* 11.4, 5.6 Hz, 1 H), 2.37 (td, *J=* 14.0, 7.1 Hz, 1 H), 2.21 (td, *J=* 13.5, 7.2 Hz, 1 H).

LCMS (ESI) calcd for C₁₂H₁₃N₃O [M - H] ⁻ m/z 216.11, found 215.95.

### Preparation of tert-butyl 4-(3-(4-oxo-3,4-dihydroquinazolin-2-yl)pyrrolidin-1-yl)piperidine-1-carboxylate (7)

To a solution of 2-(pyrrolidin-3-yl)quinazolin-4(3H)-one **INT-5-2** (600.00 mg, 2.79 mmol) in MeOH (30 mL) was added tert-butyl 4-oxopiperidine-1-carboxylate **6** (1.67 g, 8.36 mmol), then two drops of acetic acid and NaBH₃CN (1.05 g, 16.72 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 93 : 7) to give tert-butyl 4-(3-(4-oxo-3,4-dihydroquinazolin-2-yl)pyrrolidin-1-yl)piperidine-1-carboxylate 7 (400 mg, 34 % yield) as a white solid.

LCMS (ESI) calcd for C₂₂H₃₀N₄O₃ [M + H] ⁺ m/z 399.23, found 399.30.

### Preparation of 2-(1-(piperidin-4-yl)pyrrolidin-3-yl)quinazolin-4(3H)-one (8)

Tert-butyl 4-(3-(4-oxo-3,4-dihydroquinazolin-2-yl)pyrrolidin-1-yl)piperidine-1-carboxylate 7 (400 mg, 0.81 mmol) was added to HCl in dioxane (4 M, 10 mL), stirred for 1 h at room temperature, the reaction mixture was concentrated under reduced pressure to give 2-(1-(piperidin-4-yl)pyrrolidin-3-yl)quinazolin-4(3H)-one **8** (200.00 mg, 79% yield ) as a white solid.

LCMS (ESI) calcd for C₁₇H₂₂N₄O [M + H] ⁺ m/z 299.18, found 299.25.

### Preparation of N-methyl-5-(4-(3-(4-oxo-3,4-dihydroquinazolin-2-yl)pyrrolidin-1-yl)piperidin-1-yl)picolinamide (9)

To a solution 2-(1-(piperidin-4-yl)pyrrolidin-3-yl)quinazolin-4(3H)-one **8** (200.00 mg, 0.67 mmol) in DMF (10 mL) was added Cs₂CO₃ (2.18 g, 6.70 mmol) and 5-fluoro-N-methylpicolinamide 9 (432.44 mg, 2.01 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to N-methyl-5-(4-(3-(4-oxo-3,4-dihydroquinazolin-2-yl)pyrrolidin-1-yl)piperidin-1-yl)picolinamide **9** (100 mg, 95 % purity, 33 % yield) as a yellow solid.

LCMS (ESI) calcd for C₂₄H₂₈N₆O₂ [M + H] ⁺ m/z 433.23, found 433.25.

### Chiral resolution of N-methyl-5-(4-(3-(4-oxo-3,4-dihydroquinazolin-2-yl)pyrrolidin-1-yl)piperidin-1-yl)picolinamide (9)

Compound **9** was separated by SFC (Column: Daicel CHIRALPAK OJ -H 250 mm × 20 mm I.D., 5 µmm; Mobile phase: CO₂/MeOH[0.1%(NH₃)] = 70/30) and concentrated under reduced pressure to afford the first fraction as **Compound 10a** (16.4. mg, 100% purity, ee%: 100, off-white solid) and the second fraction as **Compound 10b** (12.3 mg, 100% purity, ee%: 100, off-white solid)

### Compound 10a

¹H NMR (400 MHz, DMSO-d₆, ppm) δ 12.16 (s, 1 H), 8.40-8.37 (m, 1 H), 8.27 (d, *J=* 2.8 Hz, 1 H), 8.07 (dd, *J=* 7.6, 1.6 Hz, 1 H), 7.82-7.75 (m, 2 H), 7.61 (d, *J= 8.0* Hz, 1 H), 7.48-7.44 (m, 1 H), 7.40 (dd, *J= 8.8,* 2.8 Hz, 1 H), 3.82 (d, *J=* 12.8 Hz, 2 H), 3.07 (t, *J=* 8.6 Hz, 2 H), 2.95 (t, *J =* 10.8 Hz, 2 H), 2.86-2.79 (m, 2 H), 2.78-2.77 (m, 3 H), 2.69-2.63 (m, 1 H), 2.33-2.32 (m, 1H), 2.20-2.13 (m, 2 H), 1.96-1.93 (m, 2 H), 1.58-1.47 (m, 2 H).

LCMS (ESI) calcd for C₂₄H₂₈N₆O₂ [M + H] ⁺ m/z 433.23, found 433.20.

### Compound 10b

¹H NMR (400 MHz, DMSO-d₆, ppm) δ 8.39-8.38 (m, 1 H), 8.28 (d, *J=* 3.2 Hz, 1 H), 8.08 (dd, *J=* 7.6, 1.6 Hz, 1 H), 7.83-7.76 (m, 2 H), 7.61 (d, *J* = 8.0 Hz, 1 H), 7.47 (t, *J =* 7.6 Hz, 1 H), 7.40 (dd, *J=* 8.8, 2.8 Hz, 1 H), 3.83 (d, *J =* 12.8 Hz, 2 H), 3.09 (d, *J =* 8.4 Hz, 2 H), 2.95 (t, *J* = 12.4 Hz, 2 H), 2.88-2.79 (m, 2 H), 2.79 (d, *J =* 4.8 Hz, 3 H), 2.68-2.66 (m, 1 H), 2.39-2.31 (m, 1H), 2.21-2.14 (m, 2 H), 1.97-1.94 (m, 2 H), 1.59-1.47 (m, 2 H).

LCMS (ESI) calcd for C₂₄H₂₈N₆O₂ [M + H] ⁺ m/z 433.23, found 433.20.

### Example 7 - Synthesis of Compound 14 (a reference example, not in accordance with the present claims)

### Preparation of INTS-3

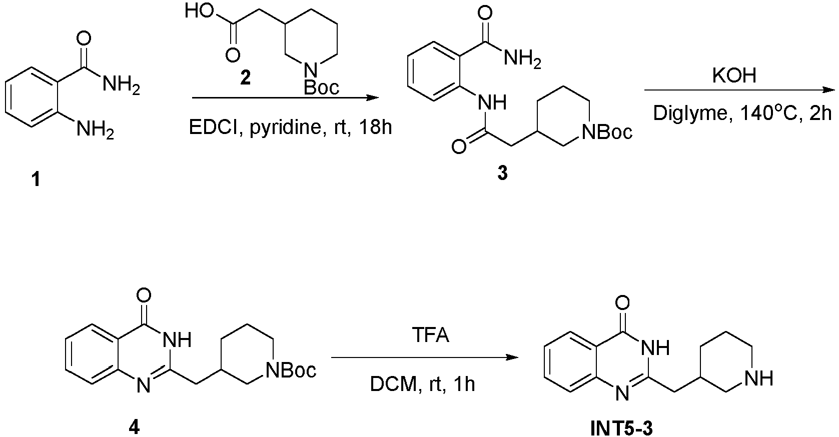

### Preparation of tert-butyl 3-(2-((2-carbamoylphenyl)amino)-2-oxoethyl)piperidine-1-carboxylate (3)

To a solution of 2-aminobenzamide 1 (2.80 g, 20.60 mmol) and 2-(1-(tert-butoxycarbonyl) piperidin-3-yl)acetic acid 2 (5.00 g, 20.60 mmol) in pyridine (50 mL) was added EDCI (3.95 g, 20.60 mmol). then the mixture was stirred for 18 h at room temperature. The reaction mixture was quenched with water, the aqueous layer was extracted with EtOAc (50 mL x 3). The combined organic layers was washed with 1M HCl solution and brine, dried over Na₂SO₄, concentrated under reduced pressure to give product of tert-butyl 3-(2-((2-carbamoylphenyl)amino)-2-oxoethyl)piperidine-1-carboxylate **3** (7.00 g, 94% yield) as a white solid.

LCMS (ESI) calcd for C₁₉H₂₇N₃O₄ [M + H] ⁺ m/z 362.20, found 362.25.

### Preparation of tert-butyl 3-((4-oxo-3,4-dihydroquinazolin-2-yl)methyl)piperidine-1-carboxylate (4)

To the solution of tert-butyl 3-(2-((2-carbamoylphenyl)amino)-2-oxoethyl)piperidine-1-carboxylate **3** (7.60 g, 21.00 mmol) in Diglyme (80 mL) was added KOH (1.18 g, 21.00 mmol), heated to 140 °C stirred for 2 h, cooled to 0 °C, ice-water (100 mL) added then adjusted pH < 7 using 1M HCl solution, precipitate formed, filtered, the filter cake washed with ice-water (50 mL*3), then dried to give product of tert-butyl 3-((4-oxo-3,4-dihydroquinazolin-2-yl)methyl)piperidine-1-carboxylate **4** (6.80 g, 94 % yield) as a white solid.

LCMS (ESI) calcd for C₁₉H₂₅N₃O₃ [M + H] ⁺ m/z 344.19, found 344.15.

### Preparation of 2-(piperidin-3-ylmethyl)quinazolin-4(3H)-one (INT-5-3)

To a solution of tert-butyl 3-((4-oxo-3,4-dihydroquinazolin-2-yl)methyl)piperidine-1-carboxylate **4** (2.00 g, 5.80 mmol) in DCM (10 mL) were added TFA (10 mL), stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-3-ylmethyl)quinazolin-4(3H)-one **INT-5-3** (1.20 g, 85% yield) as a white solid .

LCMS (ESI) calcd for C₁₄H₁₇N₃O [M + H] ⁺ m/z 244.14, found 244.20.

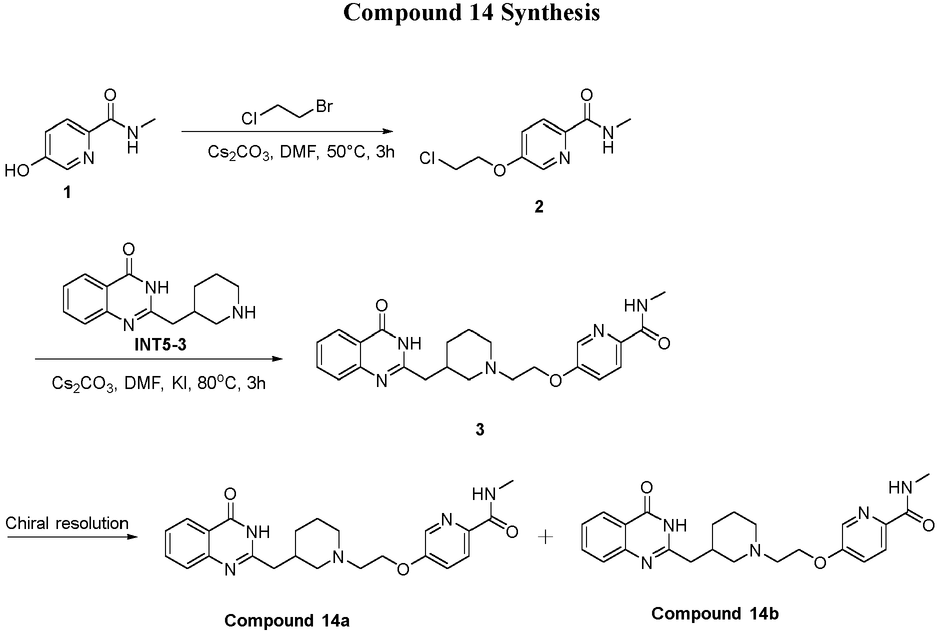

### Preparation of tert-butyl 4-((2-carbamoylphenyl)carbamoyl)piperidine-1-carboxylate (2)

To a solution of 5-hydroxy-N-methylpicolinamide **1** (500.00 mg, 3.29 mmol) and Cs₂CO₃ (3.22 g, 9.87 mmol) in DMF (15 mL) was added 1-bromo-2-chloroethane **2** (943.64 mg, 6.58 mmol). then the mixture was stirred for 3 h at 50 °C. The reaction mixture was quenched with water, aqueous layer was extracted with EtOAc (50 mL x 3), the combined organic layers was concentrated under reduced pressure. the residue was purified by flash chromatography (eluting with PE/ EtOAc = 100 : 0 to 80 : 20) to give tert-butyl 4-((2-carbamoylphenyl)carbamoyl)piperidine-1-carboxylate **2** (500.00 mg, 71% yield ) as a colorless oil.

LCMS (ESI) calcd for C₉H₁₁ClN₂O₂ [M + H] ⁺ m/z 215.05, found 215.00.

### Preparation of N-methyl-5-(2-(3-((4-oxo-3,4-dihydroquinazolin-2-yl)methyl)piperidin-1-yl)ethoxy)picolinamide (3)

To a solution of tert-butyl 4-((2-carbamoylphenyl)carbamoyl)piperidine-1-carboxylate **2** (500.00 mg, 2.33 mmol) and Cs₂CO₃ (2.28 g, 6.99 mmol) in DMF (15 mL) were added 2-(piperidin-3-ylmethyl)quinazolin-4(3H)-one **INT5-3** (566.91 mg, 2.33 mmol) and KI (1.16 g, 6.99 mmol). then the mixture was stirred for 3 h at 80 °C. The reaction mixture was quenched with water, aqueous layer was extracted with EtOAc (50 mL x 3), the combined organic layers was concentrated under reduced pressure. the residue was purified by flash chromatography (eluting with DCM/ MeOH = 100 : 0 to 92 : 8) to give N-methyl-5-(2-(3-((4-oxo-3,4-dihydroquinazolin-2-yl)methyl)piperidin-1-yl)ethoxy)picolinamide **3** (200.00 mg, 20% yield ) as a yellow solid.

LCMS (ESI) calcd for C₂₃H₂₇N₅O₃ [M + H] ⁺ m/z 422.21, found 422.10.

### Chiral resolution of N-methyl-5-(2-(3-((4-oxo-3,4-dihydroquinazolin-2-yl)methyl)piperidin-1-yl)ethoxy)picolinamide (Compound 14)

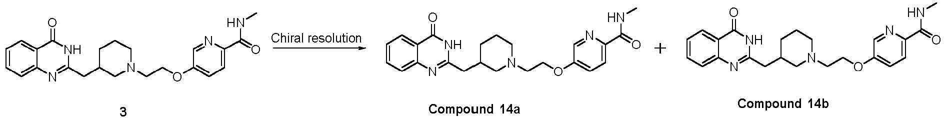

Compound 3 was separated by SFC (Column: Daicel CHIRALPAK OJ -H 250 mm × 20 mm I.D., 5 µmm; Mobile phase: CO₂/MeOH[0.1%(FA)] = 70/30) and concentrated under reduced pressure to afford the first fraction as **Compound 14a** (31.6 mg, 100% purity, ee%: 100, white solid) and the second fraction as **Compound 14b** (28.1 mg, 99% purity, ee%: 99, white solid)

### Compound 14a

¹H NMR (400 MHz, DMSO-d₆, ppm) δ 12.15 (s, 1 H), 8.53 (q, *J =* 9.6, 4.8 Hz, 1 H), 8.23 (d, *J=* 2.8 Hz, 1 H), 8.07 (dd, *J= 8.0,* 1.2 Hz, 1 H), 7.92 (d, *J =* 8.8 Hz, 1 H), 7.79-7.73 (m, 1 H), 7.56 (d, *J =* 8.0 Hz, 1 H), 7.49-7.42 (m, 2 H), 4.18 *(t, J =* 5.6 Hz, 2 H), 2.90-2.81 (m, 2 H), 2.79 (d, *J= 4.8* Hz, 3 H), 2.73-2.65 (m, 2 H), 2.55-2.51 (m,2 H), 2.17-2.00 (m, 2 H), 1.93-1.85 (m, 1 H), 1.70-1.59 (m, 2 H), 1.51-1.38 (m, 1 H), 1.02-0.99 (m, 1 H).

LCMS (ESI) calcd for C₂₃H₂₇N₅O₃ [M + H] ⁺ m/z 422.21, found 422.25.

### Compound 14b

¹H NMR (400 MHz, DMSO-d₆, ppm) δ 12.15 (s, 1 H), 8.53 (q, *J=* 9.6, 4.8 Hz, 1 H), 8.23 (d, *J=* 2.8 Hz, 1 H), 8.07 (dd, *J= 8.0,* 1.2 Hz, 1 H), 7.92 (d, *J =* 8.8 Hz, 1 H), 7.79-7.73 (m, 1 H), 7.56 *(d, J =* 8.0 Hz, 1 H), 7.49-7.42 (m, 2 H), 4.18 *(t, J =* 5.6 Hz, 2 H), 2.90-2.81 (m, 2 H), 2.79 (d, *J= 4.8* Hz, 3 H), 2.73-2.65 (m, 2 H), 2.55-2.51 (m,2 H), 2.17-2.00 (m, 2 H), 1.93-1.85 (m, 1 H), 1.70-1.59 (m, 2 H), 1.51-1.38 (m, 1 H), 1.02-0.99 (m, 1 H).

LCMS (ESI) calcd for C₂₃H₂₇N₅O₃ [M + H] ⁺ m/z 422.21, found 422.25.

### Example 8 - Synthesis of Compound 17 (an embodiment of the invention)

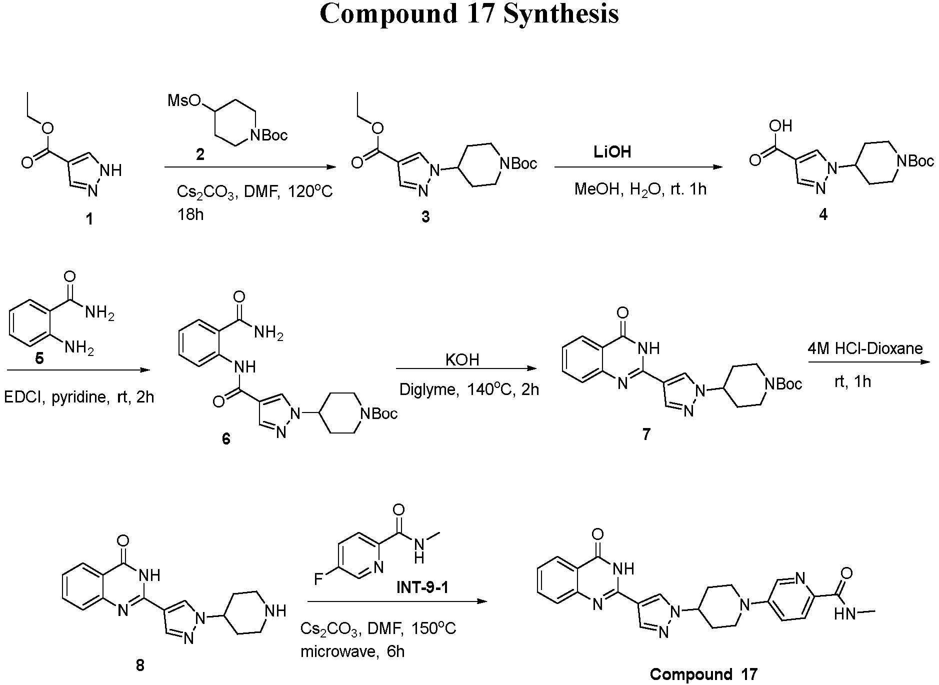

### Preparation of tert-butyl 4-(4-(ethoxycarbonyl)-1H-pyrazol-1-yl)piperidine-1-carboxylate (3)

To a solution of ethyl 1H-pyrazole-4-carboxylate **1** (2.00 g, 14.30 mmol) and tert-butyl 4-((methylsulfonyl)oxy)piperidine-1-carboxylate **2** (4.99 g, 17.87 mmol) in DMF (60 mL) was added Cs₂CO₃ (10.02 g, 30.74 mmol). Then the mixture was stirred for 18 h at 120 °C. The reaction mixture was quenched with water, the aqueous layer was extracted with EtOAc (200 mL x 3). The combined organic layer was washed with brine, dried over Na₂SO₄, concentrated under reduced pressure. The residue was purified by flash chromatography (eluting with DCM/MeOH = 100 : 0 to 95 : 5) to give to give product of tert-butyl 4-(4-(ethoxycarbonyl)-1H-pyrazol-1-yl)piperidine-1-carboxylate **3** (2.00 g, 41 % yield) as a white solid .

LCMS (ESI) calcd for C₁₆H₂₅N₃O₄ [M + H] ⁺ m/z 324.18, found 324.20.

### Preparation of 1-(1-(tert-butoxycarbonyl)piperidin-4-yl)-1H-pyrazole-4-carboxylic acid (4)

To a solution of tert-butyl 4-(4-(ethoxycarbonyl)-1H-pyrazol-1-yl)piperidine-1-carboxylate **3** (1.50 g, 4.80 mmol) in MeOH/H₂O = 1 : 1 (40 mL) was added LiOH (460.00 mg, 19.20 mmol). The mixture was stirred at rt for 1 h. The reaction mixture was concentrated under reduced pressure at 40 °C to obtain residue. To this was added water and acidified with 1 M aqueous HCl to adjust pH= 4 ~ 5 at 0 °C, the aqueous layer was extracted with EtOAc (200 mL x 3). The combined organic layer was washed with brine, dried over Na₂SO₄, concentrated under reduced pressure. The residue was purified by flash chromatography (eluting with DCM/MeOH = 100 : 0 to 95 : 5) to give 1-(1-(tert-butoxycarbonyl)piperidin-4-yl)-1H-pyrazole-4-carboxylic acid **4** (1.00 g, 67% yield) as a white solid.

LCMS (ESI) calcd for C₁₄H₂₁N₃O₄ [M - 56 + H] ⁺ m/z 240.15, found 240.20.

¹H NMR (400 MHz, DMSO-d₆, ppm) δ 12.29 (s, 1 H), 8.30 (s, 1H), 7.81 (s, 1 H), 4.52-4.30 (m, 1 H), 4.03 (d, *J=* 11.2 Hz, 2 H), 2.88 (s, 2 H), 1.99 (d, *J=* 12.4 Hz, 2 H), 1.78 (dt, *J=* 12.4, 8.0 Hz, 2 H), 1.41 (s, 9 H).

### Preparation of tert-butyl 4-(4-((2-carbamoylphenyl)carbamoyl)-1H-pyrazol-1-yl)piperidine-1-carboxylate (6)

To a solution of 2-aminobenzamide **5** (299.66 mg, 2.20 mmol) and 1-(1-(tert-butoxycarbonyl)piperidin-4-yl)-1H-pyrazole-4-carboxylic acid **4** (650.00 mg, 2.20 mmol) in pyridine (25 mL) was added EDCI (421.91 mg, 2.20 mmol). then the mixture was stirred for 12 h at room temperature. The reaction mixture was quenched with water, the aqueous layer was extracted with EtOAc (50 mL x 3). The combined organic layers was washed by 1M HCl solution and brine, dried over Na₂SO₄, concentrated under reduced pressure to give product of tert-butyl 4-(4-((2-carbamoylphenyl)carbamoyl)-1H-pyrazol-1-yl)piperidine-1-carboxylate **6** (720.00 mg, 71% yield) as a white solid.

LCMS (ESI) calcd for C₂₁H₂₇N₅O₄ [M + H] ⁺ m/z 414.21, found 414.25.

### Preparation of tert-butyl 4-(4-(4-oxo-3,4-dihydroquinazolin-2-yl)-1H-pyrazol-1-yl)piperidine-1-carboxylate (7)

To the solution of tert-butyl 4-(4-((2-carbamoylphenyl)carbamoyl)-1H-pyrazol-1-yl)piperidine-1-carboxylate 6 (700.00 mg, 1.69 mmol) in Diglyme (15 mL) was added KOH (113.99 mg, 2.03 mmol), heated to 140 °C stirred for 2 h, cooled to 0 °C, ice-water (10 mL) added then adjusted pH < 7 using 1M HCl solution, precipitate formed, filtered, the filter cake washed with ice-water (50 mL*3), then dried to give product of tert-butyl 4-(4-(4-oxo-3,4-dihydroquinazolin-2-yl)-1H-pyrazol-1-yl)piperidine-1-carboxylate 7 (600 mg, 67% yield) as a white solid.

LCMS (ESI) calcd for C₂₁H₂₅N₅O₃ [M + H] ⁺ m/z 396.20, found 396.25.

### Preparation of 2-(1-(piperidin-4-yl)-1H-pyrazol-4-yl)quinazolin-4(3H)-one (8)

To a solution of tert-butyl 4-(4-(4-oxo-3,4-dihydroquinazolin-2-yl)-1H-pyrazol-1-yl)piperidine-1-carboxylate 7 (350.00 mg, 0.88 mmol) at room temperature was added HCl-Dioxane (4 M, 40 mL). The reaction mixture was stirred at room temperature for 1h. After reaction completed, the mixture was concentrated under reduced pressure to obtain crude 2-(1-(piperidin-4-yl)-1H-pyrazol-4-yl)quinazolin-4(3H)-one **8** (200 mg, 73 % yield) as a white solid.

LCMS (ESI) calcd for C₁₆H₁₇N₅O [M + H] ⁺ m/z 296.14, found 296.25.

### Preparation of N-methyl-5-(4-(4-(4-oxo-3,4-dihydroquinazolin-2-yl)-1H-pyrazol-1-yl)piperidin-1-yl)picolinamide (Compound 17)

To a solution 2-(1-(piperidin-4-yl)-1H-pyrazol-4-yl)quinazolin-4(3H)-one **8** (200.00 mg, 0.68 mmol) in DMF (10 mL) was added Cs₂CO₃ (2.21 g, 6.77 mmol) and 5-fluoro-N-methylpicolinamide **INT-9-1** (260.96 mg, 1.69 mmol). The mixture was stirred for 6 h under 150 °C using microwave. The reaction mixture was concentrated under reduced pressure. The residue was purified by flash chromatography (eluting with DCM/MeOH = 100 : 0 to 92 : 8) to give crude product which was purified by prep-HPLC (Gemini 5um C18 150 × 21.2 mm, mobile phase: ACN - H₂O (0.1% FA), gradient: 25 - 75) to give N-methyl-5-(4-(4-(4-oxo-3,4-dihydroquinazolin-2-yl)-1H-pyrazol-1-yl)piperidin-1-yl)picolinamide **Compound 17** (30 mg, 98% purity, 9 % yield) as a white solid.

¹H NMR (400 MHz, DMSO-d₆, ppm) δ 12.30 (s, 1 H), 8.67 (s, 1 H), 8.43-8.40 (m, 1 H), 8.35 (d, *J =* 2.8 Hz, 1 H), 8.26 (s, 1 H), 8.09 (d, *J =* 8.0 Hz, 1 H), 7.85 (d, *J =* 8.8 Hz, 1 H), 7.80-7.76 (m, 1 H), 7.61 (d, *J=* 8.0 Hz, 1 H), 7.50-7.42 (m, 2 H), 4.56-4.53 (m, 1 H), 4.08 (d, *J =* 12.8 Hz, 2 H), 3.10 (t, *J =* 11.6, 10.8 Hz, 2 H), 2.79 (d, *J =* 4.8 Hz, 3 H), 2.19-2.17 (m, 2 H), 2.06-1.98 (m, 2 H).

LCMS (ESI) calcd for C₂₃H₂₃N₇O₂ [M + H] ⁺ m/z 430.19, found 430.25.

### Example 9 - Synthesis of Compound 40 (a reference example, not in accordance with the present claims)

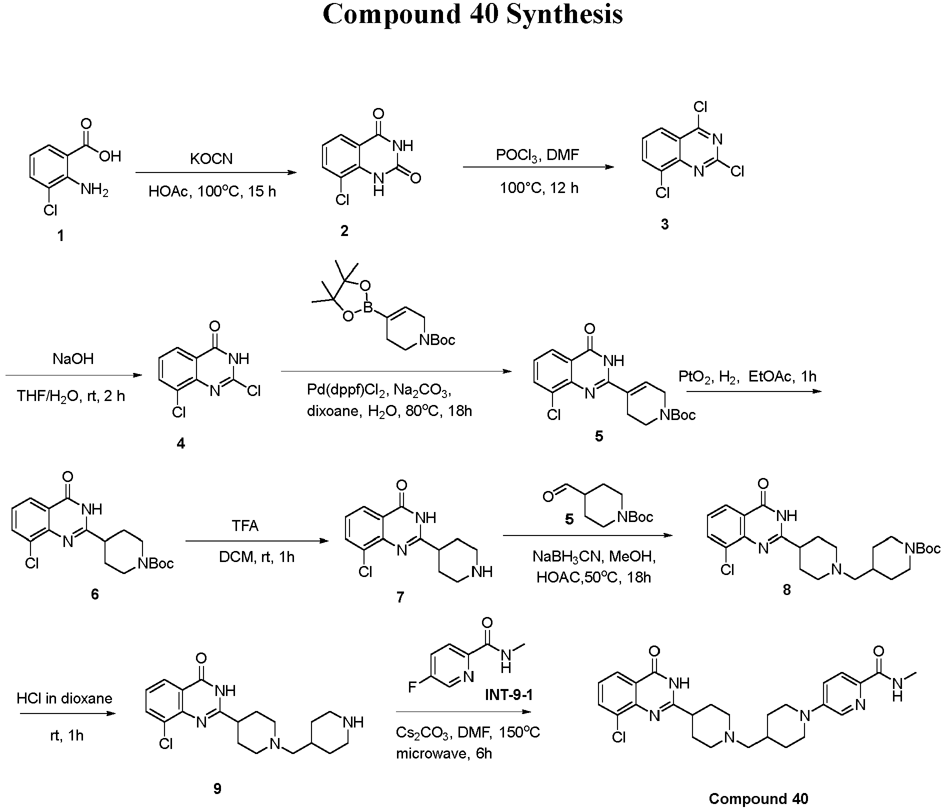

### Preparation of 8-chloroquinazoline-2,4(1H,3H)-dione (2)

To a solution of 2-amino-3-chlorobenzoic acid 1 (10.00 g, 58.30 mmol) in HOAC (100 mL) was added KOCN (14.19 g, 174.90 mmol) slowly at room temperature. The mixture was heated to 100 °C stirred for 15 hours, cooled to 0 °C, ice-water (30 mL) added. The precipitate was collected by filtration, washed with petroleum ether:EtOAc = 5:1 (100 mL), filtered to provide 8-chloroquinazoline-2,4(1H,3H)-dione 2 (5.00 g, 40 % yield) as a white solid which was used directly in next step without further purification.

LCMS (ESI) calcd for C₈H₅ClN₂O₂ [M + H] ⁺ m/z 197.00, found 196.95.

### Preparation of 2,4,8-trichloroquinazoline (3)

To a solution of 8-chloroquinazoline-2,4(1H,3H)-dione **2** (5.00 g, 25.40 mmol) in POCl₃ (45 mL) was added DMF (3 mL) slowly at room temperature. The mixture was heated to 100 °C for 12 hours. The resulting mixture was diluted with ice water (200 mL) and extracted with EtOAc (400 mL x 3). The combine organic phases were washed with brine, dried over sodium sulfate, concentrated and purified by silica gel column chromatography (eluting with EtOAc/petroleum ether, 100: 0 to 50 :50) to give 2,4,8-trichloroquinazoline **3** (2.00 g, 32 % yield) as a white solid.

LCMS (ESI) calcd for C₈H₃Cl₃N₂ [M + H] ⁺ m/z 232.94, found 232.90.

### Preparation of 2,8-dichloroquinazolin-4(3H)-one (4)

To a solution of 2,4,8-trichloroquinazoline 3 (2.00 g, 8.60 mmol) in THF/H₂O = 1:1 (200 mL) was added NaOH (690 mg, 17.20 mmol). The mixture was stirred at room temperature for 2 h. The reaction mixture was concentrated under reduced pressure at 40 °C to obtain residue. To this was added water and acidified with 1 M aqueous HCl to adjust pH 4 ~ 5 at 0 °C. The mixture was extracted with EtOAc (200 mL x 3). The combined organic phases were washed with brine (100 mL × 3), dried over anhydrous sodium sulfate and concentrated in vacuo. The residue was purified by Flash chromatography (eluent: DCM/MeOH =100: 0 to 95 :5) to afford 2,8-dichloroquinazolin-4(3H)-one 4 (1.60 g, 82% yield) as a white solid.

LCMS (ESI) calcd for C₈H₄Cₗ₂N₂O [M + H] ⁺ m/z 214.97, found 214.95.

### Preparation of tert-butyl 4-(8-chloro-4-oxo-3,4-dihydroquinazolin-2-yl)-3,6-dihydropyridine-1(2H)-carboxylate (5)

To a solution of 2,8-dichloroquinazolin-4(3H)-one **4** (400 mg, 1.86 mmol) in Dioxane/H₂O=10:1 (40 mL) were added tert-butyl 4-(4,4,5,5-tetramethyl-1,3,2-dioxaborolan-2-yl)-3,6-dihydropyridine-1(2H)-carboxylate (690 mg, 2.23 mmol), Pd(dppf)Cl₂ (50 mg, 0.07 mmol) and Na₂CO₃ (591 mg, 5.58 mmol) successively at room temperature. The reaction mixture was stirred at 80 °C for 18 h under N₂. The mixture was diluted with water (50 mL) and extracted with EtOAc (100 mL × 3). The combined organic layer was washed with brine (100 mL × 2), dried over Na₂SO₄, filtered and concentrated in vacuo to get crude product, which was purified by flash column chromatography (PE/EtOAc = 100: 0 to 20: 80) to afford tert-butyl 4-(8-chloro-4-oxo-3,4-dihydroquinazolin-2-yl)-3,6-dihydropyridine-1(2H)-carboxylate 5 (550 mg, 77% yield) as a yellow solid.

LCMS (ESI) calcd for C₁₈H₂₀ClN₃O₃ [M + H] ⁺ m/z 362.12, found 362.10.

### Preparation of tert-butyl 4-(8-chloro-4-oxo-3,4-dihydroquinazolin-2-yl)piperidine-1-carboxylate (6)

To a solution of tert-butyl 4-(8-chloro-4-oxo-3,4-dihydroquinazolin-2-yl)-3,6-dihydropyridine-1(2H)-carboxylate **5** (550 mg, 1.52 mmol) in EtOAc (200 mL) was added PtO₂ (110 mg). The mixture was evacuated and backfilled with hydrogen three times and then charged with hydrogen. The resulting mixture was stirred at room temperature for 1 hour. Then the mixture was filtered through celite and concentrated under vacuum to give crude product, which was purified by flash column chromatography (PE/EtOAc = 100: 0 to 50: 50) to afford tert-butyl 4-(8-chloro-4-oxo-3,4-dihydroquinazolin-2-yl)piperidine-1-carboxylate 6 (400 mg, 65 % yield) as a yellow solid.

LCMS (ESI) calcd for C₁₈H₂₂ClN₃O₃ [M + H] ⁺ m/z 364.13, found 364.10.

### Preparation of 8-chloro-2-(piperidin-4-yl)quinazolin-4(3H)-one (7)

To a solution of tert-butyl 4-(8-chloro-4-oxo-3,4-dihydroquinazolin-2-yl)piperidine-1-carboxylate **6** (400 mg, 1.21 mmol) in DCM (5 mL) was added TFA (2 mL), stirred for 1 h at room temperature, the mixture was concentrated under reduced pressure to give crude product, ice-water (2 mL) added then adjusted pH > 7 using ammonium hydroxide solution, precipitate formed, filtered, the filter cake washed with ice-water (5 mL*3), then dried to give product of 8-chloro-2-(piperidin-4-yl)quinazolin-4(3H)-one 7 (300 mg, 89 % yield) as a white solid.

LCMS (ESI) calcd for C₁₃H₁₄ClN₃O [M + H] ⁺ m/z 264.08, found 264.10.

### Preparation of tert-butyl 4-((4-(8-chloro-4-oxo-3,4-dihydroquinazolin-2-yl)piperidin-1-yl)methyl)piperidine-1-carboxylate (8)

To a solution of 8-chloro-2-(piperidin-4-yl)quinazolin-4(3H)-one 7 (300 mg, 1.14 mmol) in MeOH (60 mL) was added tert-butyl 4-oxopiperidine-1-carboxylate (727 mg, 3.41 mmol), then two drops of acetic acid and NaBH₃CN (428 mg, 6.82 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 90 : 10) to give tert-butyl 4-((4-(8-chloro-4-oxo-3,4-dihydroquinazolin-2-yl)piperidin-1-yl)methyl)piperidine-1-carboxylate **8** (300 mg, 51% yield ) as a white solid.

LCMS (ESI) calcd for C₂₄H₃₃ClN₄O₃ [M + H] ⁺ m/z 361.17, found 361.05.

### Preparation of 8-chloro-2-(1-(piperidin-4-ylmethyl)piperidin-4-yl)quinazolin-4(3H)-one (9)

Tert-butyl 4-((4-(8-chloro-4-oxo-3,4-dihydroquinazolin-2-yl)piperidin-1-yl)methyl)piperidine-1-carboxylate **8** (300 mg, 0.65 mmol) was added to HCl in dioxane (4 M, 30 mL), stirred for 1 h at room temperature, the reaction mixture was concentrated under reduced pressure to give 8-chloro-2-(1-(piperidin-4-ylmethyl)piperidin-4-yl)quinazolin-4(3H)-one **9** (200 mg, 81% yield ) as a white solid.

LCMS (ESI) calcd for C₁₉H₂₅ClN₄O [M + H] ⁺ m/z 361.17, found 361.05.

### Preparation of 5-(4-((4-(8-chloro-4-oxo-3,4-dihydroquinazolin-2-yl)piperidin-1-yl)methyl)piperidin-1-yl)-N-methylpicolinamide (Compound 40)

To a solution 8-chloro-2-(1-(piperidin-4-ylmethyl)piperidin-4-yl)quinazolin-4(3H)-one 9 (150 mg, 0.42 mmol) in DMF (10 mL) were added Cs₂CO₃ (1.35 g, 4.16 mmol) and 5-fluoro-N-methylpicolinamide **INT-9-1** (160 mg, 1.03 mmol). The mixture was stirred for 6 h under 150 °C using microwave. The reaction mixture was concentrated under reduced pressure. The residue was purified by flash chromatography (eluting with DCM/MeOH = 100 : 0 to 90 : 10) to give crude 5-(4-((4-(8-chloro-4-oxo-3,4-dihydroquinazolin-2-yl)piperidin-1-yl)methyl)piperidin-1-yl)-N-methylpicolinamide **Compound 40** as a yellow solid. The crude product was purified by prep-HPLC (Gemini 5um C18 150 × 21.2 mm, mobile phase: ACN - H₂O (0.1% FA), gradient: 20 - 80) to give 5-(4-((4-(8-chloro-4-oxo-3,4-dihydroquinazolin-2-yl)piperidin-1-yl)methyl)piperidin-1-yl)-N-methylpicolinamide **Compound 40** (24.2 mg, 96 % purity, 11 % yield) as a white solid.

¹H NMR (400 MHz, DMSO-d₆, ppm) δ 12.39 (s, 1 H), 8.40-8.37 (m, 1 H), 8.26 (d, *J= 2.8* Hz, 1 H), 8.04 (dd, *J =* 7.6, 1.2 Hz, 1 H), 7.94 (dd, *J =* 7.8, 1.2 Hz, 1 H), 7.81 (d, *J =* 8.8 Hz, 1 H), 7.46-7.36 (m, 2 H), 3.91 (d, *J =* 12.8Hz, 2 H), 2.97-2.94 (m, 2 H), 2.86-2.81 (m, 2 H), 2.77 (d, *J= 4.8* Hz, 3 H), 2.65-2.53 (m, 1 H), 2.18 (d, *J=* 6.8 Hz, 2 H), 1.97-1.80 (m, 9 H), 1.23-1.17 (m, 2 H).

LCMS (ESI) calcd for C₂₆H₃₁ClN₆O₂ [M + H] ⁺ m/z 495.22, found 495.15.

### Example 10 - Synthesis of Compound 52 (an embodiment of the invention)

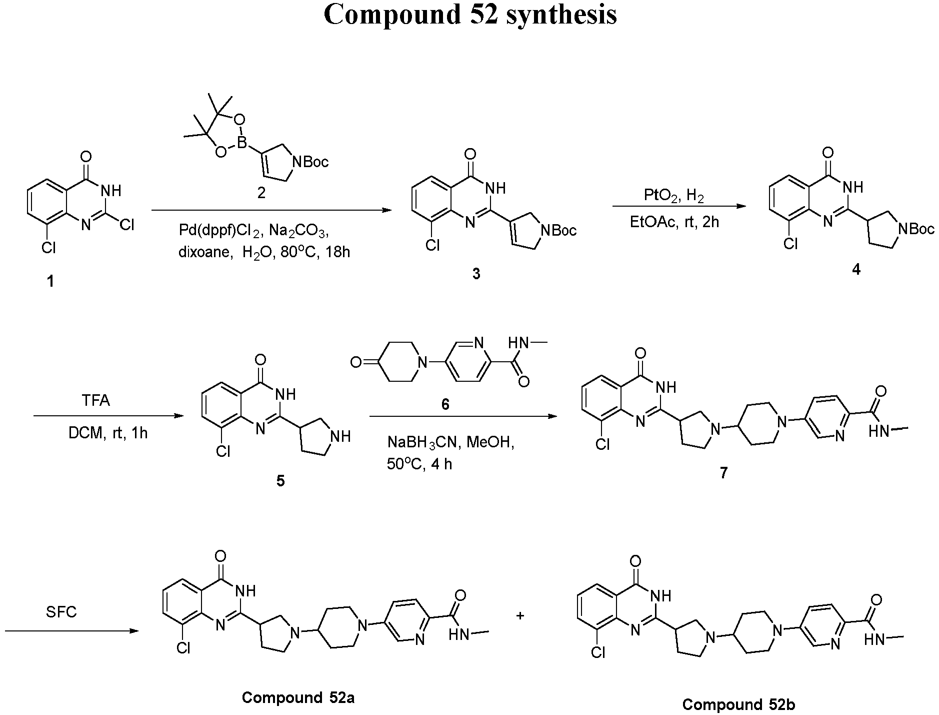

### Preparation of tert-butyl 3-(8-chloro-4-oxo-3,4-dihydroquinazolin-2-yl)-2,5-dihydro-1H-pyrrole-1-carboxylate (3)

To a solution of 2,8-dichloroquinazolin-4(3H)-one **1** (500 mg, 2.35 mmol) in 1,4-dioxane/H₂O=5:1 (60 mL) was added tert-butyl 3-(4,4,5,5-tetramethyl-1,3,2-dioxaborolan-2-yl)-2,5-dihydro-1H-pyrrole-1-carboxylate **2** (900 mg, 3.00 mmol), Pd(dppf)Cl₂ (170 mg, 0.23 mmol) and Na₂CO₃ (740 mg, 6.98 mmol) under N₂. The reaction mixture was stirred at 80 °C for 18 h. After cooling to room temperature, the reaction mixture was concentrated under reduced pressure. The residue was purified by flash chromatography (eluting with DCM/MeOH = 100 : 0 to 91 : 9) to give tert-butyl 3-(8-chloro-4-oxo-3,4-dihydroquinazolin-2-yl)-2,5-dihydro-1H-pyrrole-1-carboxylate **3** (400 mg, 40% yield ) as a white solid.

LCMS (ESI) calcd for C₁₇H₁₈ClN₃O₃ [M + H] ⁺ m/z 348.10, found 348.17.

### Preparation of tert-butyl 3-(8-chloro-4-oxo-3,4-dihydroquinazolin-2-yl)pyrrolidine-1-carboxylate (4)

To a solution of tert-butyl 3-(8-chloro-4-oxo-3,4-dihydroquinazolin-2-yl)-2,5-dihydro-1H-pyrrole-1-carboxylate **3** (400 mg, 1.15 mmol) in EtOAc (50 mL) was added PtO₂ (80 mg, 0.35 mmol) under H₂. The reaction mixture stirred for 2h at room temperature. The mixture was filtered through a Celite pad, and the filtrate was concentrated to give crude product of tert-butyl 3-(8-chloro-4-oxo-3,4-dihydroquinazolin-2-yl)pyrrolidine-1-carboxylate **4** (200 mg, 50 % yield ) as a white solid.

LCMS (ESI) calcd for C₁₇H₂₀ClN₃O₃ [M + H] ⁺ m/z 350.12, found 350.18.

### Preparation of 8-chloro-2-(pyrrolidin-3-yl)quinazolin-4(3H)-one (5)

Tert-butyl 3-(8-chloro-4-oxo-3,4-dihydroquinazolin-2-yl)pyrrolidine-1-carboxylate **4** (200 mg, 0.57 mmol) was added to HCl in dioxane (4 M, 5 mL), stirred for 1 h at room temperature, the reaction mixture was concentrated under reduced pressure to give product of 8-chloro-2-(pyrrolidin-3-yl)quinazolin-4(3H)-one **5** (130 mg, 82% yield) as a white solid.

LCMS (ESI) calcd for C₁₂H₁₂ClN₃O [M + H] ⁺ m/z 250.07, found 250.12.

### Preparation of 5-(4-(3-(8-chloro-4-oxo-3,4-dihydroquinazolin-2-yl)pyrrolidin-1-yl)piperidin-1-yl)-N-methylpicolinamide (7)

To a solution of 8-chloro-2-(pyrrolidin-3-yl)quinazolin-4(3H)-one **5** (80 mg, 0.32 mmol) in MeOH (10 mL) was added N-methyl-5-(4-oxopiperidin-1-yl)picolinamide **6** (50 mg, 0.21 mmol), then three drops of acetic acid and NaBH₃CN (15 mg, 0.24 mmol) were added at room temperature. The reaction mixture stirred at 50 °C for 4 h. After cooling to room temperature, the reaction mixture was concentrated under reduced pressure. The residue was purified by flash chromatography (eluting with DCM/MeOH = 100 : 0 to 92 : 8) to give 5-(4-(3-(8-chloro-4-oxo-3,4-dihydroquinazolin-2-yl)pyrrolidin-1-yl)piperidin-1-yl)-N-methylpicolinamide **7** (40 mg, 40% yield ) as a white solid.

LCMS (ESI) calcd for C₂₄H₂₇ClN₆O₂ [M + H] ⁺ m/z 467.19, found 467.28.

### Preparation of 5-(4-(3-(8-chloro-4-oxo-3,4-dihydroquinazolin-2-yl)pyrrolidin-1-yl)piperidin-1-yl)-N-methylpicolinamide (Compound 52a and Compound 52b)

5-(4-(3-(8-chloro-4-oxo-3,4-dihydroquinazolin-2-yl)pyrrolidin-1-yl)piperidin-1-yl)-N-methylpicolinamide **7** was separated by SFC (Column: DAICEL AS-H 4.6mmI.D.*250mmL 5µm; Mobile phase: CO2/IPA[0.1%NH3(7M Solution in MeOH)]=60/40) and concentrated under reduced pressure to afford the first fraction as **Compound 52a** (14.4 mg, 97% purity, ee%: 100, white solid) and the second fraction as **Compound 52b** (15.4 mg, 99% purity, ee%: 100, white solid)

### Compound 52a

¹H NMR (400 MHz, DMSO) δ 8.42 (d, *J=* 4.8 Hz, 1 H), 8.27 (d, *J=* 2.8 Hz, 1 H), 8.05 (d, *J=* 8.0 Hz, 1 H), 7.94 (d, *J=* 8.0 Hz, 1 H), 7.82 (d, *J=* 8.8 Hz, 1 H), 7.48-7.38 (m, 2 H), 3.83 (d, *J* = 12.8 Hz, 2 H), 3.40-3.32 (m, 1 H), 3.06 (t, *J* = 9.2 Hz, 1 H), 2.95 (dd, *J=* 22.0, 12.0 Hz, 3 H), 2.80-2.68 (m, 5 H), 2.38 (t, *J=* 10.4 Hz, 1 H), 2.24-2.15 (m, 2 H), 1.96 (d, *J= 11.2* Hz, 2 H), 1.53 (dd, *J=* 22.8, 10.8 Hz, 2 H).

LCMS (ESI) calcd for C24H27ClN6O2 [M + H] ⁺ m/z 467.19, found 467.28.

### Compound 52b

¹H NMR (400 MHz, DMSO) δ 8.49 (d, *J* = 4.8 Hz, 1 H), 8.34 (d, *J=* 2.4 Hz, 1 H), 8.11 (d, *J* = 7.6 Hz, 1 H), 8.01 (d, *J=* 7.6 Hz, 1 H), 7.89 (d, *J=* 8.8 Hz, 1 H), 7.5 5-7.45 (m, 2 H), 3.90 (d, *J = 12.4* Hz, 2 H), 3.45-3.39 (m, 1 H), 3.13 (t, *J=* 8.8 Hz, 1 H), 3.01 (dd, *J=* 22.8, 11.6 Hz, 3 H), 2.87-2.77 (m, 5 H), 2.43 *(d, J=* 10.0 Hz, 1 H), 2.26 (d, *J =* 6.4 Hz, 2 H), 2.02 (d, *J=* 11.2 Hz, 2 H), 1.59 (d, *J=* 12.0 Hz, 2 H).

LCMS (ESI) calcd for C24H27ClN6O2 [M + H] ⁺ m/z 467.19, found 467.28.

### Example 11 - Synthesis of Compound 54 (a reference example, not in accordance with the present claims)

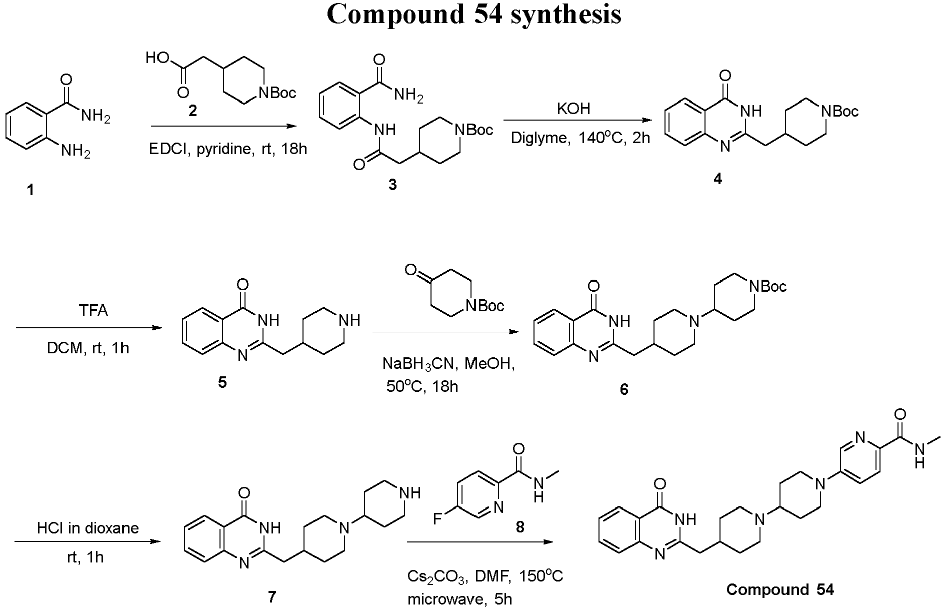

### Preparation of tert-butyl 4-{[(2-carbamoylphenyl)carbamoyl]methyl}piperidine-1-carboxylate (3)

To a solution of 2-aminobenzamide 1 (2.00 g, 14.70 mmol) and {1-[(tert-butoxy)carbonyl]piperidin-4-yl}acetic acid 2 (3.58 g, 14.70 mmol) in pyridine (50 mL) was added EDCI (2. 82 g, 14.70 mmol). then the mixture was stirred for 18 h at room temperature. The reaction mixture was quenched with water, the aqueous layer was extracted with EtOAc (50 mL x 3). The combined organic layers was washed by 1M HCl solution and brine, dried over Na₂SO₄, concentrated under reduced pressure to give tert-butyl 4-{[(2-carbamoylphenyl)carbamoyl]methyl}piperidine-1-carboxylate **3** (5.76 g, 92% yield) as a yellow oil.

LCMS (ESI) calcd for C19H27N304 [M + H] ⁺ m/z 362.20, found 262.30.

### Preparation of tert-butyl 4-[(4-oxo-3H-quinazolin-2-yl)methyl]piperidine-1-carboxylate (4)

To the solution of tert-butyl 4-{[(2-carbamoylphenyl)carbamoyl]methyl}piperidine-1-carboxylate **3** (5.76 g, 15.90 mmol) in Diglyme (40 mL) was added KOH (1.16 g, 20.67 mmol), heated to 140 °C stirred for 2 h, cooled to 0 °C, ice-water (50 mL) added then adjusted pH < 7 using 1M HCl solution, precipitate formed, filtered, the filter cake washed with ice-water (50 mL*3), then dried to give product of tert-butyl 4-[(4-oxo-3H-quinazolin-2-yl)methyl]piperidine-1-carboxylate **4** (4.20 g, 73% yield) as a white solid.

LCMS (ESI) calcd for C19H25N303 [M + H] ⁺ m/z 344.19, found 344.25.

### Preparation of 2-(piperidin-4-ylmethyl)-3H-quinazolin-4-one (5)

To a solution of tert-butyl 4-[(4-oxo-3H-quinazolin-2-yl)methyl]piperidine-1-carboxylate **4** (4.20 g, 10.00 mmol) in DCM (13 mL) was added TFA (13 mL) , stirred for 1 h at room temperature, the mixture was concentrated under reduced pressure to give crude product, ice-water (20 mL) added then adjusted pH > 7 using ammonium hydroxide solution, precipitate formed, filtered, the filter cake washed with ice-water (50 mL*3), then dried to give product of 2-(piperidin-4-ylmethyl)-3H-quinazolin-4-one **5** (2.32 g, 90% yield) as a white solid.

LCMS (ESI) calcd for C14H17N30 [M + H] ⁺ m/z 244.14, found 244.25.

### Preparation of tert-butyl 4-{4-[(4-oxo-3H-quinazolin-2-yl)methyl]piperidin-1-yl}piperidine-1-carboxylate (6)

To a solution of 2-(piperidin-4-ylmethyl)-3H-quinazolin-4-one **5** (500 mg, 2.06 mmol) in MeOH (50 mL) was added tert-butyl 4-oxopiperidine-1-carboxylate (532 mg, 2.67 mmol), then two drops of acetic acid and NaBH₃CN (504 mg, 8.01 mmol) were added at room temperature. The reaction mixture stirred at 50 °C for 18 h. After cooling to room temperature, the reaction mixture was concentrated under reduced pressure. The residue was purified by flash chromatography (eluting with DCM/MeOH = 100 : 0 to 91 : 9) to give tert-butyl 4- { 4-[(4-oxo-3H-quinazolin-2-yl)methyl]piperidin-1-yl}piperidine-1-carboxylate 6 (244 mg, 25% yield ) as a white solid.

LCMS (ESI) calcd for C24H34N4O3 [M + H] ⁺ m/z 427.26, found 427.40.

### Preparation of tert-butyl 4-[(4-oxo-3H-quinazolin-2-yl)methyl]piperidine-1-carboxylate (7)

Tert-butyl 4-{4-[(4-oxo-3H-quinazolin-2-yl)methyl]piperidin-1-yl}piperidine-1-carboxylate **6** (244 mg, 0.57 mmol) was added to HCl in dioxane (4 M, 8 mL), stirred for 1 h at room temperature, the reaction mixture was concentrated under reduced pressure to give 2-{[1-(piperidin-4-yl)piperidin-4-yl]methyl}-3H-quinazolin-4-one **7** (187 mg, 90% yield ) as a white solid.

LCMS (ESI) calcd for C19H26N40 [M + H] ⁺ m/z 327.21, found 327.44.

### Preparation of N-methyl-5-(4-{4-[(4-oxo-3H-quinazolin-2-yl)methyl]piperidin-1-yl}piperidin-1-yl)pyridine-2-carboxamide (Compound 54)

To a solution of 2-{[1-(piperidin-4-yl)piperidin-4-yl]methyl}-3H-quinazolin-4-one 7 (200 mg, 0.61 mmol) in DMF (16 mL) were added Cs₂CO₃ (1.20 g, 3.68 mmol) and 5-fluoro-N-methylpyridine-2-carboxamide **5** (208 mg, 1.35 mmol). The mixture was stirred for 5 h under 150 °C using microwave. The reaction mixture was concentrated under reduced pressure. The residue was purified by flash chromatography (eluting with DCM/MeOH = 100 : 0 to 91 : 9) to give N-methyl-5-(4-{4-[(4-oxo-3H-quinazolin-2-yl)methyl]piperidin-1-yl}piperidin-1-yl)pyridine-2-carboxamide **Compound 54** (20.11 mg, 99% purity, 7% yield) as a white solid.

¹H NMR (400 MHz, DMSO) δ 12.16 (s, 1 H), 8.37 (d, *J= 4.8* Hz, 1 H), 8.25 (d, *J= 2.8* Hz, 1 H), 8.07 (d, *J =* 8.0 Hz, 1 H), 7.83-7.74 (m, 2 H), 7.59 (d, *J =* 8.0 Hz, 1 H), 7.45 (t, *J=* 7.6 Hz, 1 H), 7.37 (dd, *J= 8.8,* 2.8 Hz, 1 H), 3.93 (d, *J =* 12.4 Hz, 2 H), 2.90-2.74 (m, 8 H), 2.14 (t, *J* = 10.8 Hz, 2 H), 1.81 (d, *J =* 11.6 Hz, 3 H), 1.64 (d, *J =* 10.8 Hz, 2 H), 1.49 (d, *J =* 8.8 Hz, 2 H), 1.23 (d, *J = 9.2* Hz, 3 H).

LCMS (ESI) calcd for C₂₄H₂₈N₆O₂ [M + H] ⁺ m/z 461.26, found 461.40.

### Example 12 - Synthesis of Compound 136rac, 136a and 136b (embodiments of the invention)

### Synthesis of Compound 136rac, 136a and 136b

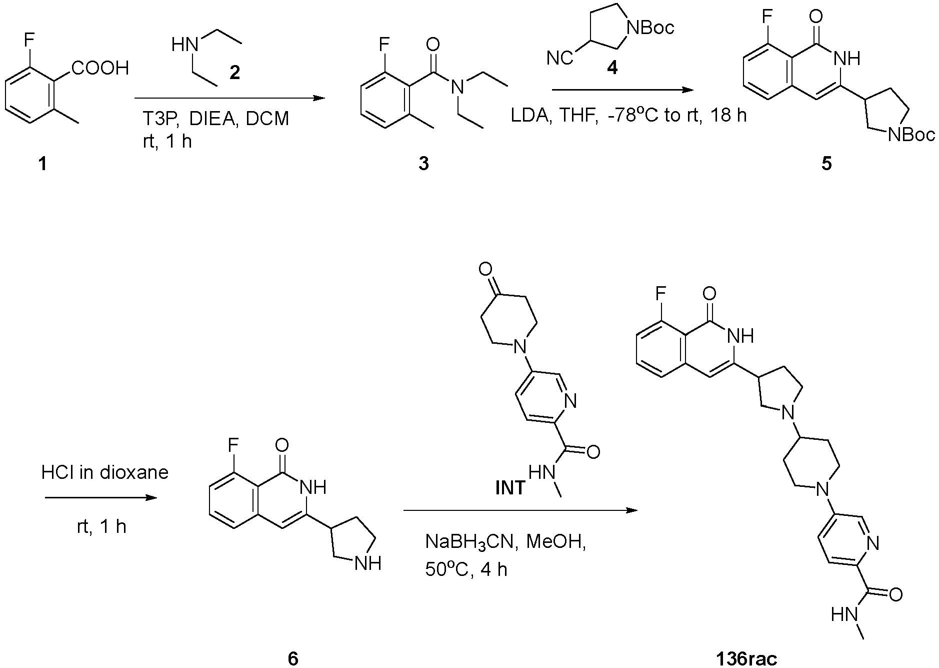

### Preparation of N,N-diethyl-2-fluoro-6-methylbenzamide (3)

To a solution of 2-fluoro-6-methylbenzoic acid 1 (1.00 g, 6.50 mmol) and diethylamine 2 (0.57 g, 7.80 mmol) in DCM (50 mL) was added tritolylphosphine (50% in EtOAc) (3.10 g, 9.75 mmol) and DIEA (4.20 g, 32.50 mmol) at room temperature. The mixture was stirred for 1 h at room temperature. The reaction mixture was concentrated under reduced pressure. The residue was purified by flash chromatography (eluting with PE/EtOAC = 100 : 0 to 90 : 10) to give N,N-diethyl-2-fluoro-6-methylbenzamide 3 (0.90 g, 63% yield ) as a white solid.

LCMS (ESI) calcd for C12H16FNO [M + H] ⁺ m/z 210.12, found 210.09.

### Preparation of 3-hydroxy-1-(1-methylphenyl)pyrrolidine-3-carboxylic acid (5)

A dry three-necked round-bottomed flask with an addition funnel at -78°C under inert atmosphere was charged with anhydrous THF (30 ml). A solution of LDA (2.5 M in THF, 0.9 ml) was added dropwise. The internal temperature was maintained below -70°C during the entire addition process. After 0.5 h at -78°C a solution of N,N-diethyl-2-fluoro-6-methylbenzamide **3** (300 mg, 1.43 mmol) in anhydrous THF (10 ml) was added dropwise and the mixture was stirred for 1 h, then tert-butyl 3-cyanopyrrolidine-1-carboxylate **4** (366 mg, 1.86 mmol) was added at -78°C. The mixture was stirred for 18 h at room temperature. The reaction mixture was concentrated under reduced pressure. The residue was purified by flash chromatography (eluting with PE/EtOAC = 100 : 0 to 80 : 20) to give 3-hydroxy-1-(1-methylphenyl)pyrrolidine-3-carboxylic acid 5 (160 mg, 32% yield ) as a white solid.

LCMS (ESI) calcd for C18H21FN2O3 [M + H] ⁺ m/z 333.15, found 333.25.

### Preparation of 8-fluoro-3-(pyrrolidin-3-yl)-2H-isoquinolin-1-one (6)

3-Hydroxy-1-(1-methylphenyl)pyrrolidine-3-carboxylic acid **5** (160 mg, 0.48 mmol) was added to HCl-dioxane (4M, 10 mL). The mixture was stirred for 1 h at room temperature. The reaction mixture was concentrated under reduced pressure to give 8-fluoro-3-(pyrrolidin-3-yl)-2H-isoquinolin-1-one 6 (120 mg, 97% yield) as a white solid.

LCMS (ESI) calcd for C13H13FN20 [M + H] ⁺ m/z 233.10, found 233.12.

### Preparation of 5-{4-[3-(8-fluoro-1-oxo-2H-isoquinolin-3-yl)pyrrolidin-1-yl]piperidin-1-yl}-N-methylpyridine-2-carboxamide (Compound 136rac)

To a solution of 8-fluoro-3-(pyrrolidin-3-yl)-2H-isoquinolin-1-one **6** (120 mg, 0.52 mmol) in MeOH (15 mL) was added N-methyl-5-(4-oxopiperidin-1-yl)pyridine-2-carboxamide **INT** (100 mg, 0.55 mmol), then two drops of acetic acid and NaBH₃CN (30 mg, 0.46 mmol) were added at room temperature. The reaction mixture stirred at 50 °C for 4 h. After cooling to room temperature, the reaction mixture was concentrated under reduced pressure. The residue was purified by flash chromatography (eluting with DCM/MeOH = 100 : 0 to 92 : 8) to give 5-{4-[3-(8-fluoro-1-oxo-2H-isoquinolin-3-yl)pyrrolidin-1-yl]piperidin-1-yl}-N-methylpyridine-2-carboxamide **Compound 136rac** (65 mg, 25% yield, 99% purity) as a yellow solid.

### Compound 136rac

¹H NMR (400 MHz, DMSO) δ 11.10 (s, 1 H), 8.41-8.38 (m, 1 H), 8.28 (d, *J =* 2.8 Hz, 1 H), 7.83 (d, *J=* 8.8 Hz, 1 H), 7.62-7.58 (m, 1 H), 7.45-7.36 (m, 2 H), 7.10 (dd, *J=* 11.6, 8.0 Hz, 1 H), 6.46 (d, *J= 1.6* Hz, 1 H), 3.84-3.80 (m, 2 H), 3.23-3.16 (m, 1 H), 3.00-2.92 (m, 3 H), 2.84-2.65 (m, 6 H), 2.38-2.33 (m, 1 H), 2.24-2.18 (m, 1 H), 1.97-1.97 (m, 2 H), 1.84-1.76 (m, 1 H), 1.60-1.49 (m, 2 H).

LCMS (ESI) calcd for C25H28FN5O2 [M + H] ⁺ m/z 450.22, found 450.30.

### Preparation of 5-{4-[3-(8-fluoro-1-oxo-2H-isoquinolin-3-yl)pyrrolidin-1-yl]piperidin-1-yl}-N-methylpyridine-2-carboxamide (Compound 136a and Compound 136b)

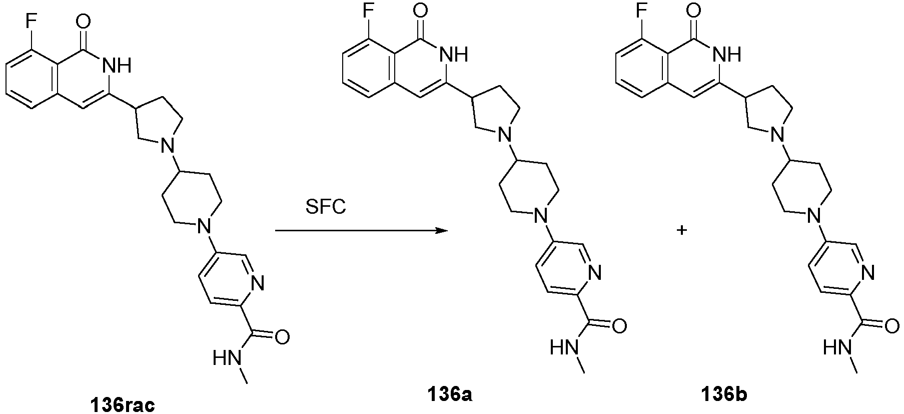

5-{4-[3-(8-fluoro-1-oxo-2H-isoquinolin-3-yl)pyrrolidin-1-yl]piperidin-1-yl}-N-methylpyridine-2-carboxamide **136rac** was separated by SFC (Column: Daicel CHIRALPAK OJ-H 250 mm × 20 mm I.D., 5 µmm; Mobile phase: CO₂/MeOH[0.1%(NH₃)] = 70/30) and concentrated under reduced pressure to afford the first fraction as **Compound 136a** (12.8 mg, 99% purity, ee%: 100, white solid) and the second fraction as **Compound 136b** (13.8 mg, 99% purity, ee%: 97, white solid)

### Compound 136a

¹H NMR (400 MHz, DMSO) δ 11.10 (s, 1 H), 8.41 (q, *J* = 4.4 Hz, 1 H), 8.28 (d, *J* = 2.8 Hz, 1 H), 7.83 (d, *J=* 8.8 Hz, 1 H), 7.62-7.58 (m, 1 H), 7.42-7.36 (m, 2 H), 7.10 (dd, *J =* 12.0, 8.0 Hz, 1 H), 6.46 (s, 1 H), 3.84-3.80 (m, 2 H), 3.22-3.15 (m, 1 H), 3.00-2.90 (m, 3 H), 2.81-2.63 (m, 6 H), 2.36-2.31 (m, 1 H), 2.23-2.18 (m, 1 H), 1.97-1.93 (m, 2 H), 1.87-1.79 (m, 1 H), 1.56-1.52 (m, 2 H).

LCMS (ESI) calcd for C25H28FN5O2 [M + H] ⁺ m/z 450.22, found 450.20.

### Compound 136b

¹H NMR (400 MHz, DMSO) δ 11.10 (s, 1 H), 8.40 (q, *J= 4.8* Hz, 1 H), 8.28 (d, *J* = 2.8 Hz, 1 H), 7.82 (d, *J = 8.8* Hz, 1 H), 7.61-7.58 (m, 1 H), 7.42-7.36 (m, 2 H), 7.10 (dd, *J =* 11.6, 8.0 Hz, 1 H), 6.46 (s, 1 H), 3.84-3.80 (m, 2 H), 3.22-3.15 (m, 1 H), 3.00-2.90 (m, 3 H), 2.81-2.63 (m, 6 H), 2.36-2.31 (m, 1 H), 2.25-2.18 (m, 1 H), 1.97-1.94 (m, 2 H), 1.87-1.79 (m, 1H), 1.56-1.52 (m, 2 H).

LCMS (ESI) calcd for C25H28FN5O2 [M + H] ⁺ m/z 450.22, found 450.25.

### Example 13 - Synthesis of Compounds 151rac, 151a and 151b (a reference example, not in accordance with the present claims)

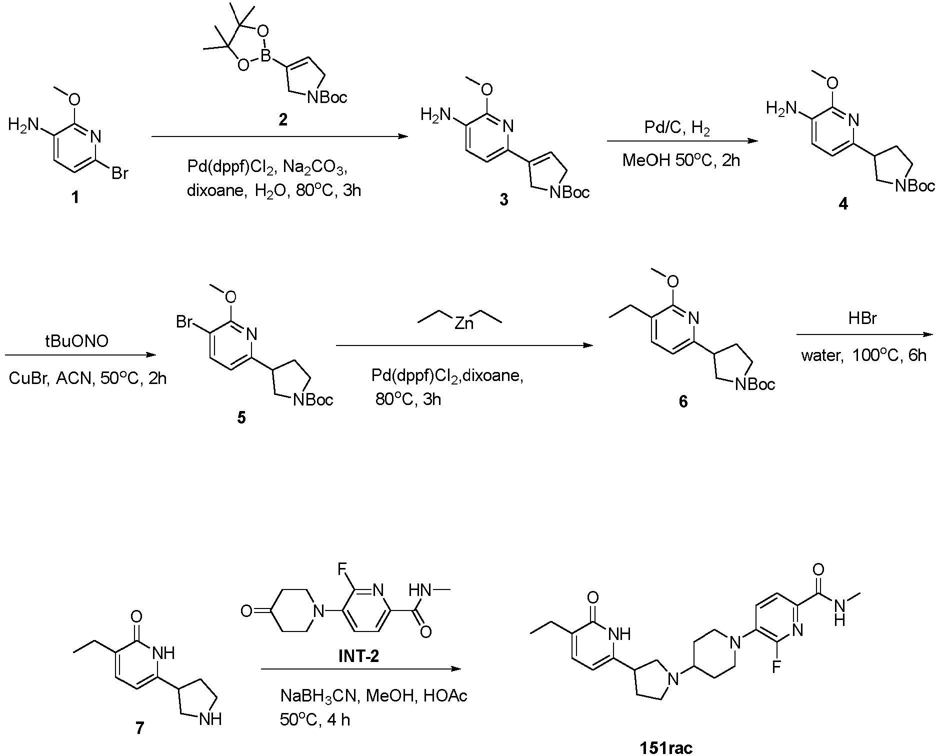

### Preparation of tert-butyl 3-(5-amino-6-methoxypyridin-2-yl)-2,5-dihydropyrrole-1-carboxylate (3)

To a solution of 6-bromo-2-methoxypyridin-3-amine **1** (1.38 g, 6.80 mmol) in 1,4-dioxane/H₂O=4:1 (20 mL) was added tert-butyl 3-(4,4,5,5-tetramethyl-1,3,2-dioxaborolan-2-yl)-2,5-dihydropyrrole-1-carboxylate **2** (2.01 g, 6.80 mmol), then Pd(dppf)Cl₂ (0.50 g, 0.68 mmol) and Na₂CO₃ (2.23 g, 21.08 mmol) were added at room temperature. The reaction mixture was stirred under nitrogen at 80 °C for 3 h under N₂. After cooling to room temperature, the reaction mixture was concentrated under reduced pressure. The residue was purified by flash chromatography (eluting with PE/EA = 100 : 0 to 85 : 15) to give tert-butyl 3-(5-amino-6-methoxypyridin-2-yl)-2,5-dihydropyrrole-1-carboxylate **3** (1.50 g, 72% yield) as a white solid.

LCMS (ESI) calcd for C15H21N303 [M + H] ⁺ m/z 292.16, found 292.19.

### Preparation of tert-butyl 3-(5-amino-6-methoxypyridin-2-yl) pyrrolidine-1-carboxylate (4)

To a solution of tert-butyl 3-(5-amino-6-methoxypyridin-2-yl)-2,5-dihydropyrrole-1-carboxylate **3** (1.50 g, 5.10 mmol) in MeOH (20 mL) was added Pd/C (0.54 g, 5.10 mmol) at room temperature. The reaction mixture was stirred under hydrogen at 50 °C for 2 h. After cooling to room temperature, the reaction mixture was filtered, the solution was concentrated under reduced pressure to give product of tert-butyl 3-(5-amino-6-methoxypyridin-2-yl)pyrrolidine-1-carboxylate **4** (1.20 g, 76% yield ) as a white solid.

LCMS (ESI) calcd for C15H23N303 [M + H] ⁺ m/z 294.17, found 294.18.

### Preparation of tert-butyl 3-(5-bromo-6-methoxypyridin-2-yl)pyrrolidine-1-carboxylate (5)

To a solution of tert-butyl 3-(5-amino-6-methoxypyridin-2-yl)pyrrolidine-1-carboxylate **4** (1.40 g, 4.80 mmol) in ACN (20 mL) was added tert-Butyl nitrite (1.14 g, 11.04 mmol). Then CuBr (1.38 g, 9.60 mmol) was added at room temperature. The reaction mixture was stirred at 50 °C for 2 h. After cooling to room temperature, the reaction mixture was concentrated under reduced pressure. The residue was purified by flash chromatography (eluting with PE/EA = 100 : 0 to 85 : 15) to give tert-butyl 3-(5-bromo-6-methoxypyridin-2-yl)pyrrolidine-1-carboxylate **5** (0.65 g, 35% yield) as a white solid.

LCMS (ESI) calcd for C15H21BrN203 [M + H] ⁺ m/z 357.07, found 357.11.

### Preparation of tert-butyl 3-(5-ethyl-6-methoxypyridin-2-yl)pyrrolidine-1-carboxylate (6)

To a solution of tert-butyl 3-(5-bromo-6-methoxypyridin-2-yl)pyrrolidine-1-carboxylate **5** (300 mg, 0.84 mmol) in 1,4-dioxane (15 mL) was added Pd(dppf)Cl₂ (61 mg, 0.08 mmol). Then Et₂Zn (5 mL, 0.14mml) was added at room temperature. The reaction mixture under nitrogen was stirred at 80 °C for 3 h. After cooling to room temperature, the reaction mixture was concentrated under reduced pressure. The residue was purified by flash chromatography (eluting with PE/EA = 100 : 0 to 85 : 15) to give tert-butyl 3-(5-ethyl-6-methoxypyridin-2-yl)pyrrolidine-1-carboxylate **6** (120 mg, 44% yield ) as a white solid.

LCMS (ESI) calcd for C17H26N203 [M + H] ⁺ m/z 307.19, found 307.25.

### Preparation of 3-ethyl-6-(pyrrolidin-3-yl)-1H-pyridin-2-one (7)

Tert-butyl 3-(5-ethyl-6-methoxypyridin-2-yl)pyrrolidine-1-carboxylate **6** (120 mg, 0.39 mmol) was added to HBr in water (48%, 15 mL). The reaction mixture was stirred at 100 °C for 6 h. After cooling to room temperature, the reaction mixture was concentrated under reduced pressure to give 3-ethyl-6-(pyrrolidin-3-yl)-1H-pyridin-2-one 7 (70 mg, 88% yield) as a white solid.

LCMS (ESI) calcd for C11H16N2O [M + H] ⁺ m/z 193.13, found 193.14.

### Preparation of 5-(4-(3-(5-ethyl-6-oxo-1,6-dihydropyridin-2-yl)pyrrolidin-1-yl)piperidin-1-yl)-6-fluoro-N-methylpicolinamide (151rac)

To a solution of 6-fluoro-N-methyl-5-(4-oxopiperidin-1-yl)picolinamide **INT-2** (100 mg, 0.40 mmol) in MeOH (40 mL) was added 3-ethyl-6-(pyrrolidin-3-yl)-1H-pyridin-2-one **7** (115 mg, 0.60 mmol), then two drops of acetic acid and NaBH₃CN (38 mg, 0.61 mmol) were added at room temperature. The reaction mixture stirred at 50 °C for 12 h. After cooling to room temperature, the reaction mixture was concentrated under reduced pressure. The residue was purified by flash chromatography (eluting with DCM/MeOH = 100 : 0 to 90 : 10) to afford 5-(4-(3-(5-ethyl-6-oxo-1,6-dihydropyridin-2-yl)pyrrolidin-1-yl)piperidin-1-yl)-6-fluoro-N-methylpicolinamide **151rac** (40 mg, 95% purity, 22.34% yield ) as a white solid.

LCMS (ESI) calcd for C₂₃H₃₀FN₅O₂ [M + H] ⁺ m/z 428.24, found 428.10.

### Chiral resolution of 5-(4-(3-(5-ethyl-6-oxo-1,6-dihydropyridin-2-yl)pyrrolidin-1-yl)piperidin-1-yl)-6-fluoro-N-methylpicolinamide (Compound 151a and Compound 151b)

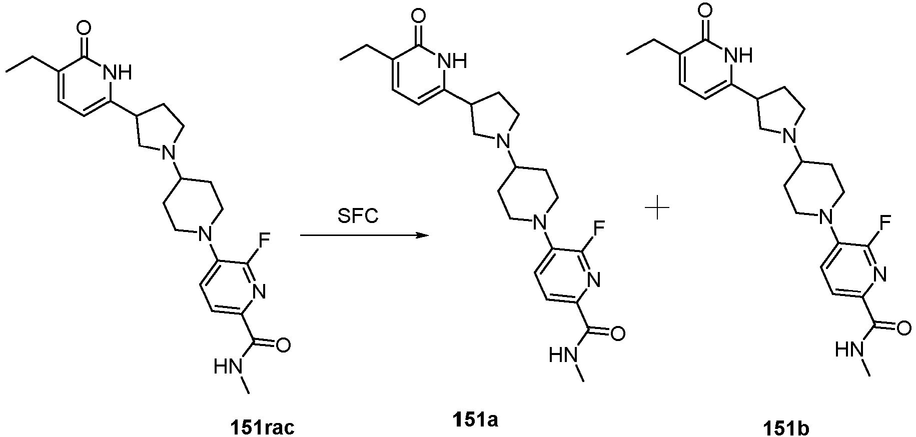

Compound **151** was separated by SFC (Column: Daicel Chiralpak AD-H SFC 250 mm × 20 mm I.D., 5 µmm; Mobile phase: CO₂/MeOH[0.1%(NH₃)] = 80/20) and concentrated under reduced pressure to afford the first fraction as **Compound 151a** (15.8 mg, 92% purity, ee%: 100, white solid) and the second fraction as **Compound 151b** (15.9 mg, 95% purity, ee%: 100, white solid)

### Compound 151a

¹H NMR (400 MHz, DMSO-d₆, ppm) *δ:* 11.28 (s, 1 H), 8.44-8.37 (m, 1 H), 7.83 (dd, *J =* 8.0, 1.2 Hz, 1 H), 7.61-7.52 (m, 1 H), 7.17 (d, *J=* 7.2 Hz, 1 H), 6.03 (d, *J* = 6.4 Hz, 1 H), 3.56-3.43 (m, 2 H), 3.17-3.09 (m, 1 H), 2.90-2.74 (m, 7 H), 2.68-2.60 (m, 2 H), 2.35-2.17 (m, 4 H), 1.96 (d, *J* = 12.0 Hz, 2 H), 1.79-1.70 (m, 1 H), 1.63-1.52 (m, 2 H), 1.06 (t, *J=* 7.2 Hz, 3 H).

LCMS (ESI) calcd for C₂₃H₃₀FN₅O₂ [M + H] ⁺ m/z 428.24, found 428.10.

### Compound 151b

¹H NMR (400 MHz, DMSO-d₆, ppm) *δ:* 11.28 (s, 1 H), 8.45-8.35 (m, 1 H), 7.83 (dd, *J =* 8.0, 1.2 Hz, 1 H), 7.64-7.51 (m, 1 H), 7.17 (d, *J= 6.8* Hz, 1 H), 6.03 (d, *J* = 6.8 Hz, 1 H), 3.58-3.41 (m, 2 H), 3.17-3.09 (m, 1 H), 2.93-2.74 (m, 7 H), 2.68-2.60 (m, 2 H), 2.35-2.16 (m, 4 H), 2.01-1.92 (m, 2 H), 1.79-1.70 (m, 1 H), 1.64-1.52 (m, 2 H), 1.06 (t, *J* = 7.2 Hz, 3 H).

LCMS (ESI) calcd for C₂₃H₃₀FN₅O₂ [M + H] ⁺ m/z 428.24, found 428.20.

### Synthesis of INT-2

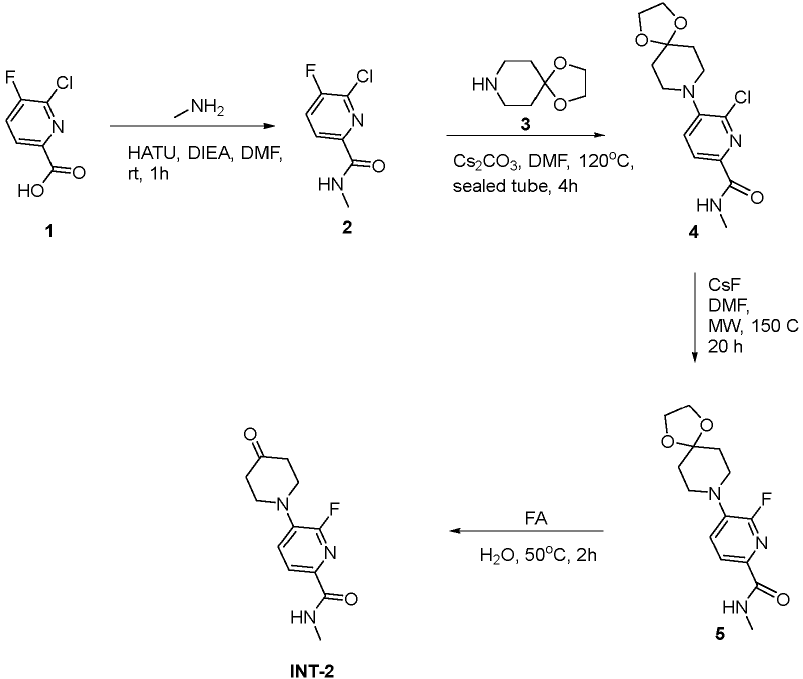

### Preparation of 6-chloro-5-fluoro-N-methylpicolinamide (2)

To a solution of 6-chloro-5-fluoropicolinic acid 1 (2.00 g, 11.40 mmol) in DMF (50 mL) was added methanamine (420 mg, 13.68 mmol), DIEA (4.42 g, 34.20 mmol), HATU (6.50 g, 17.10 mmol) at room temperature successively. The mixture was kept stirring at room temperature for 1 h. The resulting mixture was diluted with water and extracted with EtOAc (200 mL x 3). The combined organic layer was dried over Na₂SO₄ and concentrated under reduced pressure. The residue was purified by flash chromatography (eluting with PE/EtOAc = 100 : 0 to 70 : 30) to afford 6-chloro-5-fluoro-N-methylpicolinamide 2 (2.00 g, 88 % yield ) as a white solid.

LCMS (ESI) calcd for C₇H₆ClFN₂O [M + H] ⁺ m/z 189.02, found 188.90.

### Preparation of 6-chloro-N-methyl-5-(1,4-dioxa-8-azaspiro[4.5]decan-8-yl)picolinamide (4)

To a solution 6-chloro-5-fluoro-N-methylpicolinamide **2** (2.00 g, 10.60 mmol) in DMF (20 mL) was added Cs₂CO₃ (6.91 g, 21.20 mmol) and 1,4-dioxa-8-azaspiro[4.5]decane **3** (3.04 g, 21.20 mmol). The mixture was stirred for 4 h under 120 °C using a sealed tube. The reaction mixture was concentrated under reduced pressure. The residue was purified by flash chromatography (eluting with PE/EtOAC = 100 : 0 to 50 : 50) to afford 6-chloro-N-methyl-5-(1,4-dioxa-8-azaspiro[4.5]decan-8-yl)picolinamide **4** (1.60 g, 42 % yield ) as a white solid. LCMS (ESI) calcd for C₁₄H₁₈ClN₃O₃ [M + H] ⁺ m/z 312.10, found 311.95.

### Preparation of 6-fluoro-N-methyl-5-(1,4-dioxa-8-azaspiro[4.5]decan-8-yl)picolinamide (5)

To a solution 6-chloro-N-methyl-5-(1,4-dioxa-8-azaspiro[4.5]decan-8-yl)picolinamide 4 (300 mg, 0.96 mmol) in DMF (20 mL) was added CsF (293 mg, 1.93 mmol). The mixture was stirred for 20 h under 150 °C using microwave. The reaction mixture was concentrated under reduced pressure. The residue was purified by flash chromatography (eluting with PE/EtOAC = 100 : 0 to 30 : 70) to 6-fluoro-N-methyl-5-(1,4-dioxa-8-azaspiro[4.5]decan-8-yl)picolinamide **5** (200 mg, 69% yield ) as a yellow solid.

LCMS (ESI) calcd for C₁₄H₁₈FN₃O₃ [M + H] ⁺ m/z 296.13, found 295.95.

### Preparation of 6-fluoro-N-methyl-5-(4-oxopiperidin-1-yl)picolinamide (INT-2)

To the solution of 6-fluoro-N-methyl-5-(1,4-dioxa-8-azaspiro[4.5]decan-8-yl)picolinamide **5** (200 mg, 0.68 mmol) in H₂O (3 mL) was added formic acid (2 mL), stirred at 50 °C for 1 h, The aqueous solution was adjusted to pH 7-8 with aqueous NaHCO₃ solution. The mixture was diluted with water (50 mL) and extracted with EtOAc (100 mL × 3). The combined organic layer was washed with brine (100 mL × 2), dried over Na₂SO₄, concentrated to give crude product of 6-fluoro-N-methyl-5-(4-oxopiperidin-1-yl)picolinamide **INT-2** (100 mg, 47% yield) as a yellow solid.

LCMS (ESI) calcd for C₁₂H₁₄FN₃O₂ [M + H] ⁺ m/z 252.11, found 251.90.

### Example 14 - Synthesis of Compounds 154rac, 154a and 154b (embodiments of the invention)

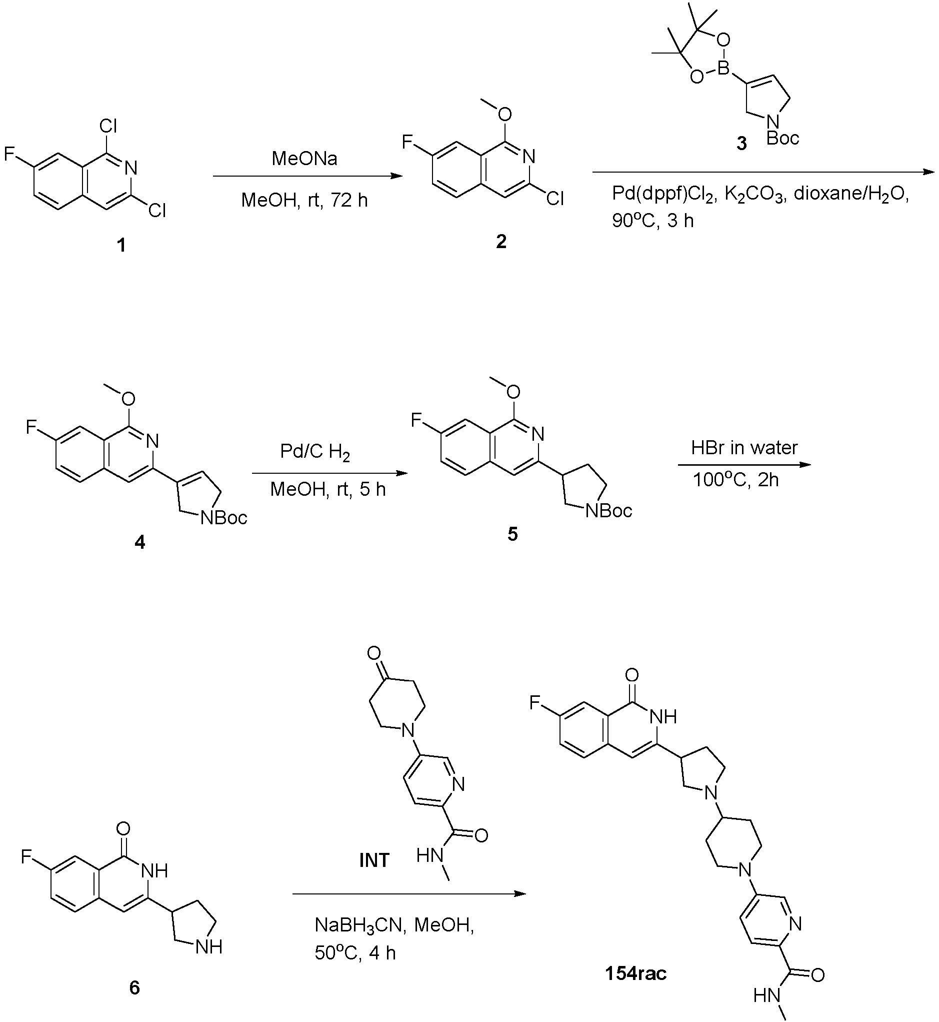

### Preparation of 3-chloro-7-fluoro-1-methoxyisoquinoline (2)

To a solution of 1,3-dichloro-7-fluoroisoquinoline 1 (500 mg, 2.32 mmol) in MeOH (50 mL), was added MeONa (150 mg, 2.78 mmol) at room temperature. The mixture was stirred for 72 h at room temperature. The reaction mixture was concentrated under reduced pressure. The residue was purified by flash chromatography (eluting with PE/EtOAC = 100 : 0 to 98 : 2) to give 3-chloro-7-fluoro-1-methoxyisoquinoline 2 (160 mg, 33% yield ) as a white solid.

LCMS (ESI) calcd for C10H7ClFNO [M + H] ⁺ m/z 212.02, found 211.90.

### Preparation of tert-butyl 3-(7-fluoro-1-methoxyisoquinolin-3-yl)-2,5-dihydro-1H-pyrrole-1-carboxylate (4)

To a solution of 3-chloro-7-fluoro-1-methoxyisoquinoline **2** (160 mg, 0.76 mmol) in dioxane:H₂O=5:1 (20 mL) was added tert-butyl 3-(4,4,5,5-tetramethyl-1,3,2-dioxaborolan-2-yl)-2,5-dihydro-1H-pyrrole-1-carboxylate **3** (270 mg, 0.92 mmol), Pd(dppf)Cl₂ (50 mg, 0.07 mmol) and K₂CO₃ (250 mg, 1.81 mmol) at room temperature. The reaction mixture was stirred under N₂ at 90 °C for 3 h. After cooling to room temperature, the reaction mixture was concentrated under reduced pressure. The residue was purified by flash chromatography (eluting with PE/EtOAc = 100 : 0 to 95 : 5) to afford tert-butyl 3-(7-fluoro-1-methoxyisoquinolin-3-yl)-2,5-dihydro-1H-pyrrole-1-carboxylate 4 (160 mg, 61% yield ) as a white solid.

LCMS (ESI) calcd for C19H21FN203 [M + H] ⁺ m/z 345.15, found 345.11.

### Preparation of tert-butyl 3-(7-fluoro-1-methoxyisoquinolin-3-yl)pyrrolidine-1-carboxylate (5)

To a solution of tert-butyl 3-(7-fluoro-1-methoxyisoquinolin-3-yl)-2,5-dihydro-1H-pyrrole-1-carboxylate **4** (160 g, 0.46 mmol) in MeOH (20 mL) was added Pd/C (35 mg, 0.29 mmol) under H₂. The reaction mixture was stirred at room temperature for 5 h. The mixture was filtered through a Celite pad and the filtrate was concentrated to give tert-butyl 3-(7-fluoro-1-methoxyisoquinolin-3-yl)pyrrolidine-1-carboxylate **5** (140 mg, 88 % yield ) as a white solid. LCMS (ESI) calcd for C19H23FN2O3 [M + H] ⁺ m/z 347.17, found 347.18.

### Preparation of 7-fluoro-3-(pyrrolidin-3-yl)isoquinolin-1(2H)-one (6)

Tert-butyl 3-(7-fluoro-1-methoxyisoquinolin-3-yl)pyrrolidine-1-carboxylate **5** (140 mg, 0.40 mmol) was added to HBr in water (48 %, 3 mL). The mixture was stirred at 100 °C for 2 h. The reaction mixture was concentrated under reduced pressure to give 7-fluoro-3-(pyrrolidin-3-yl)isoquinolin-1(2H)-one **6** (90 mg, 97% yield) as a white solid.

LCMS (ESI) calcd for C13H13FN20 [M + H] ⁺ m/z 233.10, found 232.95.

### Preparation of 5-(4-(3-(7-fluoro-1-oxo-1,2-dihydroisoquinolin-3-yl)pyrrolidin-1-yl)piperidin-1-yl)-N-methylpicolinamide (Compound 154rac)

To a solution of 7-fluoro-3-(pyrrolidin-3-yl)isoquinolin-1(2H)-one **6** (90 mg, 0.39 mmol) in MeOH (15 mL) was added N-methyl-5-(4-oxopiperidin-1-yl)picolinamide **(INT,** 100 mg, 0.43 mmol), then two drops of acetic acid and NaBH₃CN (25 mg, 0.40 mmol) were added at room temperature. The reaction mixture stirred at 50 °C for 4 h. After cooling to room temperature, the residue was purified by prep-HPLC (columns: Gemini 5 um C18 150 × 21.2 mm, mobile phase: ACN - H₂O (0.1% FA), gradient: 10 - 25) to give 5-(4-(3-(7-fluoro-1-oxo-1,2-dihydroisoquinolin-3-yl)pyrrolidin-1-yl)piperidin-1-yl)-N-methylpicolinamide **154rac** (35 mg, 99% purity, 20% yield) as a white solid.

### Compound 154rac

1H NMR (400 MHz, DMSO) δ 11.35 (s, 1 H), 8.39 (q, *J=* 4.8 Hz, 1 H), 8.30 (d, *J=* 2.8 Hz, 1 H), 7.87-7.76 (m, 2 H), 7.70 (dd, *J= 8.8,* 5.2 Hz, 1 H), 7.59 (td, *J= 8.8,* 2.8 Hz, 1 H), 7.43 (dd, *J=* 8.8, 2.8 Hz, 1 H), 6.57 (s, 1 H), 4.02-3.85 (m, 2 H), 3.31-2.71 (m, 11 H), 2.40-2.25 (m, 1 H), 2.13-1.88 (m, 3 H), 1.68-1.52 (m, 2 H).

LCMS (ESI) calcd for C25H28FN5O2 [M + H] ⁺ m/z 450.22, found 450.23.

### Preparation of 5-(4-(3-(7-fluoro-1-oxo-1,2-dihydroisoquinolin-3-yl)pyrrolidin-1-yl)piperidin-1-yl)-N-methylpicolinamide (154a and 154b)

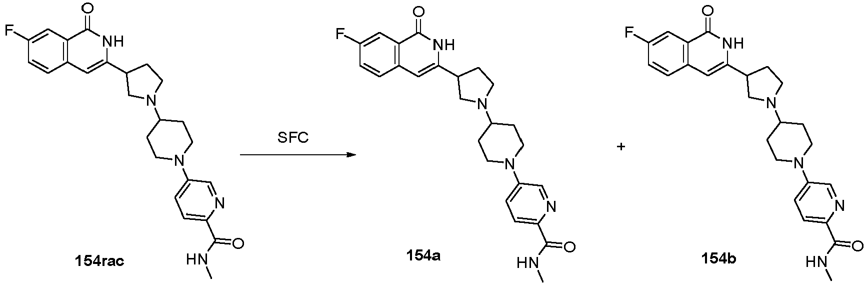

5-(4-(3-(7-fluoro-1-oxo-1,2-dihydroisoquinolin-3-yl)pyrrolidin-1-yl)piperidin-1-yl)-N-methylpicolinamide **154rac** was separated by SFC (Column: DAICEL OJ-H 4.6mmI.D.*250mmL 5µm; Mobile phase: CO2/MeOH[0.1%NH3(7M Solution in MeOH)]=75/25) and concentrated under reduced pressure to afford the first fraction as **154a** (10 mg, 99% purity, ee%: 100, white solid) and the second fraction as **154b** (10 mg, 97% purity, ee%: 100, white solid)

### Compound 154a

1H NMR (400 MHz, DMSO) δ 11.25 (s, 1 H), 8.39 (d, *J=* 4.8 Hz, 1 H), 8.28 (d, *J=* 2.8 Hz, 1 H), 7.82 (d, *J =* 8.4 Hz, 1 H), 7.77 (dd, *J =* 9.2, 2.8 Hz, 1 H), 7.68 (dd, *J = 8.8,* 5.6 Hz, 1 H), 7.56 (td, *J =* 8.8, 2.8 Hz, 1 H), 7.40 (dd, *J=* 8.8, 2.8 Hz, 1 H), 6.51 (s, 1 H), 3.87-3.77 (m, 2 H), 3.25-3.17 (m, 1 H), 3.03-2.91 (m, 3 H), 2.85-2.75 (m, 4 H), 2.74-2.66 (m, 2 H), 2.35-2.32 (m, 1 H), 2.28-2.17 (m, 1 H), 2.01-1.90 (m, 2 H), 1.88-1.78 (m, 1 H), 1.60-1.47 (m, 2 H). LCMS (ESI) calcd for C25H28FN5O2 [M + H] ⁺ m/z 450.22, found 450.20.

### Compound 154b

1H NMR (400 MHz, DMSO) δ 11.25 (s, 1 H), 8.39 (d, *J=* 4.8 Hz, 1 H), 8.28 (d, *J=* 2.8 Hz, 1 H), 7.82 (d, *J =* 8.8 Hz, 1 H), 7.77 (dd, *J =* 9.2, 2.8 Hz, 1 H), 7.68 (dd, *J =* 8.8, 5.2 Hz, 1 H), 7.56 (td, *J=* 8.8, 2.8 Hz, 1 H), 7.40 (dd, *J=* 8.8, 2.8 Hz, 1 H), 6.51 (s, 1 H), 3.88-3.78 (m, 2 H), 3.26-3.16 (m, 1 H), 3.03-2.91 (m, 3 H), 2.83-2.78 (m, 4 H), 2.74-2.66 (m, 2 H), 2.35-2.31 (m, 1 H), 2.28-2.17 (m, 1 H), 2.00-1.92 (m, 2 H), 1.89-1.79 (m, 1 H), 1.61-1.47 (m, 2 H). LCMS (ESI) calcd for C25H28FN5O2 [M + H] ⁺ m/z 450.22, found 450.20.

### Example 15 - Synthesis of Compounds 162rac, 162a and 162b (embodiments of the invention)

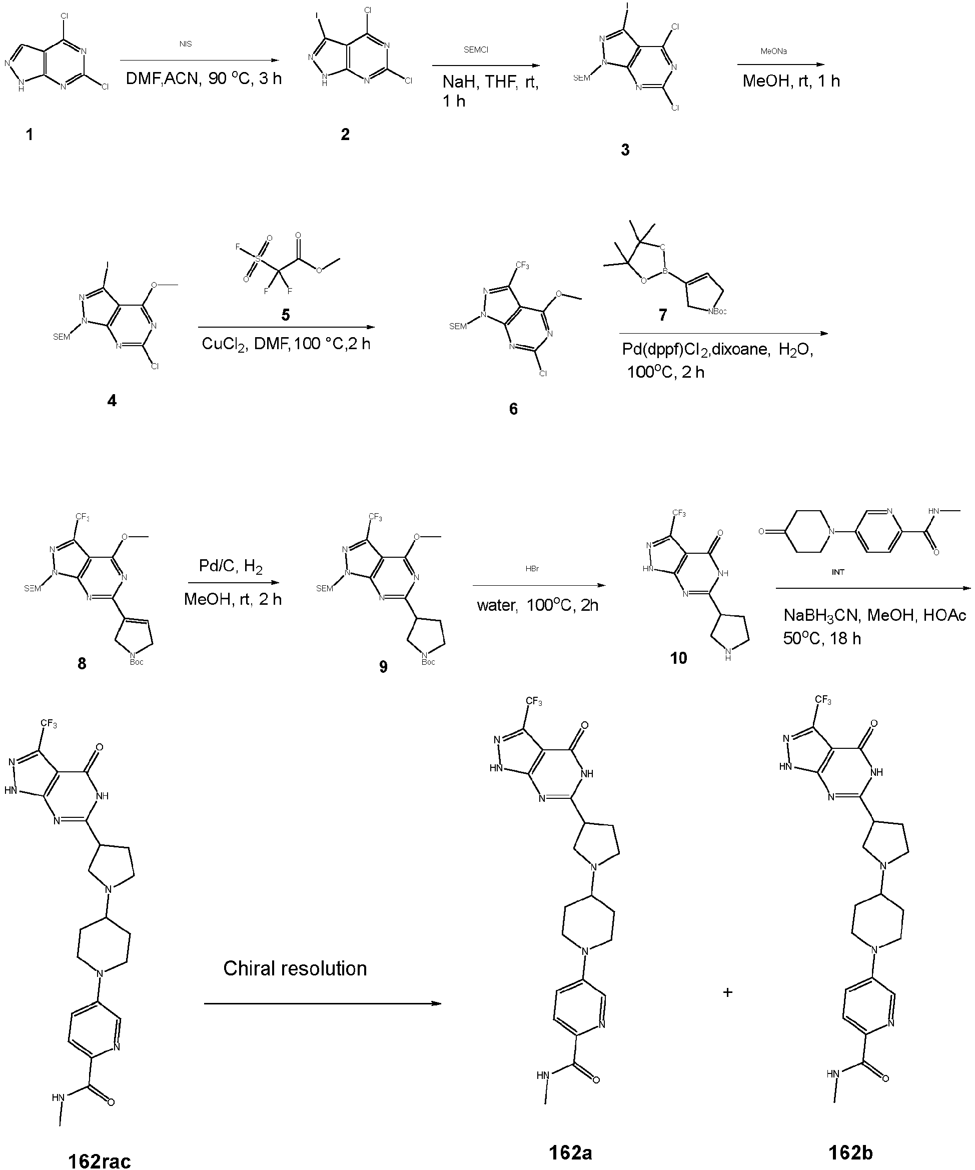

### Preparation of 4, 6-dichloro-3-iodo-1H-pyrazolo[3,4-d]pyrimidine (2)

To a solution of 4,6-dichloro-1H-pyrazolo[3,4-d]pyrimidine 1 (5 g, 26.50 mmol) in ACN/DMF = 1:1 (100 mL) was added NIS (8.9 g, 39.75 mmol). The resulting mixture was stirred at 90 °C for 3 h. The residue was purified by flash chromatography (eluting with PE/EtOAc = 100: 0 to 72: 28) to give 4,6-dichloro-3-iodo-1H-pyrazolo[3,4-d]pyrimidine 2 (2.9 g, 35% yield ) as white solid.

LCMS (ESI) calcd for C5HCl2IN4 [M + H] ⁺ m/z 314.86, found 314.85.

### Preparation of 4,6-dichloro-3-iodo-1-((2-(trimethylsilyl)ethoxy)methyl)-1H-pyrazolo[3,4-d]pyrimidine (3)

To a solution of 4,6-dichloro-3-iodo-1H-pyrazolo[3,4-d]pyrimidinee 2 (2.9 g, 9.24 mmol) in THF (40 mL) was added NaH (290 mg, 11.96 mmol) at 0 °C, stirred 10 min, then added SEMCl (2.3 g, 13.80 mmol). The resulting mixture was stirred at room temperature for 1 h. The reaction mixture was concentrated under reduced pressure to give 4,6-dichloro-3-iodo-1-((2-(trimethylsilyl)ethoxy)methyl)-1H-pyrazolo[3,4-d]pyrimidine **3** (3.6 g, 88% yield ) as white solid.

LCMS (ESI) calcd for C11H15Cl2IN4OSi [M + H] ⁺ m/z 444.94, found 444.85.

### Preparation of 6-chloro-3-iodo-4-methoxy-1-((2-(trimethylsilyl)ethoxy)methyl)-1H-pyrazolo[3,4-d]pyrimidine (4)

To a solution of 4,6-dichloro-3-iodo-1-((2-(trimethylsilyl)ethoxy)methyl)-1H-pyrazolo[3,4-d]pyrimidine **3** (1.5 g, 3.39 mmol) in MeOH (45 mL) was added MeONa (183 mg, 3.39 mmol). The mixture was stirred at room temperature for 1 h. The residue was purified by flash chromatography (eluting with PE/EtOAc = 100: 0 to 95: 5) to give 6-chloro-3-iodo-4-methoxy-1-((2-(trimethylsilyl)ethoxy)methyl)-1H-pyrazolo[3,4-d]pyrimidine **4** (282 mg, 19% yield) as a white solid.

LCMS (ESI) calcd for C12H18ClIN4O2Si [M + H] ⁺ m/z 440.99 found 441.01.

### Preparation of 6-chloro-4-methoxy-3-(trifluoromethyl)-1-((2-(trimethylsilyl)ethoxy)methyl)-1H-pyrazolo[3,4-d]pyrimidine (6)

To a solution of 6-chloro-3-iodo-4-methoxy-1-((2-(trimethylsilyl)ethoxy)methyl)-1H-pyrazolo[3,4-d]pyrimidine **4** (282 mg, 0.64 mmol) and methyl 2,2-difluoro-2-(fluorosulfonyl)acetate **5** (491 mg, 2.56 mmol) in DMF (15 mL) was added CuCl₂ (131 mg, 0.98 mmol) under N₂. The reaction mixture was stirred at 100 °C for 2 h. The residue was purified by flash chromatography (eluting with PE/EtOAc = 100: 0 to 96: 4) to 6-chloro-4-methoxy-3-(trifluoromethyl)-1-((2-(trimethylsilyl)ethoxy)methyl)-1H-pyrazolo[3,4-d]pyrimidine **6** (220 mg, 90% yield ) as a white solid.

LCMS (ESI) calcd for C13H18ClF3N4O2Si [M + H] + m/z 383.08, found 383.08.

### Preparation of tert-butyl 3-(4-methoxy-3-(trifluoromethyl)-1-((2-(trimethylsilyl)ethoxy)methyl)-1H-pyrazolo[3,4-d]pyrimidin-6-yl)-2,5-dihydro-1H-pyrrole-1-carboxylate (8)

To a solution of tert-butyl 3-(4-methoxy-5-(trifluoromethyl)-7-((2-(trimethylsilyl)ethoxy)methyl)-7H-pyrrolo[2,3-d]pyrimidin-2-yl)-2,5-dihydro-1H-pyrrole-1-carboxylate **6** (200 mg, 0.52 mmol), tert-butyl 3-(4,4,5,5-tetramethyl-1,3,2-dioxaborolan-2-yl)-2,5-dihydro-1H-pyrrole-1-carboxylate 7 (154 mg, 0.52 mmol) and Pd(dppf)Cl₂ (23 mg, 0.031 mmol) in 1,4-dioxane/H₂O=10:1 (11 mL) was added Na₂CO₃ (165 mg, 1.56 mmol) under N₂. The reaction mixture was stirred at 100 °C for 2 h. The residue was purified by flash chromatography (eluting with PE/EtOAc = 100: 0 to 93: 7) to give tert-butyl 3-(4-methoxy-3-(trifluoromethyl)-1-((2-(trimethylsilyl)ethoxy)methyl)-1H-pyrazolo[3,4-d]pyrimidin-6-yl)-2,5-dihydro-1H-pyrrole-1-carboxylate **8** (280 mg, 94% yield ) as an orange oil.

LCMS (ESI) calcd for C27H32F3N5O4Si [M + H] + m/z 516.22, found 516.23.

### Preparation of tert-butyl 3-(4-methoxy-3-(trifluoromethyl)-1-((2-(trimethylsilyl)ethoxy)methyl)-1H-pyrazolo[3,4-d]pyrimidin-6-yl)pyrrolidine-1-carboxylate (9)

To a solution of tert-butyl 3-(4-methoxy-5-(trifluoromethyl)-7-((2-(trimethylsilyl)ethoxy)methyl)-7H-pyrrolo[2,3-d]pyrimidin-2-yl)pyrrolidine-1-carboxylate **8** (280 mg, 0.54 mmol) in MeOH (15 mL) was added Pd/C (114 mg, 1.08 mmol) under H₂. The reaction mixture was stirred at room temperature for 2 h. Then the mixture was filtered through celite and concentrated under vacuum to give tert-butyl 3-(4-methoxy-3-(trifluoromethyl)-1-((2-(trimethylsilyl)ethoxy)methyl)-1H-pyrazolo[3,4-d]pyrimidin-6-yl)pyrrolidine-1-carboxylate **9** (158 mg, 56% yield ) as a white solid.

LCMS (ESI) calcd for C22H34F3N5O4Si [M + H] + m/z 518.23, found 518.25.

### Preparation of 6-(pyrrolidin-3-yl)-3-(trifluoromethyl)-1,5-dihydro-4H-pyrazolo[3,4-d]pyrimidin-4-one (10)

Tert-butyl 3-(4-methoxy-3-(trifluoromethyl)-1-((2-(trimethylsilyl)ethoxy)methyl)-1H-pyrazolo[3,4-d]pyrimidin-6-yl)pyrrolidine-1-carboxylate **9** (158 mg, 0.31 mmol) was added to HBr water (5 mL). The reaction mixture was stirred at 100 °C for 2 h. Then the mixture was concentrated under vacuum to give 6-(pyrrolidin-3-yl)-3-(trifluoromethyl)-1,5-dihydro-4H-pyrazolo[3,4-d]pyrimidin-4-one **10** (80 mg, 96% yield ) as a white solid.

LCMS (ESI) calcd for C10H10F3N50 [M + H] + m/z 274.08, found 274.00.

### Preparation of N-methyl-5-(4-(3-(4-oxo-3-(trifluoromethyl)-4,5-dihydro-1H-pyrazolo[3,4-d]pyrimidin-6-yl)pyrrolidin-1-yl)piperidin-1-yl)picolinamide (162rac)

To a solution of 6-(pyrrolidin-3-yl)-3-(trifluoromethyl)-1,5-dihydro-4H-pyrazolo[3,4-d]pyrimidin-4-one **10** (80 mg, 0.29 mmol), N-methyl-5-(4-oxopiperidin-1-yl)picolinamide **INT** (90 mg, 0.29 mmol) and sodium triacetoxyborohydride (92 mg, 0.44 mmol) in MeOH (10 mL) was added two drops of HOAc and the solution was stirred for 0.5 h at 50 °C. Then was added NaBH3CN (22 mg, 0.35 mmol). The mixture was stirred for 18 h at 50 °C. The residue was purified by flash chromatography (eluting with DCM/MeOH = 100 : 0 to 90 : 10) to give product of N-methyl-5-(4-(3-(4-oxo-3-(trifluoromethyl)-4,5-dihydro-1H-pyrazolo[3,4-d]pyrimidin-6-yl)pyrrolidin-1-yl)piperidin-1-yl)picolinamide **162rac** (69 mg, 48 % yield ) as white solid.

LCMS (ESI) calcd for C22H25F3N8O2 [M + H] + m/z 491.21, found 491.25.

### Chiral resolution of N-methyl-5-(4-(3-(4-oxo-3-(trifluoromethyl)-4,5-dihydro-1H-pyrazolo[3,4-d]pyrimidin-6-yl)pyrrolidin-1-yl)piperidin-1-yl)picolinamide (162a and 162b)

Compound **162rac** was separated by SFC (Column: DAICEL OJ-H 4.6 mml.D.*250 mmL, 5 µm; Mobile phase: CO₂/MeOH[0.1% NH₃ (7M Solution in MeOH)] = 70/30) and concentrated under reduced pressure to afford the first fraction as **162a** (39 mg, 93% purity, ee%: 100, white solid) and the second fraction as **162b** (31 mg, 99% purity, ee%: 100, white solid)

### Compound 162a

1H NMR (400 MHz, DMSO) δ 14.37 (s, 1 H), 12.27 (s, 1 H), 8.39 (q, *J* = 4.4 Hz, 1 H), 8.27 (d, *J =* 2.8 Hz, 1 H), 7.82 (d, *J =* 8.8 Hz, 1 H), 7.40 (dd, *J =* 8.4, 2.8 Hz, 1 H), 3.83 (d, *J =* 12.8 Hz, 2 H), 3.38-3.35 (m, 1 H), 3.06 (t, *J =* 8.4 Hz, 1 H), 2.98-2.88 (m, 2 H), 2.83-2.73 (m, 5 H), 2.68-2.61 (m, 1 H), 2.39-2.29 (m, 1 H), 2.21-2.09 (m, 2 H), 1.93 (d, *J* = 11.6 Hz, 2 H), 1.55-1.43 (m, 2 H).

LCMS (ESI) calcd for C22H25F3N8O2 [M + H] + m/z 491.21, found 491.26.

### Compound 162b

1H NMR (400 MHz, ) δ 14.41 (s, 1 H), 12.41 (s, 1 H), 8.39 (q, *J* = 4.8 Hz, 1 H), 8.28 (d, *J =* 2.0 Hz, 1 H), 7.82 (d, *J =* 8.8 Hz, 1 H), 7.41 (dd, *J =* 8.8, 2.0 Hz, 1 H), 3.86 (s, 1 H), 3.30-3.27 (m, 1 H), 3.16-3.03 (m, 1 H), 2.96-2.58 (m, 8 H), 2.34-2.10 (m, 3 H), 2.03-1.88 (m, 2 H), 1.63-1.44 (m, 2 H).

LCMS (ESI) calcd for C22H25F3N8O2 [M + H] + m/z 491.21, found 491.25.

### Example 16 - Synthesis of Compounds 184rac, 184a and 184b (embodiments of the invention)

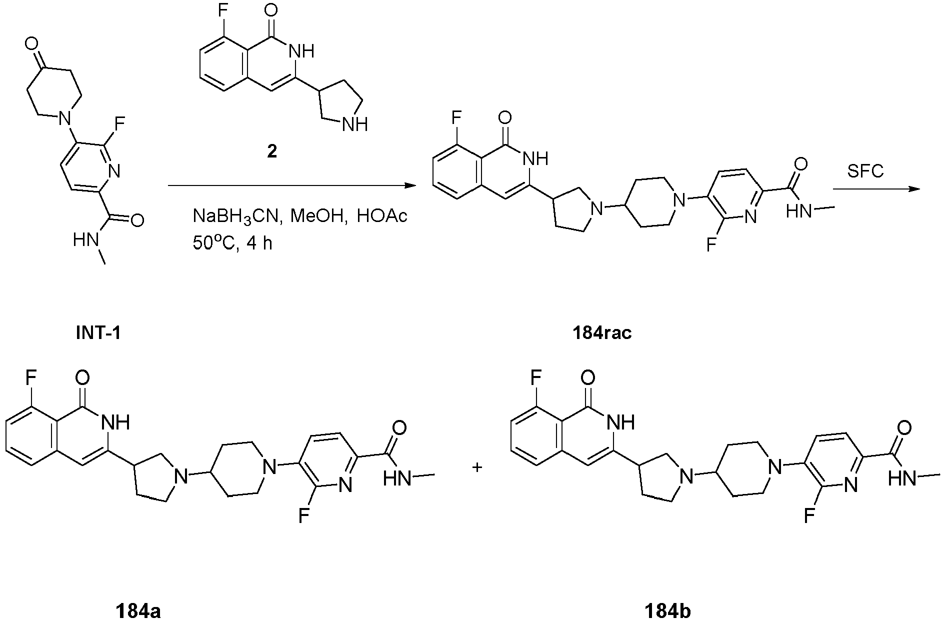

### Preparation of 6-fluoro-5-{4-[3-(8-fluoro-1-oxo-2H-isoquinolin-3-yl)pyrrolidin-1-yl]piperidin-1-yf}-N-methylpyridine-2-carboxamide (184rac)

To a solution of 6-fluoro-N-methyl-5-(4-oxopiperidin-1-yl)pyridine-2-carboxamide **INT-1** (92 mg, 0.40 mmol) in MeOH (10 mL) was added N-methyl-5-(4-oxopiperidin-1-yl)pyridine-2-carboxamide 2 (intermediate 6 in synthesis of compound 136) (100 mg, 0.40 mmol). Then two drops of acetic acid and NaBH₃CN (25 mg, 0.26 mmol) was added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 92 : 8) to give product of 6-fluoro-5-{4-[3-(8-fluoro-1-oxo-2H-isoquinolin-3-yl)pyrrolidin-1-yl]piperidin-1-yl}-N-methylpyridine-2-carboxamide **184rac** (60 mg, 27% yield ) as a colorless oil.

LCMS (ESI) calcd for C25H27F2N5O2 [M + H] ⁺ m/z 468.21, found 468.26.

### Preparation of 6-fluoro-5-{4-[3-(8-fluoro-1-oxo-2H-isoquinolin-3-yl)pyrrolidin-1-yl]piperidin-1-yl}-N-methylpyridine-2-carboxamide (184a and 184b)

6-chloro-5-{4-[3-(8-fluoro-1-oxo-2H-isoquinolin-3-yl)pyrrolidin-1-yl]piperidin-1-yl}-N-methylpyridine-2-carboxamide **184rac** was separated by SFC (Column: Daicel CHIRALPAK OJ-H 250 mm × 20 mm I.D., 5 µmm; Mobile phase: CO₂/MeOH[0.1%(NH₃)] = 80/20) and concentrated under reduced pressure to afford the first fraction as **184a** (24.5 mg, 98% purity, ee%: 100, white solid) and the second fraction as **184b** (24.4 mg, 99% purity, ee%: 97, white solid)

### Compound 184a

¹H NMR (400 MHz, DMSO) δ 11.10 (s, 1 H), 8.41 (q, *J =* 5.2 Hz, 1 H), 7.85 (d, *J =* 7.2 Hz, 1 H), 7.64-7.56 (m, 2 H), 7.39 (d, *J =* 7.6 Hz, 1 H), 7.10 (dd, *J =* 12.0, 8.0 Hz, 1 H), 6.47 (s, 1 H), 3.52-3.51 (m, 2 H), 3.22-3.16 (m, 1 H), 2.91-2.73 (m, 8 H), 2.67-2.63 (m, 1 H), 2.30-2.22 (m, 2 H), 2.00-1.97 (m, 2 H), 1.86-1.79 (m, 1 H), 1.68-1.53 (m, 2 H).

LCMS (ESI) calcd for C25H27F2N5O2 [M + H] ⁺ m/z 468.21, found 468.22.

### Compound 184b

¹H NMR (400 MHz, DMSO) δ 11.10 (s, 1 H), 8.41 (q, *J* = 4.8 Hz, 1 H), 7.85 (dd, *J* = 8.0, 1.2 Hz, 1 H), 7.64-7.56 (m, 2 H), 7.39 (d, *J* = 7.6 Hz, 1 H), 7.11 (dd, *J* = 11.6, 8.0 Hz, 1 H), 6.46 (s, 1 H), 3.53-3.49 (m, 2 H), 3.21-2.87 (m, 1 H), 2.93-2.71 (m, 8 H), 2.68-2.63 (m, 1 H), 2.28-2.19 (m, 2 H), 2.00-1.97 (m, 2 H), 1.88-1.79 (m, 1 H), 1.64-1.54 (m, 2 H).

LCMS (ESI) calcd for C25H27F2N5O2 [M + H] ⁺ m/z 468.21, found 468.18.

### Example 17 - Synthesis of compound 192 (an embodiment of the invention)

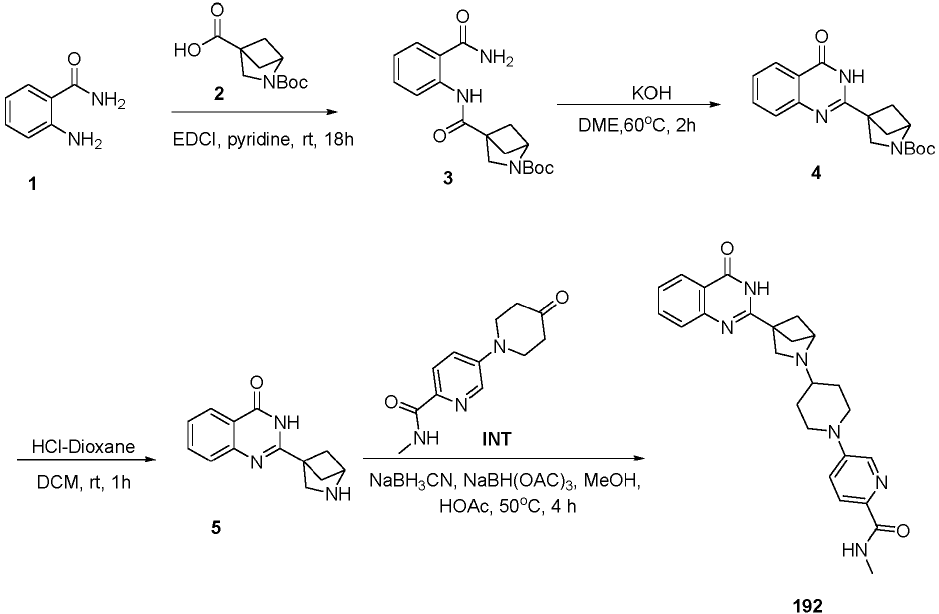

### Preparation of tert-butyl 4-((2-carbamoylphenyl)carbamoyl)-2-azabicyclo[2.1.1]hexane-2-carboxylate (3)

To a solution of 2-aminobenzamide **1** (360 mg, 2.64 mmol) and 2-(tert-butoxycarbonyl)-2-azabicyclo[2.1.1]hexane-4-carboxylic acid **2** (500 mg, 2.20 mmol) in pyridine (30 mL) was added EDCI (422 mg, 2.20 mmol). Then the mixture was stirred for 18 h at room temperature. The reaction mixture was quenched with water, the aqueous layer was extracted with EtOAc (200 mL x 3). The combined organic layers was washed by 1M HCl solution and brine, dried over Na₂SO₄, concentrated under reduced pressure to give product of tert-butyl 4-((2-carbamoylphenyl)carbamoyl)-2-azabicyclo[2.1.1]hexane-2-carboxylate **3** (600 mg, 90% yield) as a white solid.

LCMS (ESI) calcd for C₁₈H₂₃N₃O₄ [M + Na] ⁺ m/z 368.17, found 368.05.

### Preparation of tert-butyl 4-(4-oxo-3,4-dihydroquinazolin-2-yl)-2-azabicyclo[2.1.1]hexane-2-carboxylate (4)

To the solution of tert-butyl 4-((2-carbamoylphenyl)carbamoyl)-2-azabicyclo[2.1.1]hexane-2-carboxylate **3** (600 mg, 1.74 mmol) in DME (60 mL) was added KOH (195 mg, 3.47 mmol), heated to 60 °C stirred for 2 h, cooled to 25 °C, the reaction mixture was concentrated under reduced pressure. The residue was purified by flash chromatography (eluting with DCM/MeOH = 100 : 0 to 90 : 10) to give tert-butyl 4-(4-oxo-3,4-dihydroquinazolin-2-yl)-2-azabicyclo[2.1.1]hexane-2-carboxylate **4** (400 mg, 63% yield ) as a white solid.

LCMS (ESI) calcd for C₁₈H₂₁N₃O₃ [M + H] ⁺ m/z 328.16, found 328.05.

### Preparation of 2-(2-azabicyclo[2.1.1]hexan-4-yl)quinazolin-4(3H)-one (5)

Tert-butyl 4-(4-oxo-3,4-dihydroquinazolin-2-yl)-2-azabicyclo[2.1.1]hexane-2-carboxylate **4** (400 mg, 0.56 mmol) was added to HCl in dioxane (4M, 10 mL). The mixture was stirred for 1 h at room temperature. The reaction mixture was concentrated under reduced pressure to give product of 2-(2-azabicyclo[2.1.1]hexan-4-yl)quinazolin-4(3H)-one **5** (320 mg, 93% yield ) as a white solid.

LCMS (ESI) calcd for C₁₃H₁₃N₃O [M + H] ⁺ m/z 228.11, found 228.02.

### Preparation of N-methyl-5-(4-(4-(4-oxo-3,4-dihydroquinazolin-2-yl)-2-azabicyclo[2.1.1]hexan-2-yl)piperidin-1-yl)picolinamide (compound 192)

To a solution of 2-(2-azabicyclo[2.1.1]hexan-4-yl)quinazolin-4(3H)-one **5** (80 mg, 0.35 mmol) in MeOH (40 mL) was added N-methyl-5-(4-oxopiperidin-1-yl)picolinamide (**INT**, 98 mg, 0.42 mmol), then two drops of acetic acid and NaBH(OAC)₃ (149 mg, 0.70 mmol) were added at room temperature. The reaction mixture stirred at 50 °C for 1 h. Then was added NaBH₃CN (11.06 mg, 0.176 mmol). The reaction mixture stirred at 50 °C for 4 h. After cooling to room temperature, the reaction mixture was concentrated under reduced pressure. The residue was purified by C18 column (mobile phase: ACN - H₂O (0.1% FA), gradient: 10 - 95) to give N-methyl-5-(4-(4-(4-oxo-3,4-dihydroquinazolin-2-yl)-2-azabicyclo[2.1.1]hexan-2-yl)piperidin-1-yl)picolinamide **192** (25.6 mg, 99 % purity, 16% yield) as a white solid.

### Compound 192

¹H NMR (400 MHz, DMSO-*d₆*, ppm) δ: 12.17 (s, 1 H), 8.43-8.36 (m, 1 H), 8.29 (d, *J* = 2.8 Hz, 1 H), 8.17 (s, 1 H), 8.10 (dd, *J* = 8.0, 1.2 Hz, 1 H), 7.86-7.76 (m, 2 H), 7.62 (d, *J* = 7.8 Hz, 1 H), 7.52-7.47 (m, 1 H), 7.42 (dd, *J =* 8.8, 2.9 Hz, 1 H), 3.93-3.86 (m, 2 H), 3.76 (s, 1 H), 3.12 (s, 2 H), 2.97-2.90 (m, 2 H), 2.78 (d, *J* = 4.8 Hz, 3 H), 2.69-2.62 (m, 1 H), 2.19-2.14 (m, 2 H), 2.02-1.90 (m, 4 H), 1.54-1.44 (m, 2 H).

LCMS (ESI) calcd for C₂₅H₂₈N₆O₂ [M + H]⁺ m/z 445.23, found 445.15.

### Example 18 - Synthesis of compound 194a and 194b (embodiments of the invention)

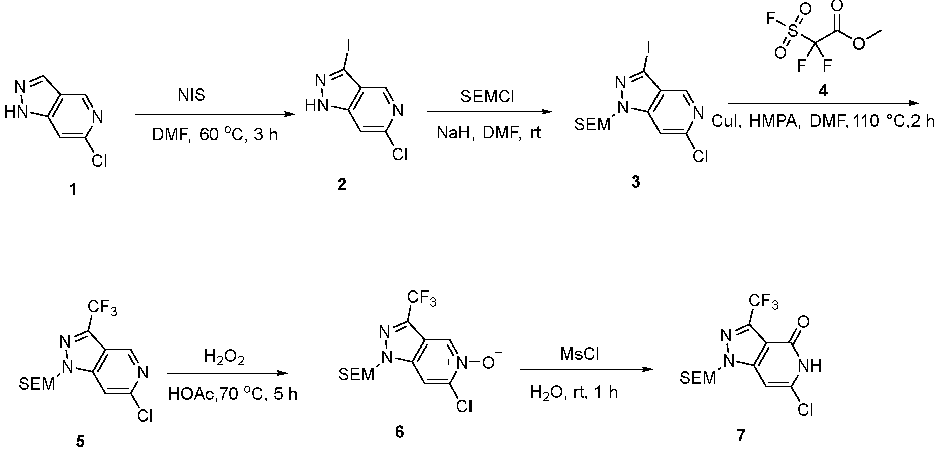

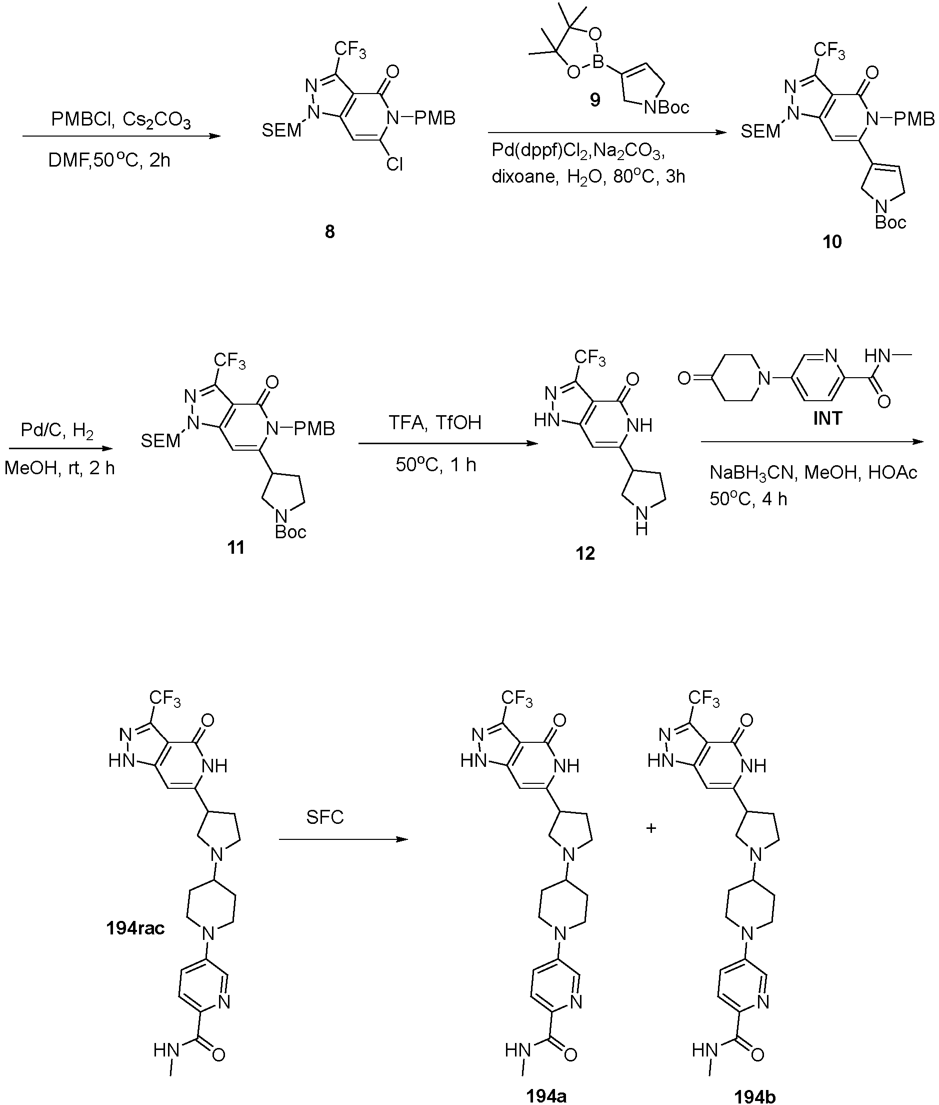

### Preparation of 6-chloro-3-iodo-1H-pyrazolo[4,3-c]pyridine (2)

To a solution of 6-chloro-1H-pyrazolo[4,3-c]pyridine **1** (10.00 g, 0.06 mol) in DMF (200 mL) was added NIS (8.44 g, 0.08 mol). The mixture was stirred for 3 h at 60 ^{°}C. The reaction mixture was quenched with water, the aqueous layer was extracted with EtOAc (50 mL x 3). The combined organic layers were concentrated under reduced pressure. The residue was purified by flash chromatography (eluting with PE/ EtOAc = 100 : 0 to 60 : 40) to give product of 6-chloro-3-iodo-1H-pyrazolo[4,3-c]pyridine **2** (10.5 g, 64% yield ) as a yellow solid.

LCMS (ESI) calcd for C6H3ClIN3 [M + H] ⁺ m/z 279.91, found 280.00.

### Preparation of 6-chloro-3-iodo-1-((2-(trimethylsilyl)ethoxy)methyl)-1H-pyrazolo[4,3-c]pyridine (3)

A suspension of 6-chloro-3-iodo-1H-pyrazolo[4,3-c]pyridine **2** (10.50 g, 0.04 mol) and NaH (2.70 g, 0.11 mol) in DMF (200 mL) was stirred for 30 min at 0 °C, then SEMCl (12.30 g, 0.08 mol) was added. The mixture was stirred for 3 h at room temperature. The reaction mixture was quenched with water, the aqueous layer was extracted with EtOAc (50 mL x 3). The combined organic layer was concentrated under reduced pressure. The residue was purified by flash chromatography (eluting with PE/ EtOAc = 100 : 0 to 70 : 30) to give product of 6-chloro-3-iodo-1-((2-(trimethylsilyl)ethoxy)methyl)-1H-pyrazolo[4,3-c]pyridine **3** (9.90 g, 61% yield ) as a yellow solid.

LCMS (ESI) calcd for C12H17ClIN3OSi [M + H] ⁺ m/z 409.99, found 410.05.

### Preparation of 6-chloro-3-(trifluoromethyl)-1-((2-(trimethylsilyl)ethoxy)methyl)-1H-pyrazolo[4,3-c]pyridine (5)

To a solution of 6-chloro-3-iodo-1-((2-(trimethylsilyl)ethoxy)methyl)-1H-pyrazolo[4,3-c]pyridine **3** (9.90 g, 0.02 mol) and methyl 2,2-difluoro-2-(fluorosulfonyl)acetate **4** (23.04 g, 0.12 mol) in DMF (80 mL) were added CuI (9.15 g, 0.04 mmol) and HMPA (21.35g, 0.12 mmol) at room temperature. The mixture was stirred for 2 h at 110 °C. The reaction mixture was quenched with water, the aqueous layer was extracted with EtOAc (50 mL x 3). The combined organic layer was concentrated under reduced pressure. The residue was purified by flash chromatography (eluting with PE/ EtOAc = 100 : 0 to 80 : 20) to give product of 6-chloro-3-(trifluoromethyl)-1-((2-(trimethylsilyl)ethoxy)methyl)-1H-pyrazolo[4,3-c]pyridine **5** (3.30 g, 38% yield) as a yellow solid.

LCMS (ESI) calcd for for C13H17ClF3N3OSi [M + H] ⁺ m/z 352.08, found 352.20.

### Preparation of 6-chloro-3-(trifluoromethyl)-1-((2-(trimethylsilyl)ethoxy)methyl)-1H-pyrazolo[4,3-c]pyridine 5-oxide (6)

To a solution of 6-chloro-3-(trifluoromethyl)-1-((2-(trimethylsilyl)ethoxy)methyl)-1H-pyrazolo[4,3-c]pyridine **5** (3.30 g, 0.01 mmol) in HOAc (20 mL) was added H₂O₂ (20 mL). The reaction mixture stirred at 70 °C for 5 h. After cooling to room temperature, the reaction mixture was quenched with water, the aqueous layer was extracted with EtOAc (30 mL x 3). The combined organic layer was concentrated under reduced pressure. The residue was purified by flash chromatography (eluting with PE/ EtOAc = 100 : 0 to 55 : 45) to give product of 6-chloro-3-(trifluoromethyl)-1-((2-(trimethylsilyl)ethoxy)methyl)-1H-pyrazolo[4,3-c]pyridine 5-oxide **6** (1.60 g, 48 % yield ) as an yellow oil.

LCMS (ESI) calcd for C13H17ClF3N3O2Si [M + H] ⁺ m/z 368.07, found 367.97.

### Preparation of 6-chloro-3-(trifluoromethyl)-1-((2-(trimethylsilyl)ethoxy)methyl)-1,5-dihydro-4H-pyrazolo[4,3-c]pyridin-4-one (7)

To a solution of 6-chloro-3-(trifluoromethyl)-1-((2-(trimethylsilyl)ethoxy)methyl)-1H-pyrazolo[4,3-c]pyridine 5-oxide **6** (800 mg, 2.16 mmol) in H₂O (15 mL) and MsCl (1.00 g, 8.62 mmol). The reaction mixture was stirred at room temperature for 1 h. The reaction mixture was quenched with water, the aqueous layer was extracted with EtOAc (20 mL x 3). The combined organic layer was concentrated under reduced pressure. The residue was purified by flash chromatography (eluting with PE/ EtOAc = 100 : 0 to 85 : 15) to give product of 6-chloro-3-(trifluoromethyl)-1-((2-(trimethylsilyl)ethoxy)methyl)-1,5-dihydro-4H-pyrazolo[4,3-c]pyridin-4-one 7 (400 mg, 48% yield) as an yellow oil.

LCMS (ESI) calcd for C13H17ClF3N3O2Si [M + H] ⁺ m/z 368.07, found 368.01.

### Preparation of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one (8)

To a solution of 6-chloro-3-(trifluoromethyl)-1-((2-(trimethylsilyl)ethoxy)methyl)-1,5-dihydro-4H-pyrazolo[4,3-c]pyridin-4-one 7 (400 mg, 1.07 mmol) and Cs₂CO₃ (1.25 g, 3.85 mmol) in DMF (15 mL) was added PMBCl (0.84 g, 5.35 mmol) at room temperature. The reaction mixture was stirred at 50 °C for 2 h. After cooling to room temperature, the combined organic layer was concentrated under reduced pressure. The residue was purified by flash chromatography (eluting with PE/ EtOAc = 100 : 0 to 85 : 15) to give product of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one **8** (200 mg, 32% yield) as a yellow oil.

LCMS (ESI) calcd for C21H25ClF3N3O3Si [M + H] ⁺ m/z 488.13, found 488.15.

### Preparation of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)-2,5-dihydro-1H-pyrrole-1-carboxylate (10)

To a solution of 6-chloro-5-(4-methoxybenzyl)-3-(trifluoromethyl)-1-((2-(trimethylsilyl)ethoxy)methyl)-1,5-dihydro-4H-pyrazolo[4,3-c]pyridin-4-one **8** (200 mg, 0.48 mmol) and tert-butyl 3-(4,4,5,5-tetramethyl-1,3,2-dioxaborolan-2-yl)-2,5-dihydropyrrole-1-carboxylate **9** (150 mg, 0.50 mmol) in Dioxan:H₂O=5:1 (15 mL) was added Pd(dppf)Cl₂ (50 mg, 0.07 mmol) and Na₂CO₃ (70 mg, 0.06 mmol) at room temperature. The reaction mixture stirred under nitrogen at 80 °C for 3 h. After cooling to room temperature, the reaction mixture was concentrated under reduced pressure and purified by flash chromatography (eluting with PE/ EtOAc = 100 : 0 to 80 : 20) to give product of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)-2,5-dihydro-1H-pyrrole-1-carboxylate 10 (140 mg, 60% yield ) as a yellow oil.

LCMS (ESI) calcd for C30H39F3N4O5Si [M + H] ⁺ m/z 621.26, found 621.30.

### Preparation of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)pyrrolidine-1-carboxylate (11)

To a solution of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)-2,5-dihydro-1H-pyrrole-1-carboxylate **10** (140 mg, 0.30 mmol) in MeOH (15 mL) was added Pd/C (60 mg, 0.57 mmol). The reaction mixture stirred under hydrogen at room temperature for 2 h. The reaction mixture was filtered, the solution was concentrated under reduced pressure to give product of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)pyrrolidine-1-carboxylate **11** (120 mg, 80% yield ) as a colourless oil.

LCMS (ESI) calcd for C30H41F3N405Si [M + H] ⁺ m/z 623.28, found 623.30.

### Preparation of 6-(pyrrolidin-3-yl)-3-(trifluoromethyl)-1,5-dihydro-4H-pyrazolo[4,3-c]pyridin-4-one (12)

To a solution of tert-butyl 3-(5-(4-methoxybenzyl)-4-oxo-3-(trifluoromethyl)-1-((2-(trimethylsilyl)ethoxy)methyl)-4,5-dihydro-1H-pyrazolo[4,3-c]pyridin-6-yl)pyrrolidine-1-carboxylate **11** (120 mg, 0.19 mmol) in TFA (5 mL) was added two drops of TfOH. The reaction mixture was stirred at 50 °C for 1 h. The reaction mixture was concentrated under reduced pressure to give product of 6-(pyrrolidin-3-yl)-3-(trifluoromethyl)-1,5-dihydro-4H-pyrazolo[4,3-c]pyridin-4-one **12** (50 mg, 90% yield ) as a yellow oil.

LCMS (ESI) calcd for C11H11F3N40 [M + H] ⁺ m/z 273.09, found 273.15.

### Preparation of N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide (racemic mixture of 194a and 194b)

To a solution of 6-(pyrrolidin-3-yl)-3-(trifluoromethyl)-1,5-dihydro-4H-pyrazolo[4,3-c]pyridin-4-one **12** (50 mg, 0.18 mmol) in MeOH (10 mL) was added N-methyl-5-(4-oxopiperidin-1-yl)pyridine-2-carboxamide **INT** (43 mg, 0.18 mmol). Then two drops of acetic acid and NaBH₃CN (12 mg, 0.18 mmol) were added at room temperature. The reaction mixture was stirred at 50 °C for 4 h. After cooling to room temperature, the reaction mixture was concentrated under reduced pressure. The residue was purified by flash chromatography (eluting with DCM/MeOH = 100 : 0 to 92 : 8) to give N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide a racemic mixture of compounds **194a** and **194b** (30 mg, 32% yield ) as a white solid.

LCMS (ESI) calcd for C23H26F3N7O2 [M + H] ⁺ m/z 490.21, found 490.17.

### Chiral separation of N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide (compounds 194a and 194b)

A racemic mixture of N-methyl-5-(4-(3-(4-oxo-3-(trifluoromethyl)-4,5-dihydro-1H-pyrazolo[4,3-c]pyridin-6-yl)pyrrolidin-1-yl)piperidin-1-yl)picolinamide **(194rac)** was separated by SFC (Column: Daicel CHIRALPAK OJ-H 250 mm × 20 mm I.D., 5 µmm; Mobile phase: CO₂/MeOH[0.1%(NH₃)] = 70/30) and concentrated under reduced pressure to afford the first fraction as **194a** (9.9 mg, 98% purity, ee%: 100, white solid) and the second fraction as **194b** (9.4 mg, 100% purity, ee%: 98, white solid)

### Compound 194a

¹H NMR (400 MHz, DMSO) δ 13.82 (s, 1 H), 11.10 (s, 1 H), 8.39 (q, *J =* 4.4 Hz, 1 H), 8.28 (d, *J* = 2.8 Hz, 1 H), 7.83 (d, *J* = 8.8 Hz, 1 H), 7.41 (dd, *J =* 8.8, 2.8 Hz,1 H), 6.39 (s, 1 H), 3.84-3.81 (m, 2 H), 3.29-3.25 (m, 1 H), 2.98 (t, *J* = 11.2 Hz, 2 H), 2.89-2.85 (m, 1 H), 2.81-2.78 (m, 5 H), 2.60-2.56 (m, 1 H), 2.39-2.30 (m, 1 H),2.30-2.17 (m, 1 H), 1.97 (d, *J* = 12.0 Hz, 2 H), 1.83-1.77 (m, 1 H), 1.56-1.51 (m, 2 H).

LCMS (ESI) calcd for C23H26F3N7O2 [M + H] ⁺ m/z 490.21, found 490.20.

### Compound 194b

¹H NMR (400 MHz, DMSO) δ 13.81 (s, 1 H), 11.11 (s, 1 H), 8.39 (q, *J* = 4.8 Hz, 1 H), 8.28 (d, *J* = 2.8 Hz, 1 H), 7.83 (d, *J* = 8.8 Hz, 1 H), 7.40 (dd, *J =* 8.8, 2.8 Hz,1 H), 6.40 (s, 1 H), 3.91-3.81 (m, 2 H), 3.27-3.23 (m, 1 H), 2.98 (t, *J =* 11.6 Hz, 2 H), 2.90-2.86 (m, 1 H), 2.80-2.77 (m, 5 H), 2.60-2.54 (m, 1 H), 2.36-2.33 (m, 1 H),2.25-2.19 (m, 1 H), 1.97 (d, *J* = 11.2 Hz, 2 H), 1.86-1.75 (m, 1 H), 1.58-1.48 (m, 2 H).

LCMS (ESI) calcd for C23H26F3N7O2 [M + H] ⁺ m/z 490.21, found 490.16.

### Example 19 - Synthesis of compounds 208a and 208b (embodiments of the invention)

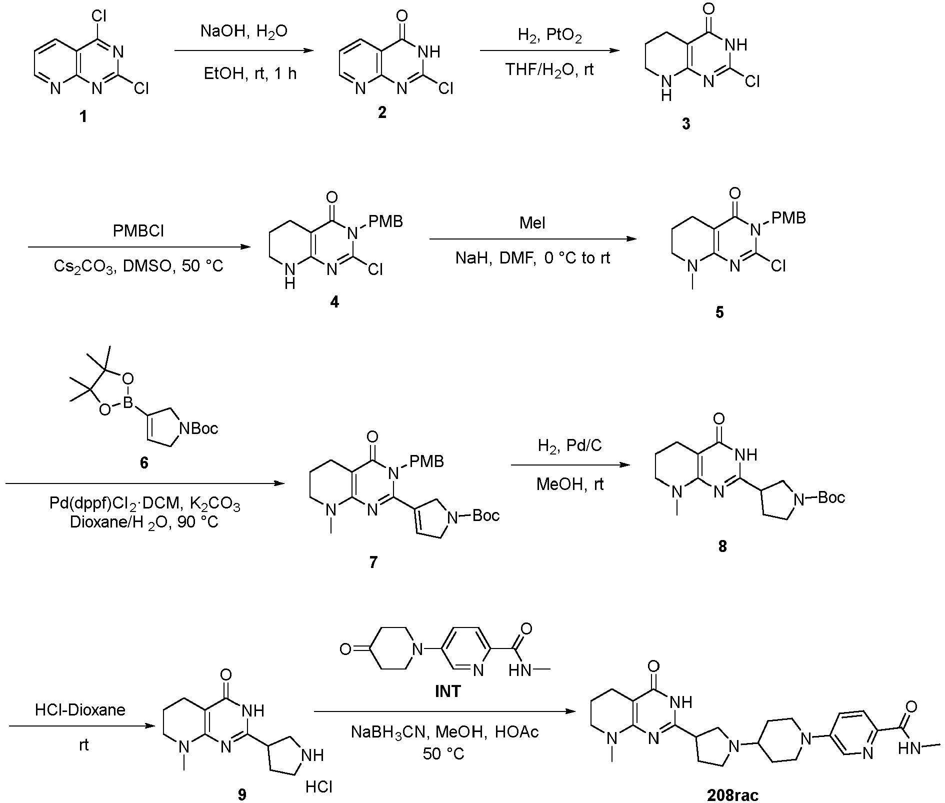

### Preparation of 2-chloropyrido[2,3-d]pyrimidin-4(3H)-one (2)

To a solution of 2,4-dichloropyrido[2,3-d] pyrimidine **1** (1.5 g, 0.0075 mol) in EtOH (30 mL) at room temperature was added NaOH (0.66 g, 0.017 mol) in H₂O (16.5 mL) at rt. The reaction mixture was stirred at rt for 1 h. The reaction solution was quenched with water, then adjusted pH to 4~5 with 1 M HCl. The solution was filtered, and the filter cake was collected and dried *in vacuo* pressure to give 2-chloropyrido[2,3-d]pyrimidin-4(3H)-one 2 (1.1 g, 90% purity, 73% yield) as yellow solid.

LCMS (ESI) calcd for C₇H₄ClN₃O [M + H] ⁺ m/z 182.00, found 181.90.

### Preparation of 2-chloro-5, 6, 7, 8-tetrahydropyrido[2,3-d]pyrimidin-4(3H)-one (3)

To a solution of 2-chloropyrido[2,3-d] pyrimidin-4(3H)-one **2** (1.1 g, 0.0061 mol) in THF/H₂O = 4:1 (50 mL) was added PtO₂ (0.14 g). Then the mixture was stirred under hydrogen for 18 h at rt. The mixture was filtered through a celite pad, and the filtrate was concentrated under reduced pressure to give 2-chloro-5,6,7,8-tetrahydropyrido[2,3-d] pyrimidin-4(3H)-one **3** (1.1 g, 95% purity, 91% yield) as a yellow solid.

LCMS (ESI) calcd for C₇H₈ClN₃O [M + H] ⁺ m/z 186.04, found 185.95.

### Preparation of 2-chloro-3-(4-methoxybenzyl)-5,6,7,8-tetrahydropyrido[2,3-d]pyrimidin-4(3H)-one (4)

To a solution of 2-chloro-5,6,7,8-tetrahydropyrido[2,3-d] pyrimidin-4(3H)-one **3** (0.9 g, 4.85 mmol) in DMSO (150 mL) at room temperature were added Cs₂CO₃ (4.7 g, 14.5 mmol) and PMBCl (1.52 g, 9.7 mmol). The reaction mixture was stirred at 50 °C for 1 h. The reaction solution was quenched with water and extracted with EtOAc (100 mL × 3). The combined organic phase was washed three times with brine and concentrated under reduced pressure. The residue was purified by flash chromatography (eluting with PE/EtOAc = 100 : 0 to 50: 50) to give 2-chloro-3-(4-methoxybenzyl)-5,6,7,8-tetrahydropyrido[2,3-d]pyrimidin-4(3H)-one **4** (1.23 g, 90% purity, 74% yield) as white solid.

LCMS (ESI) calcd for C₁₅H₁₆ClN₃O₂ [M + H] ⁺ m/z 306.09, found 305.80.

### Preparation of 2-chloro-3-(4-methoxybenzyl)-8-methyl-5,6,7,8-tetrahydropyrido[2,3-d]pyrimidin-4(3H)-one (5)

To a solution of 2-chloro-3-(4-methoxybenzyl)-5,6,7,8-tetrahydropyrido[2,3-d]pyrimidin-4(3H)-one **4** (1.2 g, 0.004 mol) in DMF (50 mL) at 0 °C were added MeI (3.05 g, 0.02 mol) and NaH (60% in mineral oil, 0.32 g, 0.008 mol). The reaction mixture was stirred at rt for 2 h. The reaction solution was quenched with water and extracted with EtOAc (100 mL × 3). The combined organic phase was washed three times with brine and concentrated under reduced pressure. The residue was purified by flash chromatography (eluting with PE/EtOAc = 100 : 0 to 50: 50) to give 2-chloro-3-(4-methoxybenzyl)-8-methyl-5,6,7,8-tetrahydropyrido[2,3-d]pyrimidin-4(3H)-one 5 (450 mg, 90% purity, 33 % yield) as yellow oil.

LCMS (ESI) calcd for C₁₆H₁₈ClN₃O₂ [M + H] ⁺ m/z 320.11, found 320.00.

### Preparation of tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate (7)

To a solution of 2-chloro-3-(4-methoxybenzyl)-8-methyl-5,6,7,8-tetrahydropyrido[2,3-d]pyrimidin-4(3H)-one **5** (450 mg, 1.41 mmol) in dioxane/H₂O = 5:1 (30 mL) at room temperature were added tert-butyl 3-(4,4,5,5-tetramethyl-1,3,2-dioxaborolan-2-yl)-2,5-dihydro-1H-pyrrole-1-carboxylate **6** (500 mg, 1.69 mmol), Pd(dppf)Cl₂·DCM (115 mg, 0.14 mmol) and K₂CO₃ (587 mg, 4.22 mmol). The reaction mixture was stirred at 90 °C for 3 h. The reaction solution was quenched with water and extracted with EtOAc (50 mL × 3). The combined organic phase was concentrated under reduced pressure. The residue was purified by flash chromatography (eluting with PE/EtOAc = 100 : 0 to 50: 50) to give tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate 7 (550 mg, 90% purity, 77% yield) as white solid.

LCMS (ESI) calcd for C₂₅H₃₂N₄O₄ [M + H] ⁺ m/z 453.24, found 453.20.

### Preparation of tert-butyl 3-(8-methyl-4-oxo-3,4,5,6,7,8-hexahydropyrido[2,3-d]pyrimidin-2-yl)pyrrolidine-1-carboxylate (8)

To a solution of tert-butyl 3-(3-(4-methoxybenzyl)-8-methyl-4-oxo-3,4,5,6,7,8-hexahydropyrido[2,3-d]pyrimidin-2-yl)-2,5-dihydro-1H-pyrrole-1-carboxylate 7 (550 mg, 1.2 mmol) in MeOH (15 mL) at room temperature were added Pd/C (55 mg). The reaction mixture was stirred at rt for 18 h under a hydrogen atmosphere. The reaction solution was filtered, and the filter cake was washed with MeOH (5 mL × 2). The filtrate was concentrated under reduced pressure to give tert-butyl 3-(8-methyl-4-oxo-3,4,5,6,7,8-hexahydropyrido[2,3-d]pyrimidin-2-yl)pyrrolidine-1-carboxylate **8** (400 mg, 90% purity, 88% yield) as yellow oil.

LCMS (ESI) calcd for C₁₇H₂₆N₄O₃ [M + H] ⁺ m/z 335.20, found 335.30.

### Preparation of 8-methyl-2-(pyrrolidin-3-yl)-5,6,7,8-tetrahydropyrido[2,3-d]pyrimidin-4(3H)-one hydrochloride (9)

A solution of tert-butyl 3-(8-methyl-4-oxo-3,4,5,6,7,8-hexahydropyrido[2,3-d]pyrimidin-2-yl)pyrrolidine-1-carboxylate **8** (200 mg, 0.60 mmol) in HCl-dioxane (4 M, 10 mL) was stirred at room temperature for 2 h under N₂ atmosphere. The precipitate formed was washed with DCM (5 mL × 3), collected and dried under reduced pressure to obtain 8-methyl-2-(pyrrolidin-3-yl)-5,6,7,8-tetrahydropyrido[2,3-d]pyrimidin-4(3H)-one hydrochloride **9** (110 mg, 90% purity, 70% yield) as white solid.

LCMS (ESI) calcd for C₁₂H₁₈N₄O [M + H] ⁺ m/z 235.15, found 235.00.

### Preparation of N-methyl-5-(4-(3-(8-methyl-4-oxo-3,4,5,6,7,8-hexahydropyrido[2,3-d]pyrimidin-2-yl)pyrrolidin-1-yl)piperidin-1-yl)picolinamide (208a and 208b)

To a solution of 8-methyl-2-(pyrrolidin-3-yl)-5,6,7,8-tetrahydropyrido[2,3-d]pyrimidin-4(3H)-one hydrochloride **9** (110 mg, 0.46 mmol) in MeOH (1 mL) was added TEA (1 mL) at rt and stirred for 5 mins, then the reaction was concentrated to dryness under reduced pressure. The residue was dissolved in MeOH (15 mL) and AcOH (0.01 mL) at room temperature. N-methyl-5-(4-oxopiperidin-1-yl)picolinamide **INT** (131 mg, 0.56 mmol) and NaBH₃CN (88 mg, 1.40 mmol) were added. The reaction mixture was stirred at 50 °C for 1 h. The reaction solution was concentrated under reduced pressure and the residue was purified by flash chromatography (eluting with DCM/MeOH = 100 : 0 to 90: 10) to give a racemic mixture of N-methyl-5-(4-(3-(8-methyl-4-oxo-3,4,5,6,7,8-hexahydropyrido[2,3-d]pyrimidin-2-yl)pyrrolidin-1-yl)piperidin-1-yl)picolinamide **208rac** (120 mg, 99% purity, 55 % yield) as a white solid.

LCMS (ESI) calcd for C₂₄H₃₃N₇O₂ [M + H] ⁺ m/z 452.27, found 452.20.

### Chiral resolution of N-methyl-5-(4-(3-(8-methyl-4-oxo-3,4,5,6,7,8-hexahydropyrido[2,3-d]pyrimidin-2-yl)pyrrolidin-1-yl)piperidin-1-yl)picolinamide (208rac)

A racemic mixture of compounds **208a** and **208b** was separated by SFC (Column: Daicel Chiralpak IH SFC, 250 mm ×20 mm I.D., 5 µmm; Mobile phase: CO₂/MeOH [0.1% NH₃] = 70/30) and concentrated under reduced pressure to afford the first fraction as **208a** (52.3 mg, 99% purity, 100% ee, white solid) and the second fraction as **208b** (54.2 mg, 100% purity, 98% ee, white solid).

### Compound 208a

¹H NMR (400 MHz, DMSO-*d₆*, ppm) δ: 11.37 (s, 1 H), 8.43-8.34 (m, 1 H), 8.26 (d, *J* = 2.8 Hz, 1 H), 7.81 (d, *J* = 8.8 Hz, 1 H), 7.39 (dd, *J =* 8.8, 3.2 Hz, 1 H), 3.82-3.77(m, 2 H), 3.23-3.20 (m, 2 H), 3.13-3.09 (m, 1 H), 3.03 (s, 3 H), 2.99-2.92 (m, 3 H), 2.78 (d, *J* = 4.8 Hz, 3 H), 2.68-2.66 (m, 1 H), 2.59-2.57 (m, 1 H), 2.35-2.26 (m, 4 H), 2.07-2.00 (m, 2 H), 1.94-1.88 (m, 2 H), 1.78-1.72 (m, 2 H), 1.53-1.44 (m, 2 H).

### LCMS (ESI) calcd for C₂₄H₃₃N₇O₂ [M + H] ⁺ m/z 452.27, found 452.30.

### Compound 208b

¹H NMR (400 MHz, DMSO-*d*₆, ppm) δ: 11.35 (s, 1 H), 8.41-8.33 (m, 1 H), 8.26 (d, *J* = 2.8 Hz, 1 H), 7.81 (d, *J =* 8.8 Hz, 1 H), 7.39 (dd, *J* = 8.8, 2.8 Hz, 1 H), 3.85-3.75 (m, 2 H), 3.24-3.19 (m, 2 H), 3.14-3.07 (m, 1 H), 3.03 (s, 3 H), 3.00-2.88 (m, 3 H), 2.77 (d, *J* = 4.8 Hz, 3 H), 2.67-2.62 (m, 1 H), 2.61-2.56 (m, 1 H), 2.35-2.21 (m, 4 H), 2.09-1.99 (m, 2 H), 1.96-1.87 (m, 2 H), 1.79-1.70 (m, 2 H), 1.55-1.43 (m, 2 H).

LCMS (ESI) calcd for C₂₄H₃₃N₇O₂ [M + H] ⁺ m/z 452.27, found 452.30.

### Example 20 - Synthesis of INT-3 (an intermediate useful in the synthesis of some compounds of the invention)

### Preparation of tert-butyl 3-(4-fluoro-1-oxo-1,2-dihydroisoquinolin-3-yl)pyrrolidine-1-carboxylate (INT-3)

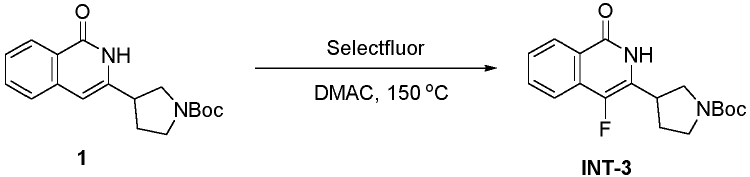

To a solution of tert-butyl 3-(1-oxo-1,2-dihydroisoquinolin-3-yl)pyrrolidine-1-carboxylate **1** (500 mg, 1.59 mmol) in DMAC (20 mL) was added Selectfluor (563 mg, 1.59 mmol) at rt. The mixture was stirred at 150 °C for 10 min. After cooling to rt, the reaction mixture was added into cold water and then extracted with EtOAc (100 mL × 3). The combined organic layer was washed with brine, dried over Na₂SO₄ and concentrated under reduced pressure. The residue was purified by flash silica chromatography (eluting with PE/EtOAc = 100 : 0 to 50 : 50) to give tert-butyl 3-(4-fluoro-1-oxo-1,2-dihydroisoquinolin-3-yl)pyrrolidine-1-carboxylate **INT-3** (100 mg, 60% purity, 11% yield) as yellow oil.

LCMS (ESI) calcd for C₁₈H₂₁FN₂O₃ [M + H] ⁺ m/z 333.15, found 332.90.

### Example 21 - synthesis of compound 259 (an embodiment of the invention)

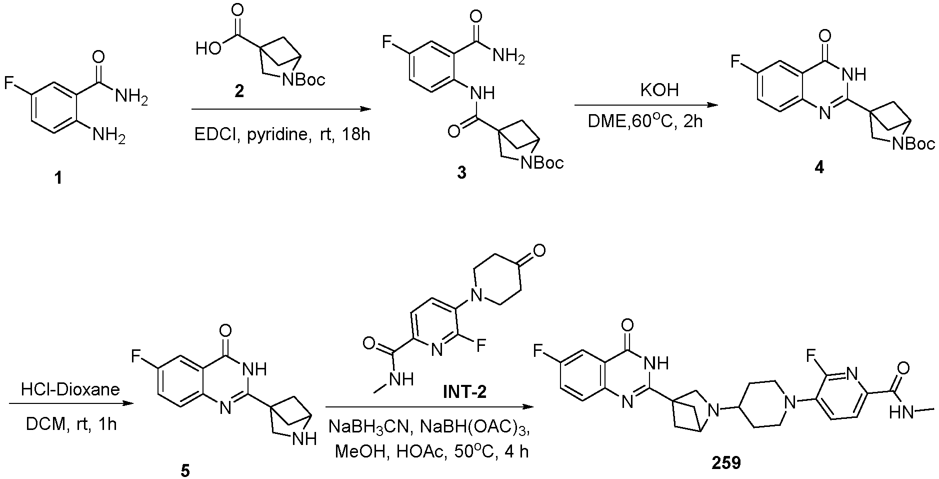

### Preparation of tert-butyl 4-((2-carbamoyl-4-fluorophenyl)carbamoyl)-2-azabicyclo[2.1.1]hexane-2-carboxylate (3)

To a solution of 2-amino-5-fluorobenzamide **1** (30 mg, 2.21 mmol) and 2-(tert-butoxycarbonyl)-2-azabicyclo[2.1.1]hexane-4-carboxylic acid **2** (520 mg, 2.20 mmol) in pyridine (20 mL) was added EDCI (423 mg, 2.21 mmol), then the mixture was stirred for 18 h at room temperature. The reaction mixture was quenched with water, the aqueous layer was extracted with EtOAc (150 mL x 3). The combined organic layers were washed with 1M HCl solution and brine, dried over Na₂SO₄, concentrated under reduced pressure to give product of tert-butyl 4-((2-carbamoyl-4-fluorophenyl)carb amoyl)-2-azabicyclo[2.1.1]hexane-2-carboxylate **3** (500 mg, 56% yield) as a white solid.

LCMS (ESI) calcd for C₁₈H₂₂FN₃O₄ [M + Na] ⁺ m/z 386.16, found 385.95.

### Preparation of tert-butyl 4-(6-fluoro-4-oxo-3,4-dihydroquinazolin-2-yl)-2-azabicyclo[2.1.1]hexane-2-carboxylate (4)

To the solution of *tert*-butyl 4-((2-carbamoyl-4-fluorophenyl)carbamoyl)-2-azabicyclo[2.1.1]hexane-2-carboxylate **3** (500 mg, 1.38 mmol) in DME (50 mL) was added KOH (154 mg, 2.75 mmol). The solution was stirred at 60 °C for 2 h, cooled to room temperature, then the reaction mixture was concentrated under reduced pressure. The residue was purified by flash chromatography (eluting with DCM/MeOH = 100 : 0 to 90 : 10) to give tert-butyl 4-(6-fluoro-4-oxo-3,4-dihydroquinazolin-2-yl)-2-azabicyclo[2.1.1]hexane-2-carboxylate **4** (400 mg, 76% yield ) as a white solid.

LCMS (ESI) calcd for C₁₈H₂₀FN₃O₃ [M + H] ⁺ m/z 346.15, found 345.85.

### Preparation of 2-(2-azabicyclo[2.1.1]hexan-4-yl)-6-fluoroquinazolin-4(3H)-one (5)

To a solution of tert-butyl 4-(6-fluoro-4-oxo-3,4-dihydroquinazolin-2-yl)-2-azabicyclo[2.1.1]hexane-2-carboxylate **4** (400 mg, 1.16 mmol) in DCM (10 mL) was added HCl in dioxane (4M, 20 mL). The mixture was stirred for 1 h at room temperature. The reaction mixture was concentrated under reduced pressure to give product of 2-(2-azabicyclo[2.1.1]hexan-4-yl)-6-fluoroquinazolin-4(3H)-one **5** (200 mg, 63% yield ) as a white solid.

LCMS (ESI) calcd for C₁₅H₁₇N₃O [M + H] ⁺ m/z 246.10, found 245.95.

### Preparation of 6-fluoro-5-(4-(4-(6-fluoro-4-oxo-3,4-dihydroquinazolin-2-yl)-2-azabicyclo[2.1.1]hexan-2-yl)piperidin-1-yl)-N-methylpicolinamide (Compound 259)

To a solution of 2-(2-azabicyclo[2.1.1]hexan-4-yl)-6-fluoroquinazolin-4(3H)-one 5 (60 mg, 0.24 mmol) in MeOH (20 mL) was added 6-fluoro-N-methyl-5-(4-oxopiperidin-1-yl)picolinamide **INT-2** (61 mg, 0.24 mmol), then two drops of acetic acid and NaBH(OAC)₃ (104 mg, 0.49 mmol) were added at room temperature. After 1 h, NaBH₃CN (8 mg, 0.13 mmol) was added. The reaction mixture stirred at 50 ^{°}C for 4 h. After cooling to room temperature, the reaction mixture was concentrated under reduced pressure. The residue was purified by C₁₈ column (mobile phase: ACN - H₂O (0.1% FA), gradient: 10 - 95) to give 6-fluoro-5-(4-(4-(6-fluoro-4-oxo-3,4-dihydroquinazolin-2-yl)-2-azabicyclo[2.1.1]hexan-2-yl)piperidin-1-yl)-N-methylpicolinamide **259** (45.5 mg, 99 % purity, 15% yield) as a white solid.

¹H NMR (400 MHz, DMSO-*d₆*, ppm) δ: 12.29 (s, 1 H), 8.40 (q, 1 H), 7.84 (dd, *J* = 8.0, 1.2 Hz, 1 H), 7.77 (dd, *J* = 8.4, 2.8 Hz, 1 H), 7.72-7.65 (m, 2 H), 7.62-7.56 (m, 1 H), 3.70 (s, 1 H), 3.59-3.53 (m, 2 H), 3.05 (s, 2 H), 2.86 (t, *J* = 11.2 Hz, 2 H), 2.77 (d, *J* = 4.8 Hz, 3 H), 2.57-2.52 (m, 1 H), 2.15-2.09 (m, 2 H), 1.99 (d, *J* = 10.8 Hz, 2 H), 1.92-1.86 (m, 2 H), 1.58-1.47 (m, 2 H).

LCMS (ESI) calcd for C₂₅H₂₆F₂N₆O₂ [M + H] ⁺m/z 481.21, found 481.05.

### Example 22 - synthesis of compound 299 (a reference example, not in accordance with the present claims)

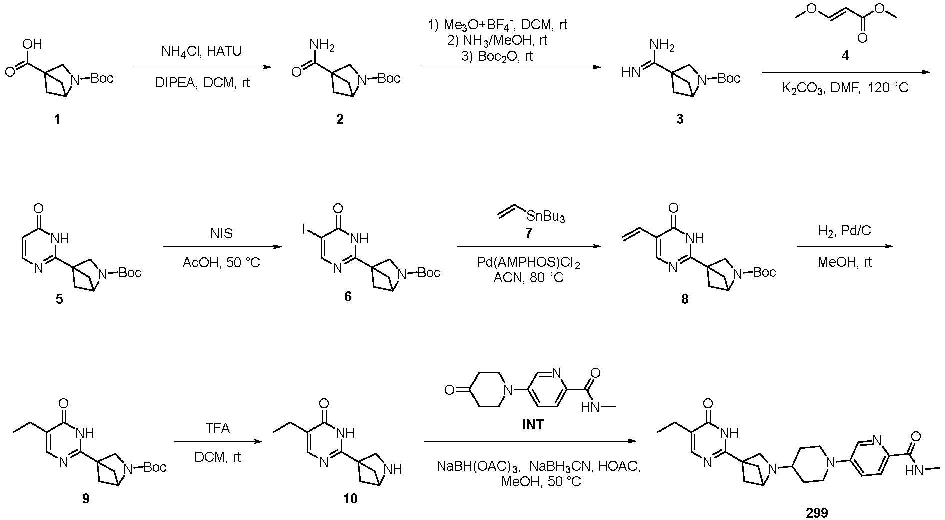

### Preparation of tert-butyl ((1-(6-(methylcarbamoyl)pyridin-3-yl)piperidin-4-yl)methyl)carbamate (2)

To a solution of 2-(tert-butoxycarbonyl)-2-azabicyclo[2.1.1]hexane-4-carboxylic acid **1** (500 mg, 2.19 mmol) in DCM (20 mL) were added NH₄Cl (234 mg, 4.38 mmol), DIPEA (1415 mg, 10.95 mmol) and HATU (1249 mg, 3.28 mmol) at room temperature successively. The mixture was kept stirring at room temperature for 1 h. The resulting mixture was diluted with water and extracted with DCM (50 mL × 3). The combined organic layers were dried over Na₂SO₄ and concentrated under reduced pressure. The residue was purified by flash chromatography (eluting with MeOH/DCM, 3% to 5%) to give tert-butyl 4-carbamoyl-2-azabicyclo[2.1.1]hexane-2-carboxylate 2 (400 mg, 90% purity, 72% yield) as a white solid.

LCMS (ESI) calcd for C₁₁H₁₈N₂O₃ [M - 56 + H] ⁺ m/z 171.13, found 171.10.

### Preparation of tert-butyl 4-carbamimidoyl-2-azabicyclo[2.1.1]hexane-2-carboxylate (3)

Tert-butyl 4-carbamoyl-2-azabicyclo[2.1.1]hexane-2-carboxylate **2** (400 mg, 1.76 mmol) was added to a suspension of Me₃O⁺BF₄⁻ (312 mg, 2.1 mmol) in anhydrous DCM (10 mL). The mixture was stirred under argon atmosphere at rt for 2 h. Solvent was then removed to provide crude imidate salt. This crude was dissolved in NH₃/MeOH (7 M, 10 mL) and stirred at rt for 16 h. Then Boc₂O (1152 mg, 5.28 mmol) was added. The mixture was stirred at rt for 10 min and then concentrated to give crude tert-butyl 4-carbamimidoyl-2-azabicyclo[2.1.1]hexane-2-carboxylate 3 (500 mg, 50% purity, 62% yield) as a white solid.

LCMS (ESI) calcd for C₁₁H₁₉N₃O₂ [M + H] ⁺ m/z 226.15, found 226.05.

### Preparation of tert-butyl 4-(6-oxo-1,6-dihydropyrimidin-2-yl)-2-azabicyclo[2.1.1]hexane-2-carboxylate (5)

To a solution of tert-butyl 4-carbamimidoyl-2-azabicyclo[2.1.1]hexane-2-carboxylate **3** (500 mg, 2.21 mmol) in DMF (20 mL) were added K₂CO₃ (916 mg, 6.63 mmol) and methyl (E)-3-methoxyacrylate **4** (770 mg, 6.63 mmol) in one portion. The reaction mixture was heated with stirring at 120 °C for 2 h. The reaction mixture was concentrated and purified by silica gel column chromatography (eluting with EtOAc/PE, 50% to 100%) to obtain tert-butyl 4-(6-oxo-1,6-dihydropyrimidin-2-yl)-2-azabicyclo[2.1.1]hexane-2-carboxylate 5 (200 mg, 90% purity, 29% yield) as a white solid.

LCMS (ESI) calcd for C₁₄H₁₉N₃O₃ [M + H] ⁺ m/z 278.15, found 277.95.

### Preparation of tert-butyl 4-(5-iodo-6-oxo-1,6-dihydropyrimidin-2-yl)-2-azabicyclo[2.1.1]hexane-2-carboxylate (6)

To a solution of tert-butyl 4-(6-oxo-1,6-dihydropyrimidin-2-yl)-2-azabicyclo[2.1.1]hexane-2-carboxylate **5** (215 mg, 0.77 mmol) in AcOH (5 mL) was added NIS (260 mg, 1.16 mmol) in one portion. The reaction mixture was heated with stirring at 50 °C for 2 h. The reaction mixture was concentrated and purified by silica gel column chromatography (eluting with EtOAc/PE, 50% to 70%) to obtain tert-butyl 4-(5-iodo-6-oxo-1,6-dihydropyrimidin-2-yl)-2-azabicyclo[2.1.1]hexane-2-carboxylate **6** (180 mg, 90% purity, 51% yield) as a white solid.

LCMS (ESI) calcd for C₁₄H₁₈IN₃O₃ [M + H] ⁺ m/z 404.04, found 403.85.

### Preparation of tert-butyl 4-(6-oxo-5-vinyl-1,6-dihydropyrimidin-2-yl)-2-azabicyclo[2.1.1]hexane-2-carboxylate (8)

A solution of tert-butyl 4-(5-iodo-6-oxo-1,6-dihydropyrimidin-2-yl)-2-azabicyclo[2.1.1]hexane-2-carboxylate **6** (180 mg, 0.44 mmol), tributyl(vinyl)stannane **7** (282 mg, 0.89 mmol) and Pd(AMPHOS)Cl₂ (32 mg, 0.04 mmol) in ACN (10 mL) was purged with N₂ for 2 min and then stirred at 80 °C for 1 h. After cooling to rt, the reaction mixture was concentrated and purified by flash chromatography (eluting with EtOAc/PE, 50% to 70%) to give tert-butyl 4-(6-oxo-5-vinyl-1,6-dihydropyrimidin-2-yl)-2-azabicyclo[2.1.1]hexane-2-carboxylate **8** (120 mg, 90% purity, 79% yield) as a white solid.

LCMS (ESI) calcd for C₁₆H₂₁N₃O₃ [M + H] ⁺ m/z 304.16, found 304.20.

### Preparation of tert-butyl 4-(5-ethyl-6-oxo-1,6-dihydropyrimidin-2-yl)-2-azabicyclo[2.1.1]hexane-2-carboxylate (9)

To a solution of give tert-butyl 4-(6-oxo-5-vinyl-1,6-dihydropyrimidin-2-yl)-2-azabicyclo[2.1.1]hexane-2-carboxylate **8** (120 mg, 0.40 mmol) in MeOH (5 mL) was added 10% Pd/C (12 mg). The mixture was evacuated and backfilled with nitrogen three times and then charged with hydrogen. The resulting mixture was stirred at room temperature for 16 hours. Then the mixture was filtered through celite and concentrated under vacuum to give crude tert-butyl 4-(5-ethyl-6-oxo-1,6-dihydropyrimidin-2-yl)-2-azabicyclo[2.1.1]hexane-2-carboxylate **9** (120 mg, 90 % purity, 89 % yield) as a white solid which was used directly in the next step without further purification.

LCMS (ESI) calcd for C₁₆H₂₃N₃O₃ [M + H] ⁺ m/z 306.18, found 306.00.

### Preparation of 2-(2-azabicyclo[2.1.1]hexan-4-yl)-5-ethylpyrimidin-4(3H)-one (10)

To a solution of tert-butyl 4-(5-ethyl-6-oxo-1,6-dihydropyrimidin-2-yl)-2-azabicyclo[2.1.1]hexane-2-carboxylate **9** (120 mg, 0.39 mmol) in DCM (4 mL) was added TFA (1 mL) dropwise at 0 °C. The reaction solution was stirred at room temperature for 1 hour then concentrated under vacuum. The resulted mixture quenched with Et₃N and concentrated under vacuum to afford 2-(2-azabicyclo[2.1.1]hexan-4-yl)-5-ethylpyrimidin-4(3H)-one **10** (120 mg, 50% purity, 74% yield).

LCMS (ESI) calcd for C₁₁H₁₅N₃O [M + H] ⁺ m/z 206.12, found 206.00.

### Preparation of 5-(4-(4-(5-ethyl-6-oxo-1,6-dihydropyrimidin-2-yl)-2-azabicyclo[2.1.1]hexan-2-yl)piperidin-1-yl)-N-methylpicolinamide (299)

To a solution of 2-(2-azabicyclo[2.1.1]hexan-4-yl)-5-ethylpyrimidin-4(3H)-one **10** (40 mg, 0.19 mmol) in MeOH (5 mL) was added N-methyl-5-(4-oxopiperidin-1-yl)picolinamide **INT** (68 mg, 0.29 mmol), then two drops of acetic acid and NaBH(OAc)₃ (82 mg, 0.39 mmol) were added at room temperature. After 1 h, NaBH₃CN (12 mg, 0.19 mmol) was added. The reaction mixture stirred at 50 °C for 1 h. After cooling to room temperature, the reaction mixture was concentrated under reduced pressure. The residue was purified by prep-HPLC (Gemini 5 um C18 150 × 21.2 mm, eluting with 10% to 40% ACN/H₂O containing 0.1% NH₃·H₂O) and prep-TLC (MeOH/DCM, 1/20) to give 5-(4-(4-(5-ethyl-6-oxo-1,6-dihydropyrimidin-2-yl)-2-azabicyclo[2.1.1]hexan-2-yl)piperidin-1-yl)-N-methylpicolinamide **299** (3.2 mg, 99 % purity, 3% yield ) as a white solid.

¹H NMR (400 MHz, DMSO-*d₆*, ppm) δ: 12.30 (s, 1 H), 8.39 (q, *J* = 4.8 Hz, 1 H), 8.27 (d, *J* = 2.8 Hz, 1 H), 7.81 (d, *J =* 8.8 Hz, 1 H), 7.72 (s, 1 H), 7.40 (dd, *J =* 8.8, 2.8 Hz, 1 H), 3.94-3.81 (m, 2 H), 3.64 (s, 1 H), 3.00-2.85 (m, 4 H), 2.78 (d, *J =* 4.8 Hz, 3 H), 2.57-2.52 (m, 1 H), 2.40-2.27 (m, 2 H), 2.07-2.01 (m, 2 H), 1.96-1.91 (m, 2 H), 1.87-1.72 (m, 2 H), 1.50-1.36 (m, 2 H), 1.07 (t, *J =* 7.6 Hz, 3 H).

LCMS (ESI) calcd for C₂₃H₃₀N₆O₂ [M + H] ⁺ m/z 423.25, found 423.15

### Example 23 - synthesis of compounds 309a and 309b (embodiments of the invention)

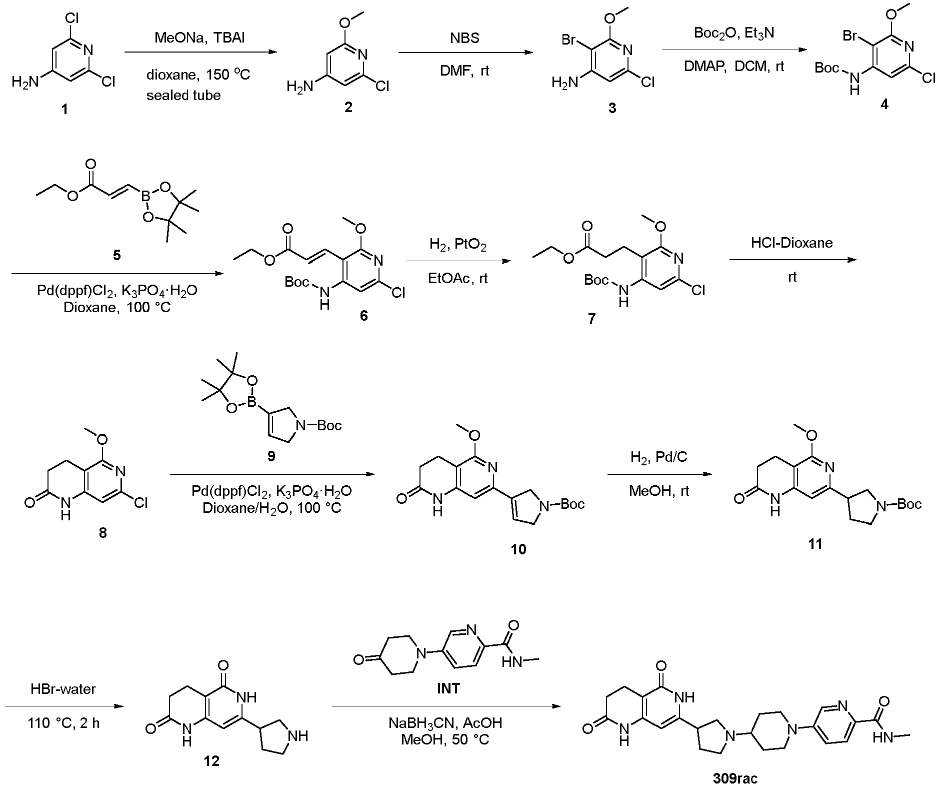

### Preparation of 2-chloro-6-methoxypyridin-4-amine (2)

To a solution of 2,6-dichloropyridin-4-amine **1** (1.0 g, 6.1 mmol) in dioxane (15 mL) was added MeONa (3.3 g, 61.0 mmol) and TBAI (230 mg, 0.6 mmol) at rt. The reaction mixture was stirred at 150 °C for 15 h in a sealed tube. The reaction solution was cooled to rt and concentrated under reduced pressure. The residue was purified by flash column chromatography (eluting with PE/EtOAc = 100 : 0 to 95 : 5) to afford 2-chloro-6-methoxypyridin-4-amine 2 (880 mg, 90% purity, 81% yield) as a white solid.

LCMS (ESI) calcd for C₆H₇ClN₂O [M + H] ⁺ m/z 159.02, found 158.93.

### Preparation of 3-bromo-6-chloro-2-methoxypyridin-4-amine (3)

To a solution of 2-chloro-6-methoxypyridin-4-amine **2** (880 mg, 5.5 mmol) in DMF (40 mL) was added NBS (1.2 g, 6.7 mmol) at rt. The reaction mixture was stirred at rt for 1 h. The reaction mixture was poured into water. The aqueous layer was extracted with EtOAc (100 mL × 3). The combined organic layers were washed with brine (100 mL × 3), dried over Na₂SO₄ and concentrated under reduced pressure. The residue was purified by flash column chromatography (eluting with PE/EtOAc = 100 : 0 to 70 : 30) to afford 3-bromo-6-chloro-2-methoxypyridin-4-amine **3** (950 mg, 90% purity, 64% yield) as a white solid.

LCMS (ESI) calcd for C₆H₆BrClN₂O [M + H] ⁺ m/z 236.94, found 236.87.

### Preparation of tert-butyl (3-bromo-6-chloro-2-methoxypyridin-4-yl)carbamate (4)

To a solution of 3-bromo-6-chloro-2-methoxypyridin-4-amine **3** (950 mg, 4.0 mmol) in DCM (30 mL) was added Boc₂O (1.31 g, 6.0 mmol), Et₃N (810 mg, 8.0 mmol) and DMAP (244 mg, 2.0 mmol) successively at rt. The reaction mixture was stirred at rt for 1 h. The reaction solution was concentrated under reduced pressure. The residue was purified by flash column chromatography (eluting with PE/EtOAc = 100 : 0 to 80 : 20) to afford a mixture of tert-butyl (3-bromo-6-chloro-2-methoxypyridin-4-yl)carbamate **4** and tert-butyl (3-bromo-6-chloro-2-methoxypyridin-4-yl)(tert-butoxycarbonyl)carbamate **4a** (750 mg, 90% purity, 49% yield) as a white solid.

LCMS (ESI) calcd for C₁₁H₁₄BrClN₂O₃ [M + H] ⁺ m/z 336.99, found 336.92.

### Preparation of ethyl (E)-3-(4-((tert-butoxycarbonyl)amino)-6-chloro-2-methoxypyridin-3-yl)acrylate (6)

To a solution of the mixture of tert-butyl (3-bromo-6-chloro-2-methoxypyridin-4-yl)carbamate **4** and tert-butyl (3-bromo-6-chloro-2-methoxypyridin-4-yl)(tert-butoxycarbonyl)carbamate **4a** (750 mg, 2.2 mmol) in dioxane/H₂O (25 mL, 10:1) was added ethyl (E)-3-(4,4,5,5-tetramethyl-1,3,2-dioxaborolan-2-yl)acrylate **5** (751 mg, 3.3 mmol), Pd(dppf)Cl₂·DCM (181 mg, 0.22 mmol) and K₃PO₄·H₂O (1.5 g, 6.6 mmol) successively at rt. The reaction mixture was stirred at 100 °C for 6 h under N₂. The reaction solution was cooled to rt and concentrated under reduced pressure. The residue was purified by flash column chromatography (eluting with PE/EtOAc = 100 : 0 to 70 : 30) to afford a mixture of ethyl (E)-3-(4-((tert-butoxycarbonyl)amino)-6-chloro-2-methoxypyridin-3-yl)acrylate **6** and ethyl (E)-3-(4-(bis(tert-butoxycarbonyl)amino)-6-chloro-2-methoxypyridin-3-yl)acrylate **6a** (410 mg, 90% purity, 46% yield) as a white solid.

LCMS (ESI) calcd for C₁₆H₂₁ClN₂O₅ [M + H] ⁺ m/z 357.11, found 357.00.

### Preparation of ethyl 3-(4-((tert-butoxycarbonyl)amino)-6-chloro-2-methoxypyridin-3-yl)propanoate (7)

A solution of the mixture of ethyl (E)-3-(4-((tert-butoxycarbonyl)amino)-6-chloro-2-methoxypyridin-3-yl)acrylate **6** and ethyl (E)-3-(4-(bis(tert-butoxycarbonyl)amino)-6-chloro-2-methoxypyridin-3-yl)acrylate **6a** (410 mg, 0.9 mmol), and PtO₂ (50 mg) in EtOAc (10 mL) was stirred at rt for 1 h under H₂ atmosphere. The resulting solution was filtered through diatomaceous earth and the filter cake was washed with DCM (20 mL). The filtrate was concentrated under reduced pressure to give a mixture of ethyl 3-(4-((tert-butoxycarbonyl)amino)-6-chloro-2-methoxypyridin-3-yl)propanoate **7** and ethyl 3-(4-(bis(tert-butoxycarbonyl)amino)-6-chloro-2-methoxypyridin-3-yl)propanoate **7a** (210 mg, 90% purity, 45% yield) as a colorless oil.

LCMS (ESI) calcd for C₁₆H₂₃ClN₂O₅ [M + H] ⁺ m/z 359.13, found 359.08.

### Preparation of 7-chloro-5-methoxy-3,4-dihydro-1,6-naphthyridin-2(1H)-one (8)

A solution of the mixture of ethyl 3-(4-((tert-butoxycarbonyl)amino)-6-chloro-2-methoxypyridin-3-yl)propanoate **7** and ethyl 3-(4-(bis(tert-butoxycarbonyl)amino)-6-chloro-2-methoxypyridin-3-yl)propanoate **7a** (210 mg, 0.6 mmol) in HCl-dioxane (10 mL, 4 M) was stirred at rt for 2 h. The reaction mixture was concentrated under reduced pressure to give 7-chloro-5-methoxy-3,4-dihydro-1,6-naphthyridin-2(1H)-one **8** (110 mg, 90% purity, 79% yield) as a white solid.

LCMS (ESI) calcd for C₉H₉ClN₂O₂ [M + H] ⁺ m/z 213.04, found 212.92.

### Preparation of tert-butyl 3-(5-methoxy-2-oxo-1,2,3,4-tetrahydro-1,6-naphthyridin-7-yl)-2,5-dihydro-1H-pyrrole-1-carboxylate (10)

To a solution of 7-chloro-5-methoxy-3,4-dihydro-1,6-naphthyridin-2(1H)-one **8** (110 mg, 0.5 mmol) in dioxane/H₂O (10 mL, 10:1) was added tert-butyl 3-(4,4,5,5-tetramethyl-1,3,2-dioxaborolan-2-yl)-2,5-dihydro-1H-pyrrole-1-carboxylate **9** (184 mg, 0.6 mmol), Pd(dppf)Cl₂ (42 mg, 0.06 mmol) and K₃PO₄· H₂O (357 mg, 1.6 mmol) successively at rt. The reaction mixture was stirred at 100 °C for 6 h under N₂ atmosphere. The reaction solution was cooled to rt and concentrated under reduced pressure. The residue was purified by flash column chromatography (eluting with PE/EtOAc = 100 : 0 to 20 : 80) to afford tert-butyl 3-(5-methoxy-2-oxo-1,2,3,4-tetrahydro-1,6-naphthyridin-7-yl)-2,5-dihydro-1H-pyrrole-1-carboxylate **10** (150 mg, 90% purity, 75% yield) as a white solid.

LCMS (ESI) calcd for C₁₈H₂₃N₃O₄ [M + H] ⁺ m/z 346.17, found 346.05.

### Preparation of tert-butyl 3-(5-methoxy-2-oxo-1,2,3,4-tetrahydro-1,6-naphthyridin-7-yl)pyrrolidine-1-carboxylate (11)

A solution of tert-butyl 3-(5-methoxy-2-oxo-1,2,3,4-tetrahydro-1,6-naphthyridin-7-yl)-2,5-dihydro-1H-pyrrole-1-carboxylate **10** (150 mg, 0.4 mmol) and Pd/C (20 mg) in MeOH (10 mL) was stirred at rt for 2 h under H₂ atmosphere. The resulting solution was filtered through diatomaceous earth and the filter cake was washed with DCM (20 mL × 3). The filtrate was concentrated under reduced pressure to give tert-butyl 3-(5-methoxy-2-oxo-1,2,3,4-tetrahydro-1,6-naphthyridin-7-yl)pyrrolidine-1-carboxylate **11** (140 mg, 90% purity, 83% yield) as a colorless oil.

LCMS (ESI) calcd for C₁₈H₂₅N₃O₄ [M + H] ⁺ m/z 348.18, found 347.95.

### Preparation of 7-(pyrrolidin-3-yl)-4,6-dihydro-1,6-naphthyridine-2,5(1H,3H)-dione (12)

A solution of tert-butyl 3-(5-methoxy-2-oxo-1,2,3,4-tetrahydro-1,6-naphthyridin-7-yl)pyrrolidine-1-carboxylate **11** (120 mg, 0.34 mmol) in HBr (48% in H₂O, 5 mL) was stirred at 110 °C for 2 h. The reaction solution was cooled to rt and concentrated under reduced pressure. The residue was dissolved in MeOH (5 mL) and Et₃N (1 mL) was added dropwise. The reaction mixture was stirred at rt for 5 min. The reaction solution was concentrated under reduced pressure to give 7-(pyrrolidin-3-yl)-4,6-dihydro-1,6-naphthyridine-2,5(1H,3H)-dione **12** (65 mg, 90% purity, 72% yield) as a brown solid.

LCMS (ESI) calcd for C₁₂H₁₅N₃O₂ [M + H] ⁺ m/z 234.12, found 234.06.

### Preparation of 5-(4-(3-(2,5-dioxo-1,2,3,4,5,6-hexahydro-1,6-naphthyridin-7-yl)pyrrolidin-1-yl)piperidin-1-yl)-N-methylpicolinamide (racemic mixture of compounds 309a and 309b)

To a solution of 7-(pyrrolidin-3-yl)-4,6-dihydro-1,6-naphthyridine-2,5(1H,3H)-dione **12** (65 mg, 0.3 mmol) in MeOH (10 mL) was added N-methyl-5-(4-oxopiperidin-1-yl)picolinamide **INT** (78 mg, 0.3 mmol), AcOH (2 drops) and NaBH₃CN (35 mg, 0.6 mmol) and at rt. The reaction mixture was stirred at 50 ° C for 1 h. The reaction mixture was quenched with water (2 mL) and the reaction solution was concentrated under reduced pressure. The residue was purified by flash column chromatography (eluting with DCM/MeOH = 100 : 0 to 90 : 10) to afford 5-(4-(3-(2,5-dioxo-1,2,3,4,5,6-hexahydro-1,6-naphthyridin-7-yl)pyrrolidin-1-yl)piperidin-1-yl)-N-methylpicolinamide a racemic mixture of compounds **309a** and **309b** (35 mg, 98% purity, 27% yield )as a white solid.

### Chiral resolution of 5-(4-(3-(2,5-dioxo-1,2,3,4,5,6-hexahydro-1,6-naphthyridin-7-yl)pyrrolidin-1-yl)piperidin-1-yl)-N-methylpicolinamide (racemic mixture of compounds 309a and 309b)

A racemic mixture of compounds 309a and 309b was separated by SFC (Column: Daicel Chiralpak IH SFC; 20 mm I.D.× 250 mm, 5 µmm; Mobile phase: CO₂/MeOH [0.1% NH₃ (7 M Solution in MeOH)]=60/40) and concentrated under reduced pressure to afford the first fraction as **309a** (4.8 mg, 95% purity, 100% ee, white solid) and the second fraction as **309b** (8.7 mg, 95% purity, 100% ee, white solid).

### Compound 309a

¹H NMR (400 MHz, DMSO-*d*₆, ppm) δ: 11.08 (s, 1 H), 10.04 (s, 1 H), 8.43-8.34 (m, 1 H), 8.27 (d, *J =* 2.8 Hz, 1 H), 7.81 (d, *J =* 8.8 Hz, 1 H), 7.48-7.36 (m, 1 H), 5.83 (s, 1 H), 3.89-3.76 (m, 2 H), 3.14-3.07 (m, 1 H), 3.01-2.91 (m, 2 H), 2.84-2.72 (m, 5 H), 2.60-2.56 (m, 3 H), 2.43-2.41 (m, 3 H), 2.24-2.10 (m, 2 H), 1.96-1.88 (m, 2 H), 1.72-1.62 (m, 1 H), 1.55-1.46 (m, 2 H).

LCMS (ESI) calcd for C₂₄H₃₀N₆O₃ [M + H] ⁺m/z 451.24, found 451.40.

### Compound 309b

¹H NMR (400 MHz, DMSO-*d₆*, ppm) δ: 11.07 (s, 1 H), 10.04 (s, 1 H), 8.46-8.32 (m, 1 H), 8.27 (d, *J =* 2.8 Hz, 1 H), 7.81 (d, *J =* 8.8 Hz, 1 H), 7.40 (dd, *J* = 8.8, 2.8 Hz, 1 H), 5.83 (s, 1 H), 3.87-3.76 (m, 2 H), 3.14-3.04 (m, 1 H), 3.02-2.91 (m, 2 H), 2.84-2.75 (m, 5 H), 2.58-2.54 (m, 3 H), 2.44-2.37 (m, 3 H), 2.30-2.24 (m, 1 H), 2.23-2.12 (m, 1 H), 1.97-1.90 (m, 2 H), 1.75-1.63 (m, 1 H), 1.59-1.44 (m, 2 H).

LCMS (ESI) calcd for C₂₄H₃₀N₆O₃ [M + H] ⁺ m/z 451.24, found 451.20.

### Example 24 - synthesis of compound 324rac (an embodiment of the invention)

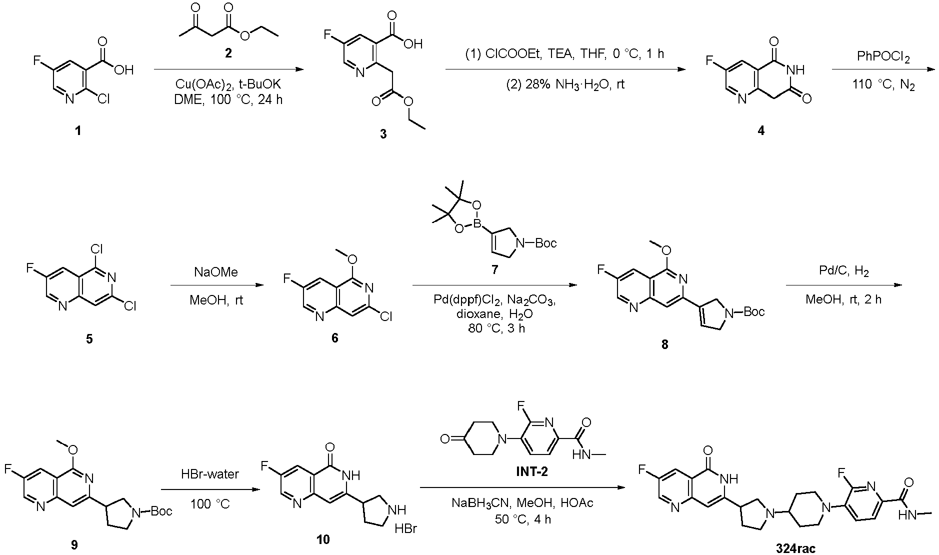

### Preparation of 2-(2-ethoxy-2-oxoethyl)-5-fluoropyridine-3-carboxylic acid (3)

To a solution of ethyl 3-oxobutanoate **2** (4.57 g, 0.0351 mol) in DME (250 mL) was added t-BuOK (5.25 g, 0.0468 mol). The mixture was stirred at room temperature for 1 hour. Cu(OAc)₂ (1.70 g, 0.00936 mol) and 2-chloro-5-fluoropyridine-3-carboxylic acid **1** (4.1 g, 0.0234 mol) were added and the resulting mixture was stirred at 100 °C for 24 hours under nitrogen atmosphere. The mixture was diluted with water, acidified with 1 M HCl to pH = 2 and extracted with ethyl acetate. The combined organic layers were dried over sodium sulfate, filtered, and concentrated under reduced pressure. The residue was purified by column chromatography on silica (DCM/MeOH =100: 0 to 90: 10) to afford 2-(2-ethoxy-2-oxoethyl)-5-fluoropyridine-3-carboxylic acid **3** (2.4 g, 90% purity, 40% yield) as a white solid.

LCMS (ESI) calcd for C₁₀H₁₀FNO₄ [M + H] ⁺ m/z 228.06, found 228.00.

### Preparation of 3-fluoro-6,8-dihydro-1,6-naphthyridine-5,7-dione (4)

To a solution of 2-(2-ethoxy-2-oxoethyl)-5-fluoropyridine-3-carboxylic acid **3** (2400 mg, 10.56 mmol) and TEA (2137 mg, 21.12 mmol) in THF (40 mL) was added ethyl carbonochloridate (2281 mg, 21.12 mmol) dropwise at 0 ⁰C under N₂ atmosphere. The mixture was stirred at this temperature for 1 hour before NH₃·H₂O (28%, 1285 mg) was added dropwise and the resulting mixture was stirred at room temperature for 1 hour. The mixture was diluted with water and extracted with ethyl acetate. The combined organic layers were washed with brine, dried over sodium sulfate, filtered and concentrated. The residue was purified by Biotage Isolera One (C₁₈ column, eluting with 5% to 90% MeCN/H₂O containing 0.1% FA) to afford 3-fluoro-6,8-dihydro-1,6-naphthyridine-5,7-dione **4** (505 mg, 90 % purity, 23 % yield) as a white solid.

LCMS (ESI) calcd for C₈H₅FN₂O₂ [M + H] ⁺ m/z 181.03, found 180.90.

### Preparation of 5,7-dichloro-3-fluoro-1,6-naphthyridine (5)

A solution of 3-fluoro-6,8-dihydro-1,6-naphthyridine-5,7-dione **4** (300 mg, 1.66 mmol) in PhPOCl₂ (3 mL) was stirred at 110 °C for 12 hours under nitrogen atmosphere. The mixture was cooled and adjusted pH to 9 ~ 10 with aqueous KOH (5% wt) and diluted with ethyl acetate. The mixture was washed with brine, dried over anhydrous sodium sulfate, filtered, and concentrated under reduced pressure. The residue was purified by silica gel column chromatography (PE/EtOAc = 100 : 0 to 50 : 50) to afford 5,7-dichloro-3-fluoro-1,6-naphthyridine **5** (125 mg, 90 % purity, 31 % yield) as a white solid.

LCMS (ESI) calcd for C₈H₃C₂FN₂ [M + H] ⁺ m/z 216.97, found 216.90.

### Preparation of 7-chloro-3-fluoro-5-methoxy-1,6-naphthyridine (6)

To a solution of 5,7-dichloro-3-fluoro-1,6-naphthyridine **5** (195 mg, 0.8985 mmol) in MeOH (20 mL) was added MeONa (48 mg, 0.8985 mmol), then stirred for 2 h at room temperature. The reaction mixture was separated with EtOAc (20 mL × 3) and brine. The combined organic phase was concentrated under reduced pressure. The residue was purified by flash chromatography (eluting with PE/EtOAc = 100 : 0 to 95 : 5) to give 7-chloro-3-fluoro-5-methoxy-1,6-naphthyridine **6** (135 mg, 90 % purity, 63 % yield) as white solid.

LCMS (ESI) calcd for C₉H₆ClFN₂O [M + H] ⁺ m/z 213.02, found 212.90.

### Preparation of tert-butyl 3-(3-fluoro-5-methoxy-1,6-naphthyridin-7-yl)-2,5-dihydro-1H-pyrrole-1-carboxylate (8)

A mixture of 7-chloro-3-fluoro-5-methoxy-1,6-naphthyridine **6** (100 mg, 0.470 mmol), tert-butyl 3-(4,4,5,5-tetramethyl-1,3,2-dioxaborolan-2-yl)-2,5-dihydro-1H-pyrrole-1-carboxylate 7 (167 mg, 0.564 mmol), Pd(dppf)Cl₂ (38 mg, 0.047 mmol) and Na₂CO₃ (306 mg, 0.941 mmol) in dioxane and H₂O (3:1, 2 mL) was degassed with N₂ and heated at 80 °C for 3 h under N₂ atmosphere. The reaction mixture was concentrated and purified by flash silica chromatography to obtain tert-butyl 3-(3-fluoro-5-methoxy-1,6-naphthyridin-7-yl)-2,5-dihydro-1H-pyrrole-1-carboxylate **8** (100 mg, 90% purity, 24% yield) as a colorless oil.

LCMS (ESI) calcd for C₁₈H₂₀FN₃O₃ [M + H] ⁺ m/z 346.15, found 346.15.

### Preparation of tert-butyl 3-(3-fluoro-5-methoxy-1,6-naphthyridin-7-yl)pyrrolidine-1-carboxylate (9)

A mixture of 3-(3-fluoro-5-methoxy-1,6-naphthyridin-7-yl)-2,5-dihydro-1H-pyrrole-1-carboxylate **8** (100 mg, 0.289 mmol) and Pd/C (31 mg) in MeOH (2 mL) was degassed with H₂ and stirred at rt for 2 h under H₂ atmosphere. The reaction mixture was filtered. The filtrate was concentrated under reduced pressure to obtain tert-butyl 3-(3-fluoro-5-methoxy-1,6-naphthyridin-7-yl)pyrrolidine-1-carboxylate **9** (100 mg, 60% purity, 59% yield) as a yellow oil.

LCMS (ESI) calcd for C₁₈H₂₂FN₃O₃ [M + H] ⁺ m/z 348.16, found 348.05.

### Preparation of 3-fluoro-7-(pyrrolidin-3-yl)-1,6-naphthyridin-5(6H)-one hydrobromide (10)

A solution of tert-butyl 3-(3-fluoro-5-methoxy-1,6-naphthyridin-7-yl)pyrrolidine-1-carboxylate **9** (100 mg, 0.287 mmol) in HBr-water (48%, 10 mL) was heated at 100 °C for 2 h. The mixture was concentrated under reduced pressure to obtain 3-fluoro-7-(pyrrolidin-3-yl)-1,6-naphthyridin-5(6H)-one hydrobromide **10** (80 mg, 90% purity, 79% yield) as a white solid.

LCMS (ESI) calcd for C₁₂H₁₂FN₃O [M + H] ⁺ m/z 234.10, found 233.95.

### Preparation of 6-fluoro-5-(4-(3-(3-fluoro-5-oxo-5,6-dihydro-1,6-naphthyridin-7-yl)pyrrolidin-1-yl)piperidin-1-yl)-N-methylpicolinamide (compound 324rac)

To a solution of 3-fluoro-7-(pyrrolidin-3-yl)-6H-1,6-naphthyridin-5-one hydrobromide **10** (40 mg, 0.127 mmol) in MeOH (10 mL) was added 6-fluoro-N-methyl-5-(4-oxopiperidin-1-yl)pyridine-2-carboxamide **INT-2** ( 32 mg, 0.127 mmol), then two drops of acetic acid and NaBH₃CN (40 mg, 0.636 mmol) were added at room temperature. The reaction mixture stirred at 50 °C for 4 h. After cooling to room temperature, the reaction mixture was concentrated under reduced pressure. The residue was purified by flash chromatography to give 6-fluoro-5-(4-(3-(3-fluoro-5-oxo-5,6-dihydro-1,6-naphthyridin-7-yl)pyrrolidin-1-yl)piperidin-1-yl)-N-methylpicolinamide racemic **324rac** (13.2 mg, 95 % purity, 21 % yield ) as a white solid.

¹H NMR (400 MHz, DMSO-*d*₆ ppm) δ 11.56 (s, 1 H), 8.92 (d, *J =* 2.8 Hz, 1 H), 8.42-8.35 (m, 1 H), 8.23-8.17 (m, 1 H), 8.16 (s, 1 H), 7.84 (d, *J =* 8.0 Hz, 1 H), 7.62-7.54 (m, 1 H), 6.61 (s, 1 H), 3.54-3.49 (m, 2 H), 3.31-3.27 (m, 1 H), 2.97-2.84 (m, 4 H), 2.83-2.79 (m, 1 H), 2.77 (d, *J* = 4.8 Hz, 3 H), 2.70-2.64 (m, 1 H), 2.38-2.31 (m, 1 H), 2.30-2.23 (m, 1 H), 1.99 (d, *J* = 12.4 Hz, 2 H), 1.92-1.81 (m, 1 H), 1.67-1.55 (m, 2 H).

LCMS (ESI) calcd for C₂₄H₂₆F₂N₆O₂ [M + H] ⁺ m/z 469.21, found 469.10.

### Example 25 - Synthesis of compounds 337a and 337b (embodiments of the invention)

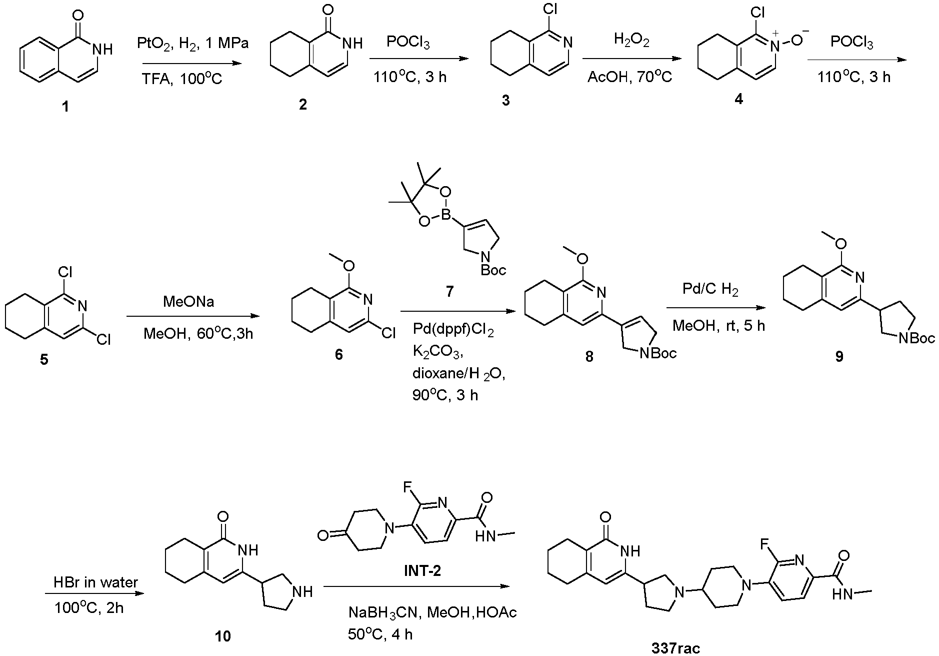

### Preparation of 5,6, 7, 8-tetrahydroisoquinolin-1(2H)-one (2)

2H-isoquinolin-1-one (8 g, 0.05 mol) and PtO₂ (0.9 g, 0.003 mol) were added to TFA (80 mL), stirred under H₂ (1Mpa) at 100 °C for 12h. The precipitate was collected by filtration, extracted with EtOAc (400 mL x 3). The combine organic phases were washed with brine, dried over sodium sulfate, concentrated, and purified by silica gel column chromatography (eluent: PE/EtOAC =100: 0 to 80 :20) to give 5,6,7,8-tetrahydroisoquinolin-1(2H)-one **2** (7.00 g, 77% yield) as a black oil.

LCMS (ESI) calcd for C₉H₁₁NO [M + H] ⁺ m/z 150.08, found 150.00

### Preparation of 1-chloro-5,6,7,8-tetrahydroisoquinoline (3)

A solution of sodium 5,6,7,8-tetrahydro-2H-isoquinolin-1-one **2** (7 g, 0.047 mmol) in POCl₃ (100 mL) was stirred at 110 °C for 3 h. After completion of the reaction, the mixture was cooled to room temperature, taken into ice-water, neutralized with NH₃.H₂O under cooling, extracted with EtOAc (300 mLx3). The combined organic layer was washed with brine, dried over anhydrous Na₂SO₄ and concentrated. The reaction mixture was concentrated under reduced pressure. The residue was purified by flash column chromatography (eluting with PE/EtOAc = 100 : 0 to 80 : 20) to afford 1-chloro-5,6,7,8-tetrahydroisoquinoline **3** (5 g, 90% purity, 57% yield) as a colorless oil.

LCMS (ESI) calcd for C₉H₁₀ClN [M + H] ⁺ m/z 168.05, found 168.00.

### Preparation of 1-chloro-5,6,7,8-tetrahydroisoquinoline 2-oxide (4)

1-chloro-5,6,7,8-tetrahydroisoquinoline **3** (5 g, 0.029 mol) was added to AcOH/H₂O₂ (100 mL). The reaction mixture was stirred at 70 °C for 2 h. The precipitate was collected by filtration, extracted with EtOAc (200 mL x 3). The combine organic phases were washed with brine, dried over sodium sulfate, concentrated, and purified by silica gel column chromatography (eluent: DCM/MeOH =100: 0 to 90 :10) to give 1-chloro-5,6,7,8-tetrahydroisoquinoline 2-oxide **4** (2.00 g, 73% yield) as a yellow oil.

LCMS (ESI) calcd for C₉H₁₀ClNO [M + H] ⁺ m/z 184.05, found 184.00.

### Preparation of 1,3-dichloro-5,6,7,8-tetrahydroisoquinoline (5)

1-chloro-5,6,7,8-tetrahydroisoquinoline 2-oxide **4** (2.00 g, 0.019 mmol) was added to POCl₃ (50 mL) slowly at room temperature. The mixture was heated to 110 °C stirred for 3 h. The resulting mixture was diluted with ice water (200 mL) and extracted with EtOAc (400 mL x 3). The combined organic phases were washed with brine, dried over sodium sulfate, concentrated, and purified by silica gel column chromatography (eluent: PE/EtOAc =100: 0 to 70 :30) to give 1,3-dichloro-5,6,7,8-tetrahydroisoquinoline **5** (370 mg, 15% yield) as a yellow oil.

LCMS (ESI) calcd for C₉H₉Cl₂N [M + H] ⁺ m/z 202.01, found 201.95.

### Preparation of 3-chloro-1-methoxy-5,6,7,8-tetrahydroisoquinoline (6)

To a solution of 1,3-dichloro-5,6,7,8-tetrahydroisoquinoline **5** (350 mg, 1.73 mmol) in MeOH (15 mL) was added MeONa (280 mg, 5.20 mmol), the mixture was heated to 60 °C with stirring for 3h. The precipitate was collected by filtration, extracted with EtOAc (100 mL x 3). The combined organic phases were washed with brine, dried over sodium sulfate, concentrated, and purified by silica gel column chromatography (eluent: PE/EtOAC =100: 0 to 90 :10) to afford 3-chloro-1-methoxy-5,6,7,8-tetrahydroisoquinoline **6** (250 mg, 66% yield) as a white solid.

LCMS (ESI) calcd for C₁₀H₁₂ClNO [M + H] ⁺ m/z 198.06, found 197.95.

### Preparation of tert-butyl 3-(1-methoxy-5,6,7,8-tetrahydroisoquinolin-3-yl)-2,5-dihydro-1H-pyrrole-1-carboxylate (8)

To a solution of 3-chloro-1-methoxy-5,6,7,8-tetrahydroisoquinoline 6 (240 mg, 1.21 mmol) in dioxane/H₂O=10:1 (60 mL) were added tert-butyl 3-(4,4,5,5-tetramethyl-1,3,2-dioxaborolan-2-yl)-2,5-dihydro-1H-pyrrole-1-carboxylate 7 (394 mg, 1.33 mmol), Pd(dppf)Cl₂ (88 mg, 0.12 mmol) and Na₂CO₃ (503 mg, 3.64 mmol) successively at room temperature. The reaction mixture was stirred at 90 °C for 3 h under N₂. The mixture was diluted with water (50 mL) and extracted with EtOAc (100 mL × 3). The combined organic layer was washed with brine (100 mL × 2), dried over Na₂SO₄, filtered and concentrated in vacuo to obtain the crude product, which was purified by flash column chromatography (PE/EtOAc = 100: 0 to 85: 15) to afford tert-butyl 3-(1-methoxy-5,6,7,8-tetrahydroisoquinolin-3-yl)-2,5-dihydro-1H-pyrrole-1-carboxylate **8** (140 mg, 31% yield) as a yellow solid.

LCMS (ESI) calcd for C₁₉H₂₆N₂O₃ [M + H] ⁺ m/z 331.19, found 331.15.

### Preparation of tert-butyl 3-(1-methoxy-5,6,7,8-tetrahydroisoquinolin-3-yl)pyrrolidine-1-carboxylate (9)

To a solution of tert-butyl 3-(1-methoxy-5,6,7,8-tetrahydroisoquinolin-3-yl)-2,5-dihydro-1H-pyrrole-1-carboxylate **8** (130 mg, 0.39 mmol) in MeOH (50 mL) was added Pd/C (30 mg). The mixture was evacuated and backfilled with hydrogen three times and then charged with hydrogen. The resulting mixture was stirred at room temperature for 5 h. Then the mixture was filtered through celite and concentrated under vacuum to give crude product, which was purified by flash column chromatography (PE/EtOAc = 100: 0 to 50: 50) to afford tert-butyl 3-(1-methoxy-5,6,7,8-tetrahydroisoquinolin-3-yl)pyrrolidine-1-carboxylate **9** (130 mg, 89% yield) as a yellow solid.

LCMS (ESI) calcd for C₁₉H₂₈N₂O₃ [M + H] ⁺ m/z 333.21, found 333.10.

### Preparation of 3-(pyrrolidin-3-yl)-5, 6, 7, 8-tetrahydroisoquinolin-1(2H)-one (10)

Tert-butyl 3-(1-methoxy-5,6,7,8-tetrahydroisoquinolin-3-yl)pyrrolidine-1-carboxylate **9** (115 mg, 0.71 mmol) was added to HBr in water (10 mL). The mixture was stirred for 2 h at 100°C. The reaction mixture was concentrated under reduced pressure to give product of 3-(pyrrolidin-3-yl)-5,6,7,8-tetrahydroisoquinolin-1(2H)-one **10** (80 mg, 95% yield) as a white solid.

LCMS (ESI) calcd for C₁₃H₁₈N₂O [M + H] ⁺ m/z 219.14, found 219.05.

### Preparation of 6-fluoro-N-methyl-5-(4-(3-(1-oxo-1,2,5,6,7,8-hexahydroisoquinolin-3-yl)pyrrolidin-1-yl)piperidin-1-yl)picolinamide (racemic mixture of compounds 337a and 337b)

To a solution of 3-(pyrrolidin-3-yl)-5,6,7,8-tetrahydroisoquinolin-1(2H)-one **10** (60 mg, 0.27 mmol), 6-fluoro-N-methyl-5-(4-oxopiperidin-1-yl)pyridine-2-carboxamide **INT-2** (69 mg, 0.27 mmol) and AcOH (two drops) in MeOH (5 mL) was added sodium triacetoxyborohydride (146 mg, 0.69 mmol) and then stirred at 50 °C for 1 h, then NaBH₃CN (21 mg, 0.33 mmol) was added and stirred for 4 h. After cooling to room temperature, the reaction mixture was concentrated under reduced pressure. The residue was purified by flash chromatography (eluting with DCM/MeOH = 100: 0 to 90: 10) to give 6-fluoro-N-methyl-5-(4-(3-(1-oxo-1,2,5,6,7,8-hexahydroisoquinolin-3-yl)pyrrolidin-1-yl)piperidin-1-yl)picolinamide, a racemic mixture of compounds **337a** and **337b** (22 mg, 18 % yield ) as a white solid.

LCMS (ESI) calcd for C₂₅H₃₂FN₅O₂ [M + H] ⁺ m/z 454.25, found 454.15.

### Chiral resolution of 6-fluoro-N-methyl-5-(4-(3-(1-oxo-1,2,5,6,7,8-hexahydroisoquinolin-3-yl)pyrrolidin-1-yl)piperidin-1-yl)picolinamide (racemic mixture of compounds 337a and 337b)

A racemic mixture of compounds **337a** and **337b** was separated by SFC (Column: DAICEL OD-H 4.6mmI.D.*250mmL 5 *µ* m; Mobile phase: CO2/MeOH [0.1%NH3(7M Solution in MeOH)]=70/30) and concentrated under reduced pressure to afford the first fraction as **337a** (7 mg, 95% purity, ee%: 100, white solid) and the second fraction as **337b** (5 mg, 92% purity, ee%: 100, white solid).

### Compound 337a

1H NMR (400 MHz, DMSO) δ 11.03 (s, 1H), 8.40 (d, *J* = 4.8 Hz, 1H), 7.83 (d, *J* = 7.8 Hz, 1H), 7.57 (dd, *J* = 10.6, 8.4 Hz, 1H), 5.83 (s, 1H), 3.55-3.44 (m, 2H), 3.14-3.04 (m, 1H), 2.86 (t, *J =* 11.6 Hz, 3H), 2.75 (t, *J =* 6.4 Hz, 4H), 2.63 (dd, *J =* 14.8, 8.0 Hz, 2H), 2.44 (s, 2H), 2.27 (s, 3H), 2.21-2.10 (m, 1H), 1.95 (d, *J* = 13.2 Hz, 2H), 1.73 (dd, *J =* 12.8, 8.4 Hz, 1H), 1.58 (dd, *J =* 22.4, 12.0 Hz, 6H).

LCMS (ESI) calcd for C₂₅H₃₂FN₅O₂ [M + H] ⁺ m/z 454.25, found 454.20.

### Compound 337b

1H NMR (400 MHz, DMSO) δ 11.03 (s, 1H), 8.40 (d, *J* = 4.4 Hz, 1H), 7.83 (d, *J* = 8.0 Hz, 1H), 7.70-7.45 (m, 1H), 5.83 (s, 1H), 3.49 (d, *J =* 11.2 Hz, 2H), 3.08 (d, *J =* 6.4 Hz, 1H), 2.85 (t, *J* = 11.2 Hz, 3H), 2.76 (d, *J* = 4.4 Hz, 4H), 2.69-2.57 (m, 2H), 2.44 (s, 2H), 2.27 (s, 3H), 2.20-2.10 (m, 1H), 1.95 (d, *J* = 12.4 Hz, 2H), 1.80-1.68 (m, 1H), 1.68-1.51 (m, 6H).

LCMS (ESI) calcd for C₂₅H₃₂FN₅O₂ [M + H] ⁺ m/z 454.25, found 454.15.

### Example 26 - Synthesis of compounds 343a and 343b (embodiments of the invention)

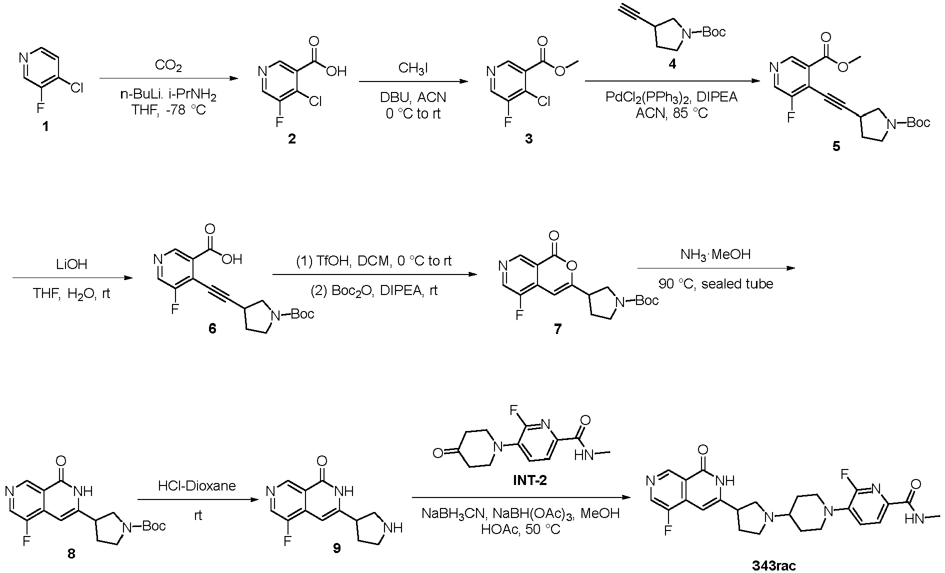

### Preparation of 4-chloro-5-fluoronicotinic acid (2)

N-BuLi (2.4 M in hexanes, 10 mL, 23.9 mmol) was added to THF (100 mL) and cooled to -78 °C. To this solution was added i-PrNH₂ (2.54 g, 25.0 mmol) slowly followed by 4-chloro-3-fluoropyridine **1** (3 g, 22.8 mmol). After stirring for 2 h at -78 °C, crushed dry ice was added in one portion. The reaction mixture was kept stirring until the reaction mixture warmed to room temperature. The reaction was quenched by addition of aqueous ammonium chloride solution and acidified to pH = 2 using conc. HCl and extracted with ethyl acetate (3 x 200 mL). The combined organic layers were washed with brine (10 mL), dried over Na₂SO₄ and concentrated under reduced pressure to afford 4-chloro-5-fluoronicotinic acid **2** (2.2 g, 70% purity, 36% yield) as a yellow solid.

LCMS (ESI) calcd for C₆H₃ClFNO₂ [M + H] ⁺ m/z 175.99, found 175.80.

### Preparation of methyl 4-chloro-5-fluoronicotinate (3)

To a solution of 4-chloro-5-fluoronicotinic acid **2** (2.2 g, 11.6 mmol) in ACN (100 mL) were added DBU (5.29 g, 34.8 mmol) and CH₃I (8.2 g, 58.0 mmol) dropwise at 0 °C. The mixture was stirred at rt for 5 hours. The resulting mixture was diluted with water (300 mL) and extracted with EtOAc (100 mL x 3). The combined organic phases were washed with brine, dried over sodium sulfate, concentrated, and purified by silica gel column chromatography (eluting with EtOAc/PE, 20% to 50%) to give methyl 4-chloro-5-fluoronicotinate **3** (1.5 g, 90% purity, 63% yield) as a white solid.

LCMS (ESI) calcd for C₇H₅ClFNO₂ [M + H] ⁺ m/z 190.00, found 189.95.

### Preparation of methyl 4-((1-(tert-butoxycarbonyl)pyrrolidin-3-yl)ethynyl)-5-fluoronicotinate (5)

A mixture of methyl 4-chloro-5-fluoronicotinate **3** (150 mg, 0.79 mmol), tert-butyl 3-ethynylpyrrolidine-1-carboxylate **4** (233 mg, 1.19 mmol), PdCl₂(PPh₃)₂ (55 mg, 0.079 mmol) and DIPEA (511 mg, 3.96 mmol) in ACN (15 mL) was heated at 85 °C for 5 hours under an atmosphere of N₂. After cooling to ambient temperature, the mixture was filtered through celite, and the filtrate was concentrated under vacuum. The residue was diluted with water and extracted with EtOAc. The combined organic phases were washed with water and brine, dried over sodium sulfate, concentrated under reduced pressure, and purified by flash chromatography (eluting with EtOAc/PE, 20% to 40%) to obtain methyl 4-((1-(tert-butoxycarbonyl)pyrrolidin-3-yl)ethynyl)-5-fluoronicotinate 5 (150 mg, 90% purity, 48% yield) as a yellow solid.

LCMS (ESI) calcd for C₁₈H₂₁FN₂O₄ [M + H] ⁺ m/z 349.15, found 348.95.

### Preparation of 4-((1-(tert-butoxycarbonyl)pyrrolidin-3-yl)ethynyl)-5-fluoronicotinic acid (6)

To a solution of methyl 4-((1-(tert-butoxycarbonyl)pyrrolidin-3-yl)ethynyl)-5-fluoronicotinate **5** (150 mg, 0.43 mmol) in THF/H₂O (3/1, 10 mL) was added LiOH (31 mg, 1.29 mmol). The mixture was stirred at room temperature for 2 h. Then the organic solvent was removed under reduced pressure. The aqueous solution was adjusted to pH 3-4 with 1 M HCl and extracted with DCM (50 mL × 3). The combined organic layer was washed with brine, dried over Na₂SO₄ and concentrated under reduced pressure to give 4-((1-(tert-butoxycarbonyl)pyrrolidin-3-yl)ethynyl)-5-fluoronicotinic acid **6** (120 mg, 90% purity, 75% yield) as a yellow oil.

LCMS (ESI) calcd for C₁₇H₁₉FN₂O₄ [M + H] ⁺ m/z 335.14, found 335.00.

### Preparation of tert-butyl 3-(5-fluoro-1-oxo-1H-pyrano[3,4-c]pyridin-3-yl)pyrrolidine-1-carboxylate (7)

To a solution of 4-((1-(tert-butoxycarbonyl)pyrrolidin-3-yl)ethynyl)-5-fluoronicotinic acid **6** (120 mg, 0.36 mmol) in DCM (10 mL) was added TfOH (161 mg, 1.07 mmol) dropwise at 0 °C. The mixture was stirred at rt for 16 hours. The reaction was quenched with DIPEA (231 mg, 1.79 mmol), then Boc₂O (156 mg, 0.716 mmol) was added. After stirring at rt for 1 h, the resulting mixture were concentrated under reduced pressure and the residue was purified by silica gel column chromatography (eluting with EtOAc/PE, 20% to 40%) to give tert-butyl 3-(5-fluoro-1-oxo-1H-pyrano[3,4-c]pyridin-3-yl)pyrrolidine-1-carboxylate 7 (70 mg, 90% purity, 52% yield) as a white solid.

LCMS (ESI) calcd for C₁₇H₁₉FN₂O₄ [M -t-Bu + H] ⁺ m/z 279.14, found 278.85.

### Preparation of tert-butyl 3-(5-fluoro-1-oxo-1,2-dihydro-2,7-naphthyridin-3-yl)pyrrolidine-1-carboxylate (8)

A solution of tert-butyl 3-(5-fluoro-1-oxo-1H-pyrano[3,4-c]pyridin-3-yl)pyrrolidine-1-carboxylate 7 (70 mg, 0.21 mmol) in NH₃-MeOH (7 M, 5 mL) was stirred at 90 °C for 1 h in a sealed tube. Then the reaction was concentrated under reduced pressure to provide tert-butyl 3-(5-fluoro-1-oxo-1,2-dihydro-2,7-naphthyridin-3-yl)pyrrolidine-1-carboxylate **8** (70 mg, 90% purity, 90% yield) as a yellow solid.

LCMS (ESI) calcd for C₁₇H₂₀FN₃O₃ [M + H] ⁺ m/z 334.15, found 333.95.

### Preparation of 5-fluoro-3-(pyrrolidin-3-yl)-2, 7-naphthyridin-1(2H)-one (9)

A mixture of tert-butyl 3-(5-fluoro-1-oxo-1,2-dihydro-2,7-naphthyridin-3-yl)pyrrolidine-1-carboxylate **8** (70 mg, 0.44 mmol) in HCl dioxane solution (4 M, 5 mL) was stirred at room temperature for 1 hours. The mixture was concentrated under reduced pressure then quenched with Et₃N. The resulting solution was concentrated under vacuum to give 5-fluoro-3-(pyrrolidin-3-yl)-2,7-naphthyridin-1(2H)-one **9** (50 mg, 90% purity, 92% yield) as a yellow oil.

LCMS (ESI) calcd for C₁₂H₁₂FN₃O [M + H] ⁺ m/z 234.10, found 234.05.

### Preparation of 6-fluoro-5-(4-(3-(5-fluoro-1-oxo-1,2-dihydro-2,7-naphthyridin-3-yl)pyrrolidin-1-yl)piperidin-1-yl)-N-methylpicolinamide (racemic mixture of compounds 343a and 343b)

To a solution of 5-fluoro-3-(pyrrolidin-3-yl)-2,7-naphthyridin-1(2H)-one **9** (70 mg, 0.30 mmol) in MeOH (10 mL) was added 6-fluoro-N-methyl-5-(4-oxopiperidin-1-yl)picolinamide **INT-2** (113 mg, 0.45 mmol), then two drops of acetic acid, NaBH₃CN (19 mg, 0.30 mmol) and NaBH(OAc)₃ (191 mg, 0.90 mmol) were added at room temperature. The reaction mixture stirred at 50 °C for 1 h. After cooling to room temperature, the reaction mixture was concentrated under reduced pressure. The residue was purified by flash chromatography (eluting with DCM/MeOH = 100 : 0 to 90 : 10) to give 6-fluoro-5-(4-(3-(5-fluoro-1-oxo-1,2-dihydro-2,7-naphthyridin-3-yl)pyrrolidin-1-yl)piperidin-1-yl)-N-methylpicolinamide, a racemic mixture of compounds **343a** and **343b** (50 mg, 95% purity, 33% yield) as a white solid.

### Chiral resolution of 6-fluoro-5-(4-(3-(5-fluoro-1-oxo-1,2-dihydro-2,7-naphthyridin-3-yl)pyrrolidin-1-yl)piperidin-1-yl)-N-methylpicolinamide (racemic mixture of compounds 343a and 343b)

A racemic mixture of compounds 343a and 343b was separated by SFC (Column: Daicel Chiralpak IH 20 mm I.D. × 250 mm, 5 µm; Mobile phase: CO₂/MeOH [0.1% (NH₃)] = 70/30) and concentrated under reduced pressure to afford the first fraction as **343a** (19.7 mg, 99% purity, 100% ee, white solid) and the second fraction as **343b** (24.7 mg, 98% purity, 90% ee, white solid)

### Compound 343a

¹H NMR (400 MHz, DMSO-*d*₆, ppm) δ: 11.74 (s, 1 H), 9.09 (s, 1 H), 8.68 (d, *J* = 2.0 Hz, 1 H), 8.40 (d, *J =* 4.8 Hz, 1 H), 7.87-7.80 (m, 1 H), 7.58 (dd, *J =* 10.6, 8.2 Hz, 1 H), 6.54 (s, 1 H), 3.58-3.47 (m, 2 H), 3.29-3.16 (m, 1 H), 2.91-2.83 (m, 5 H), 2.77 (d, *J* = 4.8 Hz, 3 H), 2.72-2.58 (m, 1 H), 2.47-2.23 (m, 2 H), 2.10-1.96 (m, 2 H), 1.95-1.81 (m, 1 H), 1.77-1.50 (m, 2 H).

LCMS (ESI) calcd for C₂₄H₂₆F₂N₆O₂ [M + H] ⁺ m/z 469.21, found 469.07.

### Compound 343a

¹H NMR (400 MHz, DMSO-*d*₆, ppm) δ: 11.77 (s, 1 H), 9.09 (s, 1 H), 8.68 (d, *J* = 2.0 Hz, 1 H), 8.45-8.31 (m, 1 H), 7.89-7.76 (m, 1 H), 7.58 (dd, *J =* 10.6, 8.2 Hz, 1 H), 6.55 (s, 1 H), 3.54-3.46 (m, 2 H), 3.29-3.23 (m, 1 H), 2.94-2.82 (m, 5 H), 2.77 (d, *J* = 4.8 Hz, 3 H), 2.69-2.58 (m, 1 H), 2.36-2.25 (m, 2 H), 2.02-1.94 (m, 2 H), 1.91-1.78 (m, 1 H), 1.67-1.56 (m, 2 H).

LCMS (ESI) calcd for C₂₄H₂₆F₂N₆O₂ [M + H] ⁺ m/z 469.21, found 469.10.

### Example 27 - Synthesis of compound 361 (an embodiment of the invention)

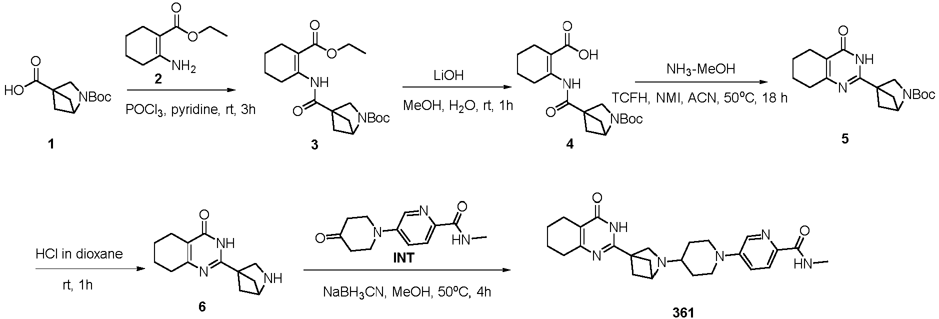

### Preparation of tert-butyl 4-((2-(ethoxycarbonyl)cyclohex-1-en-1-yl)carbamoyl)-2-azabicyclo[2.1.1]hexane-2-carboxylate (3)

To a solution of 2-(tert-butoxycarbonyl)-2-azabicyclo[2.1.1]hexane-4-carboxylic acid **1** (110 mg, 0.48 mmol) in pyridine (10 mL) was added ethyl 2-aminocyclohex-1-ene-1-carboxylate **2** (160 mg, 0.95 mmol) at room temperature, then added 10 drops of POCl₃. The reaction mixture stirred at room temperature for 3 h. After cooling to room temperature, the reaction mixture was poured into water, The aqueous was extracted with organic solvent (50 mL) 3 times. The combined organic layers were washed with brine (50 mL) and dried over Na₂SO₄, then concentrated under reduced pressure. The residue was purified by flash chromatography (eluting with PE/EtOAc = 100: 0 to 85: 15) to give tert-butyl 4-((2-(ethoxycarbonyl)cyclohex-1-en-1-yl)carbamoyl)-2-azabicyclo[2.1.1]hexane-2-carboxylate 3 (100 mg, 55% yield ) as a white solid.

LCMS (ESI) calcd for C20H30N2O5 [M - 56 + H] ⁺ m/z 323.22, found 323.05.

### Preparation of 2-(2-(tert-butoxycarbonyl)-2-azabicyclo[2.1.1]hexane-4-carboxamido)cyclohex-1-ene-1-carboxylic acid (4)

To a solution of tert-butyl 4-((2-(ethoxycarbonyl)cyclohex-1-en-1-yl)carbamoyl)-2-azabicyclo[2.1.1]hexane-2-carboxylate **3** (100 mg, 0.26 mmol) in MeOH : H2O=1 : 1 (10 mL) was added LiOH (30 mg, 1.25 mmol).The reaction mixture stirred at room temperature for 1 h. The reaction mixture was concentrated under reduced pressure to give 2-(2-(tert-butoxycarbonyl)-2-azabicyclo[2.1.1]hexane-4-carboxamido)cyclohex-1-ene-1-carboxylic acid **4** (80 mg, 88 % yield ) as a white solid.

LCMS (ESI) calcd for C18H26N2O5 [M - 56 + H] ⁺ m/z 295.18, found 295.05.

### Preparation of tert-butyl 4-(4-oxo-3,4,5,6,7,8-hexahydroquinazolin-2-yl)-2-azabicyclo[2.1.1]hexane-2-carboxylate (5)

To a solution of 2-(2-(tert-butoxycarbonyl)-2-azabicyclo[2.1.1]hexane-4-carboxamido)cyclohex-1-ene-1-carboxylic acid **4** (80 mg, 0.23 mmol) in ACN (10 mL) was added N,N,N',N'-tetramethylchloroformamidinium hexafluorophosphate / TCFH (150 mg, 0.54 mmol) and N-methyl imidazole / NMI (50 mg, 0.61 mmol) at room temperature. After 10 mins, excess of NH₃-MeOH (7 M, 5 mL) was added at 50 °C. The mixture was stirred at 50 °C for 18 h. The residue was concentrated under reduced pressure, then purified by flash chromatography (eluting with DCM/MeOH = 100: 0 to 96: 4) to give tert-butyl 4-(4-oxo-3,4,5,6,7,8-hexahydroquinazolin-2-yl)-2-azabicyclo[2.1.1]hexane-2-carboxylate **5** (60 mg, 79% yield ) as a white solid.

LCMS (ESI) calcd for C18H25N3O3 [M + H] ⁺ m/z 332.19, found 332.10.

### Preparation of 2-(2-azabicyclo[2.1.1]hexan-4-yl)-3,6,7,8-tetrahydroquinazolin-4(3H)-one (6)

Tert-butyl 4-(4-oxo-3,4,5,6,7,8-hexahydroquinazolin-2-yl)-2-azabicyclo[2.1.1]hexane-2-carboxylate **5** (60 mg, 0.18 mmol) was added to HCl in dioxane (4 M, 3 mL), stirred for 1 h at room temperature, the reaction mixture was concentrated under reduced pressure to give 2-(2-azabicyclo[2.1.1]hexan-4-yl)-5,6,7,8-tetrahydroquinazolin-4(3H)-one **6** (40 mg, 96 % yield ) as a white solid.

LCMS (ESI) calcd for C13H17N3O [M + H] ⁺ m/z 232.14, found 232.05.

### Preparation of N-methyl-5-(4-(4-(4-oxo-3,4,5,6,7,8-hexahydroquinazolin-2-yl)-2-azabicyclo[2.1.1]hexan-2-yl)piperidin-1-yl)picolinamide (compound 361)

To a solution of 2-(2-azabicyclo[2.1.1]hexan-4-yl)-5,6,7,8-tetrahydroquinazolin-4(3H)-one **6** (40 mg, 0.17 mmol) in MeOH (10 mL) was added N-methyl-5-(4-oxopiperidin-1-yl)picolinamide **INT** (40 mg, 0.17 mmol) and 3 drops of HOAc, then added NaBH₃CN (10 mg, 0.16 mmol). The reaction mixture stirred at 50 °C for 4 h. After cooling to room temperature, the reaction mixture was concentrated under reduced pressure. The residue was purified by prep-HPLC (columns: Gemini 5 um C18 150 × 21.2 mm, mobile phase: ACN - H₂O (0.1% FA), gradient: 10 - 25) to give N-methyl-5-(4-(4-(4-oxo-3,4,5,6,7,8-hexahydroquinazolin-2-yl)-2-azabicyclo[2.1.1]hexan-2-yl)piperidin-1-yl)picolinamide **361** (30 mg, 97% purity, 38% yield) as a white solid.

### Compound 361

¹H NMR (400 MHz, DMSO) δ 12.11 (s, 1 H), 8.38 (q, *J =* 4.8 Hz, 1 H), 8.28 (d, *J = 2.8* Hz, 1 H), 7.82 (d, *J =* 8.8 Hz, 1 H), 7.40 (dd, *J =* 8.8, 2.8 Hz, 1 H), 3.87 (d, *J =* 12.8 Hz, 2 H), 3.69 (s, 1 H), 2.98 (s, 2 H), 2.91 (t, *J =* 11.2 Hz, 2 H), 2.78 (d, *J =* 4.8 Hz, 3 H), 2.61-2.54 (m, 1 H), 2.48-2.44 (m, 2 H), 2.31 (t, *J =* 6.0 Hz, 2 H), 2.09-2.02 (m, 2 H), 1.99-1.92 (m, 2 H), 1.84-1.78 (m, 2 H), 1.72-1.59 (m, 4 H), 1.51-1.41 (m, 2 H).

LCMS (ESI) calcd for C25H32N6O2 [M + H] ⁺ m/z 449.26, found 449.20.

### Example 28 - Synthesis of compound 375 (an embodiment of the invention)

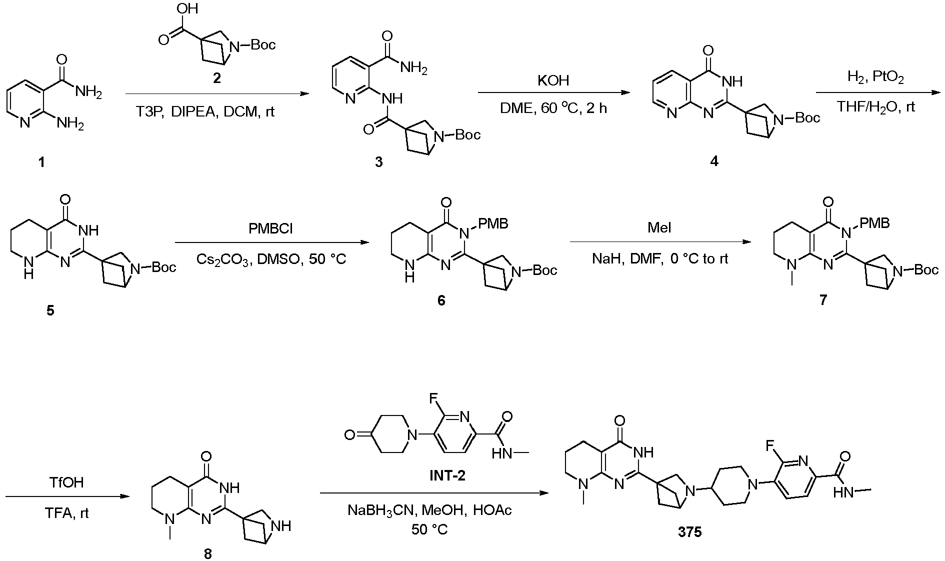

### Preparation of tert-butyl 4-((3-carbamoylpyridin-2-yl)carbamoyl)-2-azabicyclo[2.1.1]hexane-2-carboxylate (3)

To a solution of 2-(tert-butoxycarbonyl)-2-azabicyclo[2.1.1]hexane-4-carboxylic acid **2** (500 mg, 2.19 mmol) in DCM (15 mL) were added 2-aminonicotinamide **1** (330 mg, 2.41 mmol), T3P (50% wt in EtOAc, 4.18 g, 6.57 mmol) and DIPEA (850 mg, 6.57 mmol) at room temperature successively. The mixture was kept stirring at room temperature for 1 h. The reaction solution was concentrated under reduced pressure and the residue was purified by flash chromatography (eluting with DCM/ MeOH = 100 : 0 to 90 : 10) to give tert-butyl 4-((3-carbamoylpyridin-2-yl)carbamoyl)-2-azabicyclo[2.1.1]hexane-2-carboxylate **3** (330 mg, 90% purity, 39% yield) as white solid.

LCMS (ESI) calcd for C₁₇H₂₂N₄O₄ [M + H] ⁺ m/z 347.16, found 346.85.

### Preparation of tert-butyl 4-(4-oxo-3,4-dihydropyrido[2,3-d]pyrimidin-2-yl)-2-azabicyclo[2.1.1]hexane-2-carboxylate (4)

To a solution of tert-butyl 4-((3-carbamoylpyridin-2-yl)carbamoyl)-2-azabicyclo[2.1.1]hexane-2-carboxylate **3** (330 mg, 0.95 mmol) in DME (20 mL) at rt was added KOH (160 mg, 2.85 mmol). The reaction mixture was stirred at 60 °C for 6 h. The reaction solution was filtered to remove KOH. The filtrate was diluted with water and extracted with EtOAc (10 mL × 3). The water phase was purified by silica gel C18 column (eluting with 10% to 50% MeCN/H₂O, containing 0.1% formic acid) to give tert-butyl 4-(4-oxo-3,4-dihydropyrido[2,3-d]pyrimidin-2-yl)-2-azabicyclo[2.1.1]hexane-2-carboxylate **4** (220 mg, 80% purity, 56% yield) as white solid.

LCMS (ESI) calcd for C₁₇H₂₀N₄O₃ [M + H] ⁺ m/z 329.15, found 329.00.

### Preparation of tert-butyl 4-(4-oxo-3,4,5,6,7,8-hexahydropyrido[2,3-d]pyrimidin-2-yl)-2-azabicyclo[2.1.1]hexane-2-carboxylate (5)

To a solution of tert-butyl 4-(4-oxo-3,4-dihydropyrido[2,3-d]pyrimidin-2-yl)-2-azabicyclo[2.1.1]hexane-2-carboxylate **4** (200 mg, 0.607 mmol) in THF/H₂O = 4:1 (20 mL) was added PtO₂ (41 mg). Then the mixture was stirred under hydrogen for 18 h at rt. The mixture was filtered through a celite pad, and the filtrate was concentrated under reduced pressure to give tert-butyl 4-(4-oxo-3,4,5,6,7,8-hexahydropyrido[2,3-d]pyrimidin-2-yl)-2-azabicyclo[2.1.1]hexane-2-carboxylate **5** (200 mg, 90% purity, 88% yield) as a yellow solid.

LCMS (ESI) calcd for C₁₇H₂₄N₄O₃ [M + H] ⁺ m/z 333.18, found 333.25.

### Preparation of tert-butyl 4-(3-(4-methoxybenzyl)-4-oxo-3,4,5,6,7,8-hexahydropyrido[2,3-d]pyrimidin-2-yl)-2-azabicyclo[2.1.1]hexane-2-carboxylate (6)

To a solution of tert-butyl 4-(4-oxo-3,4,5,6,7,8-hexahydropyrido[2,3-d]pyrimidin-2-yl)-2-azabicyclo[2.1.1]hexane-2-carboxylate **5** (200 mg, 0.6 mmol) in DMSO (10 mL) at room temperature were added Cs₂CO₃ (586 mg, 1.8 mmol) and PMBCl (188 mg, 1.2 mmol). The reaction mixture was stirred at 50 °C for 1 h. The reaction solution was quenched with water and extracted with EtOAc (30 mL × 3). The combined organic phase was washed three times with brine and concentrated under reduced pressure. The residue was purified by flash chromatography (eluting with PE/EtOAc = 100 : 0 to 50: 50) to give tert-butyl 4-(3-(4-methoxybenzyl)-4-oxo-3,4,5,6,7,8-hexahydropyrido[2,3 -d]pyrimidin-2-yl)-2-azabicyclo[2.1.1]hexane-2-carboxylate **6** (150 mg, 90% purity, 49% yield) as white solid.

LCMS (ESI) calcd for C₂₅H₃₂N₄O₄ [M + H] ⁺ m/z 453.24, found 453.05.

### Preparation of tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate (7)

To a solution of methyl tert-butyl 4-(3-(4-methoxybenzyl)-4-oxo-3,4,5,6,7,8-hexahydropyrido[2,3-d]pyrimidin-2-yl)-2-azabicyclo[2.1.1]hexane-2-carboxylate **6** (50 mg, 0.11 mmol) in DMF (10 mL) at 0 °C were added NaH (7 mg, 0.17 mmol, 60% wt) and MeI (24 mg, 0.17 mmol). The reaction mixture was stirred at rt for 1 h. The reaction solution was quenched with water and extracted with EtOAc (50 mL × 3). The organic phase was concentrated under reduced pressure and the residue was purified by flash chromatography (eluting with PE/EtOAc = 100 : 0 to 70: 30) to give tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate 7 (30 mg, 90% purity, 52% yield) as white solid.

LCMS (ESI) calcd for C₂₆H₃₄N₄O₄ [M + H] ⁺ m/z 467.26, found 467.30.

### Preparation of 2-(2-azabicyclo[2.1.1]hexan-4-yl)-8-methyl-5,6,7,8-tetrahydropyrido[2,3-d]pyrimidin-4(3H)-one (8)

To a solution of tert-butyl 4-(3-(4-methoxybenzyl)-8-methyl-4-oxo-3,4,5,6,7,8-hexahydropyrido[2,3-d]pyrimidin-2-yl)-2-azabicyclo[2.1.1]hexane-2-carboxylate **7** (30 mg, 0.064 mmol) in TFA (5 mL)was added TfOH (0.2 mL). The reaction was stirred at rt for 0.5 h. The reaction solution was adjusted to pH 8 with saturated aqueous NaHCO₃ at 0 °C. The basified solution was extracted with DCM (10 mL × 3). The combined organic layer was washed with brine, dried over Na₂SO₄ and concentrated under reduced pressure to give 2-(2-azabicyclo[2.1.1]hexan-4-yl)-8-methyl-5,6,7,8-tetrahydropyrido[2,3-d]pyrimidin-4(3H)-one **8** (25 mg, 60% purity, 94% yield) as a yellow oil.

LCMS (ESI) calcd for C₁₃H₁₈N₄O [M + H] ⁺ m/z 247.15, found 247.25.

### Preparation of 6-fluoro-N-methyl-5-(4-(4-(8-methyl-4-oxo-3,4,5,6,7,8-hexahydropyrido[2,3-d]pyrimidin-2-yl)-2-azabicyclo[2.1.1]hexan-2-yl)piperidin-1-yl)picolinamide (compound 375)

To a solution of 2-(2-azabicyclo[2.1.1]hexan-4-yl)-8-methyl-5,6,7,8-tetrahydropyrido[2,3-d]pyrimidin-4(3H)-one **8** (25 mg, 0.10 mmol) in MeOH (15 mL) and AcOH (0.01 mL) at room temperature was added 6-fluoro-N-methyl-5-(4-oxopiperidin-1-yl)picolinamide **INT-2** (31 mg, 0.12 mmol) and NaBH₃CN (10 mg, 0.15 mmol). The reaction mixture was stirred at 50 °C for 1 h. The reaction solution was concentrated under reduced pressure and the residue was purified by flash chromatography (eluting with DCM/MeOH = 100 : 0 to 90: 10) and prep-HPLC (Gemini 5um C18 150 × 21.2mm, mobile phase: ACN - H₂O (0.05% NH₃), gradient: 25 - 95) to give 6-fluoro-N-methyl-5-(4-(4-(8-methyl-4-oxo-3,4,5,6,7,8-hexahydropyrido[2,3-d]pyrimidin-2-yl)-2-azabicyclo[2.1.1]hexan-2-yl)piperidin-1-yl)picolinamide **375** (5.0 mg, 97% purity, 10 % yield) as white solid.

¹H NMR (400 MHz, DMSO-*d*₆, ppm) δ: 11.32 (s, 1 H), 8.43-8.35 (m, 1 H), 7.83 (d, *J* = 8.0 Hz, 1 H), 7.63-7.53 (m, 1 H), 3.61 (s, 1 H), 3.57-3.50 (m, 2 H), 3.25-3.18 (m, 2 H), 3.03 (s, 3 H), 2.91 (s, 2 H), 2.84 (t, *J* = 11.2 Hz, 2 H), 2.76 (d, *J* = 4.8 Hz, 3 H), 2.47-2.42 (m, 1 H), 2.37-2.31 (m, 2 H), 2.03-1.90 (m, 4 H), 1.80-1.71 (m, 4 H), 1.55-1.42 (m, 2 H).

LCMS (ESI) calcd for C₂₅H₃₂FN₇O₂ [M + H] ⁺ m/z 482.26, found 482.15.

### Example 29 - Synthesis of compound 393rac (an embodiment of the invention)

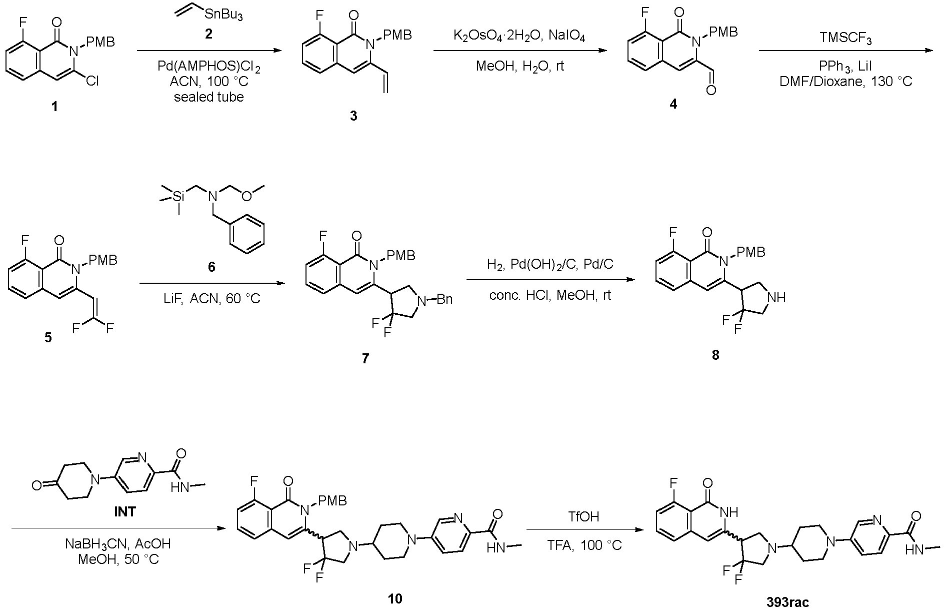

### Preparation of 8-fluoro-2-(4-methoxybenzyl)-3-vinylisoquinolin-1(2H)- (3)

To a solution of 3-chloro-8-fluoro-2-(4-methoxybenzyl)isoquinolin-1(2H)-one 1 (2000 mg, 6.294 mmol) and Pd(AMPHOS)Cl₂ (445 mg, 0.6294 mmol) in ACN (30 mL) was added tributyl(vinyl)stannane 2 (1995 mg, 6.294 mmol). The mixture was heated at 100 °C in sealed tube for 5 h. After cooling to room temperature, the reaction mixture was concentrated under reduced pressure. The residue was purified by flash chromatography (eluting with EtOAc/PE, 0 to 15%) to give 8-fluoro-2-(4-methoxybenzyl)-3-vinylisoquinolin-1(2H)-one 3 (1500 mg, 90% purity, 69% yield) as a white solid.

LCMS (ESI) calcd for C₁₉H₁₆FNO₂ [M + H] ⁺ m/z 310.12, found 309.90.

### Preparation of 8-fluoro-2-(4-methoxybenzyl)-1-oxo-1,2-dihydroisoquinoline-3-carbaldehyde (4)

To a solution of 8-fluoro-2-(4-methoxybenzyl)-3-vinylisoquinolin-1(2H)-one **3** (1500 mg, 4.85 mmol) in MeOH/H₂O (3:1, 80 mL) was added NaIO₄ (4150 mg, 19.40 mmol) and K₂OsO₄·2H₂O (178 mg, 0.48 mmol). The reaction stirred for 1 h at room temperature. The reaction mixture was diluted with water and extracted with EtOAc (200 mL x 3). The combined organic layers were washed by brine, dried over Na₂SO₄ and concentrated under reduced pressure. The residue was purified by flash chromatography on silica gel (eluting with EtOAc/PE, 0 to 25%) to give 8-fluoro-2-(4-methoxybenzyl)-1-oxo-1,2-dihydroisoquinoline-3-carbaldehyde **4** (430 mg, 90% purity, 25% yield) as a yellow solid.

LCMS (ESI) calcd for C₁₈H₁₄FNO₃ [M + H] ⁺ m/z 312.10, found 311.90.

### Preparation of 3-(2,2-difluorovinyl)-8-fluoro-2-(4-methoxybenzyl)isoquinolin-1(2H)-one (5)

To a solution of 8-fluoro-2-(4-methoxybenzyl)-1-oxo-1,2-dihydroisoquinoline-3-carbaldehyde **4** (430 mg, 1.38 mmol), PPh₃ (724 mg, 2.76 mmol) and LiI (369 mg, 2.76 mmol) in DMF/Dioxane (8%, 10 mL) was added TMSCF₃ (491 mg, 3.45 mmol). The mixture was heated at 130 °C in a sealed tube for 3 h. After cooling to room temperature, the reaction mixture was concentrated under reduced pressure. The residue was purified by flash chromatography (eluting with EtOAc/PE, 0 to 15%) to give 3-(2,2-difluorovinyl)-8-fluoro-2-(4-methoxybenzyl)isoquinolin-1(2H)-one 5 (200 mg, 60% purity, 25% yield) as a white solid.

LCMS (ESI) calcd for C₁₉H₁₄F₃NO₂ [M + H] ⁺ m/z 346.10, found 346.00.

### Preparation of 3-(1-benzyl-4,4-difluoropyrrolidin-3-yl)-8-fluoro-2-(4-methoxybenzyl)isoquinolin-1(2H)-one (7)

To a solution of 3-(2,2-difluorovinyl)-8-fluoro-2-(4-methoxybenzyl)isoquinolin-1(2H)-one **5** (260 mg, 0.75 mmol) and LiF (39 mg, 1.51 mmol) in ACN (50 mL) was added N-benzyl-1-methoxy-N-((trimethylsilyl)methyl)methanamine **6** (357 mg, 1.51 mmol). The mixture was kept stirring at 60 °C for 16 h. The resulting mixture was diluted with water and extracted with DCM (30 mL × 3). The combined organic layer was dried over Na₂SO₄ and concentrated under reduced pressure. The residue was purified by flash chromatography (eluting with EtOAc/PE, 0 to 15%) to give 3-(1-benzyl-4,4-difluoropyrrolidin-3-yl)-8-fluoro-2-(4-methoxybenzyl)isoquinolin-1(2H)-one 7 (25 mg, 90% purity, 6% yield) as a white solid.

LCMS (ESI) calcd for C₂₈H₂₅F₃N₂O₂ [M + H] ⁺ m/z 479.19, found 479.20.

### Preparation of 3-(4,4-difluoropyrrolidin-3-yl)-8-fluoro-2-(4-methoxybenzyl)isoquinolin-1(2H)-one (7)

To a solution of 3-(1-benzyl-4,4-difluoropyrrolidin-3-yl)-8-fluoro-2-(4-methoxybenzyl)isoquinolin-1(2H)-one 7 (130 mg, 0.271 mmol) in MeOH (5 mL) was added one drop of conc. HCl, 10% Pd/C (20 mg) and 10% Pd(OH)₂/C (20 mg). The mixture was evacuated and backfilled with hydrogen three times and then charged with hydrogen. The resulting mixture was stirred at room temperature for 16 hours. Then the mixture was filtered through celite and concentrated under vacuum to give crude 3-(4,4-difluoropyrrolidin-3-yl)-8-fluoro-2-(4-methoxybenzyl)isoquinolin-1(2H)-one **8** (100 mg, 50% purity, 47% yield) as a white solid which was used directly in next step without further purification.

LCMS (ESI) calcd for C₂₁H₁₉F₃N₂O₂ [M + H] ⁺ m/z 389.14, found 389.00.

### Preparation of 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide (10)

To a solution of 3-(4,4-difluoropyrrolidin-3-yl)-8-fluoro-2-(4-methoxybenzyl)isoquinolin-1(2H)-one **8** (100 mg, 0.257 mmol) in MeOH (10 mL) was added N-methyl-5-(4-oxopiperidin-1-yl)picolinamide **INT** (90 mg, 0.386 mmol), then two drops of acetic acid and NaBH₃CN (24 mg, 0.386 mmol) were added at room temperature. The reaction mixture stirred at 50 °C for 1 h. After cooling to room temperature, the reaction mixture was concentrated under reduced pressure. The residue was purified by flash chromatography (eluting with DCM/MeOH = 100 : 0 to 90 : 10) to give 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide **10** (80 mg, 50% purity, 25% yield) as a white solid.

LCMS (ESI) calcd for C₃₃H₃₄F₃N₅O₃ [M + H] ⁺ m/z 606.26, found 606.20.

### Preparation of 5-(4-(3,3-difluoro-4-(8-fluoro-1-oxo-1,2-dihydroisoquinolin-3-yl)pyrrolidin-1-yl)piperidin-1-yl)-N-methylpicolinamide (compound 393rac)

To a solution of 5-(4-(3,3-difluoro-4-(8-fluoro-2-(4-methoxybenzyl)-1-oxo-1,2-dihydroisoquinolin-3-yl)pyrrolidin-1-yl)piperidin-1-yl)-N-methylpicolinamide **10** (80 mg, 0.13 mmol) in TFA (5 mL) was added TfOH (1 mL) dropwise at rt. The reaction mixture was stirred at 100 °C for 5 min. The pH of the resulting mixture was adjusted to around 8.0 by progressively adding saturated NaHCO₃ solution, and then extracted with DCM (50 mL × 3). The combined organic layers were dried over Na₂SO₄ and concentrated under reduced pressure. The residue was purified by prep-HPLC (Gemini 5 um C18 150 × 21.2 mm, eluting with 35% to 40% ACN/H₂O containing 0.1% FA) to give 5-(4-(3,3-difluoro-4-(8-fluoro-1-oxo-1,2-dihydroisoquinolin-3-yl)pyrrolidin-1-yl)piperidin-1-yl)-N-methylpicolinamide 393rac (6.2 mg, 93% purity, 9% yield) as a brown solid.

¹H NMR (400 MHz, CD₃OD_SPE, ppm) δ: 8.31 (d, *J* = 2.8 Hz, 1 H), 7.95 (d, *J* = 8.8 Hz, 1 H), 7.72-7.65 (m, 1 H), 7.57-7.47 (m, 1 H), 7.43 (d, *J* = 8.0 Hz, 1 H), 7.17 (dd, *J* = 11.8, 8.2 Hz, 1 H), 6.76-6.68 (m, 1 H), 4.10-3.94 (m, 2 H), 3.85-3.50 (m, 5 H), 3.28-3.25 (m, 1 H), 3.07 (t, *J* = 12.4 Hz, 2 H), 2.96-2.90 (m, 3 H), 2.24-2.09 (m, 2 H), 1.84-1.66 (m, 2 H).

LCMS (ESI) calcd for C₂₅H₂₆F₃N₅O₂ [M + H] ⁺ m/z 486.21, found 486.40.

### Assays

Exemplary compounds of the disclosure were prepared and tested to determine their effect as PARP1 and PARP2 inhibitors. Typical assays are described below.

### PARP1 biochemical dissociation-enhanced lanthanide fluorescence immunoassay (DELFIA assay)

Optiplate HB 384-well plates were coated with anti-FLAG antibody, supplied as a 4 mg/ml solution, using a Na₂CO₃/HCO₃ coating buffer at pH 9.6, overnight at 4°C, in order to achieve a final immobilisation per well of 0.3 µg. Wells were then washed 3 x 5 min in coating wash buffer (PBS/0.05 % Tween (v/v)), and blocked with 2 % BSA (w/v) in coating wash buffer overnight at 4°C. Prior to assay, wells were washed 3 x 5 min in coating wash buffer. For the assay 20 µl of 2.5 nM recombinant full length human N-terminally FLAG-tagged PARP1 was added to each well of the 384-well plate for 30 min at room temperature followed by addition of 50 nL of compound solution in DMSO using pintool technology. Following incubation for 30 min at room temperature, 5 µl of 10 µM biotin-NAD⁺ and 10 nM activation DNA (sequence shown below) in solution in 20 mM HEPES (pH 7.5), 100 mM NaCl, 2 mM DTT, 0.1 % BSA (w/v), 0.02 % Tween (v/v) assay buffer. Auto-PARylation proceeded for 2 h at room temperature prior to the addition of 5 µl of 12 mM NAD⁺ quenching solution. After 30 min at room temperature, assay solution was removed and following washing 5 times for 3 min, 100 µl of a 1:1000 dilution of DELFIA Eu-N1 Streptavidin reagent was added. Plates were then incubated for 30 min at room temperature. Reaction mixture was removed and plates washed 5 times for 3 min prior to the addition of 25 µl DELFIA enhancement solution. Following incubation for 30 min at room temperature, fluorescence was measured on a Pherastar FS (Ex337 nm, Em620 nm; integration start 60 µs; integration time 400 µs).

Typically compounds were tested from 20 µM at 3-fold dilution intervals in 12-point concentration-response curves to determine IC₅₀ values. Data was analysed using ActivityBase software and replicate values for the low (without enzyme, 0.2% DMSO) and high (0.2 % DMSO) % controls were averaged and the data obtained from the test compounds expressed as a % of 100 % using the below formulae:$% value = 100 - \left(100*\left(\left(high control - unknown\right)/\left(high control-low control\right)\right)\right.$
% data was fitted to a non-linear regression equation (log inhibitor vs response-variable slope 4-parameters) to obtain IC₅₀ values.

The IC₅₀ values for a variety of test compounds are shown in Table 1.

### Activation DNA sequence:

### Duplex Sequences

**5'-ACCCTGCTGTGGGC/ideoxyU/GGAGAACAAGGTGAT-3'**
**5'-ATCACCTTGTTCTCCAHGCCCACAGCAGGGT-3'**

5'- ACCCTGCTGTGGCGGAGAACAAGGTGAT -3'
      |      || |    ||||||||||||||||
3'- TGGGACGACACCCGHACCTCTTGTTCCACTA -5'

### PARP1 probe displacement homogeneous time-resolved fluorescence assay (HTRF assay)

10 nM full length N-terminally FLAG-tagged PARP1 was incubated with 2 nM Anti-FLAG Tb-cryptate antibody and PARP1/2 Cy5 fluorescent dye-labelled binding probe (10-fold probe K_{d} = 270 nM) in 20 mM HEPES (pH 7.5), 100 mM NaCl, 2 mM DTT, 0.1 % BSA (w/v), 0.02 % Tween (v/v) assay buffer for 40 min at room temperature. A Cy5-labelled binding probe is shown below and described in Papeo, G. et al. J. Biomol. Screen. 2014; 19:1212-1219. 6 µl of this reaction mixture was then transferred to each well of a black non-binding surface 384-well plate and 35 nl of compound solution in DMSO was then added using pintool technology. Following incubation for 1 h at room temperature, fluorescence was measured on a Pherastar FS (Ex 337 nm, Em620 nm, em665 nm; integration start 60µs; integration time 400µs) using the HTRF module.

Typically compounds were tested from 58.5 µM at factor 3 dilution intervals in 12-point concentration-response curves to determine IC₅₀ values. Data was analysed using ActivityBase software and replicate values for the low (without enzyme but with probe and Tb-cryptate antibody, 0.6% DMSO) and high (0.6 % DMSO) % controls were averaged and the data obtained from the test compounds expressed as a % of 100 % using the below formulae: $% activity = 100 * \left(value-low control\right) / \left(high control-low control\right)$ %activity data was fitted to a non-linear regression equation to obtain IC50 values
K_{d} values were calculated using Cheng-Prussoff formula: ${IC}_{50} = \left(1+\left(\left[probe concentration\right]/\left[{Km}_{probe}\right]\right)\right) * {K}_{d}$

Therefore K_{d} = IC₅₀ / (1+[[probe concentration]/[Km_{probe}])); using probe at 10 x Kₘ, this equated to K_{d} = IC₅₀/11

### PARP2 probe displacement homogeneous time-resolved fluorescence assay (HTRF assay)

This assay was performed under identical conditions as for PARP1, except that N-terminally FLAG-tagged PARP2 (amino acids 1-583) was used instead of PARP1, and PARP1/2 binding probe was used at 10-fold probe K_{d} = 540 nM. Data analysis was performed identical as for PARP1.

### Cy5 probe structure:

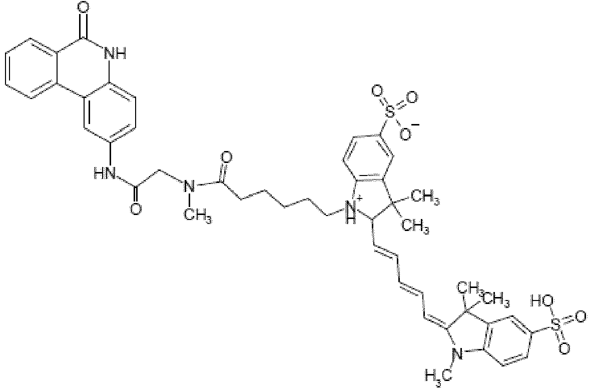

### NanoBRET cellular target occupancy assay

NanoBRET assays were employed to demonstrate cellular target engagement and selectivity at PARP1 and PARP2. These assays are based on bioluminescence resonance energy transfer (BRET) between a Nano-luc-tagged protein (eg PARP1 or PARP2) and a fluorescent group on a high affinity NAD⁺ competitive binding probe. Such cellular probe displacement assays can be utilised to measure inhibitor affinities and selectivity ratios at PARP1 and 2.

Frozen HEK293 cells transiently transfected with either PARP1-NanoLuc^{®} fusion or PARP2-NanoLuc^{®} fusion constructs (Promega) were thawed and dispensed as a suspension in 384-well microplates each at a density of 1750 cells per well. NanoBRET^{™} TE PARP Tracer-01 was then added to final concentrations of 11 and 2 nM for PARP1 and PARP2 assays, respectively. Compounds were added from 25 µM at factor 3 dilution intervals in 12-point concentration-response curves and plates were incubated for 2 hours at 37°C. BRET ratios were then measured using a NanoBRET module (LUM 610-LP 450-80) and PHERAstar FS or FSX reader following addition of NanoBRET^{™} Nano-Glo^{®} Substrate and Extracellular NanoLuc^{®} Inhibitor according to manufacturer's instructions. Kd values were calculated using Cheng-Prussoff formula:$IC 50 = \left(1+\left(\left[tracer concentration\right]/\left[Kmtracer\right]\right)\right) * Kd$

Binned potency, affinity and selectivity data for a variety of test compounds are shown in Table 1 where DELFIA and Probe Displacement HTRF assays were used. Binned potency, affinity and selectivity data for a subset of test compounds where the NanoBRET assay was used are shown in Table 2.

**TABLE 1**

| *Results of Parp 1*/*2 assays for selected compounds (DELFIA and Probe Displacement HTRF)* | | | | |
|---|---|---|---|---|
| **Compound** | **PARP1 DELFIA** | **PARP1 HTRF** | **PARP2 HTRF** | **Selectivity** |
| 1 | ++ | NT | NT | NT |
| 2* | ++++ | ++++ | + | +++ |
| 3a* | ++ | NT | NT | NT |
| 3b* | ++ | NT | NT | NT |
| 4a* | ++ | ++ | ++ | - |
| 4b* | ++ | NT | NT | NT |
| 5 | + | NT | NT | NT |
| 6a | ++++ | ++++ | - | +++ |
| 6b | ++ | ++ | + | + |
| 7a* | +++ | ++++ | ++ | +++ |
| 7b* | ++ | NT | NT | NT |
| 8* | ++++ | ++++ | - | +++ |
| 9a* | +++ | +++ | + | +++ |
| 9b* | +++ | +++ | ++ | - |
| 10a | +++ | ++++ | ++ | +++ |
| 10b | ++++ | ++++ | ++ | +++ |
| 11a* | + | ++ | - | - |
| 11b* | ++ | ++ | + | - |
| 12* | ++ | NT | NT | NT |
| 13a* | + | NT | NT | NT |
| 13b* | + | NT | NT | NT |
| 14a* | + | NT | NT | NT |
| 14b* | +++ | +++ | - | +++ |
| 15 | ++ | NT | NT | NT |
| 16a* | ++ | NT | NT | NT |
| 16b* | ++ | NT | NT | NT |
| 17 | +++ | +++ | - | +++ |
| 18 | ++ | NT | NT | NT |
| 19* | + | NT | NT | NT |
| 20* | +++ | +++ | - | ++ |
| 21* | + | NT | NT | NT |
| 22* | + | NT | NT | NT |
| 23a | ++ | NT | NT | NT |
| 23b | + | + | - | - |
| 24a* | + | NT | NT | NT |
| 24b* | + | NT | NT | NT |
| 25* | ++ | NT | NT | NT |
| 26rac | +++ | +++ | ++ | + |
| 27 | +++ | +++ | + | + |
| 28 | ++ | NT | NT | NT |
| 29* | + | NT | NT | NT |
| 30* | ++ | NT | NT | NT |
| 31a* | + | NT | NT | NT |
| 31b* | +++ | +++ | - | +++ |
| 32a | +++ | +++ | + | + |
| 32b | ++ | NT | NT | NT |
| 33a* | - | NT | NT | NT |
| 33b* | +++ | +++ | - | ++ |
| 34a | +++ | +++ | ++ | - |
| 34b | ++++ | ++++ | + | +++ |
| 35a* | ++ | NT | NT | NT |
| 35b* | +++ | +++ | - | +++ |
| 36* | ++++ | ++++ | - | +++ |
| 37rac | ++ | NT | NT | NT |
| 38a* | + | + | + | - |
| 38b* | + | + | - | - |
| 39rac* | ++ | ++ | - | + |
| 40* | ++++ | ++++ | ++ | ++ |
| 41* | ++ | ++ | - | + |
| 42rac* | ++++ | ++++ | + | +++ |
| 43a | ++ | +++ | ++ | - |
| 43b | ++++ | ++++ | ++ | + |
| 44a | ++++ | ++++ | - | +++ |
| 44b | +++ | +++ | + | + |
| 45* | ++++ | ++++ | ++ | +++ |
| 46a | ++++ | +++ | + | +++ |
| 46b | ++ | ++ | ++ | - |
| 47* | ++ | ++ | - | - |
| 48* | ++++ | ++++ | ++ | +++ |
| 49rac | ++++ | ++++ | ++ | +++ |
| 50a* | +++ | +++ | + | + |
| 50b* | +++ | +++ | - | +++ |
| 51a* | ++ | ++ | - | + |
| 51b* | + | + | + | - |
| 52a | +++ | +++ | ++ | - |
| 52b | ++++ | ++++ | ++ | +++ |
| 53a* | ++++ | ++++ | + | +++ |
| 53b* | ++ | ++ | - | + |
| 54* | ++++ | ++++ | ++ | +++ |
| 55a | ++++ | ++++ | + | +++ |
| 55b | +++ | +++ | ++ | - |
| 10rac | ++++ | ++++ | ++ | +++ |
| 56a* | ++ | +++ | - | ++ |
| 56b* | + | ++ | - | - |
| 57a | ++++ | +++ | - | +++ |
| 57b | ++ | ++ | - | + |
| 58a | ++++ | ++++ | - | +++ |
| 58b | +++ | +++ | - | ++ |
| 59* | + | ++ | - | - |
| 60a | ++++ | ++++ | - | +++ |
| 60b | ++ | +++ | - | ++ |
| 61a | ++ | +++ | + | + |
| 61b | ++++ | ++++ | + | +++ |
| 62a* | +++ | +++ | + | ++ |
| 62b* | ++ | ++ | + | - |
| 63* | ++++ | +++ | + | ++ |
| 64a | ++++ | ++++ | ++ | + |
| 64b | ++++ | ++++ | ++ | +++ |
| 65a* | ++++ | ++++ | ++ | +++ |
| 65b* | ++ | ++ | + | + |
| 66a | + | + | - | - |
| 66b | + | + | - | - |
| 67a | ++ | ++ | + | + |
| 67b | ++ | ++ | + | + |
| 68a | ++++ | ++++ | ++ | +++ |
| 68b | +++ | ++++ | ++ | + |
| 69a | ++++ | +++ | ++ | ++ |
| 69b | +++ | +++ | ++ | - |
| 70a | +++ | ++ | + | + |
| 70b | +++ | ++ | ++ | - |
| 71a | + | + | - | - |
| 71b | +++ | +++ | - | +++ |
| 72a* | +++ | +++ | + | +++ |
| 72b* | ++++ | ++++ | ++ | +++ |
| 73a* | ++ | ++ | - | - |
| 73b* | +++ | +++ | - | ++ |
| 74a | ++ | ++ | + | + |
| 74b | +++ | +++ | + | ++ |
| 75* | NT | + | - | - |
| 76a | NT | +++ | ++ | - |
| 76b | NT | +++ | ++++ | - |
| 77a | NT | ++ | - | + |
| 77b | NT | +++ | - | +++ |
| 78a | NT | ++++ | + | +++ |
| 78b | NT | +++ | ++ | + |
| 79a | NT | +++ | ++ | + |
| 79b | NT | +++ | ++ | + |
| 80a | NT | ++++ | ++ | +++ |
| 80b | NT | ++ | ++ | - |
| 81a | NT | ++ | - | + |
| 81b | NT | +++ | - | +++ |
| 82a | NT | +++ | ++ | - |
| 82b | NT | +++ | - | ++ |
| 83a | NT | ++ | + | - |
| 83b | NT | ++ | - | + |
| 84a | NT | + | - | - |
| 84b | NT | +++ | - | ++ |
| 85a | NT | +++ | ++ | + |
| 85b | ++++ | ++++ | +++ | ++ |
| 86a | +++ | +++ | ++ | + |
| 86b | ++++ | ++++ | ++++ | + |
| 87a | NT | ++ | + | + |
| 87b | NT | ++++ | + | +++ |
| 88a | NT | ++ | ++ | - |
| 88b | NT | ++++ | + | +++ |
| 89a | NT | - | - | - |
| 89b | NT | ++ | - | + |
| 90a | NT | ++ | - | + |
| 90b | NT | ++ | + | + |
| 91a | ++++ | ++++ | ++ | +++ |
| 91b | ++++ | ++++ | ++ | +++ |
| 92a | ++ | ++ | - | + |
| 92b | +++ | ++++ | + | +++ |
| 93a | ++ | ++ | - | + |
| 93b | + | + | - | - |
| 94a | +++ | +++ | + | + |
| 94b | ++++ | ++++ | - | +++ |
| 95rac | ++ | + | + | - |
| 96a | +++ | +++ | + | + |
| 96b | ++ | ++ | ++ | - |
| 97a* | - | - | - | - |
| 97b* | ++ | ++ | - | - |
| 98a | +++ | +++ | - | +++ |
| 98b | ++ | ++ | - | + |
| 99a | + | + | - | - |
| 99b | +++ | ++ | ++ | - |
| 100a | ++++ | ++++ | ++ | +++ |
| 100b | +++ | +++ | + | +++ |
| 101a | +++ | +++ | ++ | + |
| 101b | ++++ | ++++ | ++ | +++ |
| 102rac | ++++ | ++++ | + | +++ |
| 103rac | ++++ | ++++ | + | +++ |
| 104rac | ++++ | ++++ | + | +++ |
| 105rac | ++ | ++ | + | - |
| 106a | ++ | ++ | - | + |
| 106b | + | - | - | - |
| 107a | +++ | +++ | + | + |
| 107b | ++ | ++ | ++ | - |
| 108a | ++ | ++ | - | - |
| 108b | ++ | ++ | - | + |
| 109a | +++ | ++++ | - | +++ |
| 109b | +++ | ++ | + | + |
| 110a | ++++ | ++++ | - | +++ |
| 110b | ++ | +++ | ++ | - |
| 111a | +++ | +++ | ++ | + |
| 111b | ++++ | ++++ | ++ | +++ |
| 112a | ++ | ++ | + | - |
| 112b | +++ | +++ | - | +++ |
| 113a | ++ | ++ | ++ | - |
| 113b | ++ | ++ | - | + |
| 114a | ++++ | ++++ | - | +++ |
| 114b | ++ | ++ | + | + |
| 115a | ++ | ++ | + | + |
| 115b | +++ | +++ | + | +++ |
| 116a | +++ | +++ | - | +++ |
| 116b | ++ | ++ | + | - |
| 117a | +++ | +++ | ++ | + |
| 117b | ++++ | ++++ | ++ | +++ |
| 118a | ++++ | ++++ | - | +++ |
| 118b | +++ | +++ | - | ++ |
| 119rac* | ++++ | ++++ | + | +++ |
| 120rac* | ++ | ++ | - | + |
| 121a | +++ | +++ | ++ | + |
| 121b | ++++ | ++++ | ++ | +++ |
| 122a | ++++ | +++ | - | +++ |
| 122b | +++ | ++ | + | - |
| 123a | +++ | +++ | ++ | + |
| 123b | ++++ | ++++ | + | +++ |
| 124rac | ++++ | ++++ | ++ | +++ |
| 125a | ++++ | ++++ | + | +++ |
| 125b | ++++ | ++++ | + | +++ |
| 126a* | ++++ | ++++ | + | +++ |
| 126b* | ++ | ++ | - | + |
| 127rac* | +++ | +++ | - | +++ |
| 128a | +++ | +++ | + | +++ |
| 128b | ++++ | ++++ | +++ | ++ |
| 129a | ++++ | ++++ | + | +++ |
| 129b | ++++ | ++++ | +++ | +++ |
| 130a | ++++ | ++++ | ++ | + |
| 130b | ++++ | ++++ | ++ | +++ |
| 131a | +++ | ++ | + | + |
| 131b | ++++ | ++++ | - | +++ |
| 132rac* | +++ | +++ | - | +++ |
| 133a* | ++++ | ++++ | + | +++ |
| 133b* | +++ | +++ | - | +++ |
| 134rac* | ++++ | ++++ | - | +++ |
| 135rac | ++++ | ++++ | +++ | + |
| 136rac | ++++ | ++++ | +++ | +++ |
| 137a | ++++ | ++++ | ++ | +++ |
| 137b | ++++ | ++++ | +++ | + |
| 138rac | +++ | +++ | + | ++ |
| 139a | ++++ | ++++ | + | +++ |
| 139b | +++ | +++ | + | +++ |
| 140a | ++ | +++ | - | ++ |
| 140b | +++ | +++ | + | +++ |
| 141rac* | ++++ | ++++ | + | +++ |
| 142rac* | ++++ | ++++ | + | +++ |
| 143rac | +++ | ++++ | + | +++ |
| 144rac* | ++++ | ++++ | + | +++ |
| 136a | ++++ | ++++ | ++ | +++ |
| 136b | ++++ | ++++ | +++ | +++ |
| 145a | ++++ | ++++ | + | +++ |
| 145b | ++++ | ++++ | + | +++ |
| 146a | ++ | ++ | + | - |
| 146b | +++ | +++ | - | +++ |
| 147a* | + | + | - | - |
| 147b* | + | + | - | - |
| 148a* | + | - | - | - |
| 148b* | + | + | - | - |
| 149a | ++ | ++ | - | + |
| 149b | + | + | - | - |
| 150rac | ++++ | ++++ | ++++ | - |
| 151a* | ++++ | ++++ | ++ | +++ |
| 151b* | ++++ | ++++ | - | +++ |
| 152a* | ++++ | ++++ | + | +++ |
| 152b* | +++ | +++ | - | ++ |
| 153rac* | ++++ | ++++ | ++ | ++ |
| 154rac | ++++ | ++++ | +++ | + |
| 155a | +++ | +++ | ++ | + |
| 155b | ++++ | ++++ | ++ | +++ |
| 156rac | +++ | +++ | - | ++ |
| 157rac | ++++ | ++++ | + | +++ |
| 158a | ++++ | ++++ | + | +++ |
| 158b | +++ | +++ | + | +++ |
| 159a | - | - | - | - |
| 159b | ++ | ++ | - | + |
| 160rac* | ++++ | ++++ | + | +++ |
| 161a | ++ | ++ | - | - |
| 161b | ++++ | ++++ | + | +++ |
| 162a | +++ | +++ | ++ | - |
| 162b | ++++ | ++++ | +++ | ++ |
| 163rac | ++++ | ++++ | ++ | +++ |
| 164a* | ++++ | ++++ | + | +++ |
| 164b* | +++ | +++ | - | +++ |
| 165a* | ++++ | ++++ | ++ | +++ |
| 165b* | +++ | +++ | + | +++ |
| 154a | ++++ | ++++ | ++ | +++ |
| 154b | ++++ | ++++ | +++ | + |
| 166rac* | ++++ | ++++ | + | +++ |
| 167a | +++ | +++ | - | ++ |
| 167b | ++++ | ++++ | ++ | +++ |
| 168 | ++++ | ++++ | - | +++ |
| 169a* | + | - | - | - |
| 169b* | ++++ | +++ | - | +++ |
| 170rac | ++++ | ++++ | ++++ | - |
| 171rac* | ++++ | ++++ | ++ | +++ |
| 172a | ++++ | ++++ | + | +++ |
| 172b | ++++ | ++++ | +++ | +++ |
| 173 | ++ | + | - | - |
| 174rac | ++++ | ++++ | ++ | +++ |
| 174a | ++++ | ++++ | ++ | +++ |
| 174b | ++++ | ++++ | +++ | +++ |
| 175rac | ++++ | ++++ | ++ | +++ |
| 176rac | NT | ++ | - | + |
| 177rac | NT | ++++ | - | +++ |
| 178a | NT | ++ | + | - |
| 178b | NT | ++++ | + | +++ |
| 179 | NT | +++ | - | +++ |
| 180a | ++++ | ++++ | + | +++ |
| 180b | ++++ | ++++ | +++ | ++ |
| 181a | +++ | +++ | ++ | + |
| 181b | ++++ | ++++ | ++ | +++ |
| 182a | +++ | +++ | ++ | + |
| 182b | ++++ | ++++ | ++ | +++ |
| 183a | ++++ | ++++ | + | +++ |
| 183b | ++++ | ++++ | ++ | +++ |
| 184a | ++++ | ++++ | + | +++ |
| 184b | ++++ | ++++ | ++ | +++ |
| 176a | ++ | ++ | - | + |
| 176b | +++ | ++ | - | ++ |
| 170a | ++++ | ++++ | ++++ | - |
| 170b | ++++ | ++++ | ++++ | - |
| 185a | ++++ | ++++ | ++ | +++ |
| 185b | +++ | ++ | + | + |
| 186a | ++++ | ++++ | ++ | +++ |
| 186b | ++++ | ++++ | +++ | + |
| 187a | +++ | +++ | ++ | - |
| 187b | ++++ | ++++ | ++++ | - |
| 188a | ++ | ++ | - | + |
| 188b | ++++ | ++++ | ++ | +++ |
| 189a | +++ | +++ | ++ | - |
| 189b | ++++ | ++++ | +++ | ++ |
| 190a | ++ | ++ | + | + |
| 190b | ++++ | ++++ | +++ | ++ |
| 191rac | ++ | ++ | - | + |
| 192 | ++++ | ++++ | + | +++ |
| 193 | +++ | +++ | + | +++ |
| 194a | +++ | +++ | + | + |
| 194b | ++++ | ++++ | +++ | + |
| 195rac | ++++ | ++++ | ++ | +++ |
| 196a | ++++ | ++++ | ++ | +++ |
| 196b | ++++ | ++++ | + | +++ |
| 197a | ++++ | ++++ | ++ | +++ |
| 197b | +++ | +++ | + | +++ |
| 198rac | ++++ | ++++ | +++ | + |
| 199a | ++++ | ++++ | ++ | +++ |
| 199b | ++ | ++ | - | + |
| 200rac | +++ | +++ | - | +++ |
| 201a* | ++ | ++ | - | + |
| 201b* | ++++ | ++++ | + | +++ |
| 202a | +++ | +++ | ++ | + |
| 202b | ++++ | ++++ | +++ | + |
| 203rac | ++++ | ++++ | - | +++ |
| 204rac | ++++ | ++++ | - | +++ |
| 205a | ++ | ++ | - | + |
| 205b | +++ | +++ | + | + |
| 206a | ++++ | ++++ | ++ | +++ |
| 206b | ++++ | ++++ | + | +++ |
| 207a* | ++++ | ++++ | - | +++ |
| 207b* | ++ | ++ | - | - |
| 208a | ++++ | +++ | ++ | + |
| 208b | ++++ | ++++ | +++ | ++ |
| 209a | ++++ | ++++ | ++ | +++ |
| 209b | +++ | +++ | + | +++ |
| 210a* | ++ | ++ | - | + |
| 210b* | ++++ | ++++ | + | +++ |
| 211rac | ++++ | ++++ | ++ | +++ |
| 212a* | ++ | ++ | - | + |
| 212b* | - | - | - | - |
| 213a* | +++ | +++ | - | +++ |
| 213b* | ++++ | ++++ | ++ | +++ |
| 214a | +++ | +++ | ++ | ++ |
| 214b | ++++ | ++++ | ++ | +++ |
| 215rac | +++ | +++ | ++ | - |
| 216rac* | ++ | ++ | - | - |
| 217a* | ++ | ++ | - | - |
| 217b* | ++++ | ++++ | ++ | ++ |
| 218a | +++ | +++ | - | +++ |
| 218b | ++++ | ++++ | ++ | +++ |
| 219a | ++++ | ++++ | +++ | ++ |
| 219b | ++++ | ++++ | ++ | +++ |
| 220a | ++ | ++ | + | - |
| 220b | +++ | +++ | + | + |
| 221a | +++ | +++ | ++ | + |
| 221b | ++++ | ++++ | +++ | ++ |
| 222a | ++++ | ++++ | ++ | + |
| 222b | ++++ | ++++ | +++ | +++ |
| 223a | ++++ | ++++ | ++ | +++ |
| 223b | ++++ | ++++ | ++ | +++ |
| 224a | ++ | + | + | - |
| 224b | ++ | ++ | + | - |
| 225rac* | ++++ | ++++ | + | +++ |
| 226rac | +++ | +++ | - | ++ |
| 227 | ++++ | ++++ | ++ | +++ |
| 228 | ++++ | ++++ | + | +++ |
| 229rac | +++ | ++++ | ++ | +++ |
| 230rac | ++++ | ++++ | + | +++ |
| 231rac* | ++++ | ++++ | + | +++ |
| 232a | +++ | +++ | ++ | - |
| 232b | ++++ | ++++ | ++ | +++ |
| 233rac | ++++ | ++++ | ++ | +++ |
| 234rac | ++++ | ++++ | ++ | +++ |
| 235rac | ++++ | ++++ | +++ | + |
| 236a | +++ | +++ | ++ | + |
| 236b | ++++ | ++++ | +++ | + |
| 237rac | ++++ | ++++ | +++ | ++ |
| 238 | ++++ | ++++ | ++ | +++ |
| 239a* | ++++ | ++++ | - | +++ |
| 239b* | ++++ | ++++ | + | +++ |
| 240a* | +++ | +++ | - | ++ |
| 240b* | + | + | - | - |
| 241rac | ++++ | ++++ | ++ | +++ |
| 242rac* | ++++ | ++++ | ++ | ++ |
| 243rac* | +++ | +++ | - | ++ |
| 244rac | ++++ | ++++ | ++++ | - |
| 245a | ++++ | ++++ | ++ | +++ |
| 245b | +++ | +++ | - | +++ |
| 246a* | ++ | ++ | - | + |
| 246b* | ++++ | ++++ | + | +++ |
| 247rac* | ++++ | ++++ | +++ | + |
| 248rac | ++++ | ++++ | ++ | +++ |
| 249a | + | + | - | - |
| 249b | ++ | ++ | - | + |
| 250a | ++ | ++ | + | - |
| 250b | +++ | +++ | ++ | + |
| 251a | ++ | ++ | + | - |
| 251b | +++ | +++ | ++ | + |
| 252a | ++ | ++ | + | - |
| 252b | +++ | +++ | ++ | + |
| 253rac | +++ | +++ | - | + |
| 254rac | +++ | ++ | + | + |
| 255rac* | ++++ | ++++ | ++ | ++ |
| 256rac* | ++++ | ++++ | + | +++ |
| 257rac* | ++++ | ++++ | + | +++ |
| 258rac | ++++ | ++++ | ++ | +++ |
| 259 | ++++ | ++++ | ++ | +++ |
| 260 | ++++ | ++++ | + | +++ |
| 261a* | ++ | + | - | - |
| 261b* | ++++ | ++++ | + | +++ |
| 262a | ++ | + | + | - |
| 262b | ++ | ++ | + | + |
| 263rac | +++ | ++ | ++ | - |
| 264rac* | ++++ | ++++ | ++ | +++ |
| 265a | ++++ | ++++ | + | +++ |
| 265b | ++++ | ++++ | ++ | +++ |
| 266a | ++++ | ++++ | ++ | +++ |
| 266b | +++ | +++ | - | +++ |
| 267a | +++ | +++ | ++ | + |
| 267b | ++++ | ++++ | +++ | +++ |
| 268rac | ++++ | ++++ | ++ | +++ |
| 269a* | ++++ | ++++ | + | +++ |
| 269b* | ++ | ++ | - | + |
| 270a | +++ | +++ | +++ | - |
| 270b | ++++ | ++++ | +++ | + |
| 271rac | ++++ | ++++ | ++ | +++ |
| 272a | - | - | - | - |
| 272b | ++ | ++ | - | + |
| 273a | ++ | ++ | + | - |
| 274a | ++ | ++ | + | - |
| 274b | +++ | ++ | + | + |
| 275rac | ++++ | ++++ | ++ | + |
| 276rac* | ++++ | ++++ | ++ | +++ |
| 277a | ++++ | ++++ | + | +++ |
| 277b | +++ | +++ | - | ++ |
| 278rac* | +++ | +++ | - | +++ |
| 279a* | ++ | ++ | - | + |
| 279b* | + | + | - | - |
| 280a | +++ | +++ | + | ++ |
| 280b | ++++ | ++++ | +++ | ++ |
| 281a | ++++ | ++++ | +++ | + |
| 281b | ++ | ++ | ++ | - |
| 282rac* | +++ | +++ | ++ | + |
| 283a | ++ | ++ | - | + |
| 283b | ++++ | ++++ | + | +++ |
| 284 | ++++ | ++++ | - | +++ |
| 285a* | ++++ | ++++ | ++ | +++ |
| 285b* | ++++ | ++++ | - | +++ |
| 286rac* | +++ | +++ | - | +++ |
| 287a* | +++ | +++ | - | ++ |
| 287b* | + | + | - | - |
| 288rac | +++ | +++ | + | +++ |
| 273b | +++ | +++ | ++ | + |
| 289a | ++ | ++ | + | - |
| 289b | ++++ | +++ | ++ | + |
| 290rac | +++ | ++ | + | + |
| 291a | +++ | ++ | + | + |
| 291b | ++ | ++ | + | - |
| 292a | +++ | ++ | + | - |
| 292b | ++++ | ++++ | ++ | +++ |
| 293a | - | - | - | - |
| 293b | ++ | ++ | + | - |
| 294rac | ++ | ++ | - | - |
| 295 | ++++ | ++++ | - | +++ |
| 296rac | ++++ | ++++ | ++ | + |
| 297rac* | +++ | +++ | ++ | - |
| 298a* | + | + | - | - |
| 298b* | +++ | +++ | + | +++ |
| 299* | ++++ | ++++ | - | +++ |
| 300* | ++++ | ++++ | + | +++ |
| 301rac | ++ | ++ | + | - |
| 302 | ++++ | ++++ | - | +++ |
| 303 | ++++ | ++++ | - | +++ |
| 304 | ++++ | +++ | - | +++ |
| 305 | ++++ | ++++ | - | +++ |
| 306a | ++ | ++ | + | + |
| 306b | ++++ | ++++ | ++ | +++ |
| 307rac | +++ | +++ | ++ | - |
| 308a | ++ | ++ | + | + |
| 308b | ++++ | ++++ | ++ | +++ |
| 309a | ++++ | ++++ | - | +++ |
| 309b | +++ | +++ | - | ++ |
| 310* | +++ | ++++ | - | +++ |
| 311 | ++++ | ++++ | + | +++ |
| 312 | ++++ | ++++ | ++ | +++ |
| 313a* | +++ | +++ | - | +++ |
| 313b* | ++++ | ++++ | + | +++ |
| 314a | ++++ | ++++ | ++ | + |
| 314b | ++++ | ++++ | ++ | +++ |
| 315rac | ++++ | ++++ | ++ | +++ |
| 316a | ++++ | ++++ | ++ | +++ |
| 316b | +++ | +++ | ++ | + |
| 317a* | +++ | ++ | + | - |
| 317b* | ++++ | ++++ | ++ | +++ |
| 318rac | +++ | +++ | ++ | + |
| 319rac | + | + | + | - |
| 320rac | ++ | ++ | + | - |
| 321rac | ++ | ++ | ++ | - |
| 322a* | ++++ | ++++ | - | +++ |
| 322b* | ++++ | ++++ | ++ | +++ |
| 323a | ++++ | ++++ | - | +++ |
| 323b | ++++ | ++++ | +++ | +++ |
| 324rac | ++++ | ++++ | ++ | +++ |
| 325rac | ++++ | ++++ | + | +++ |
| 326a* | ++ | ++ | + | + |
| 326b* | +++ | ++++ | + | +++ |
| 327a | ++ | ++ | - | + |
| 327b | ++ | ++ | - | - |
| 328a | ++ | ++ | - | + |
| 328b | +++ | +++ | - | +++ |
| 329rac | ++++ | ++++ | ++ | +++ |
| 330 | ++++ | ++++ | +++ | + |
| 331a | ++++ | ++++ | ++ | +++ |
| 331b | +++ | +++ | + | +++ |
| 332a* | ++ | ++ | + | + |
| 332b* | ++++ | ++++ | + | +++ |
| 333b | +++ | +++ | + | +++ |
| 334rac | ++++ | +++ | + | ++ |
| 335rac | ++ | ++ | - | + |
| 336rac | ++++ | ++++ | +++ | ++ |
| 337a | ++++ | ++++ | ++ | +++ |
| 337b | ++++ | ++++ | ++ | +++ |
| 338a* | ++++ | ++++ | ++ | +++ |
| 338b* | +++ | +++ | + | ++ |
| 339a | ++++ | ++++ | +++ | + |
| 339b | ++++ | ++++ | ++ | +++ |
| 340a | ++++ | ++++ | ++ | +++ |
| 340b | +++ | +++ | ++ | + |
| 341a | ++++ | ++++ | ++ | +++ |
| 341b | +++ | +++ | ++ | + |
| 342a | ++ | ++ | ++ | - |
| 342b | ++++ | ++++ | +++ | + |
| 333a | ++++ | ++++ | ++ | +++ |
| 343a | ++++ | ++++ | ++ | +++ |
| 343b | ++++ | ++++ | + | +++ |
| 344a | ++++ | ++++ | - | +++ |
| 344b | ++++ | ++++ | + | +++ |
| 345a* | ++++ | ++++ | ++ | +++ |
| 345b* | +++ | +++ | + | + |
| 346a* | ++++ | ++++ | + | +++ |
| 346b* | ++ | ++ | - | + |
| 347a | ++++ | ++++ | ++ | +++ |
| 347b | +++ | +++ | + | +++ |
| 348a | +++ | +++ | ++ | - |
| 348b | ++ | ++ | ++ | - |
| 349a* | ++++ | ++++ | - | +++ |
| 349b* | ++++ | ++++ | ++ | +++ |
| 350a | ++++ | ++++ | ++ | +++ |
| 350b | ++++ | ++++ | +++ | ++ |
| 351a | ++++ | ++++ | + | +++ |
| 351b | ++++ | ++++ | ++ | +++ |
| 352a | ++++ | ++++ | +++ | +++ |
| 352b | ++++ | ++++ | ++ | ++ |
| 353a* | ++++ | ++++ | ++ | +++ |
| 353b* | +++ | +++ | + | +++ |
| 354a | ++++ | ++++ | +++ | + |
| 354b | ++++ | ++++ | ++ | +++ |
| 355a* | ++ | ++ | + | - |
| 355b* | ++++ | ++++ | ++ | +++ |
| 356a* | ++++ | ++++ | + | +++ |
| 356b* | ++ | ++ | + | + |
| 357a | ++++ | ++++ | ++ | +++ |
| 357b | +++ | ++++ | + | +++ |
| 358rac | ++++ | ++++ | ++ | +++ |
| 359a | +++ | +++ | + | ++ |
| 359b | +++ | ++ | - | + |
| 360rac* | ++++ | ++++ | ++ | +++ |
| 361 | ++++ | ++++ | ++ | +++ |
| 362rac* | ++++ | ++++ | + | +++ |
| 363a* | ++++ | ++++ | + | +++ |
| 363b* | ++++ | ++++ | + | +++ |
| 364a* | ++ | ++ | - | + |
| 364b* | ++++ | ++++ | ++ | ++ |
| 365rac* | ++++ | ++++ | ++ | +++ |
| 315a | ++++ | ++++ | ++ | +++ |
| 315b | ++++ | ++++ | ++ | +++ |
| 366a | +++ | +++ | - | +++ |
| 366b | ++++ | ++++ | ++ | ++ |
| 367a | ++++ | ++++ | ++ | +++ |
| 367b | ++++ | ++++ | + | +++ |
| 368 | ++++ | ++++ | - | +++ |
| 369 | ++++ | ++++ | + | +++ |
| 370a | ++++ | ++++ | ++ | +++ |
| 370b | ++++ | ++++ | +++ | ++ |
| 371a | ++++ | ++++ | +++ | +++ |
| 371b | +++ | +++ | + | ++ |
| 372a | ++++ | ++++ | ++ | + |
| 372b | ++++ | ++++ | +++ | +++ |
| 324a | ++++ | ++++ | - | +++ |
| 324b | ++++ | ++++ | ++ | +++ |
| 373 | ++++ | ++++ | + | +++ |
| 374a | +++ | +++ | ++ | + |
| 374b | ++++ | ++++ | +++ | ++ |
| 375 | ++++ | ++++ | ++ | + |
| 376 | ++++ | ++++ | ++ | +++ |
| 377a* | +++ | +++ | - | +++ |
| 377b* | ++++ | ++++ | + | +++ |
| 378a | ++++ | ++++ | ++ | +++ |
| 378b | ++++ | ++++ | + | +++ |
| 379 | ++++ | ++++ | ++ | +++ |
| 380 | ++++ | ++++ | - | +++ |
| 381 | ++++ | ++++ | + | +++ |
| 382a* | ++ | ++ | - | + |
| 382b* | ++++ | ++++ | ++ | +++ |
| 383 | ++++ | ++++ | ++ | + |
| 384 | ++++ | ++++ | ++ | + |
| 385a | ++++ | ++++ | + | +++ |
| 385b | ++++ | ++++ | +++ | +++ |
| 386a | ++++ | ++++ | ++ | +++ |
| 386b | ++++ | ++++ | + | +++ |
| 387 | ++++ | ++++ | ++ | +++ |
| 388 | ++++ | ++++ | ++ | +++ |
| 389 | ++++ | ++++ | ++ | +++ |
| 390a | ++++ | ++++ | ++ | +++ |
| 390b | ++++ | ++++ | +++ | ++ |
| 391rac* | ++++ | ++++ | + | +++ |
| 392rac | ++ | ++ | - | + |
| 393rac | ++++ | ++++ | + | +++ |
| 394 | ++++ | ++++ | +++ | + |
| 395a | ++++ | ++++ | ++ | + |
| 395b | ++++ | ++++ | +++ | ++ |
| 396a | ++++ | ++++ | ++ | +++ |
| 396b | ++++ | ++++ | ++ | +++ |
| 397a | ++++ | ++++ | ++ | +++ |
| 397b | ++++ | ++++ | +++ | +++ |
| 398a* | ++++ | ++++ | + | +++ |
| 398b* | ++++ | ++++ | ++ | +++ |
| 399a | ++++ | ++++ | ++ | +++ |
| 399b | ++++ | ++++ | +++ | + |
| 400rac | ++++ | ++++ | ++ | +++ |
| 401a | ++++ | ++++ | +++ | +++ |
| 401b | ++++ | ++++ | +++ | + |
| 402a | ++++ | ++++ | ++ | +++ |
| 402b | ++++ | ++++ | +++ | +++ |
| 403rac | ++++ | ++++ | +++ | +++ |
| 404a | +++ | +++ | + | +++ |
| 404b | ++++ | ++++ | + | +++ |
| 405rac | ++++ | ++++ | ++ | +++ |
| 415rac* | +++ | ++++ | - | +++ |
| 406rac* | ++++ | ++++ | - | +++ |
| 407a | +++ | ++++ | ++ | ++ |
| 407b | ++++ | ++++ | ++++ | + |
| 408a* | ++++ | ++++ | ++ | +++ |
| 408b* | +++ | +++ | + | + |
| 409a* | ++++ | ++++ | + | +++ |
| 409b* | ++++ | ++++ | ++ | +++ |
| 410a | ++++ | ++++ | ++ | +++ |
| 410b | ++++ | ++++ | ++ | +++ |
| 411 | ++++ | ++++ | ++ | +++ |
| 412rac | ++ | ++ | - | + |
| 413rac | ++++ | ++++ | ++ | +++ |
| 414rac | +++ | +++ | ++ | ++ |

| | | | | |
|---|---|---|---|---|
| *: a reference example, not in accordance with this present claims. | | | | |

**TABLE 2**

| *Results of Parp 1*/*2 assays for selected compounds (NanoBRET)* | | | |
|---|---|---|---|
| **Compound** | **PARP1 NanoBRET** | **PARP2 NanoBRET** | **Selectivity** |
| 10b | ++++ | + | +++ |
| 64b | ++++ | ++ | +++ |
| 68a | ++++ | + | +++ |
| 72b* | ++++ | + | +++ |
| 86b | ++++ | +++ | + |
| 129b | ++++ | ++ | +++ |
| 137b | ++++ | ++ | +++ |
| 144rac* | ++++ | - | +++ |
| 136b | ++++ | ++ | +++ |
| 150rac | +++ | ++ | + |
| 151a* | ++++ | - | +++ |
| 157rac | ++++ | - | +++ |
| 154b | ++++ | ++ | +++ |
| 170rac | ++++ | +++ | + |
| 174rac | ++++ | + | +++ |
| 174a | ++++ | + | +++ |
| 174b | ++++ | ++ | +++ |
| 175rac | ++++ | + | +++ |
| 180b | ++++ | ++ | +++ |
| 183b | ++++ | + | +++ |
| 184b | ++++ | ++ | +++ |
| 186b | ++++ | +++ | +++ |
| 192 | ++++ | + | +++ |
| 196a | ++++ | ++ | +++ |
| 197a | ++++ | ++ | +++ |
| 198rac | ++++ | ++ | + |
| 223b | ++++ | ++ | +++ |
| 228 | ++++ | - | +++ |
| 239b | ++++ | + | +++ |
| 255rac* | ++++ | + | +++ |
| 258rac | ++++ | ++ | +++ |
| 259 | ++++ | + | +++ |
| 260 | ++++ | - | +++ |
| 285a* | ++++ | ++ | +++ |
| 295 | +++ | - | ++ |
| 299* | +++ | - | +++ |
| 300* | ++++ | - | +++ |
| 302 | +++ | - | +++ |
| 312 | ++ | + | ++ |
| 313b* | ++++ | + | +++ |
| 314b | ++++ | ++ | +++ |
| 315rac | ++++ | ++ | +++ |
| 316a | ++++ | ++ | +++ |
| 322b* | ++++ | ++ | +++ |
| 323a | ++++ | - | +++ |
| 323b | ++++ | ++ | +++ |
| 324rac | ++++ | + | +++ |
| 329rac | ++++ | + | +++ |
| 330 | +++ | ++ | + |
| 337a | ++++ | + | +++ |
| 337b | ++++ | ++ | +++ |
| 339a | ++++ | +++ | + |
| 343a | ++++ | + | +++ |
| 344b | ++++ | - | +++ |
| 350b | ++++ | +++ | +++ |
| 352a | ++++ | ++ | +++ |
| 353a* | ++++ | ++ | +++ |
| 354a | ++++ | +++ | ++ |
| 354b | ++++ | ++ | +++ |
| 357a | ++++ | + | +++ |

| | | | |
|---|---|---|---|
| *: a reference example, not in accordance with the present claims. | | | |

### Key

DELFIA, Probe Displacement HTRF and NanoBRET assay categories:
- indicates IC₅₀ or K_{d} value above 10 µM
+ indicates IC₅₀ or K_{d} value above 1 µM up to 10 µM
++ indicates IC₅₀ or K_{d} value above 100 nM up to 1 µM
+++ indicates IC₅₀ or K_{d} value above 10 nM up to 100 nM
++++ indicates IC₅₀ or K_{d} value of 10 nM or less

Selectivity categories:
- indicates a value of less than 10
+ indicates a value of 10 to less than 50
++ indicates a value of 50 to less than 100
+++ indicate a value of at least 100

The selectivity values relate to the selectivity preference of PARP1 over PARP2. They are calculated from the ratio of K_{d} values for PARP1 and PARP2 inhibition as K_{d} (PARP2) / K_{d} (PARP1).

## Claims

1. A PARP1 inhibitor compound for use in medicine, which compound comprises any of the following structures: 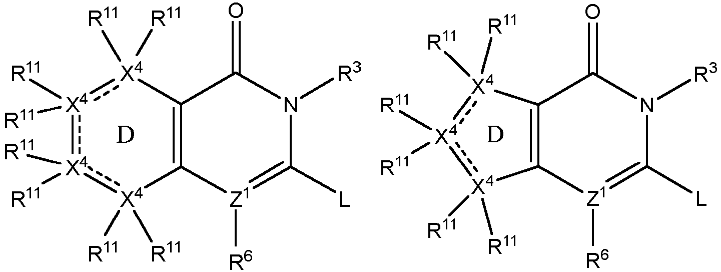 wherein:
the dotted lines indicate that the ring D may comprise single bonds, or a combination of both single and double bonds, and may be aliphatic or aromatic;
each X⁴ may be the same or different and is independently selected from C, N, O and S;
R³ is H;
when Z¹ is N, R⁶ is absent; when Z¹ is C, R⁶ is selected from H, halogen, C₁ to C₃ alkyl, C₁ to C₃ haloalkyl, C₁ to C₃ alcohol or C₁ to C₃ aminooalkyl;
each R¹¹ is independently selected from H, a halogen, a nitrile group, a linear or branched C₁-C₃ alkyl group, a linear or branched C₁-C₃ halogenated alkyl group, an - OH group, a linear or branched C₁-C₃ alcohol group, a halogenated linear or branched C₁-C₃ alcohol group, -NH₂, a linear or branched primary secondary or tertiary C₁-C₃ amine group, a halogenated linear or branched primary, secondary or tertiary C₁-C₃ amine group, a linear or branched C₁-C₃ alkoxy group, a linear or branched C₁-C₃ halogenated alkoxy group, and/or a pair of R¹¹ groups attached to the same atom forming =O; and/or when a pair of R¹¹ groups attached to different atoms together forms a ring with ring D atoms, the pair of R¹¹ groups comprises -CH₂CH₂CH₂-;
Z¹ is selected from C and N;
group L comprises a group having the following structure: 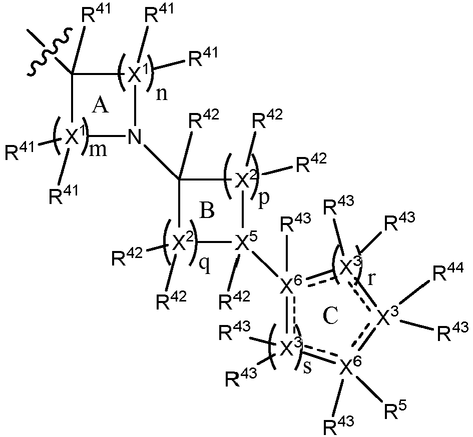
wherein:
n is a number independently selected from 0, 1, 2, 3, 4, 5 and 6;
m is a number independently selected from 0, 1, 2, 3, 4, 5 and 6;
m + n is a number selected from 2, 3, 4, 5, and 6;
p is a number independently selected from 0, 1, 2, 3, 4, 5 and 6;
q is a number independently selected from 0, 1, 2, 3, 4, 5 and 6;
p + q is a number selected from 2, 3, 4, 5, and 6;
r is a number independently selected from 0, 1, 2, 3, 4, and 5;
s is a number independently selected from 0, 1, 2, 3, 4, and 5;
r + s is a number selected from 1, 2, 3, 4, and 5;
each X¹ may be the same or different and is independently selected from C, N, O and S;
each X² may be the same or different and is independently selected from C, N, O and S;
each X³ may be the same or different and is independently selected from C, N, O and S;
X⁵ is independently selected from C and N;
each X⁶ may be the same or different and is independently selected from C and N;
each R⁴¹ R⁴², R⁴³ and R⁴⁴ may be the same or different, and may be present or absent, and is selected from H, deuterium, a halogen, a nitrile group, a substituted or unsubstituted C₁-C₆ alkyl group, a substituted or unsubstituted linear or branched C₁-C₆ halogenated alkyl group, a cyclopropyl group, an -OH group or a substituted or unsubstituted linear or branched C₁-C₆ alcohol group, a substituted or unsubstituted linear or branched C₁-C₇ amino carbonyl group, an -NH₂ group or a substituted or unsubstituted C₁-C₆ amino group and a substituted or unsubstituted C₁-C₆ alkoxy group; wherein, when a pair of R⁴¹ groups attached to different atoms together forms a ring with ring A atoms, and/or a pair of R⁴² groups attached to different atoms together forms a ring with ring B atoms, each of the pair of R⁴¹ groups and/or pair of R⁴² groups independently comprises -CH₂- or -CH₂CH₂-;
the dotted lines indicate that the ring C may comprise single bonds, or a combination of both single and double bonds, and may be aliphatic or aromatic; and
R⁵ is selected from H and a substituted or unsubstituted organic group.

2. A compound for use according to claim 1, wherein ring A of the group L comprises any of the following structures:

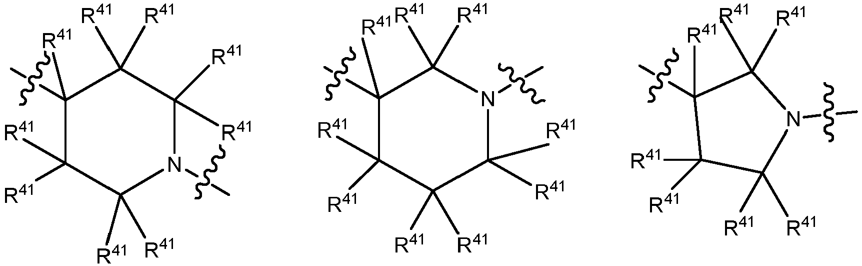

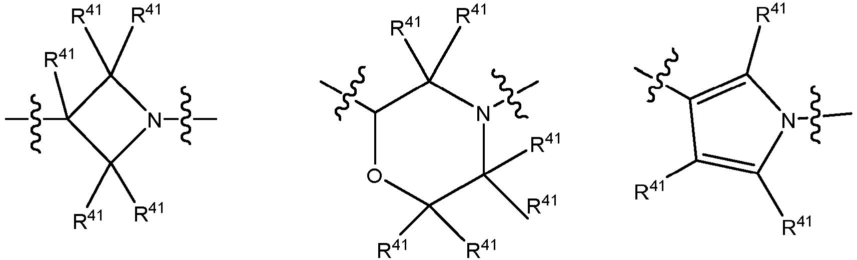

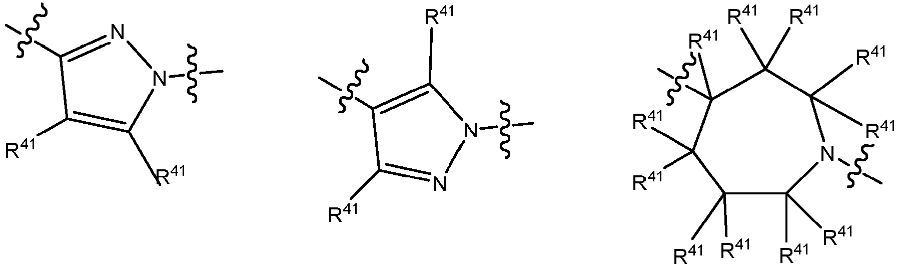

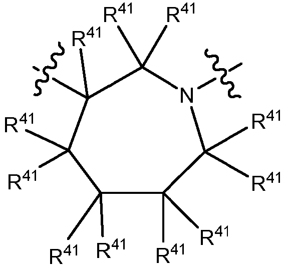

3. A compound for use according to claim 2, wherein ring A of the group L comprises any of the following structures:

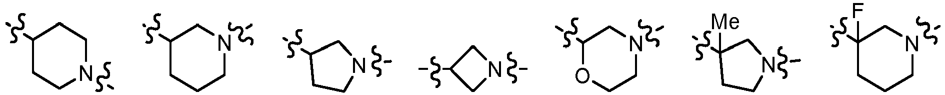

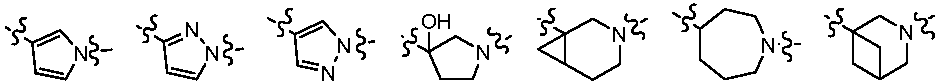

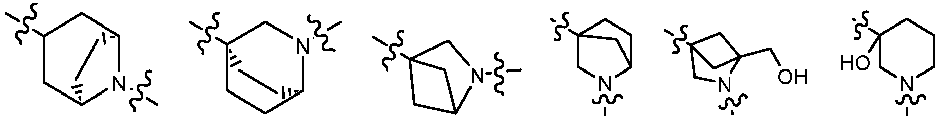

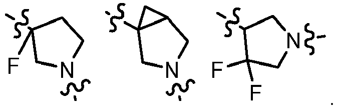

4. A compound for use according to any preceding claim, wherein ring C of the group L comprises any of the following structures:

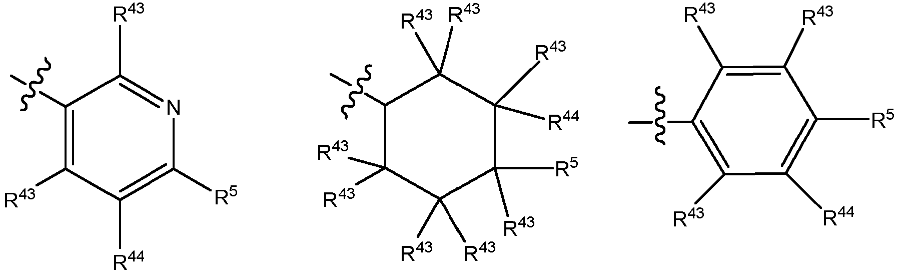

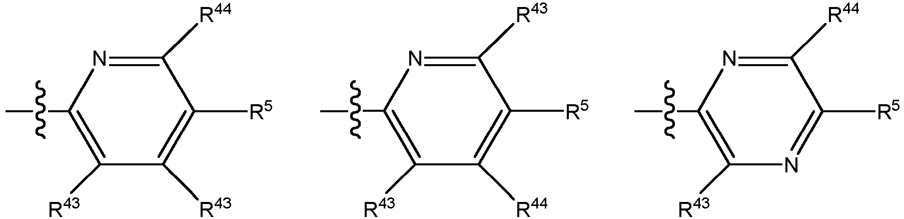

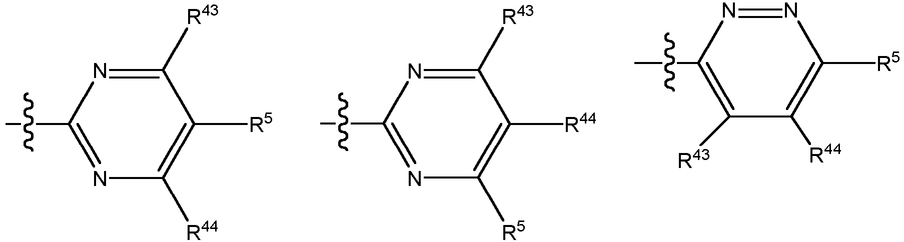

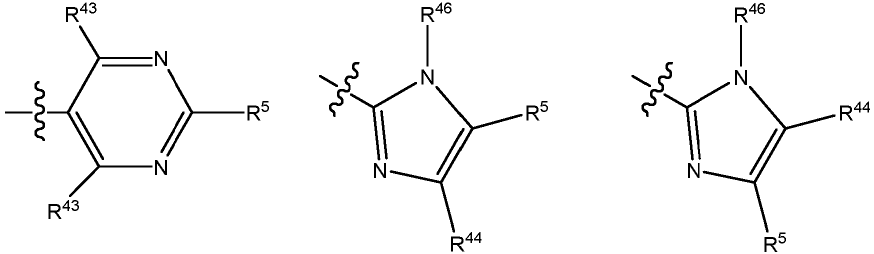

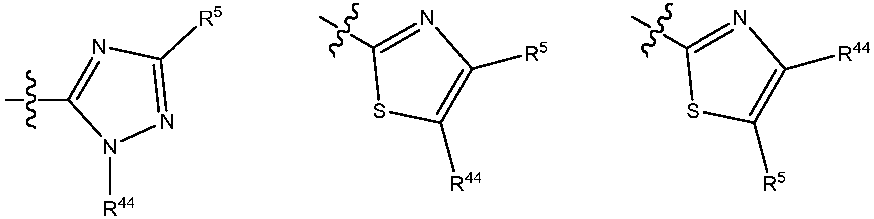

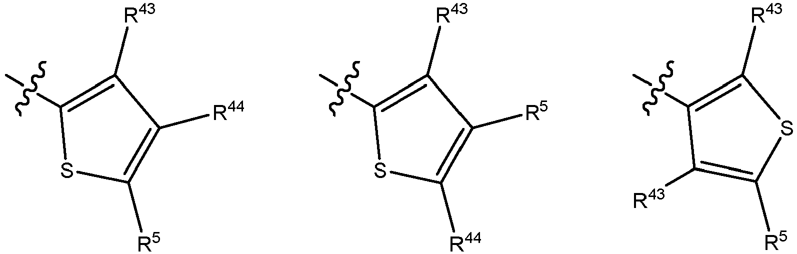

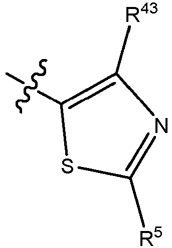

5. A compound for use according to claim 4, wherein ring C of the group L comprises any of the following structures:

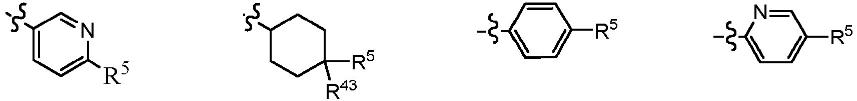

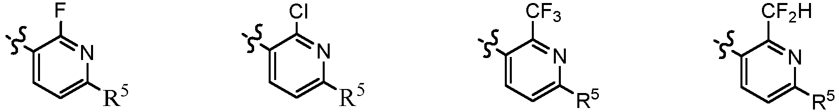

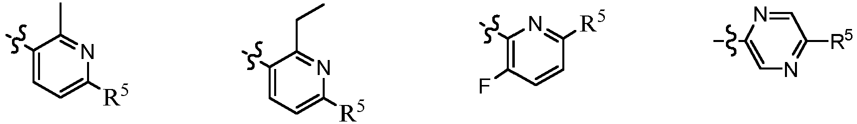

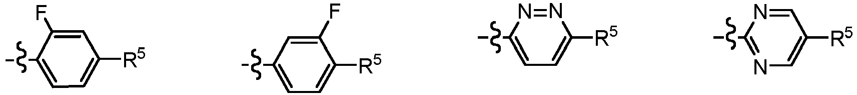

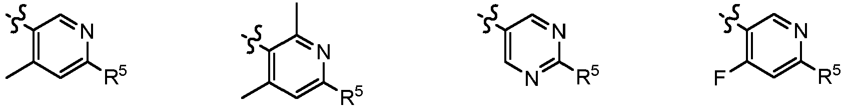

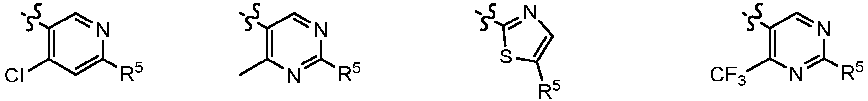

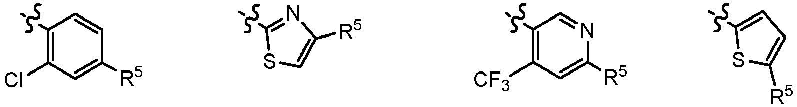

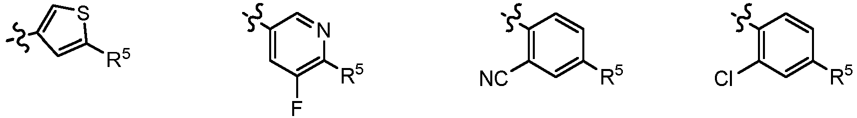

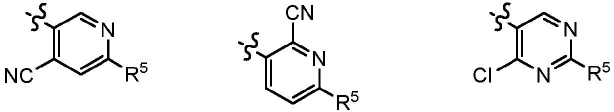

6. A compound according to any preceding claim, wherein the group R⁵ is selected from H, -F, -Cl, -Br, -I, -CN, -CONR⁵¹R⁵¹, -NR⁵¹COR⁵², -SO₂NR⁵¹R⁵¹, -NR⁵¹SO₂R⁵³, -O-CR⁵²R⁵²R⁵², -CR⁵²R⁵²NR⁵¹R⁵¹ and any of the following structures: 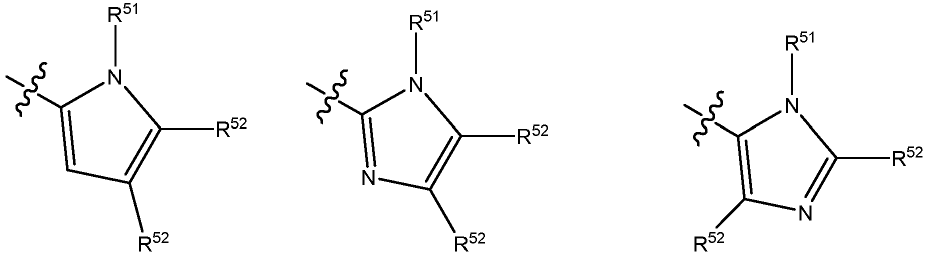 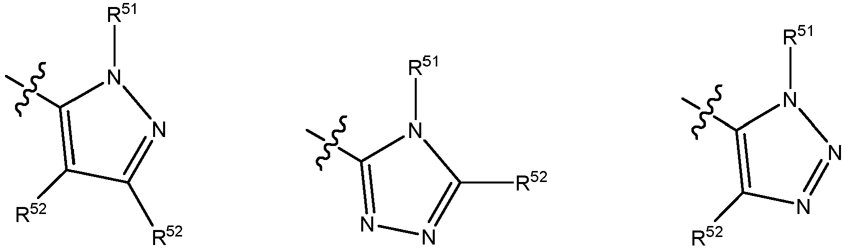 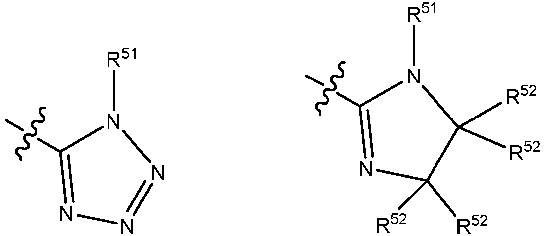 wherein, each of R⁵¹, R⁵² and R⁵³ may be the same or different and are independently selected from H and a substituted or unsubstituted organic group; optionally wherein the group R⁵ is selected from -F, -Cl, -CN, -CONH₂, -CONHMe, - CONHEt, -CONMe₂, -CONHCOMe, -CONHCH₂-CH₂OMe, -CONH-CH₂-CH₂F, - CONH-CH₂-CF₃, -CONH-CH₂-CHF₂, -OCHF₂, -NHCOMe, -NHSO₂Me, -SO₂NHMe, -CONHSO₂Me,

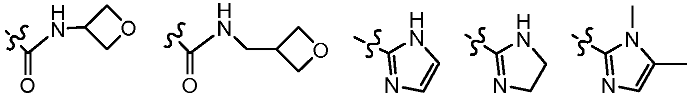

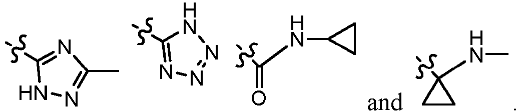

7. A compound for use according to any preceding claim, wherein X⁵ is N.

8. A compound for use according to claim 7, wherein the ring B of the group L comprises any of the following structures:

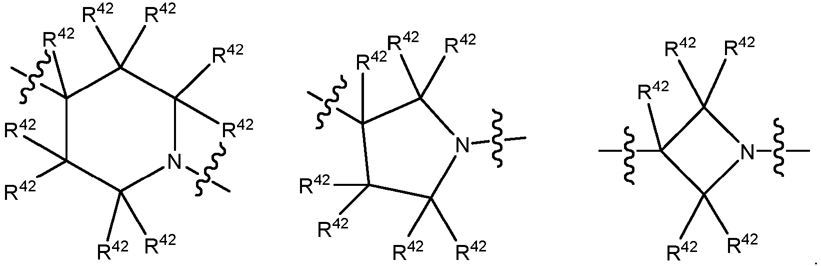

9. A compound for use according to claim 8, wherein the ring B of the group L comprises any of the following structures:

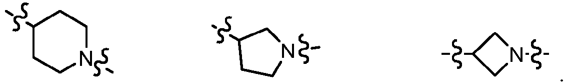

10. A compound according to any preceding claim, wherein the group L comprises a group having the following structure:

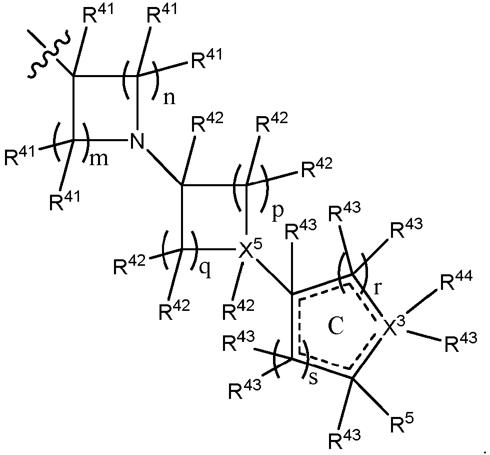

11. A compound for use according to any preceding claim, wherein the group L comprises a group selected from the following structures:

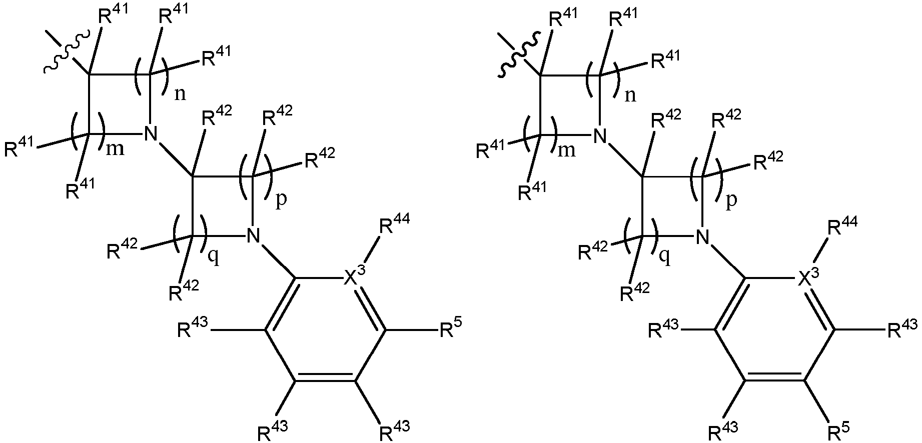

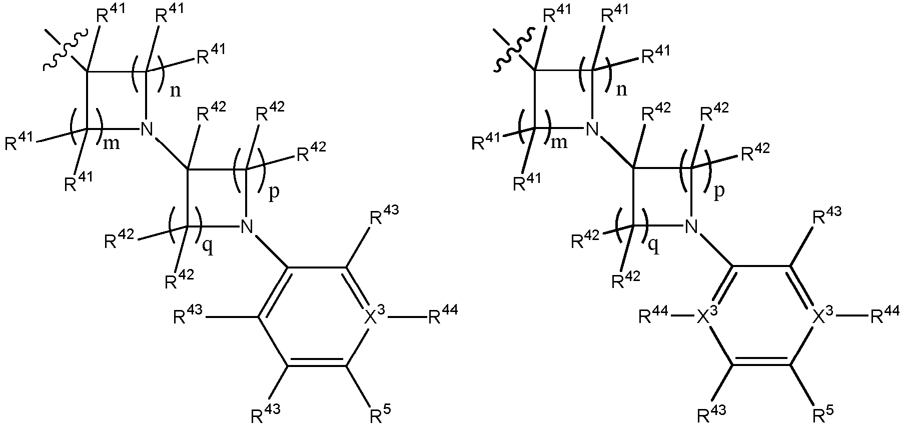

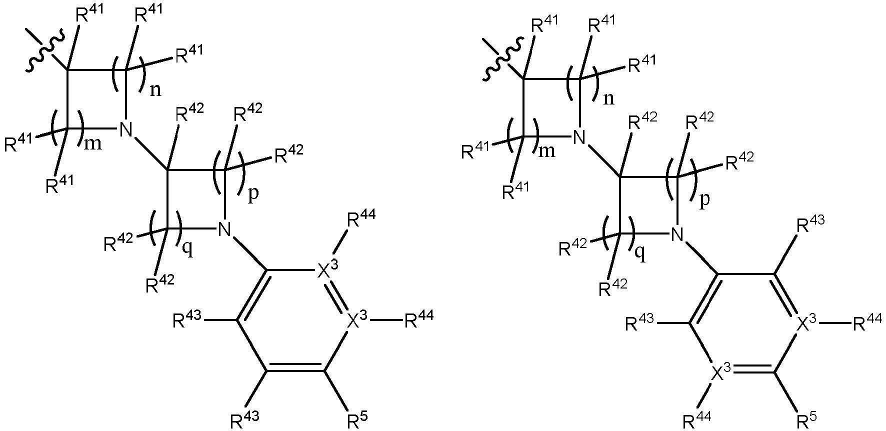

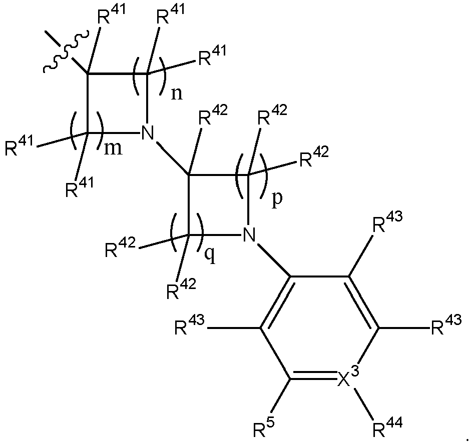

12. A compound for use according to any preceding claim, wherein m is selected from 1 or 2, n is selected from 2 or 3, p is selected from 1, 2 or 3 and q is selected from 1 or 2.

13. A compound for use according to claim 11 or claim 12, wherein the group L comprises a group having any the following structures:

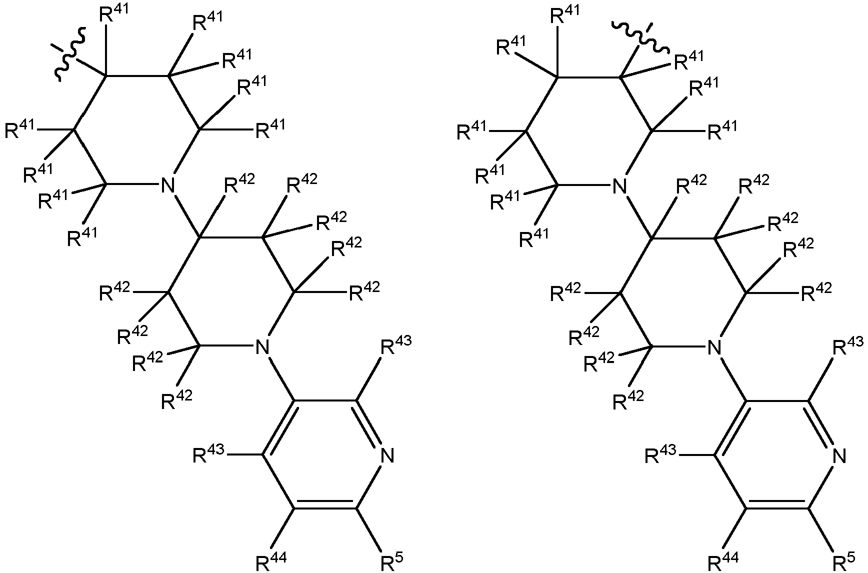

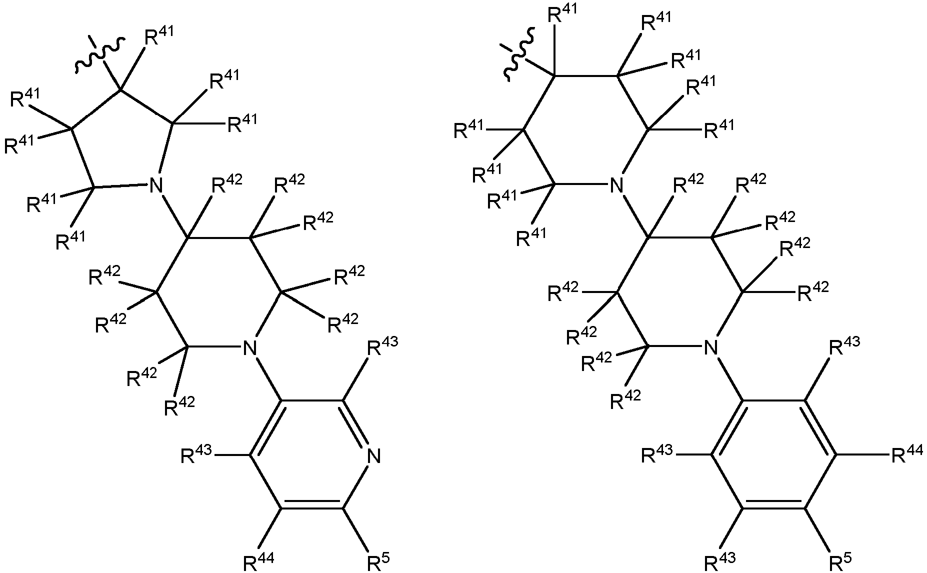

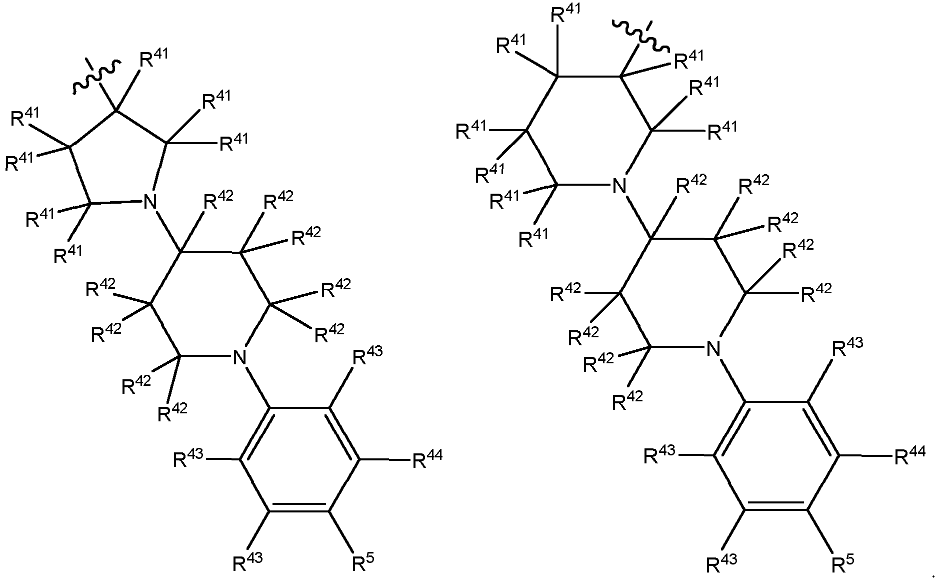

14. A compound for use according to any preceding claim, wherein the group L comprises a group having any the following structures:

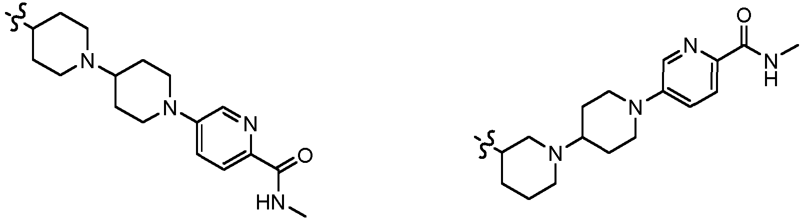

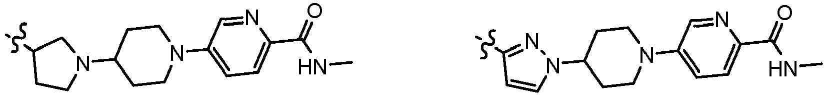

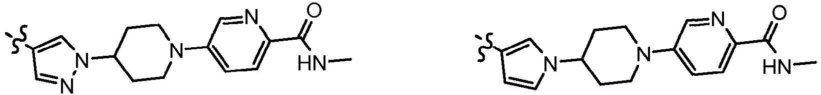

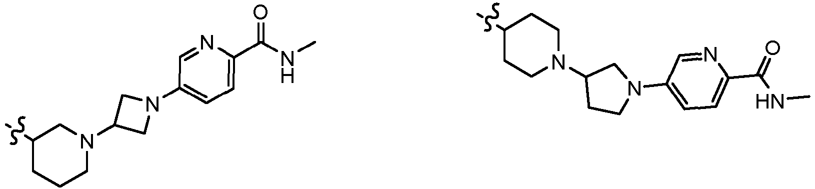

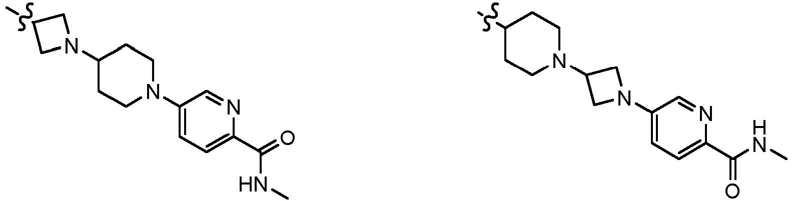

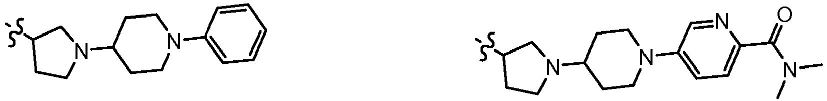

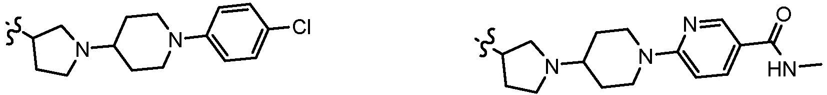

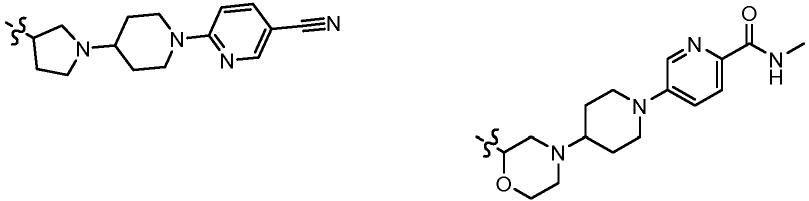

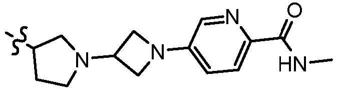

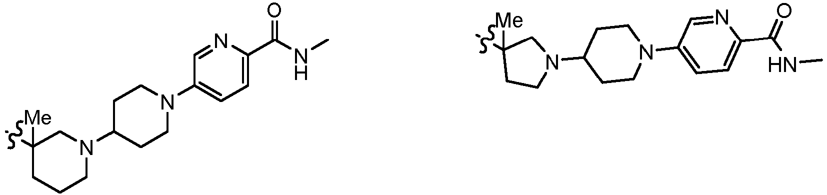

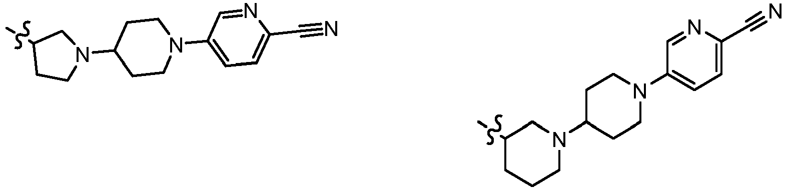

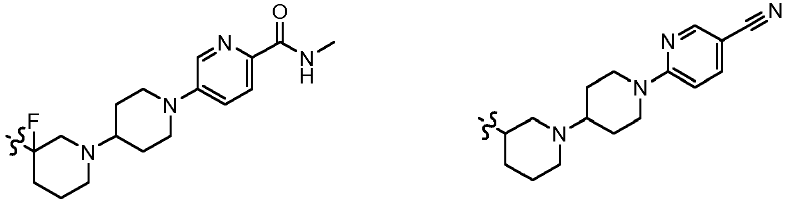

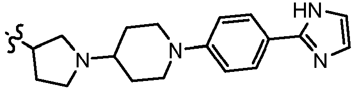

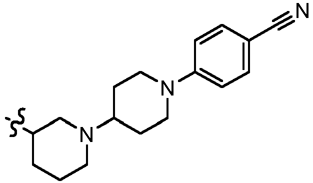

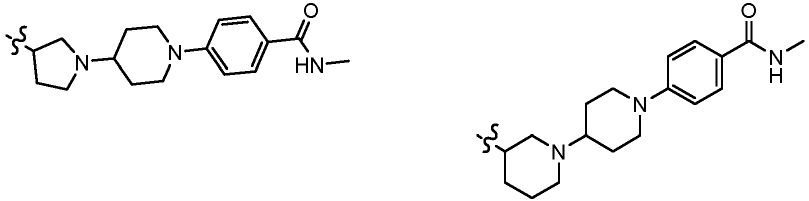

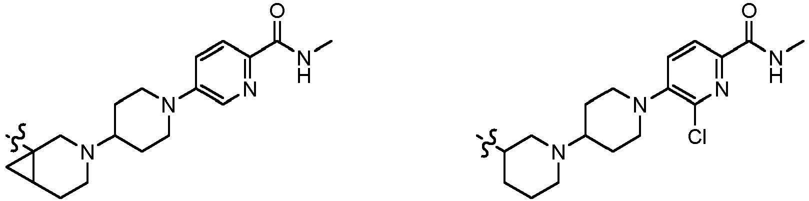

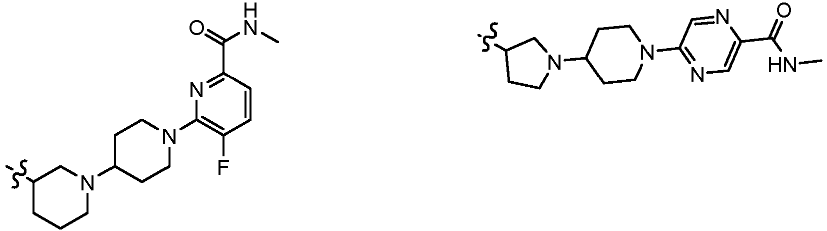

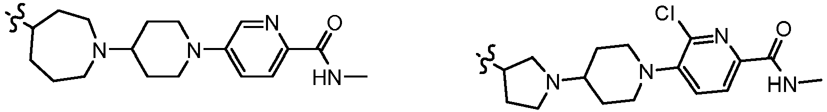

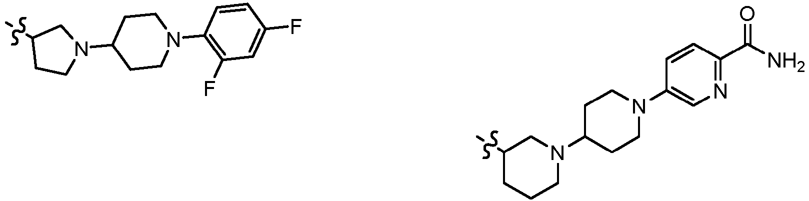

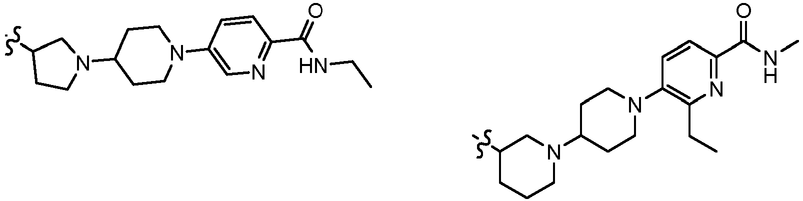

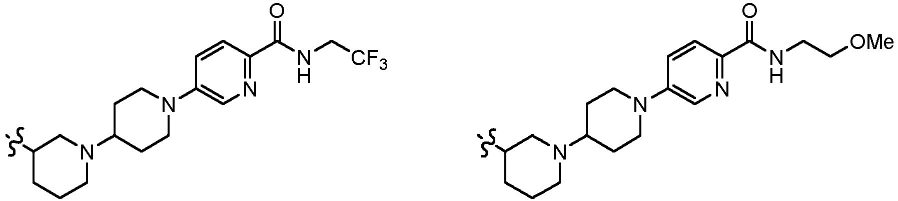

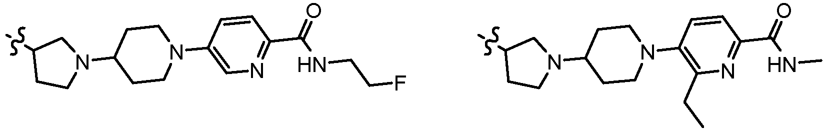

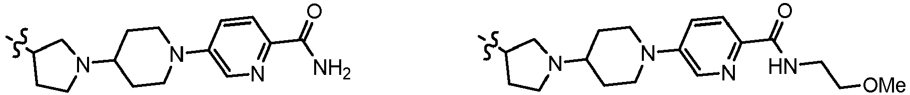

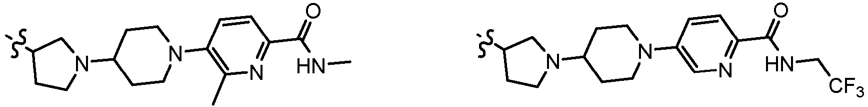

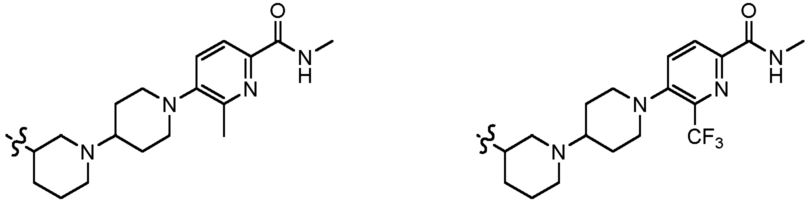

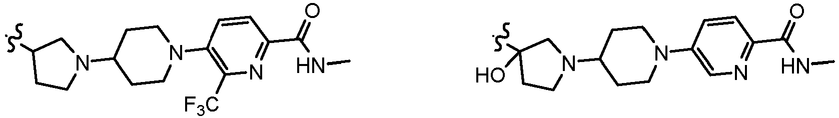

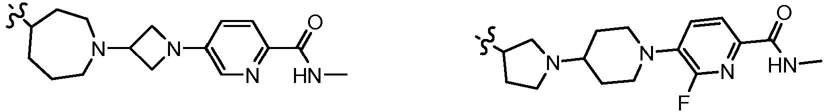

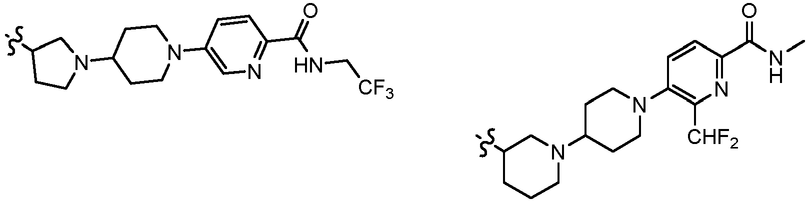

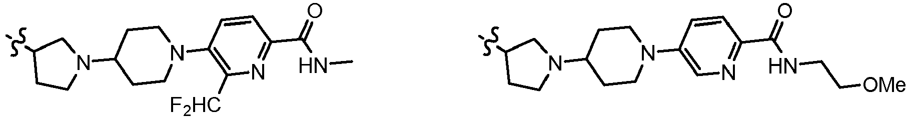

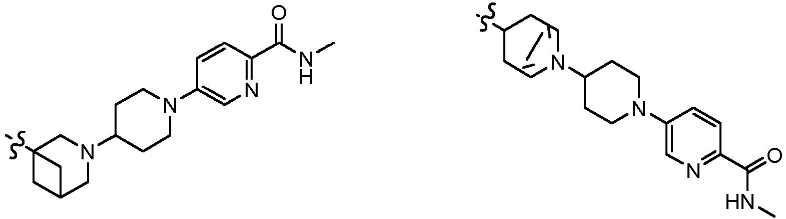

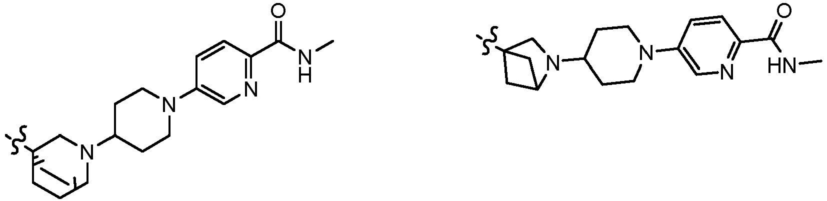

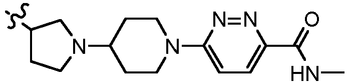

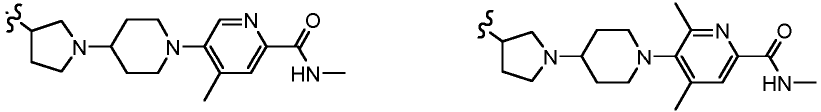

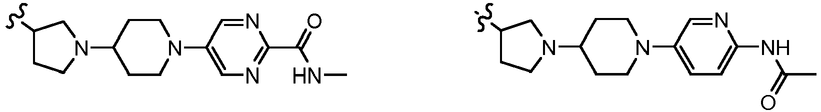

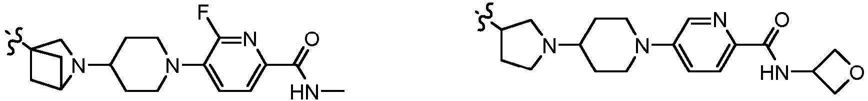

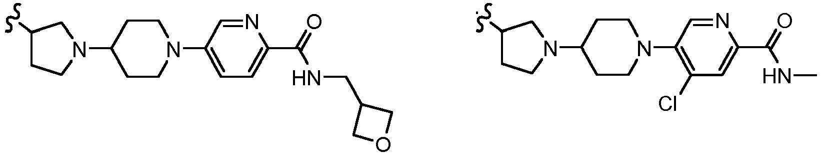

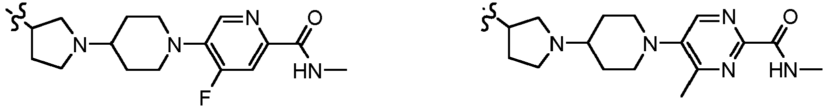

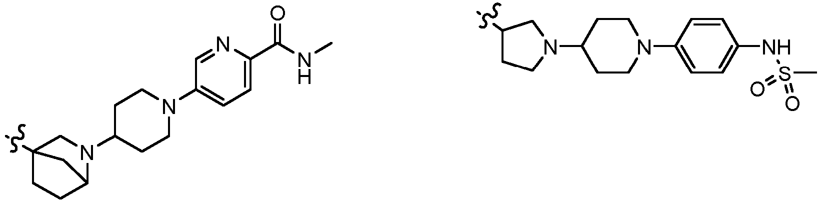

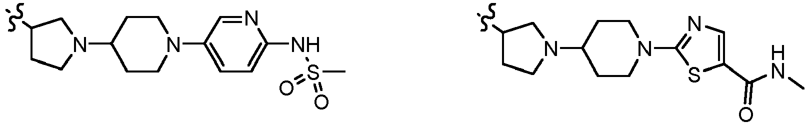

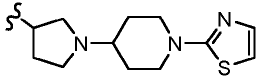

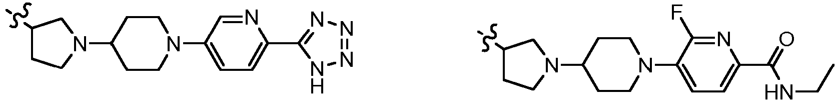

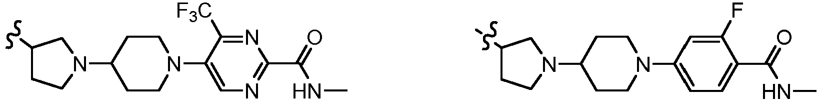

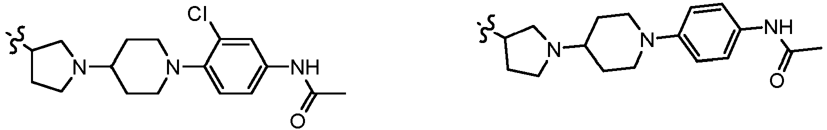

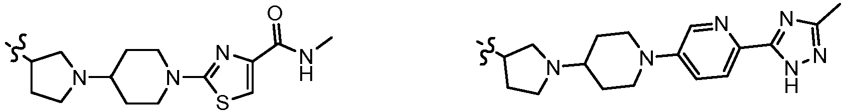

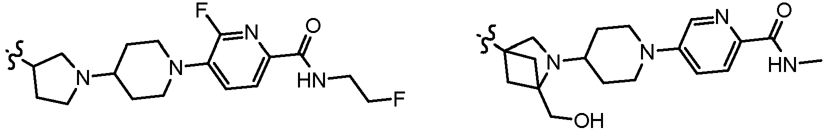

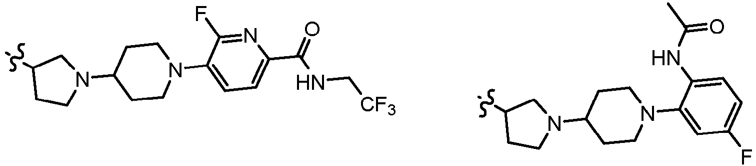

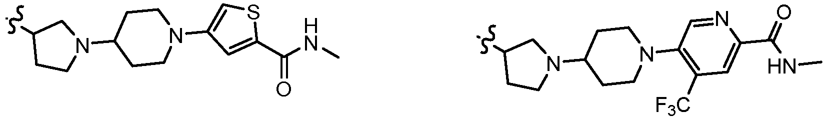

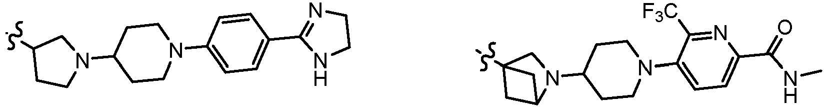

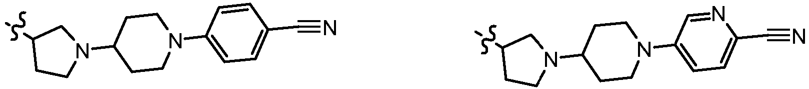

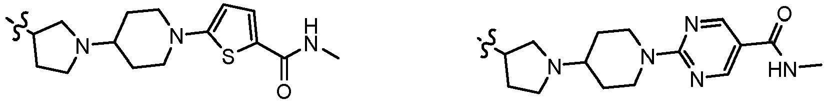

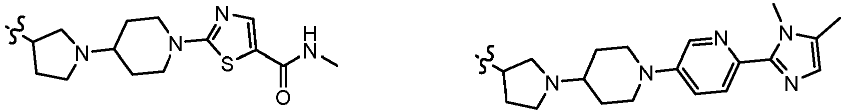

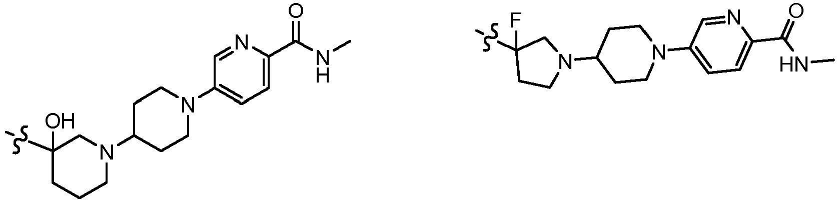

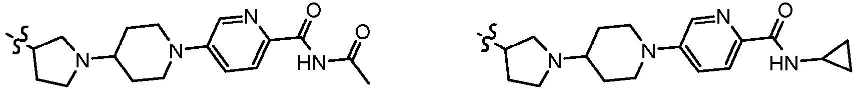

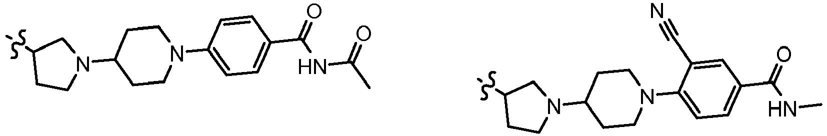

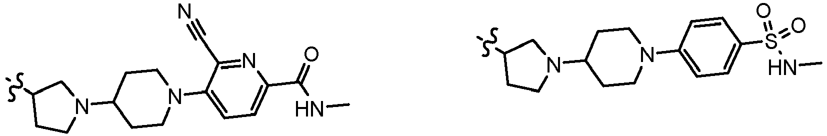

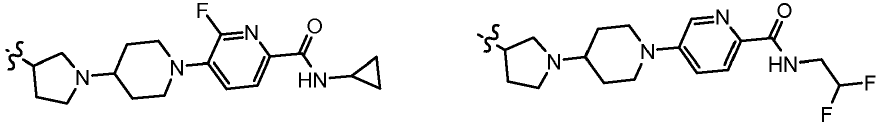

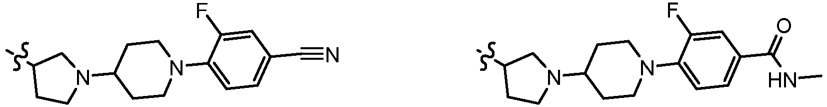

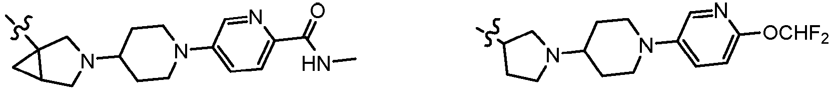

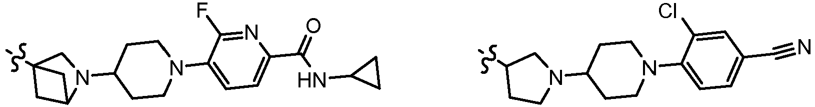

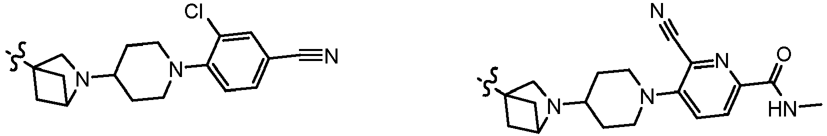

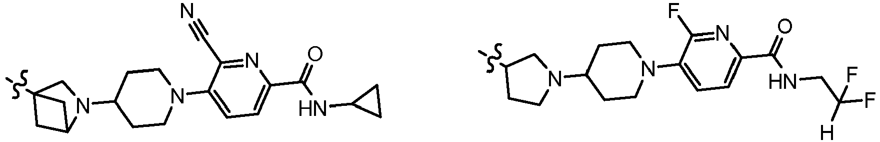

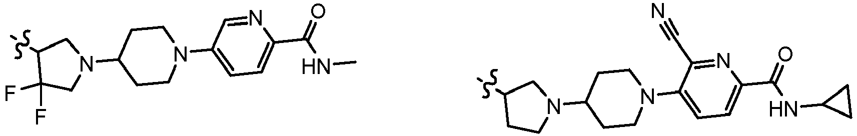

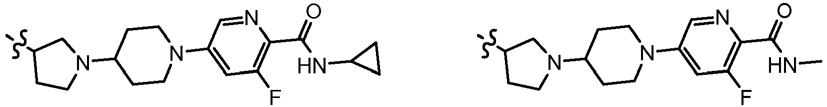

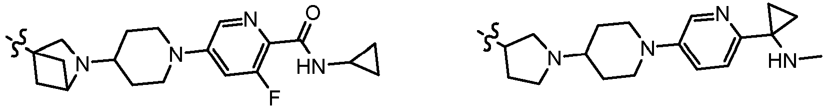

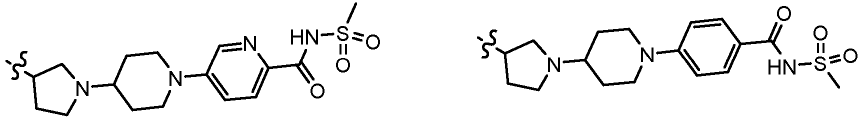

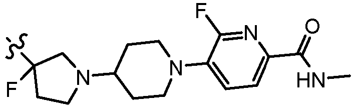

15. A compound according to any preceding claim, which compound comprises any of the following structures: 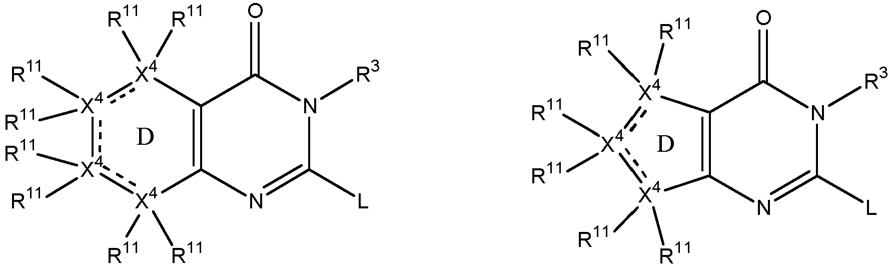 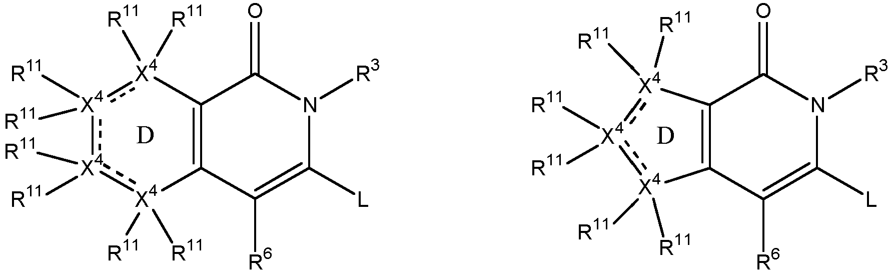 preferably wherein at least one X⁴ is C.

16. A compound for use according to claim 15, which compound comprises any of the following structures: 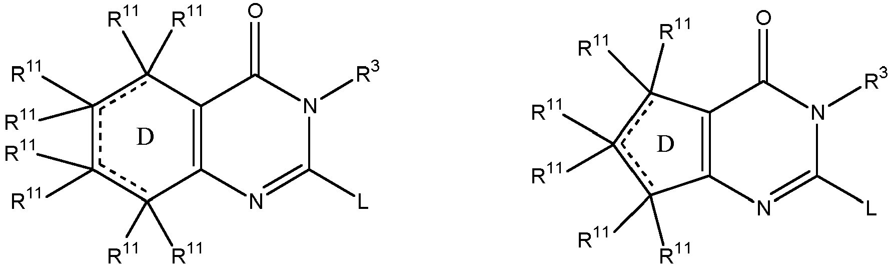 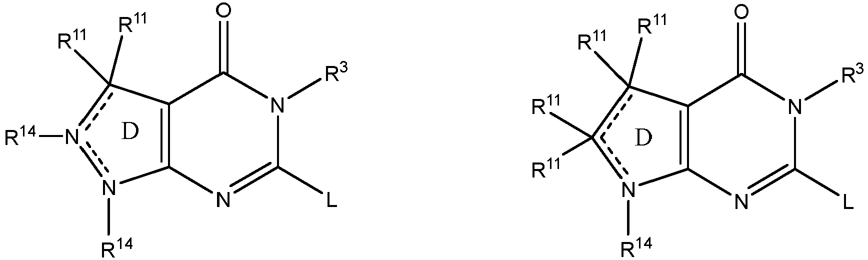 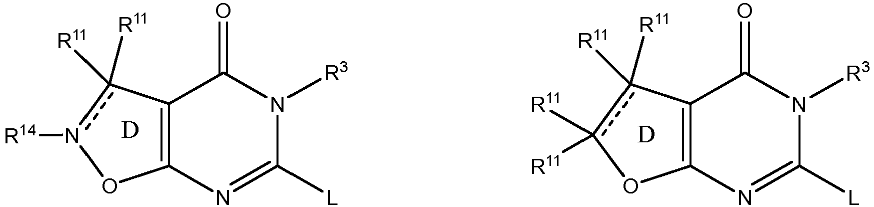 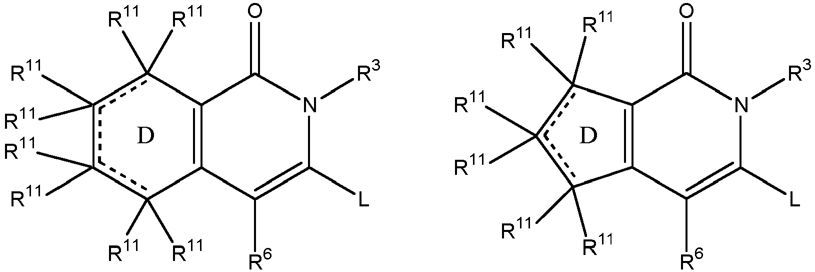 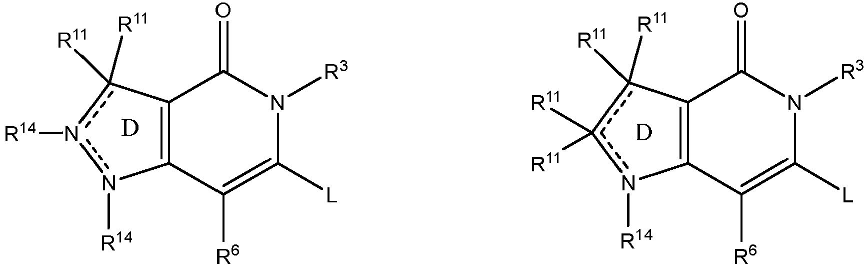 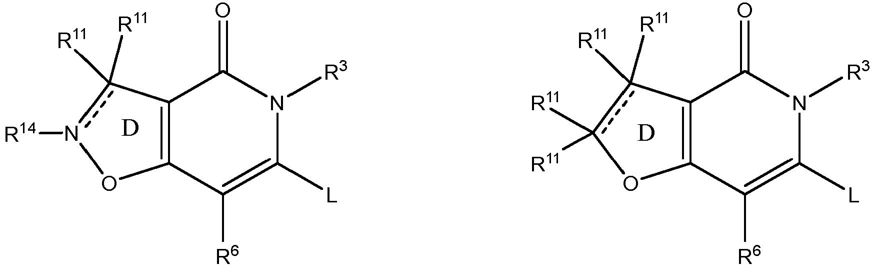 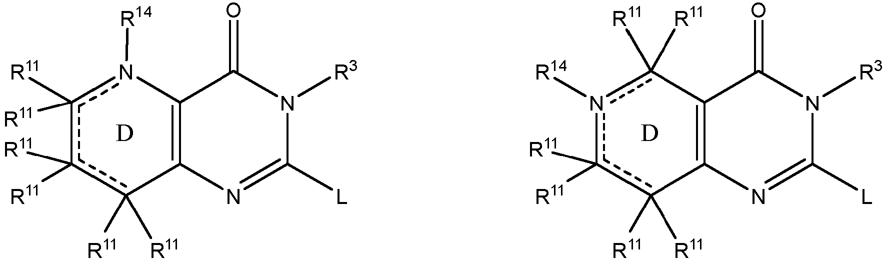 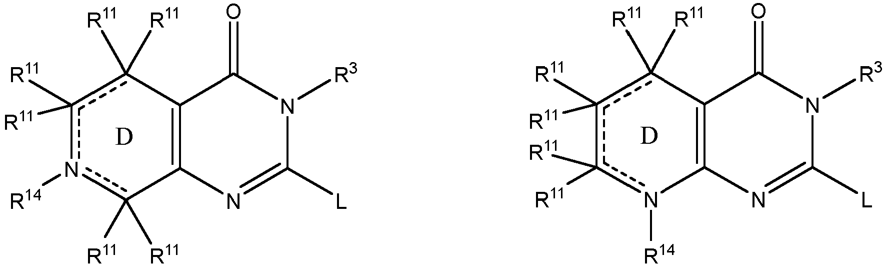 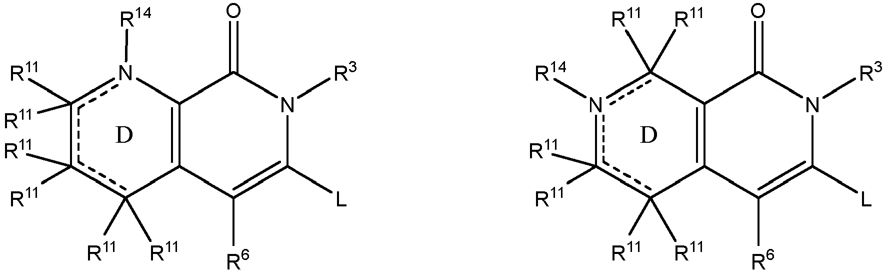 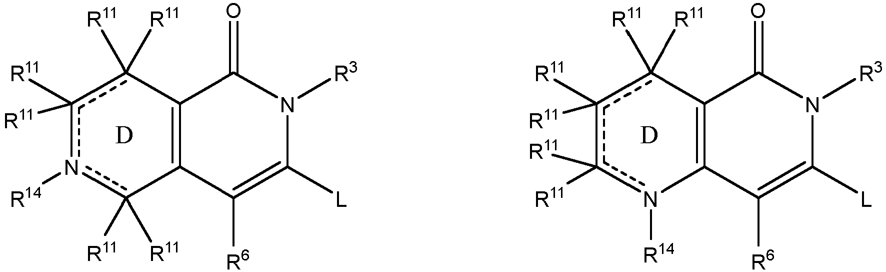 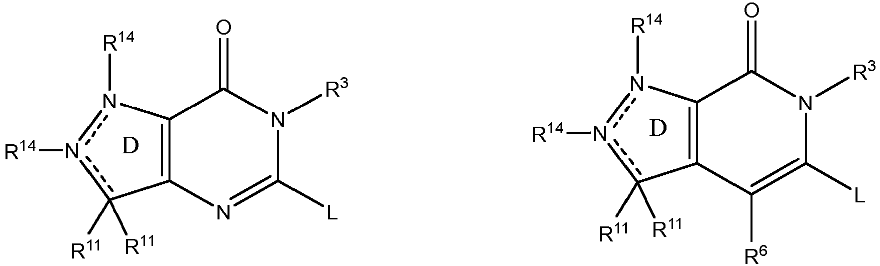 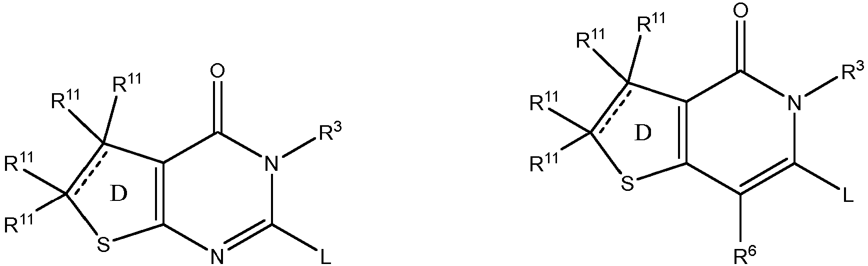 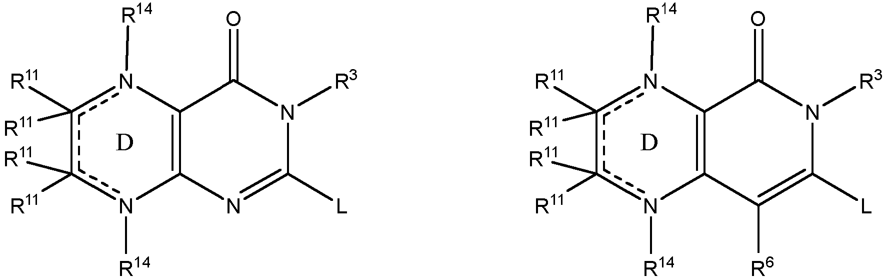 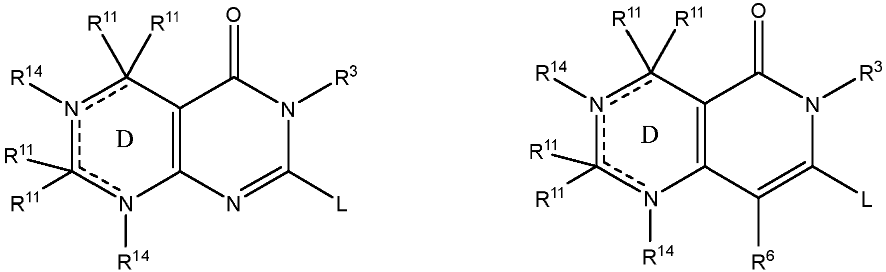 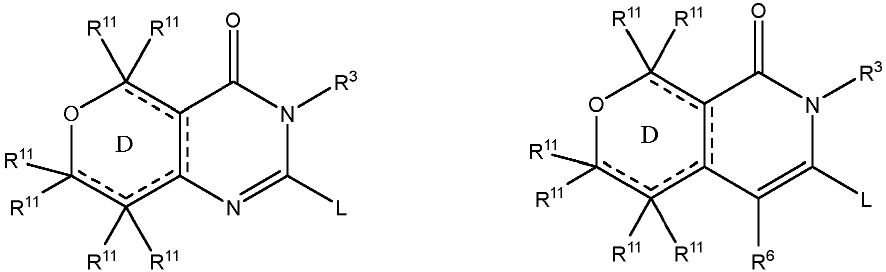 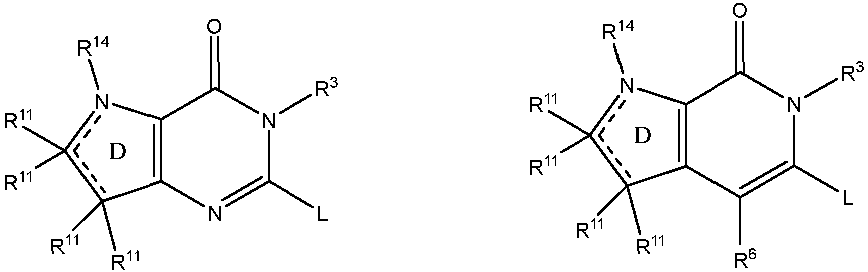 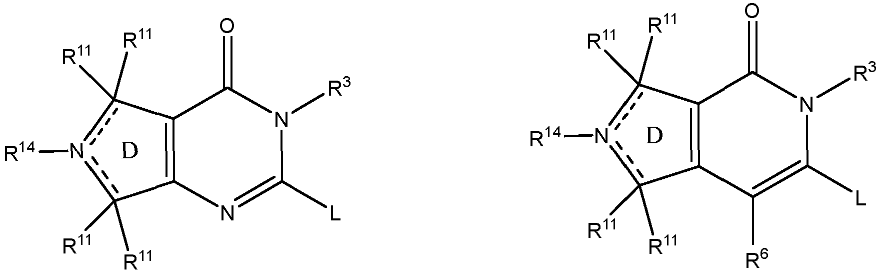 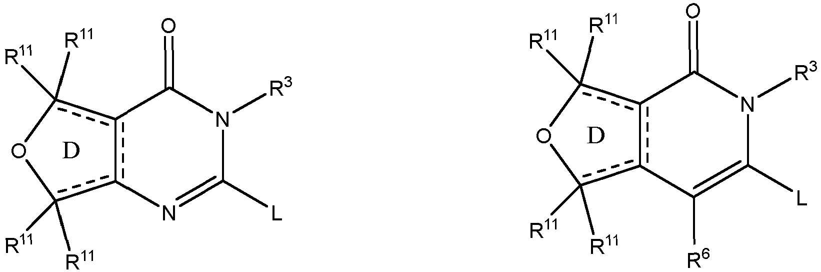 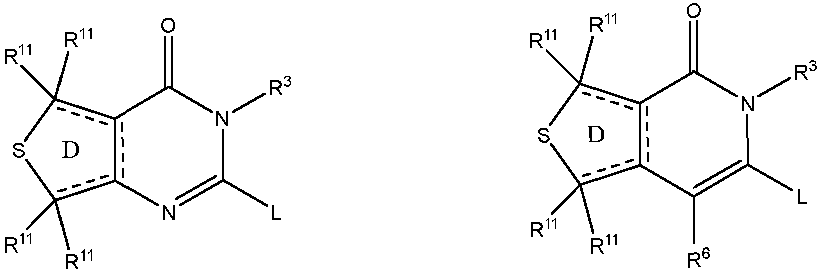 wherein R¹⁴ may be present or absent and if present is selected from H, C₁ to C₃ alkyl or C₁ to C₃ fluoroalkyl.

17. A compound according to claim 16, which compound comprises any of the following structures:

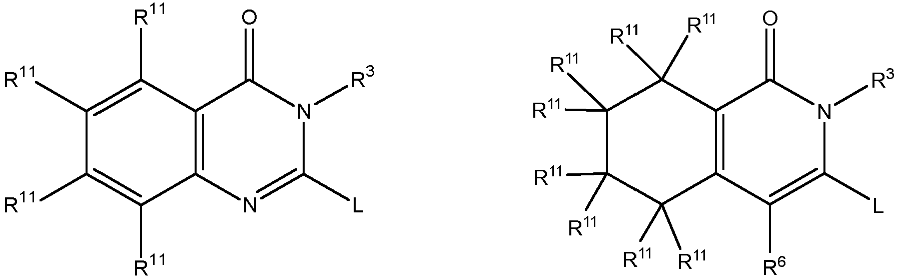

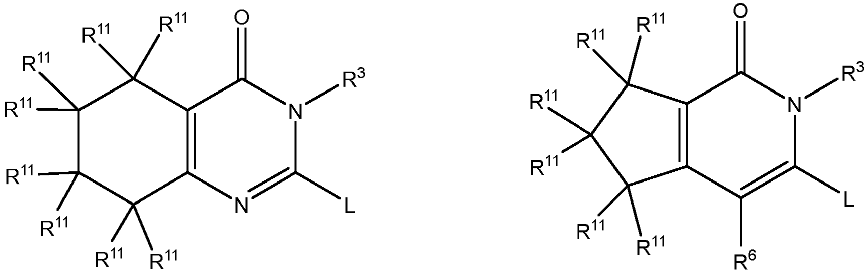

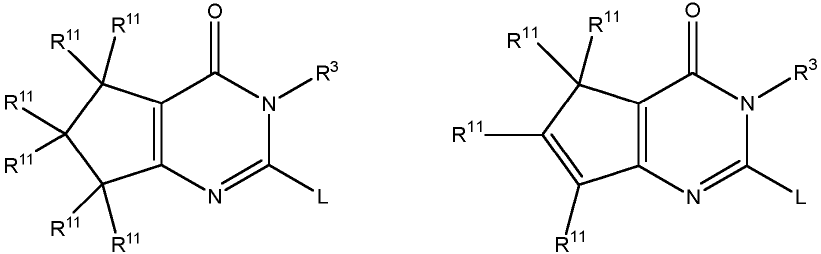

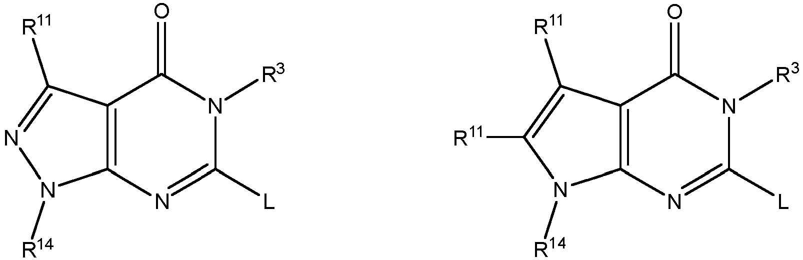

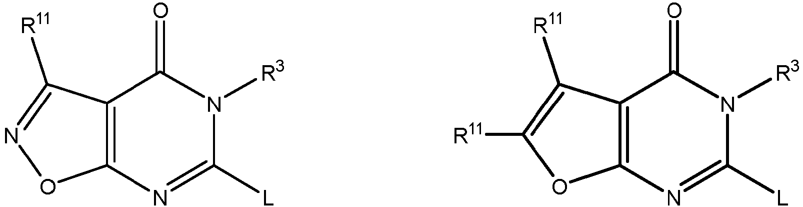

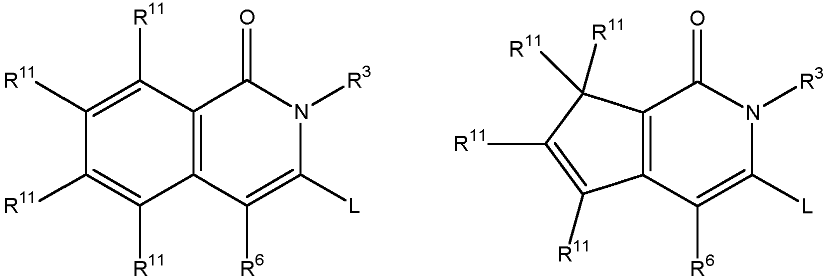

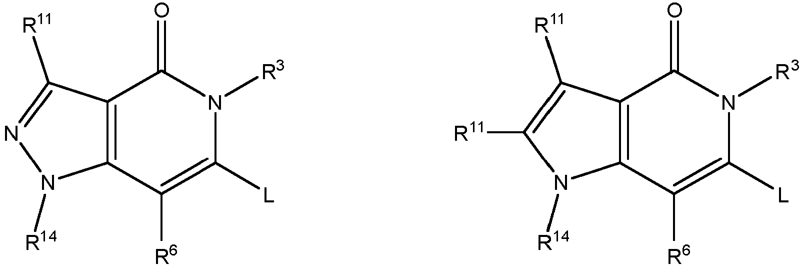

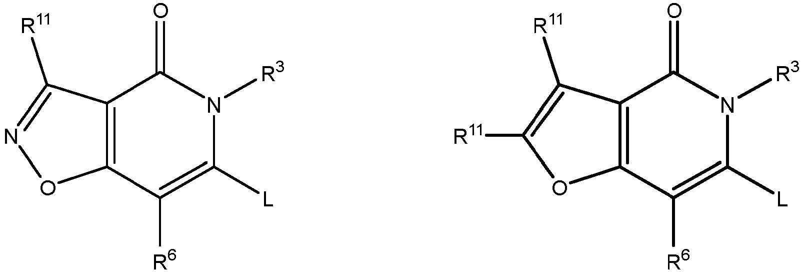

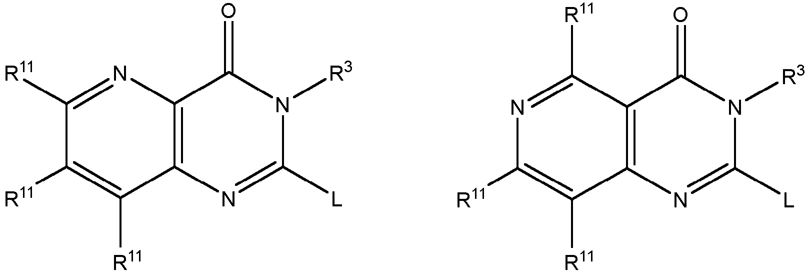

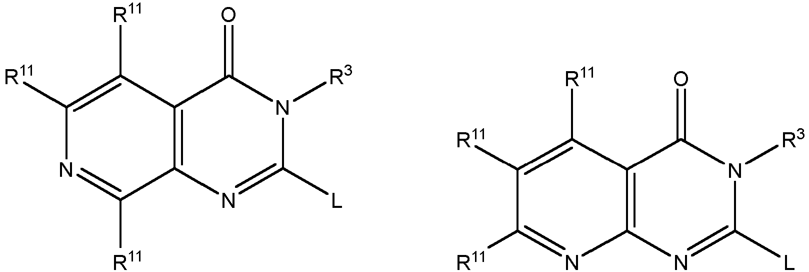

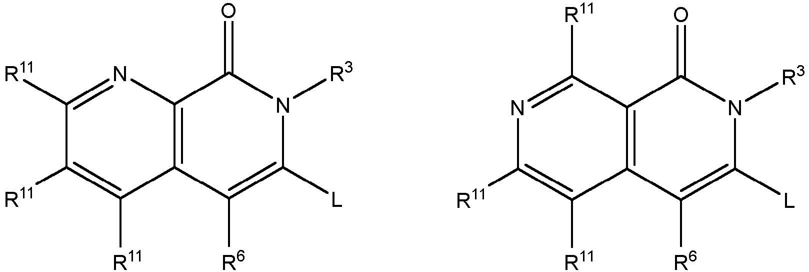

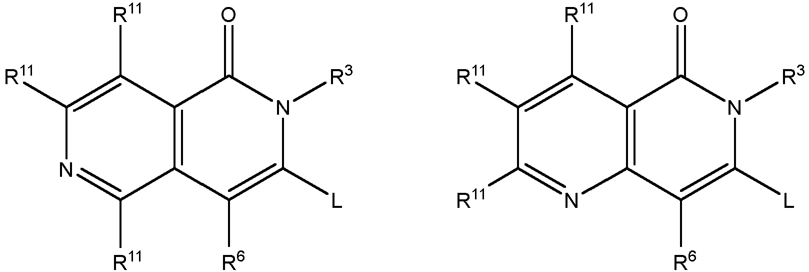

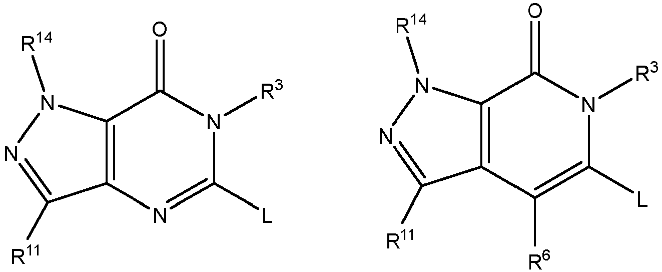

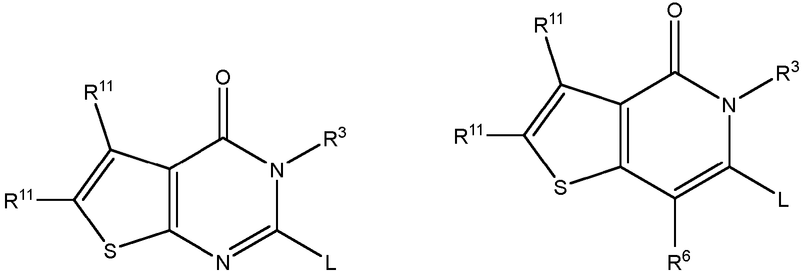

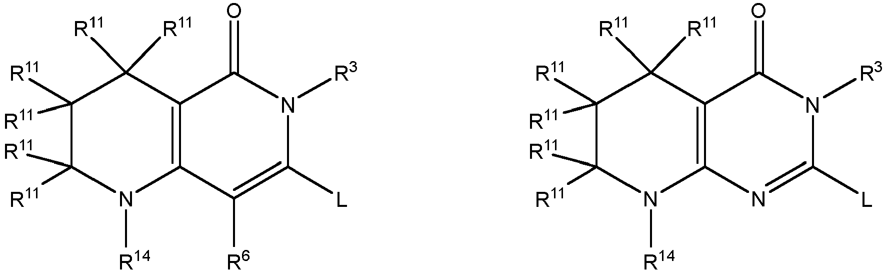

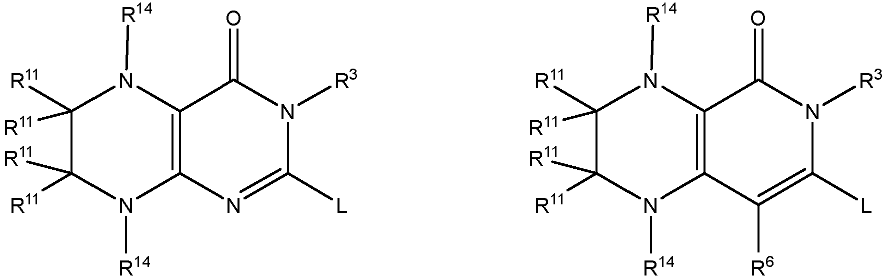

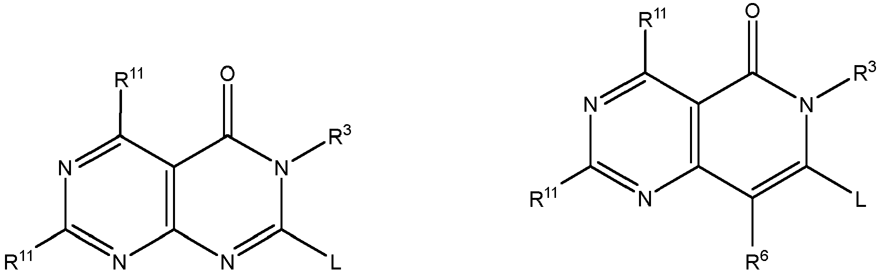

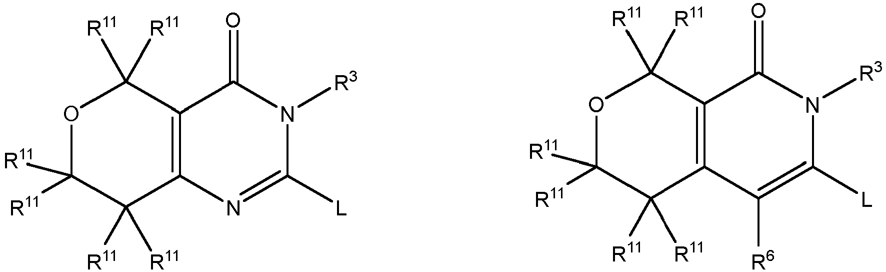

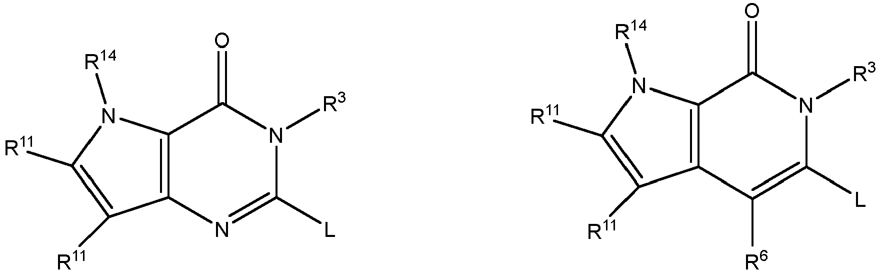

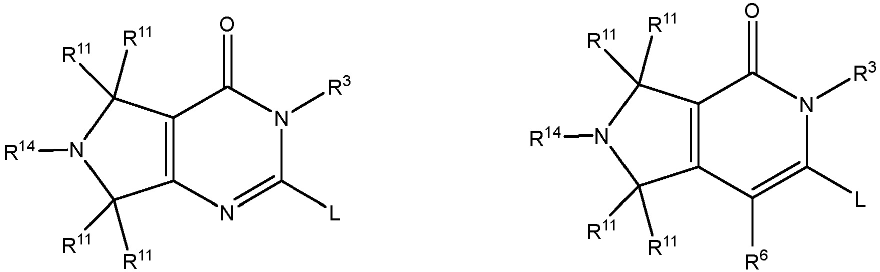

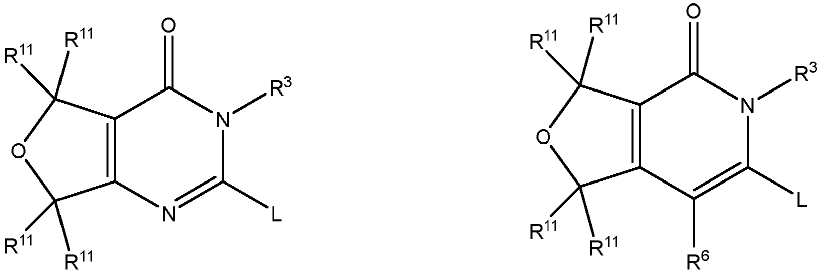

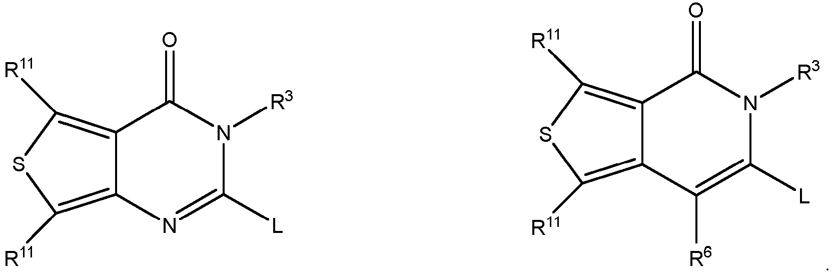

18. A compound for use according to any preceding claim, wherein each R¹¹ is independently selected from H, Cl, F, CHF₂, CF₃, CH₃, OH, CH₃O, and NH₂, and/or a pair of R¹¹ groups attached to the same atom forming =O; and independently, when a pair of R¹¹ groups attached to different atoms together forms a ring with ring D atoms, the pair of R¹¹ groups comprises -CH₂CH₂CH₂-.

19. A compound for use according to claim 18, which compound comprises any of the following structures:

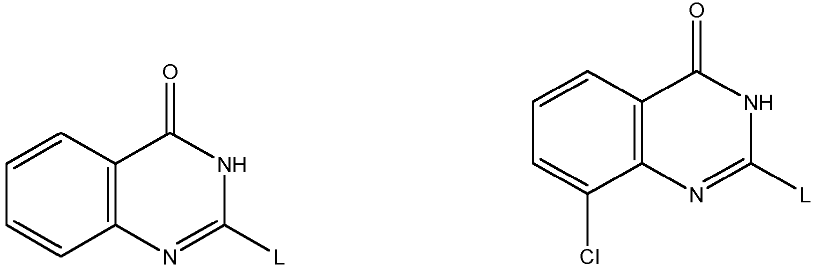

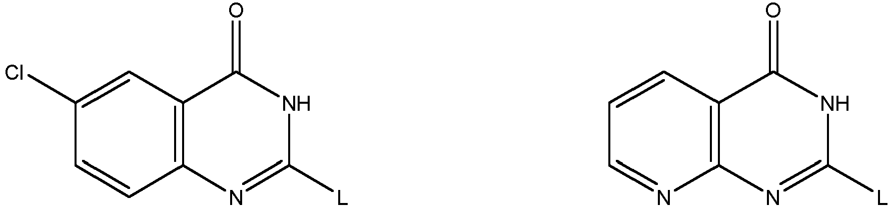

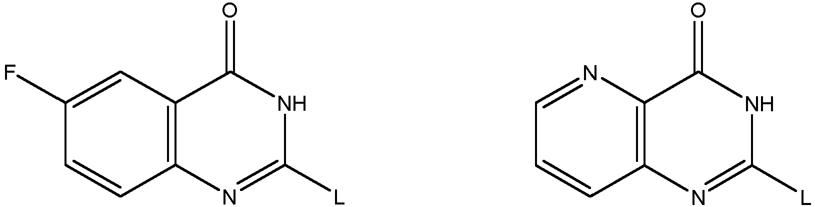

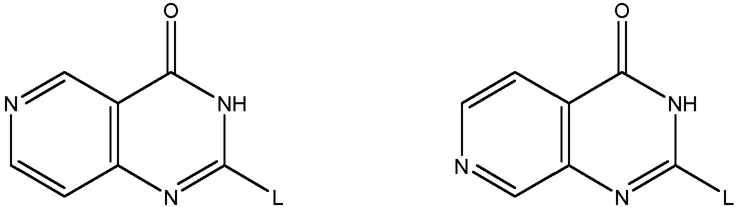

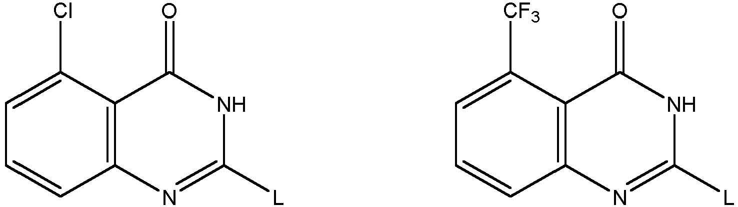

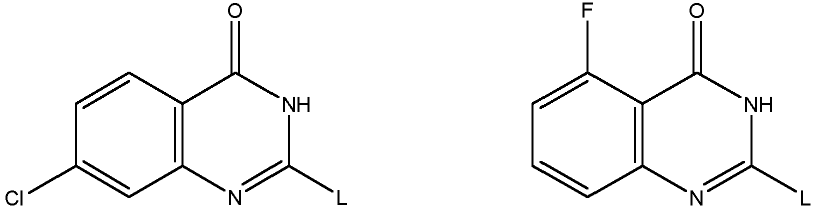

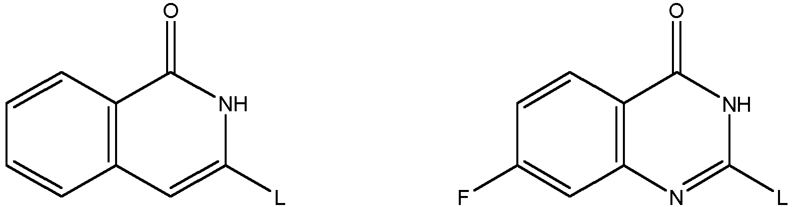

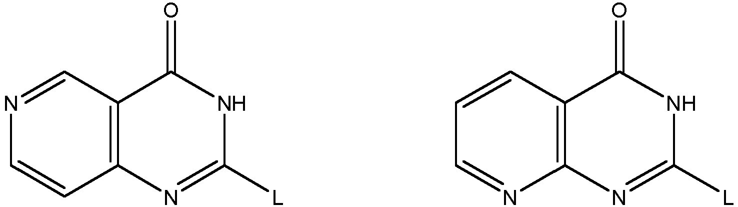

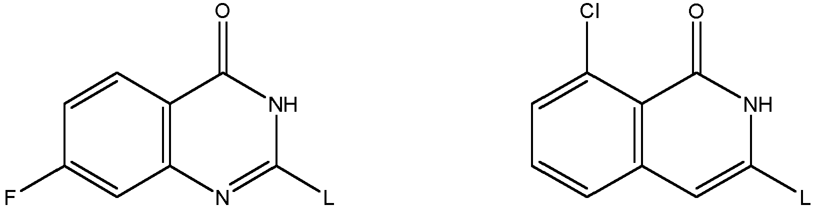

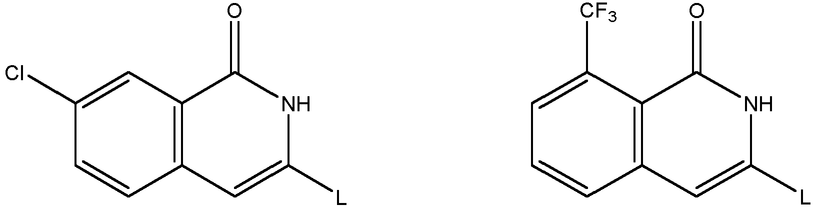

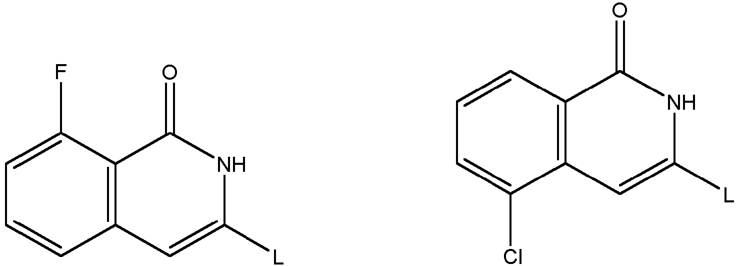

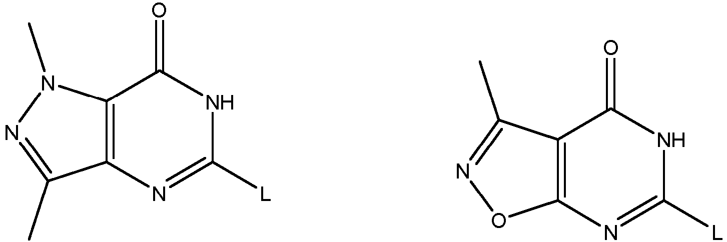

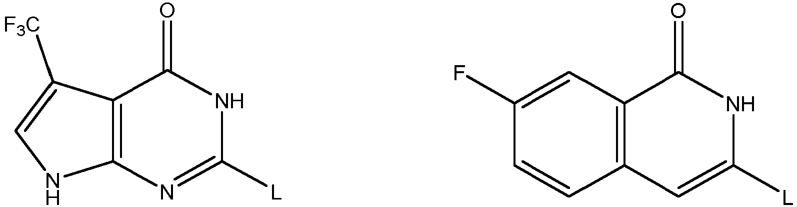

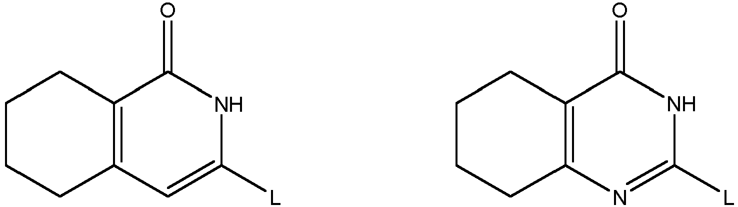

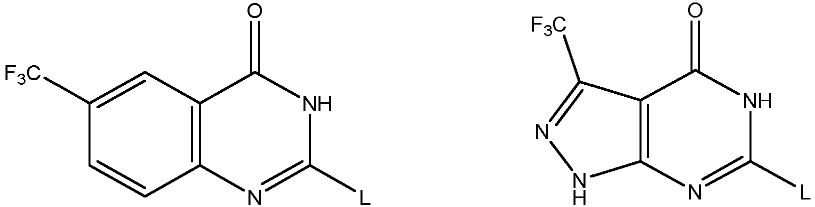

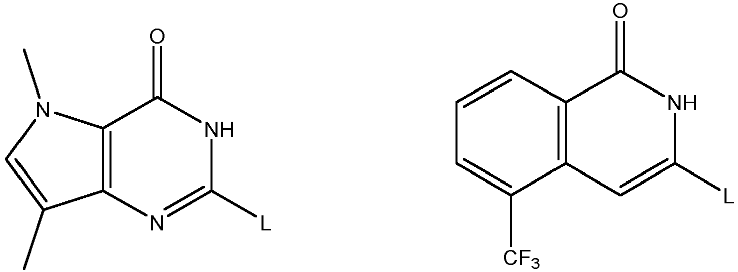

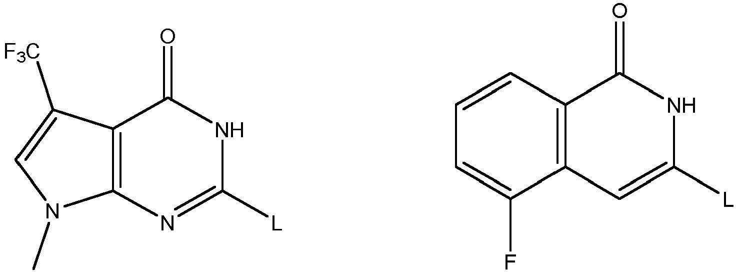

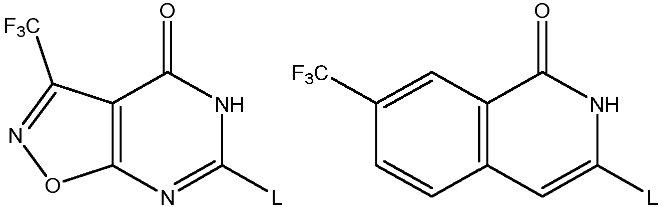

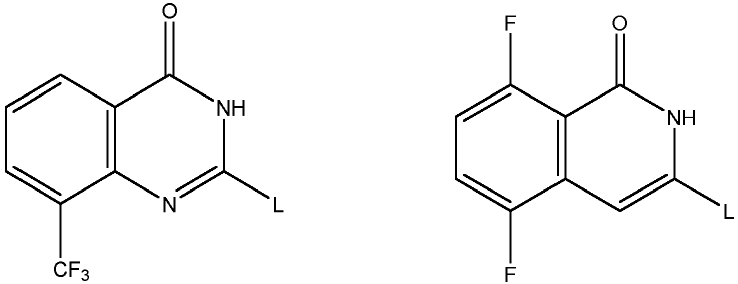

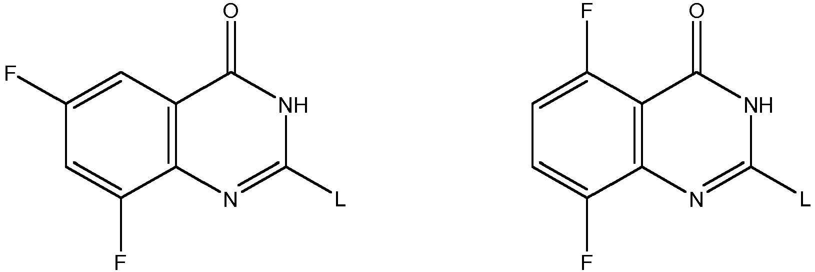

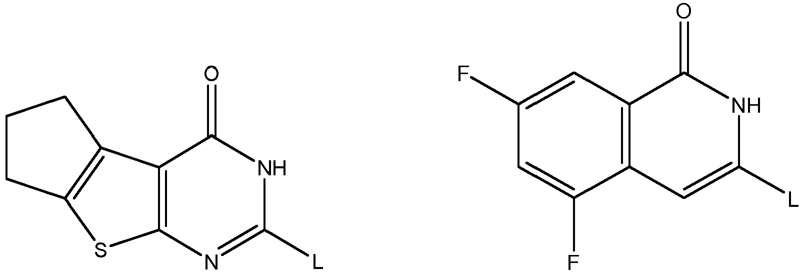

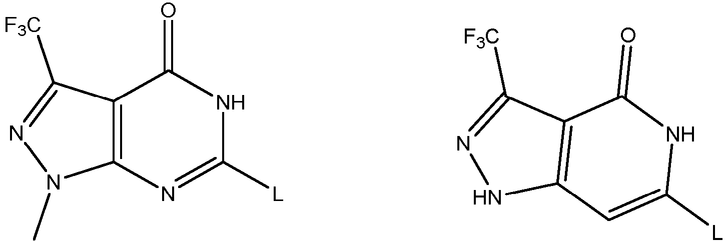

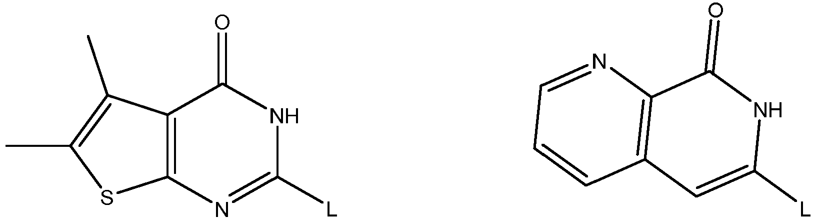

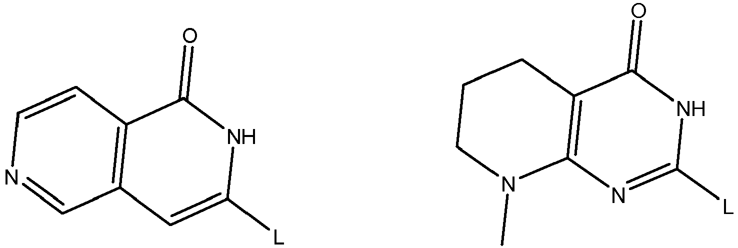

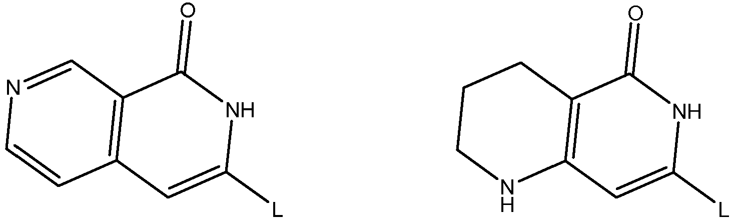

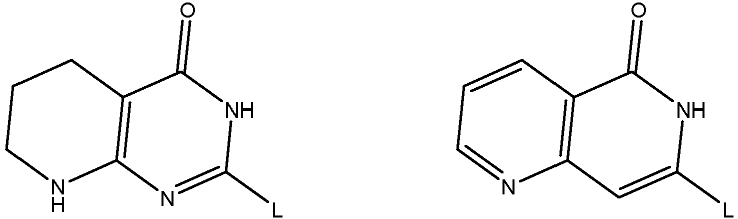

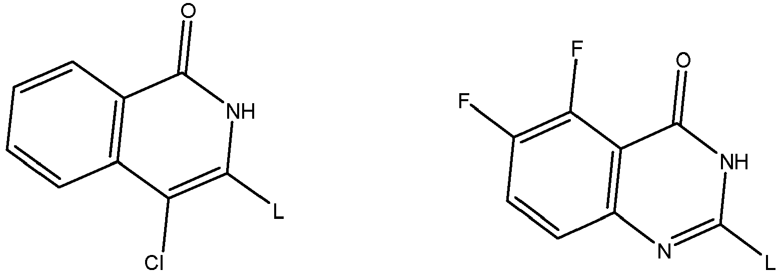

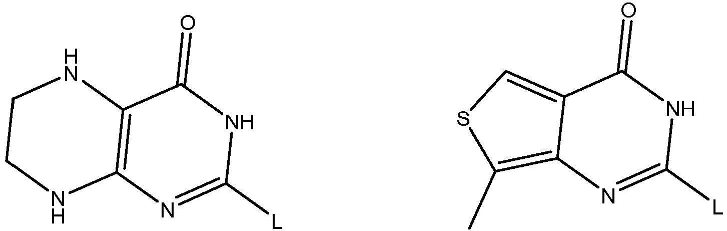

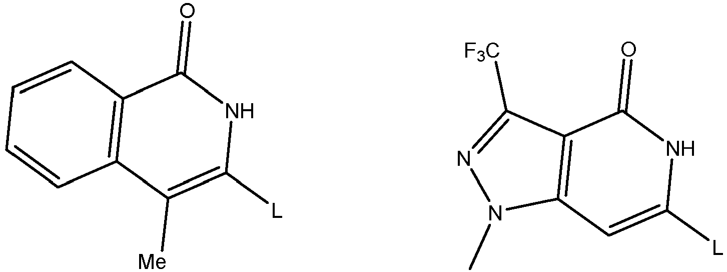

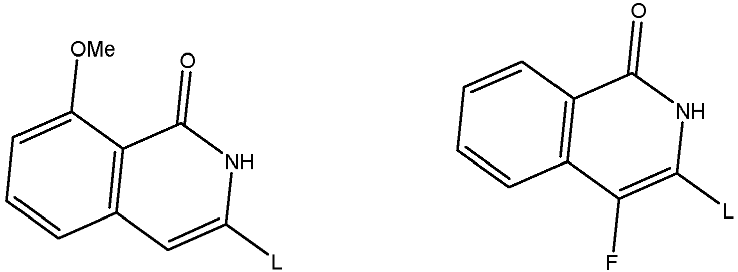

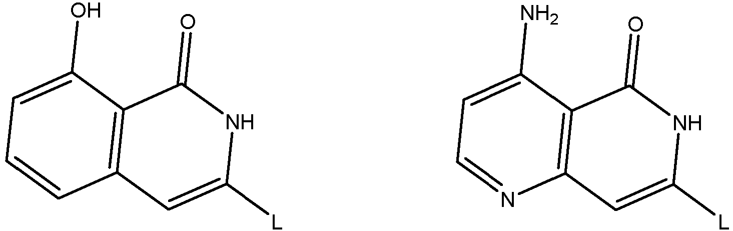

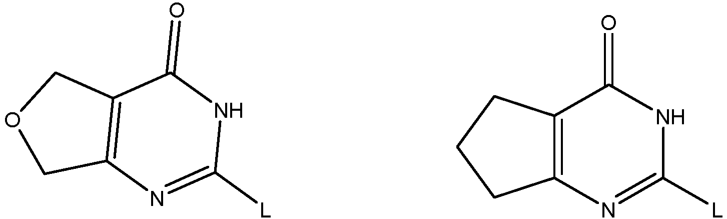

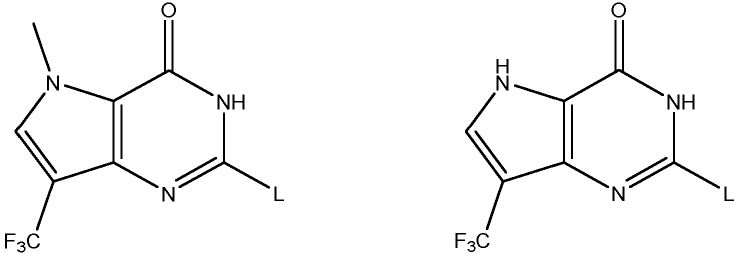

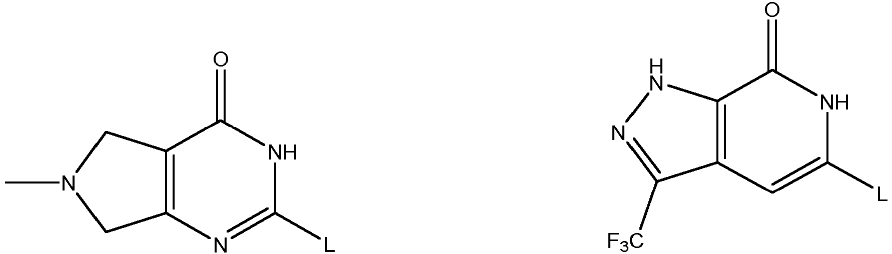

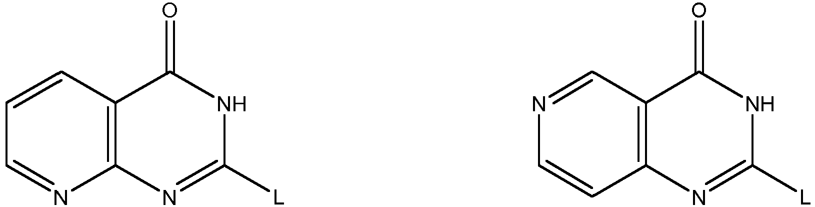

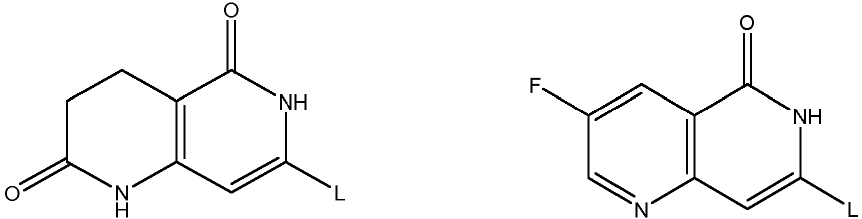

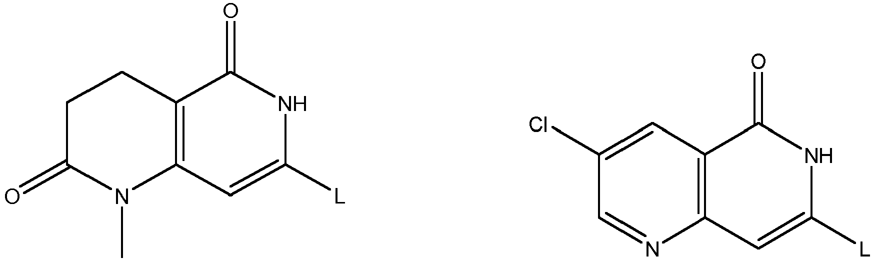

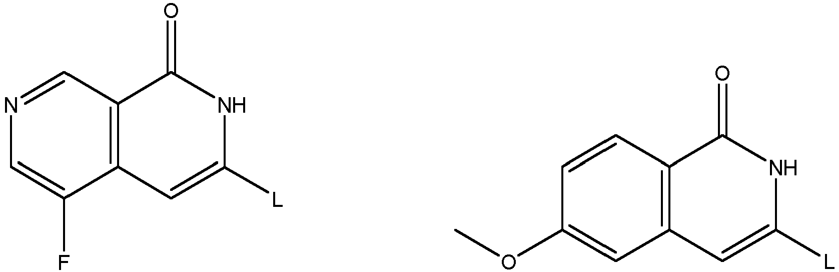

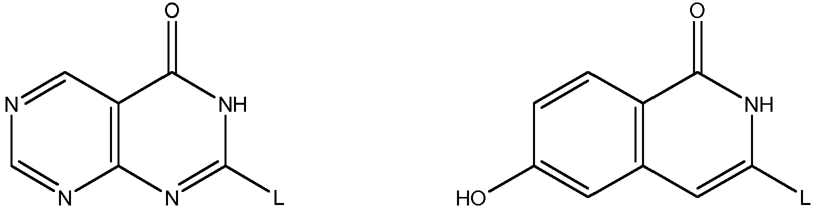

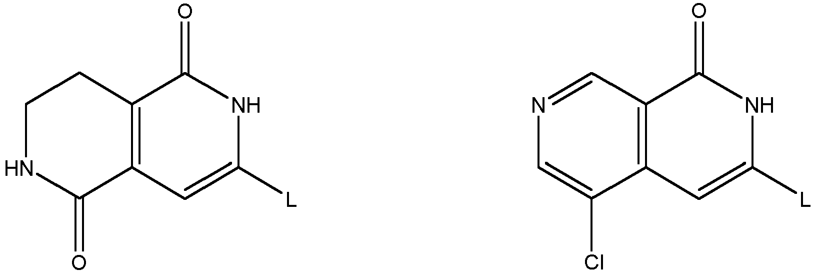

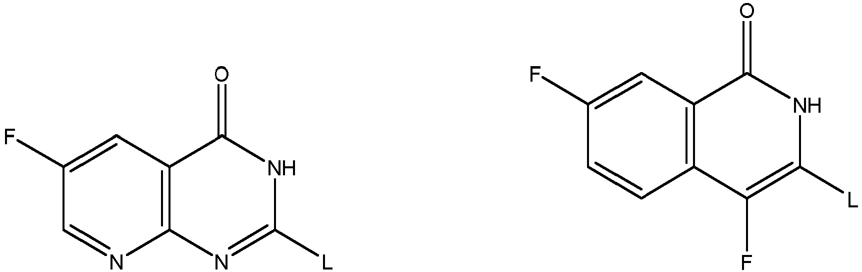

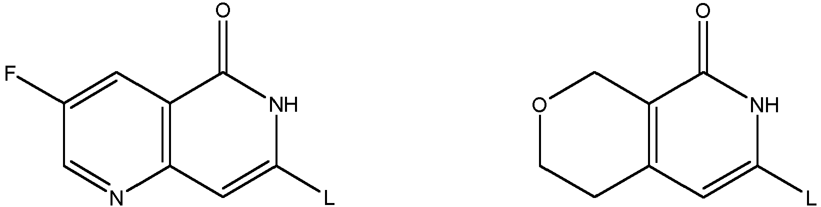

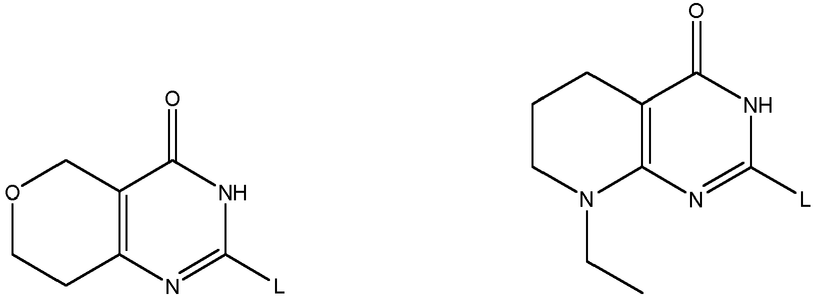

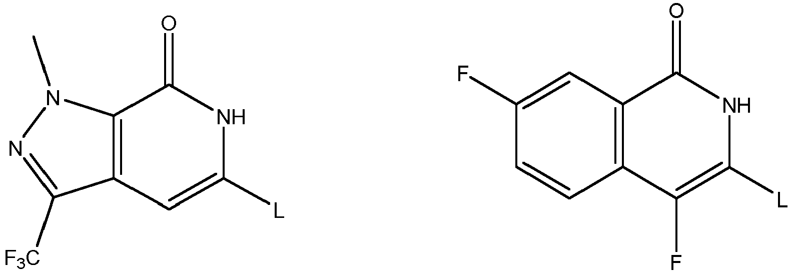

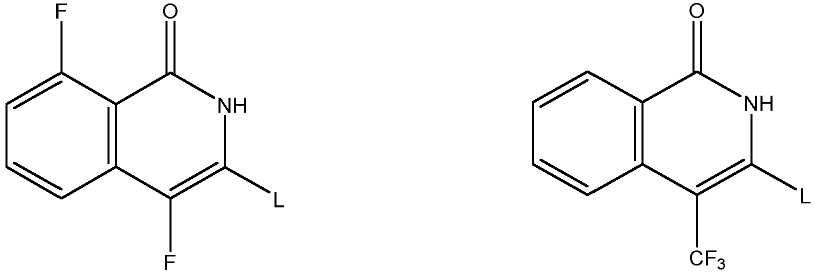

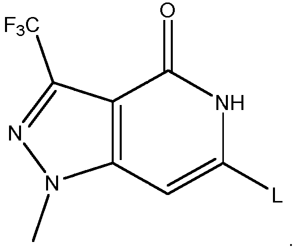

20. A compound for use according to any preceding claim, which compound comprises a formula selected from one of the following:

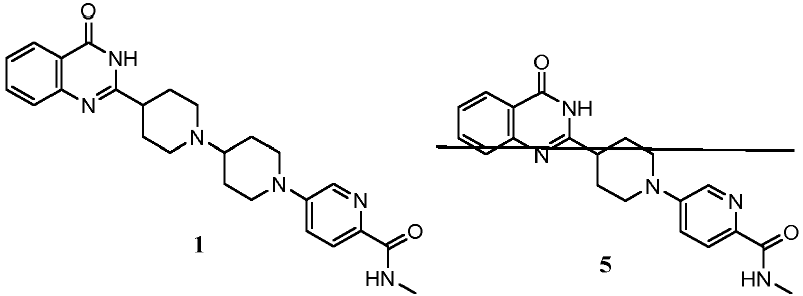

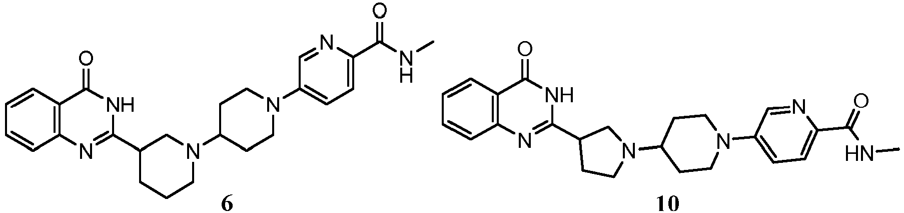

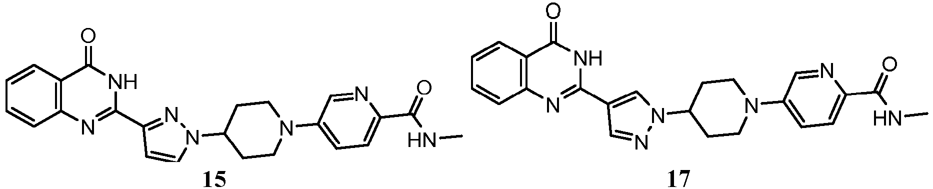

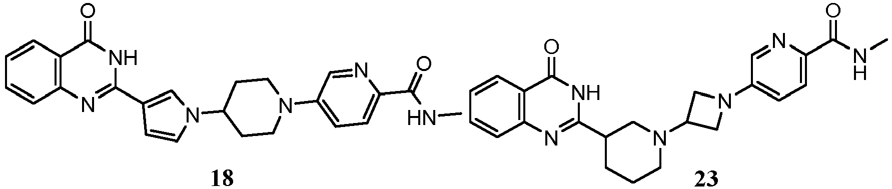

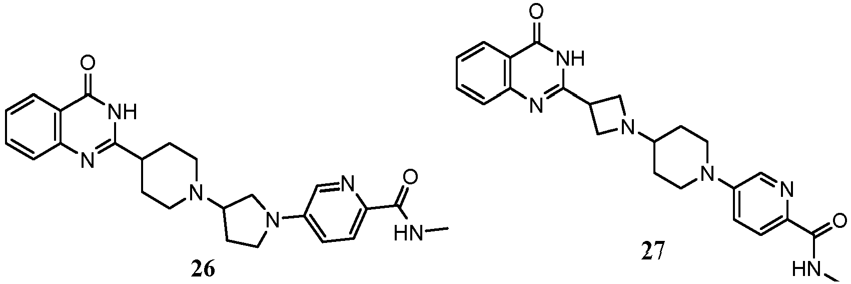

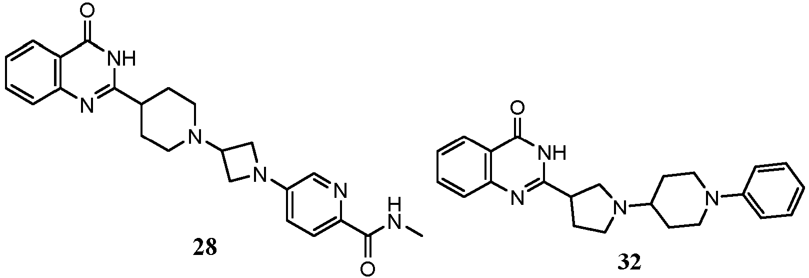

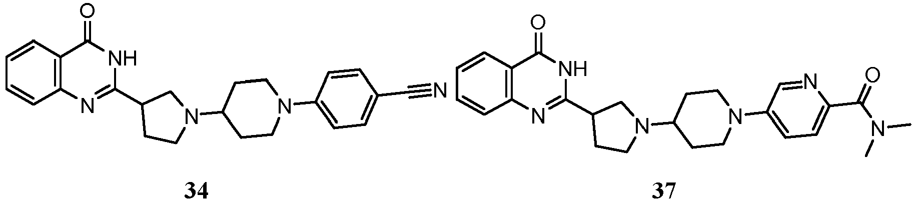

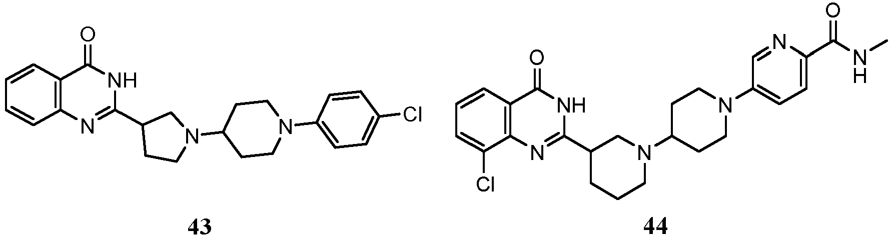

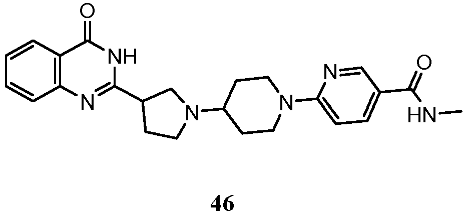

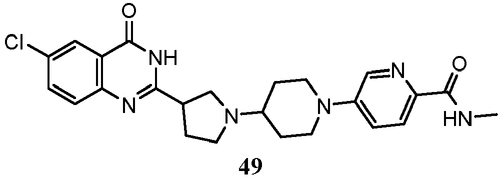

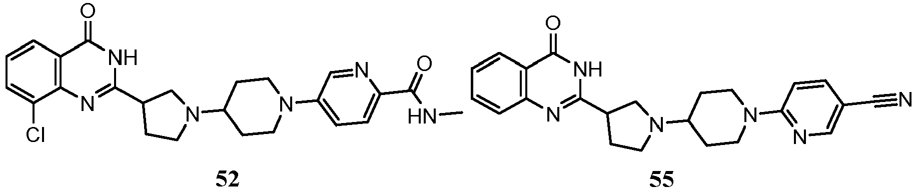

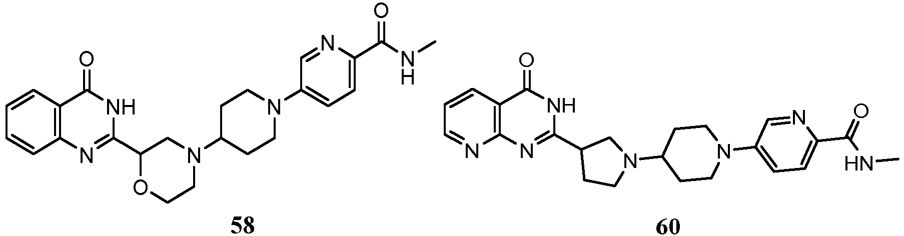

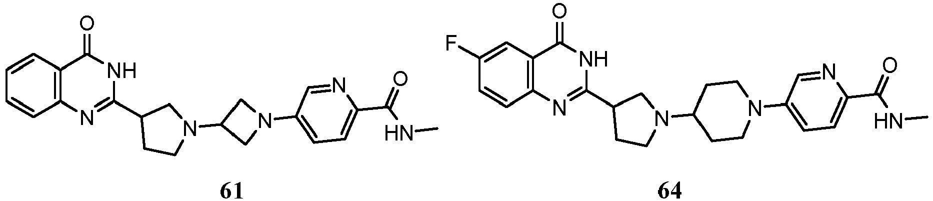

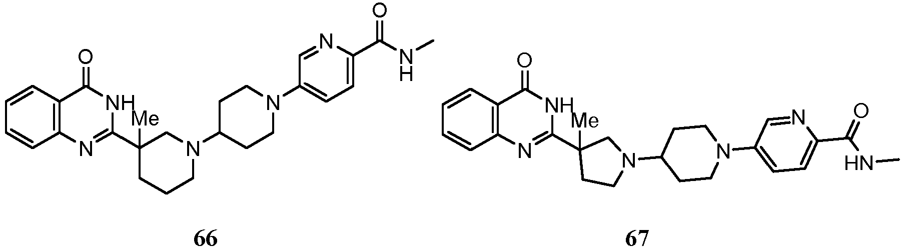

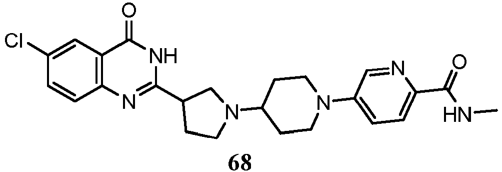

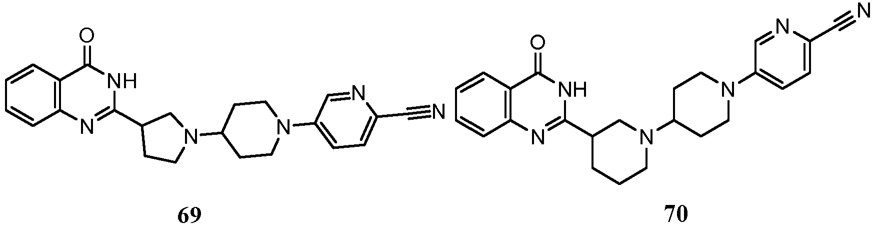

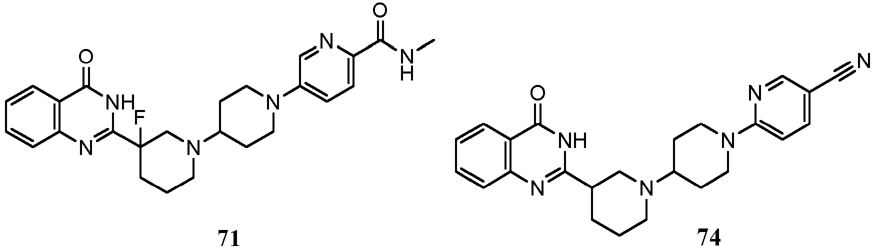

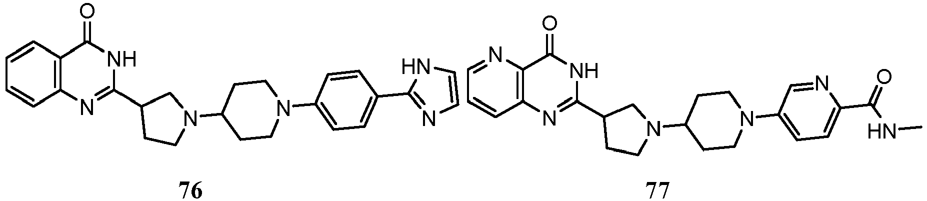

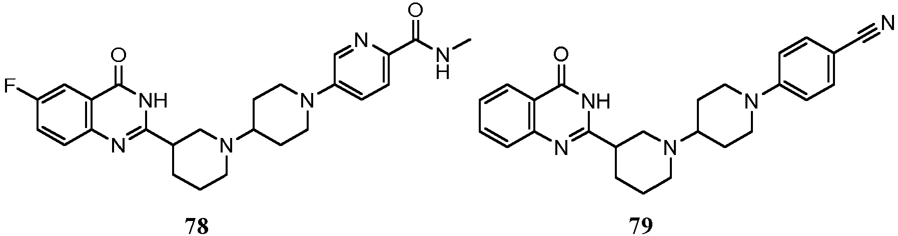

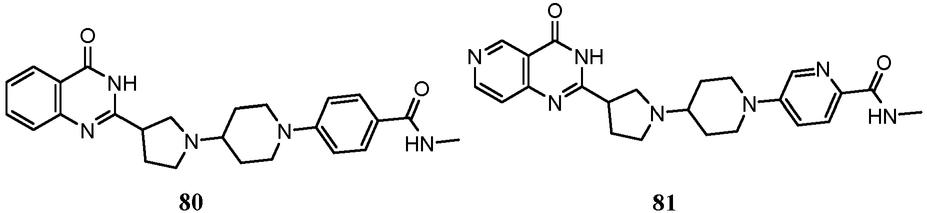

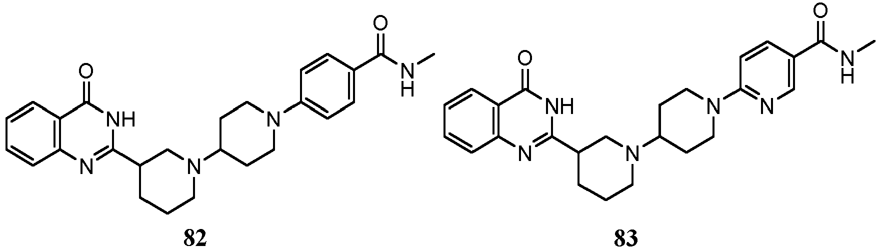

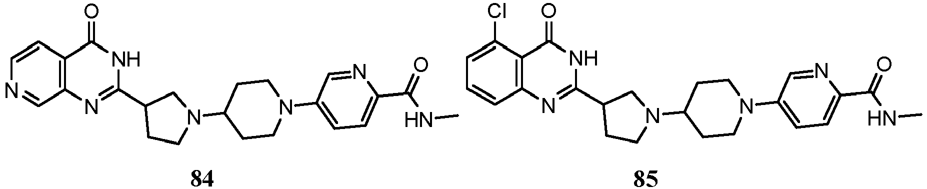

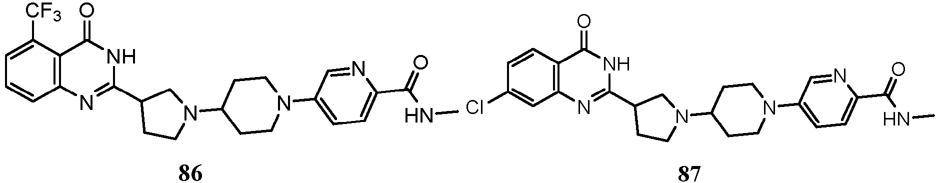

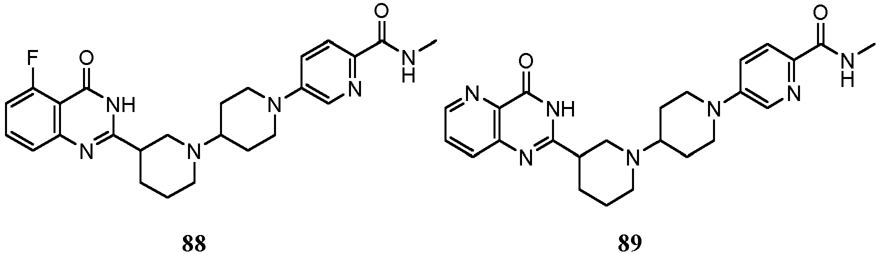

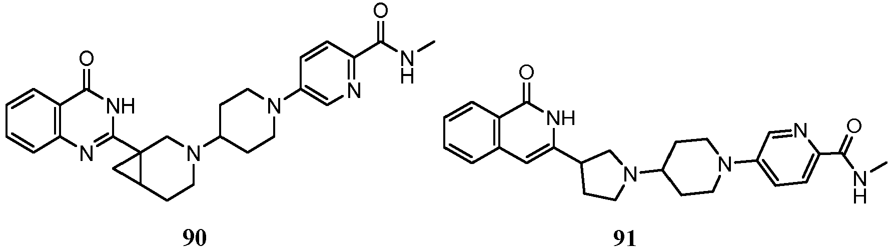

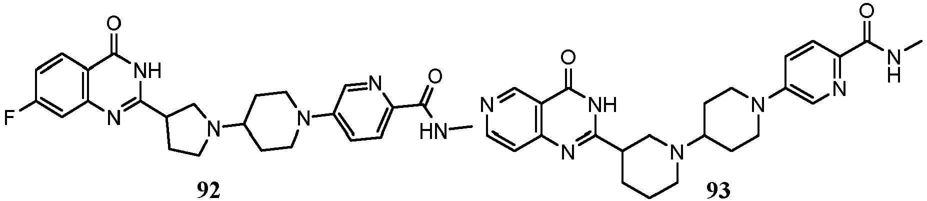

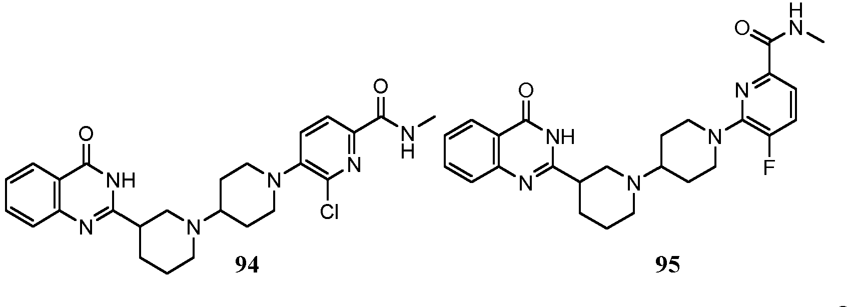

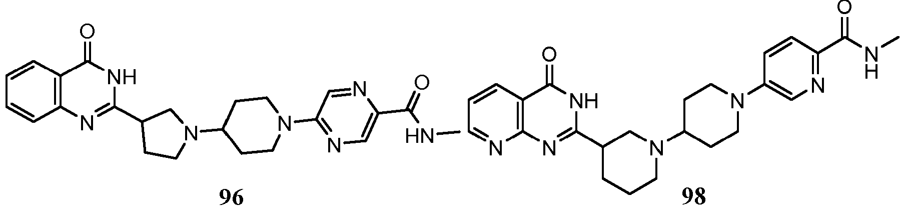

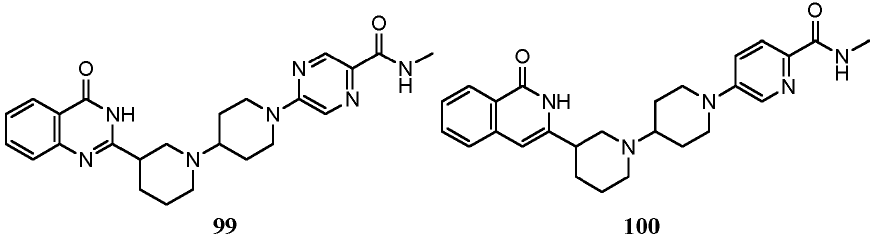

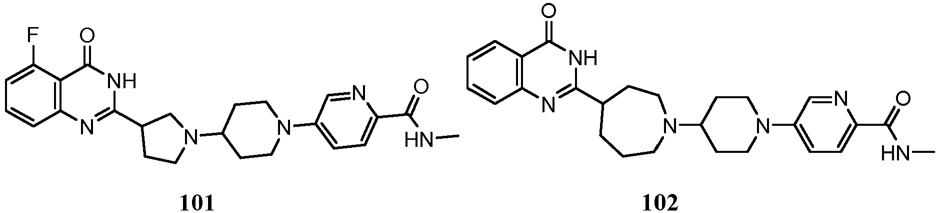

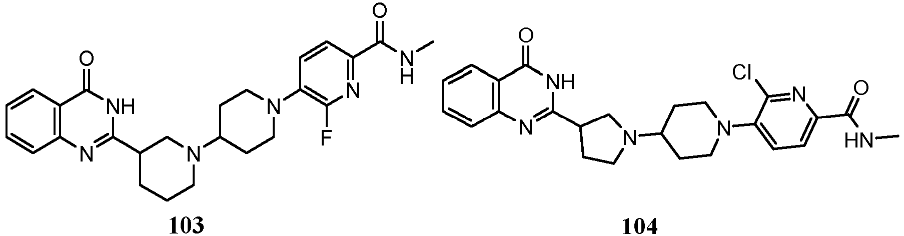

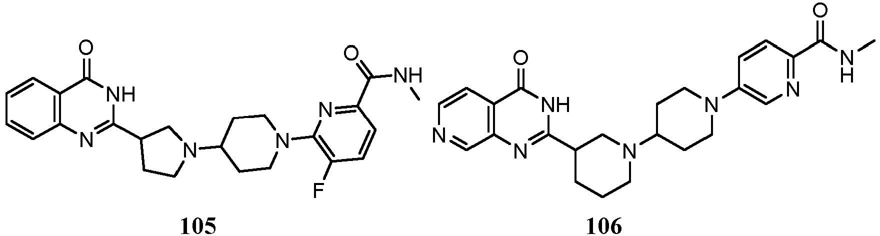

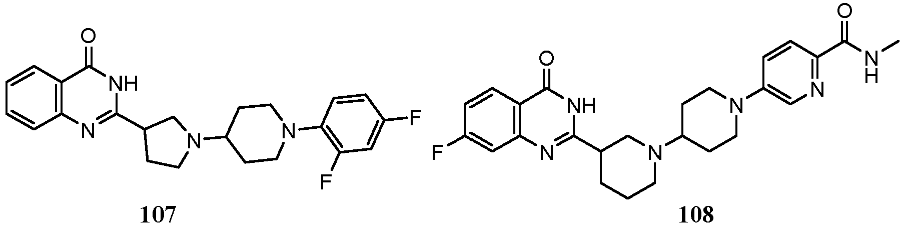

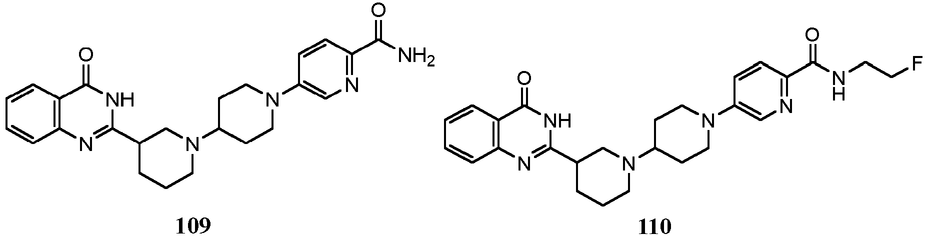

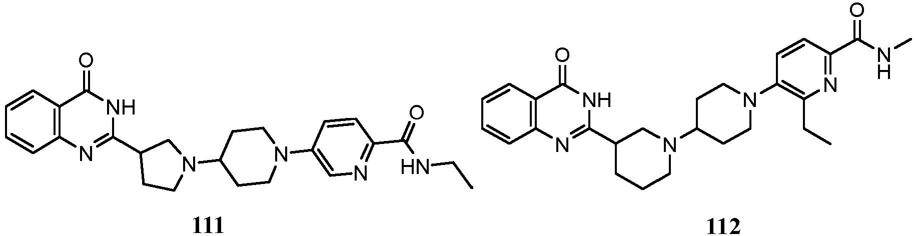

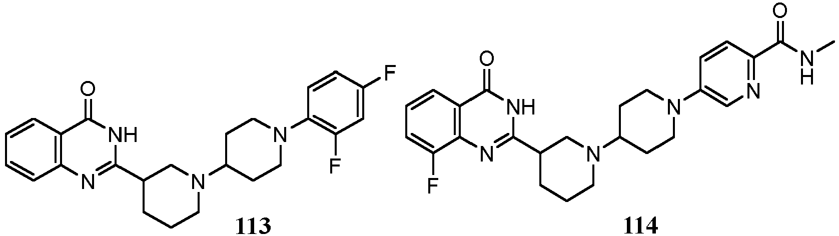

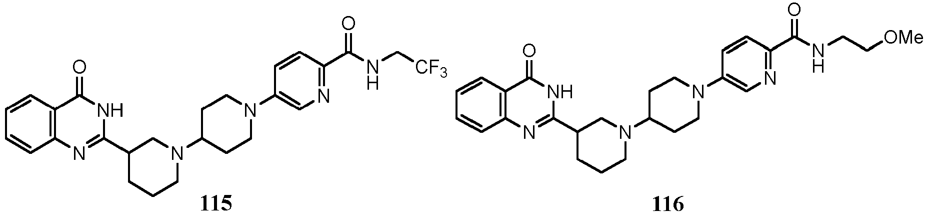

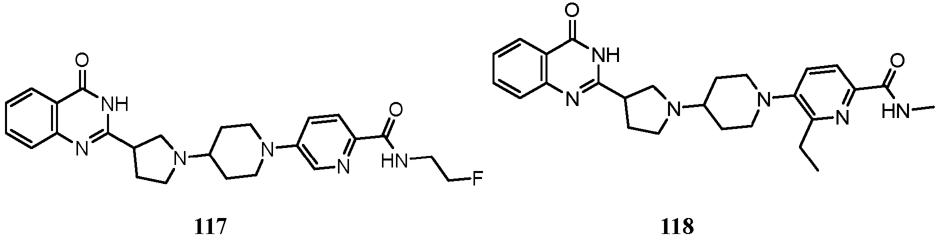

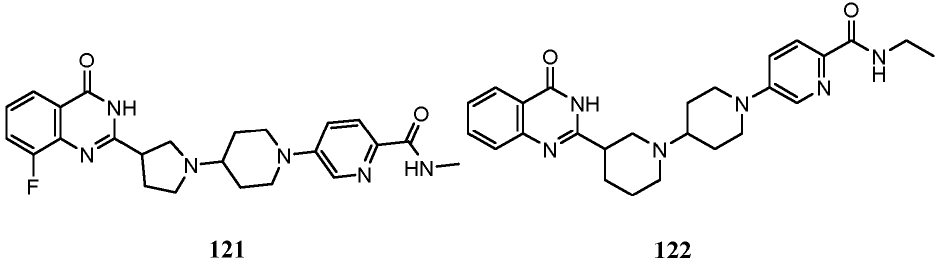

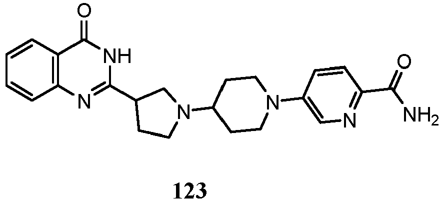

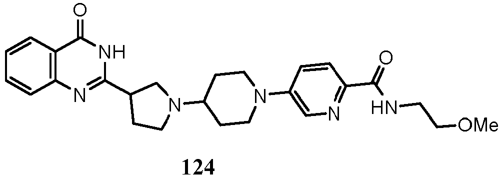

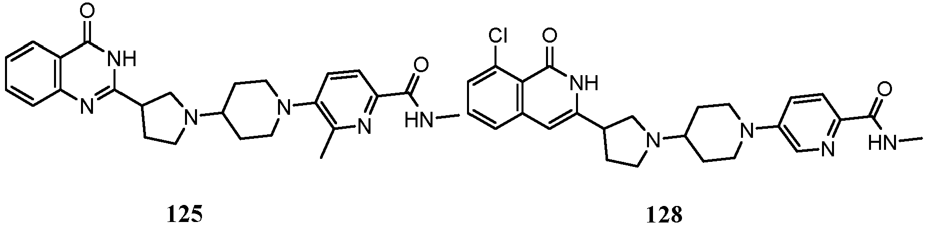

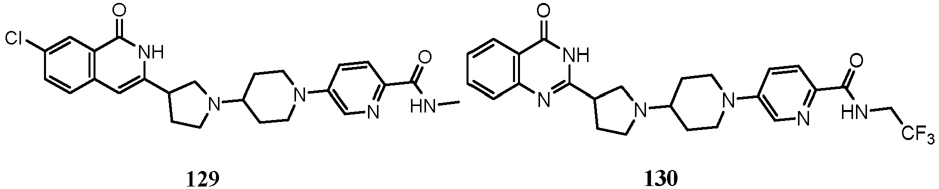

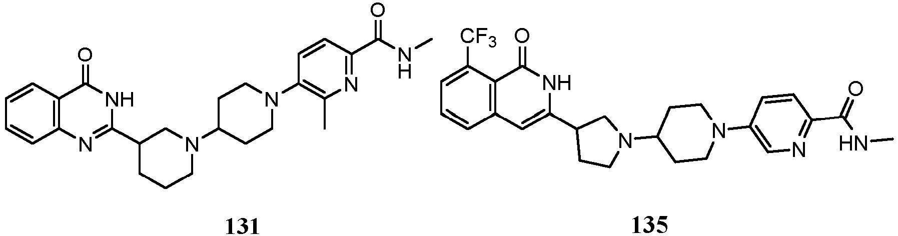

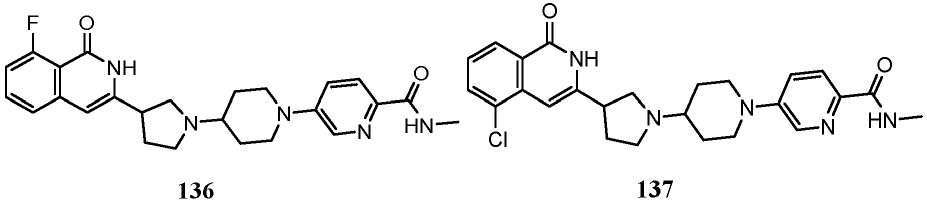

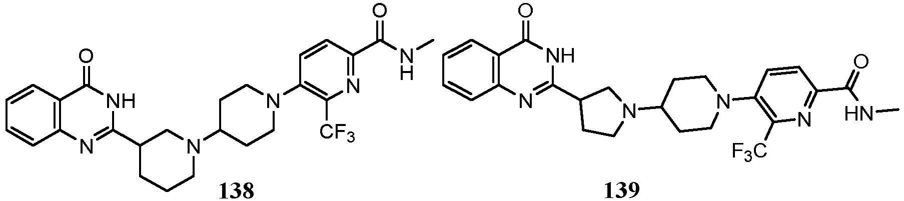

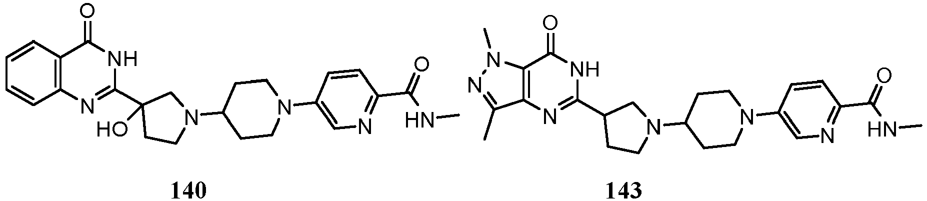

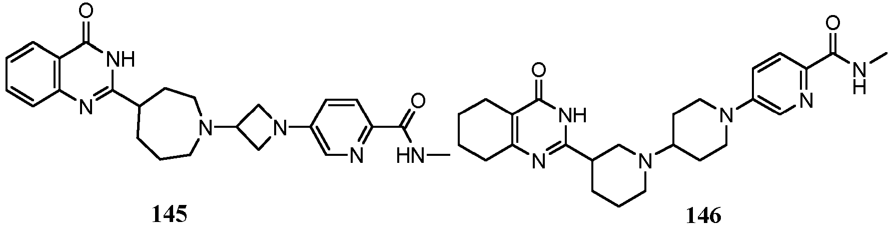

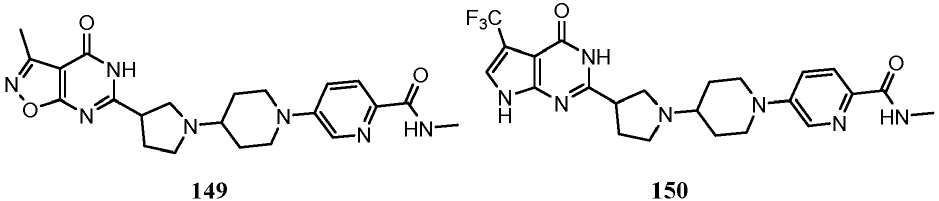

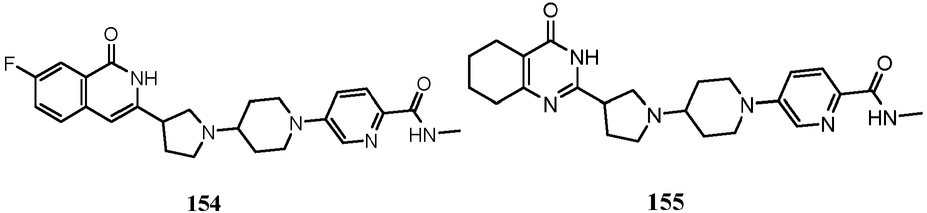

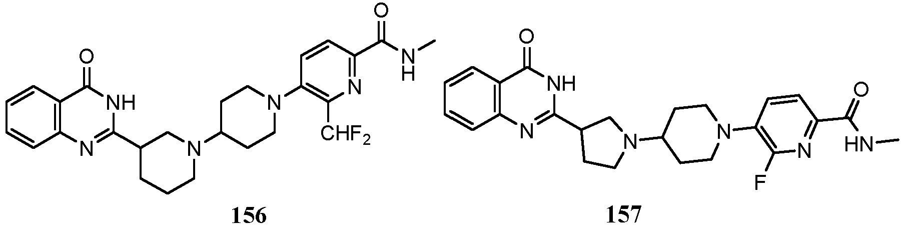

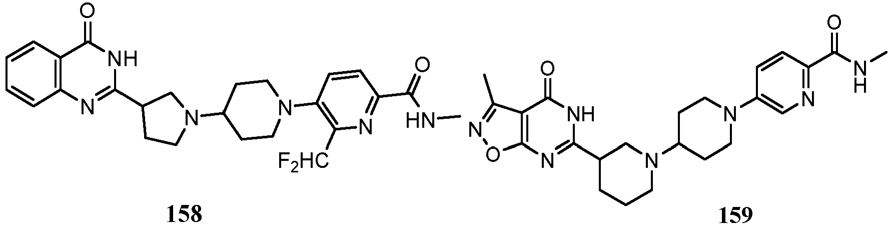

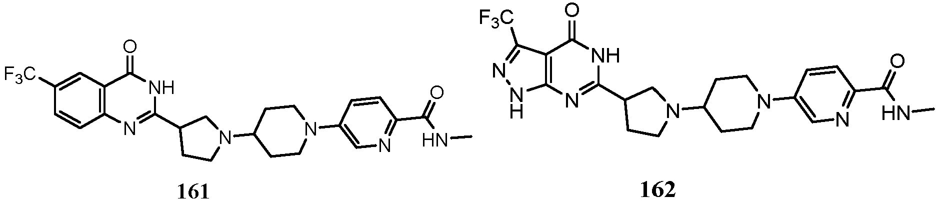

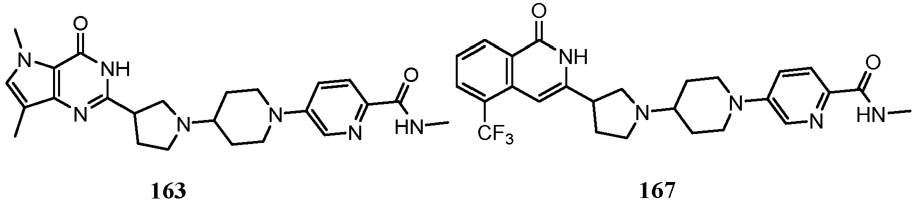

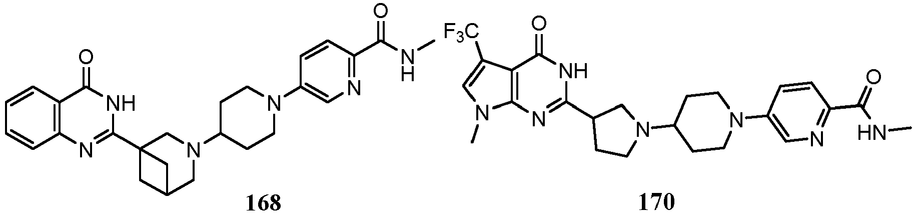

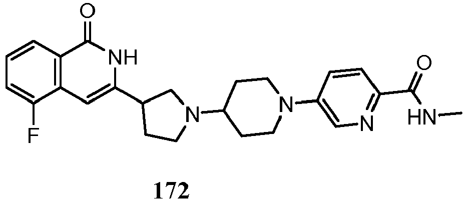

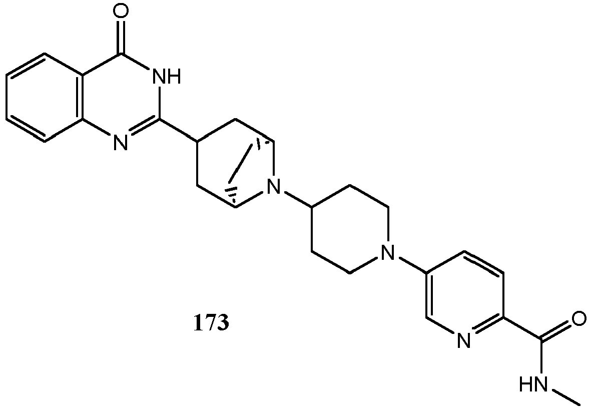

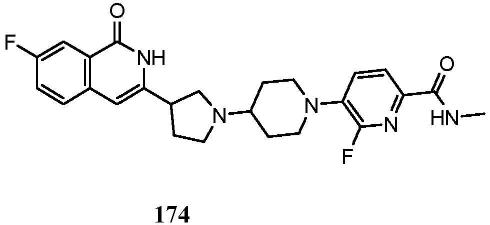

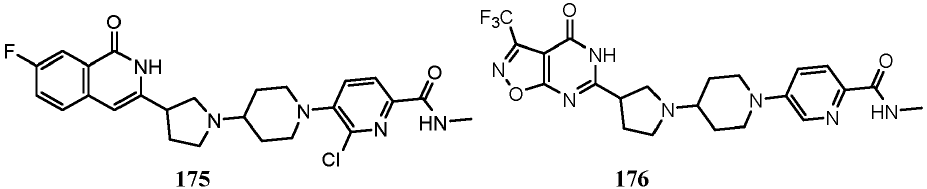

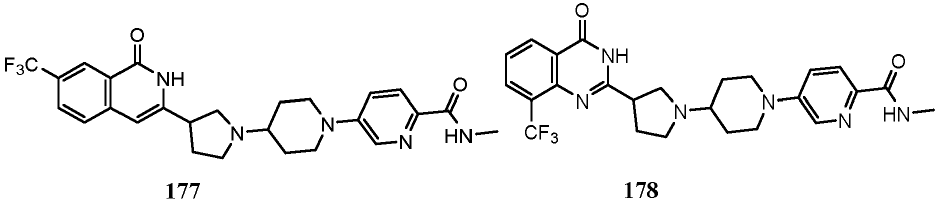

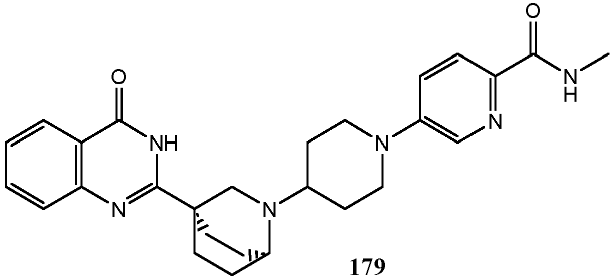

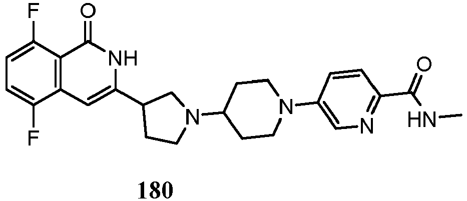

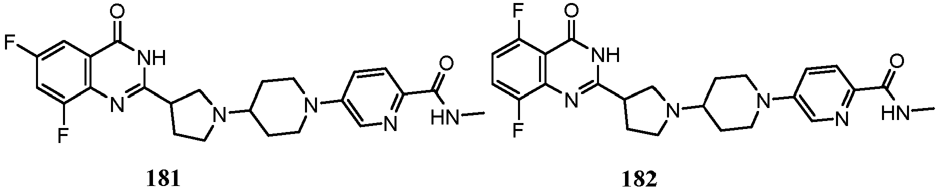

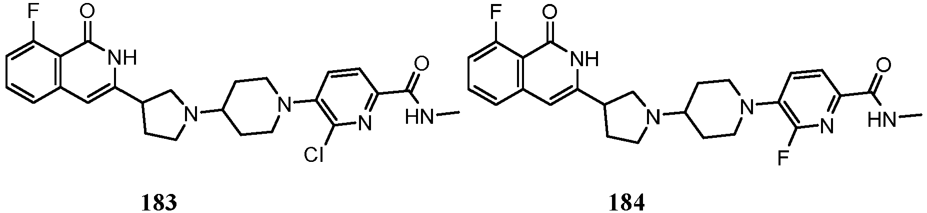

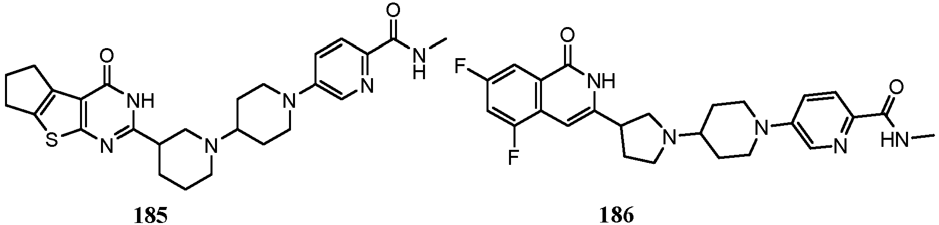

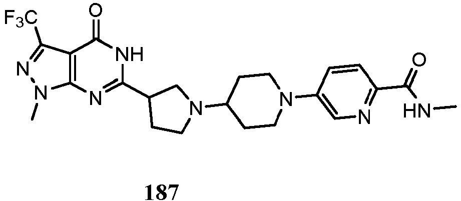

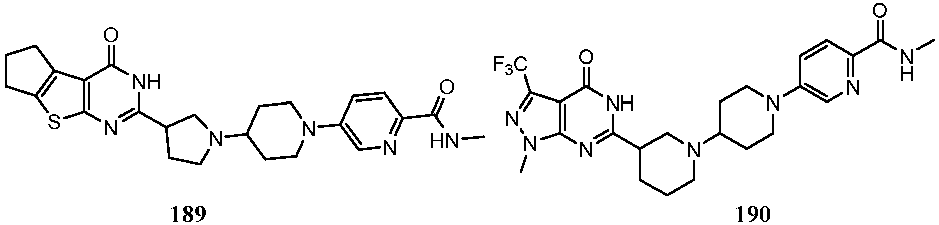

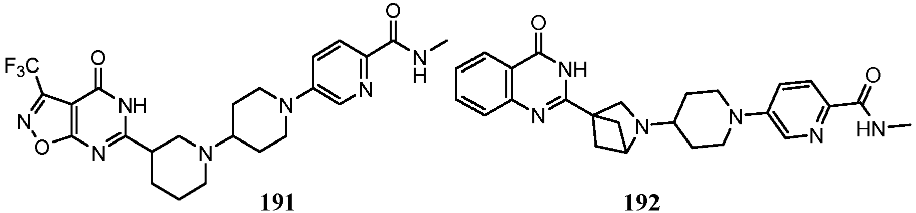

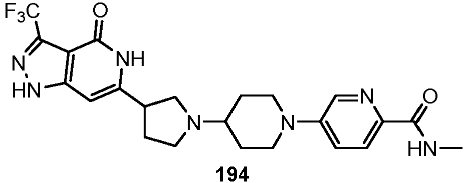

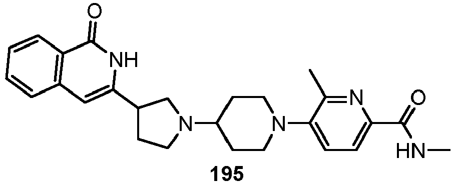

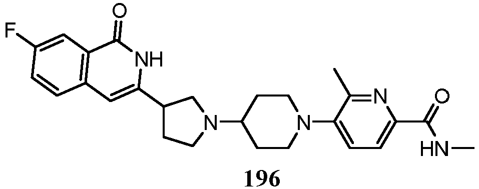

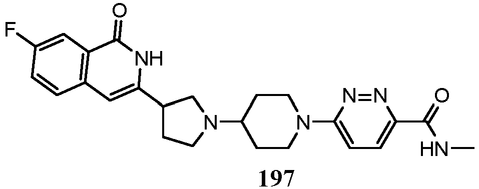

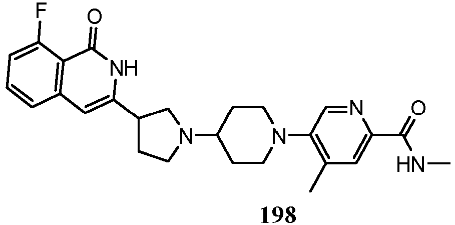

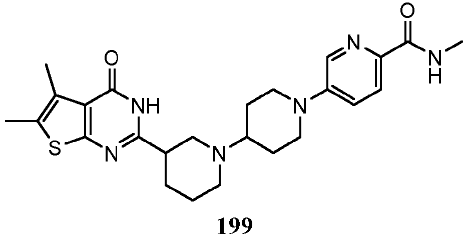

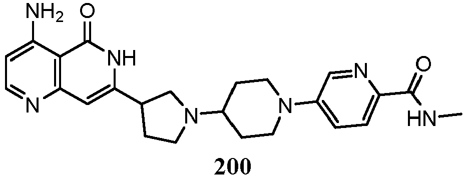

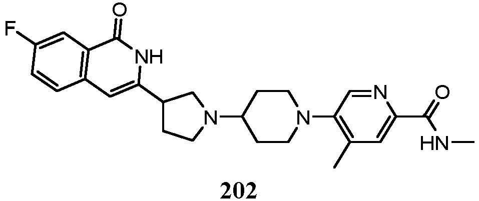

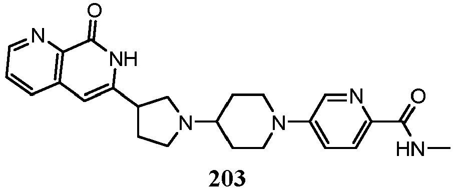

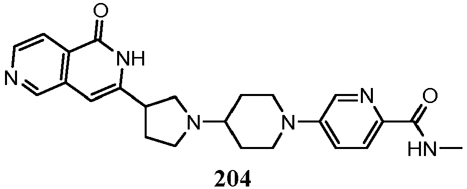

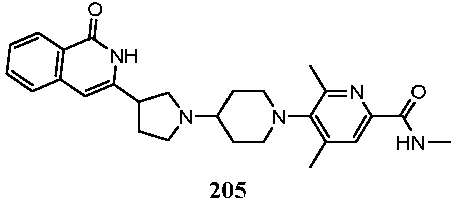

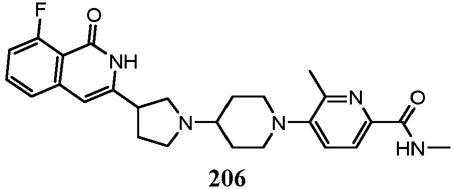

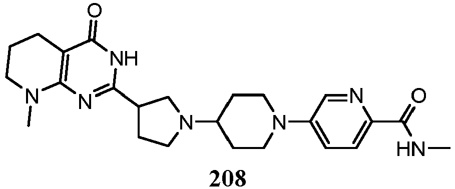

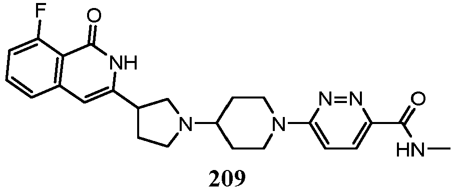

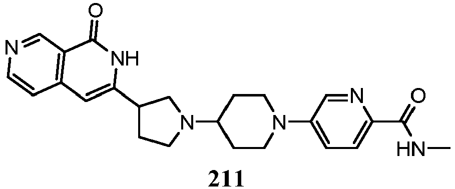

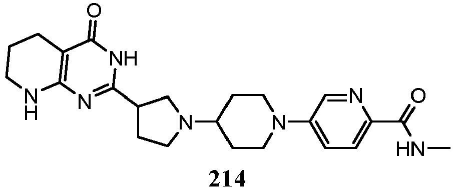

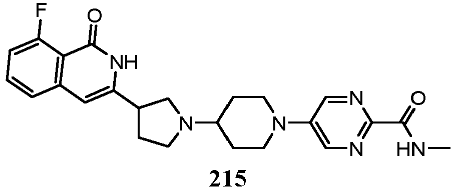

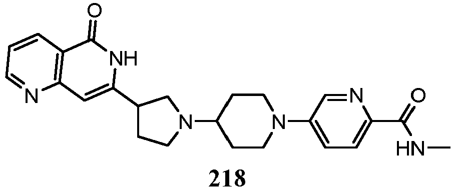

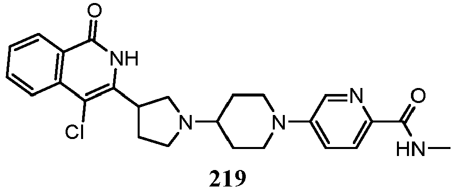

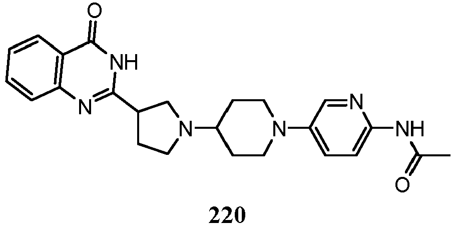

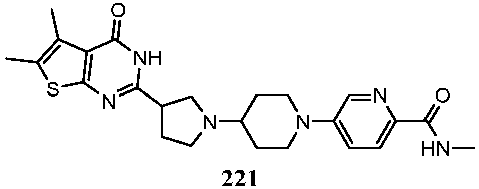

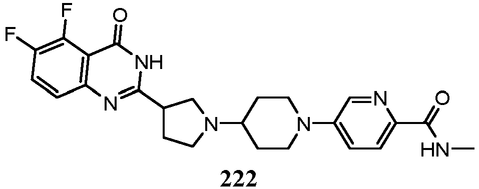

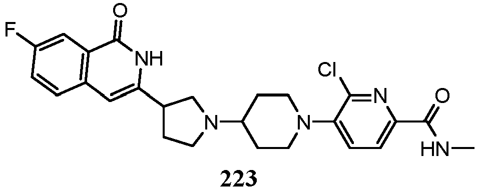

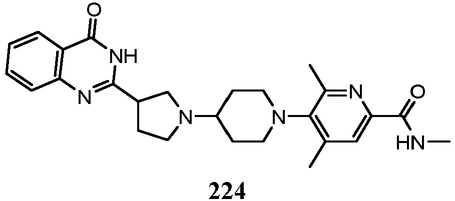

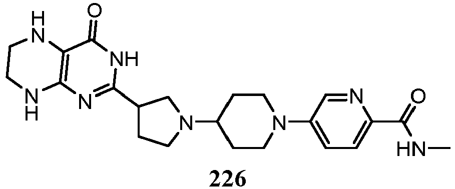

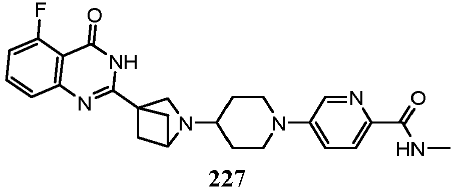

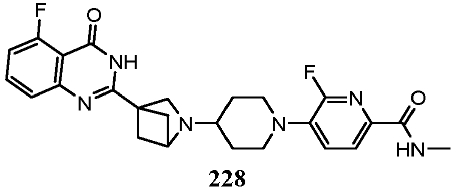

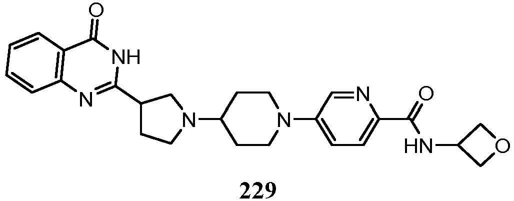

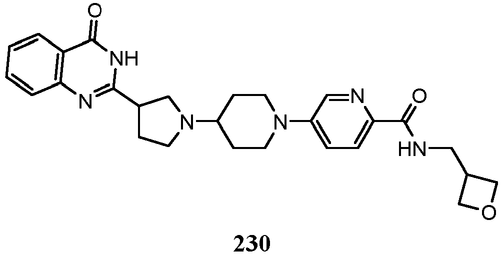

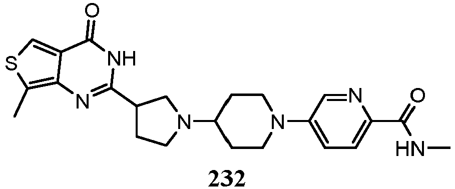

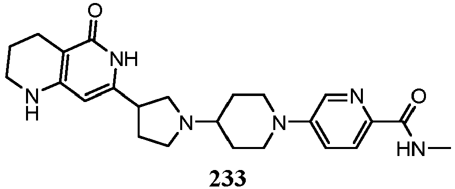

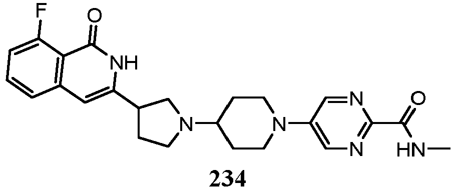

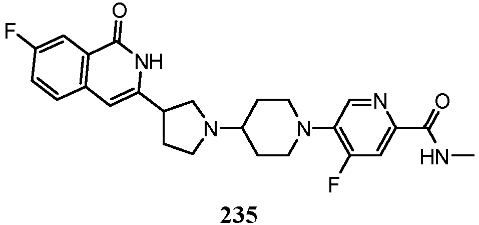

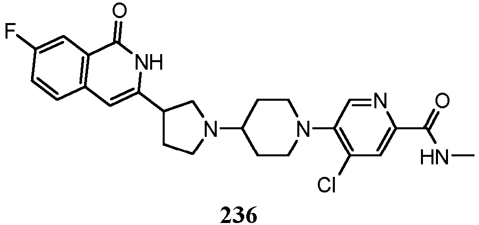

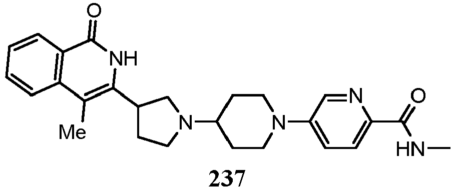

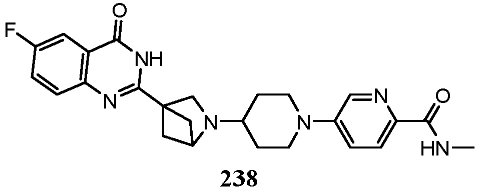

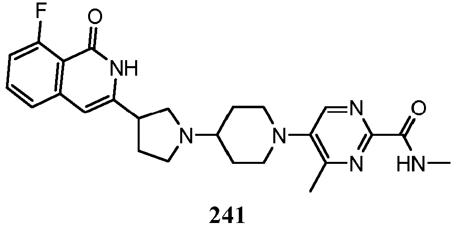

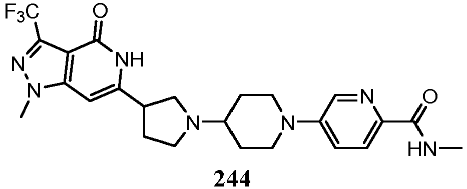

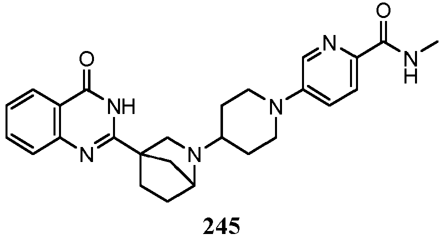

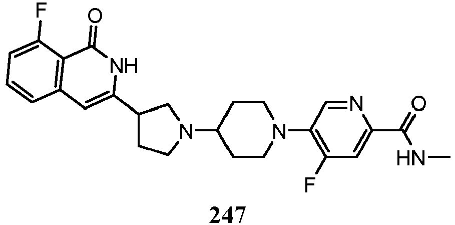

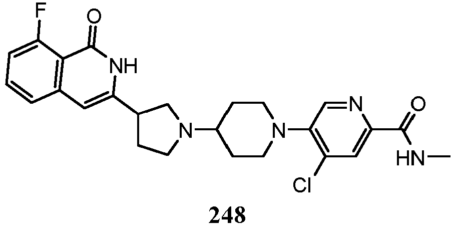

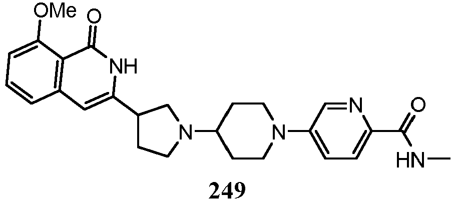

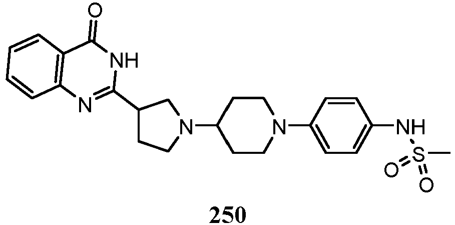

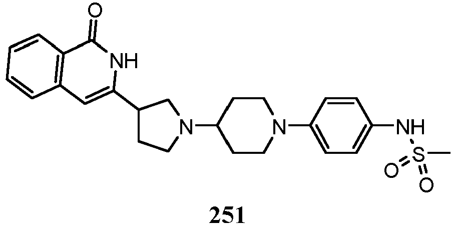

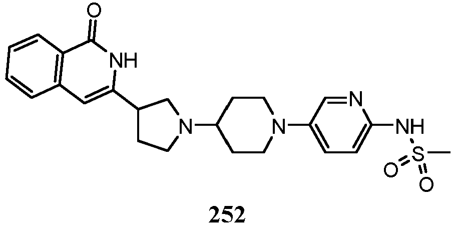

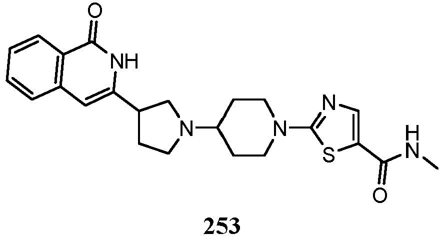

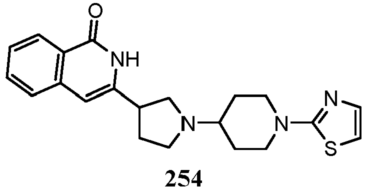

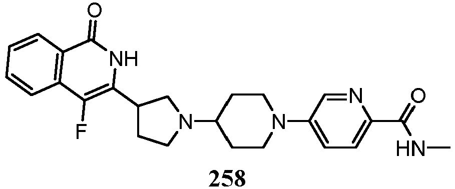

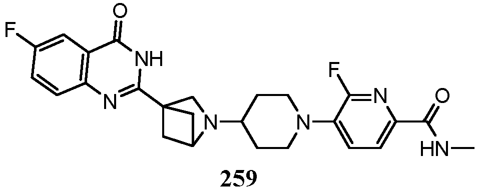

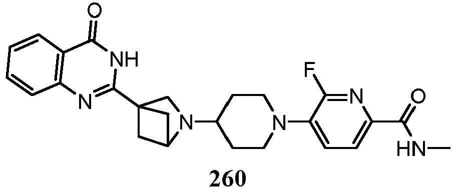

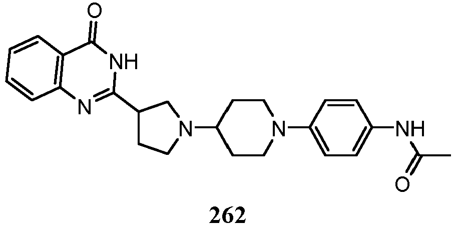

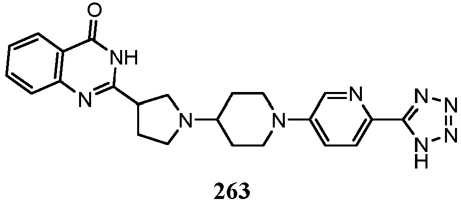

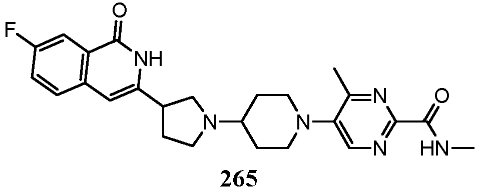

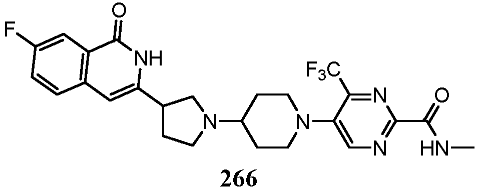

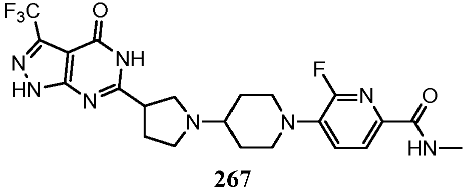

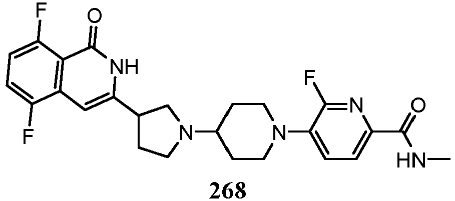

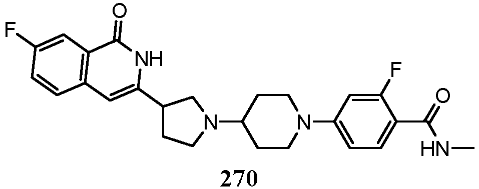

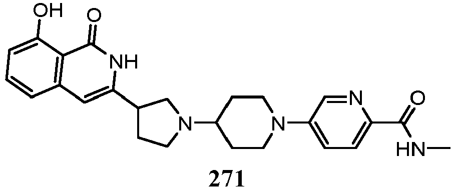

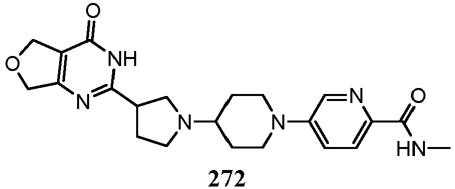

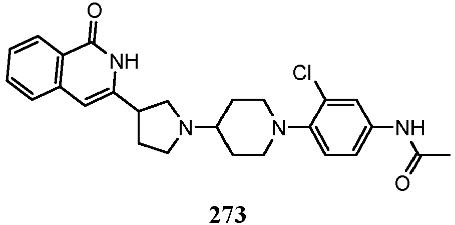

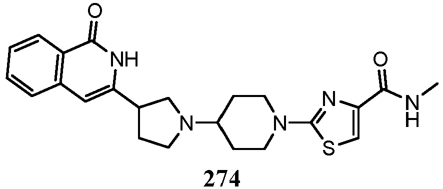

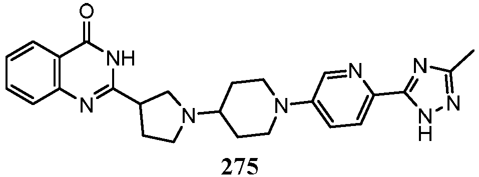

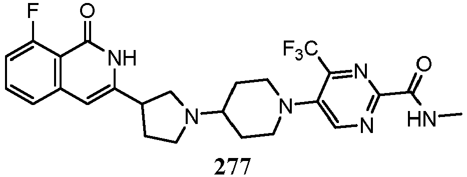

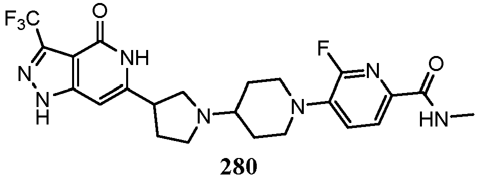

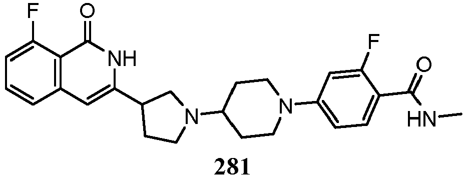

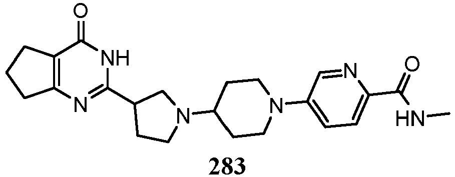

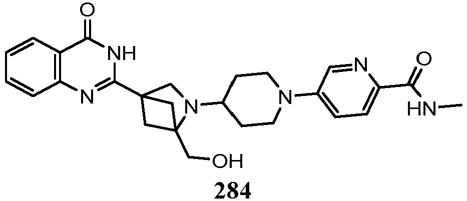

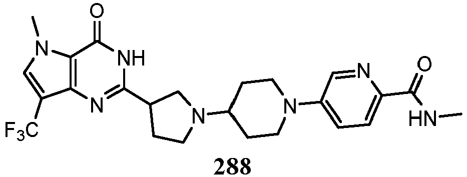

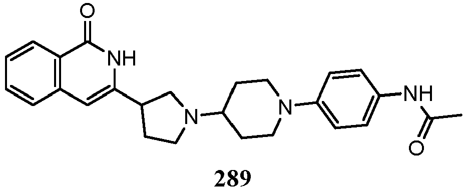

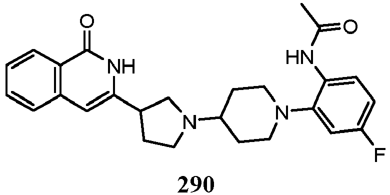

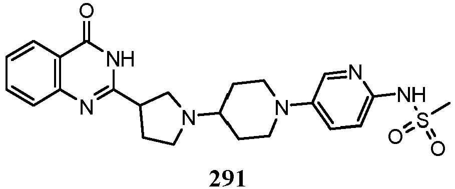

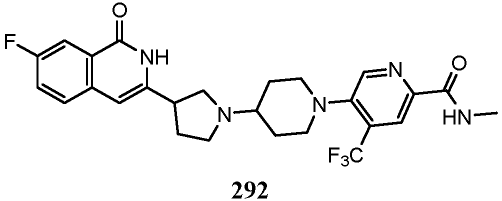

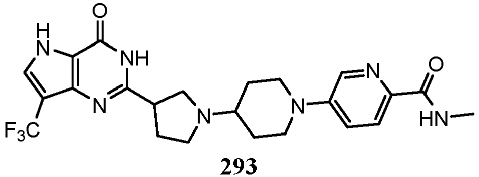

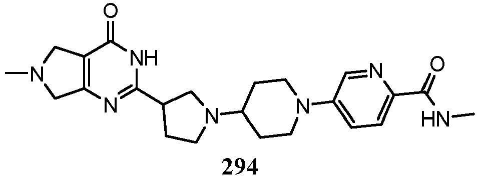

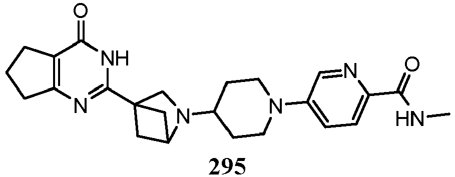

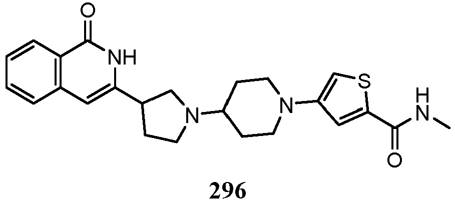

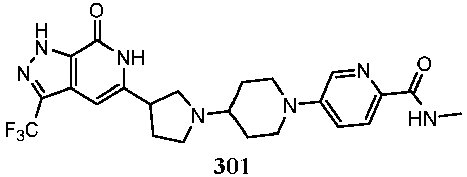

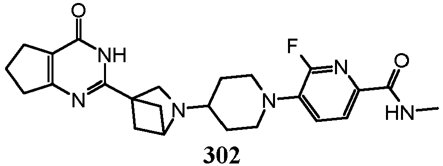

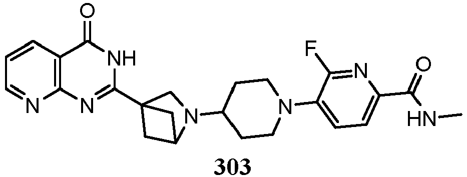

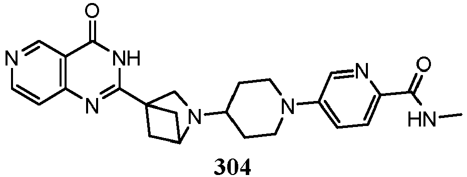

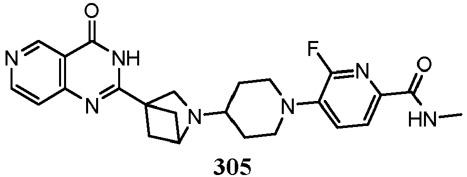

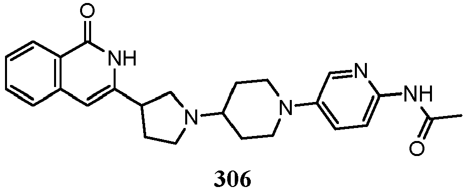

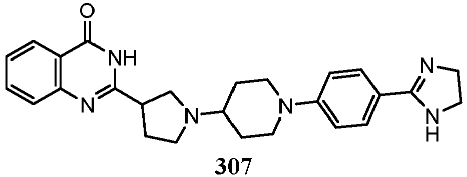

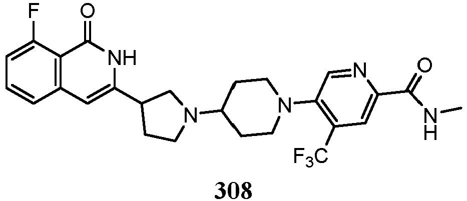

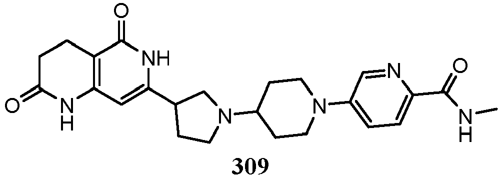

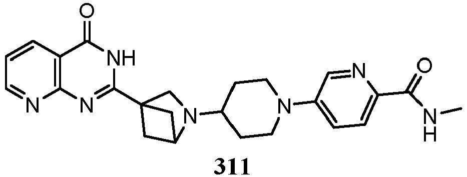

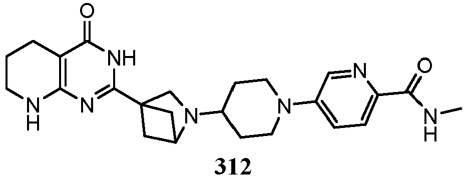

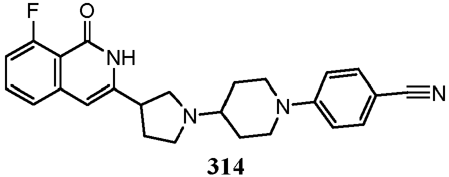

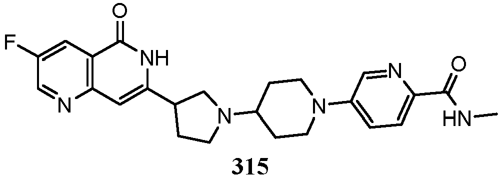

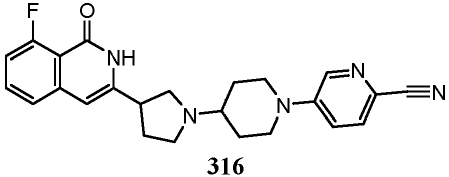

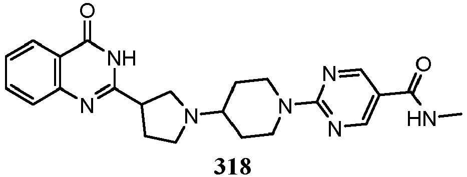

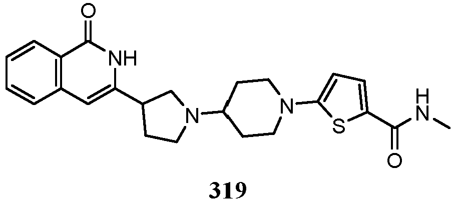

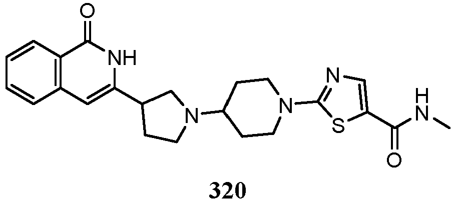

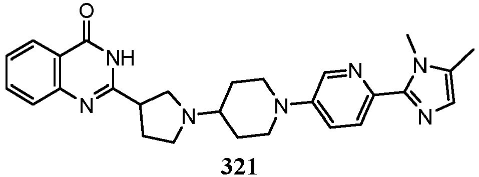

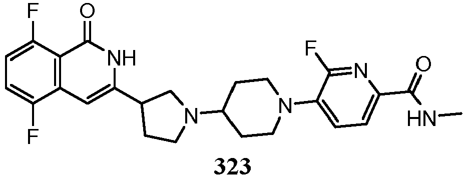

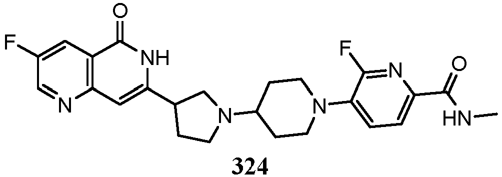

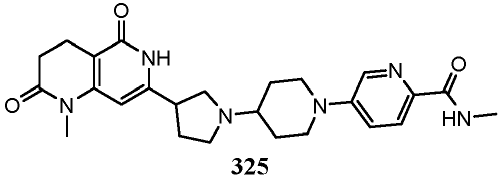

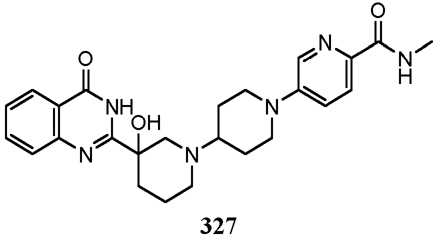

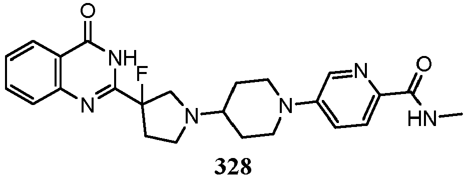

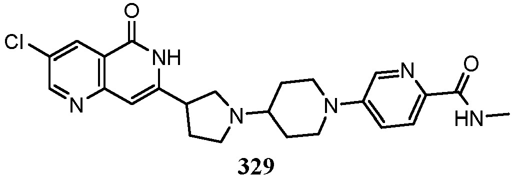

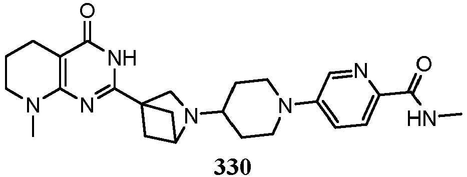

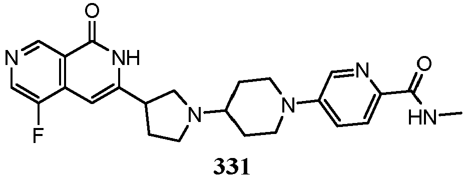

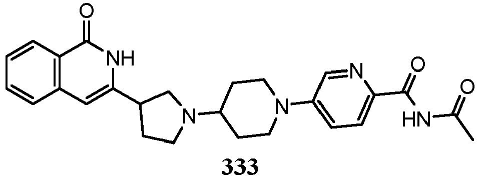

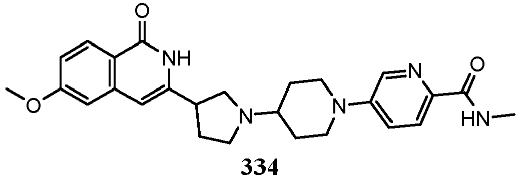

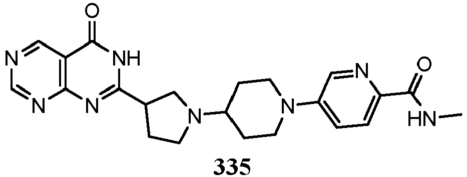

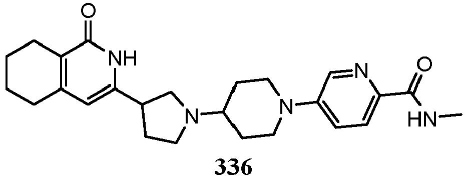

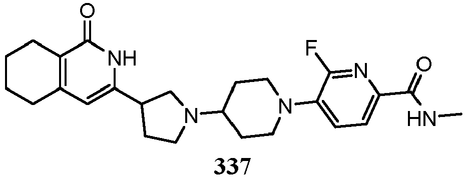

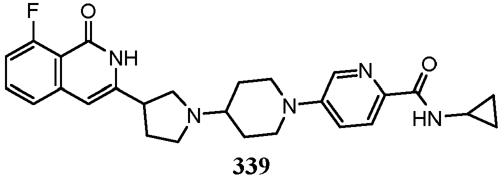

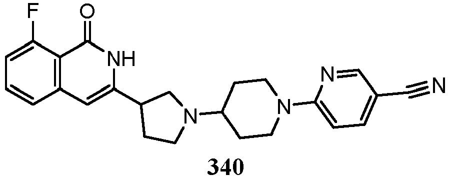

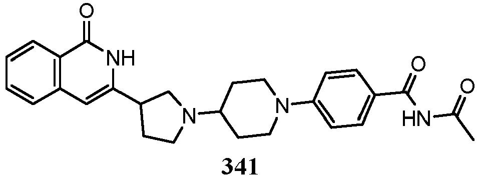

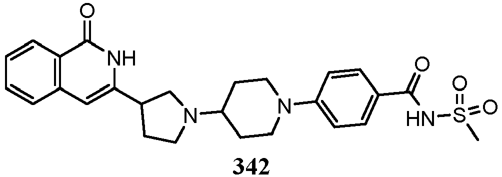

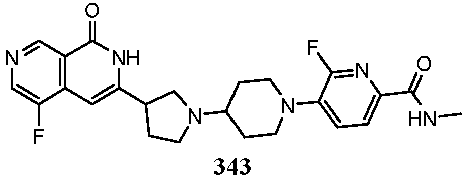

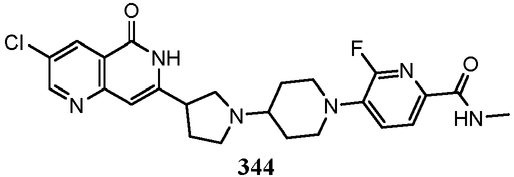

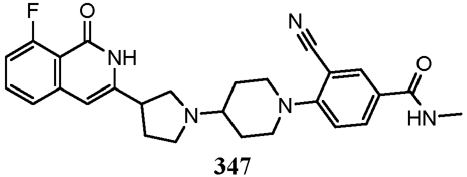

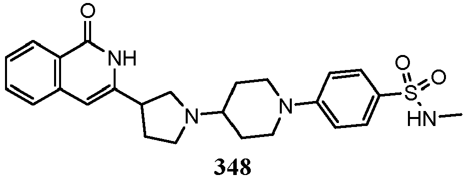

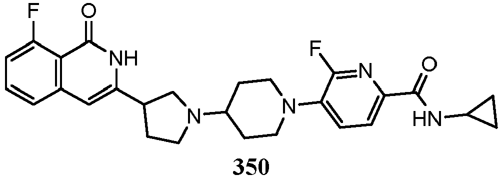

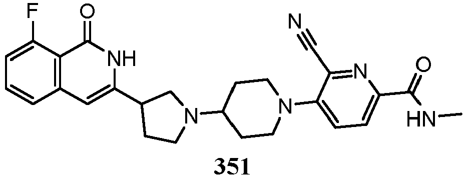

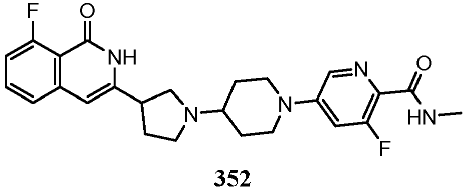

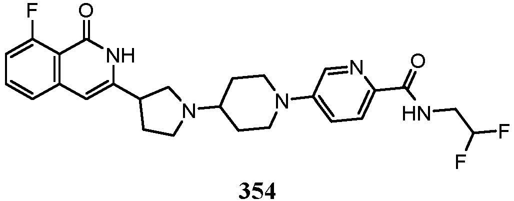

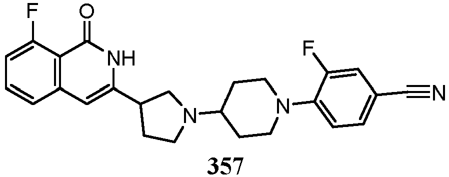

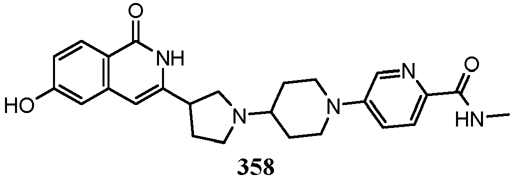

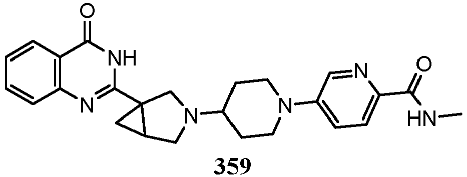

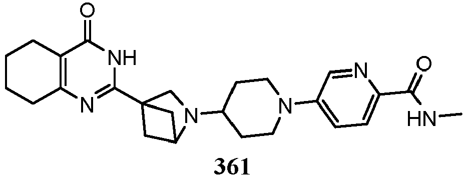

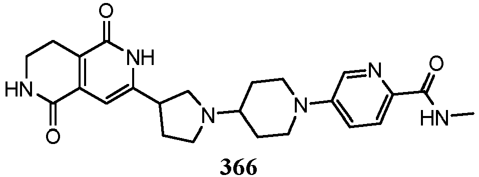

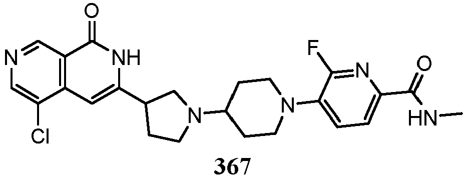

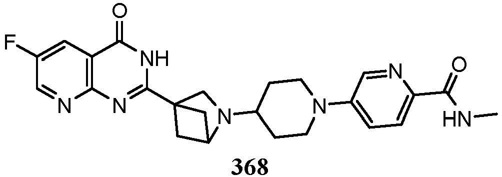

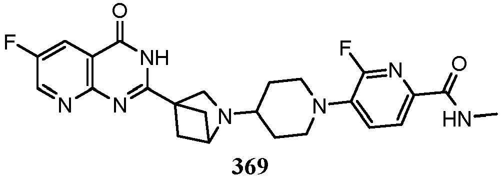

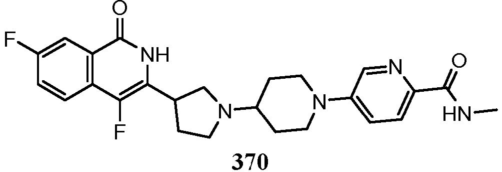

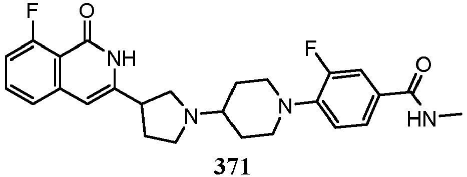

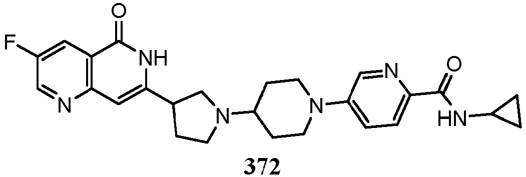

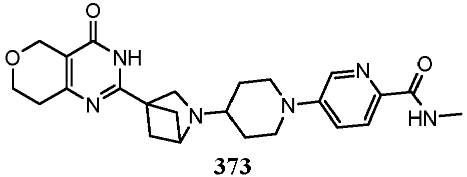

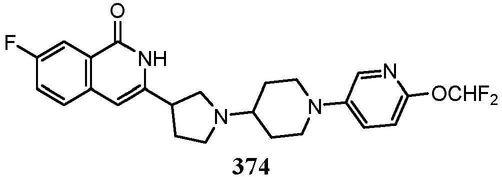

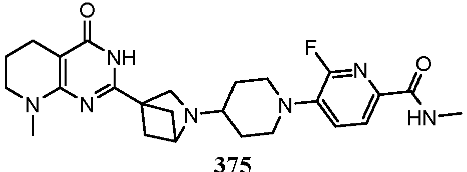

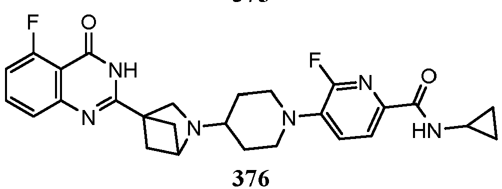

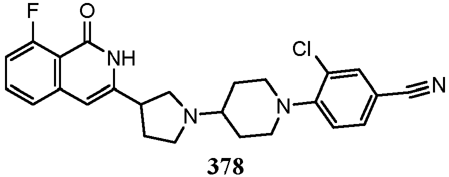

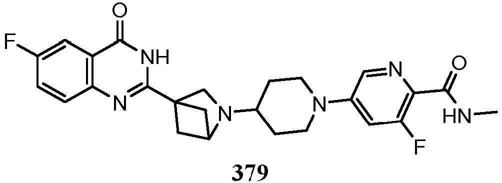

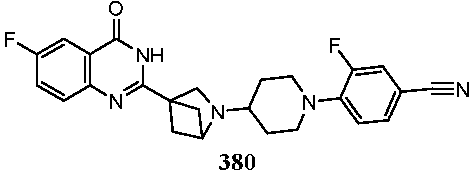

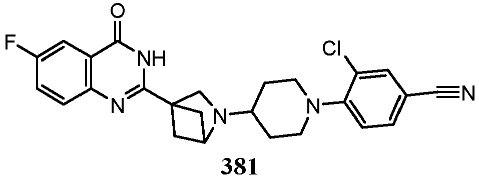

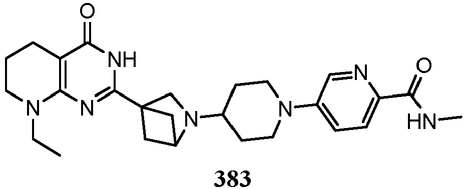

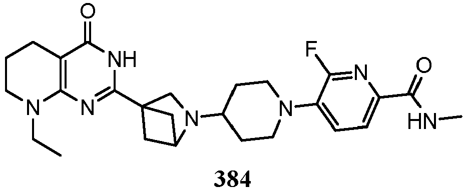

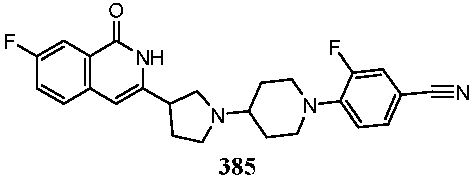

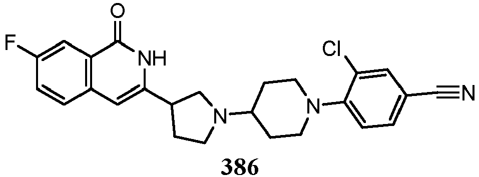

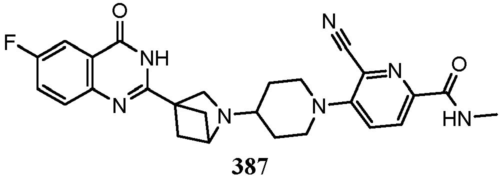

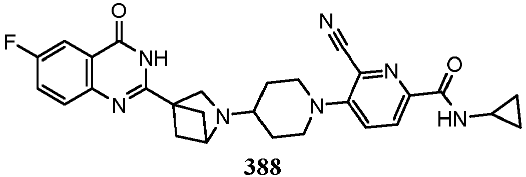

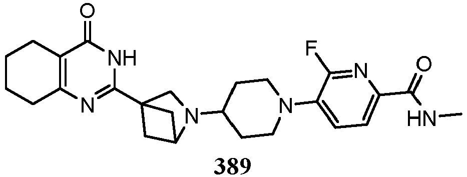

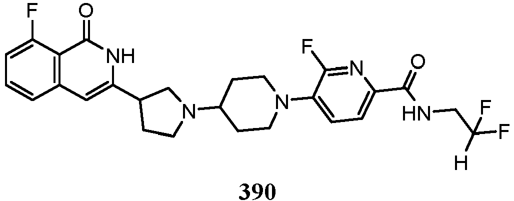

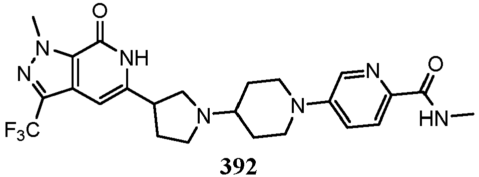

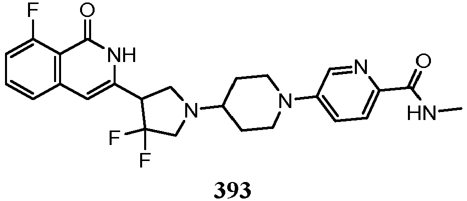

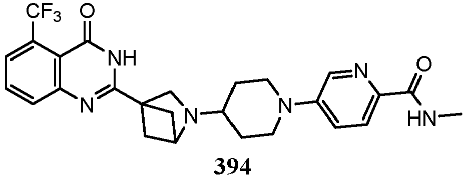

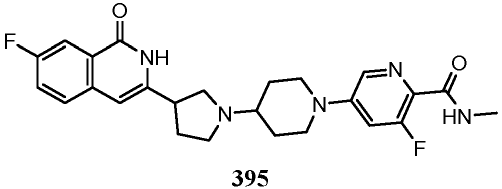

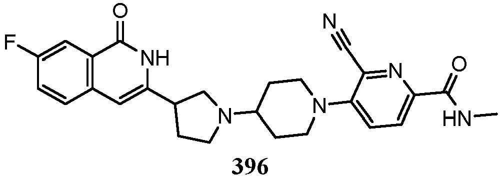

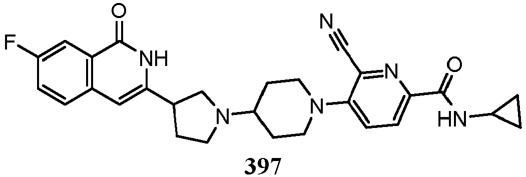

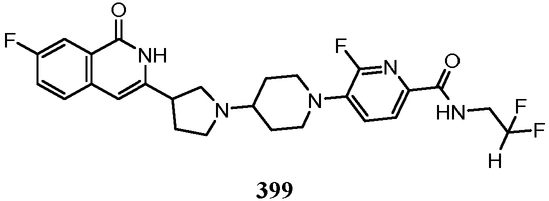

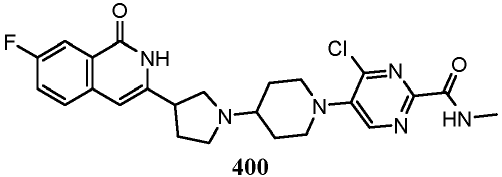

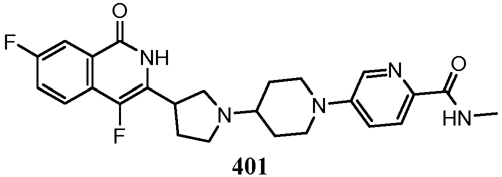

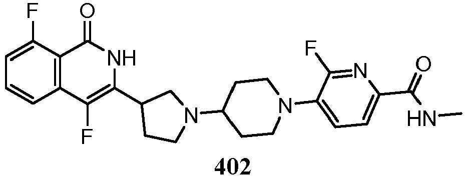

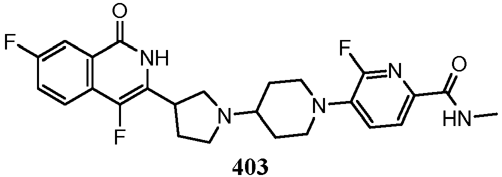

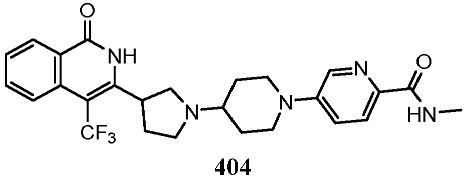

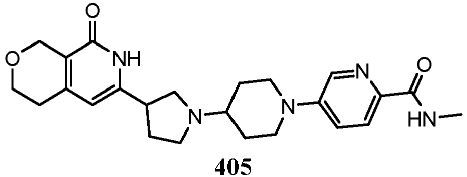

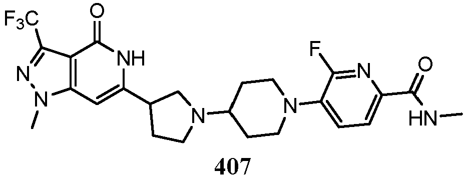

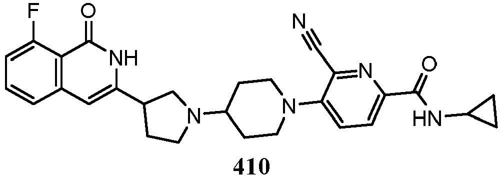

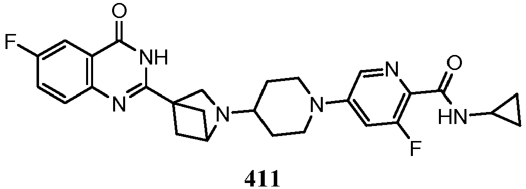

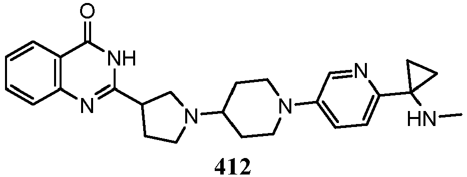

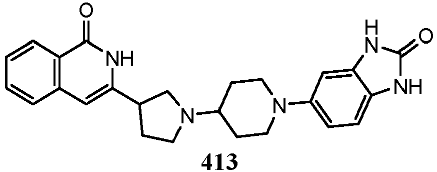

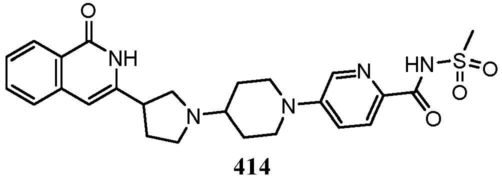

21. A compound for use according to any preceding claim, which compound comprises:
- an isolated enantiomer, or
- a mixture of two or more enantiomers, or
- a mixture of two or more diastereomers, and/or epimers, or
- a racemic mixture, or
- a tautomer of the compound.

22. A compound for use according to any preceding claim which is selective for PARP1 over PARP2.

23. A pharmaceutical composition comprising a compound as defined in any preceding claim.

24. A pharmaceutical composition according to claim 23, further comprising a pharmaceutically acceptable additive and/or excipient, and/or wherein the compound is in the form of a pharmaceutically acceptable salt, hydrate, acid, ester, or other alternative form of the compound.

25. A compound for use according to any of claims 1 to 22, or a pharmaceutical composition according to claim 22 or claim 23, which is for use in treating a cancer.

26. A compound or pharmaceutical composition for use according to claim 25, wherein:
i) the cancer is a cancer selected from: a solid or liquid tumour including cancer of the eye, brain, spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer, an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes , leukaemias and lymphomas, nasopharyngeal and gastrointestinal cancers;
and/or
ii) the cancer is deficient in a DNA damage response repair pathway, such as Homologous Recombination dependent DNA Double Strand Break DNA repair activity; and/or
iii) the cancer is deficient in BRCA1 and/or BRCA2 function.

27. A pharmaceutical composition for use according to claim 25 or claim 26, further comprising a further agent for treating cancer;
optionally wherein the further agent for treating cancer is selected from anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, immunotherapeutic agents, hormone-deprivation therapy, proapoptotic agents and cell cycle signalling inhibitors; further optionally wherein the further agent is an immunotherapeutic agent selected from an anti-tumour vaccine, an oncolytic virus, an immune stimulatory antibody such as anti-CTLA4, anti-PD1, anti-PDL-1, anti-OX40, anti-41BB, anti-CD27, anti-CD40, anti-LAG3, anti-TIM3, and anti-GITR, a pattern recognition receptor agonist such as a STING, TLR-9 or RIG-I Helicase agonist, an IDO or TDO inhibitor, a novel adjuvant, a peptide, a cytokine, a chimeric antigen receptor T cell therapy, a small molecule immune modulator, tumour microenvironment modulators, and anti-angiogenic agents.

28. A pharmaceutical kit for treating a cancer, which pharmaceutical kit comprises:
a) a compound as defined in any of claims 1 to 22; and
b) a further agent for treating cancer;
wherein the compound and the further agent are suitable for administration simultaneously, sequentially or separately; and
preferably wherein the further agent for treating cancer is selected from anti-microtubule agents, platinum coordination complexes, alkylating agents, antibiotic agents, topoisomerase II inhibitors, antimetabolites, topoisomerase I inhibitors, senolytic agents, hormones and hormone analogues, signal transduction pathway inhibitors, other DNA damage repair pathway inhibitors, non-receptor tyrosine kinase angiogenesis inhibitors, antibody-drug conjugates, hormone-deprivation therapy, immunotherapeutic agents, proapoptotic agents and cell cycle signalling inhibitors;

29. A compound, which is selected from any of the compounds defined in claim 20, optionally wherein the compound comprises:
- an isolated enantiomer, or
- a mixture of two or more enantiomers, or
- a mixture of two or more diastereomers, and/or epimers, or
- a racemic mixture, or
- a tautomer of the compound.

30. A method of synthesis of a PARP1 inhibitor compound as defined in any of claims 1 to 22, which method comprises conducting a reaction between i) a first reactant comprising ring E bearing a portion of substituent group L and ii) a second reactant comprising the remainder of substituent group L, so as to form the PARP1 inhibitor compound.

31. A method according to claim 30, wherein the reaction comprises alkylation, reductive amination or amide formation so as to form group L.

## Patentansprüche

1. PARP1-Inhibitor-Verbindung zur Verwendung in der Medizin, wobei diese Verbindung eine beliebige der folgenden Strukturen umfasst: 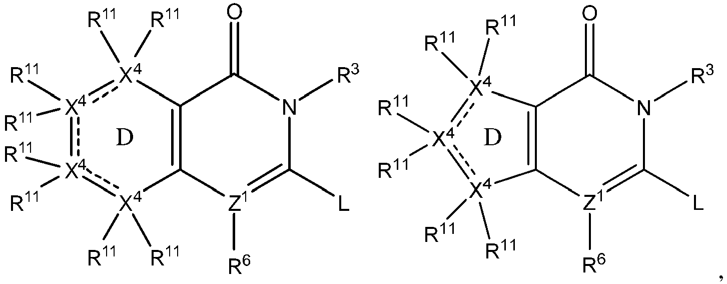 wobei:
die Strichlinien angeben, dass der Ring D Einfachbindungen oder eine Kombination von sowohl Einfach- als auch Doppelbindungen umfassen kann und aliphatisch oder aromatisch sein kann;
jedes X⁴ gleich oder verschieden sein kann und unabhängig aus C, N, O und S ausgewählt ist;
R³ H ist;
wenn Z¹ N ist, R⁶ fehlt; wenn Z¹ C ist, R⁶ aus H, Halogen, C₁- bis C₃-Alkyl, C₁- bis C₃-Haloalkyl, C₁- bis C₃-Alkohol oder C₁- bis C₃-Aminoalkyl ausgewählt ist;
jedes R¹¹ unabhängig aus H, einem Halogen, einer Nitrilgruppe, einer linearen oder verzweigten C₁-C₃-Alkylgruppe, einer linearen oder verzweigten halogenierten C₁-C₃-Alkylgruppe, einer -OH-Gruppe, einer linearen oder verzweigten C₁-C₃-Alkoholgruppe, einer halogenierten linearen oder verzweigten C₁-C₃-Alkoholgruppe, - NH₂, einer linearen oder verzweigten primären, sekundären oder tertiären C₁-C₃-Amingruppe, einer halogenierten linearen oder verzweigten primären, sekundären oder tertiären C₁-C₃-Amingruppe, einer linearen oder verzweigten C₁-C₃-Alkoxygruppe, einer linearen oder verzweigten halogenierten C₁-C₃-Alkoxygruppe ausgewählt ist, und/oder ein Paar von R¹¹-Gruppen, die an dasselbe Atom angelagert sind, =O bildet; und/oder wenn ein Paar von R¹¹-Gruppen, die an unterschiedliche Atome angelagert sind, zusammen einen Ring mit Ring-D-Atomen bildet, das Paar von R¹¹-Gruppen - CH₂CH₂CH₂- umfasst;
Z¹ aus C und N ausgewählt ist;
eine Gruppe L eine Gruppe mit der folgenden Struktur umfasst: 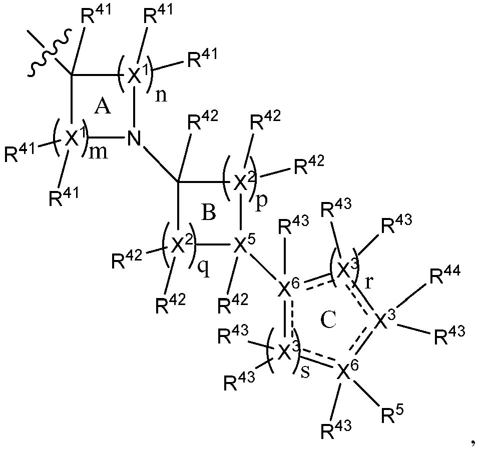
wobei:
n eine Zahl ist, die unabhängig aus 0, 1, 2, 3, 4, 5 und 6 ausgewählt ist;
m eine Zahl ist, die unabhängig aus 0, 1, 2, 3, 4, 5 und 6 ausgewählt ist;
m + n eine Zahl ist, die aus 2, 3, 4, 5 und 6 ausgewählt ist;
p eine Zahl ist, die unabhängig aus 0, 1, 2, 3, 4, 5 und 6 ausgewählt ist;
q eine Zahl ist, die unabhängig aus 0, 1, 2, 3, 4, 5 und 6 ausgewählt ist;
p + q eine Zahl ist, die aus 2, 3, 4, 5 und 6 ausgewählt ist;
r eine Zahl ist, die unabhängig aus 0, 1, 2, 3, 4 und 5 ausgewählt ist;
s eine Zahl ist, die unabhängig aus 0, 1, 2, 3, 4 und 5 ausgewählt ist;
r + s eine Zahl ist, die aus 1, 2, 3, 4 und 5 ausgewählt ist;
jedes X¹ gleich oder verschieden sein kann und unabhängig aus C, N, O und S ausgewählt ist;
jedes X² gleich oder verschieden sein kann und unabhängig aus C, N, O und S ausgewählt ist;
jedes X³ gleich oder verschieden sein kann und unabhängig aus C, N, O und S ausgewählt ist;
X⁵ unabhängig aus C und N ausgewählt ist;
jedes X⁶ gleich oder verschieden sein kann und unabhängig aus C und N ausgewählt ist;
jedes R⁴¹, R⁴², R⁴³ und R⁴⁴ gleich oder verschieden sein kann und vorliegen oder fehlen kann und aus H, Deuterium, einem Halogen, einer Nitrilgruppe, einer substituierten oder unsubstituierten C₁-C₆-Alkylgruppe, einer substituierten oder unsubstituierten linearen oder verzweigten halogenierten C₁-C₆-Alkylgruppe, einer Cyclopropylgruppe, einer -OH-Gruppe oder einer substituierten oder unsubstituierten linearen oder verzweigten C₁-C₆-Alkoholgruppe, einer substituierten oder unsubstituierten linearen oder verzweigten C₁-C₇-Aminocarbonylgruppe, einer -NH₂-Gruppe oder einer substituierten oder unsubstituierten C₁-C₆-Aminogruppe und einer substituierten oder unsubstituierten C₁-C₆-Alkoxygruppe ausgewählt ist; wobei, wenn ein Paar von R⁴¹-Gruppen, die an unterschiedliche Atome angelagert sind, zusammen einen Ring mit Ring-D-Atomen bildet und/oder ein Paar von R⁴²-Gruppen, die an unterschiedliche Atome angelagert sind, zusammen einen Ring mit Ring-B-Atomen bildet, jedes von dem Paar von R⁴¹-Gruppen und/oder dem Paar von R⁴²-Gruppen unabhängig -CH₂- oder -CH₂CH₂- umfasst;
die Strichlinien angeben, dass der Ring C Einfachbindungen oder eine Kombination von sowohl Einfach- als auch Doppelbindungen umfassen kann und aliphatisch oder aromatisch sein kann; und
R⁵ aus H und einer substituierten oder unsubstituierten organischen Gruppe ausgewählt ist.

2. Verbindung zur Verwendung nach Anspruch 1, wobei Ring A der Gruppe L eine beliebige der folgenden Strukturen umfasst:

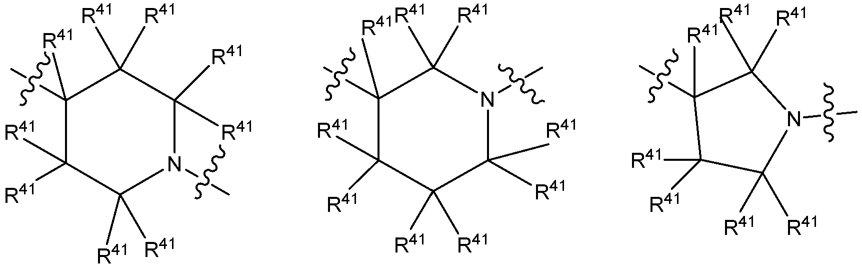

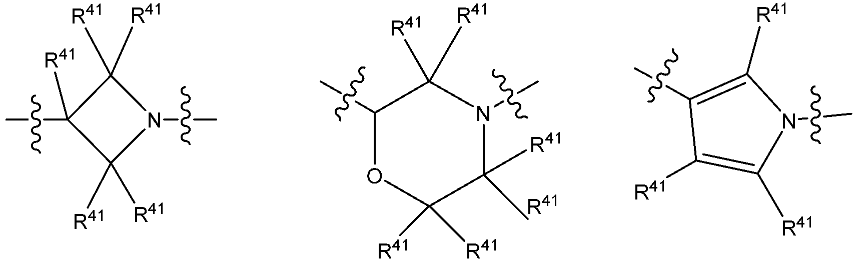

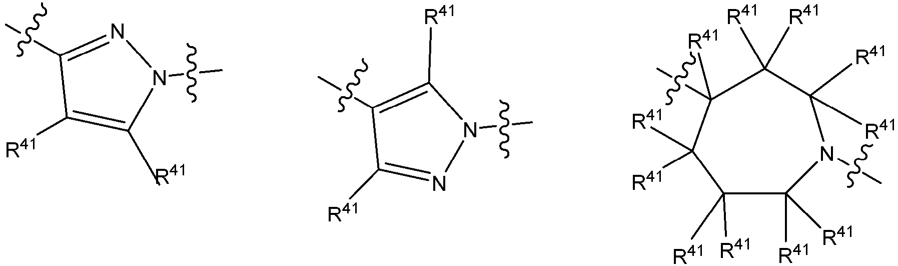

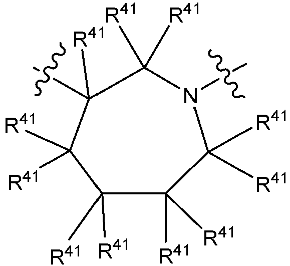

3. Verbindung zur Verwendung nach Anspruch 2, wobei Ring A der Gruppe L eine beliebige der folgenden Strukturen umfasst:

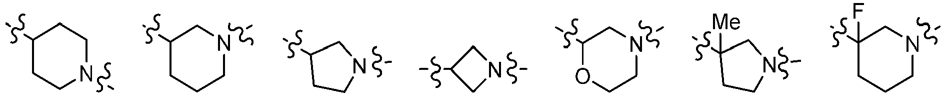

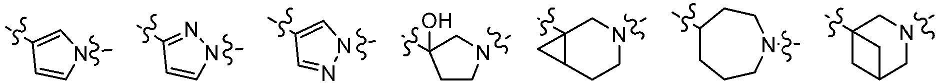

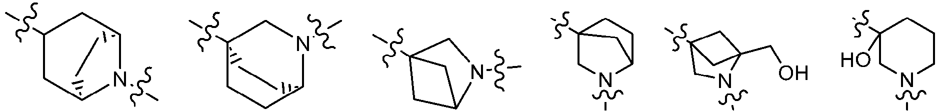

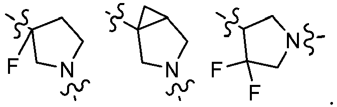

4. Verbindung zur Verwendung nach einem vorhergehenden Anspruch, wobei Ring C der Gruppe L eine beliebige der folgenden Strukturen umfasst:

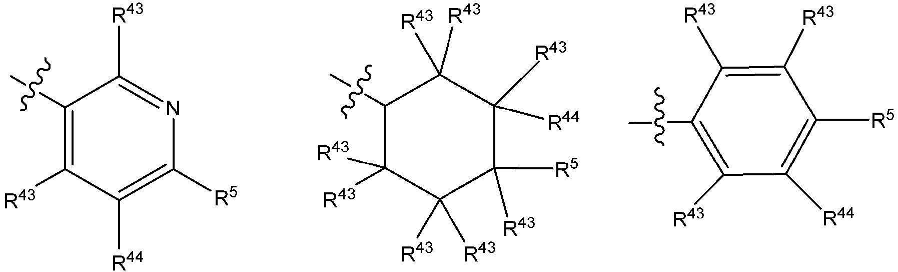

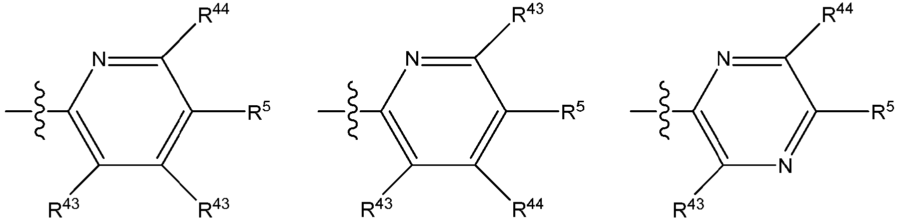

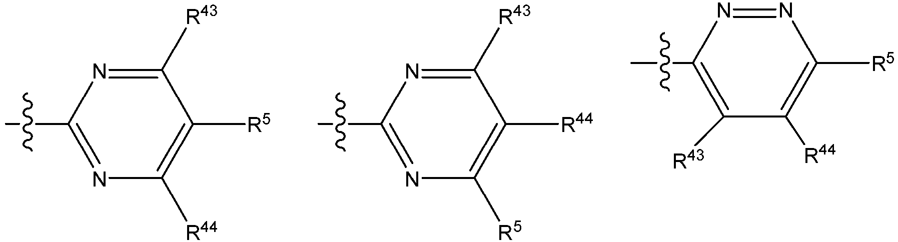

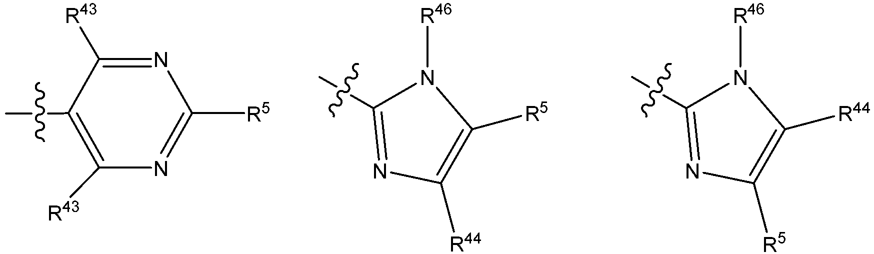

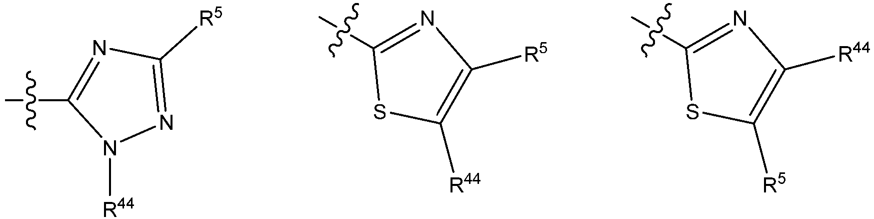

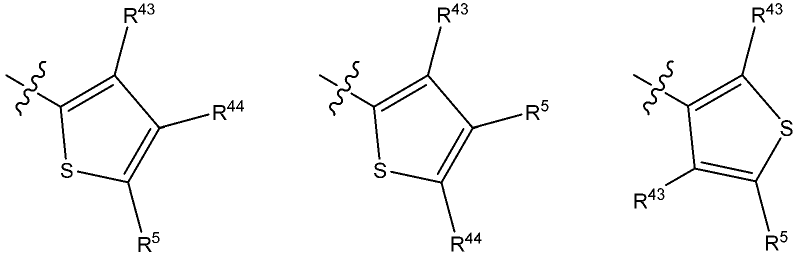

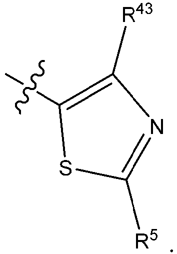

5. Verbindung zur Verwendung nach Anspruch 4, wobei Ring C der Gruppe L eine beliebige der folgenden Strukturen umfasst:

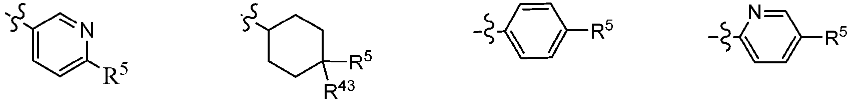

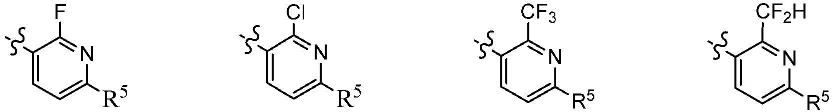

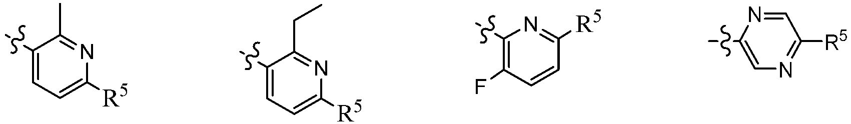

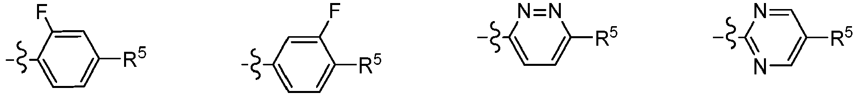

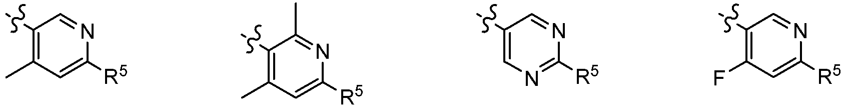

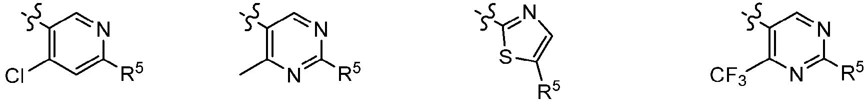

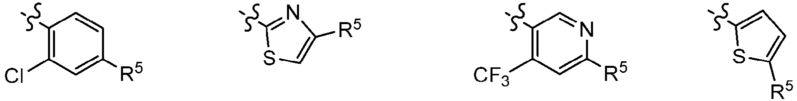

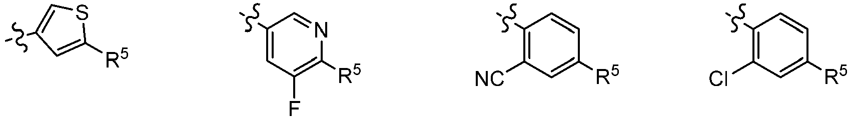

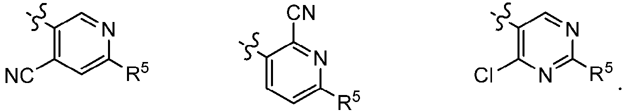

6. Verbindung nach einem vorhergehenden Anspruch, wobei die Gruppe R⁵ aus H, -F, - Cl, -Br, -I, -CN, -CONR⁵¹R⁵¹, -NR⁵¹COR⁵², -SO₂NR⁵¹R⁵¹, -NR⁵¹SO₂R⁵³, -O-CR⁵²R⁵²R⁵², - CR⁵²R⁵²NR⁵¹R⁵¹ und einer beliebigen der folgenden Strukturen ausgewählt ist: 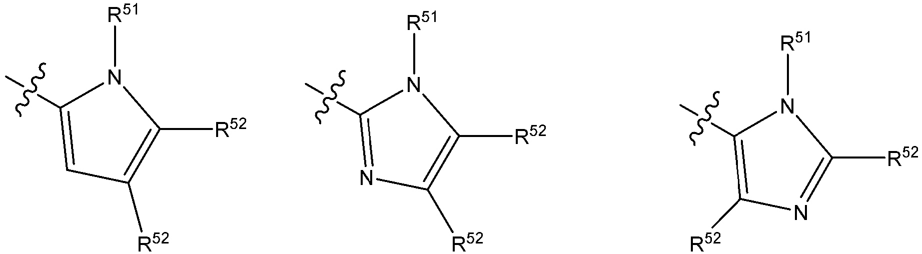 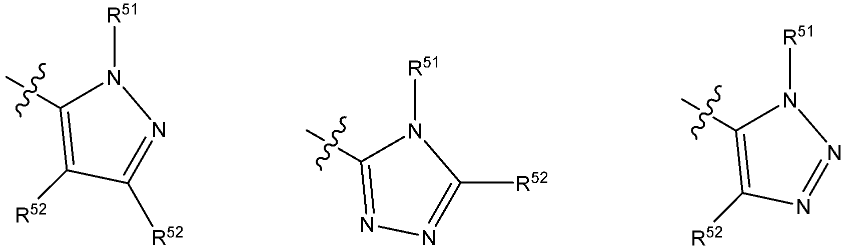 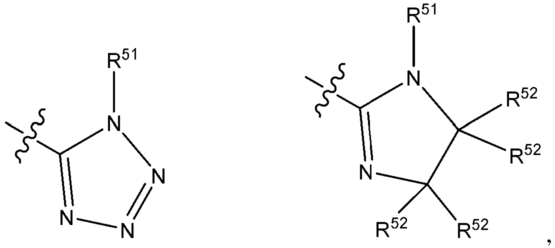 wobei jedes von R⁵¹, R⁵² und R⁵³ gleich oder verschieden sein kann und unabhängig aus H und einer substituierten oder unsubstituierten organischen Gruppe ausgewählt ist;
optional wobei die Gruppe R⁵ aus -F, -Cl, -CN, -CONH₂, -CONHMe, -CONHEt, - CONMe₂, -CONHCOMe, -CONHCH₂-CH₂OMe, -CONH-CH₂-CH₂F, -CONH-CH₂-CF₃, -CONH-CH₂-CHF₂, -OCHF₂, -NHCOMe, -NHSO₂Me, -SO₂NHMe, - CONHSO₂Me, 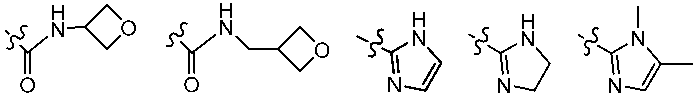 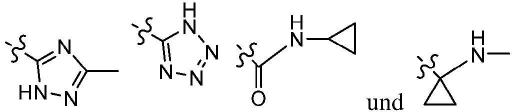 ausgewählt ist.

7. Verbindung zur Verwendung nach einem vorhergehenden Anspruch, wobei X⁵ N ist.

8. Verbindung zur Verwendung nach Anspruch 7, wobei der Ring B der Gruppe L eine beliebige der folgenden Strukturen umfasst:

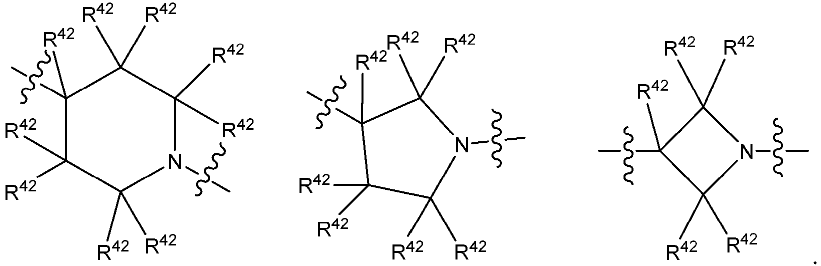

9. Verbindung zur Verwendung nach Anspruch 8, wobei der Ring B der Gruppe L eine beliebige der folgenden Strukturen umfasst:

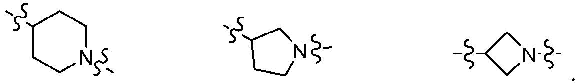

10. Verbindung nach einem vorhergehenden Anspruch, wobei die Gruppe L eine Gruppe mit der folgenden Struktur umfasst:

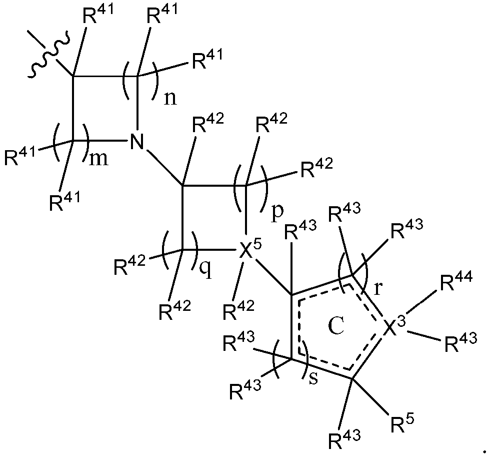

11. Verbindung zur Verwendung nach einem vorhergehenden Anspruch, wobei die Gruppe L eine Gruppe umfasst, die aus den folgenden Strukturen ausgewählt ist:

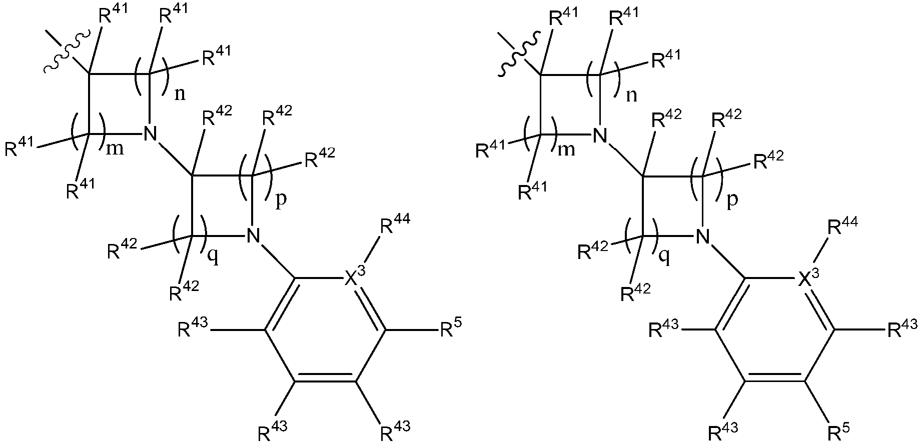

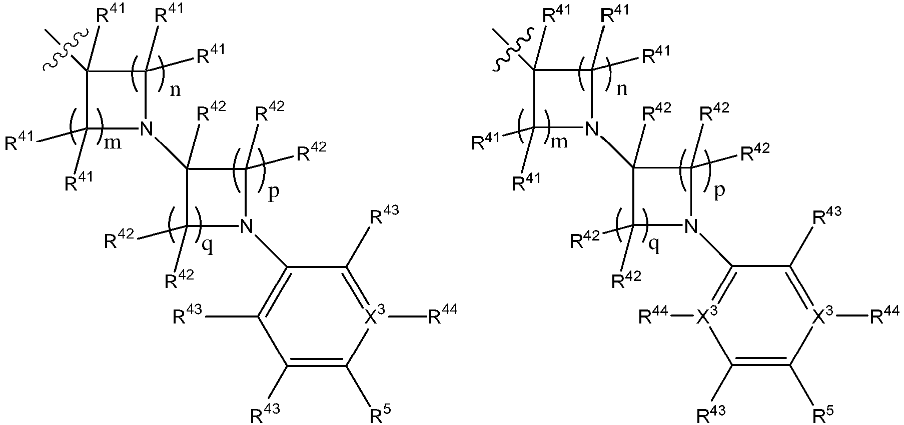

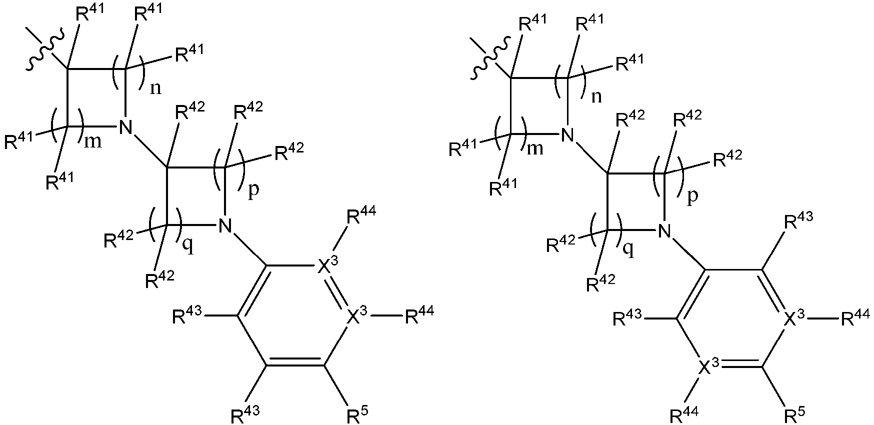

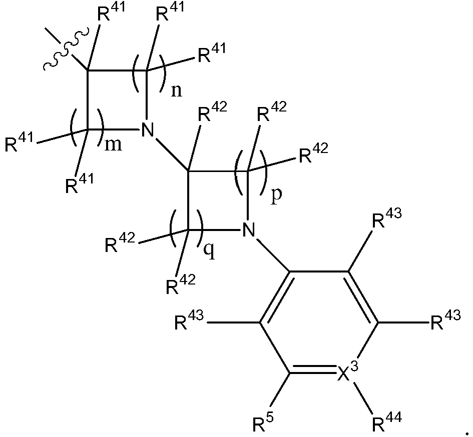

12. Verbindung zur Verwendung nach einem vorhergehenden Anspruch, wobei m aus 1 oder 2 ausgewählt ist, n aus 2 oder 3 ausgewählt ist, p aus 1, 2 oder 3 ausgewählt ist und q aus 1 oder 2 ausgewählt ist.

13. Verbindung zur Verwendung nach Anspruch 11 oder Anspruch 12, wobei die Gruppe L eine Gruppe mit einer beliebigen der folgenden Strukturen umfasst:

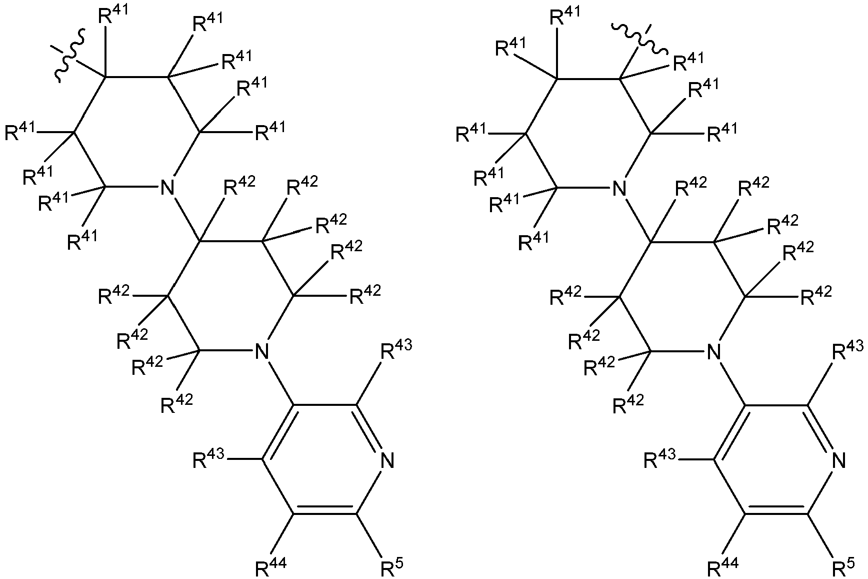

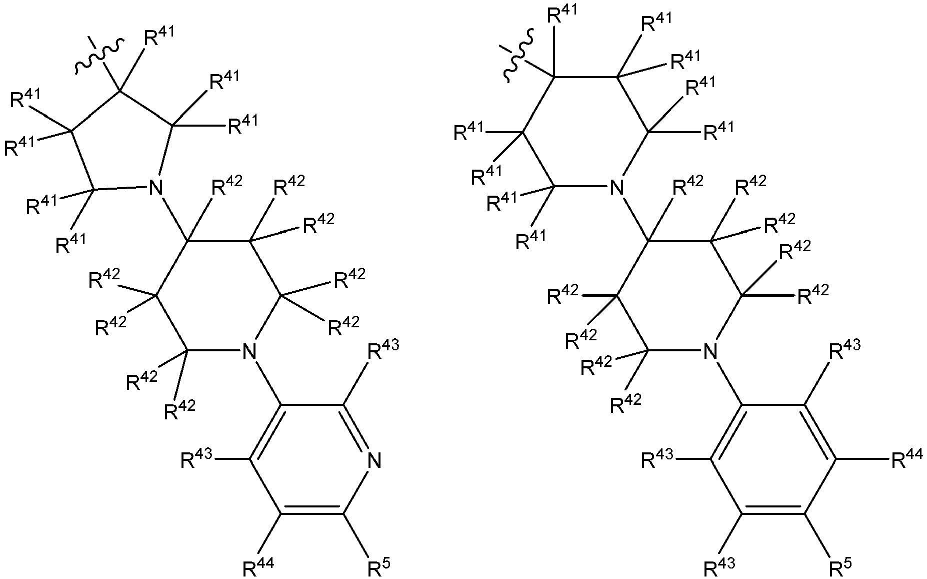

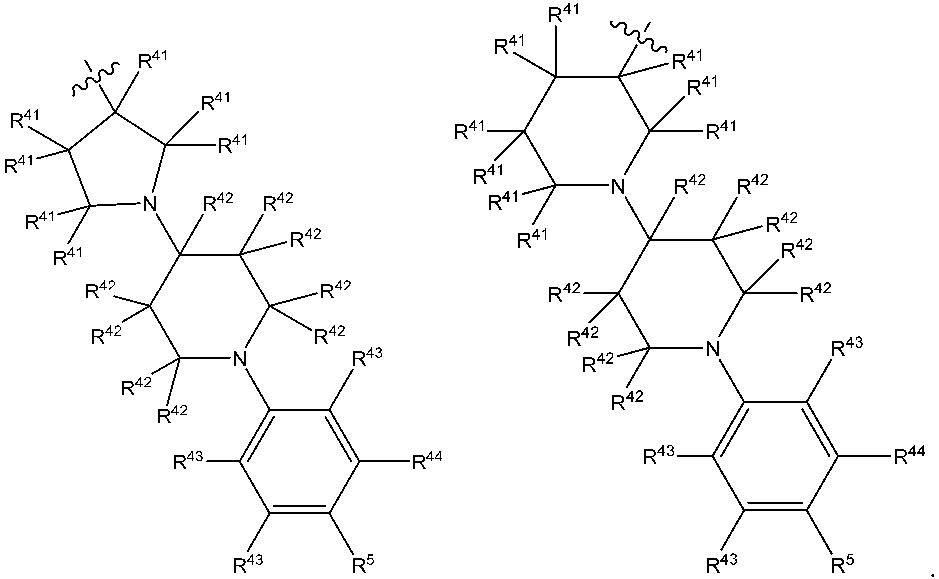

14. Verbindung zur Verwendung nach einem vorhergehenden Anspruch, wobei die Gruppe L eine Gruppe mit einer beliebigen der folgenden Strukturen umfasst:

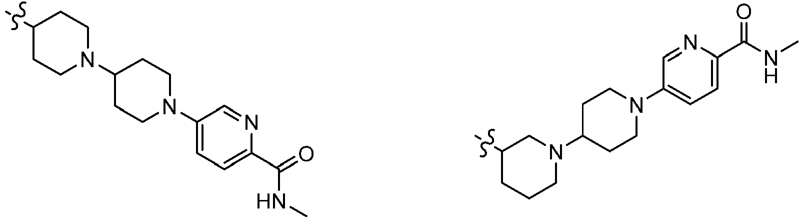

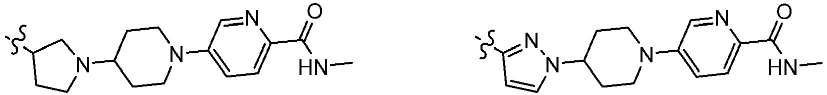

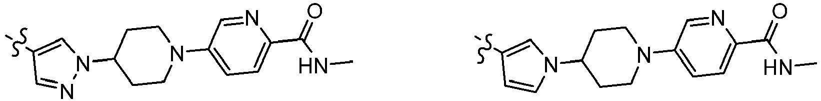

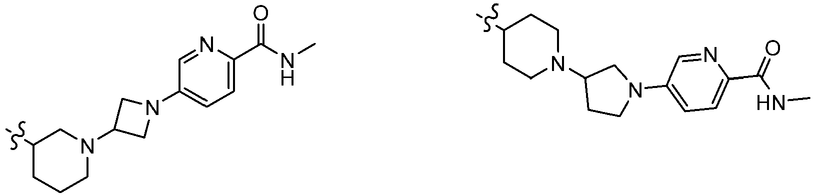

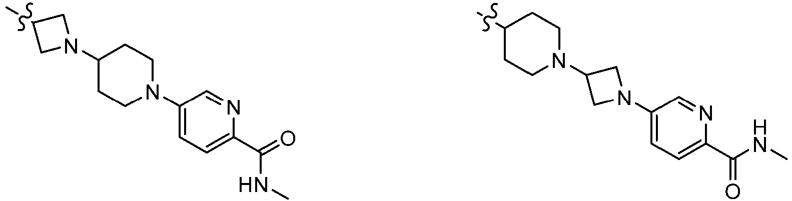

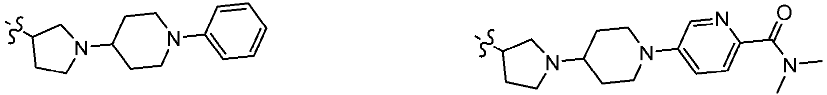

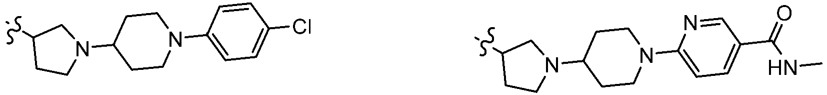

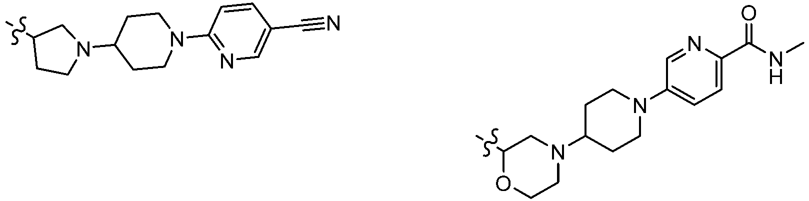

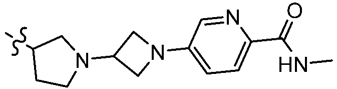

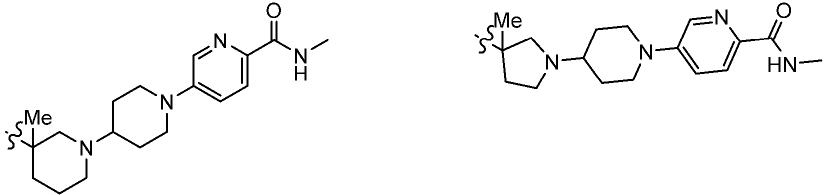

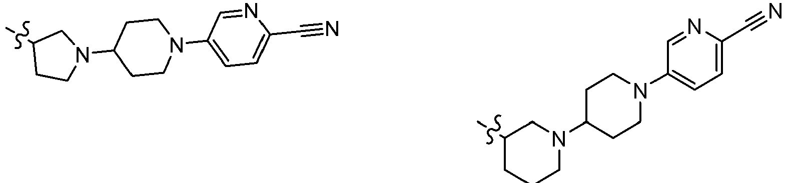

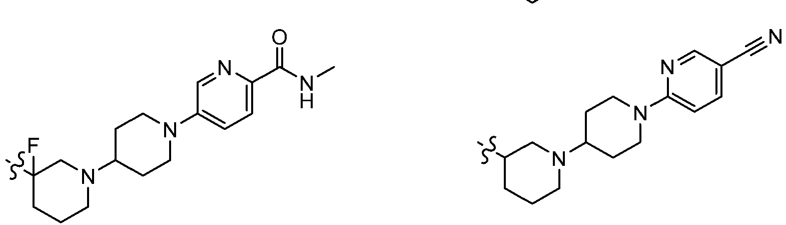

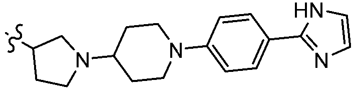

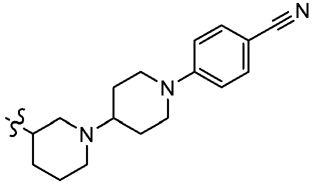

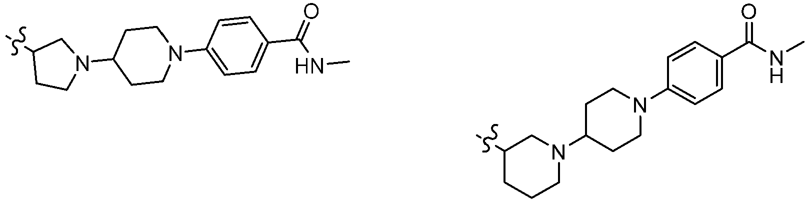

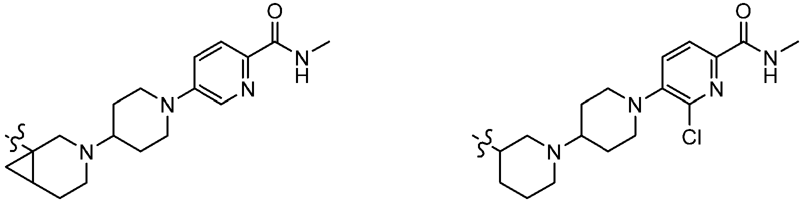

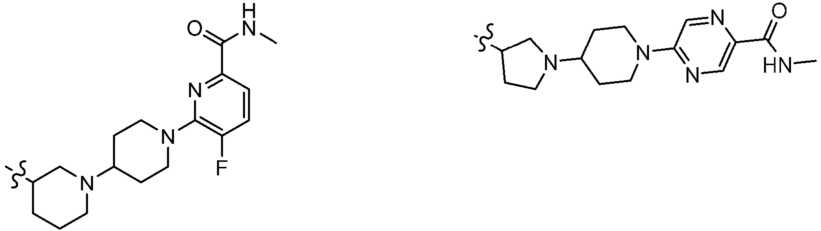

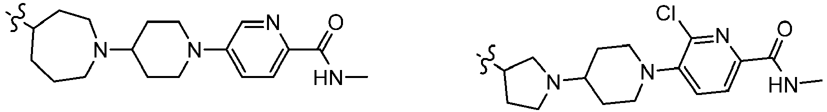

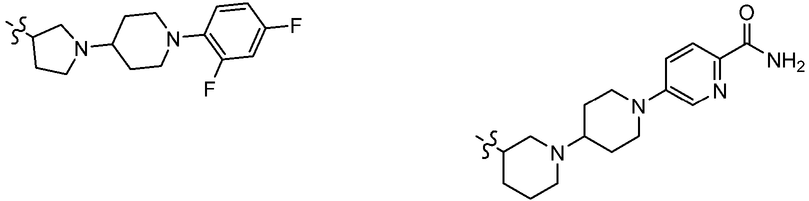

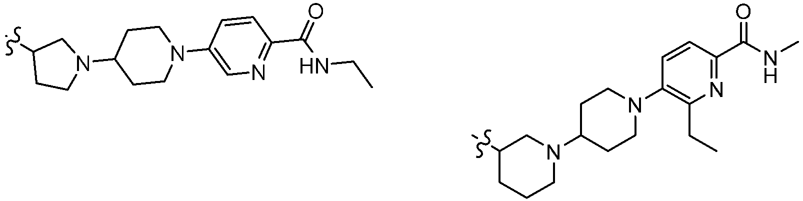

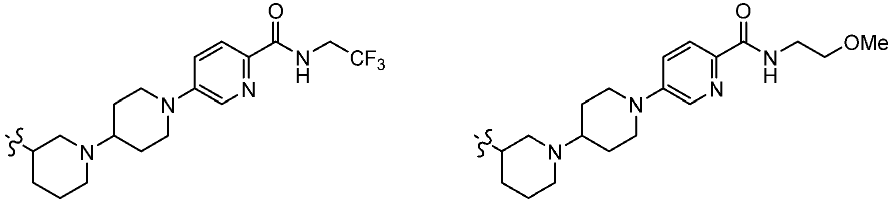

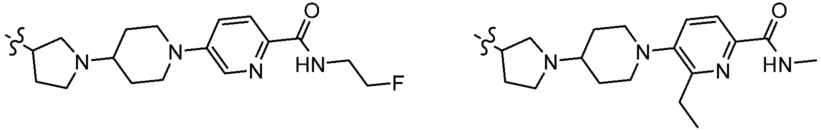

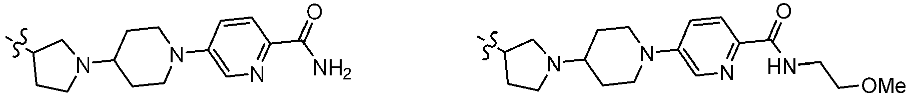

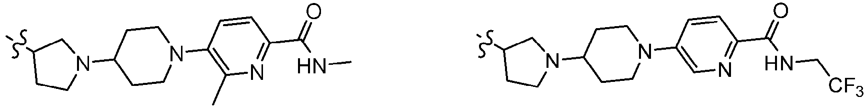

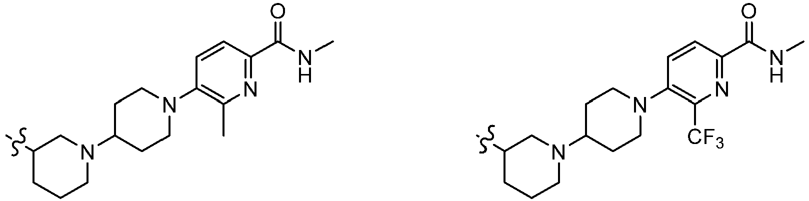

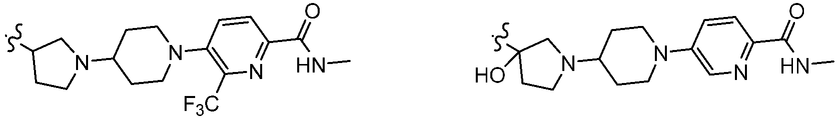

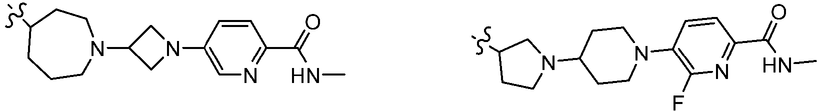

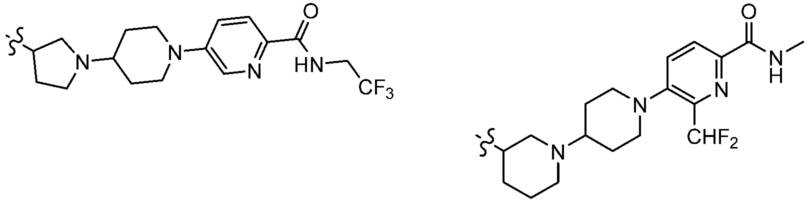

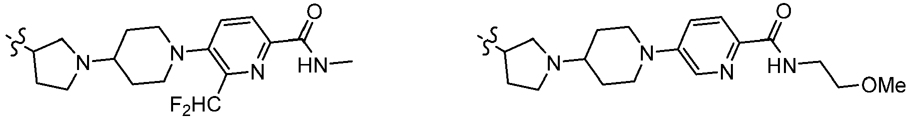

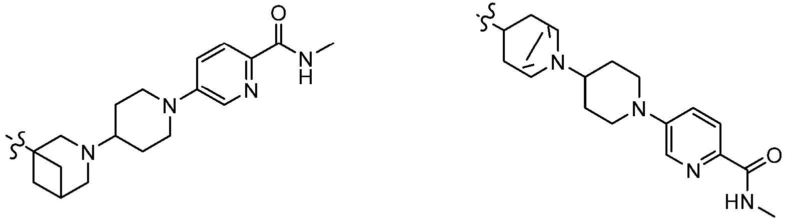

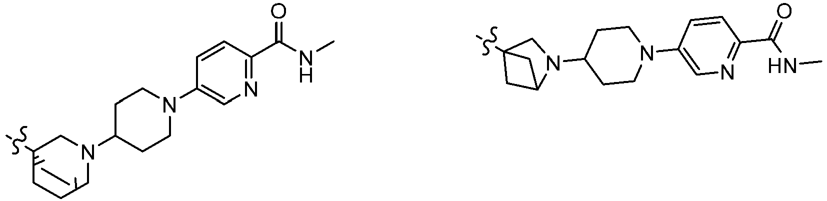

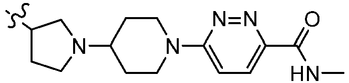

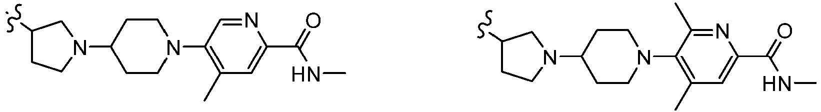

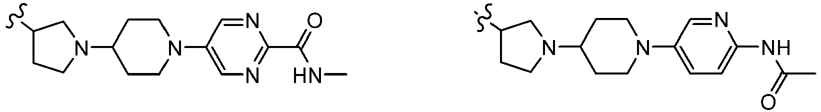

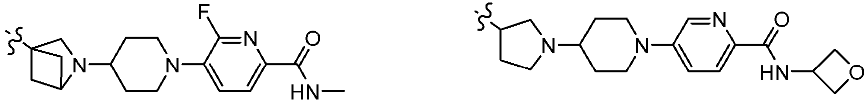

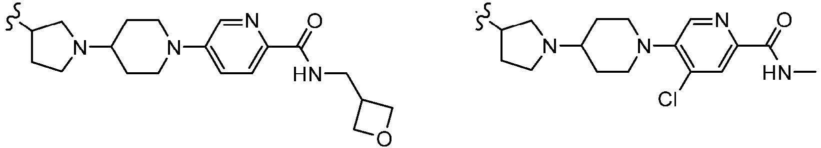

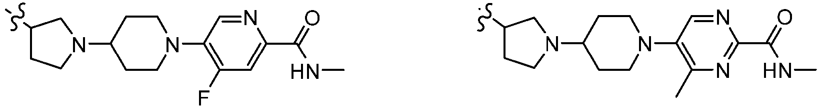

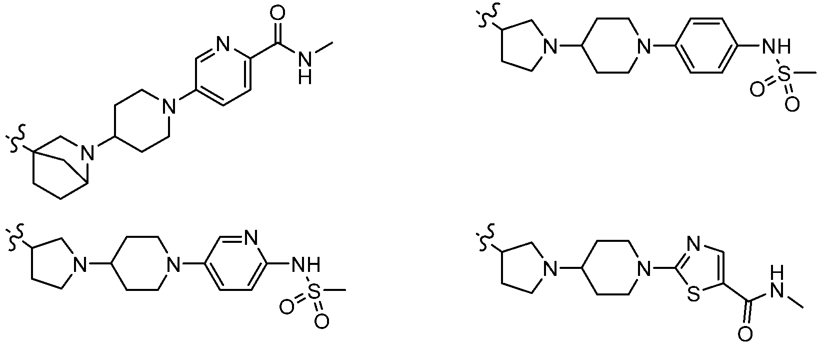

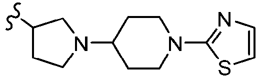

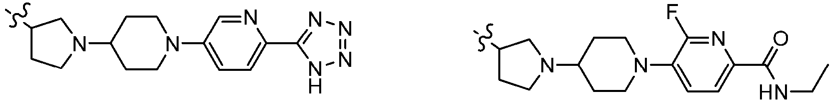

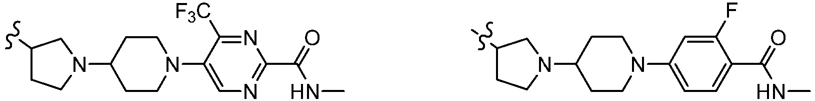

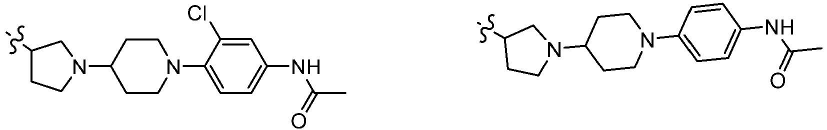

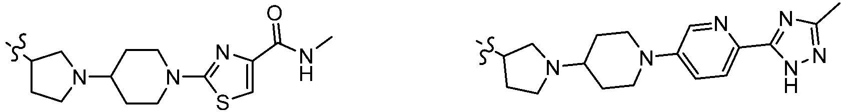

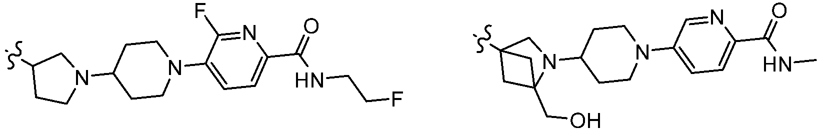

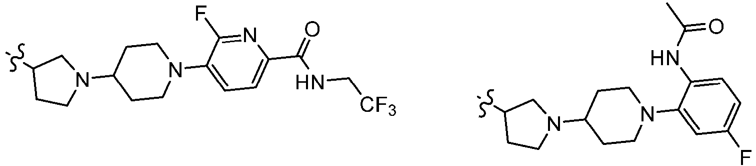

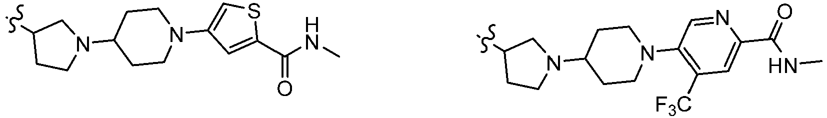

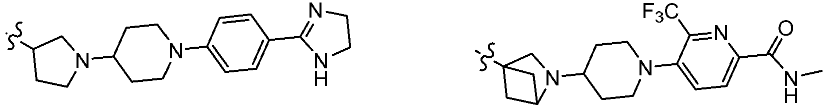

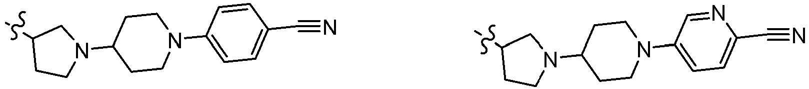

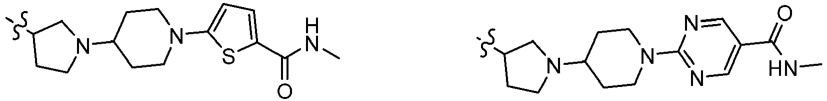

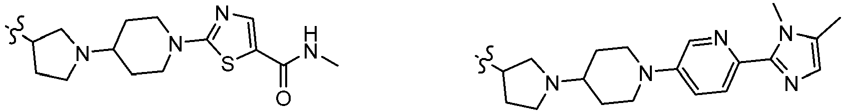

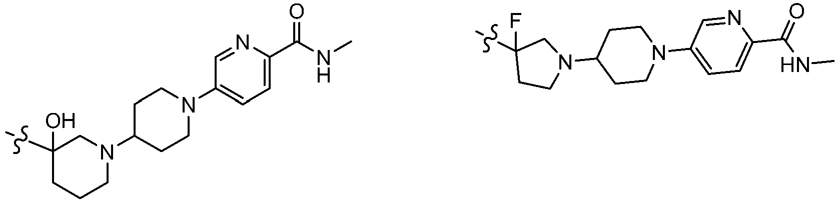

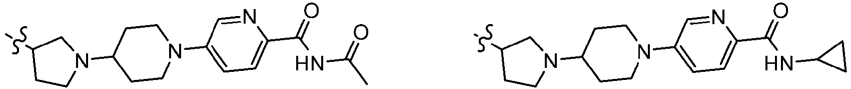

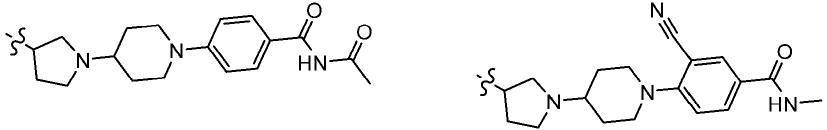

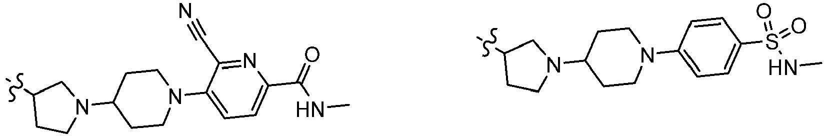

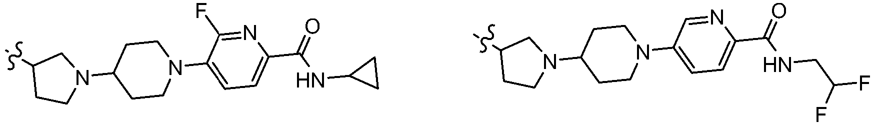

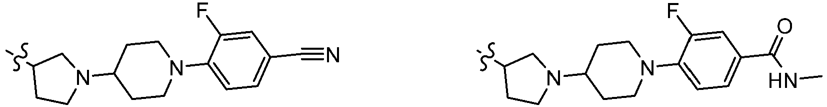

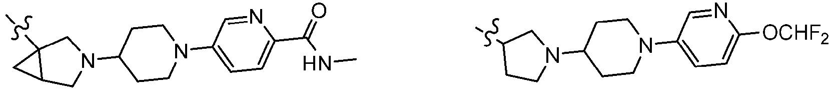

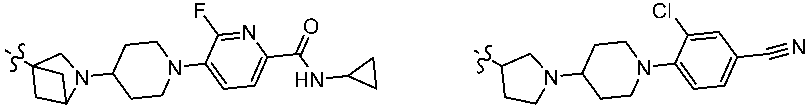

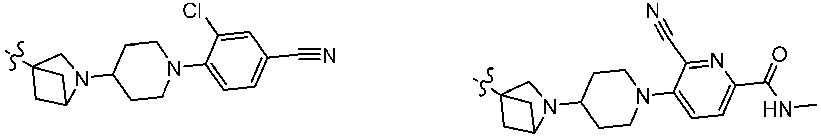

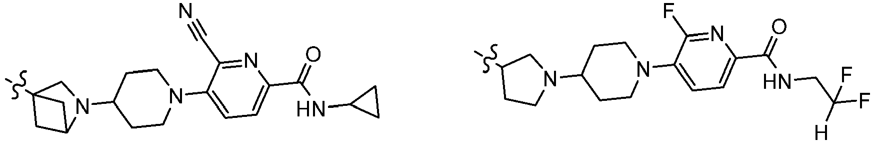

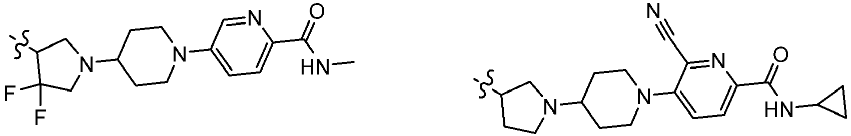

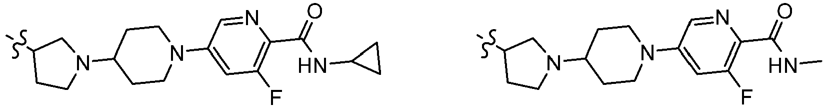

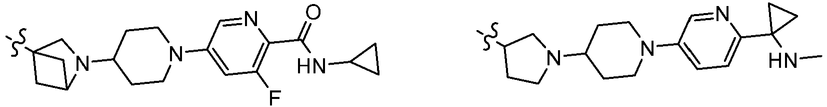

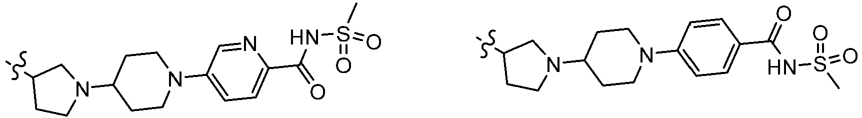

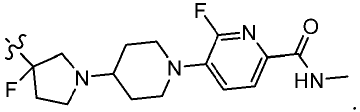

15. Verbindung nach einem vorhergehenden Anspruch, wobei diese Verbindung eine beliebige der folgenden Strukturen umfasst: 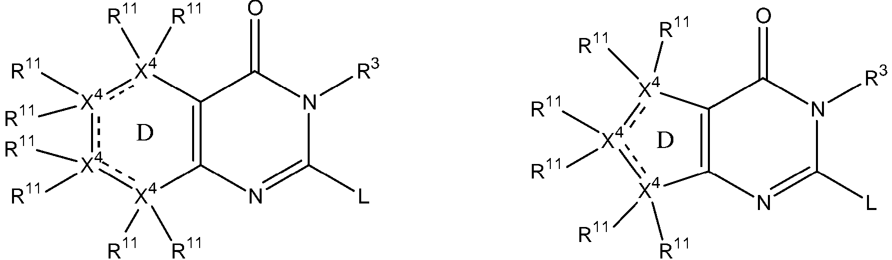 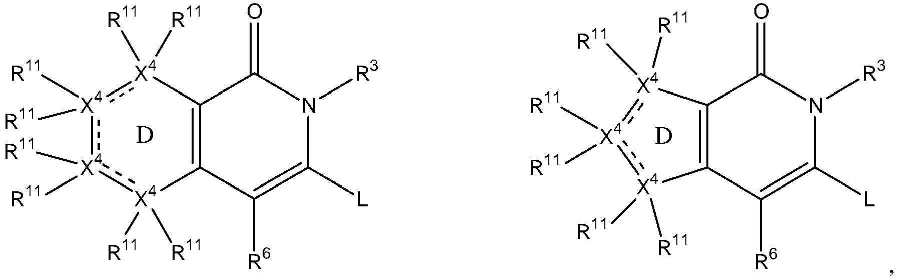 vorzugsweise wobei mindestens eines von X⁴ C ist.

16. Verbindung zur Verwendung nach Anspruch 15, wobei diese Verbindung eine beliebige der folgenden Strukturen umfasst: 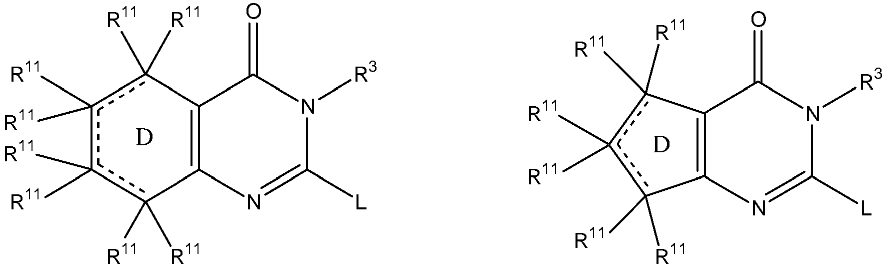 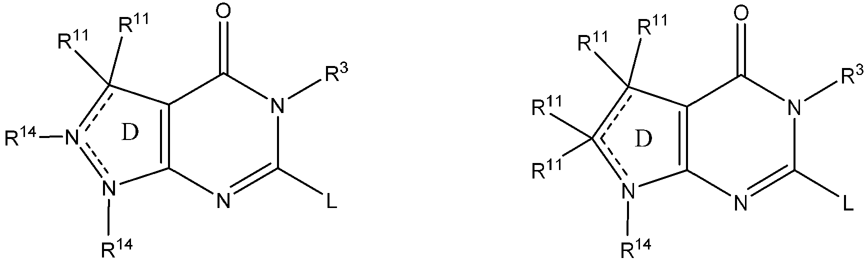 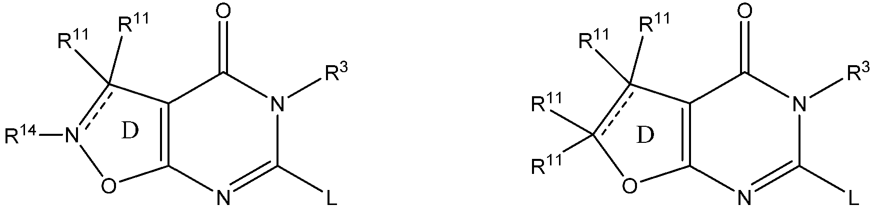 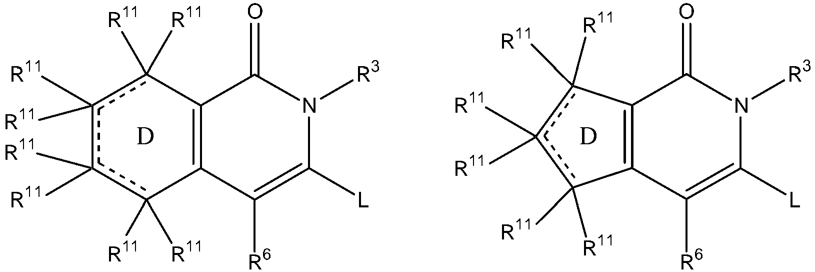 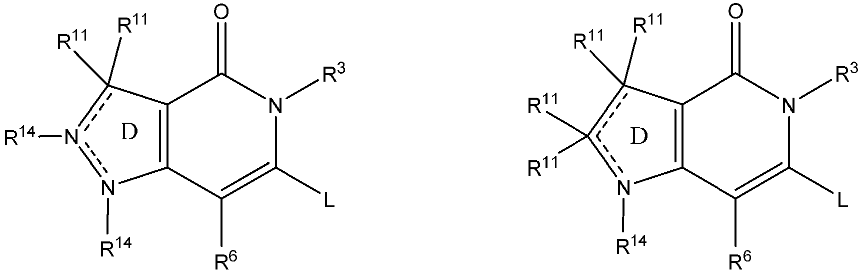 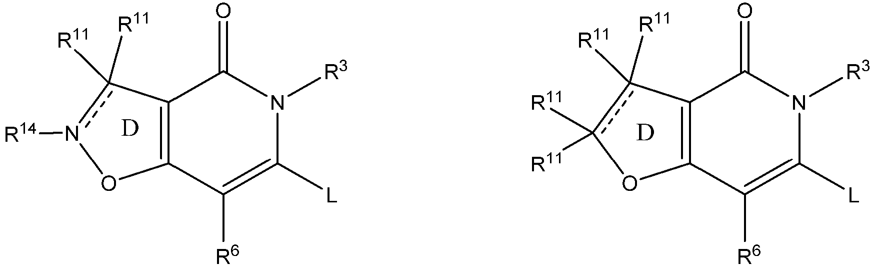 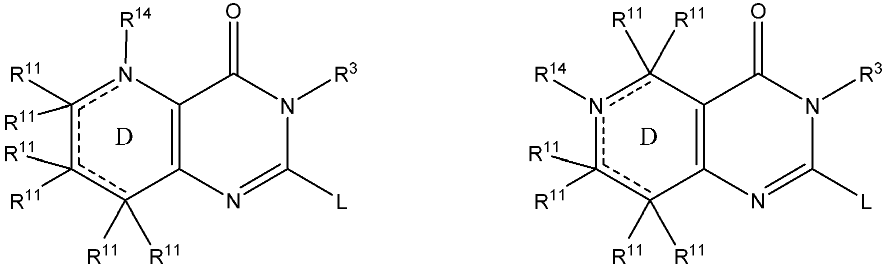 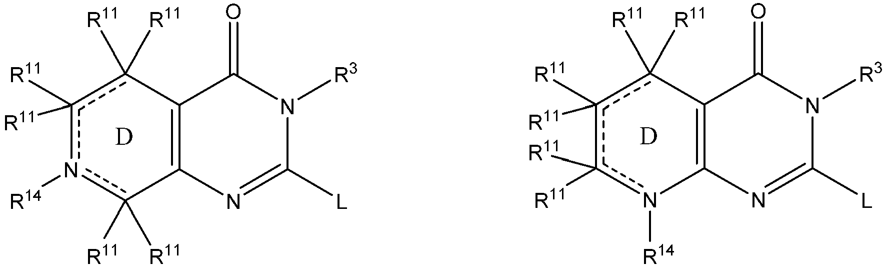 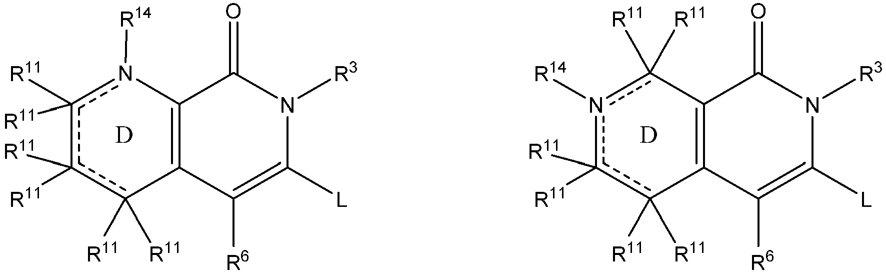 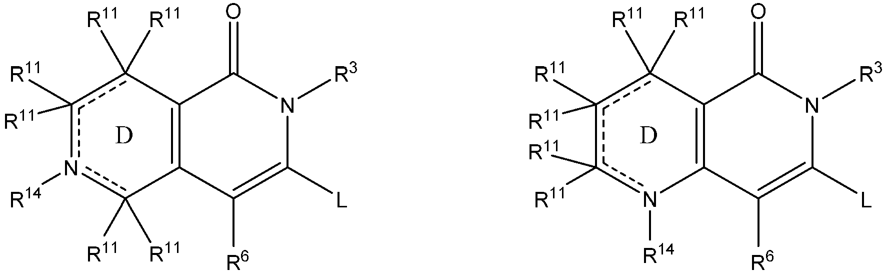 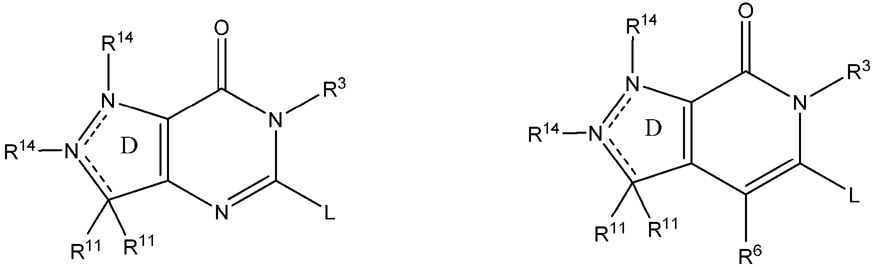 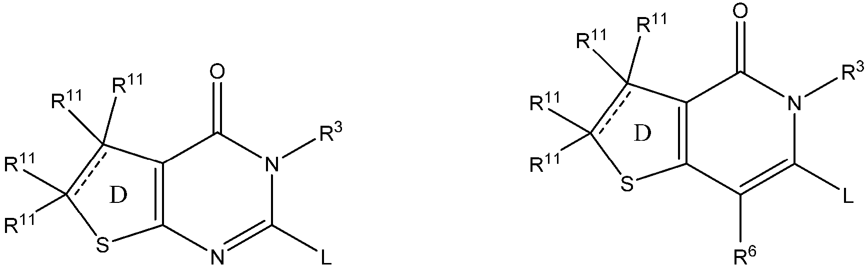 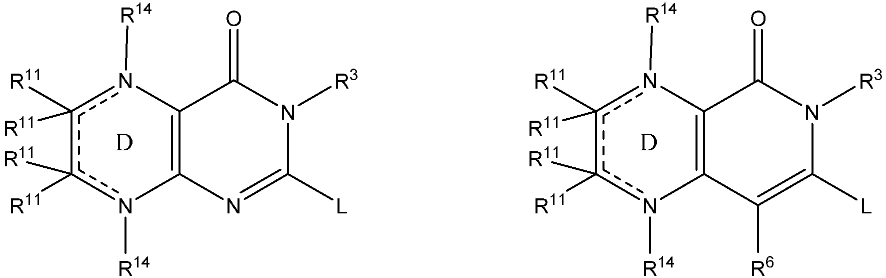 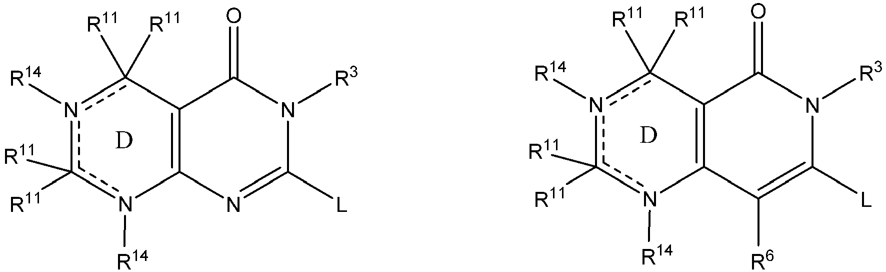 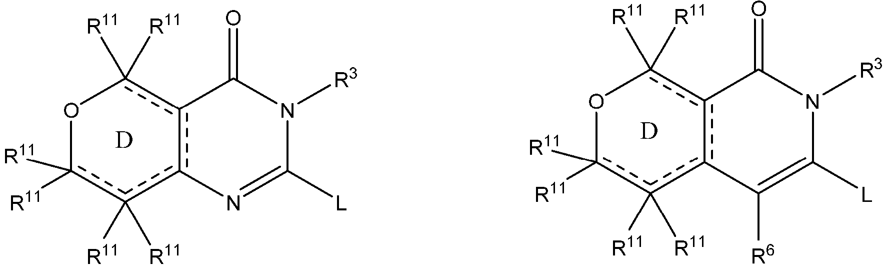 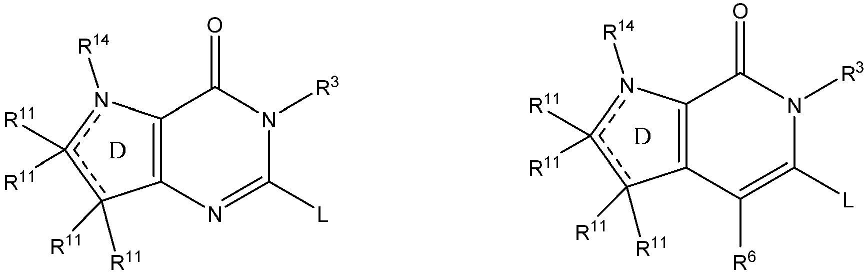 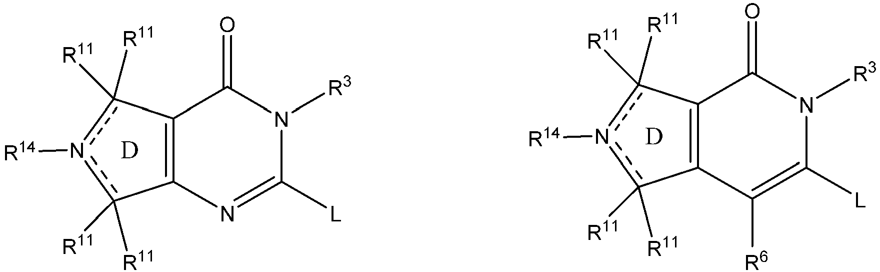 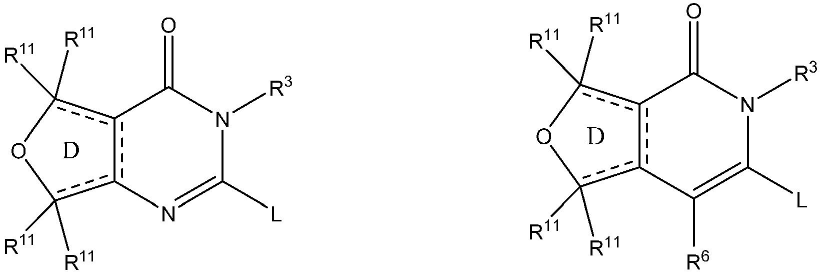 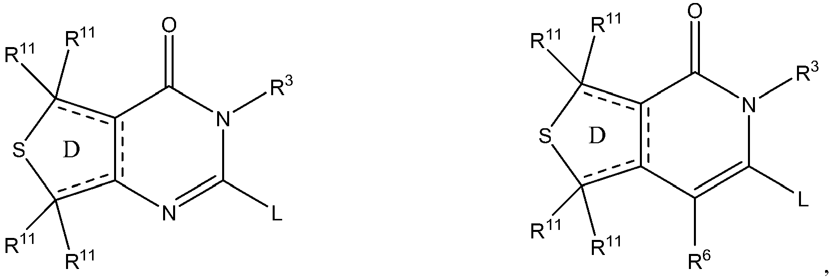 wobei R¹⁴ vorliegen oder fehlen kann und wenn es vorliegt, aus H, C₁- bis C₃-Alkyl oder C₁-bis C₃-Fluoralkyl ausgewählt ist.

17. Verbindung nach Anspruch 16, wobei diese Verbindung eine beliebige der folgenden Strukturen umfasst:

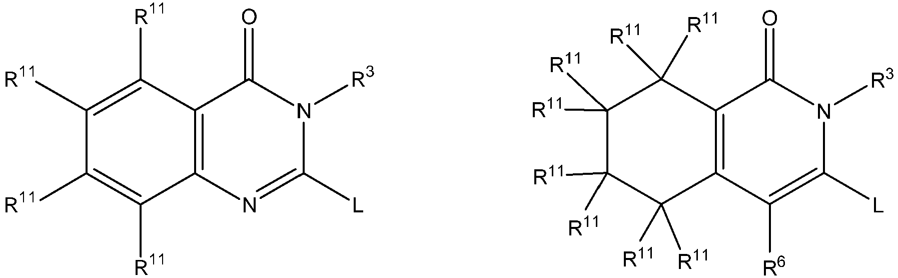

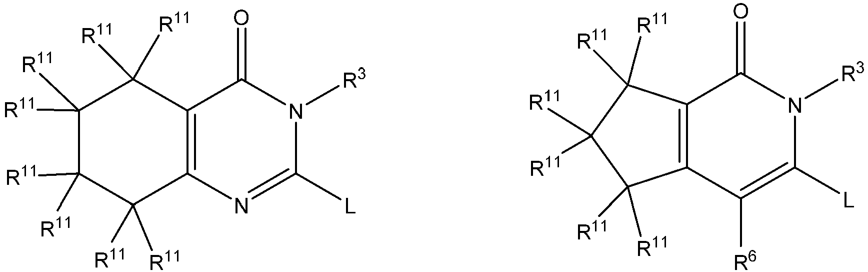

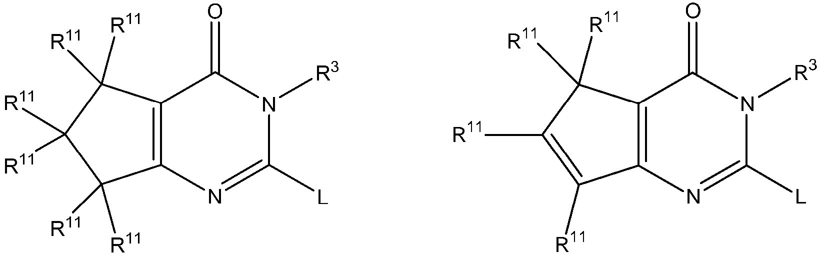

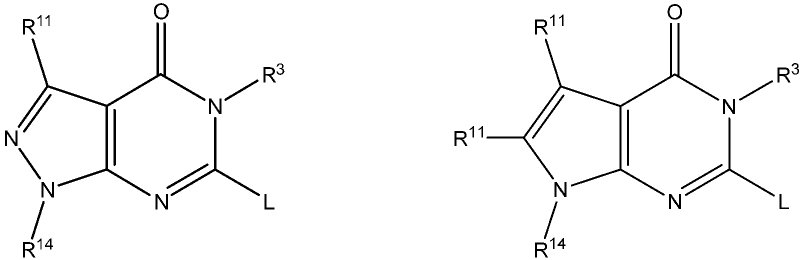

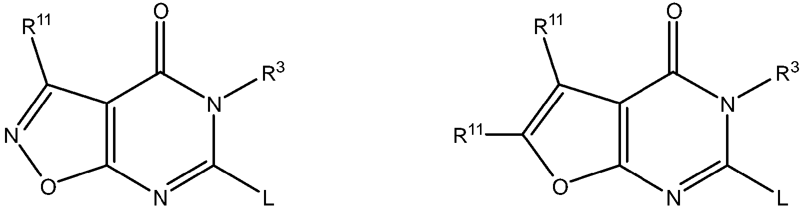

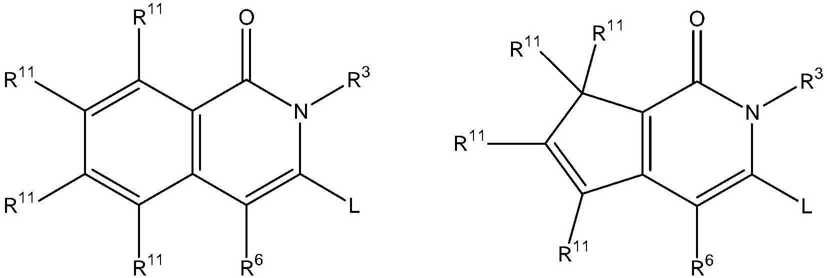

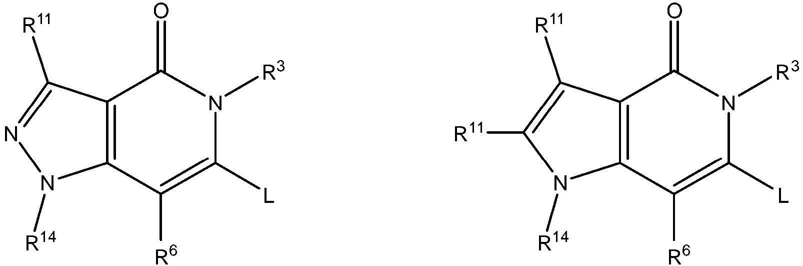

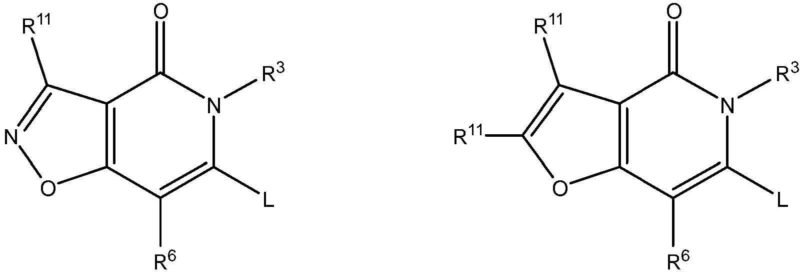

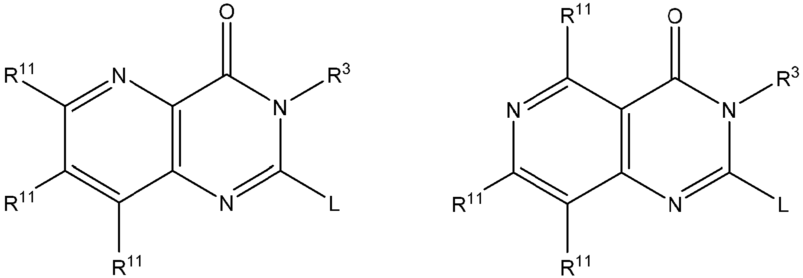

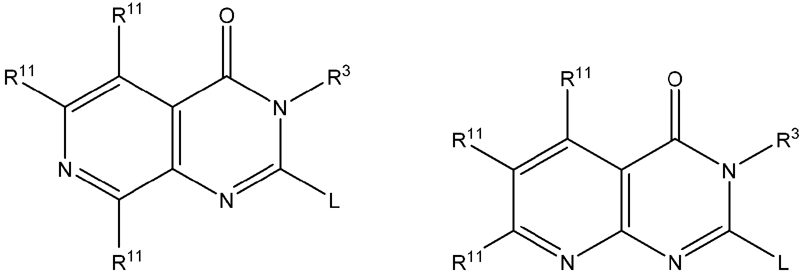

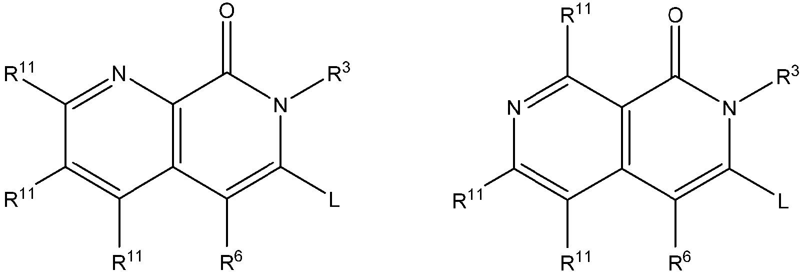

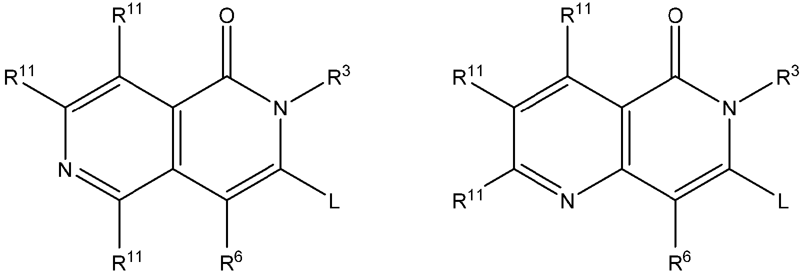

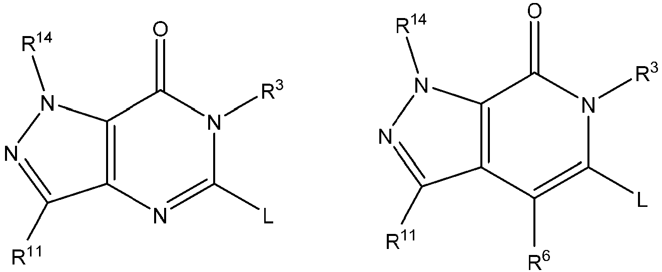

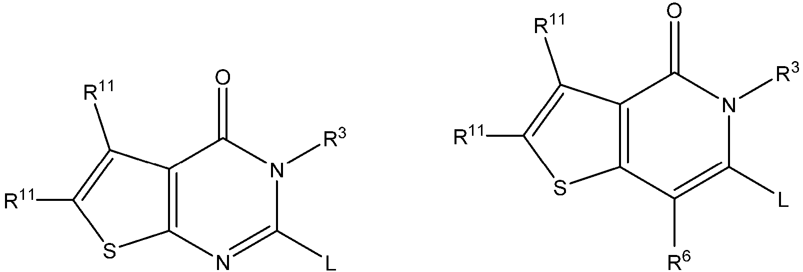

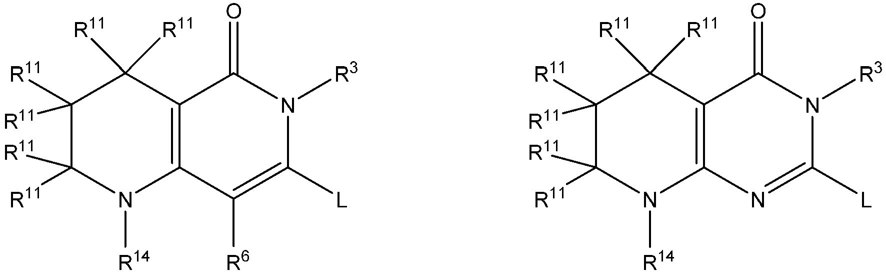

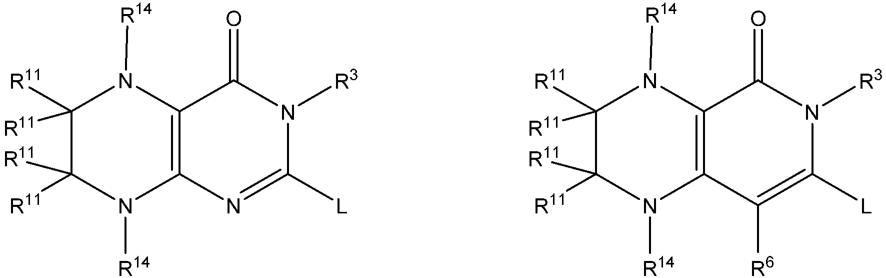

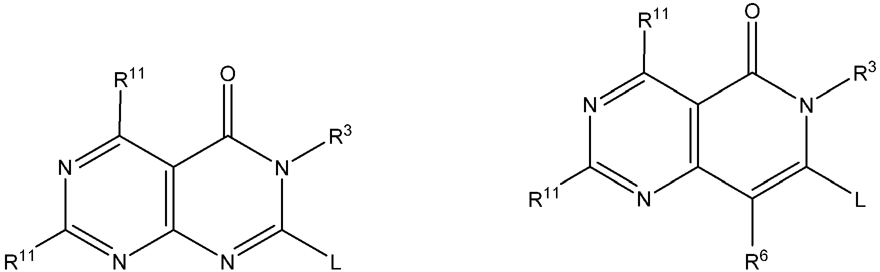

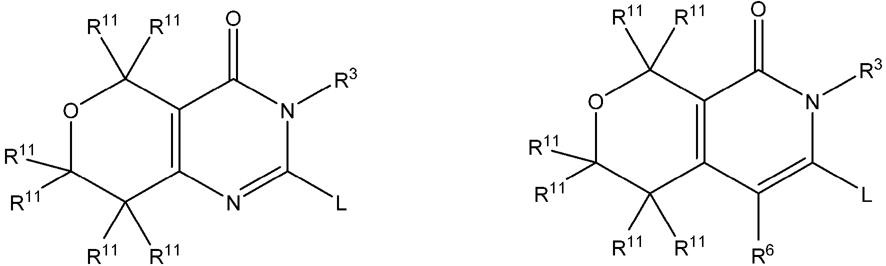

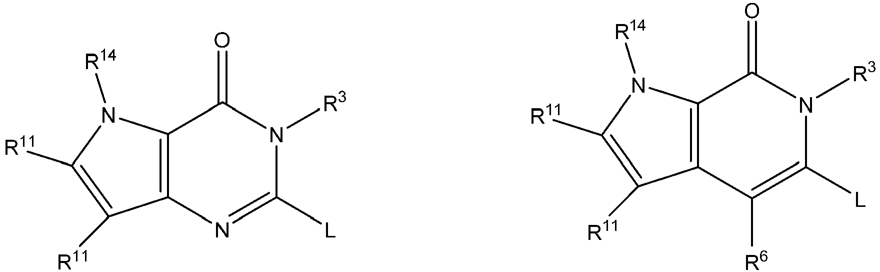

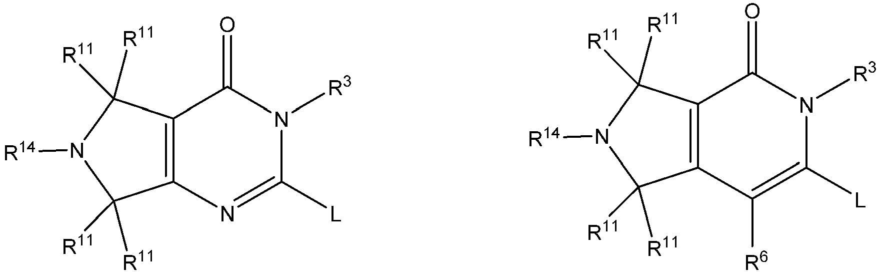

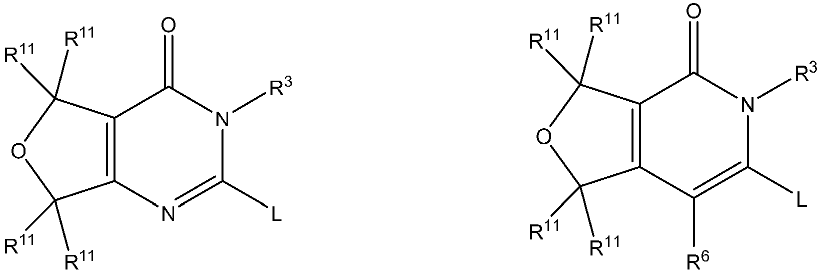

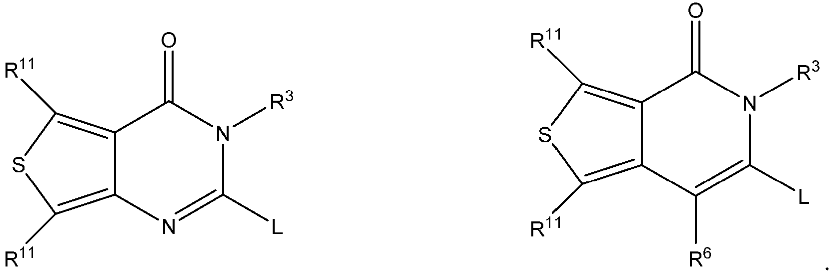

18. Verbindung zur Verwendung nach einem vorhergehenden Anspruch, wobei jedes R¹¹ unabhängig aus H, Cl, F, CHF₂, CF₃, CH₃, OH, CH₃O und NH₂ ausgewählt ist und/oder ein Paar von R¹¹-Gruppen, die an dasselbe Atom angelagert sind, =O bildet; und unabhängig, wenn ein Paar von R¹¹-Gruppen, die an unterschiedliche Atome angelagert sind, zusammen einen Ring mit Ring-D-Atomen bildet, das Paar von R¹¹-Gruppen -CH₂CH₂CH₂- umfasst.

19. Verbindung zur Verwendung nach Anspruch 18, wobei diese Verbindung eine beliebige der folgenden Strukturen umfasst:

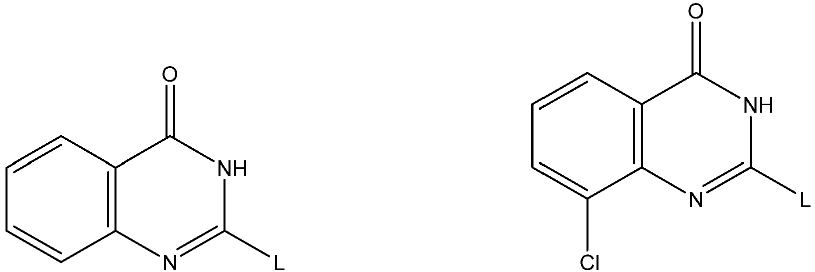

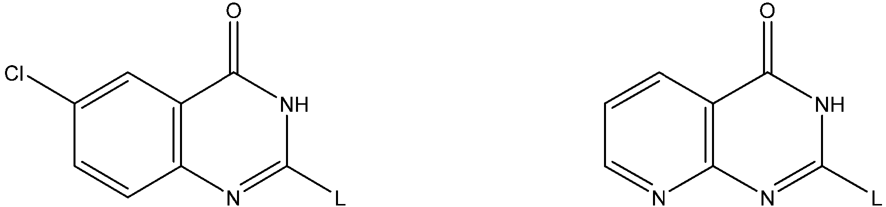

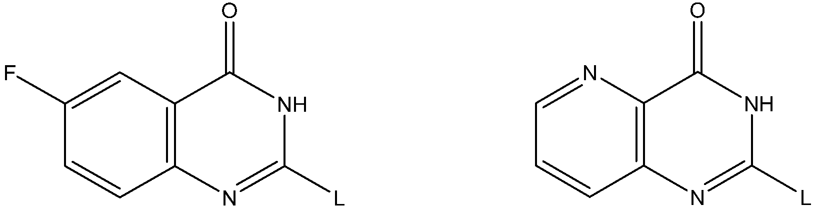

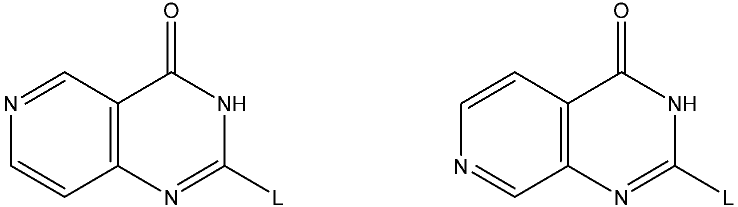

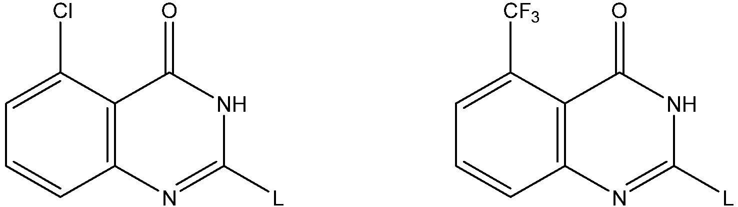

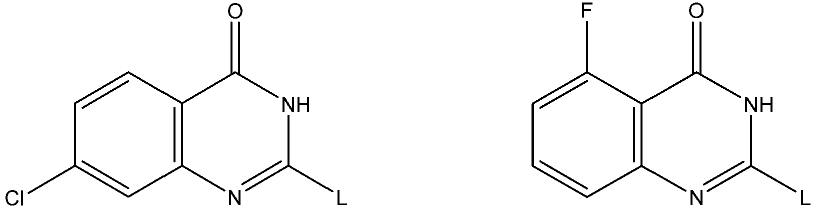

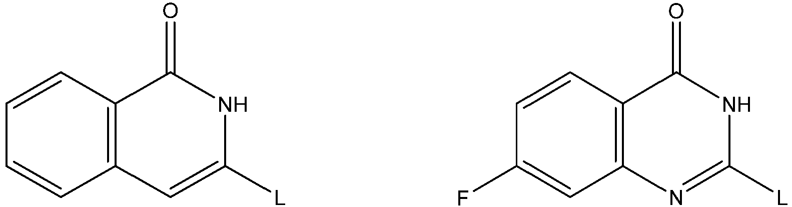

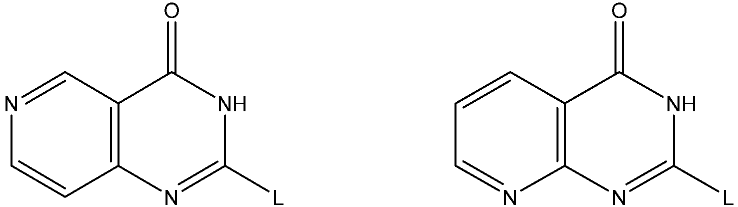

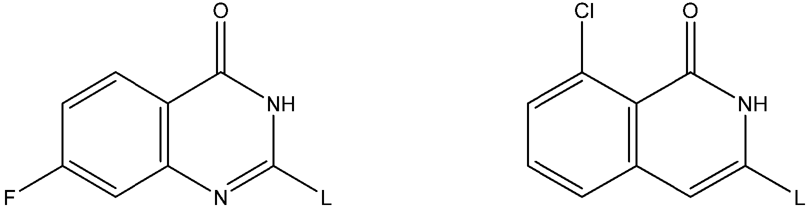

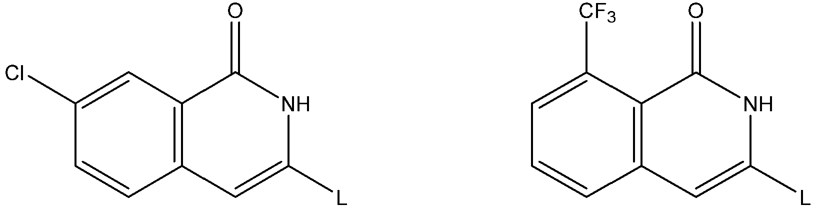

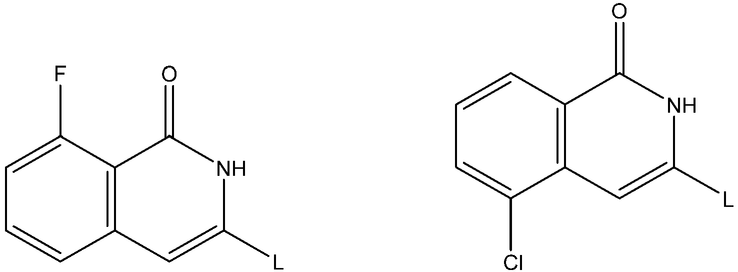

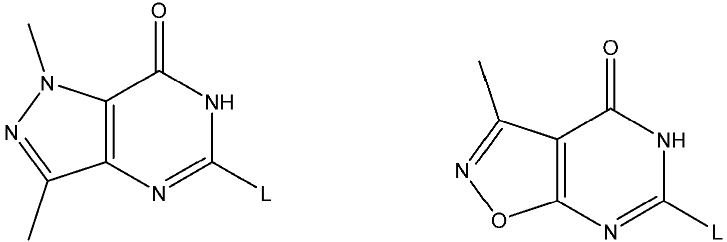

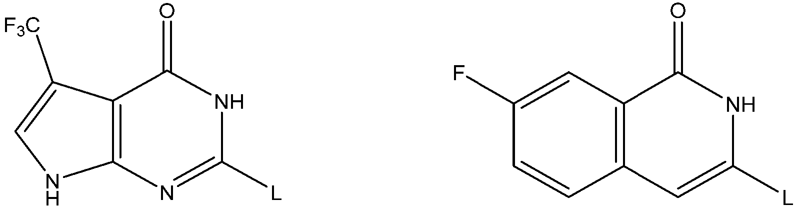

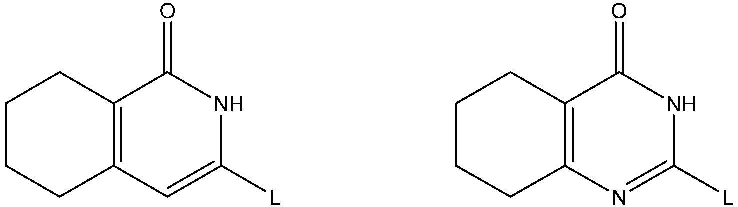

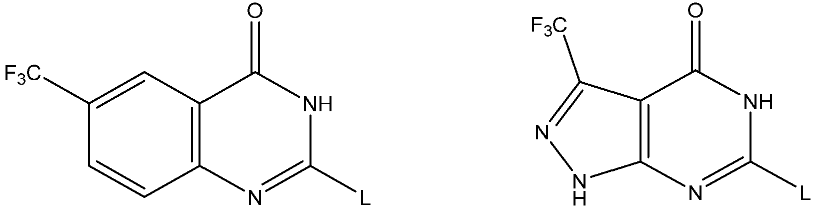

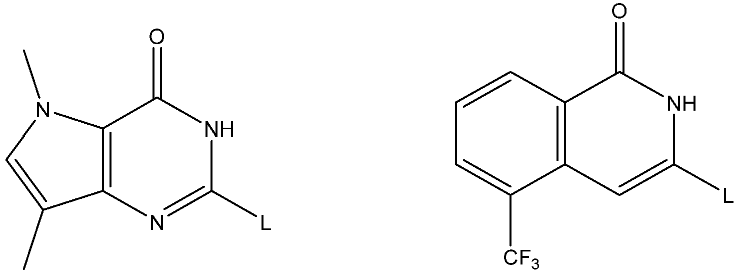

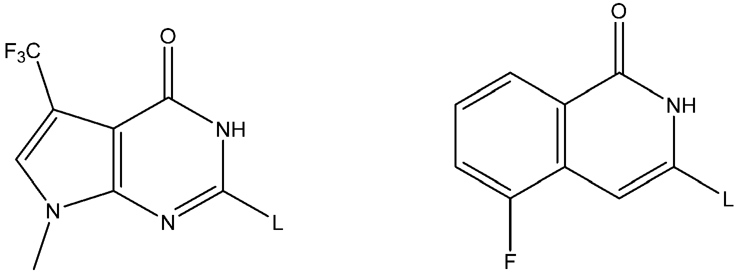

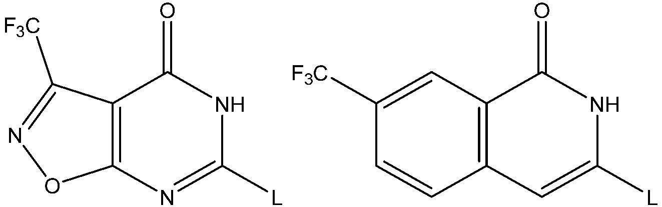

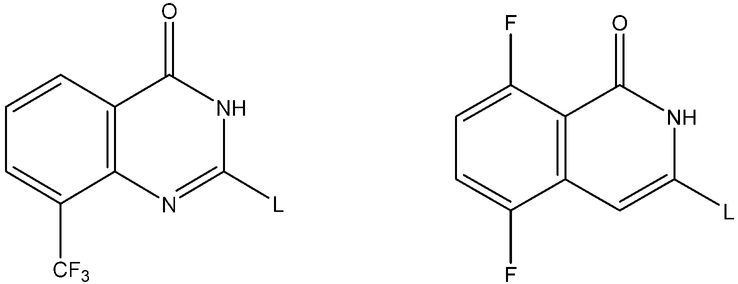

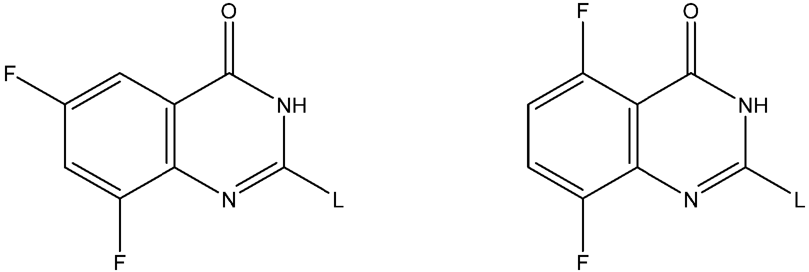

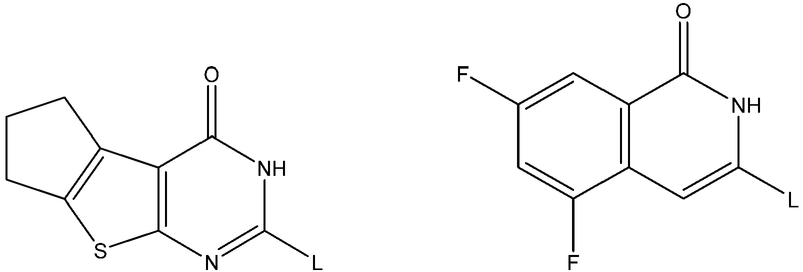

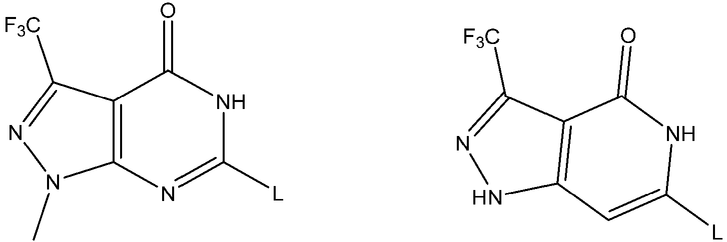

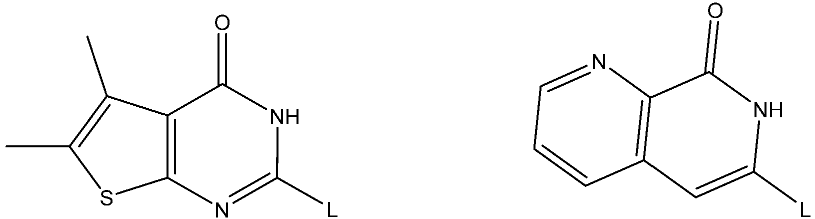

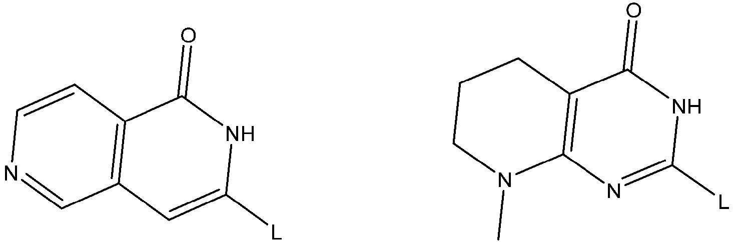

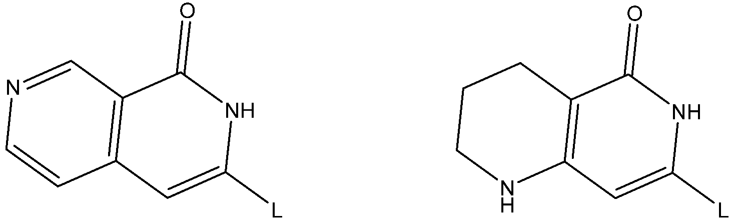

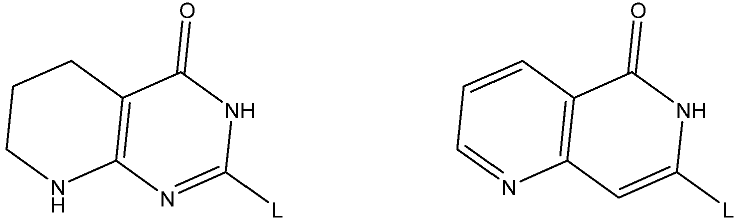

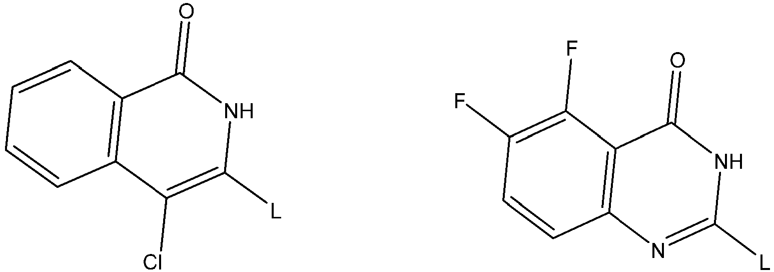

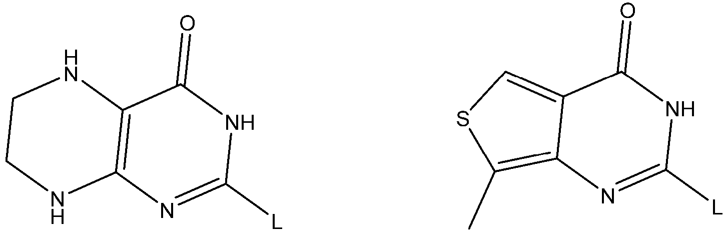

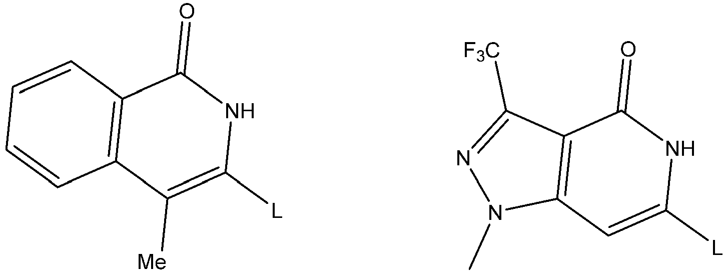

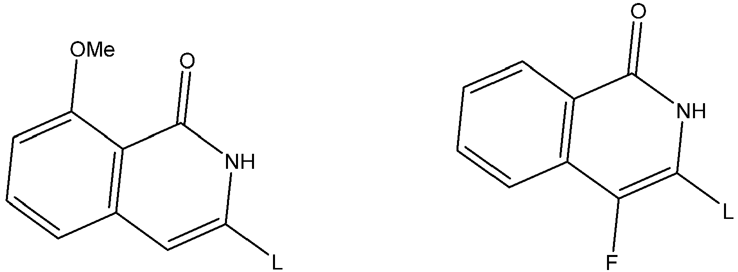

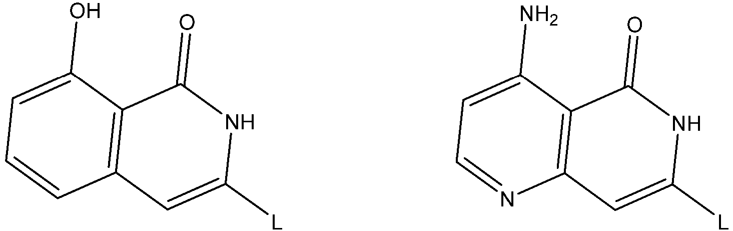

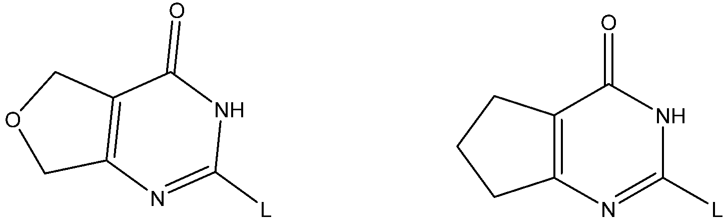

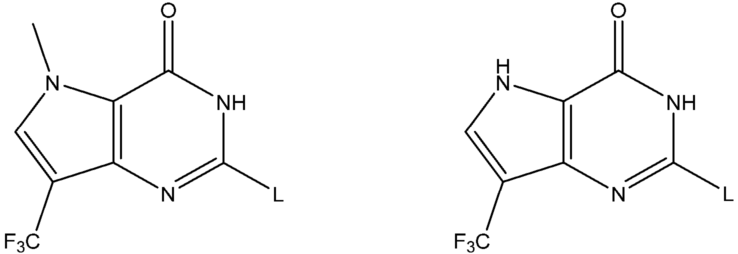

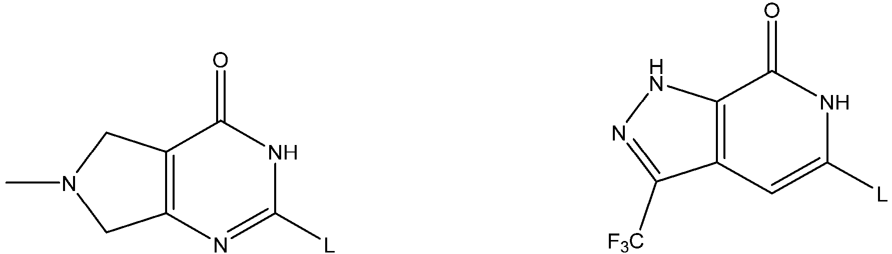

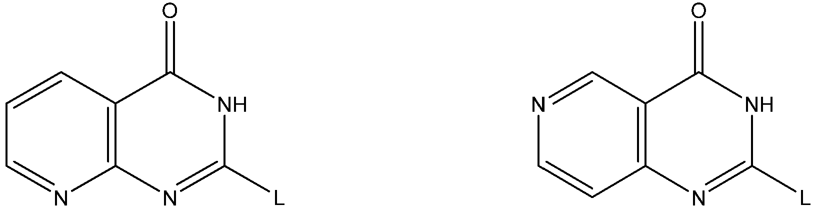

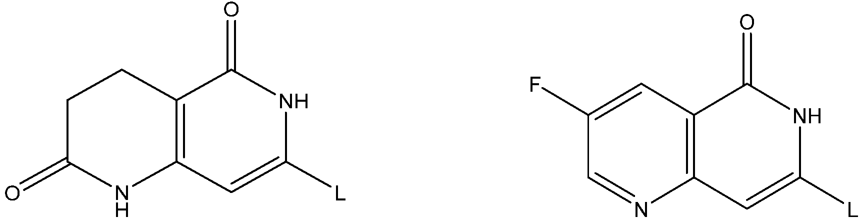

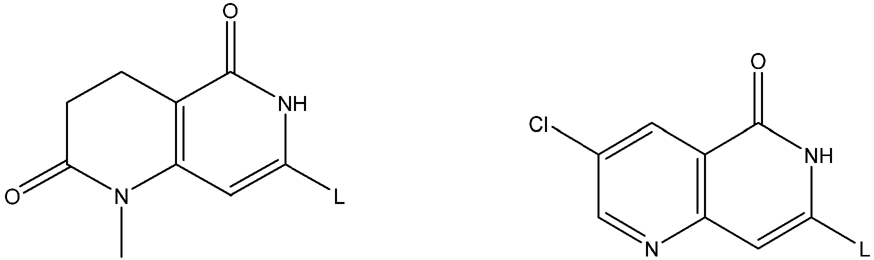

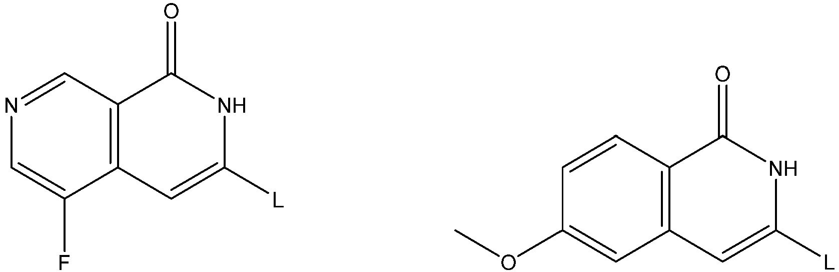

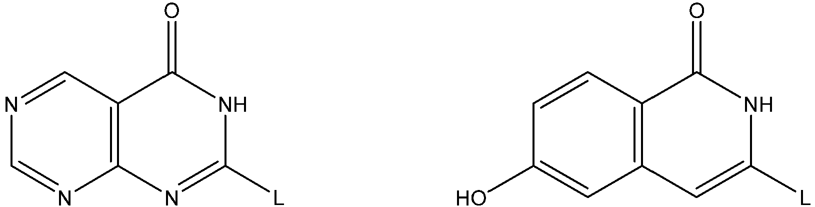

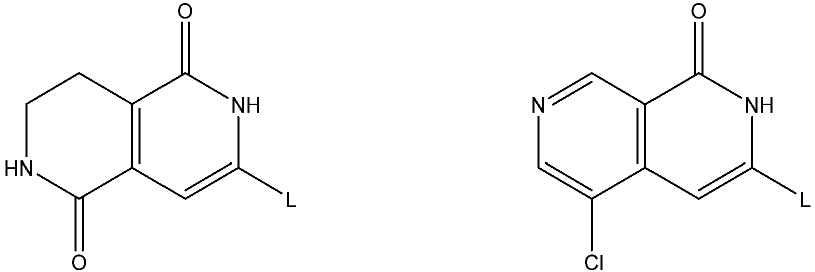

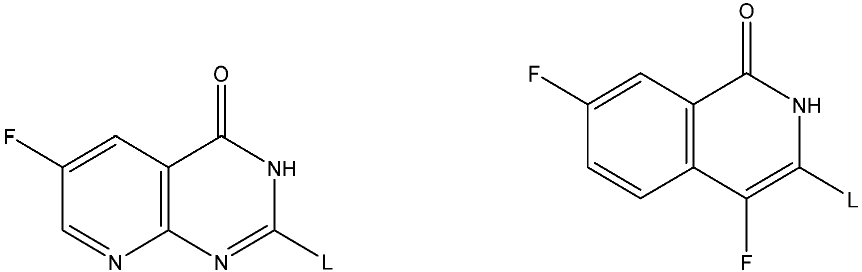

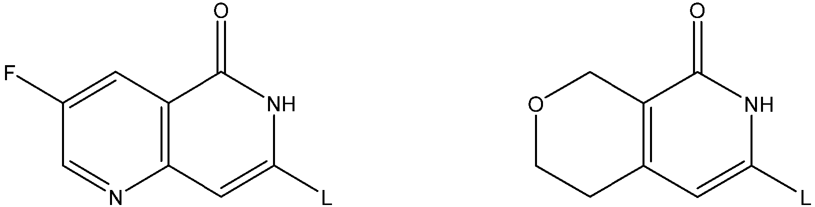

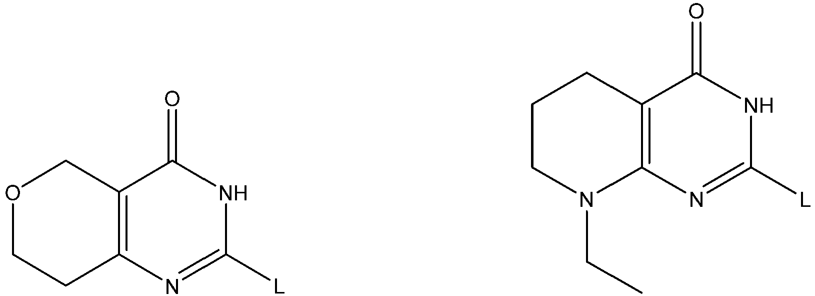

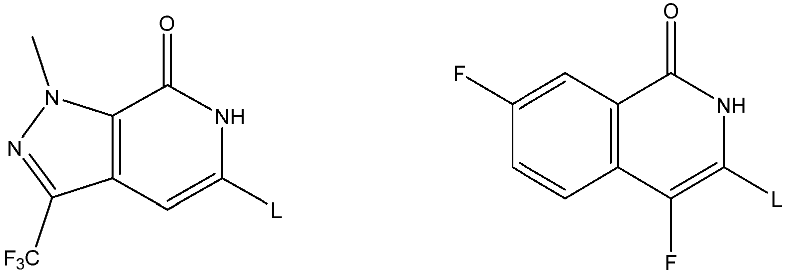

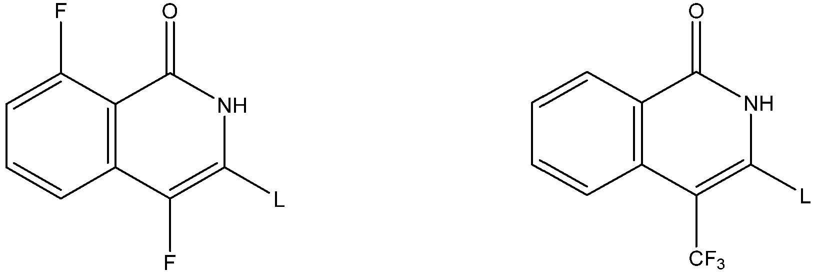

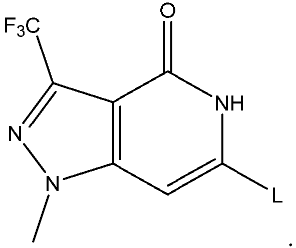

20. Verbindung zur Verwendung nach einem vorhergehenden Anspruch, wobei diese Verbindung eine Formel umfasst, die aus einer der folgenden ausgewählt ist:

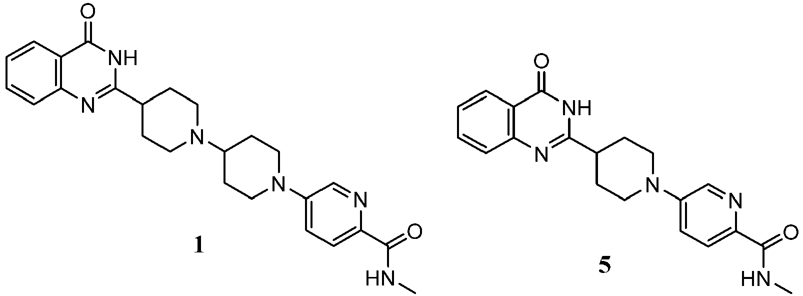

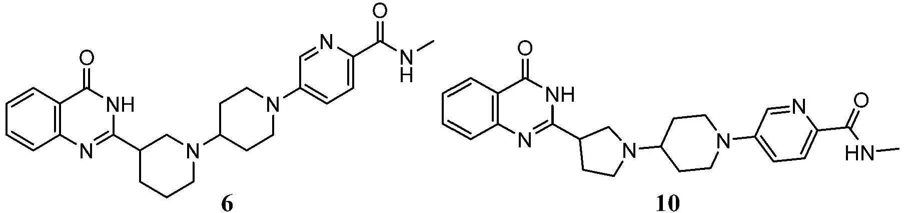

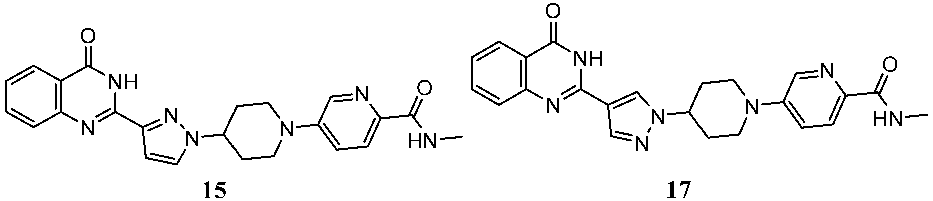

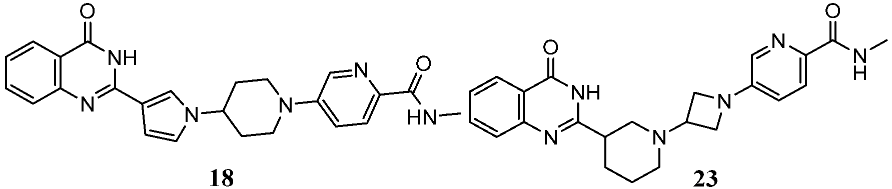

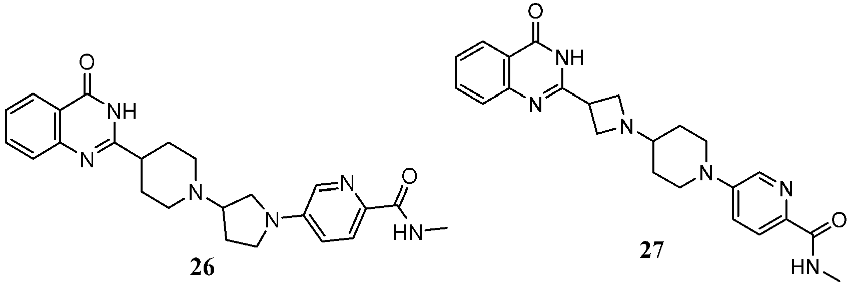

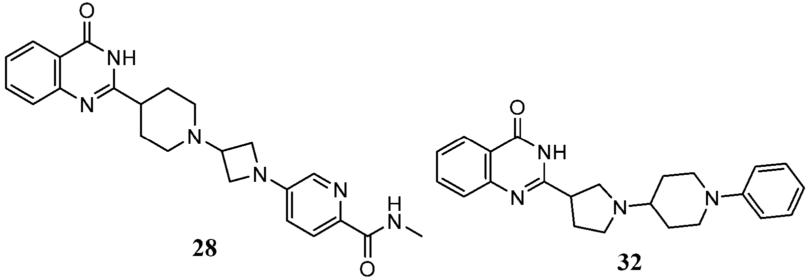

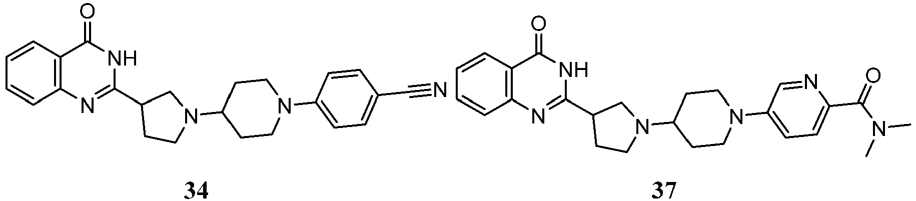

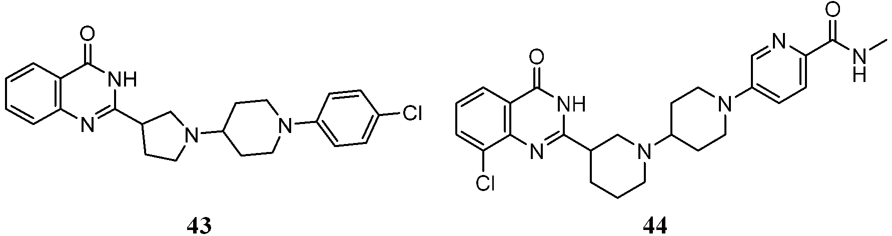

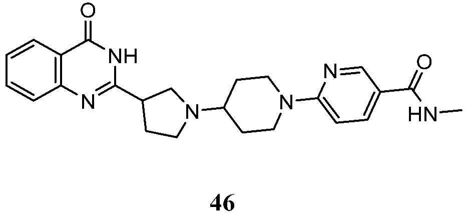

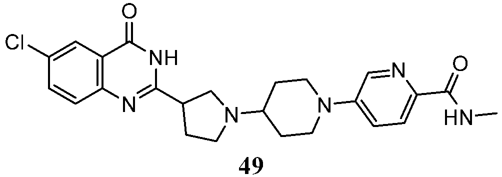

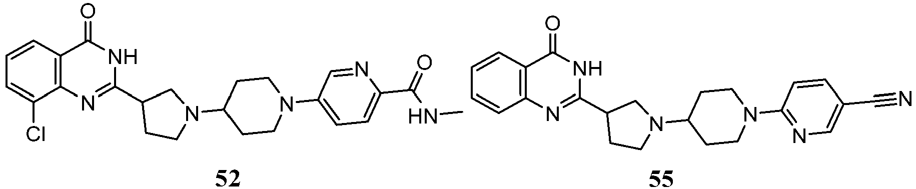

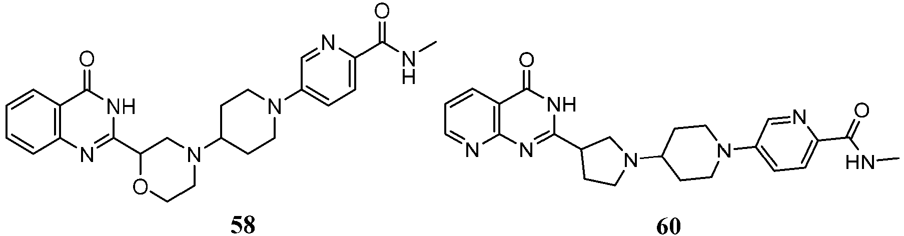

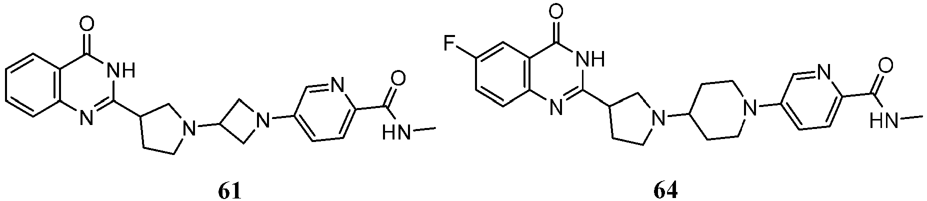

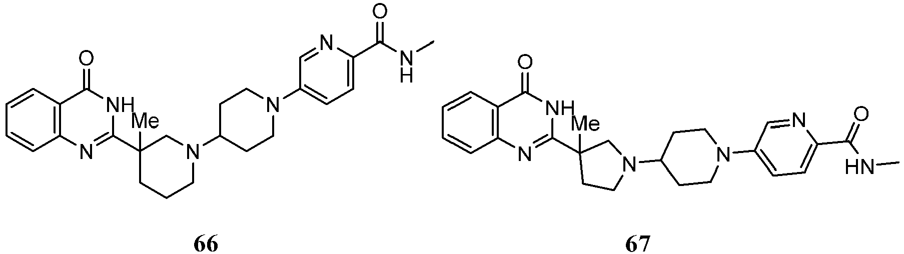

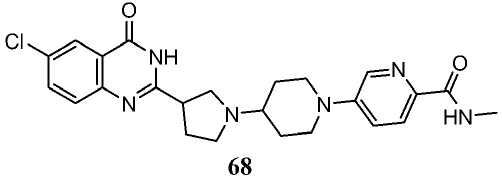

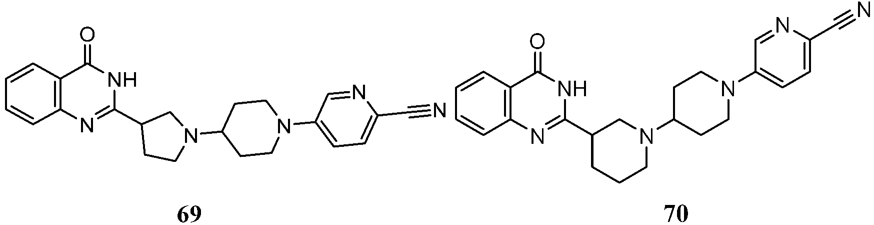

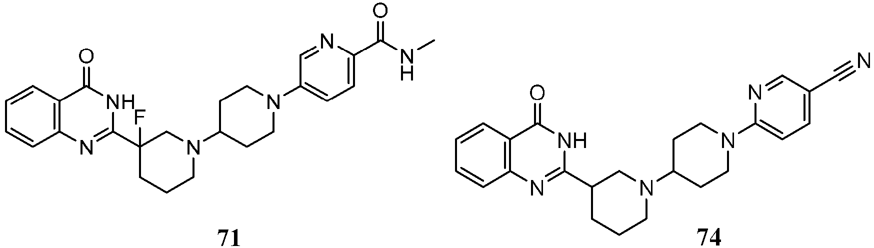

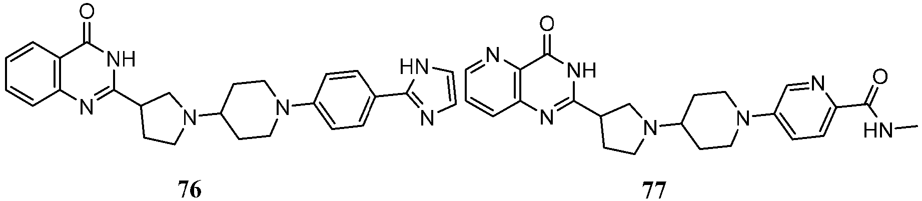

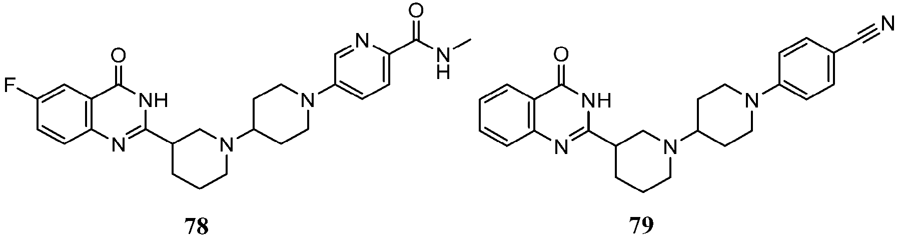

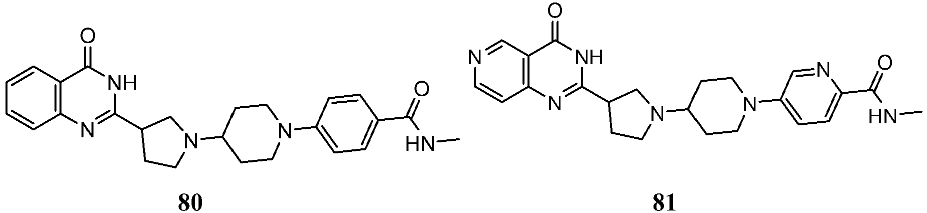

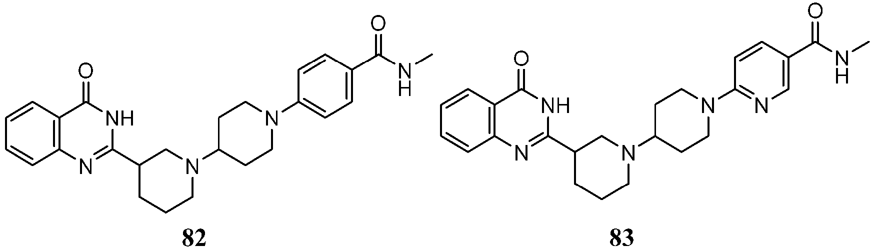

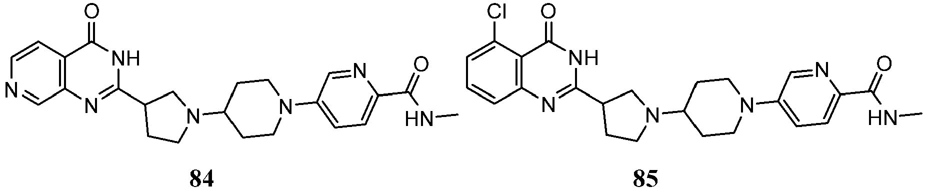

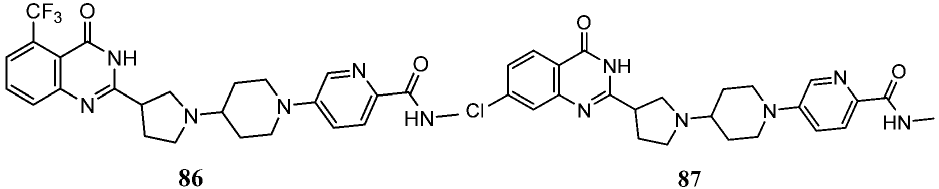

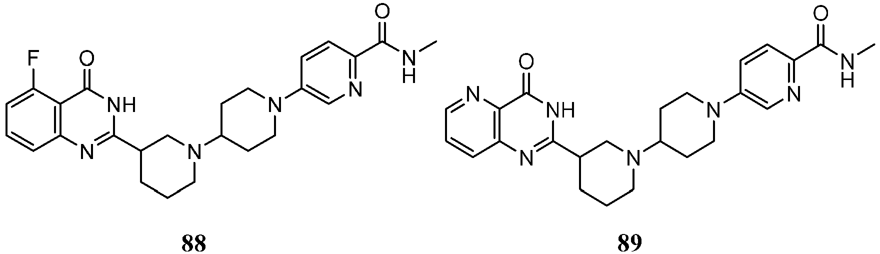

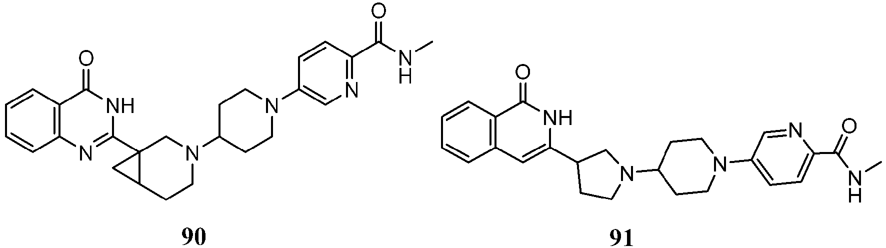

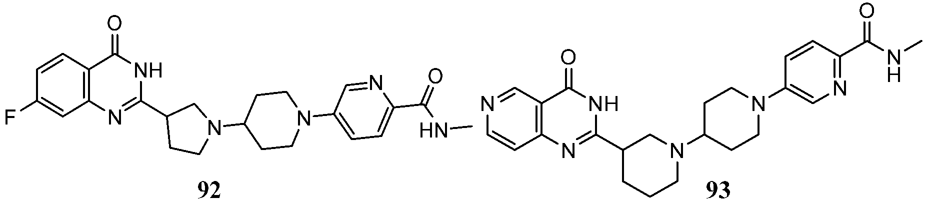

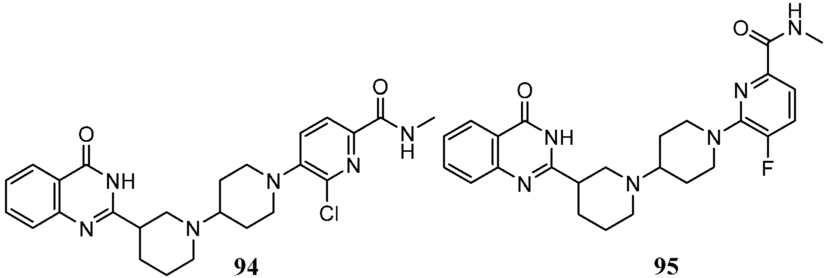

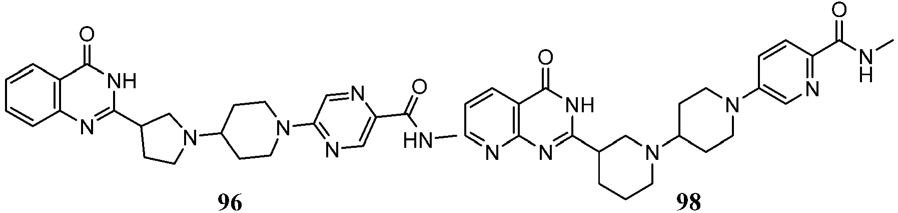

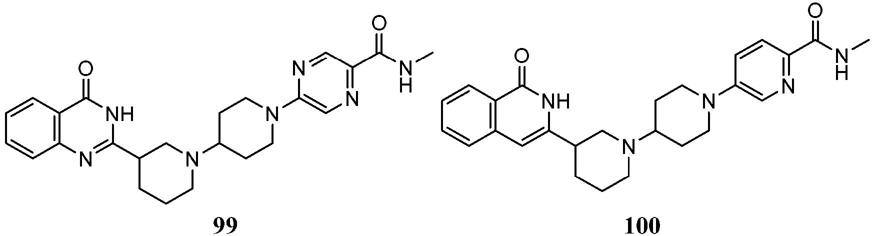

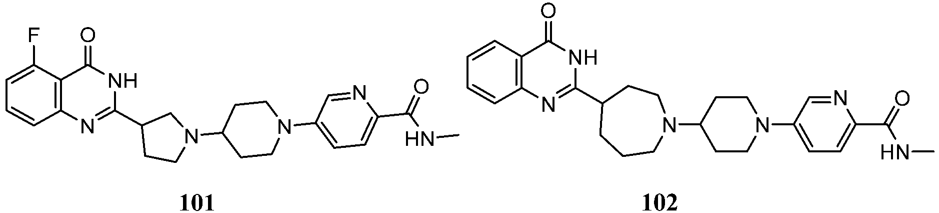

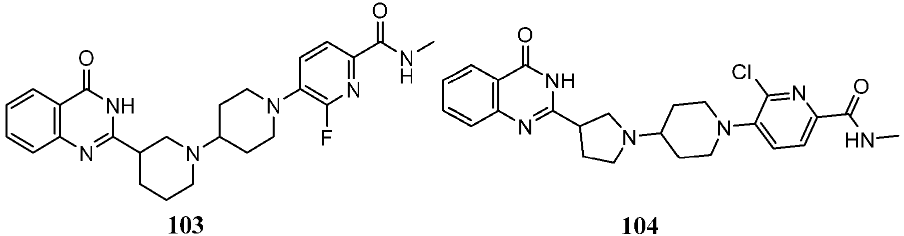

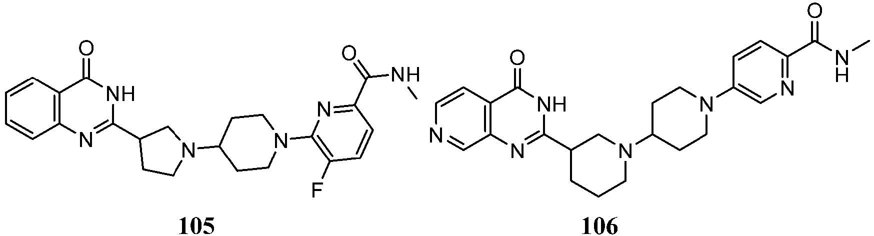

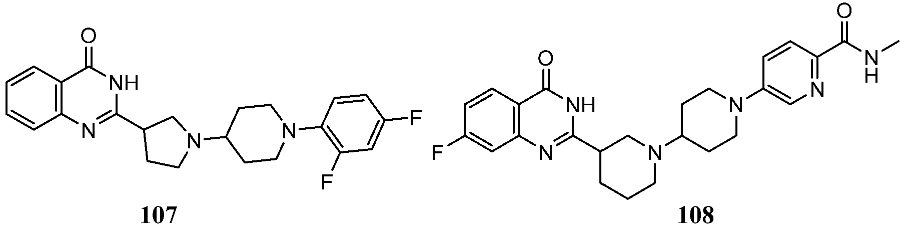

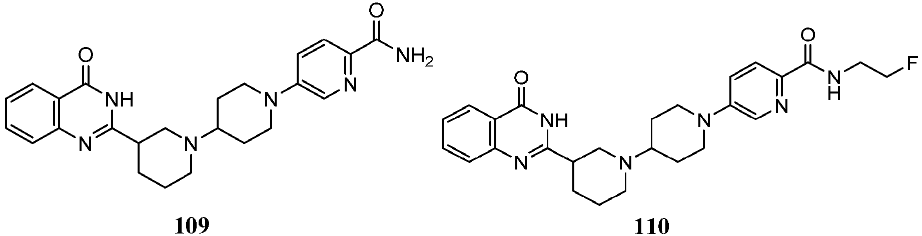

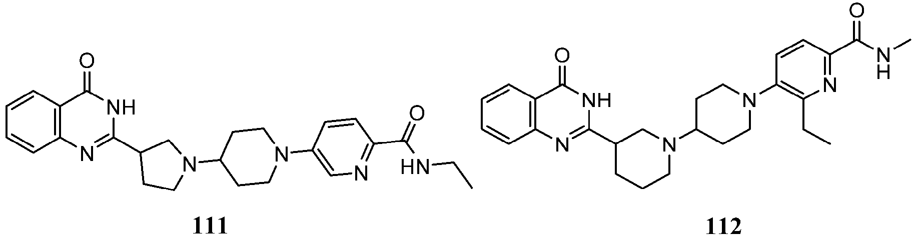

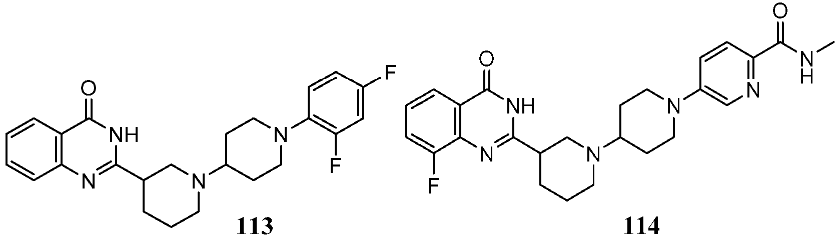

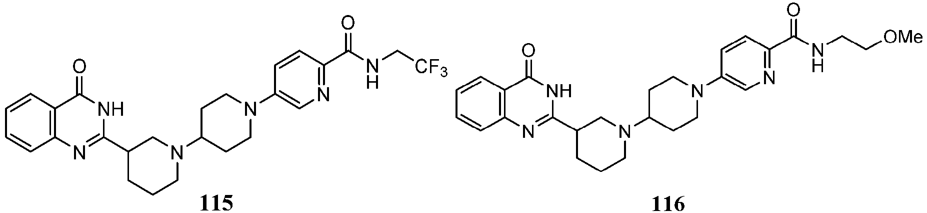

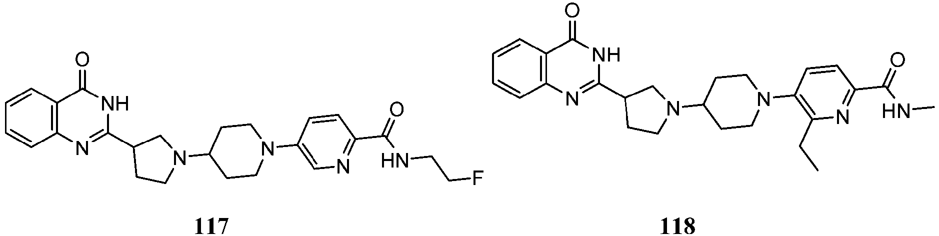

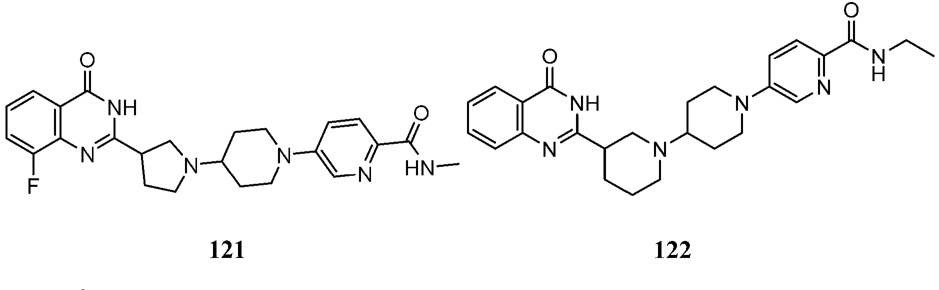

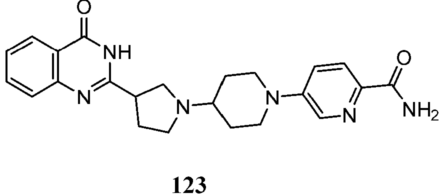

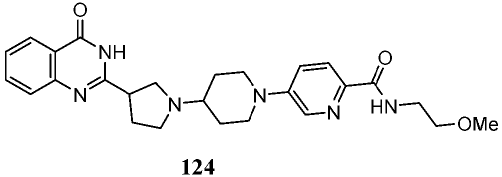

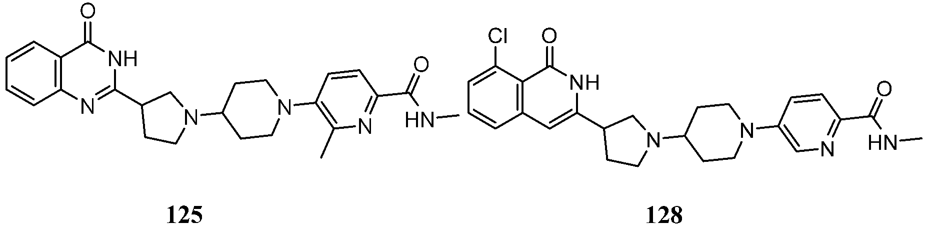

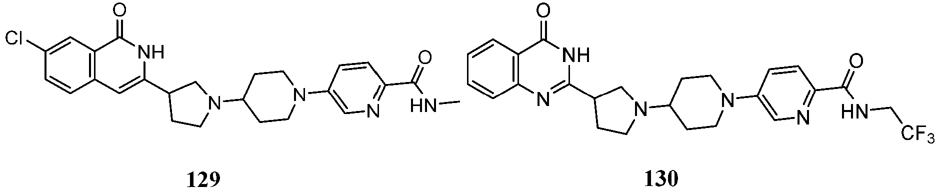

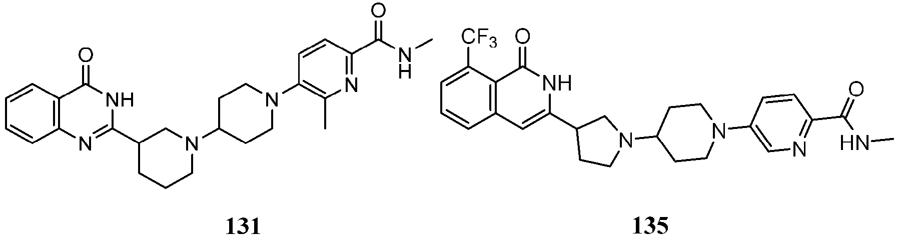

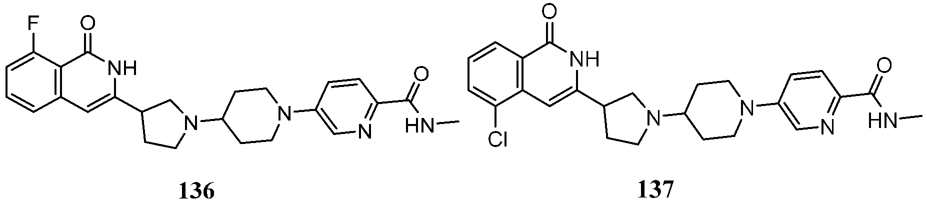

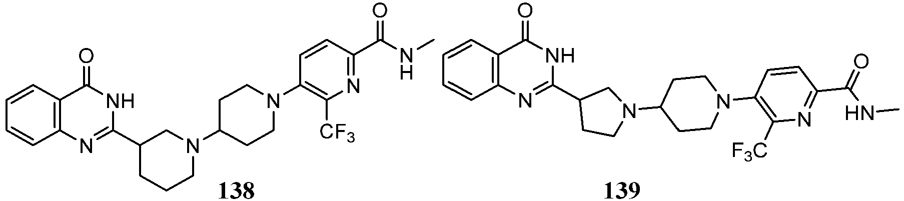

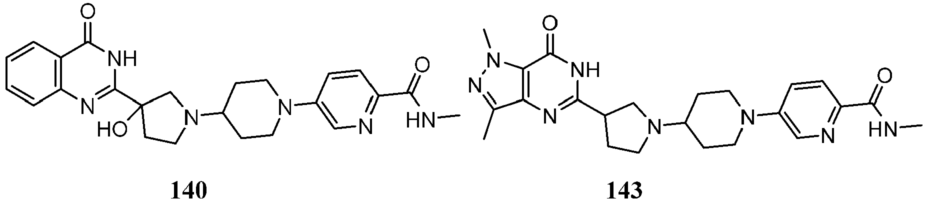

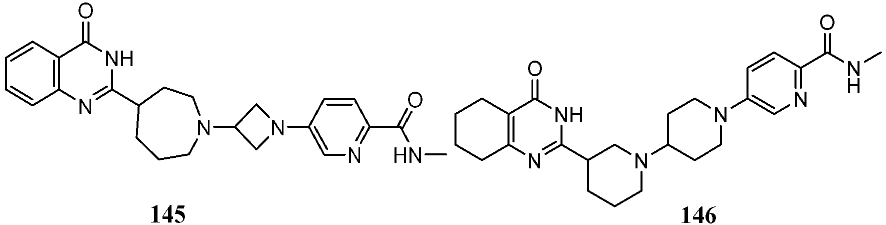

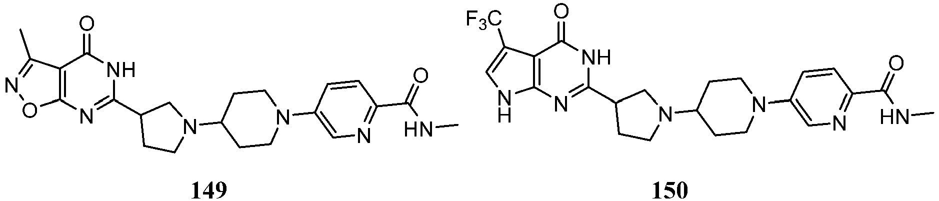

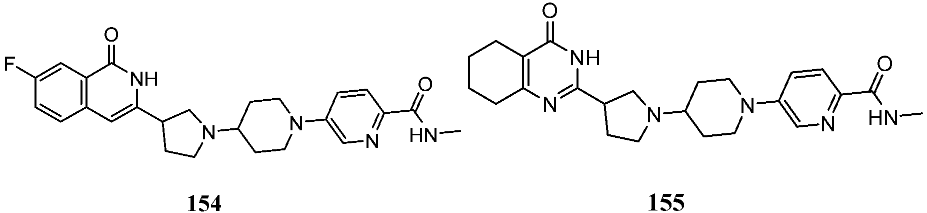

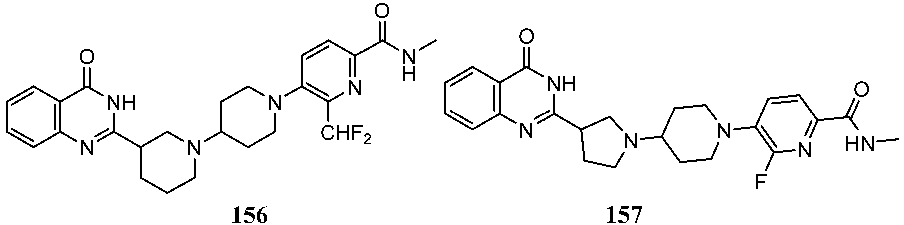

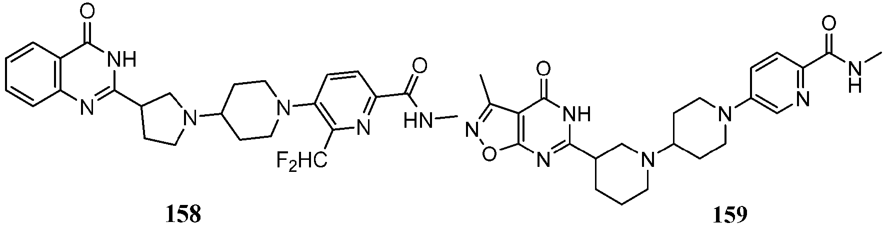

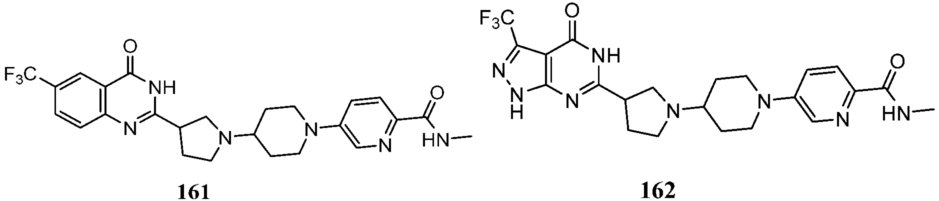

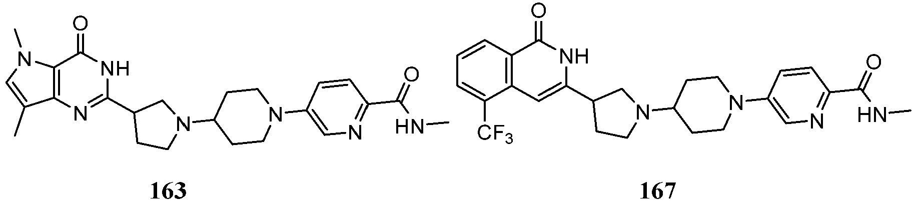

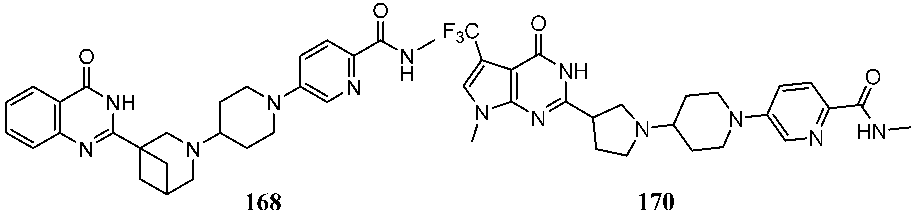

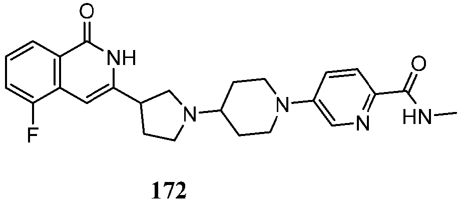

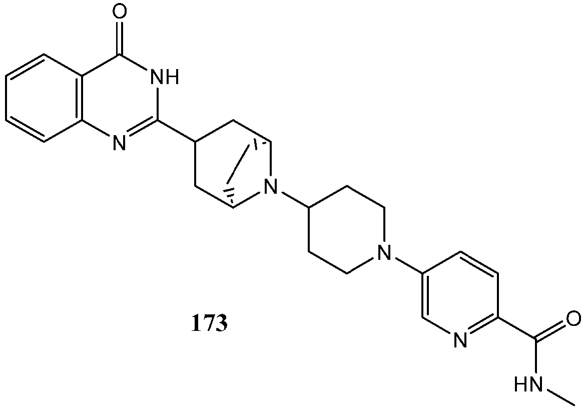

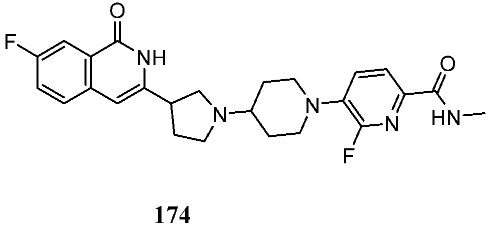

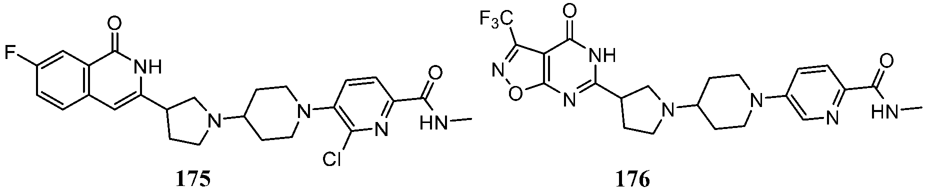

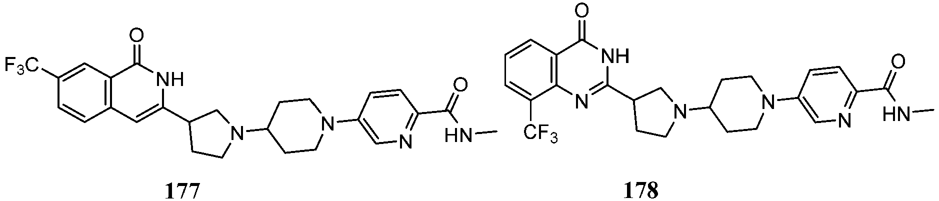

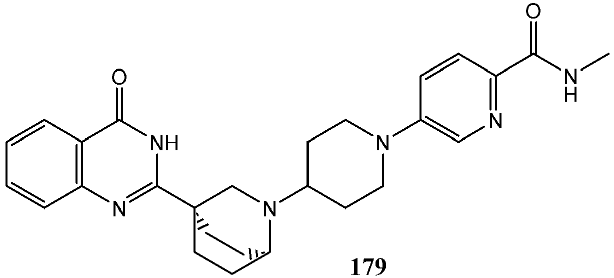

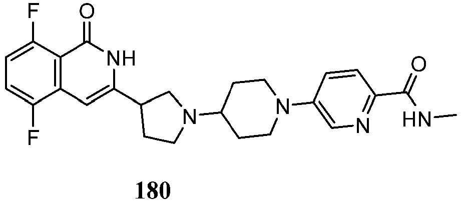

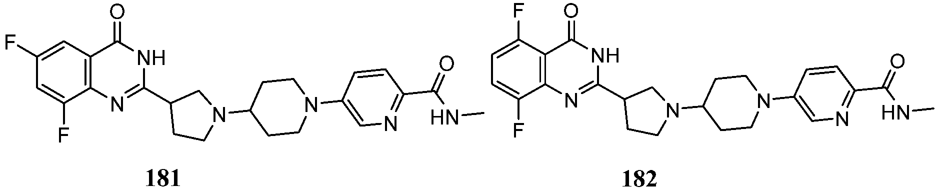

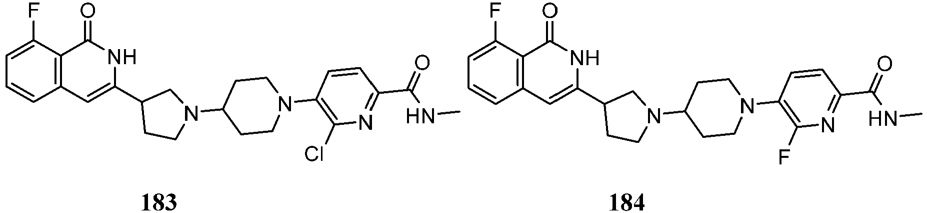

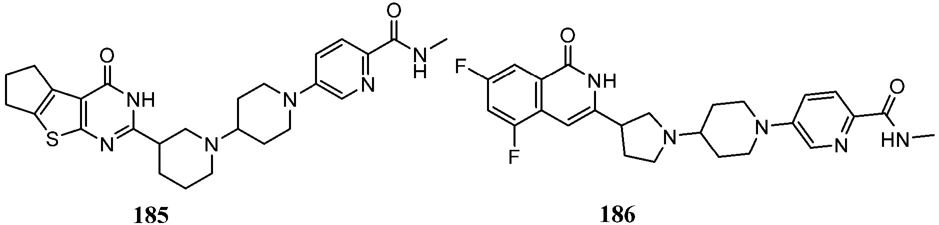

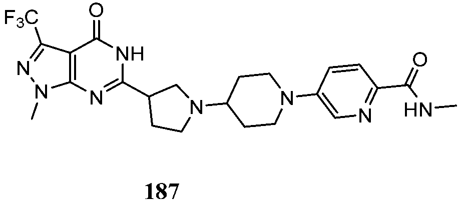

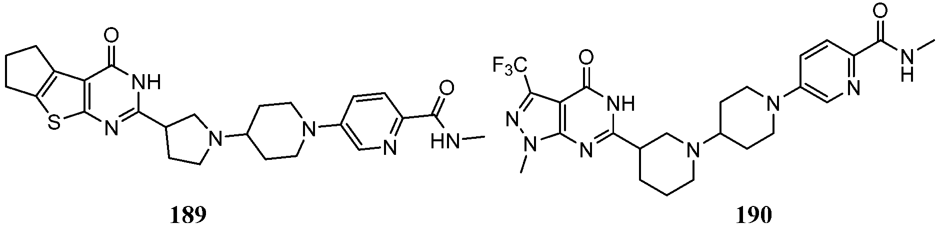

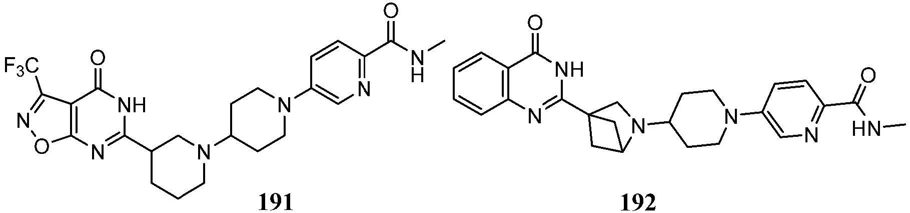

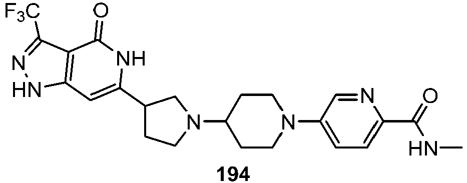

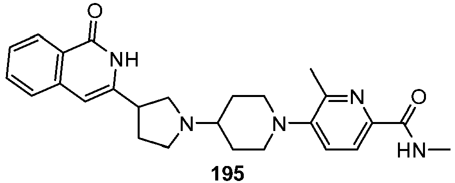

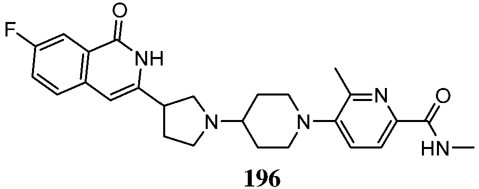

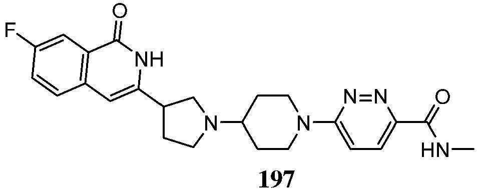

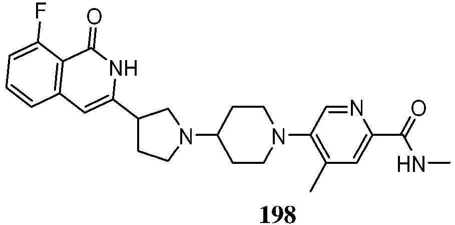

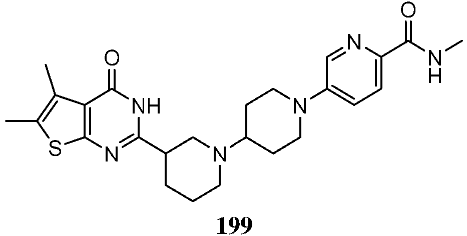

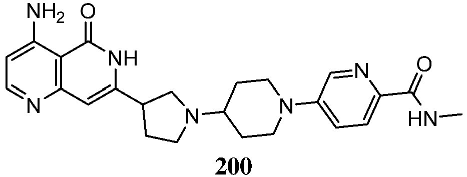

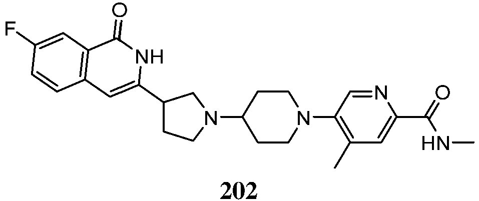

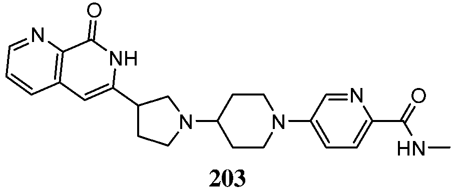

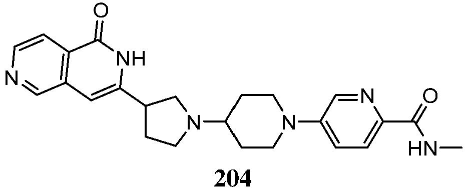

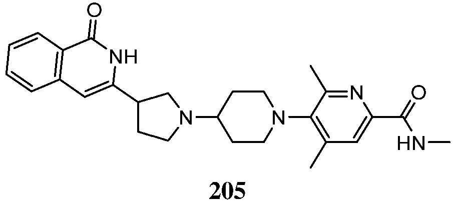

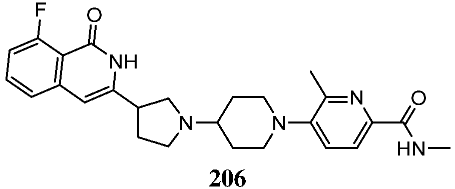

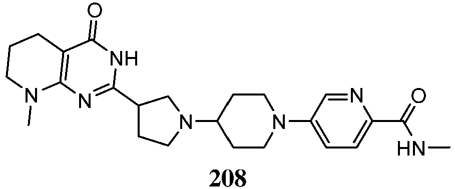

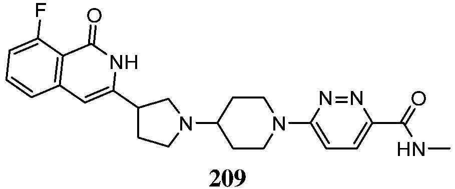

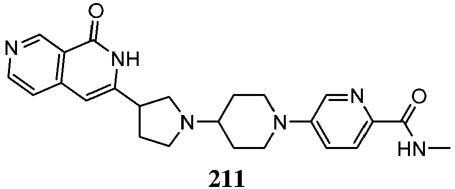

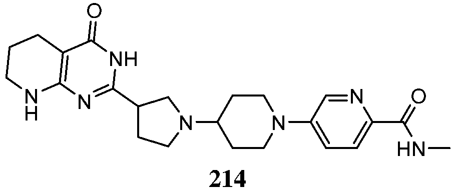

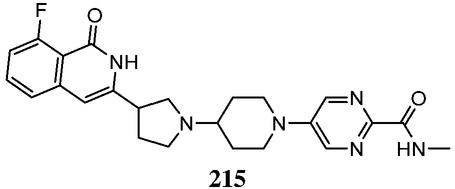

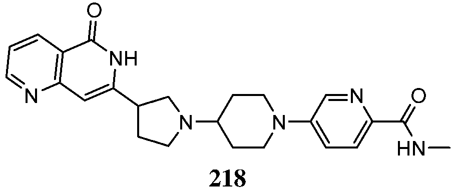

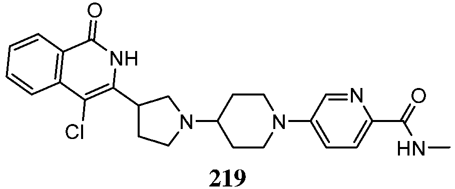

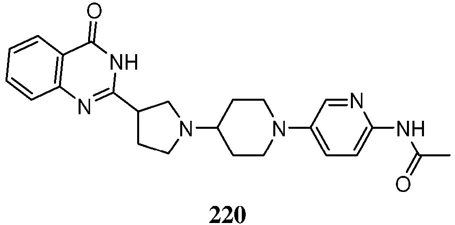

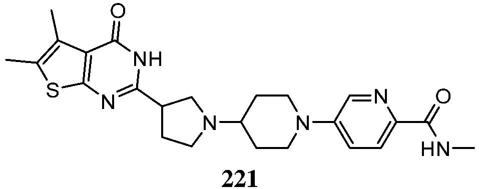

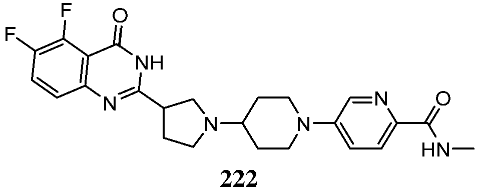

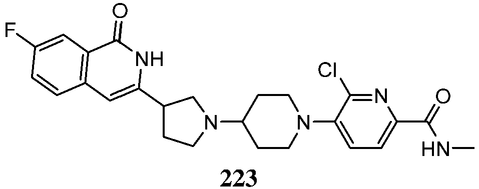

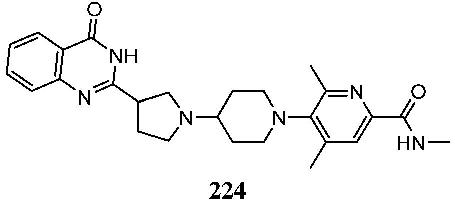

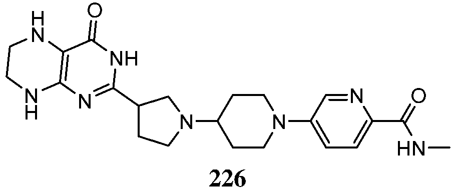

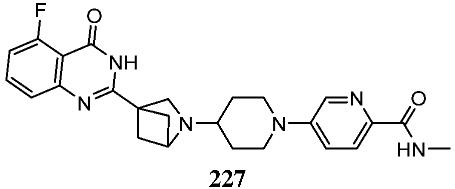

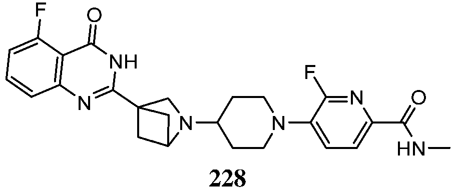

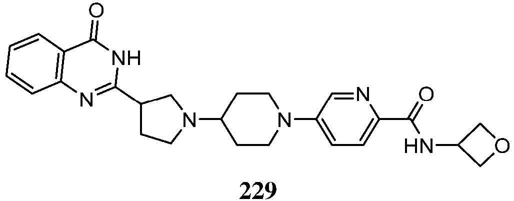

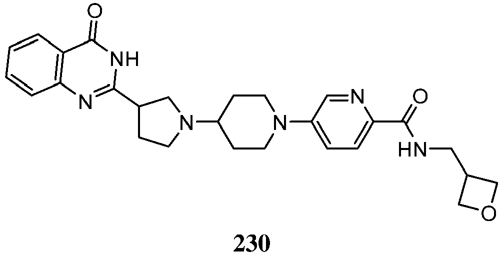

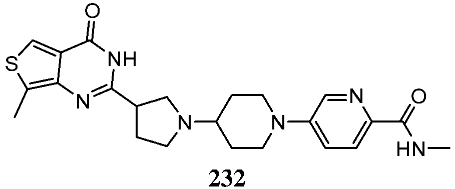

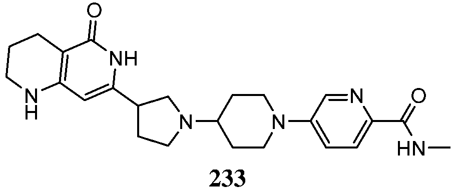

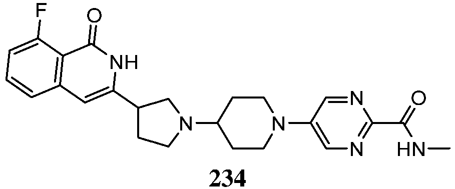

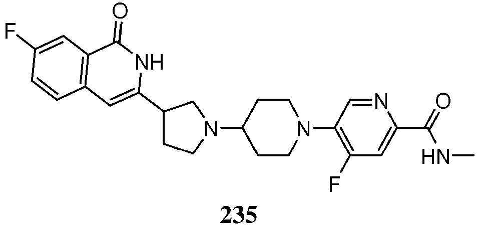

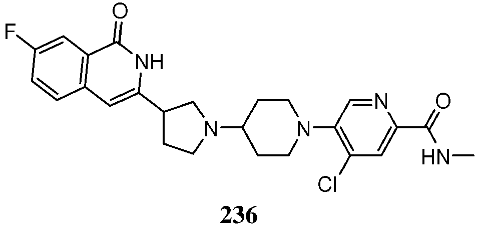

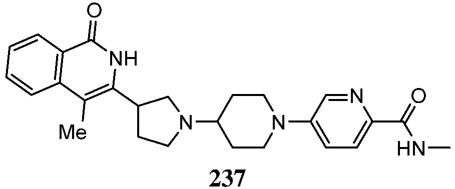

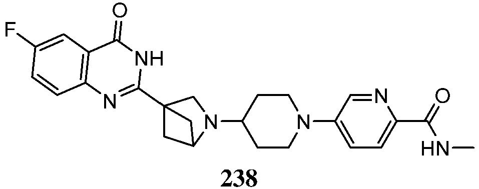

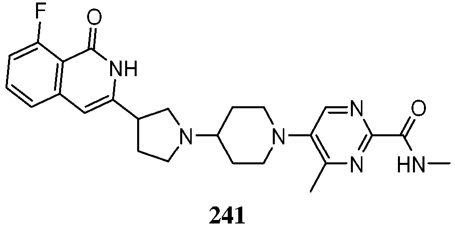

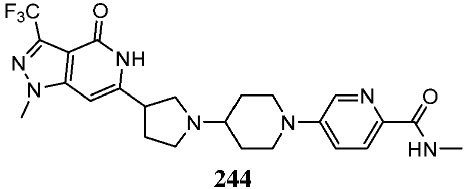

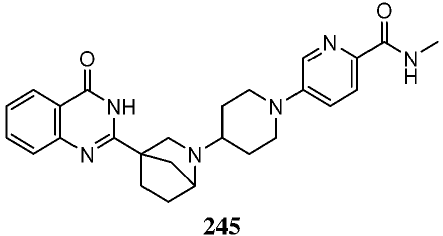

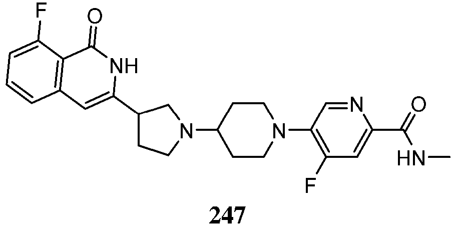

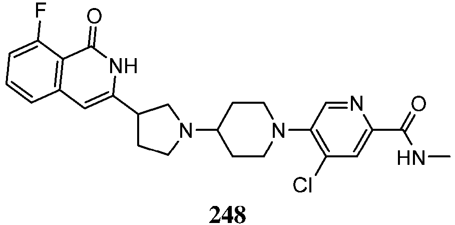

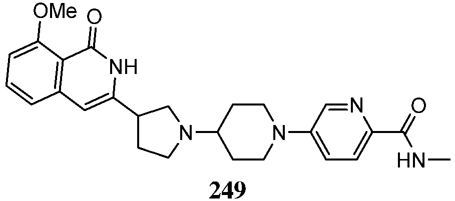

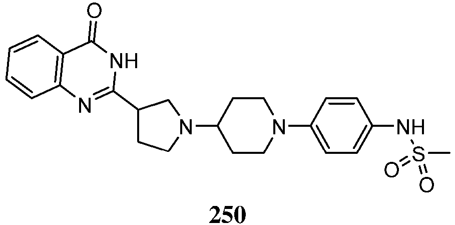

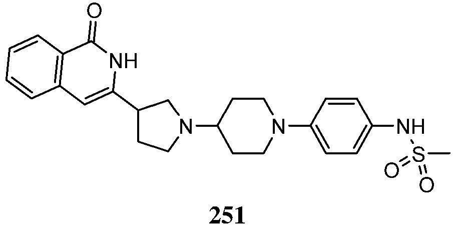

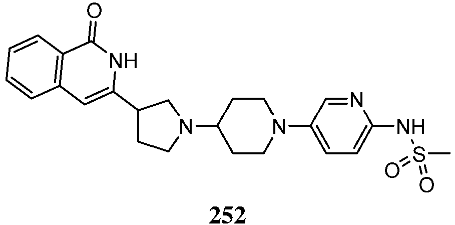

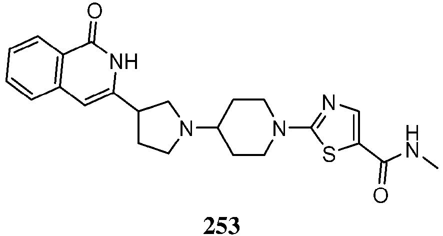

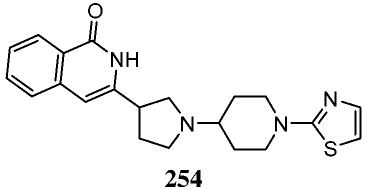

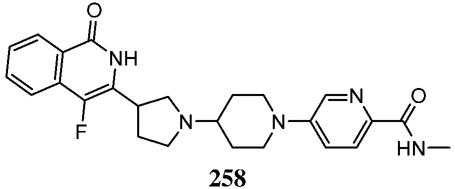

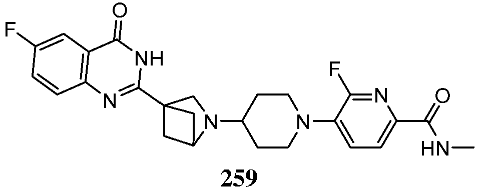

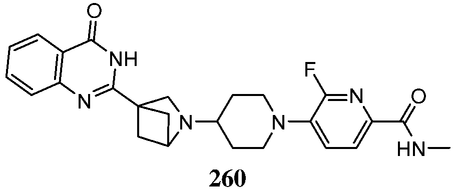

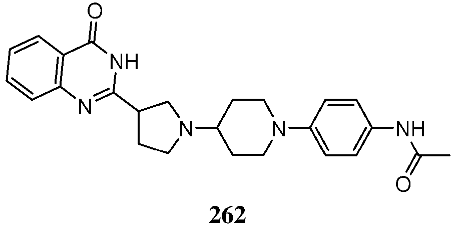

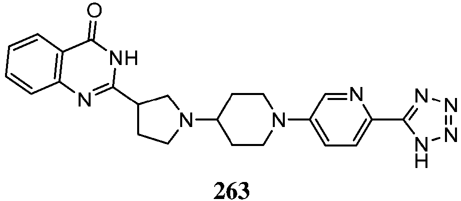

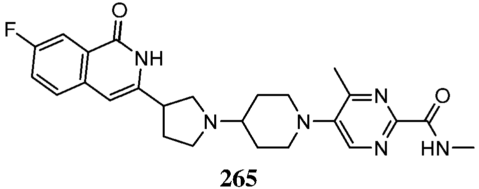

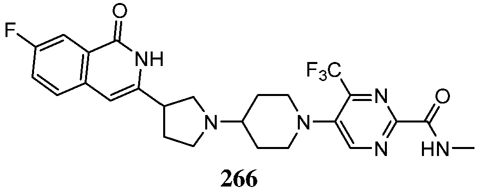

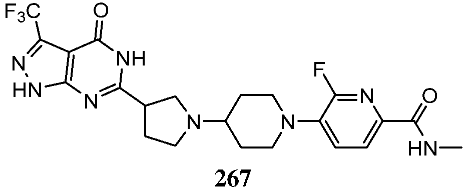

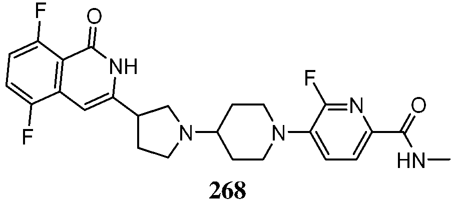

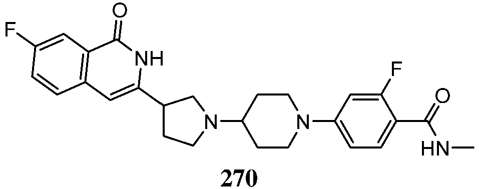

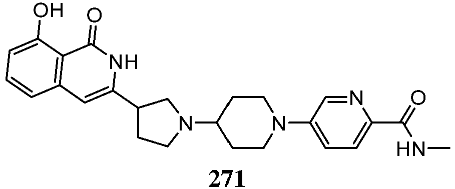

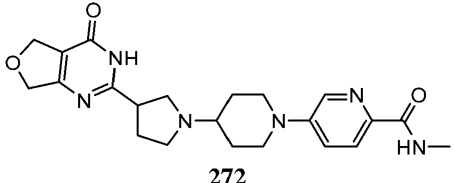

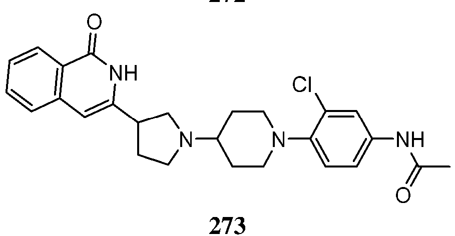

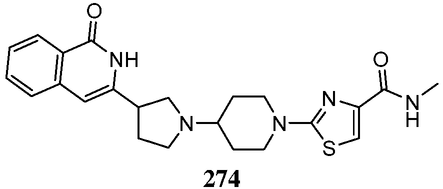

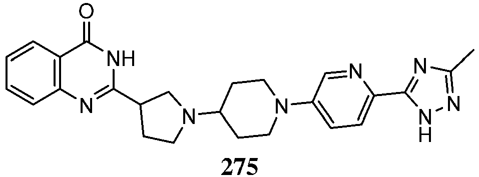

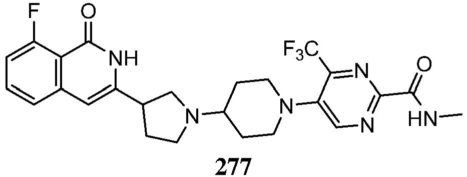

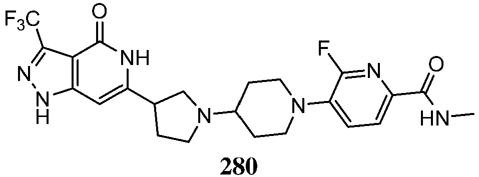

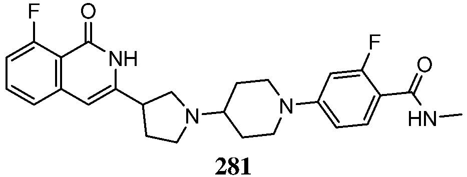

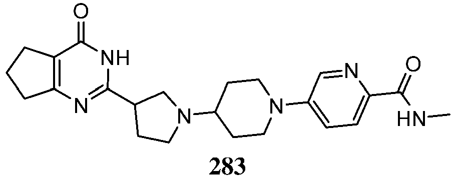

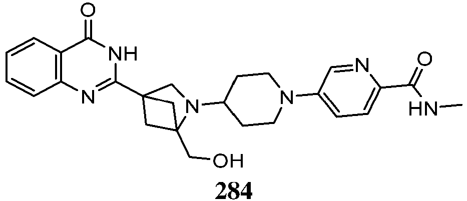

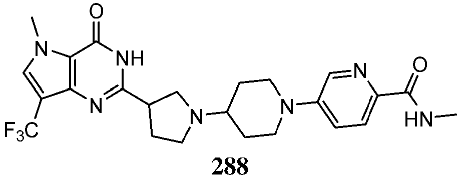

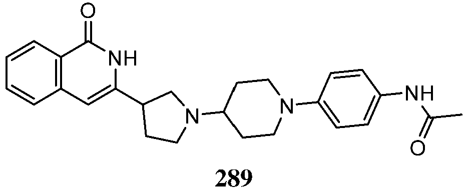

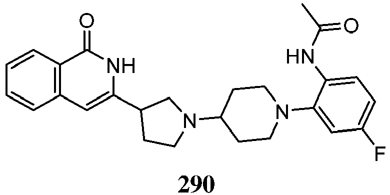

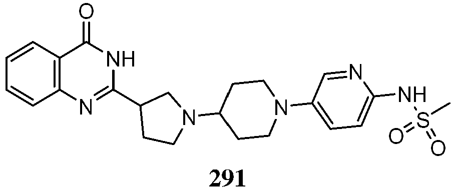

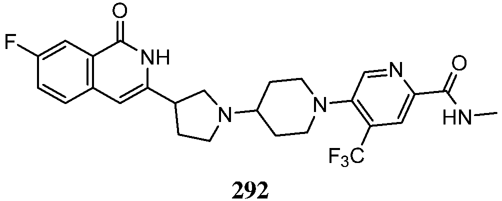

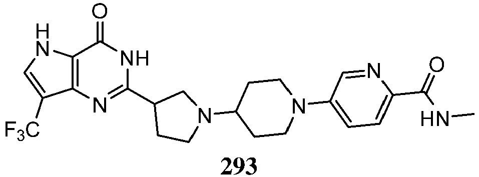

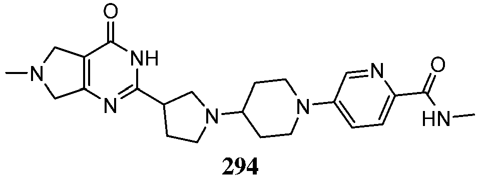

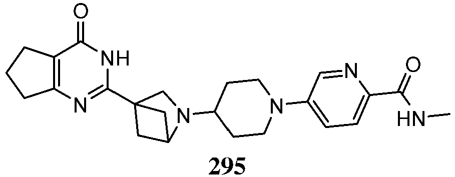

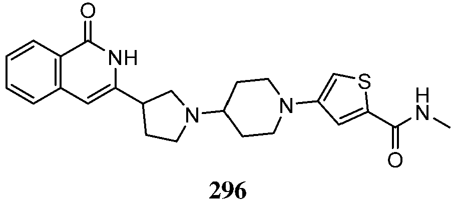

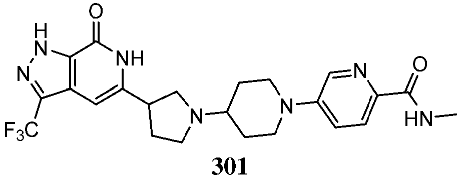

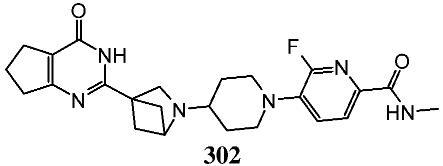

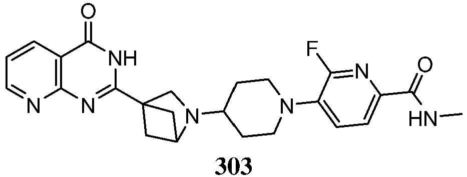

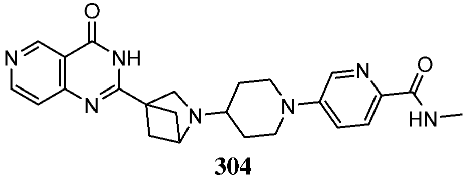

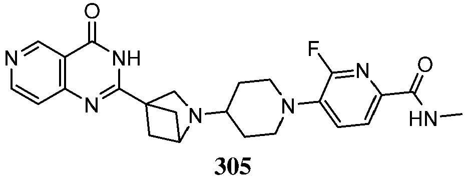

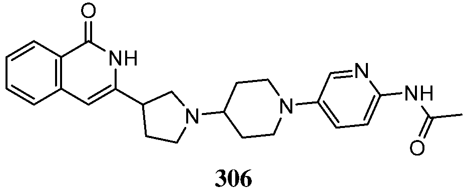

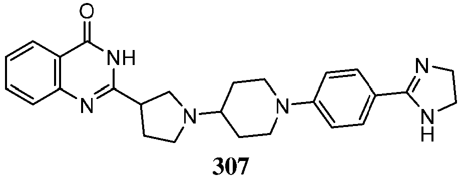

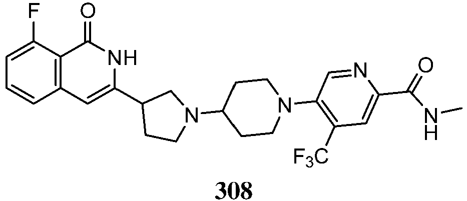

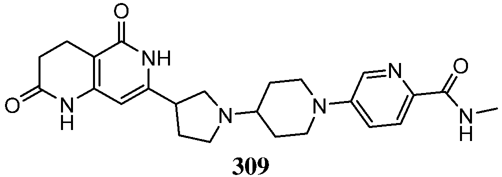

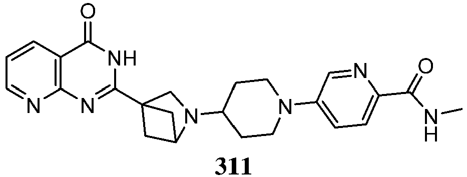

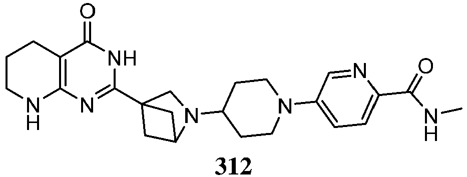

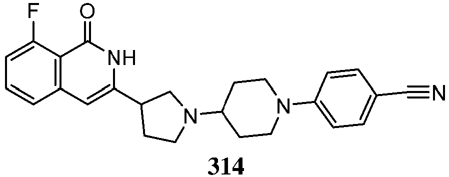

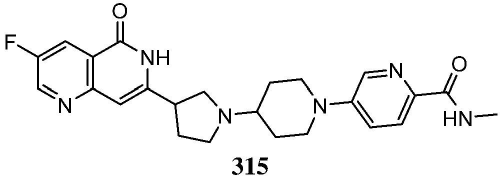

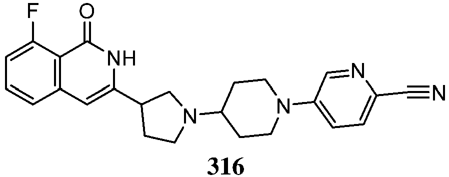

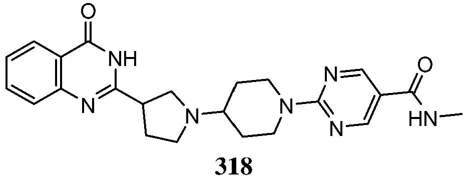

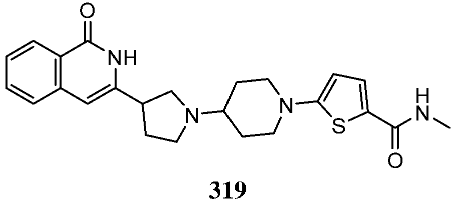

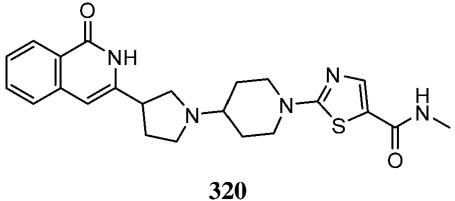

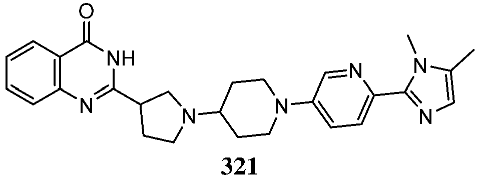

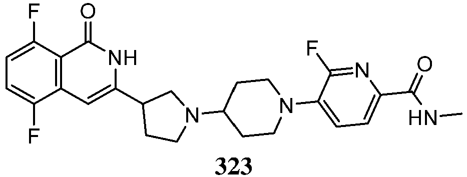

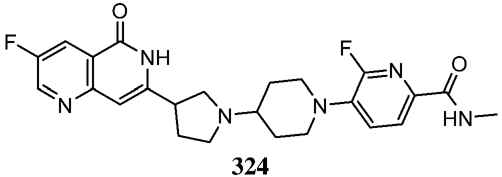

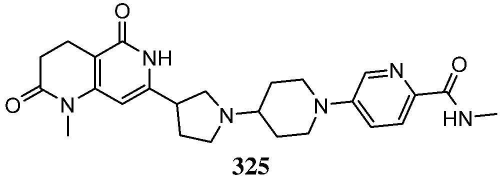

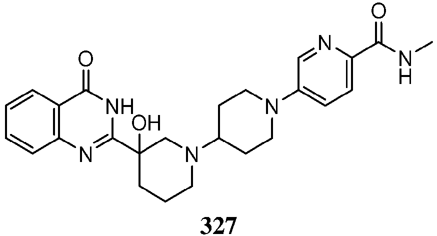

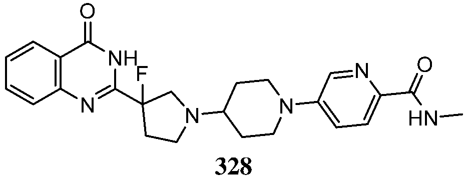

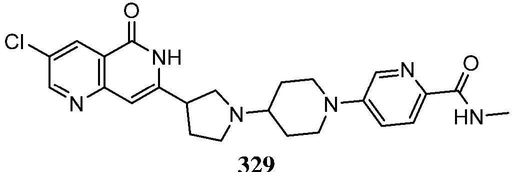

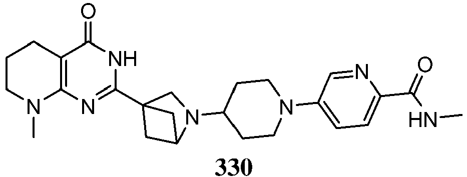

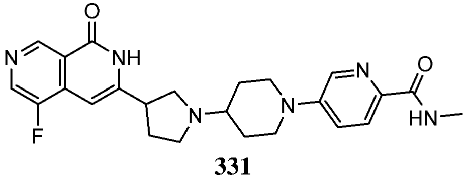

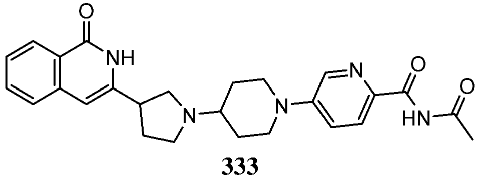

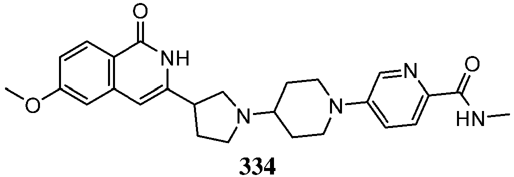

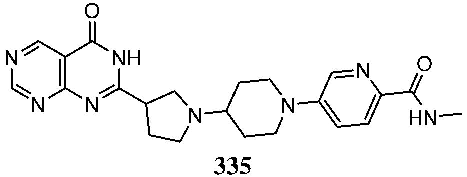

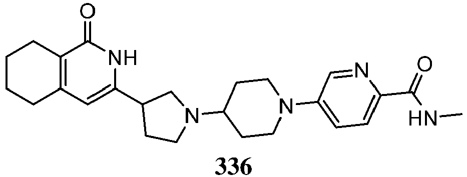

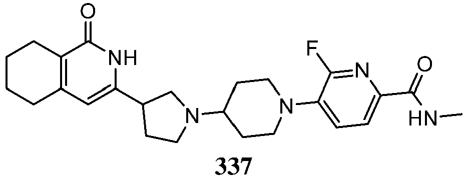

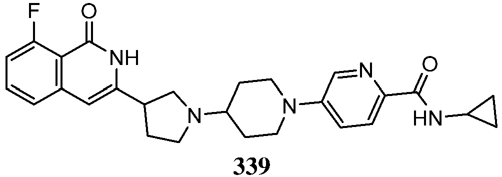

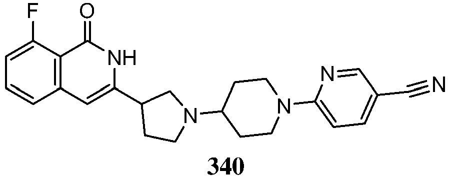

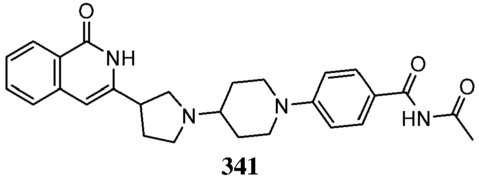

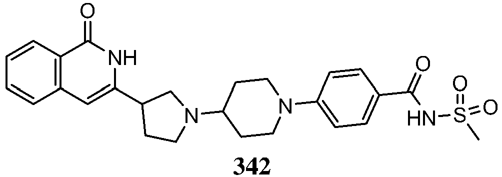

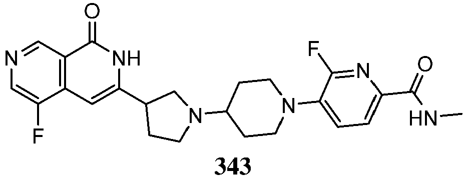

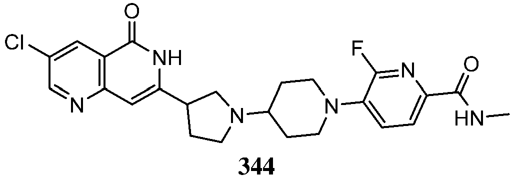

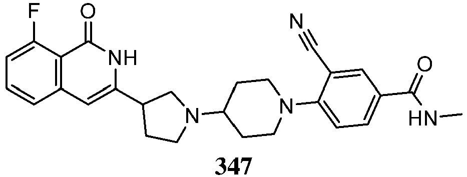

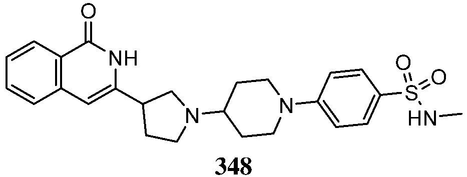

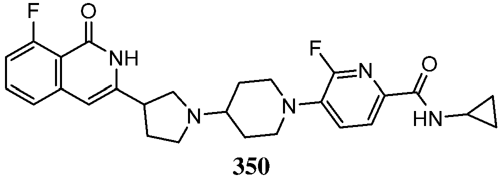

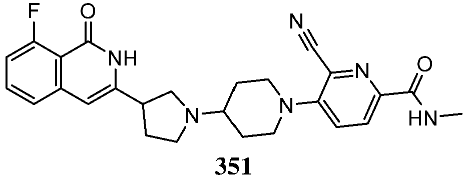

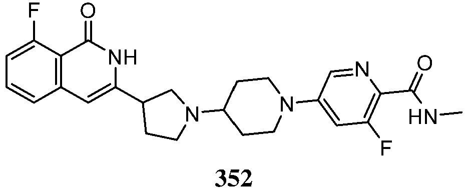

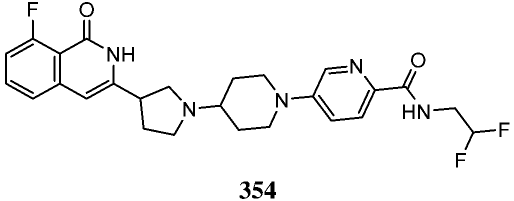

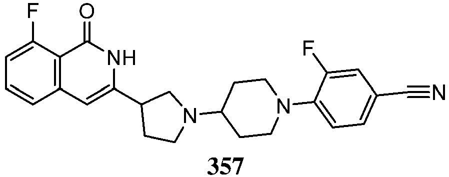

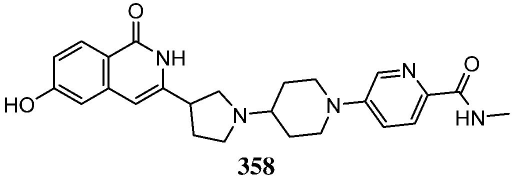

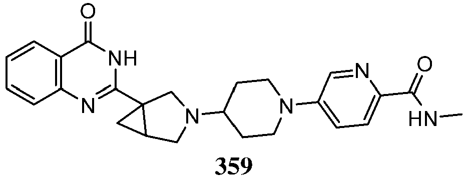

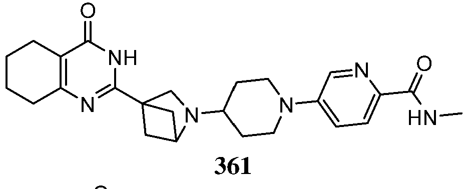

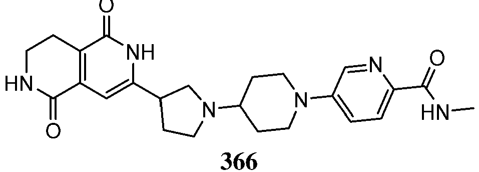

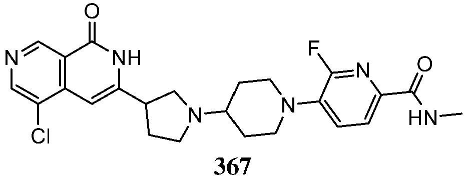

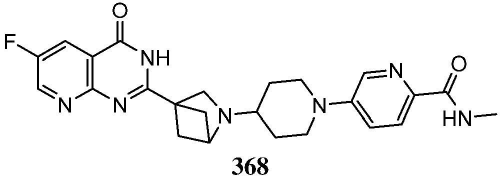

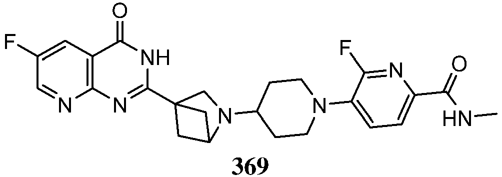

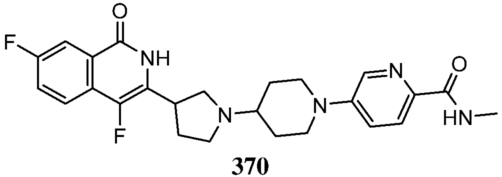

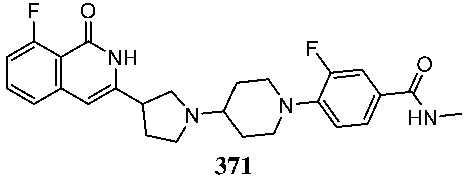

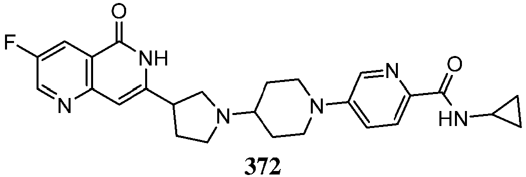

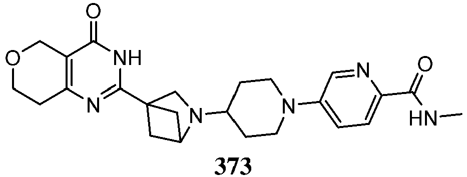

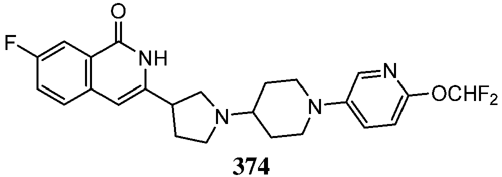

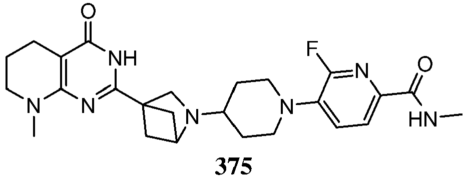

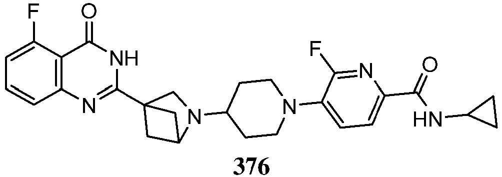

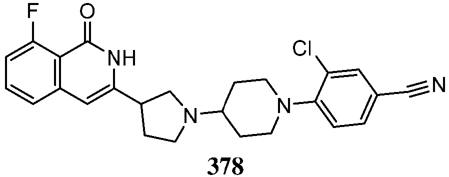

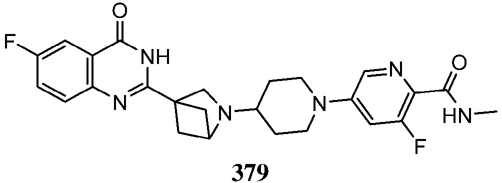

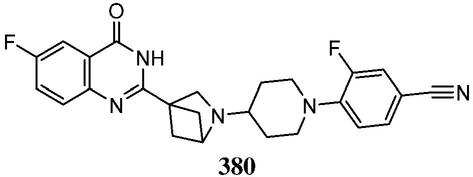

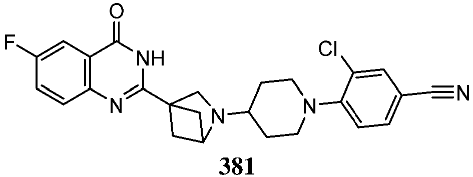

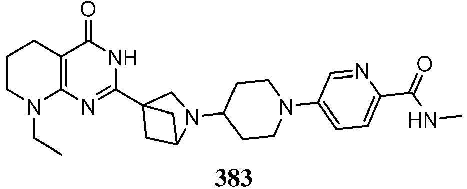

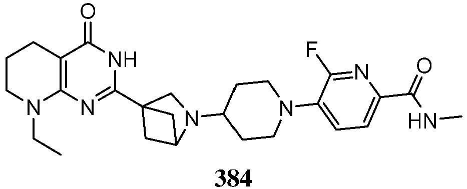

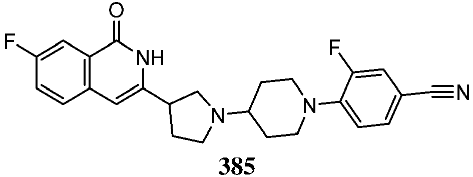

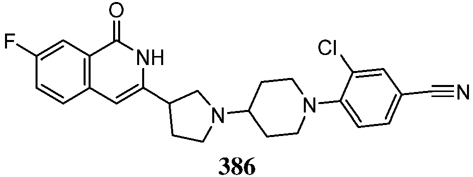

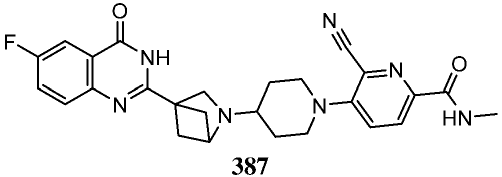

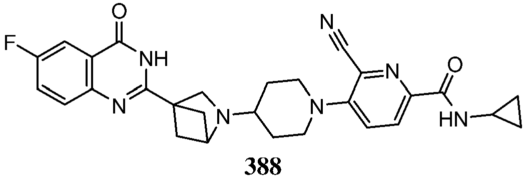

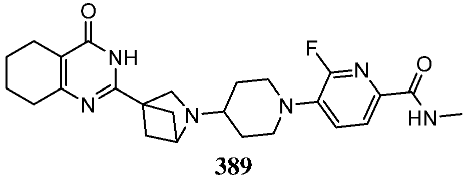

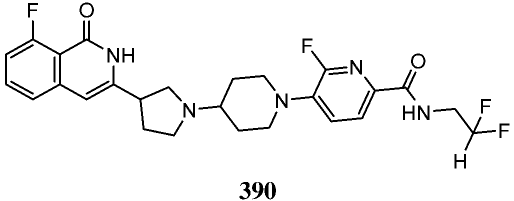

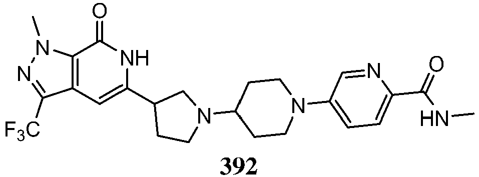

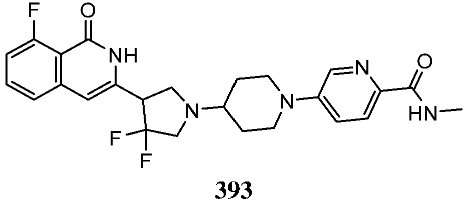

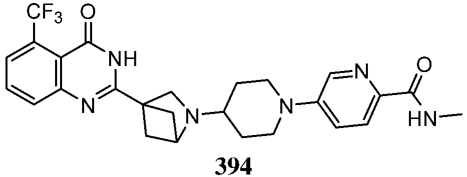

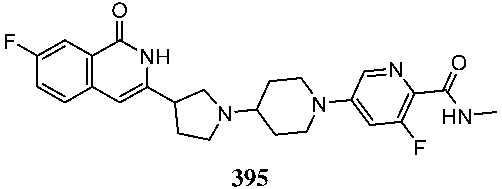

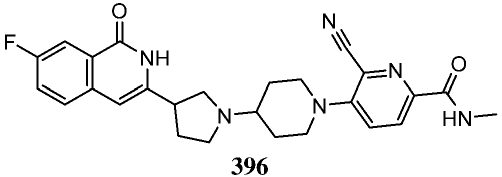

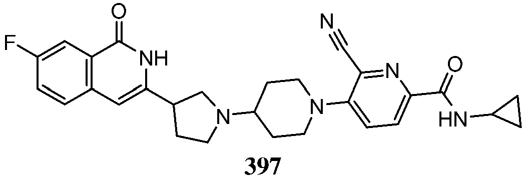

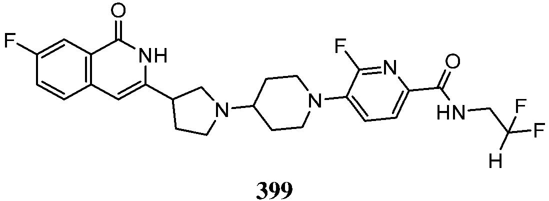

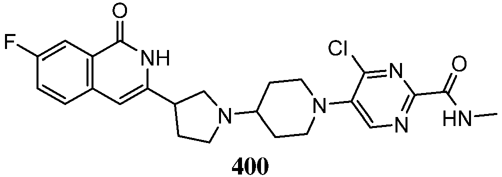

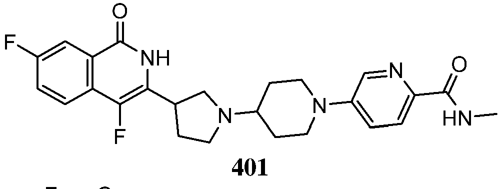

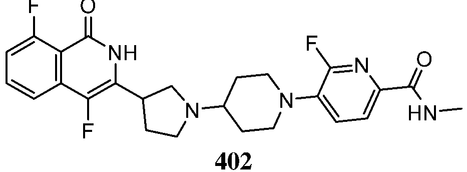

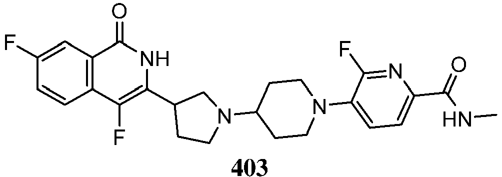

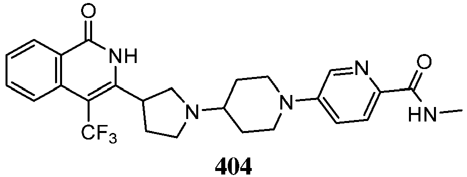

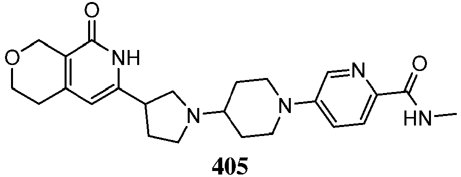

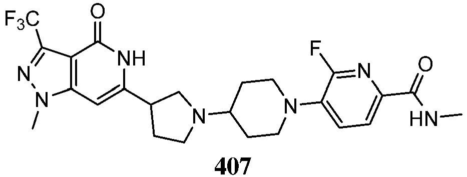

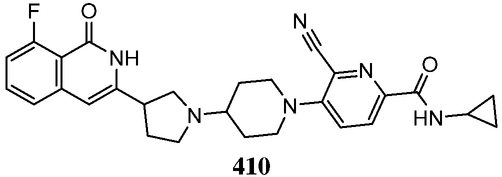

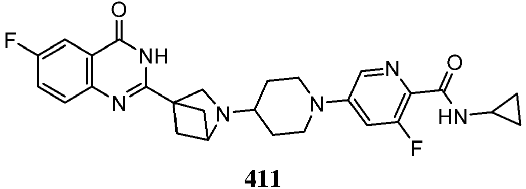

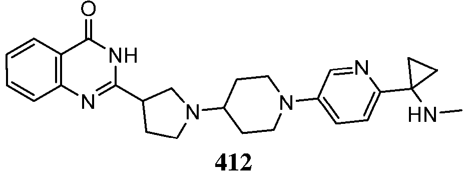

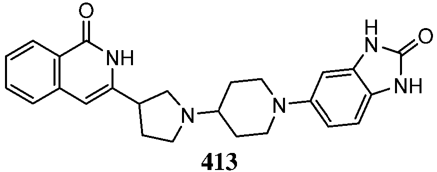

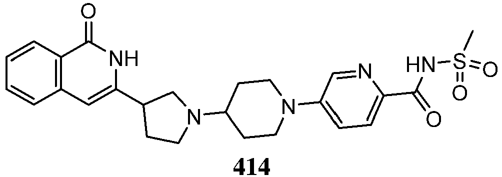

21. Verbindung zur Verwendung nach einem vorhergehenden Anspruch, wobei diese Verbindung umfasst:
- ein isoliertes Enantiomer, oder
- ein Gemisch von zwei oder mehr Enantiomeren, oder
- ein Gemisch von zwei oder mehr Diastereomeren und/oder Epimeren, oder
- ein racemisches Gemisch, oder
- ein Tautomer der Verbindung.

22. Verbindung zur Verwendung nach einem vorhergehenden Anspruch, die für PARP1 gegenüber PARP2 selektiv ist.

23. Pharmazeutische Zusammensetzung, umfassend eine wie in einem vorhergehenden Anspruch definierte Verbindung.

24. Pharmazeutische Zusammensetzung nach Anspruch 23, ferner umfassend einen pharmazeutisch unbedenklichen Zusatzstoff und/oder Hilfsstoff, und/oder wobei die Verbindung in der Form eines pharmazeutisch unbedenklichen Salzes, eines pharmazeutisch unbedenklichen Hydrats, einer pharmazeutisch unbedenklichen Säure, eines pharmazeutisch unbedenklichen Esters oder einer anderen alternativen Form der Verbindung ist.

25. Verbindung zur Verwendung nach einem der Ansprüche 1 bis 22 oder pharmazeutische Zusammensetzung nach Anspruch 22 oder Anspruch 23, die zur Verwendung bei der Behandlung eines Krebses ist.

26. Verbindung oder pharmazeutische Zusammensetzung zur Verwendung nach Anspruch 25, wobei:
i) der Krebs ein Krebs ist, der ausgewählt ist aus: einem soliden oder flüssigen Tumor, einschließend Augen-, Hirn-, Rückenmarks-, Mund-, Lippen-, Rachen-, Mundhöhlen-, Nasenhöhlen-, Dünndarm-, Dickdarm-, Nebenschilddrüsen-, Gallenblasen-, Kopf-Hals-, Brust-, Knochen, Gallengangs-, Gebärmutterhals-, Herz-, Hypopharynx-, Lungen-, Bronchial-, Leber-, Haut-, Harnleiter-, Harnröhren-, Hoden-, Vaginal-, Anal-, Kehlkopf-, Eierstock-, Schilddrüsen-, Speiseröhren-, Nasopharynx-, Hypophysen-, Speicheldrüsen-, Prostata-, Bauchspeicheldrüsen-, Nebennierenkrebs; einem Endometrialkrebs, Oralkrebs, Melanom, Neuroblastom, Magenkrebs, einer Angiomatose, einem Hämangioblastom, einem Phäochromozytom, einer Bauchspeicheldrüsenzyste, einem Nierenzellkarzinom, Wilms- Tumor, Plattenepithelkarzinom, Sarkom, Osteosarkom, Kaposi-Sarkom, Rhabdomyosarkom, Leberzellkarzinom, PTEN-Hamartoma-Tumor-Syndromen, Leukämien und Lymphomen, Nasopharynx- und Magen-Darm-Krebsen;
und/oder
ii) der Krebs defizient an einem DNA-Damage-Response-Repair-Weg ist, wie von homologer Rekombination abhängige DNA-Doppelstrangbruch-DNA-Reparaturaktivität; und/oder
iii) der Krebs defizient an BRCA1- und/oder BRCA2-Funktion ist.

27. Pharmazeutische Zusammensetzung zur Verwendung nach Anspruch 25 oder Anspruch 26, ferner umfassend ein weiteres Mittel zur Behandlung von Krebs;
optional wobei das weitere Mittel zur Behandlung von Krebs aus gegen Mikrotubuli wirkenden Mitteln, Platinkoordinationskomplexen, Alkylierungsmitteln, Antibiotika, Topoisomerase-II-Inhibitoren, Antimetaboliten, Topoisomerase-I-Inhibitoren, Senolytika, Hormonen und Hormon-Analoga, Signaltransduktionsweg-Inhibitoren, anderen DNA-Damage-Repair-Weg-Inhibitoren, Nicht-Rezeptor-Tyrosinkinase-Angiogenese-Inhibitoren, Antikörper-Wirkstoff-Konjugaten, Immuntherapeutika, Hormonentzugstherapie, proapoptotischen Mitteln und Zellzyklussignalisierungsinhibitoren ausgewählt ist;
ferner optional wobei das weitere Mittel ein Immuntherapeutikum ist, das aus einem Impfstoff gegen Tumoren, einem onkolytischen Virus, einem immunstimulierenden Antikörper, wie Anti-CTLA4, Anti-PD1, Anti-PDL-1, Anti-OX40, Anti-41BB, Anti-CD27, Anti-CD40, Anti-LAG3, Anti-TIM3 und Anti-GITR, einem Mustererkennungsrezeptor-Agonisten, wie einem STING-, TLR-9- oder RIG-I-Helikase-Agonisten, einem IDO- oder TDO-Inhibitor, einem neuartigen Adjuvans, einem Peptid, einem Zytokin, einer chimären Antigenrezeptor-T-Zelltherapie, einem Kleinmolekül-Immunmodulator, Tumormikroumgebungsmodulatoren und antiangiogenen Mitteln ausgewählt ist.

28. Pharmazeutisches Kit zur Behandlung eines Krebses, wobei dieses pharmazeutische Kit umfasst:
a) eine wie in einem der Ansprüche 1 bis 22 definierte Verbindung; und
b) ein weiteres Mittel zur Behandlung von Krebs;
wobei die Verbindung und das weitere Mittel zur simultanen, sequenziellen oder separaten Verabreichung geeignet sind; und
vorzugsweise wobei das weitere Mittel zur Behandlung von Krebs aus gegen Mikrotubuli wirkenden Mitteln, Platinkoordinationskomplexen, Alkylierungsmitteln, Antibiotika, Topoisomerase-II-Inhibitoren, Antimetaboliten, Topoisomerase-I-Inhibitoren, Senolytika, Hormonen und Hormon-Analoga, Signaltransduktionsweg-Inhibitoren, anderen DNA-Damage-Repair-Weg-Inhibitoren, Nicht-Rezeptor-Tyrosinkinase-Angiogenese-Inhibitoren, Antikörper-Wirkstoff-Konjugaten, Hormonentzugstherapie, Immuntherapeutika proapoptotischen Mitteln und Zellzyklussignalisierungsinhibitoren ausgewählt ist.

29. Verbindung, die aus einer beliebigen der in Anspruch 20 definierten Verbindungen ausgewählt ist,
optional wobei die Verbindung umfasst:
- ein isoliertes Enantiomer, oder
- ein Gemisch von zwei oder mehr Enantiomeren, oder
- ein Gemisch von zwei oder mehr Diastereomeren und/oder Epimeren, oder
- ein racemisches Gemisch, oder
- ein Tautomer der Verbindung.

30. Verfahren zur Synthese einer wie in einem der Ansprüche 1 bis 22 definierten PARP1-Inhibitorverbindung, wobei dieses Verfahren ein Durchführen einer Reaktion zwischen i) einem ersten Reaktanten, umfassend einen Ring E, der einen Abschnitt einer Substituentengruppe L trägt, und ii) einem zweiten Reaktanten umfasst, der den Rest einer Substituentengruppe L umfasst, um die PARP1-Inhibitorverbindung zu bilden.

31. Verfahren nach Anspruch 30, wobei die Reaktion eine Alkylierung, reduktive Aminierung oder Amidbildung umfasst, um eine Gruppe L zu bilden.

## Revendications

1. Un composé inhibiteur de PARP1 destiné à être utilisé en médecine, lequel composé comprend l'une quelconque des structures suivantes : 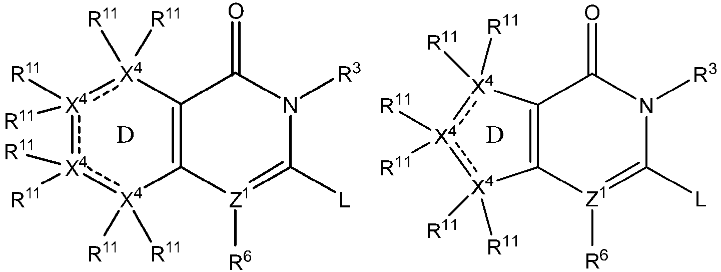 dans lequel :
les lignes pointillées indiquent que le cycle D peut comprendre des liaisons simples, ou une combinaison d'à la fois des liaisons doubles et simples, et peut être aliphatique ou aromatique ;
chaque X⁴ peut être identique ou différent et est sélectionné indépendamment parmi C, N, O et S ;
R³ est H ;
quand Z¹ est N, R⁶ est absent ; quand Z¹ est C, R⁶ est sélectionné parmi H, un halogène, un alkyle en C₁ à C₃, un haloalkyle en C₁ à C₃, un alcool en C₁ à C₃ ou un aminoalkyle en C₁ à C₃ ;
chaque R¹¹ est sélectionné indépendamment parmi H, un halogène, un groupe nitrile, un groupe alkyle en C₁-C₃ linéaire ou ramifié, un groupe alkyle halogéné en C₁-C₃ linéaire ou ramifié, un groupe -OH, un groupe alcool en C₁-C₃ linéaire ou ramifié, un groupe alcool halogéné en C₁-C₃ linéaire ou ramifié, -NH₂, un groupe amine en C₁-C₃ primaire, secondaire ou tertiaire, linéaire ou ramifié, un groupe amine halogéné en C₁-C₃ primaire, secondaire ou tertiaire, linéaire ou ramifié, un groupe alkoxy en C₁-C₃ linéaire ou ramifié, un groupe alkoxy halogéné en C₁-C₃ linéaire ou ramifié, et/ou une paire de groupes R¹¹ liés au même atome formant =O ; et/ou, quand une paire de groupes R¹¹ liés à des atomes différents forment ensemble un cycle avec des atomes de cycle D, la paire de groupes R¹¹ comprennent -CH₂CH₂CH₂- ;
Z¹ est sélectionné parmi C et N ;
le groupe L comprend un groupe ayant la structure suivante : 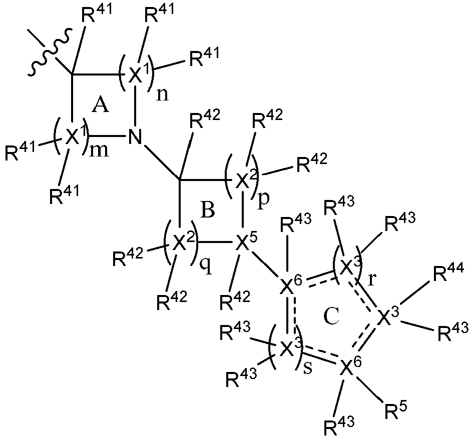
dans lequel :
n est un nombre sélectionné indépendamment parmi 0, 1, 2, 3, 4, 5 et 6 ;
m est un nombre sélectionné indépendamment parmi 0, 1, 2, 3, 4, 5 et 6 ;
m + n est un nombre sélectionné parmi 2, 3, 4, 5 et 6 ;
p est un nombre sélectionné indépendamment parmi 0, 1, 2, 3, 4, 5 et 6 ;
q est un nombre sélectionné indépendamment parmi 0, 1, 2, 3, 4, 5 et 6 ;
p + q est un nombre sélectionné parmi 2, 3, 4, 5 et 6 ;
r est un nombre sélectionné indépendamment parmi 0, 1, 2, 3, 4 et 5 ;
s est un nombre sélectionné indépendamment parmi 0, 1, 2, 3, 4 et 5 ;
r + s est un nombre sélectionné parmi 1, 2, 3, 4 et 5 ;
chaque X¹ peut être identique ou différent et est sélectionné indépendamment parmi C, N, O et S ;
chaque X² peut être identique ou différent et est sélectionné indépendamment parmi C, N, O et S ;
chaque X³ peut être identique ou différent et est sélectionné indépendamment parmi C, N, O et S ;
X⁵ est sélectionné indépendamment parmi C et N ;
chaque X⁶ peut être identique ou différent et est sélectionné indépendamment parmi C et N ;
chaque R⁴¹ R⁴², R⁴³ et R⁴⁴ peut être identique ou différent, et peut être présent ou absent, et est sélectionné parmi H, un deutérium, un halogène, un groupe nitrile, un groupe alkyle en C₁-C₆ non substitué ou substitué, un groupe alkyle halogéné en C₁-C₆ linéaire ou ramifié, non substitué ou substitué, un groupe cyclopropyle, un groupe -OH ou un groupe alcool en C₁-C₆ linéaire ou ramifié, non substitué ou substitué, un groupe amino carbonyle en C₁-C₇ linéaire ou ramifié, non substitué ou substitué, un groupe -NH₂ ou un groupe amino en C₁-C₆ et un groupe alkoxy en C₁-C₆ non substitué ou substitué ; dans lequel, quand une paire de groupes R⁴¹ liés à des atomes différents forment ensemble un cycle avec des atomes de cycle A, et/ou une paire de groupes R⁴² liés à des atomes différents forment ensemble un cycle avec des atomes de cycle B, chacun de la paire de groupes R⁴¹ et/ou paire de groupes R⁴² comprend indépendamment - CH₂- ou -CH₂CH₂- ;
les lignes pointillées indiquent que le cycle C peut comprendre des liaisons simples, ou une combinaison d'à la fois des liaisons doubles et simples, et peut être aliphatique ou aromatique ; et
R⁵ est sélectionné parmi H et un groupe organique non substitué ou substitué.

2. Un composé destiné à être utilisé selon la revendication 1, dans lequel un cycle A du groupe L comprend l'une quelconque des structures suivantes :

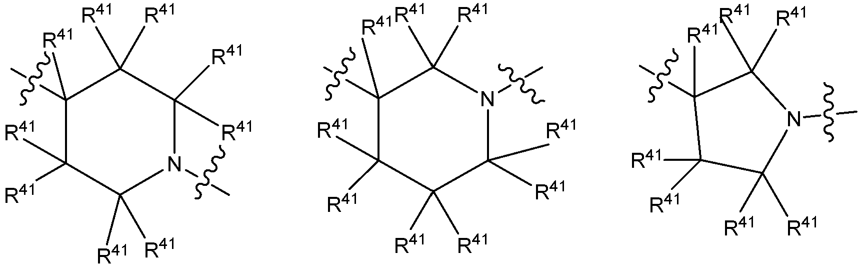

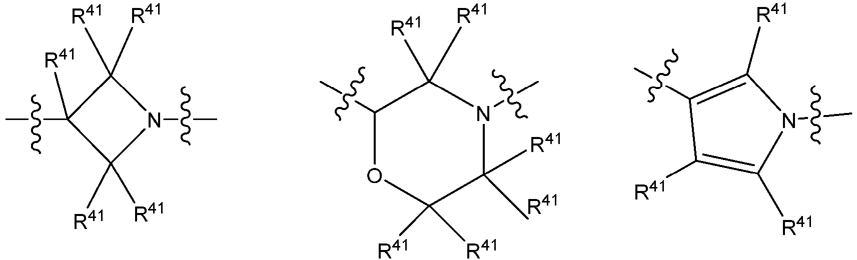

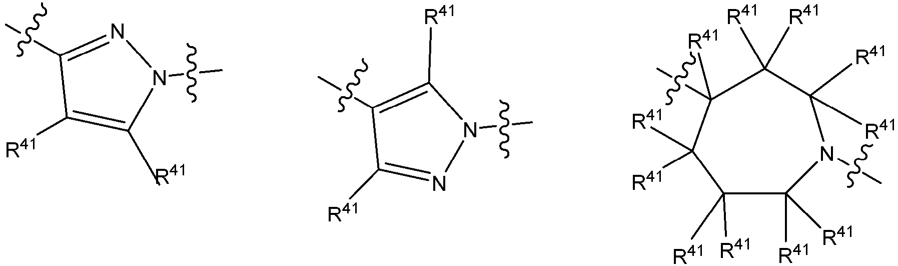

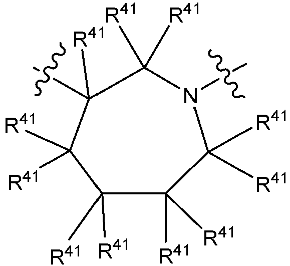

3. Un composé destiné à être utilisé selon la revendication 2, dans lequel un cycle A du groupe L comprend l'une quelconque des structures suivantes :

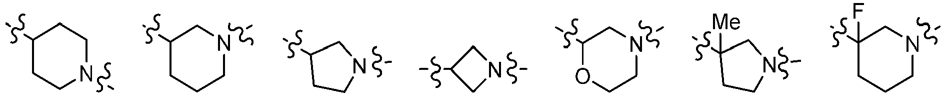

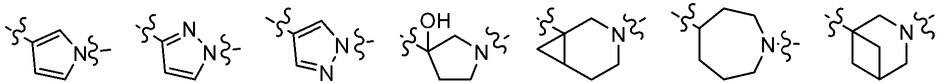

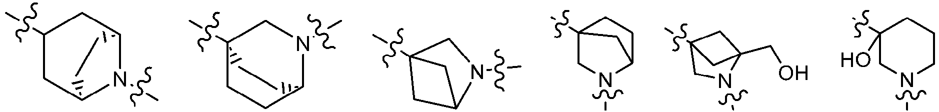

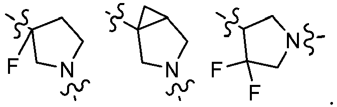

4. Un composé destiné à être utilisé selon l'une quelconque des revendications précédentes, dans lequel un cycle C du groupe L comprend l'une quelconque des structures suivantes :

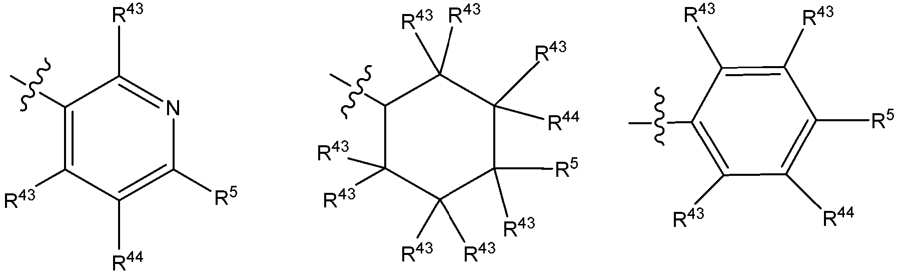

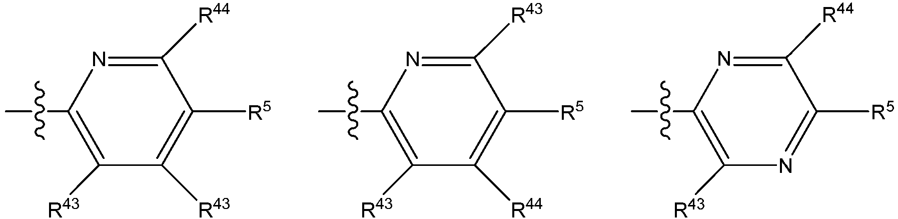

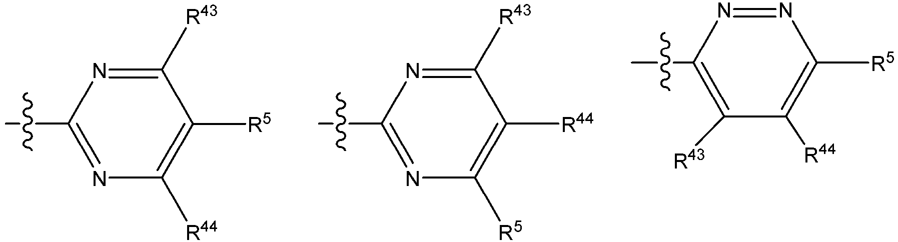

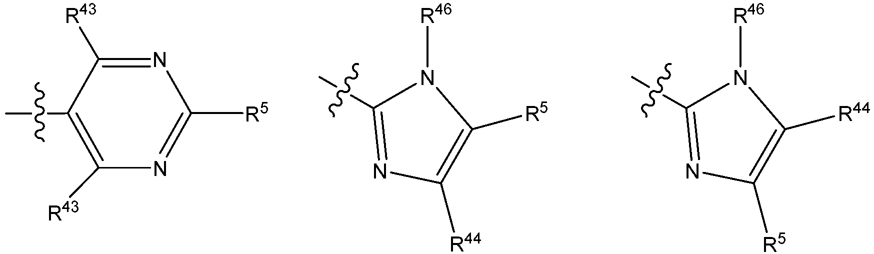

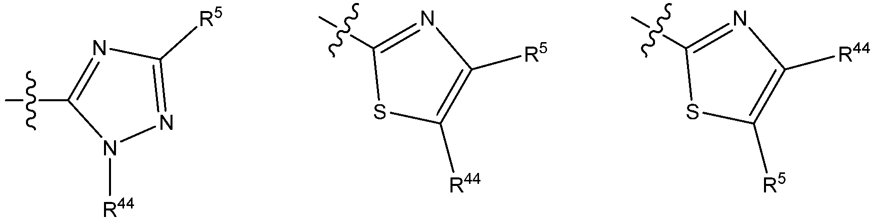

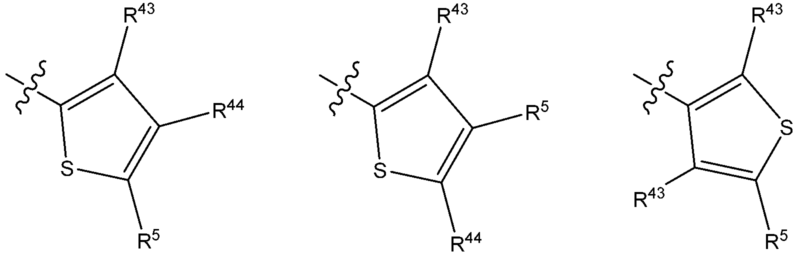

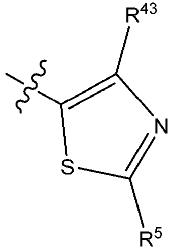

5. Un composé destiné à être utilisé selon la revendication 4, dans lequel un cycle C du groupe L comprend l'une quelconque des structures suivantes :

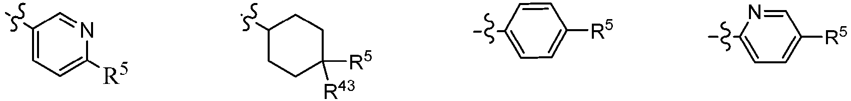

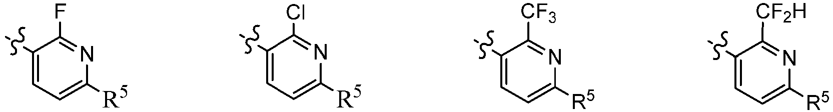

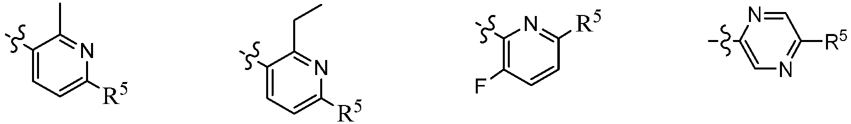

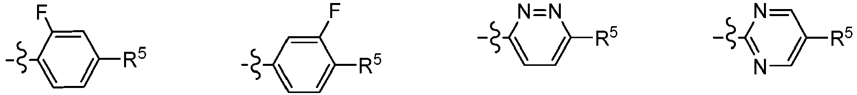

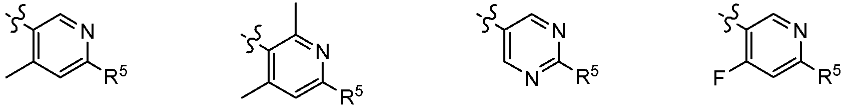

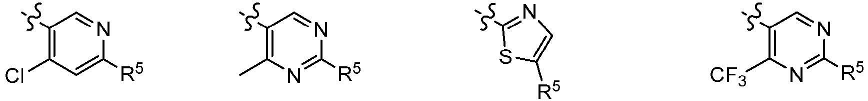

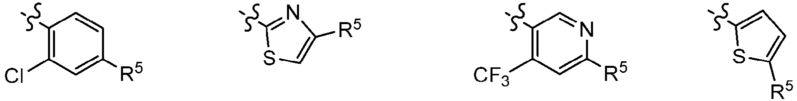

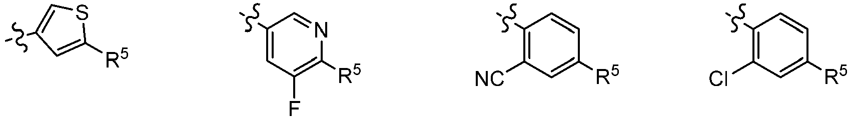

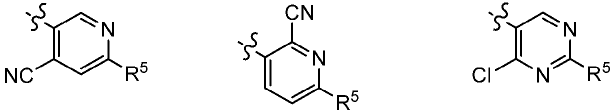

6. Un composé selon l'une quelconque des revendications précédentes, dans lequel le groupe R⁵ est sélectionné parmi H, -F, -Cl, -Br, -I, -CN, -CONR⁵¹R⁵¹, -NR⁵¹COR⁵², - SO₂NR⁵¹R⁵¹, -NR⁵¹SO₂R⁵³, -O-CR⁵²R⁵²R⁵², -CR⁵²R⁵²NR⁵¹R⁵¹ et l'une quelconque des structures suivantes : 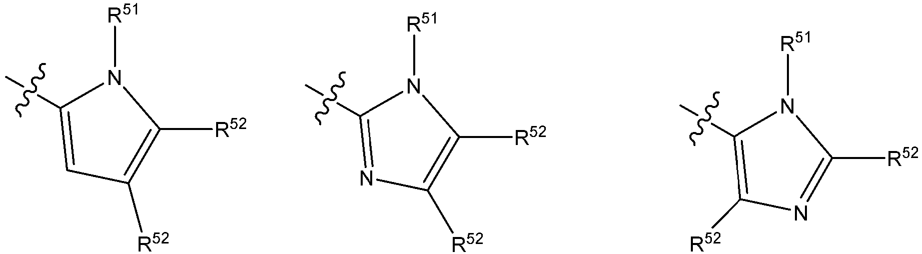 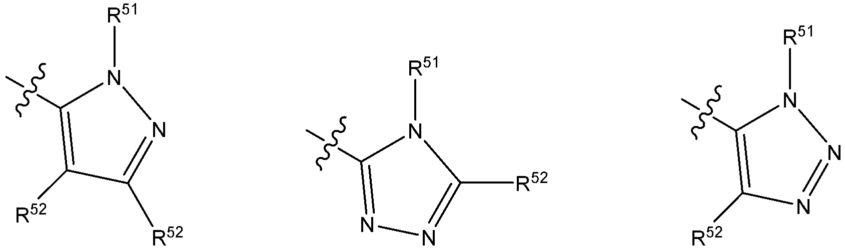 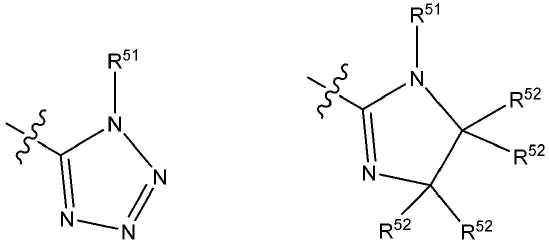 dans lequel, chacun de R⁵¹, R⁵² et R⁵³ peut être identique ou différent et est sélectionné indépendamment parmi H et un groupe organique non substitué ou substitué ;
en option, dans lequel le groupe R⁵ est sélectionné parmi -F, -Cl, -CN, -CONH₂, - CONHMe, -CONHEt, -CONMe₂, -CONHCOMe, -CONHCH₂-CH₂OMe, -CONH-CH₂-CH₂F, -CONH-CH₂-CF₃, -CONH-CH₂-CHF₂, -OCHF₂, -NHCOMe, -NHSO₂Me, -SO₂NHMe, -CONHSO₂Me,

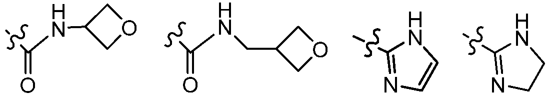

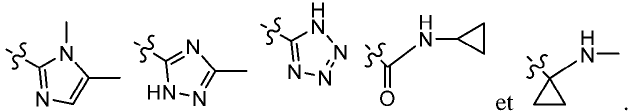

7. Un composé destiné à être utilisé selon l'une quelconque des revendications précédentes, dans lequel X⁵ est N.

8. Un composé destiné à être utilisé selon la revendication 7, dans lequel le cycle B du groupe L comprend l'une quelconque des structures suivantes :

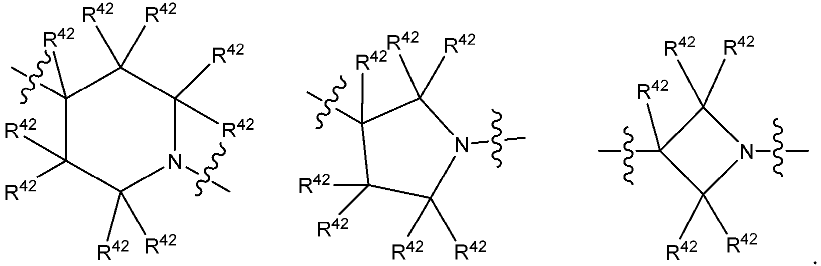

9. Un composé destiné à être utilisé selon la revendication 8, dans lequel le cycle B du groupe L comprend l'une quelconque des structures suivantes :

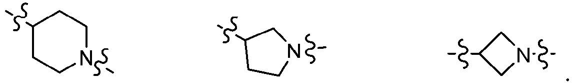

10. Un composé selon l'une quelconque des revendications précédentes, dans lequel le groupe L comprend un groupe ayant la structure suivante :

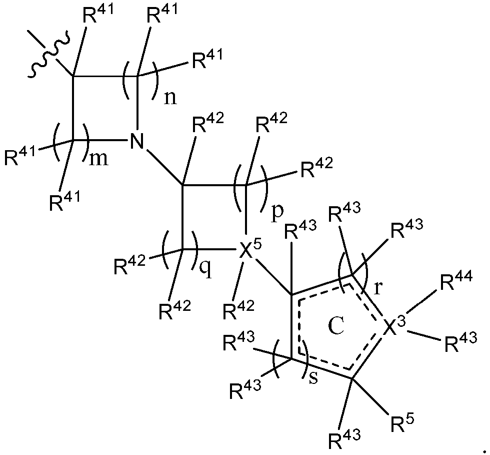

11. Un composé destiné à être utilisé selon l'une quelconque des revendications précédentes, dans lequel le groupe L comprend un groupe sélectionné parmi les structures suivantes :

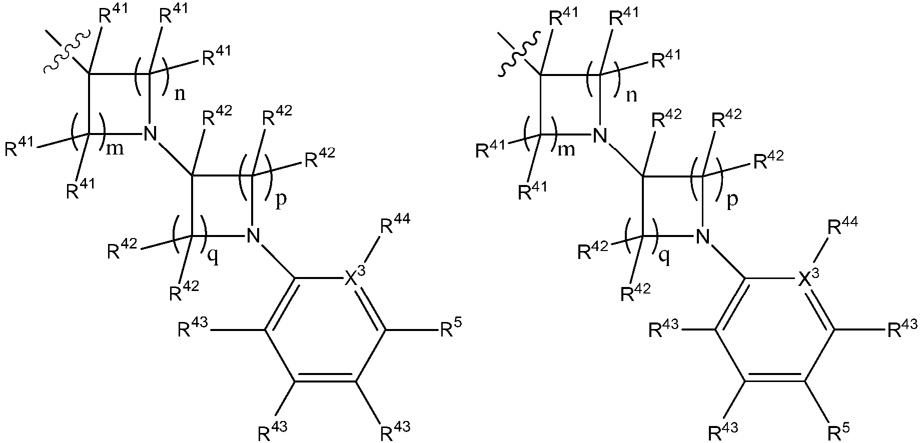

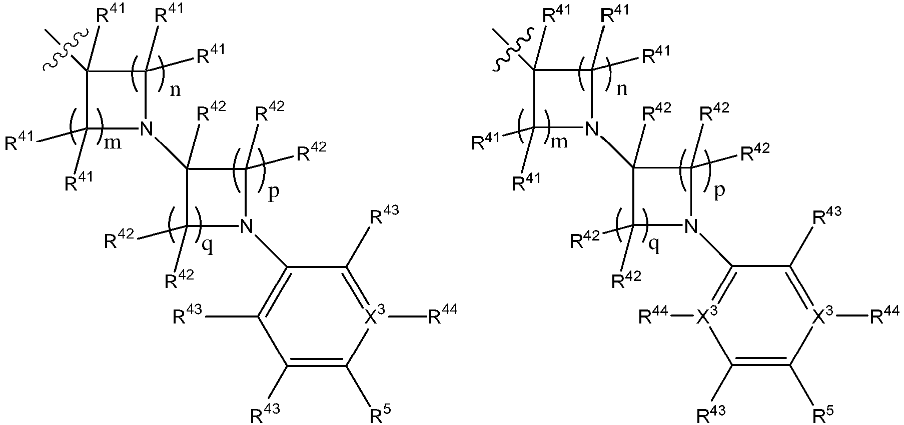

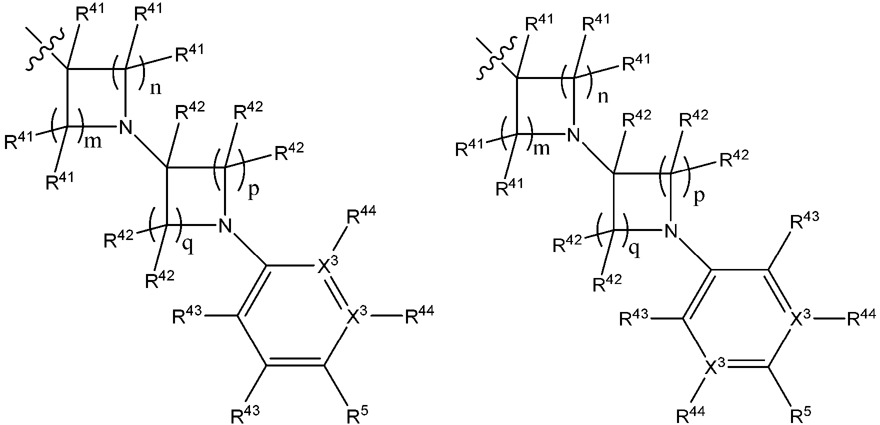

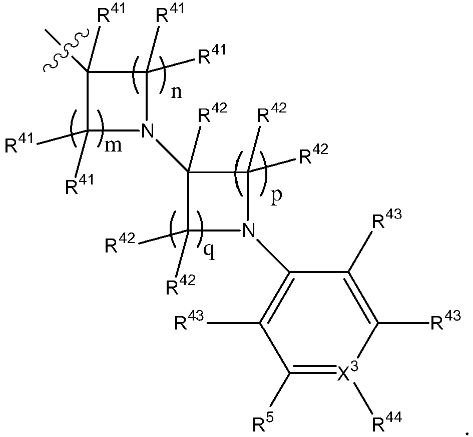

12. Un composé destiné à être utilisé selon l'une quelconque des revendications précédentes, dans lequel m est sélectionné parmi 1 ou 2, n est sélectionné parmi 2 ou 3, p est sélectionné parmi 1, 2 ou 3 et q est sélectionné parmi 1 ou 2.

13. Un composé destiné à être utilisé selon la revendication 11 ou la revendication 12, dans lequel le groupe L comprend un groupe ayant l'une quelconque des structures suivantes :

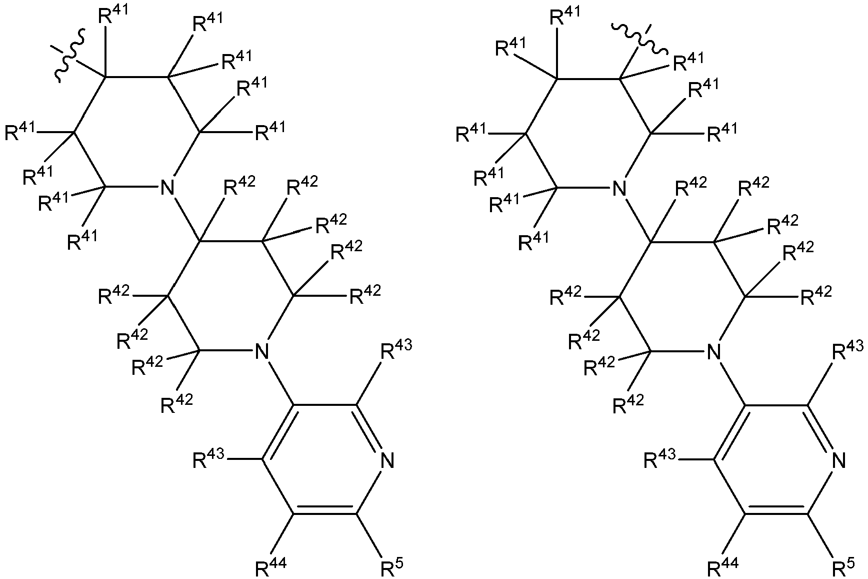

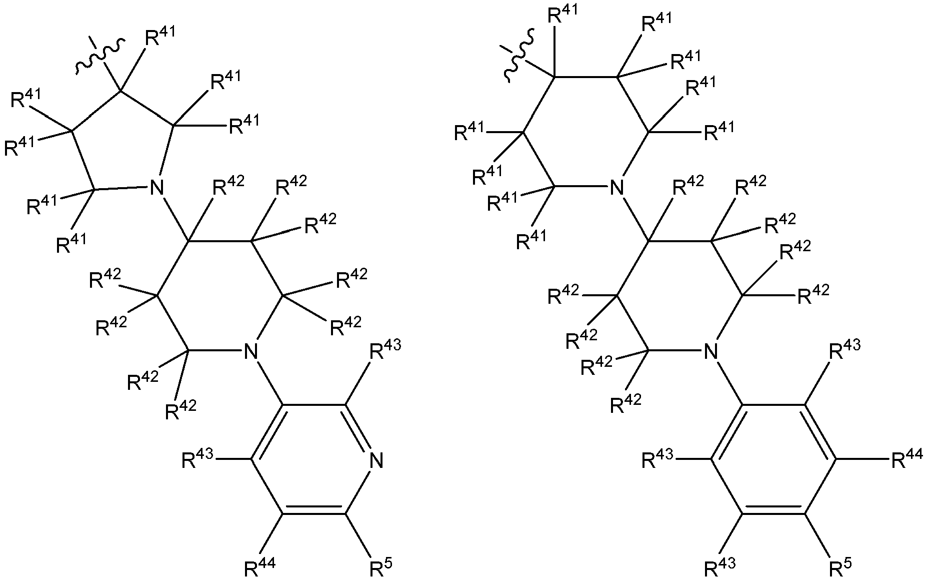

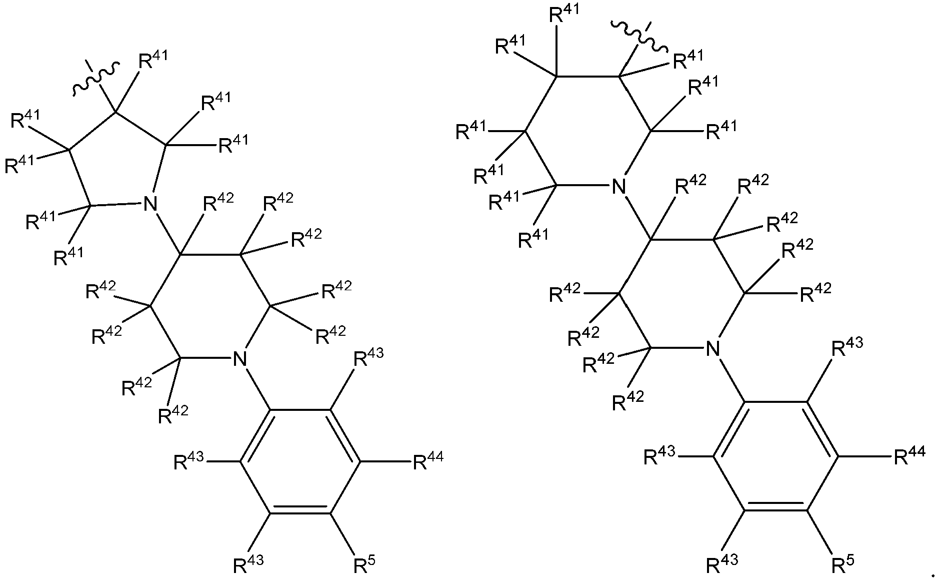

14. Un composé destiné à être utilisé selon l'une quelconque des revendications précédentes, dans lequel le groupe L comprend un groupe ayant l'une quelconque des structures suivantes :

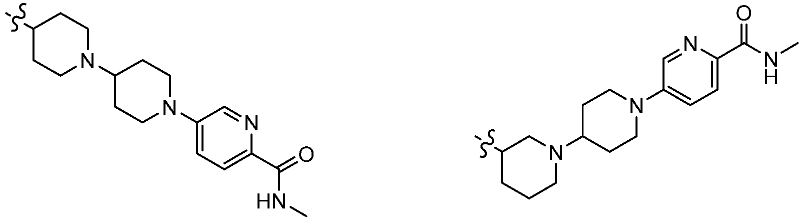

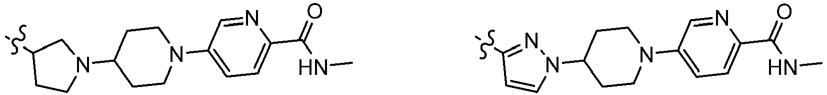

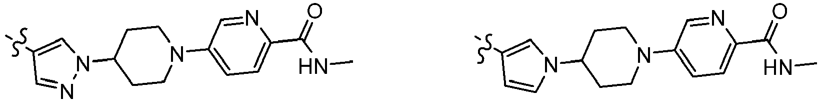

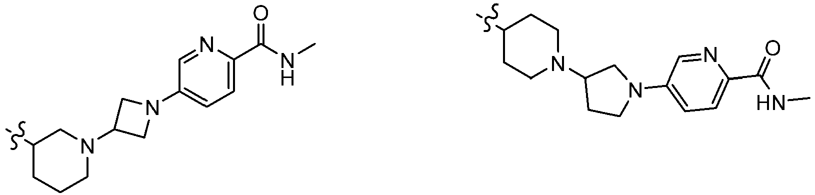

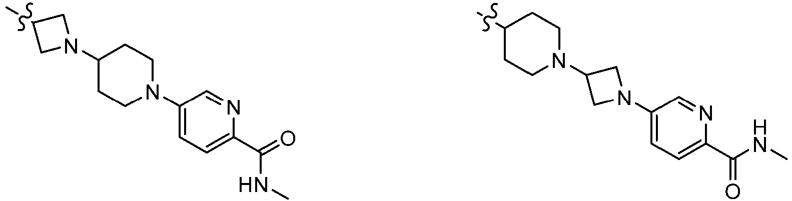

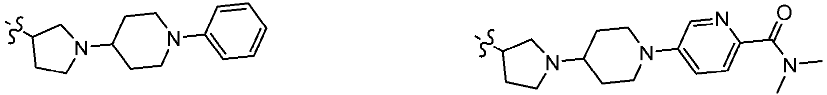

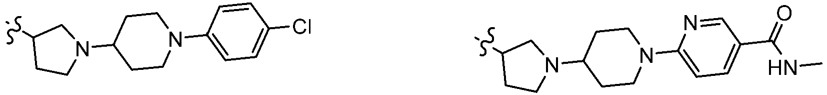

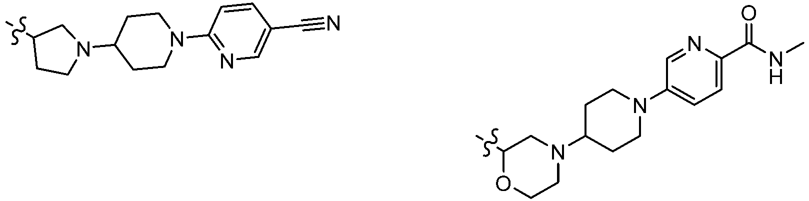

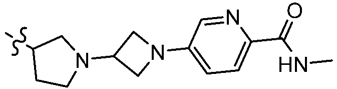

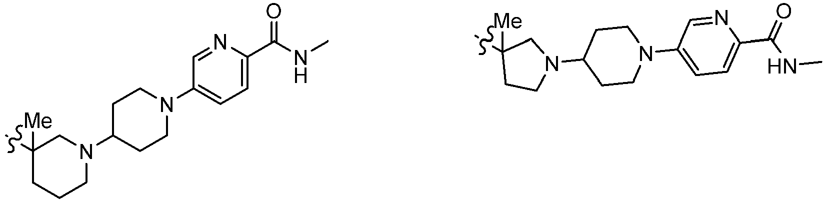

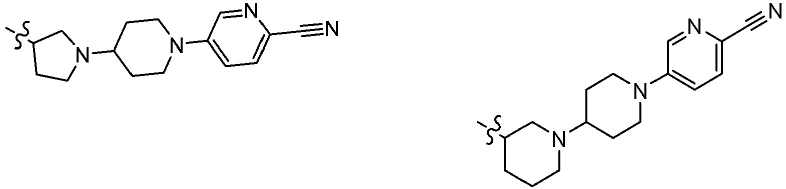

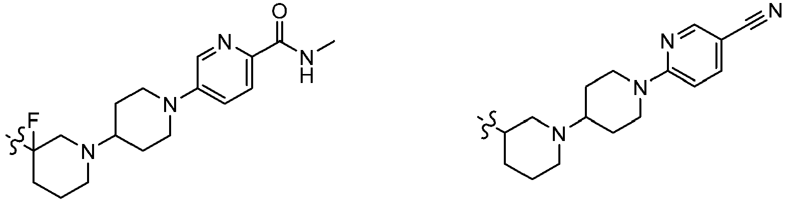

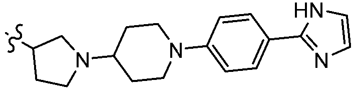

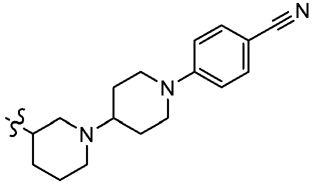

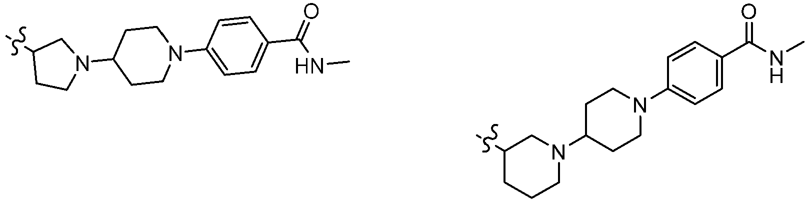

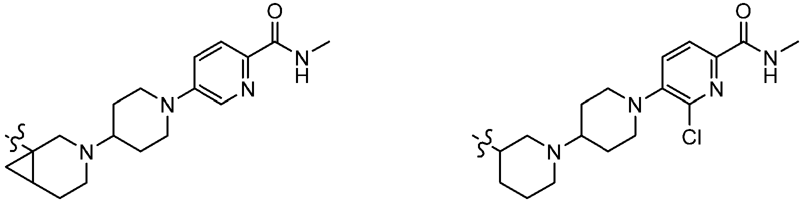

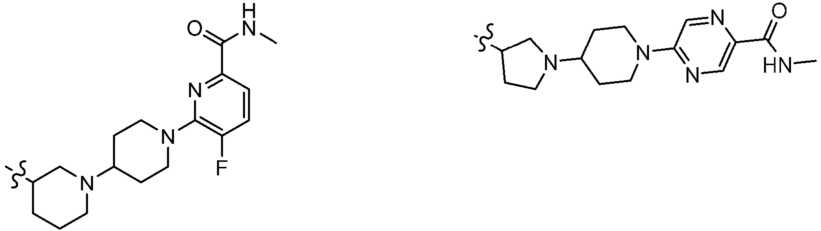

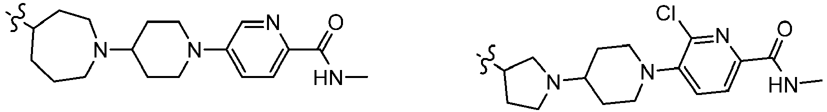

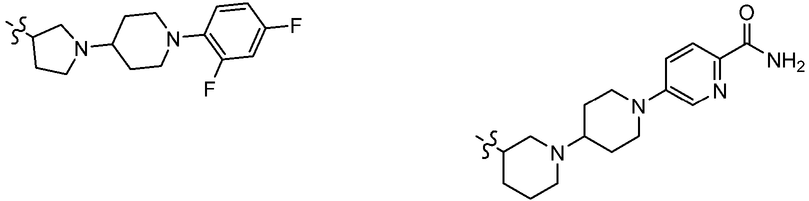

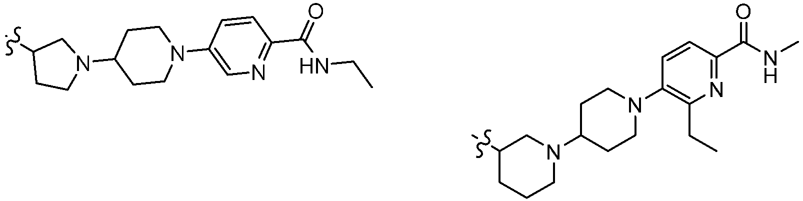

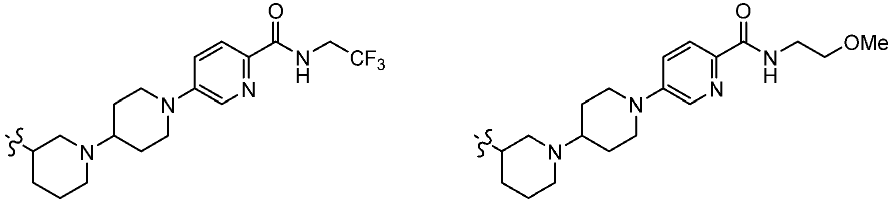

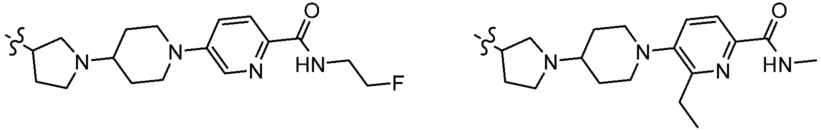

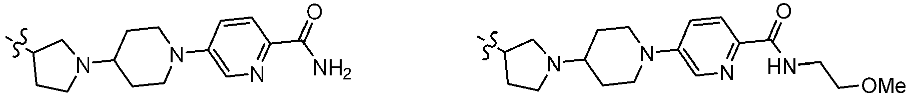

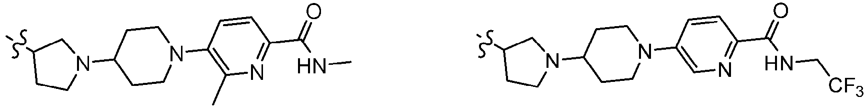

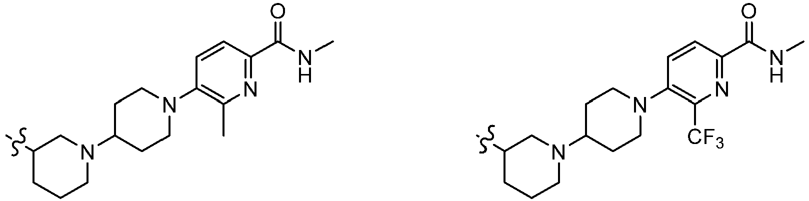

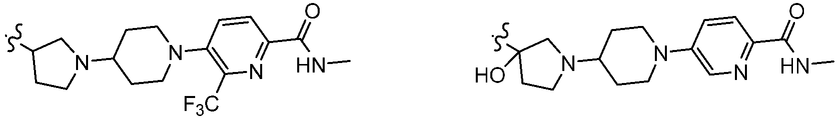

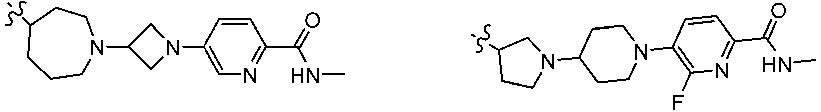

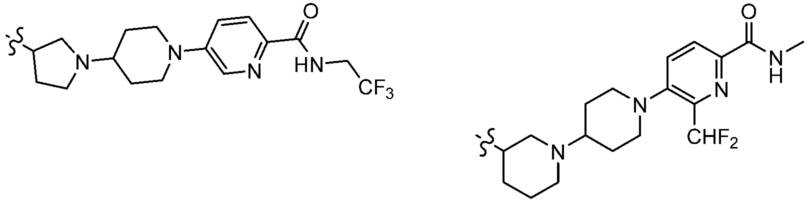

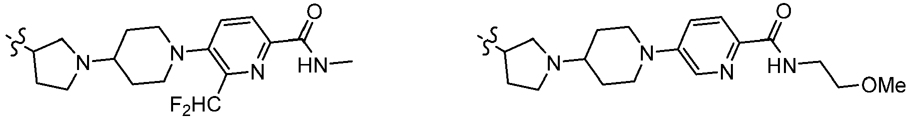

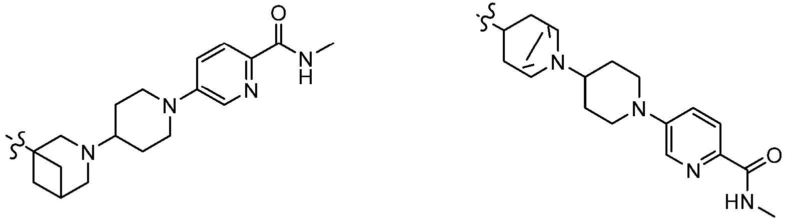

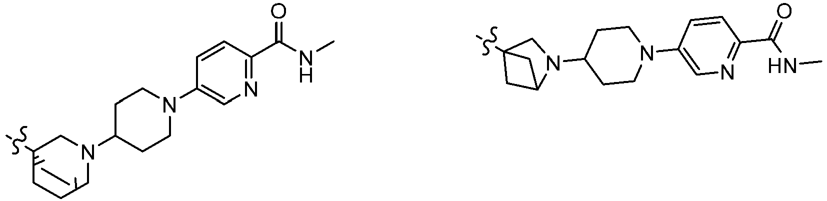

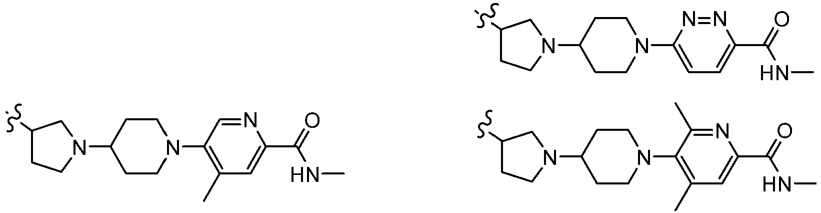

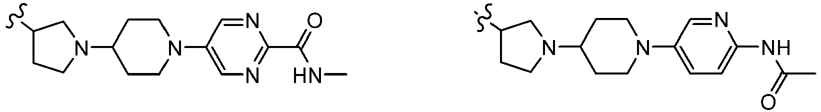

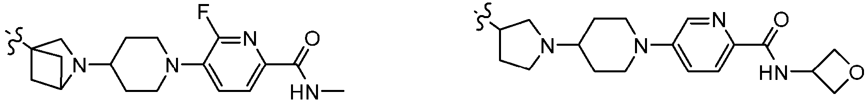

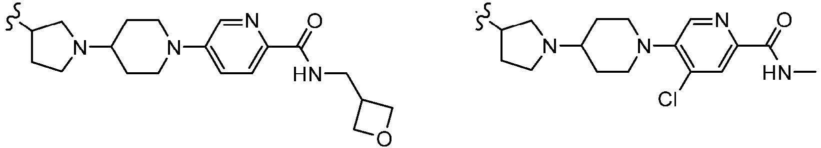

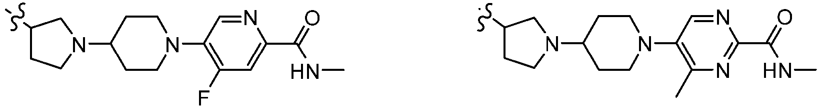

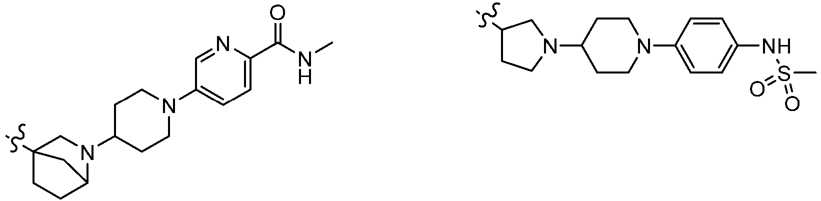

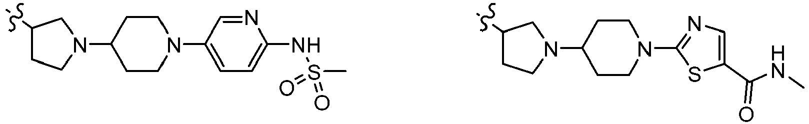

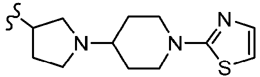

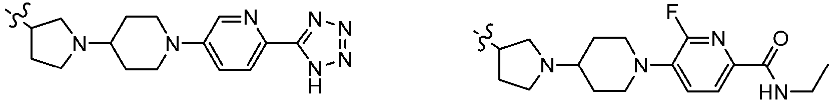

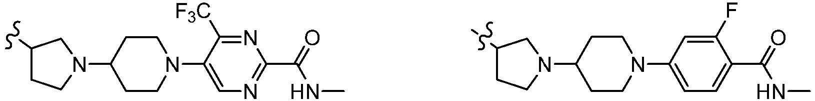

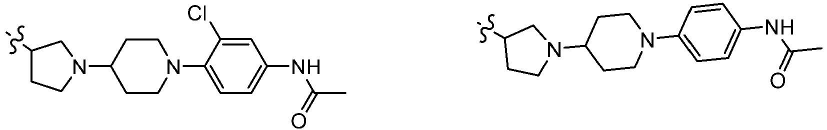

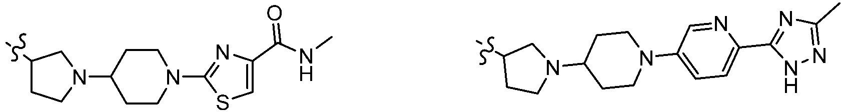

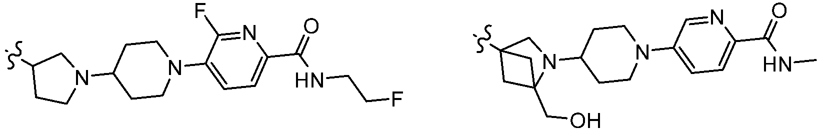

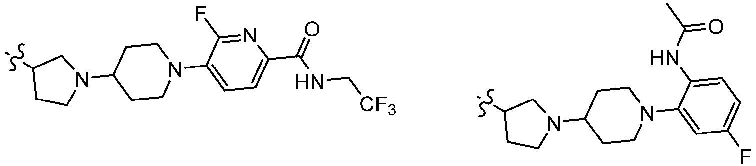

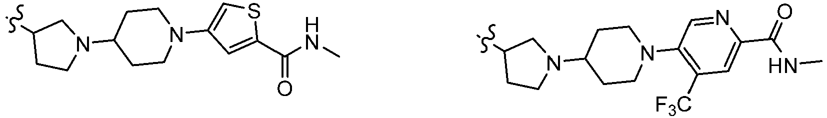

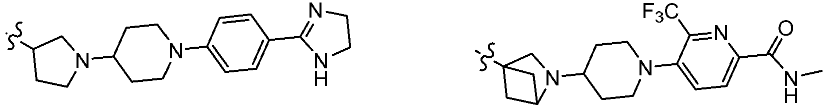

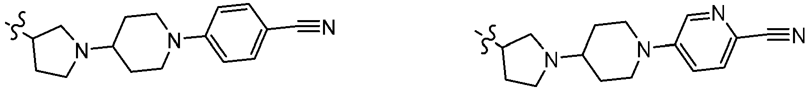

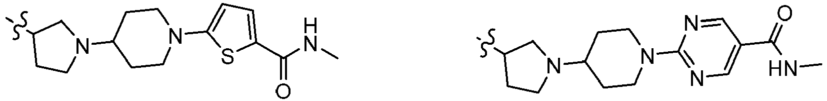

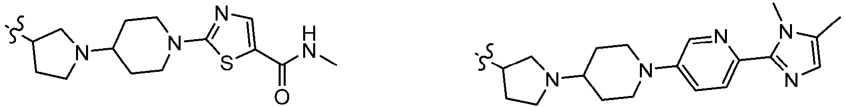

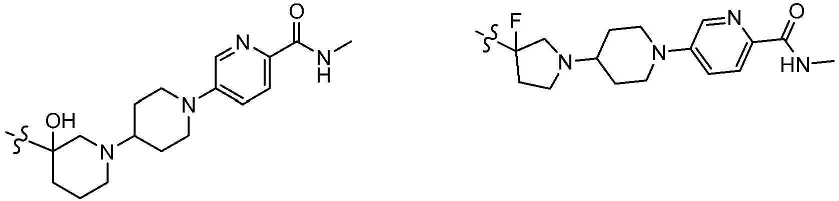

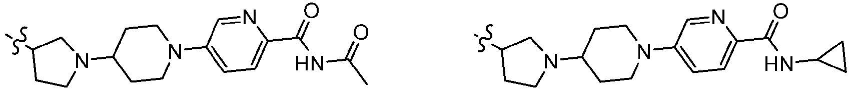

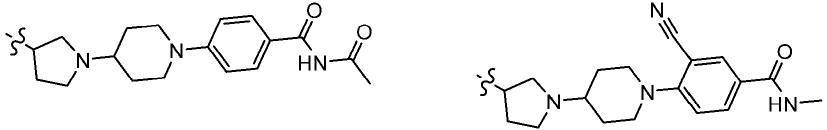

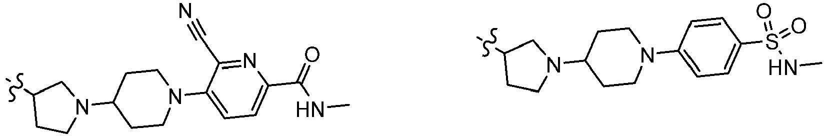

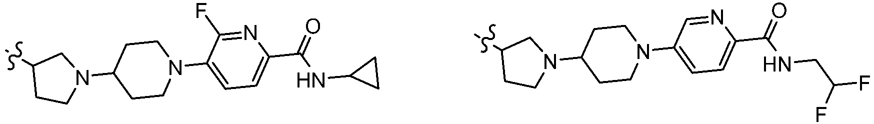

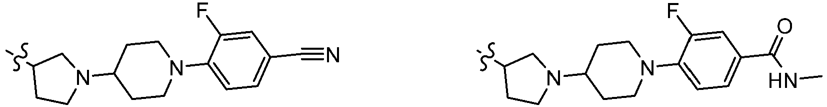

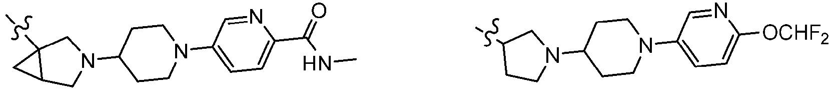

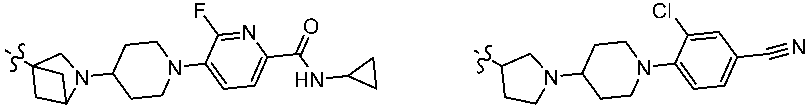

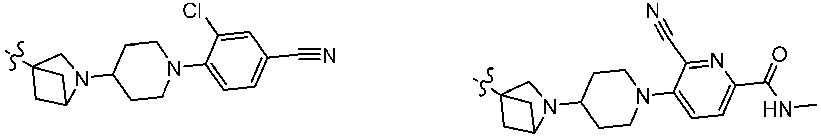

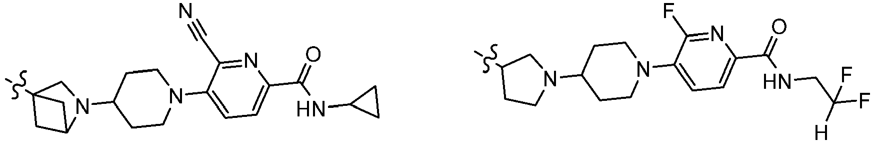

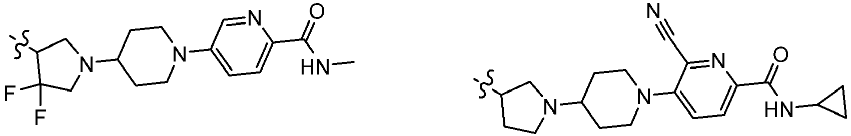

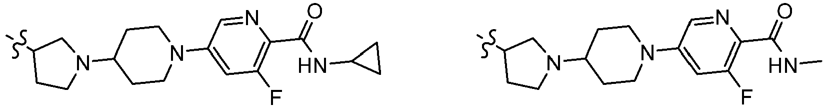

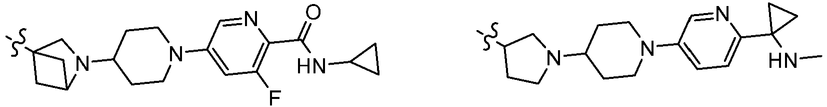

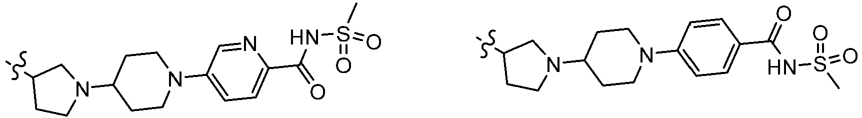

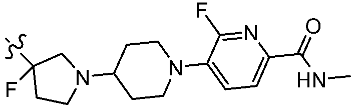

15. Un composé selon l'une quelconque des revendications précédentes, lequel composé comprend l'une quelconque des structures suivantes : 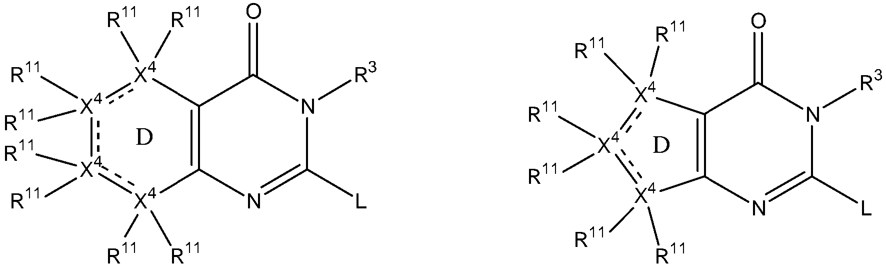 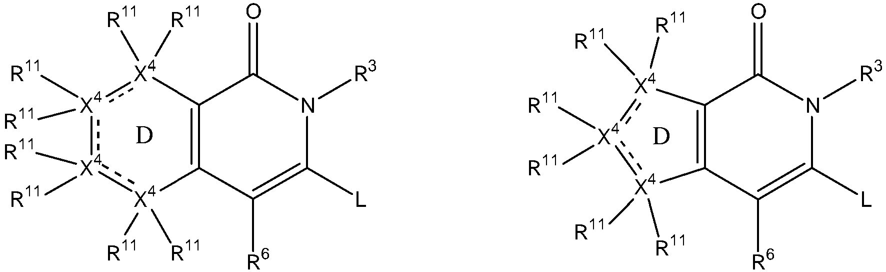 de préférence, dans lequel au moins un X⁴ est C.

16. Un composé destiné à être utilisé selon la revendication 15, lequel composé comprend l'une quelconque des structures suivantes : 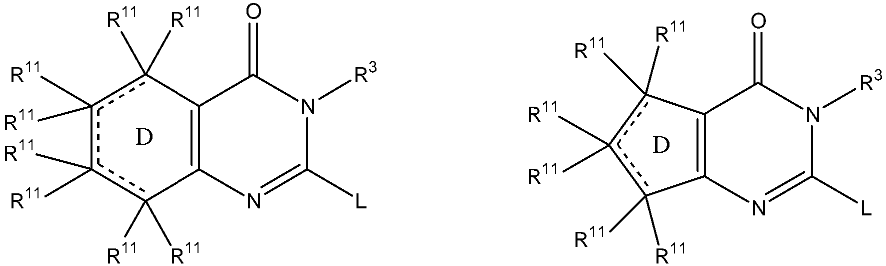 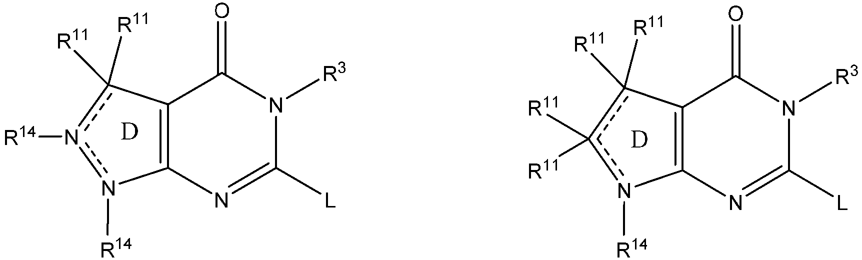 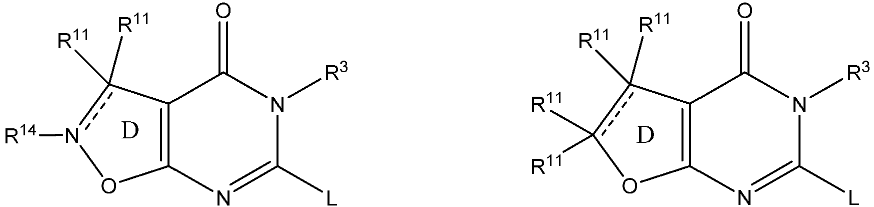 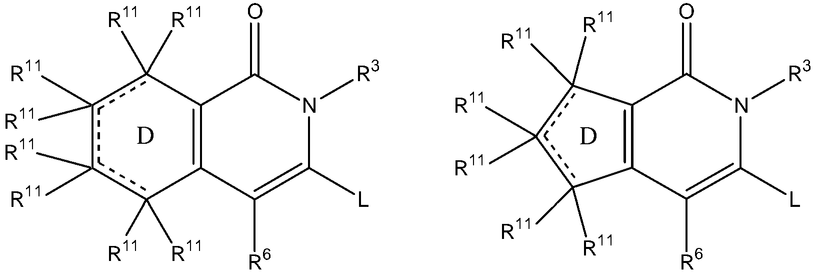 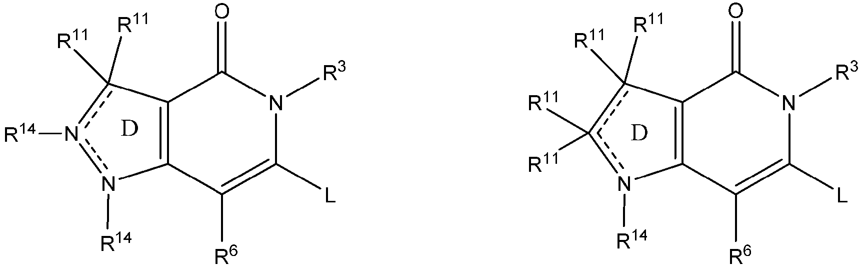 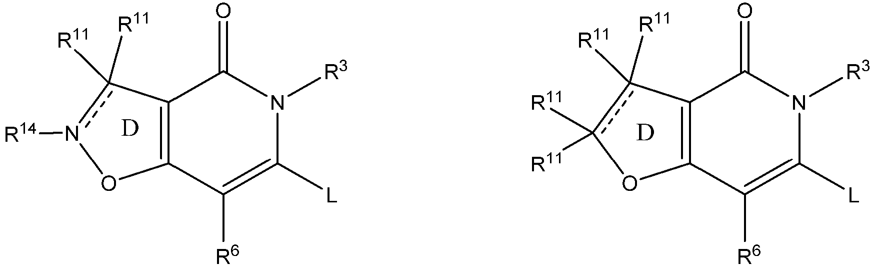 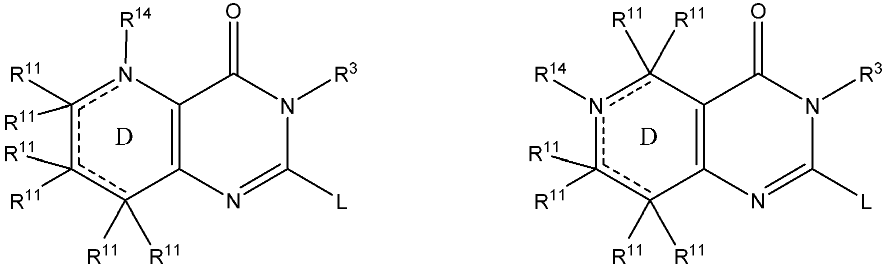 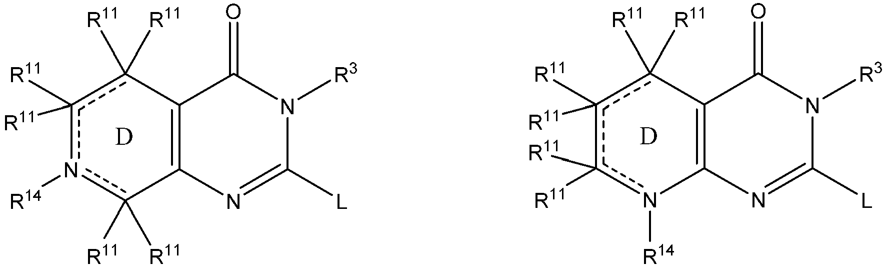 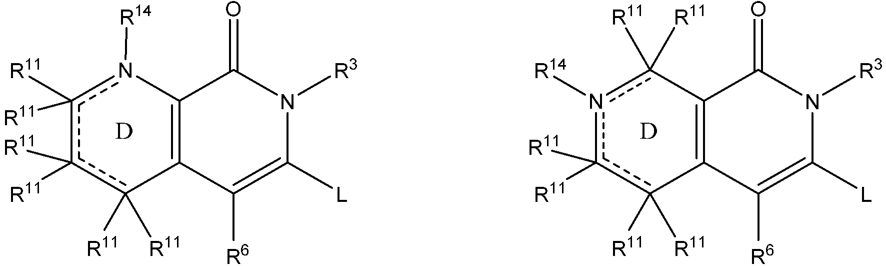 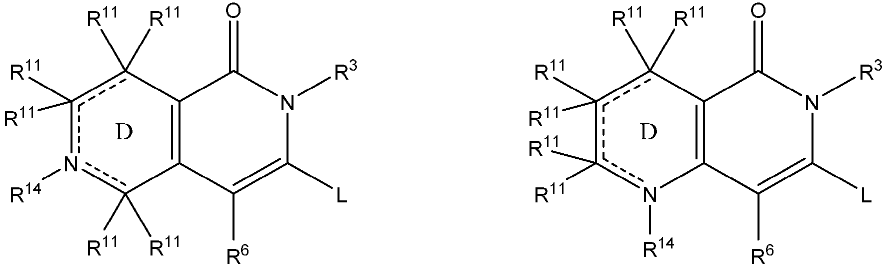 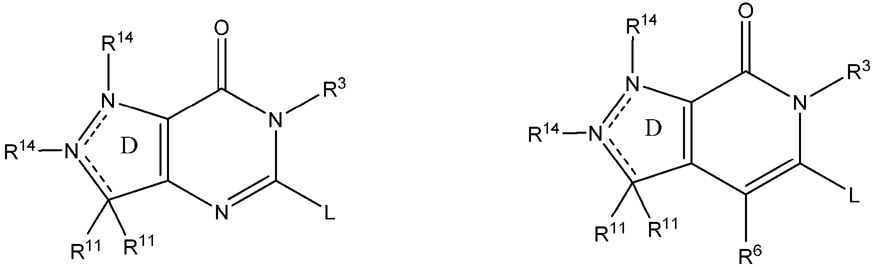 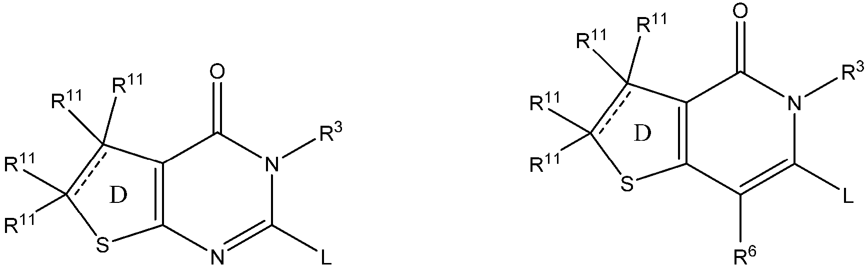 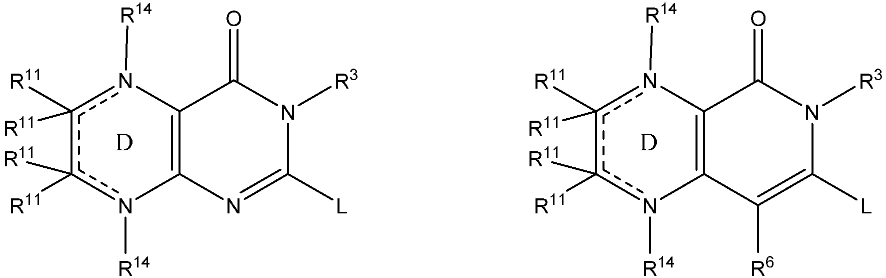 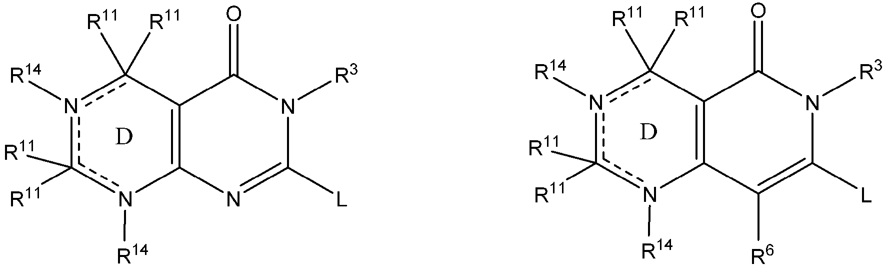 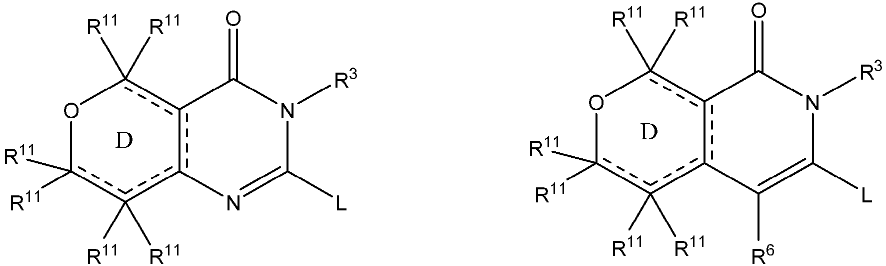 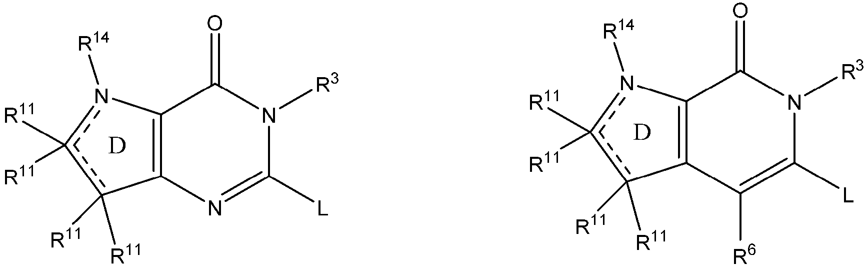 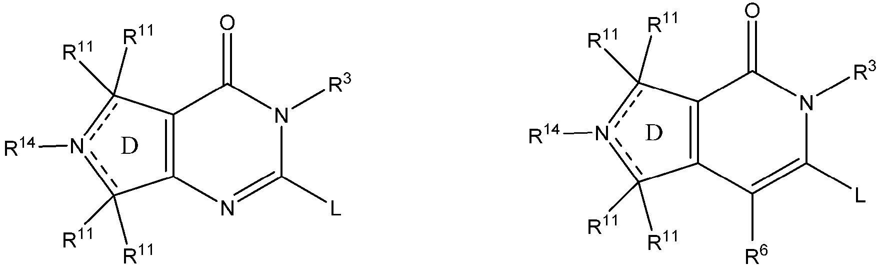 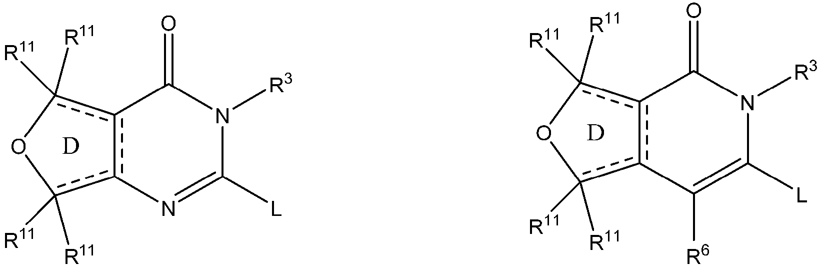 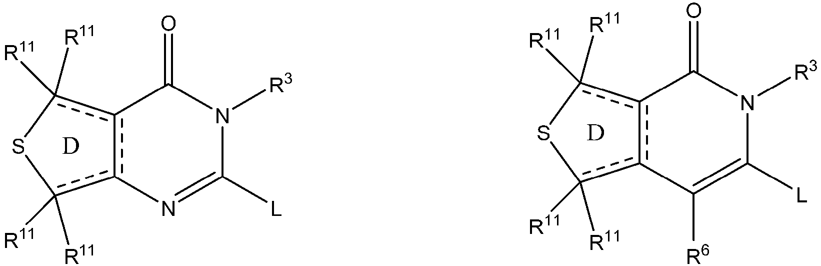 dans lequel R¹⁴ peut être présent ou absent et, s'il est présent, est sélectionné parmi H, un alkyle en C₁ à C₃ ou un fluoroalkyle en C₁ à C₃.

17. Un composé selon la revendication 16, lequel composé comprend l'une quelconque des structures suivantes :

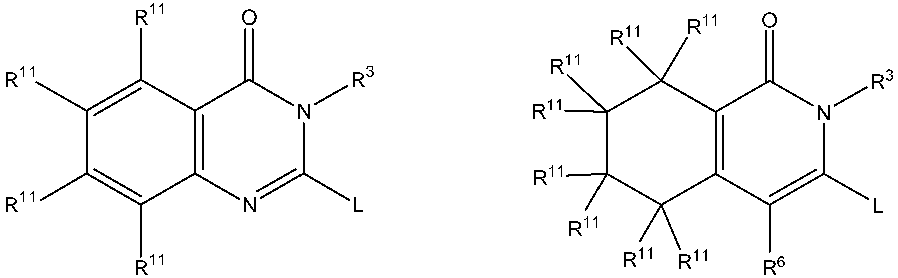

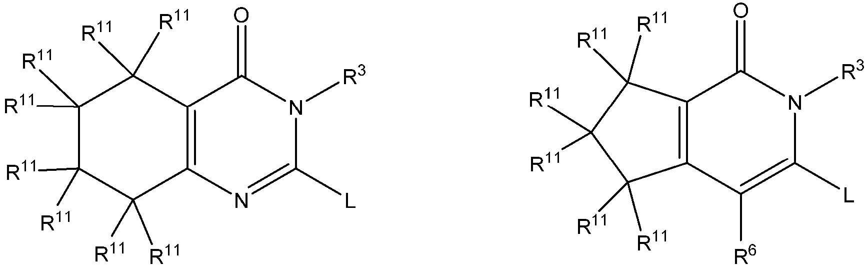

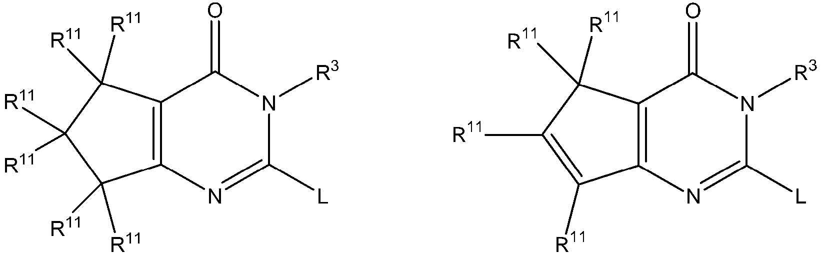

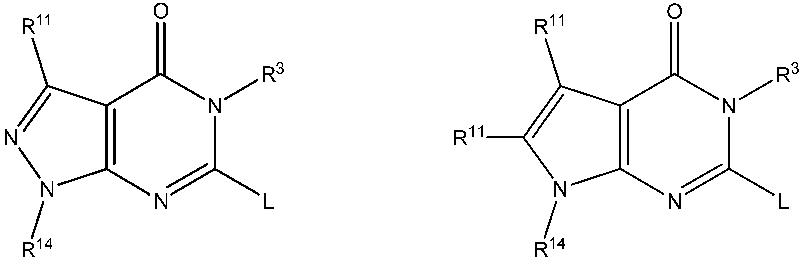

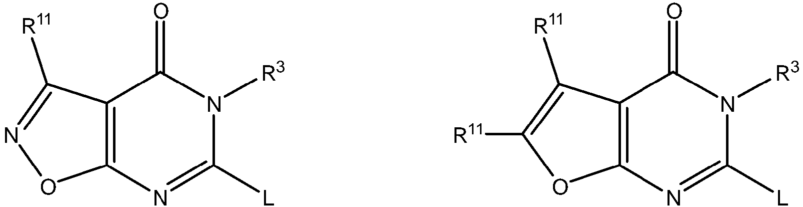

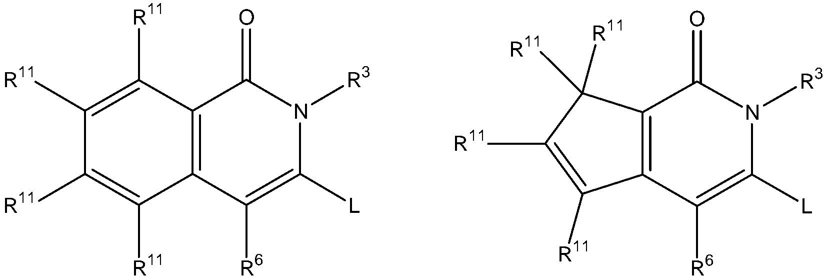

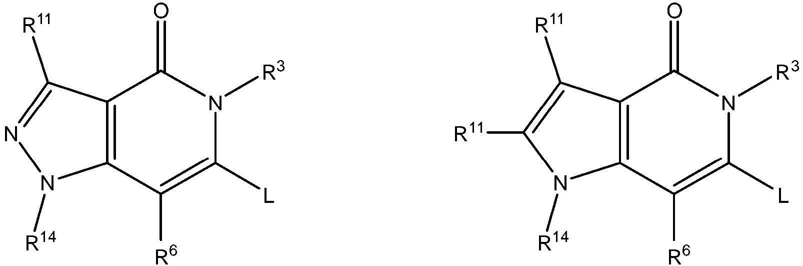

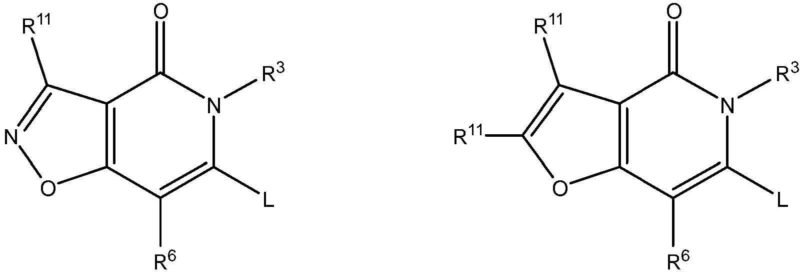

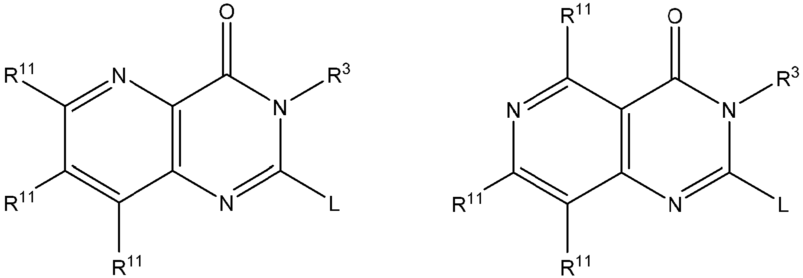

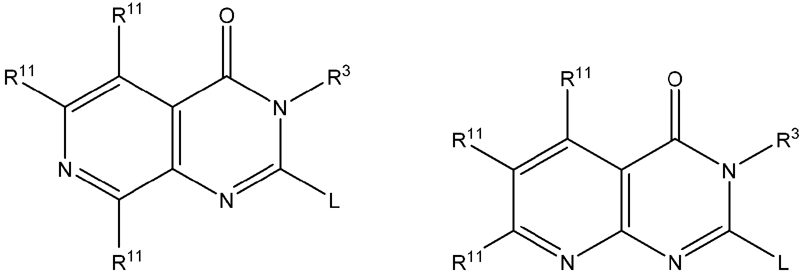

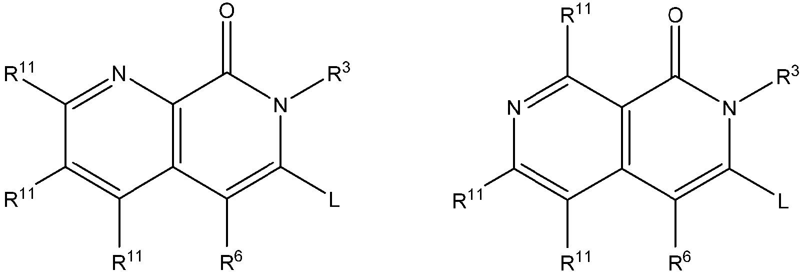

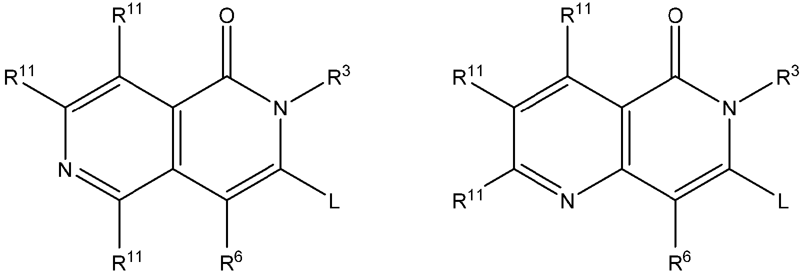

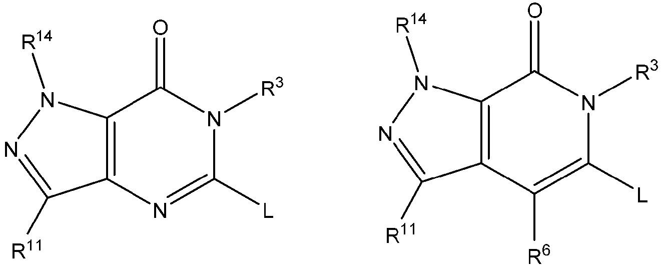

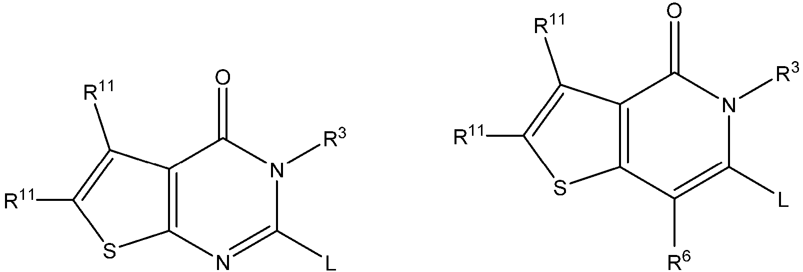

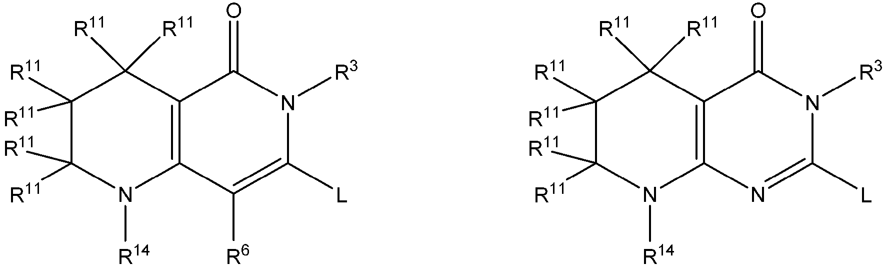

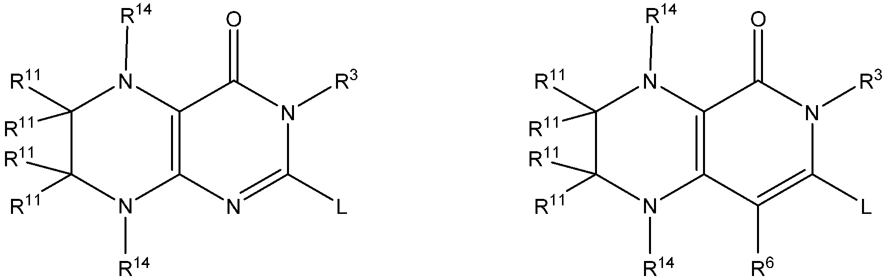

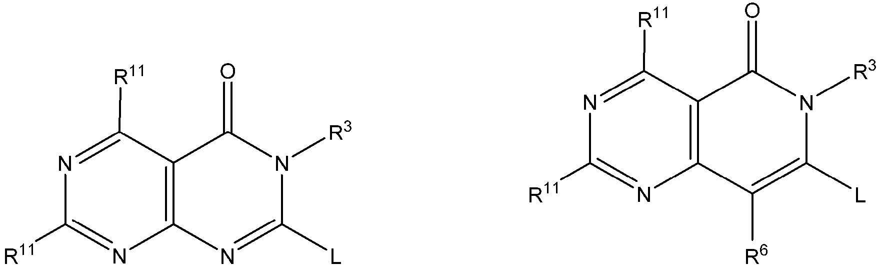

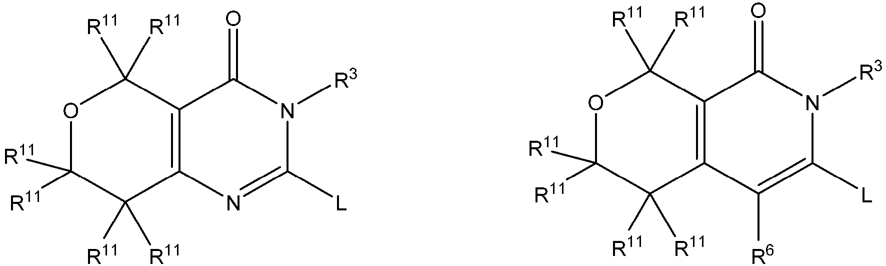

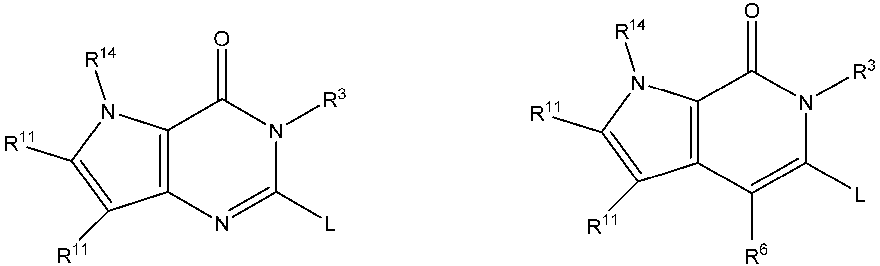

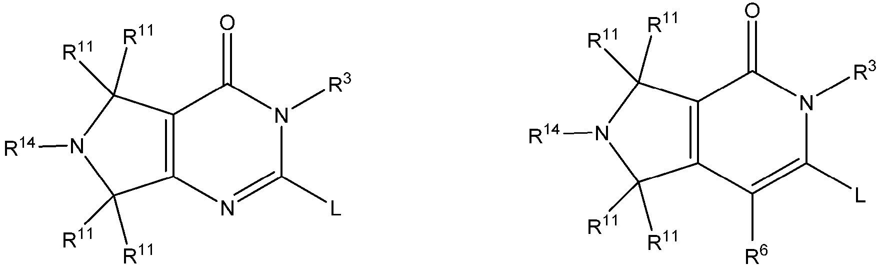

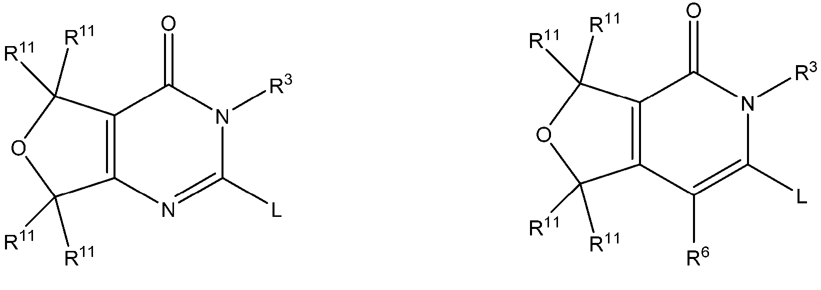

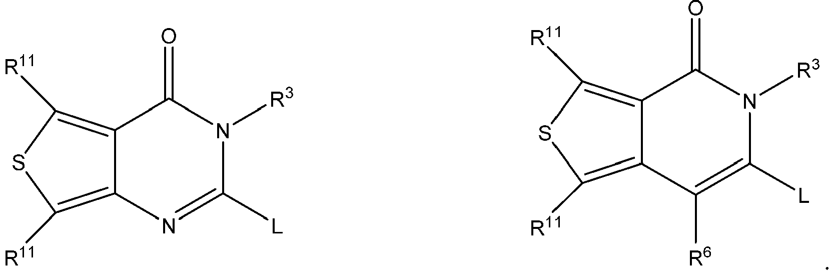

18. Un composé destiné à être utilisé selon l'une quelconque des revendications précédentes, dans lequel les R¹¹ sont chacun sélectionnés indépendamment parmi H, Cl, F, CHF₂, CF₃, CH₃, OH, CH₃O et NH₂, et/ou une paire de groupes R¹¹ liés au même atome formant =O ; et indépendamment, quand une paire de groupes R¹¹ liés à des atomes différents forment ensemble un cycle avec des atomes de cycle D, la paire de groupes R¹¹ comprennent -CH₂CH₂CH₂-.

19. Un composé destiné à être utilisé selon la revendication 18, lequel composé comprend l'une quelconque des structures suivantes :

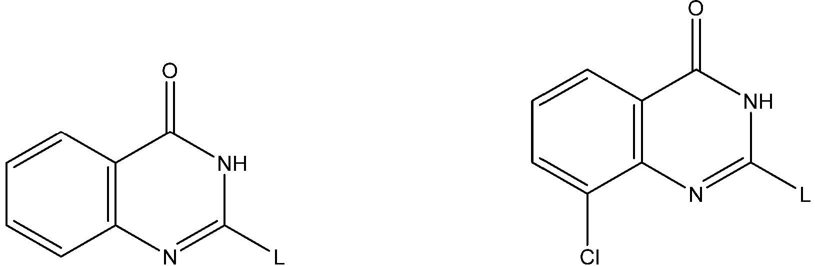

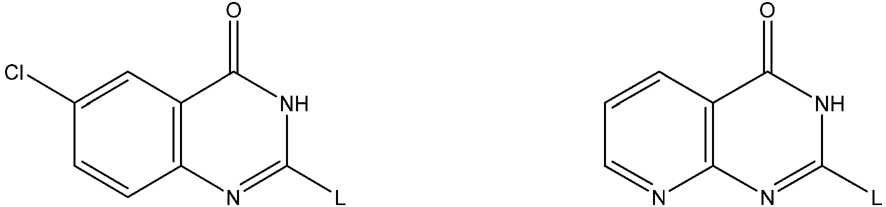

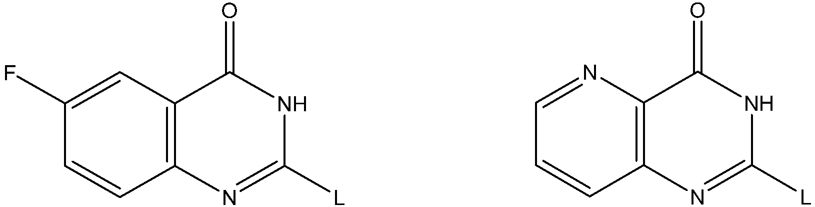

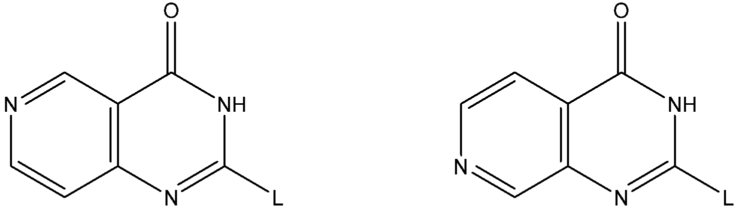

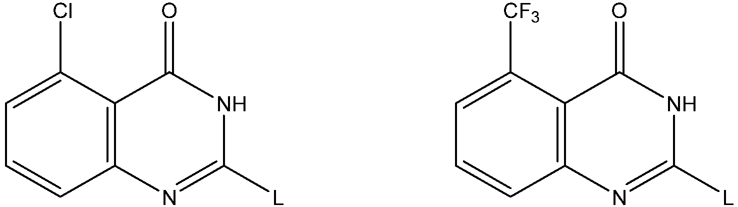

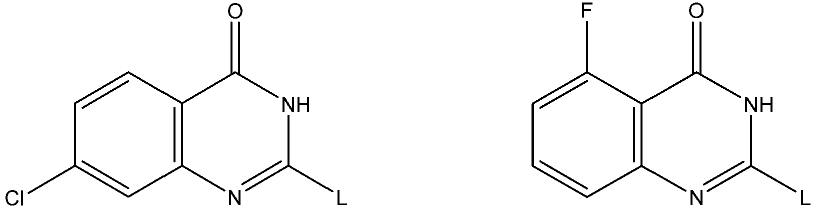

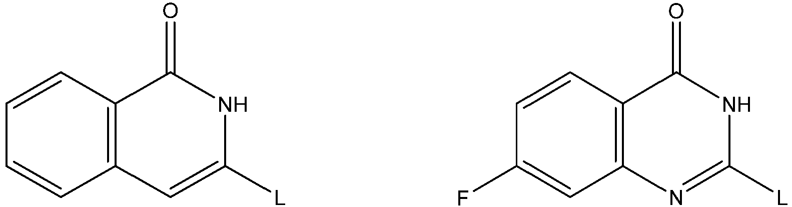

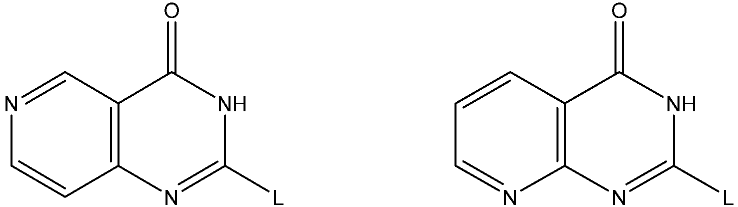

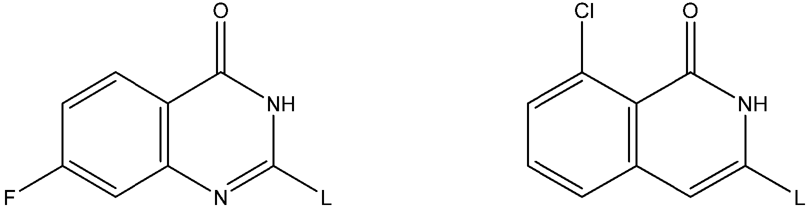

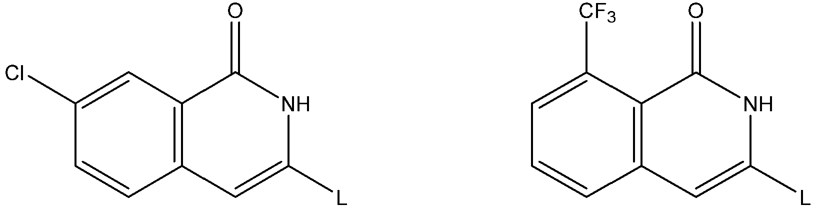

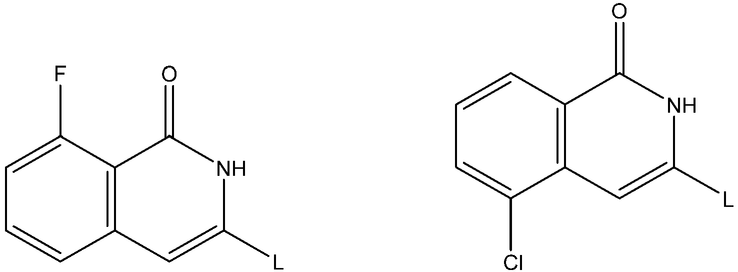

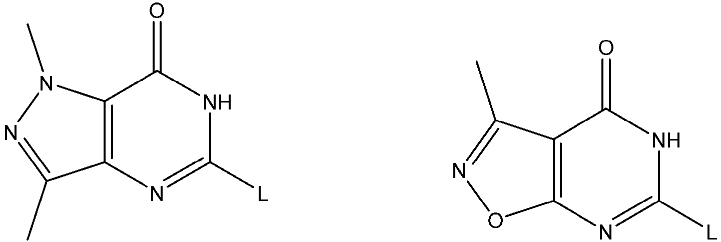

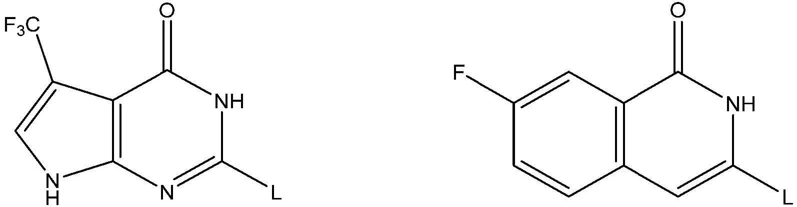

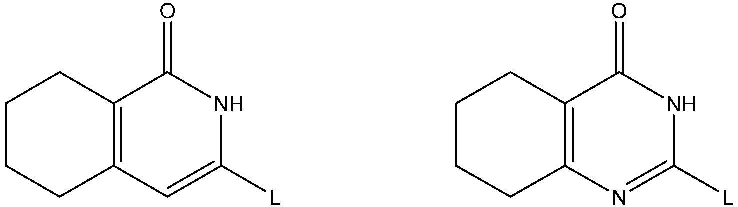

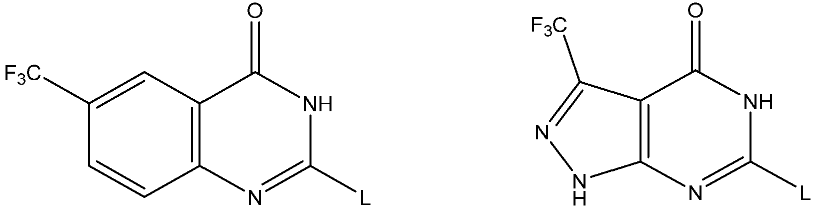

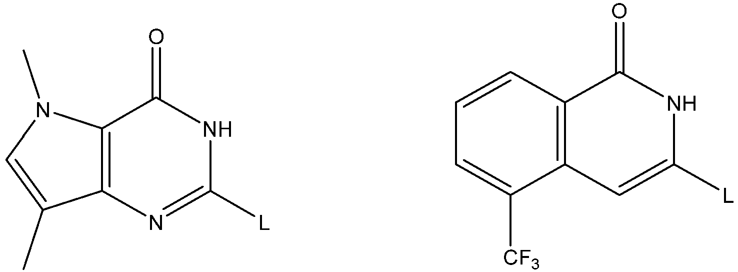

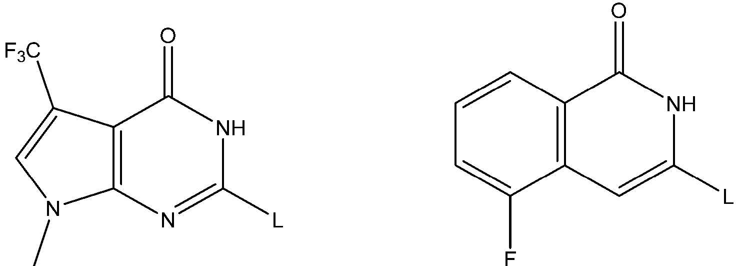

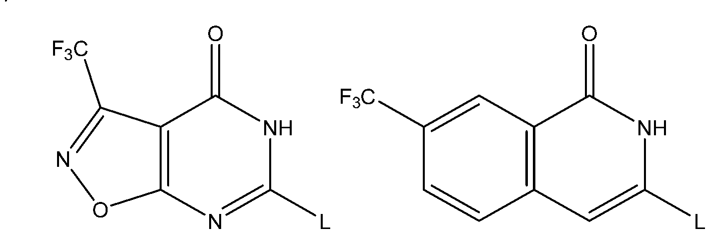

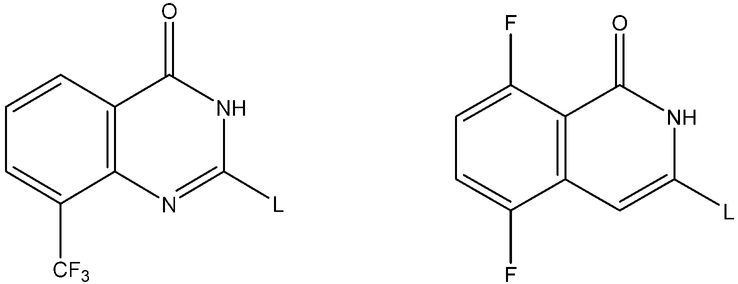

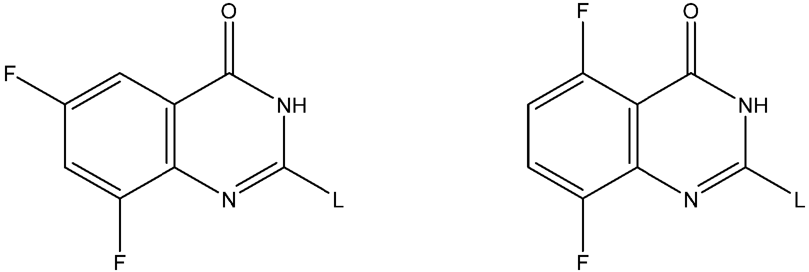

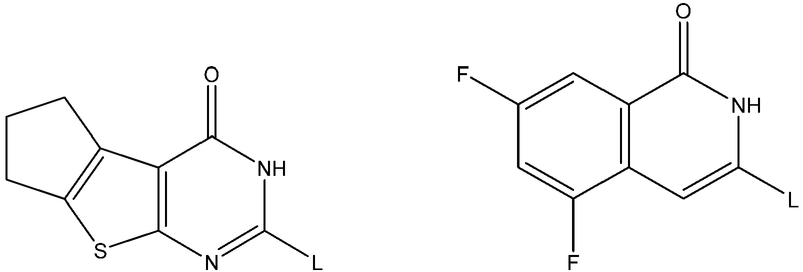

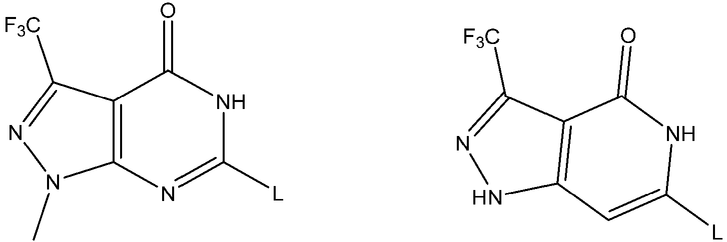

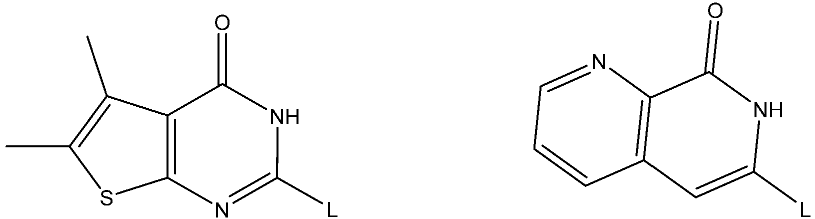

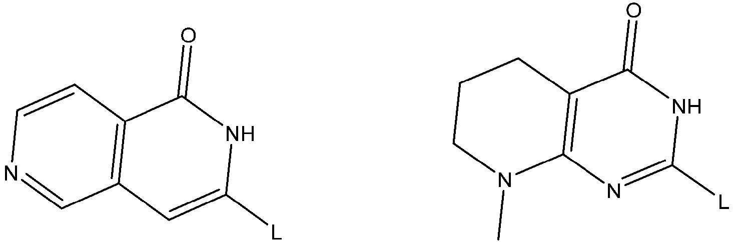

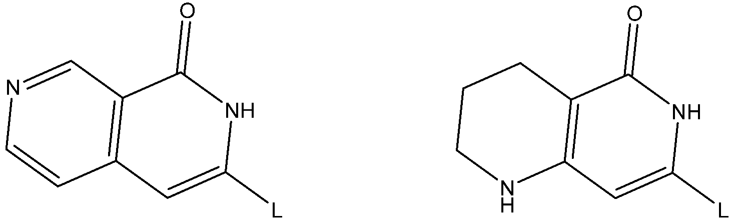

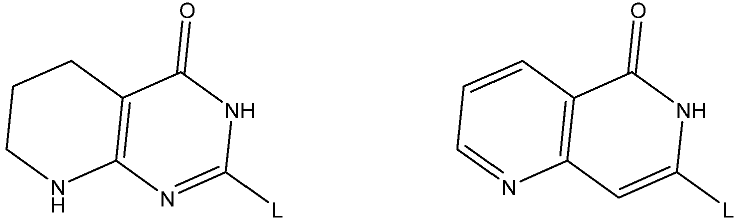

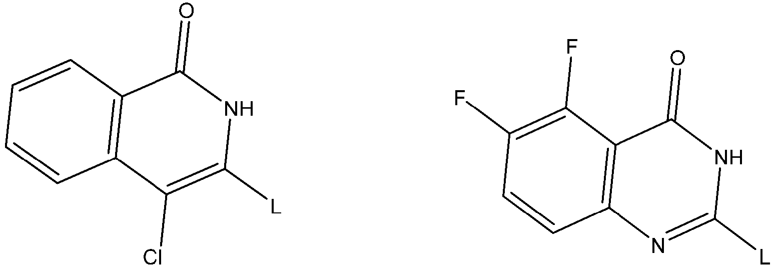

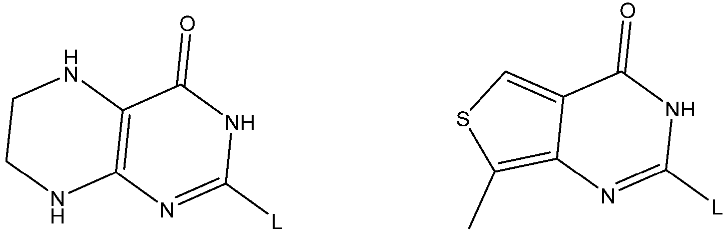

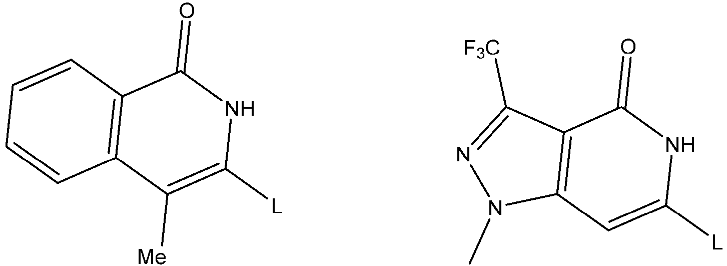

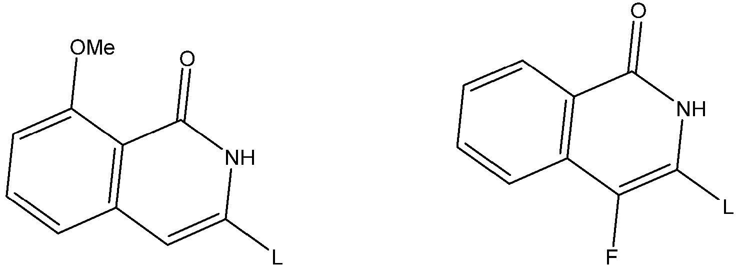

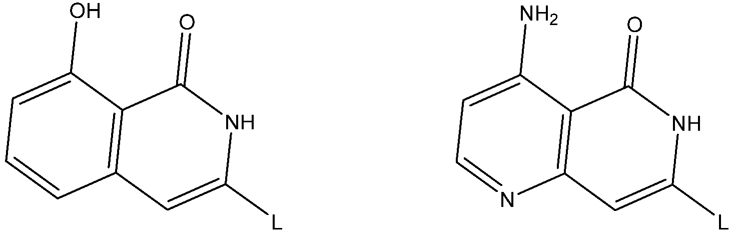

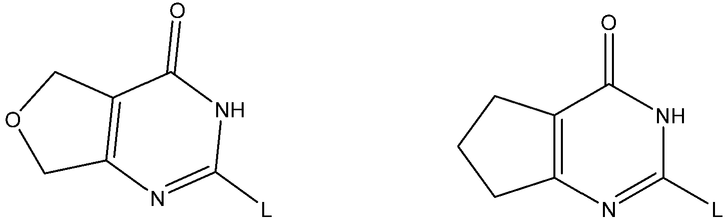

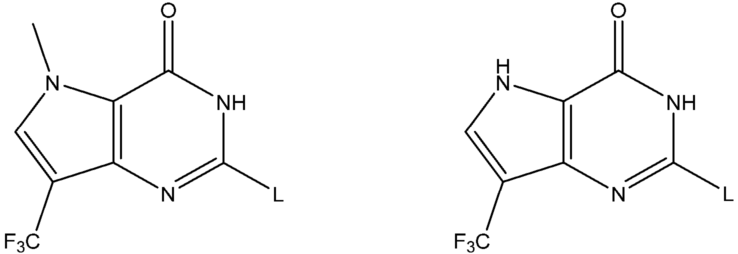

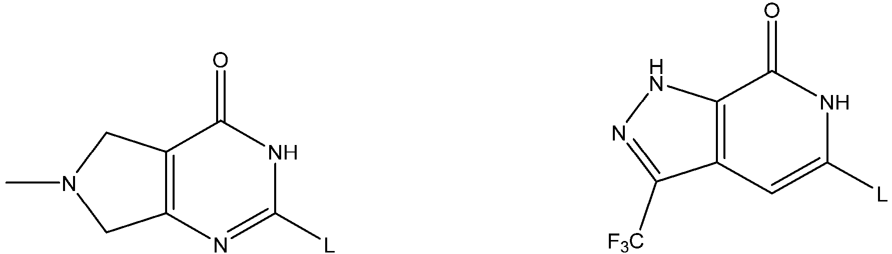

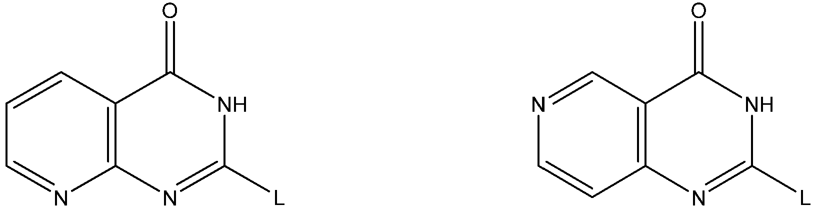

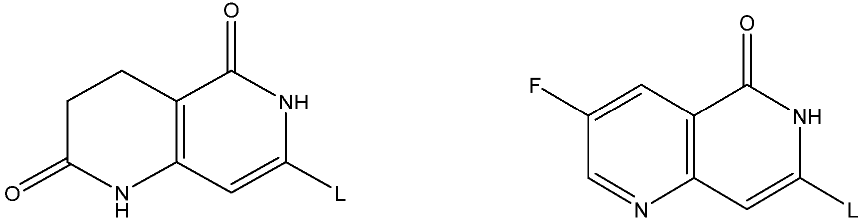

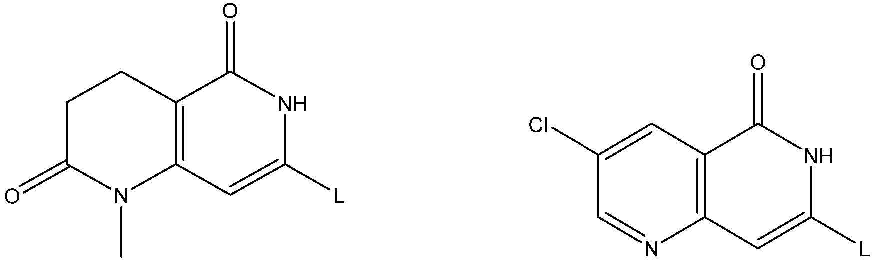

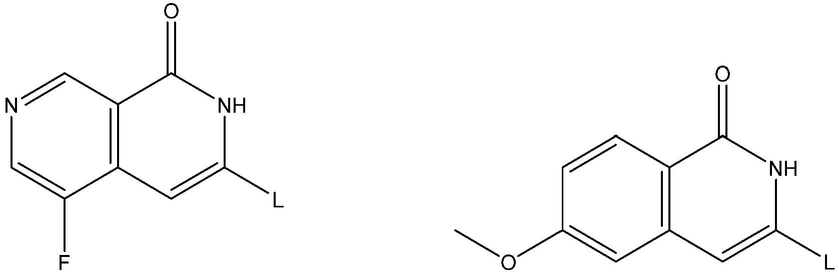

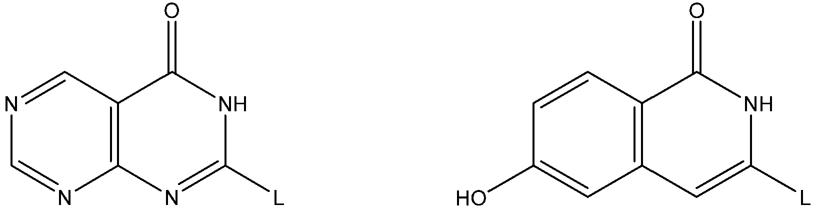

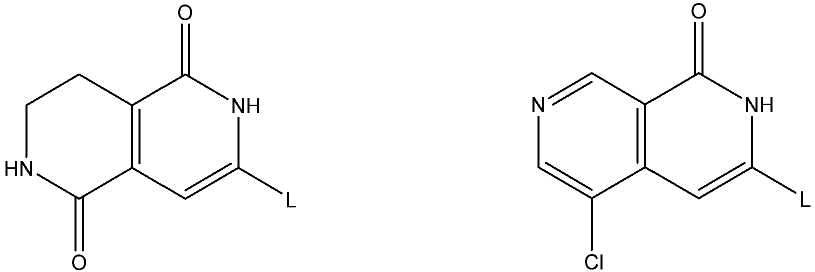

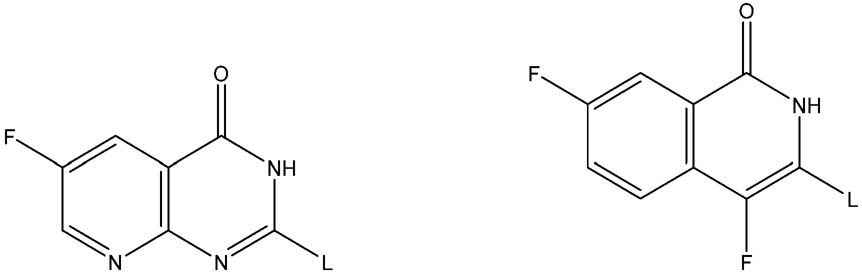

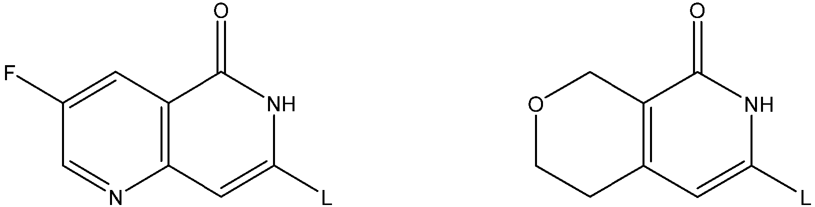

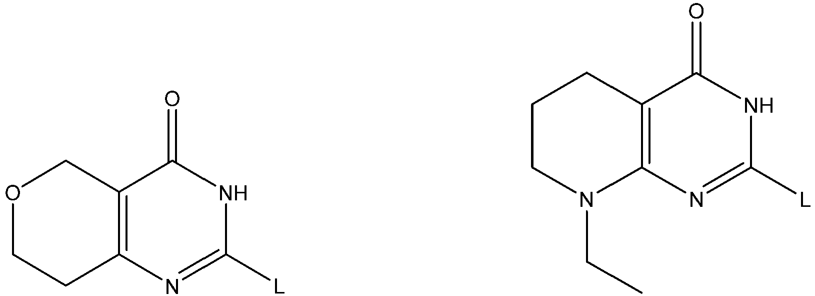

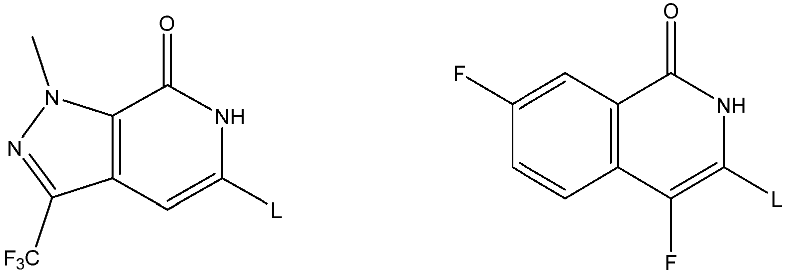

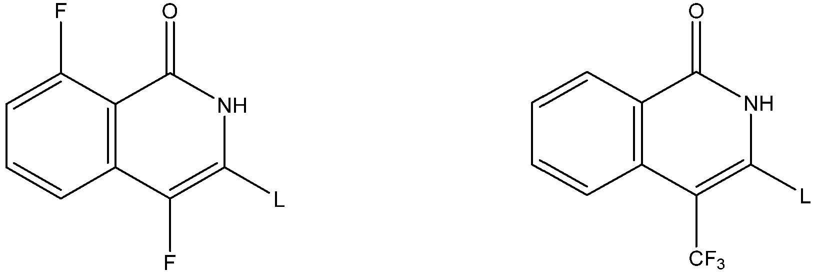

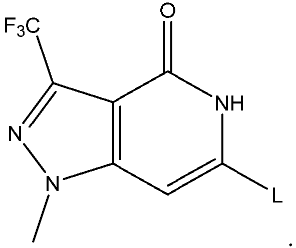

20. Un composé destiné à être utilisé selon l'une quelconque des revendications précédentes, lequel composé comprend une formule sélectionnée parmi l'une des suivantes :

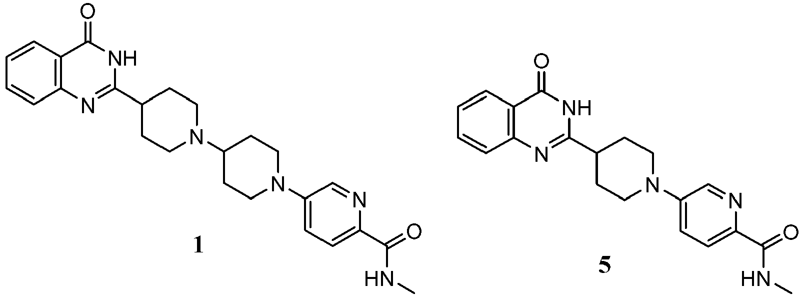

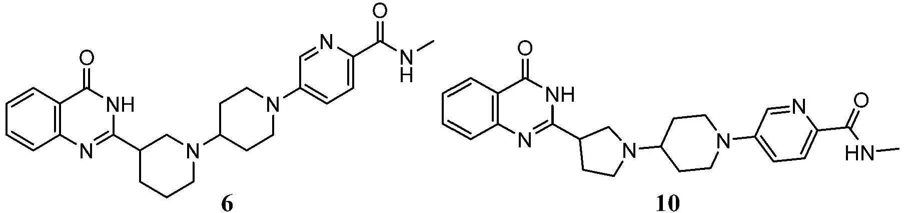

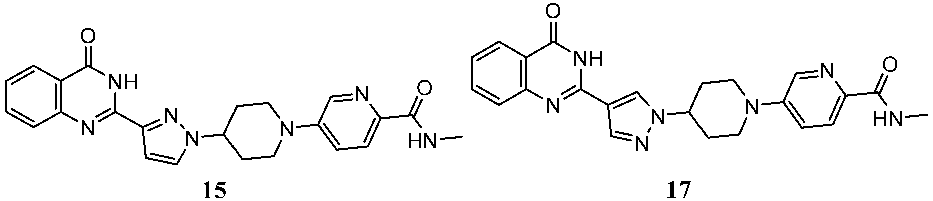

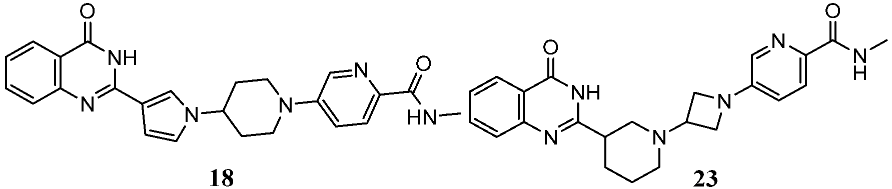

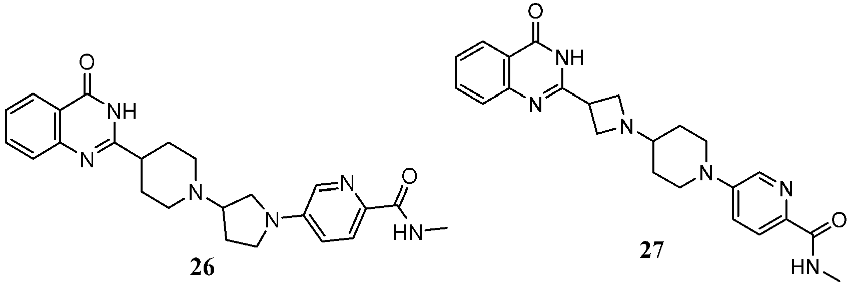

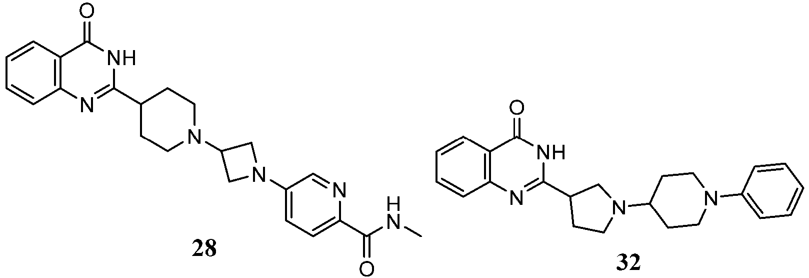

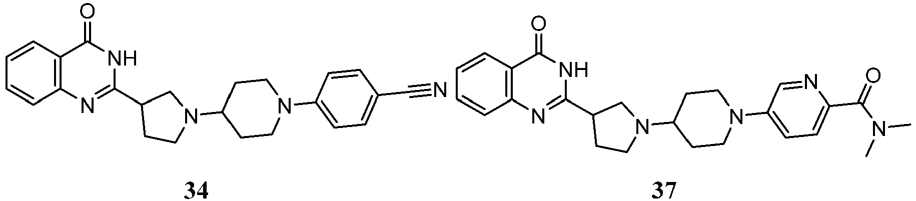

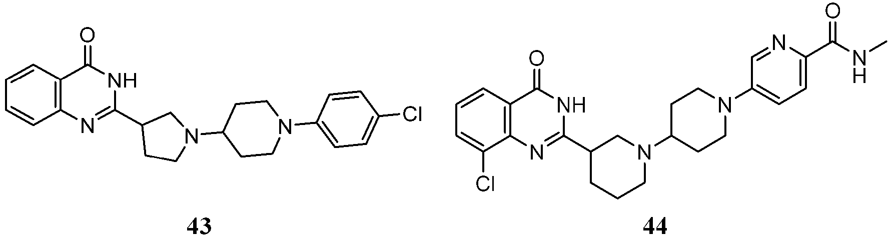

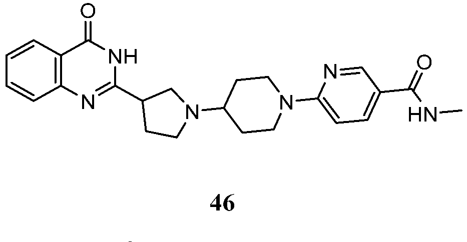

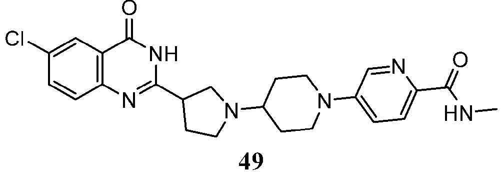

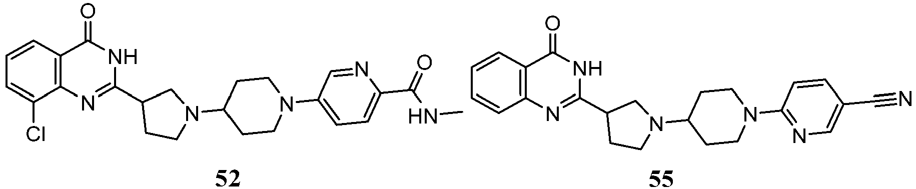

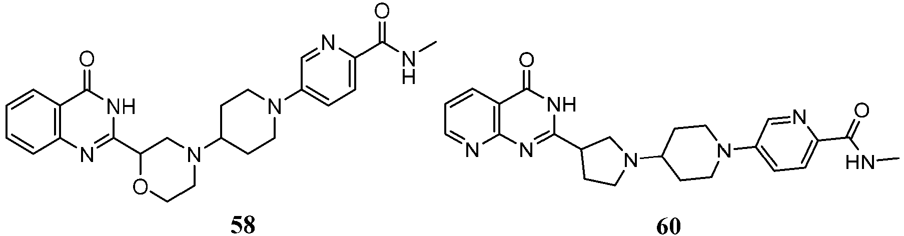

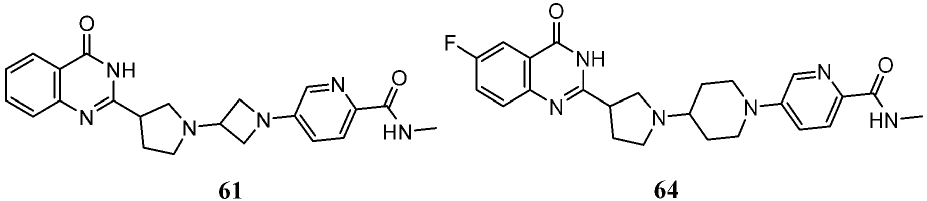

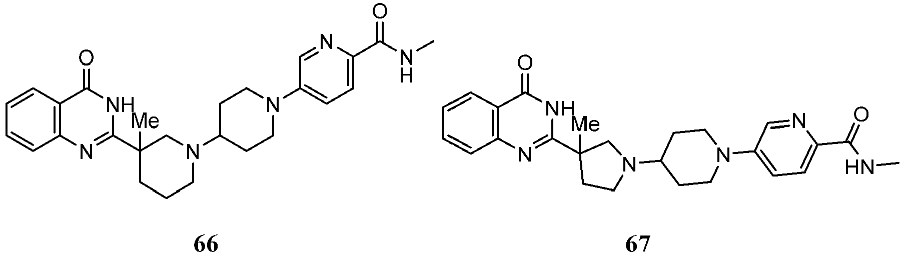

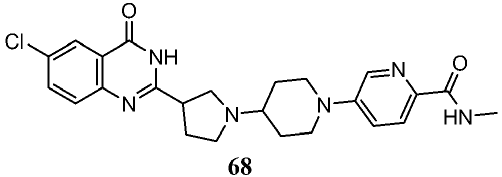

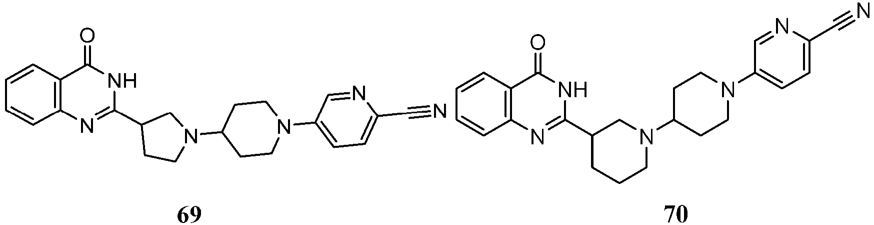

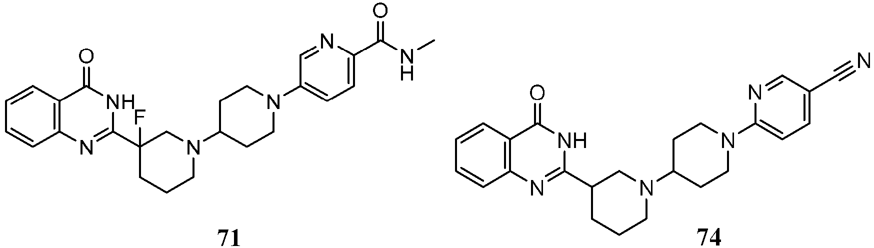

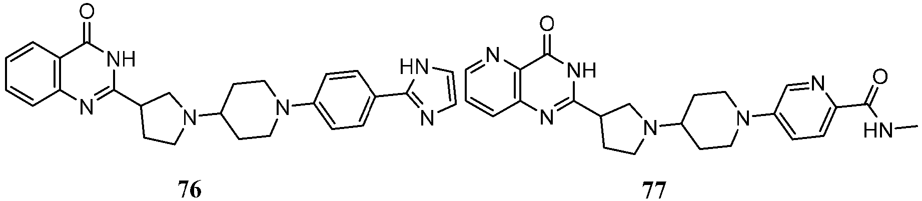

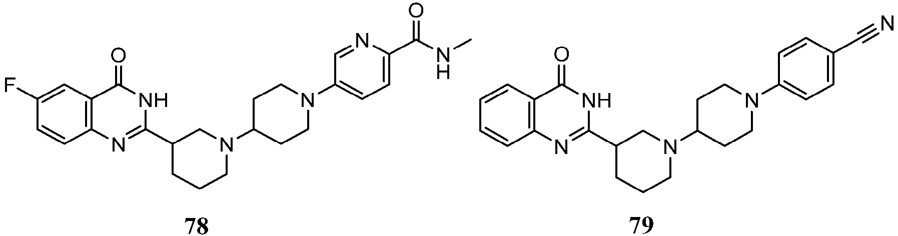

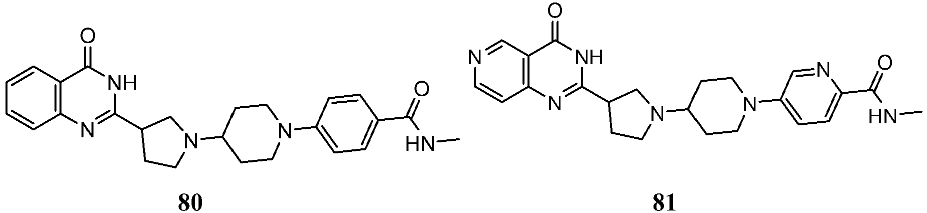

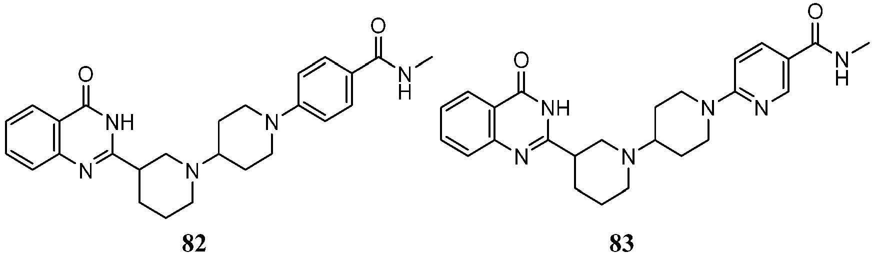

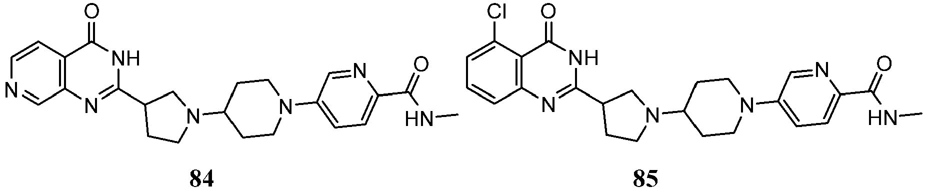

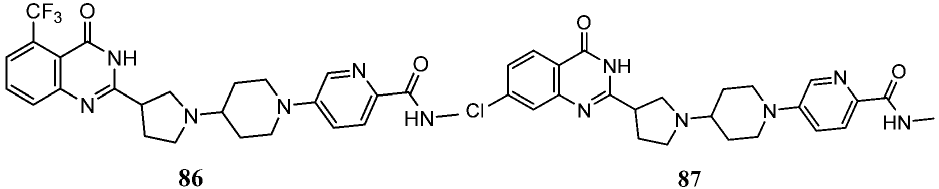

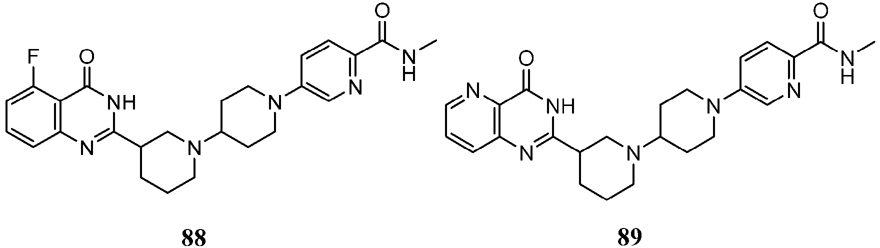

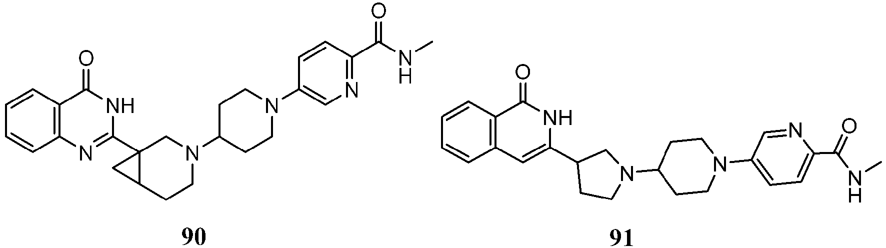

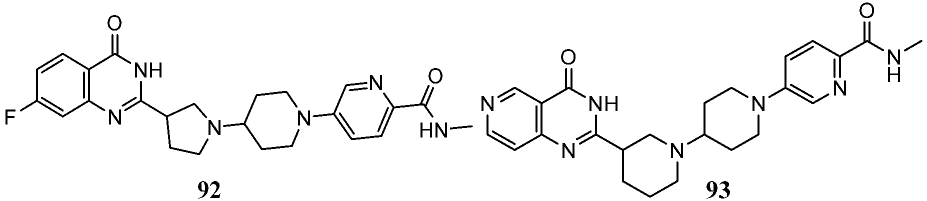

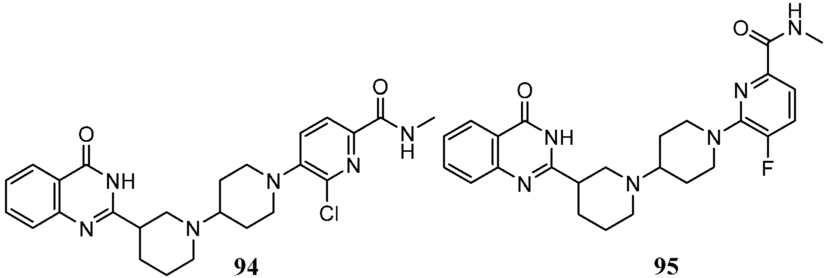

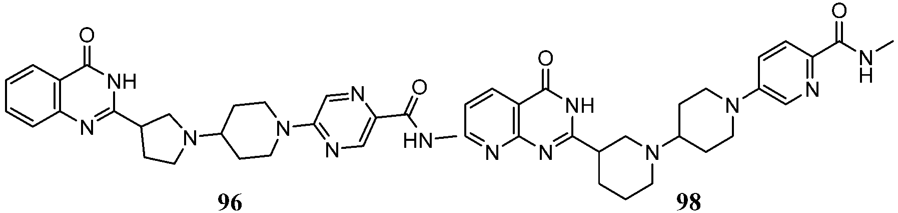

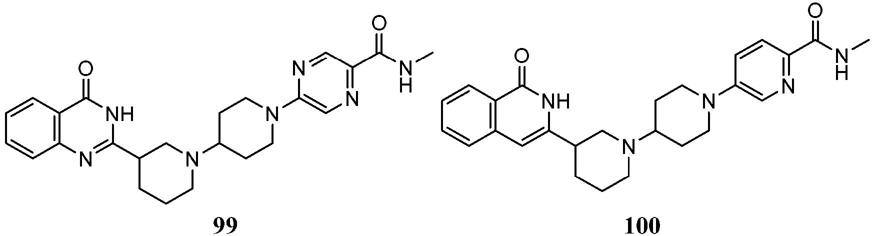

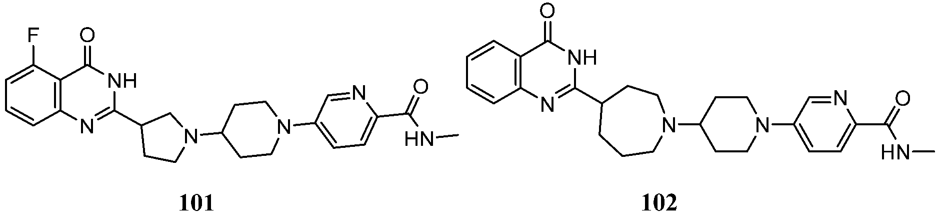

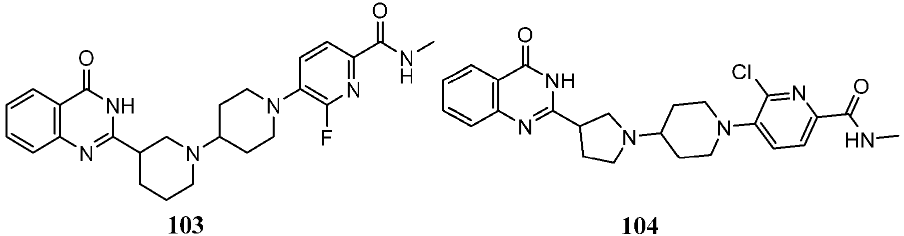

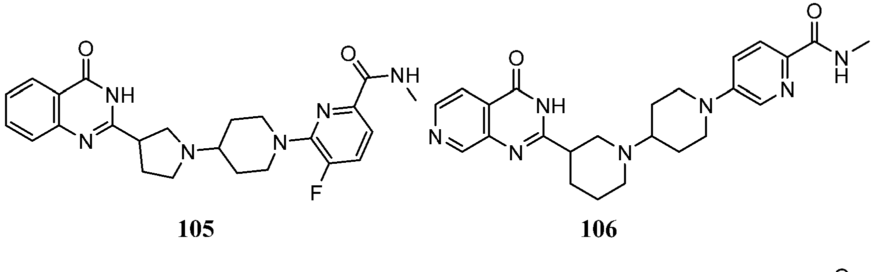

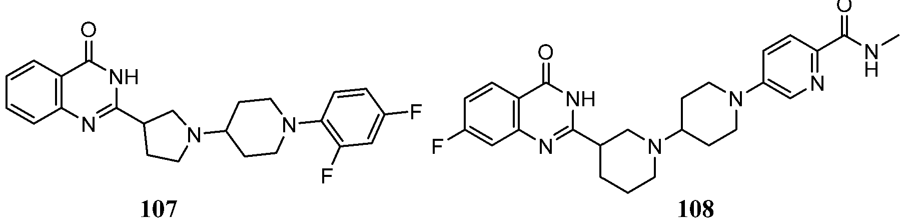

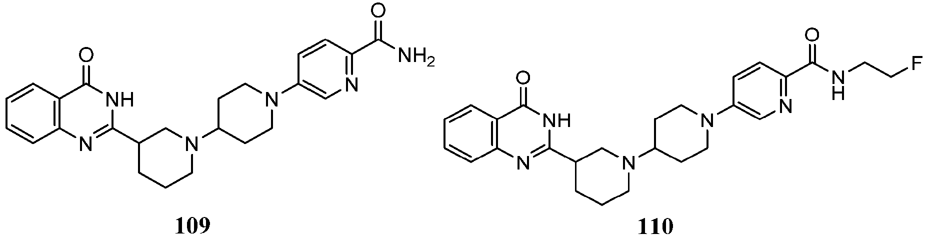

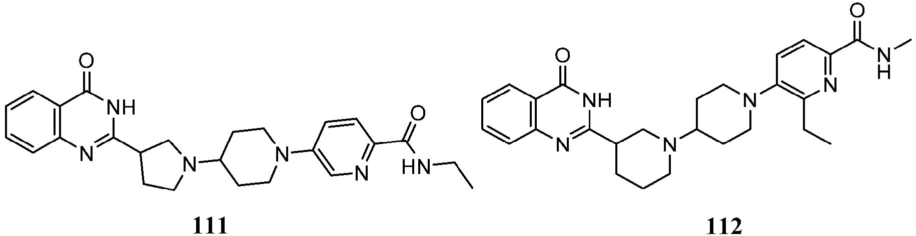

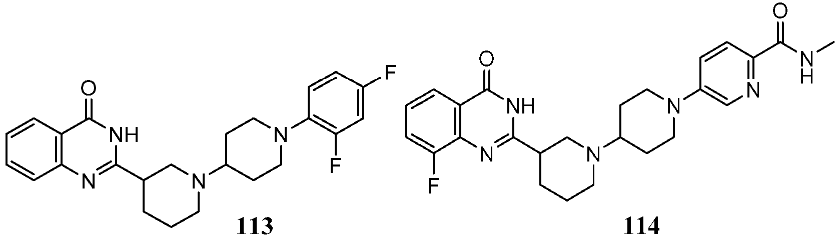

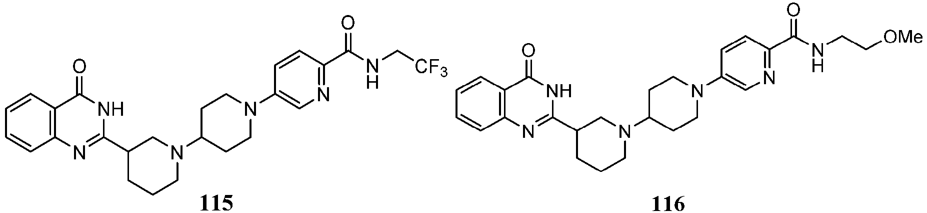

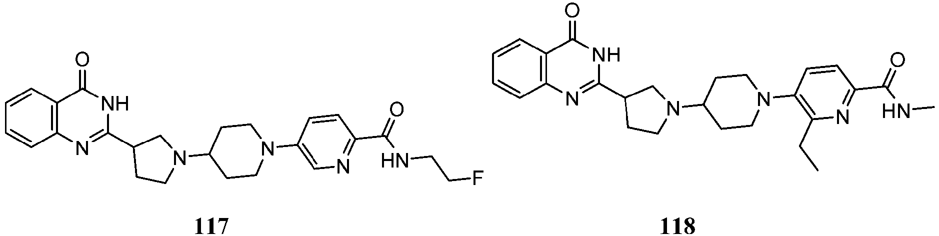

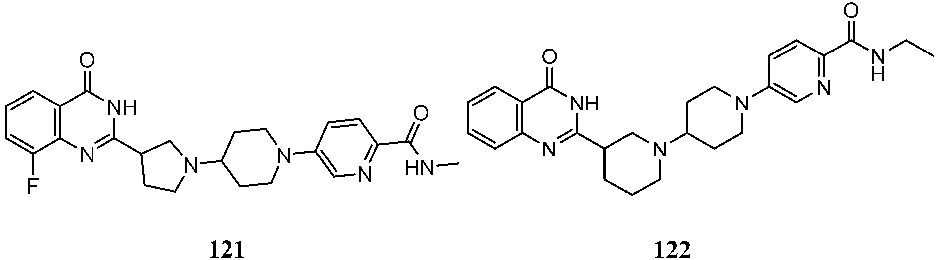

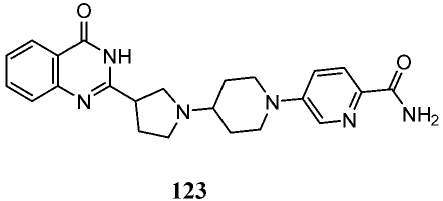

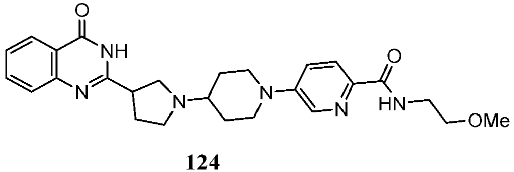

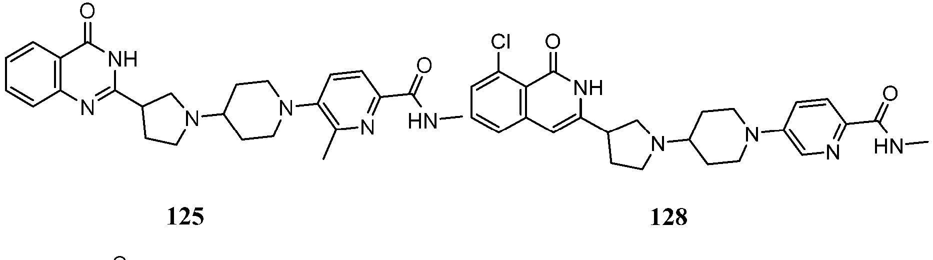

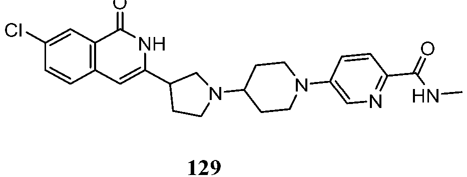

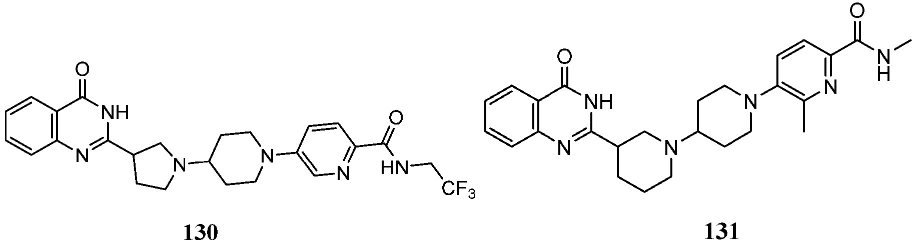

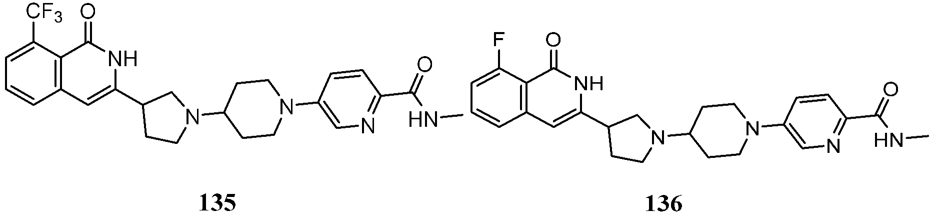

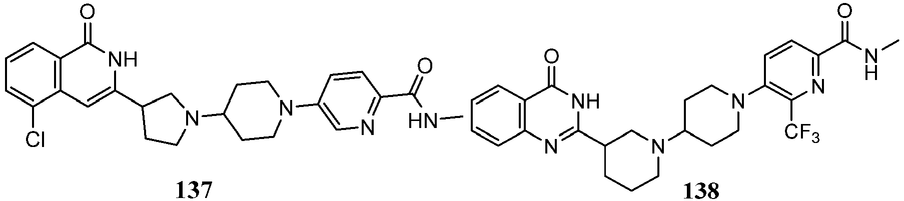

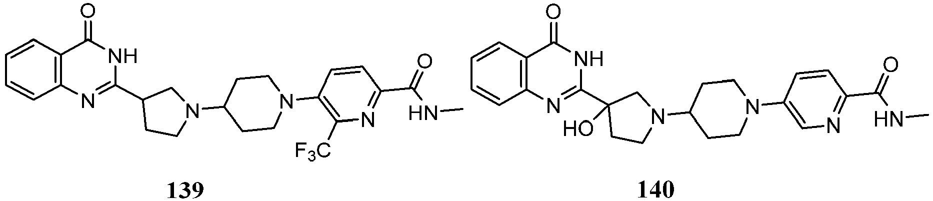

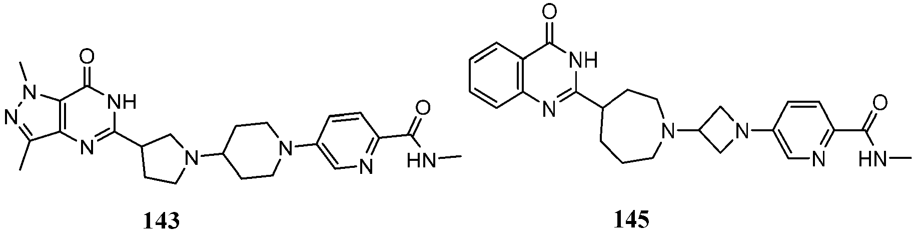

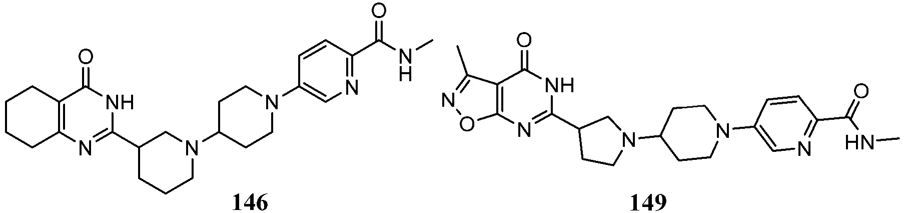

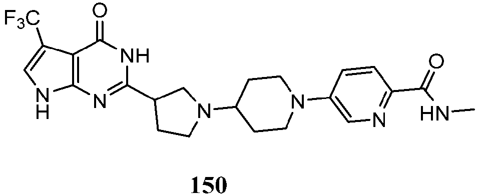

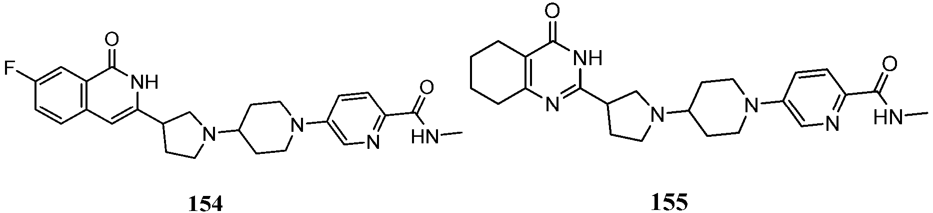

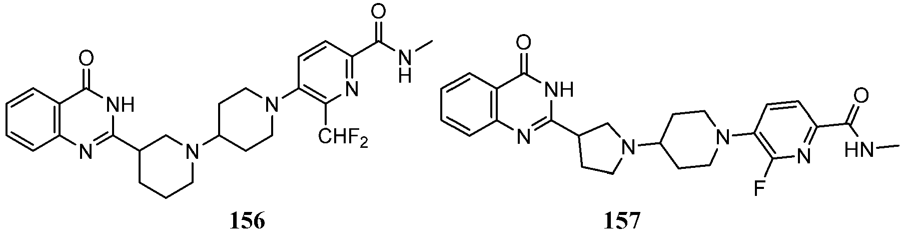

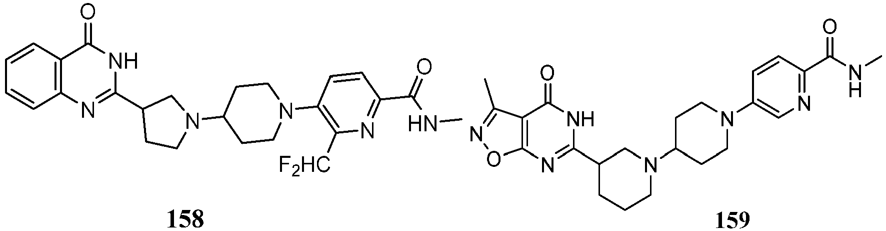

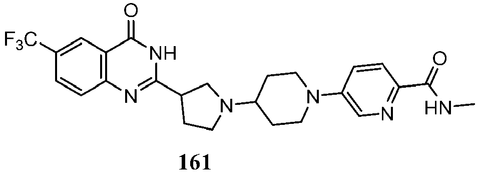

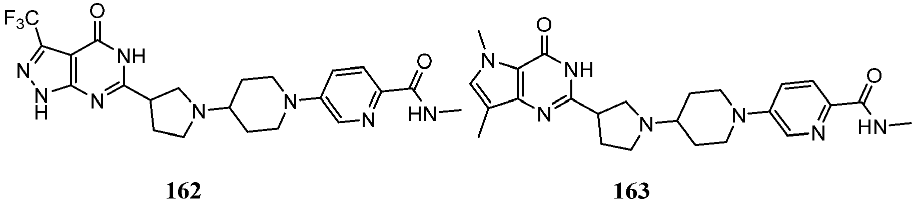

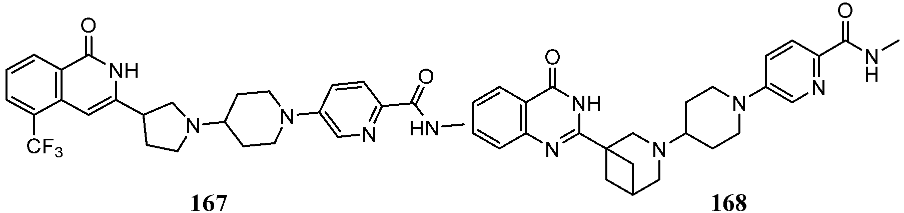

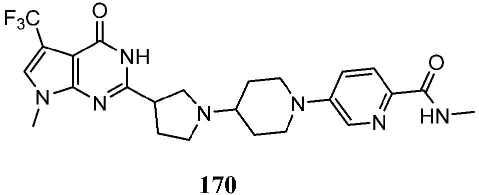

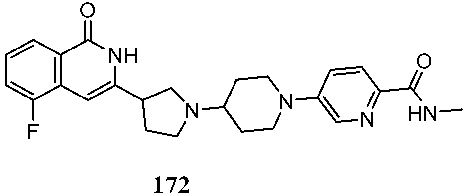

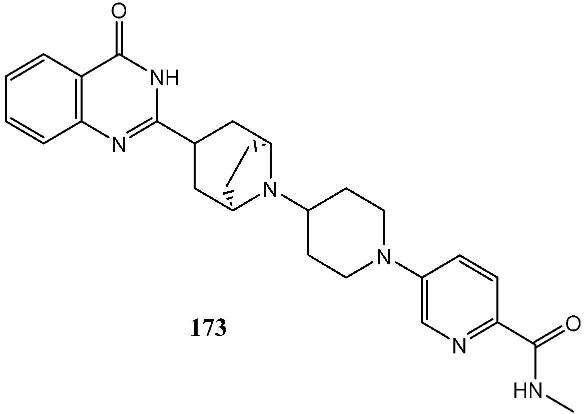

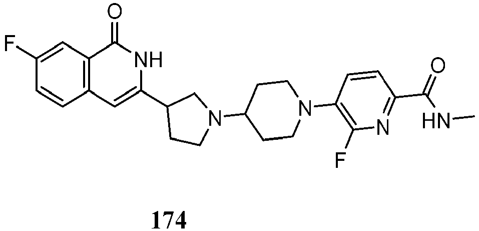

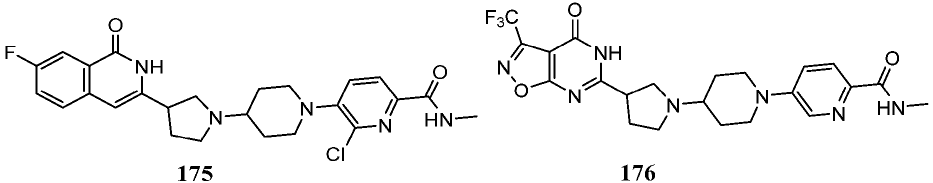

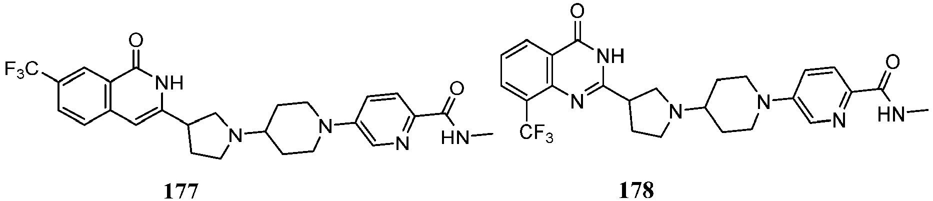

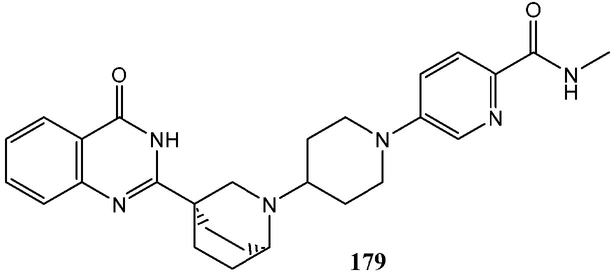

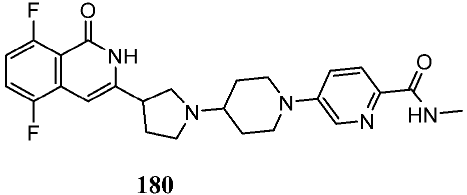

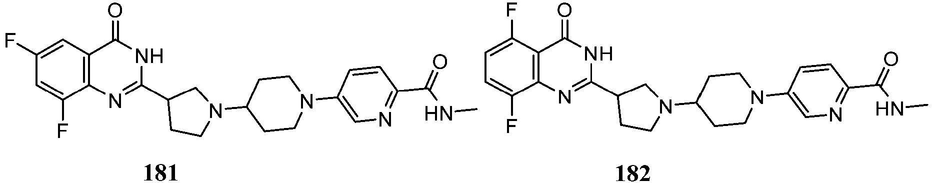

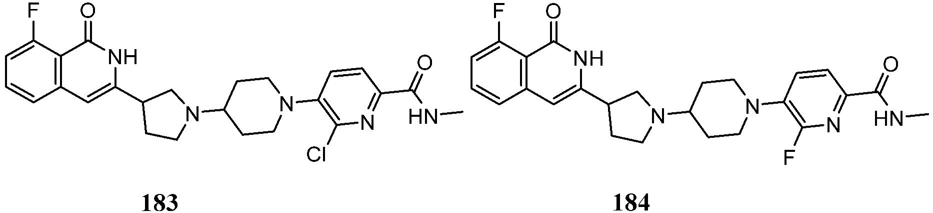

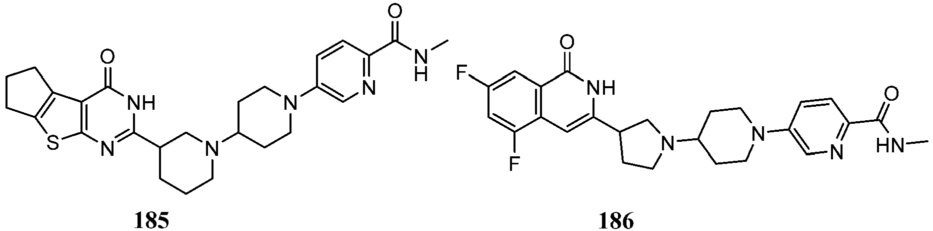

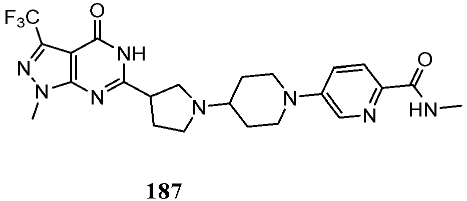

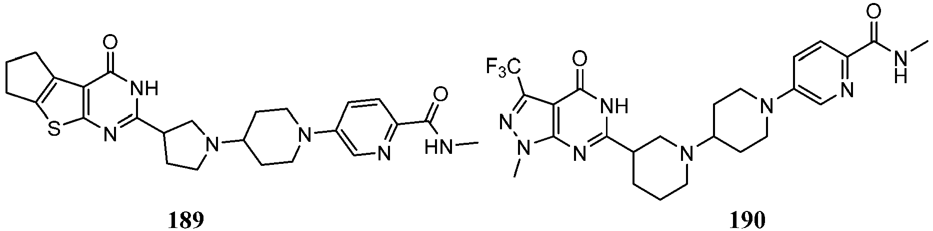

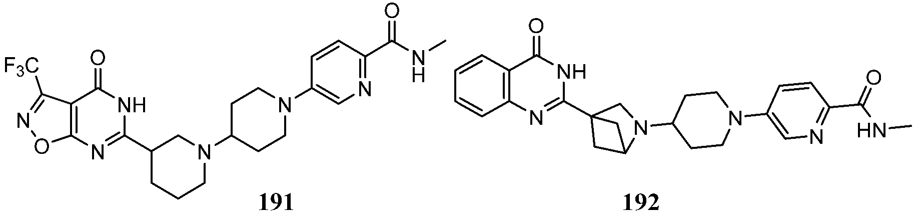

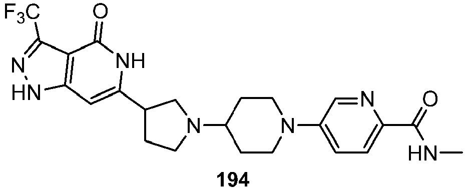

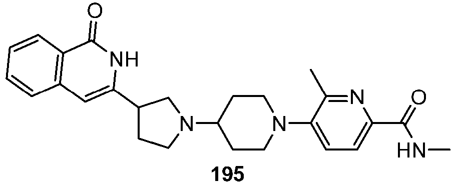

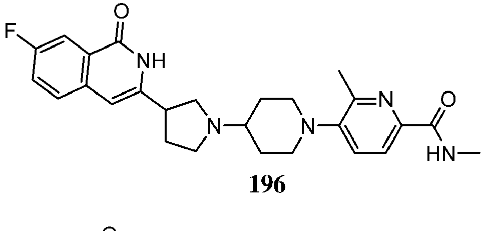

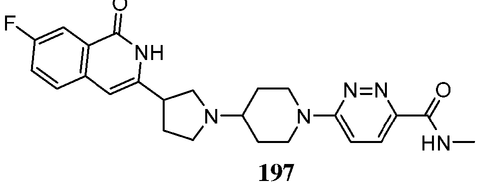

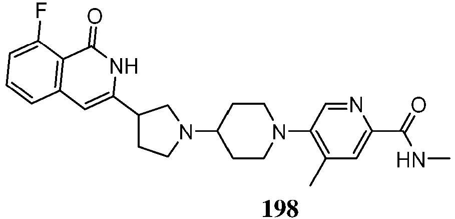

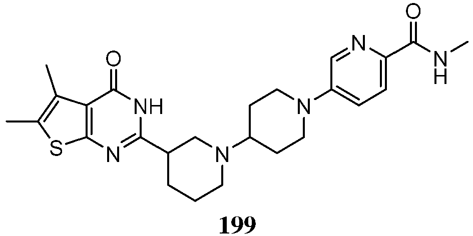

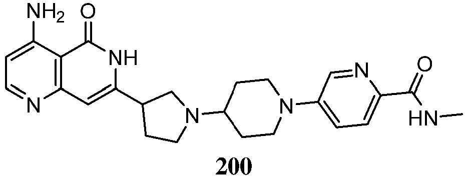

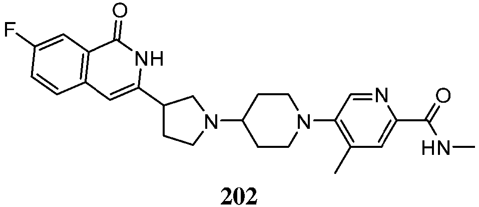

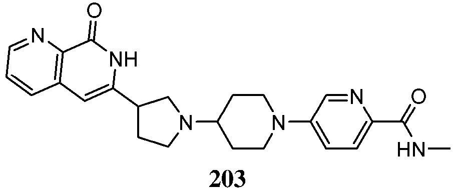

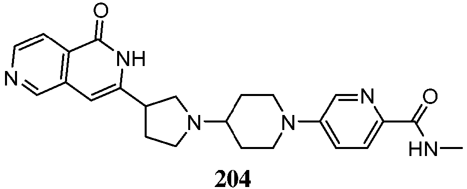

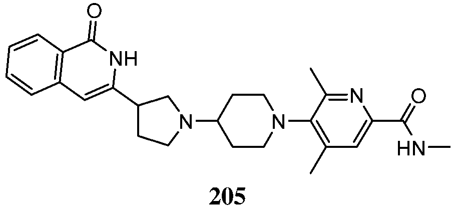

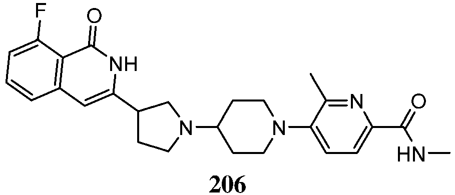

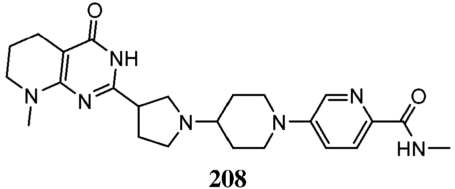

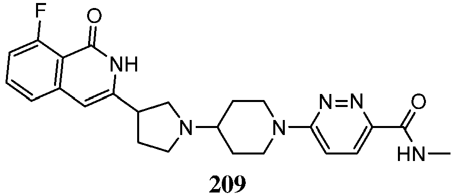

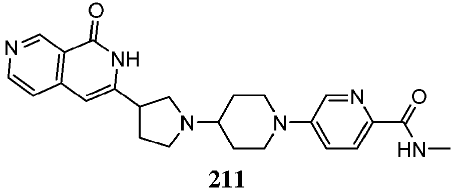

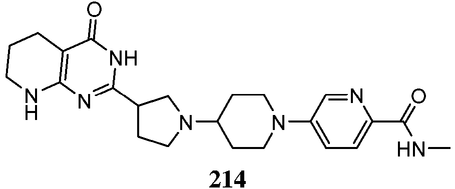

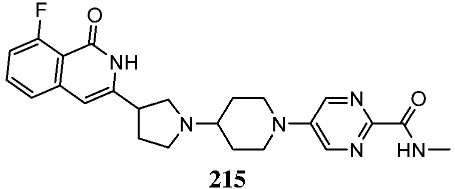

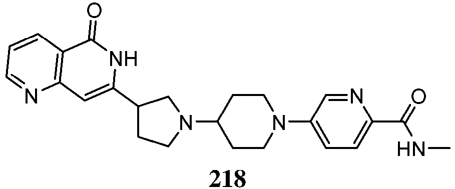

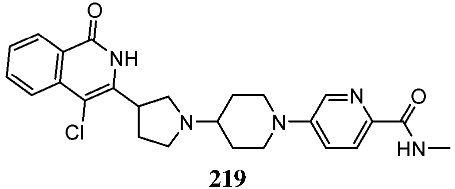

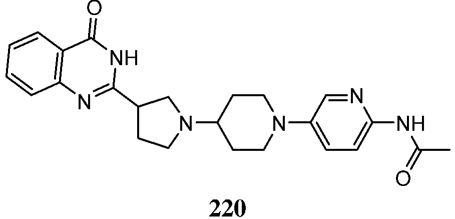

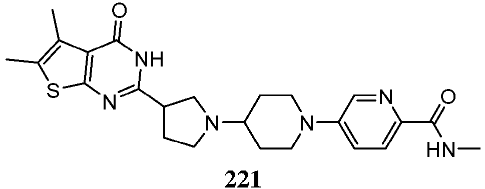

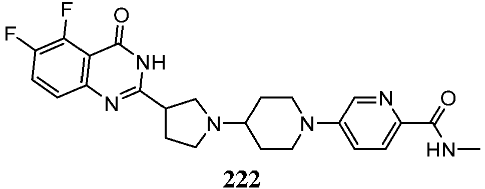

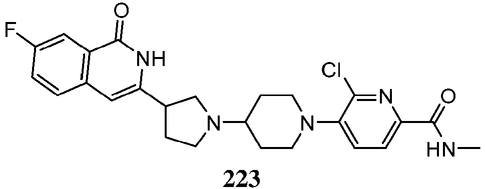

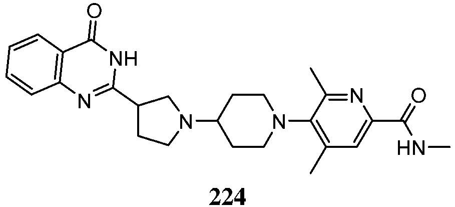

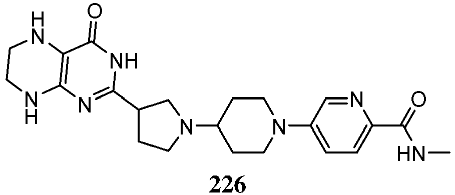

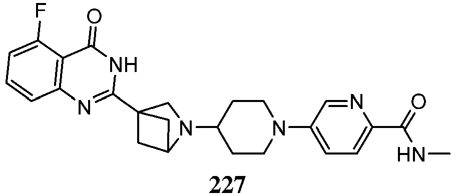

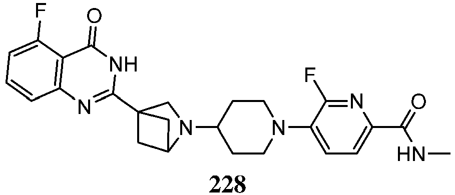

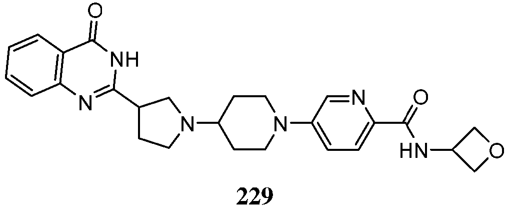

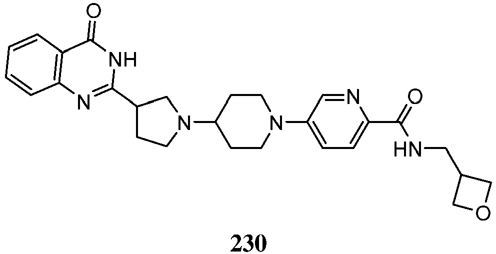

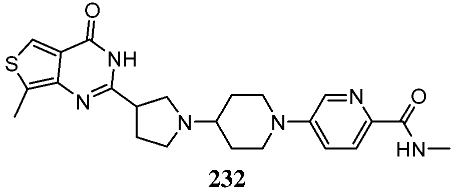

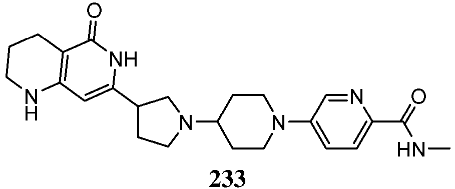

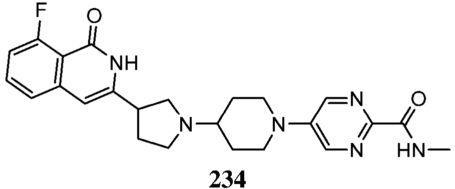

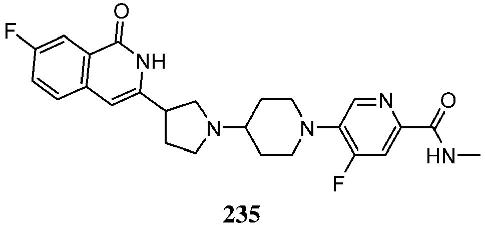

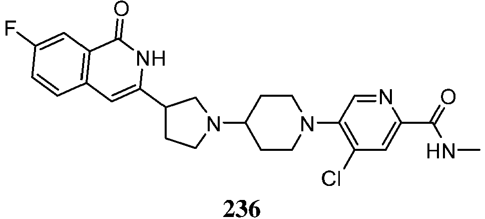

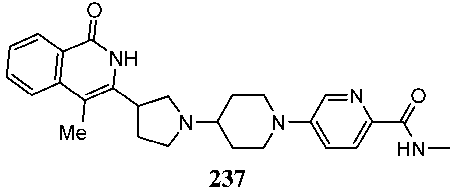

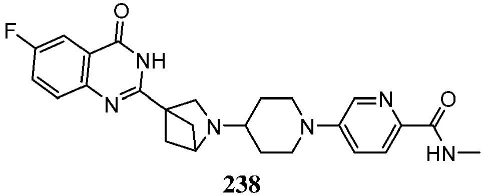

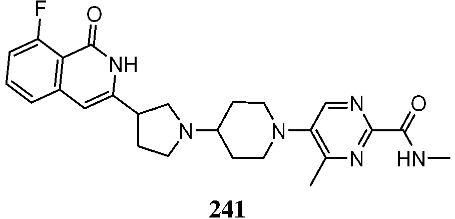

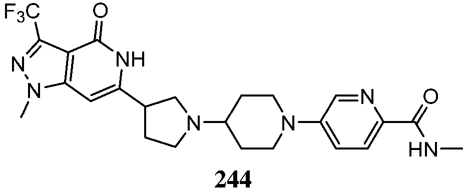

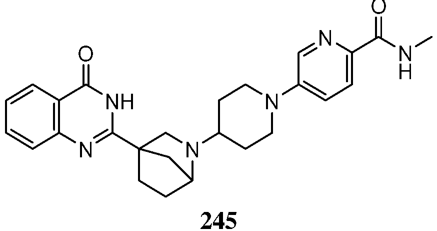

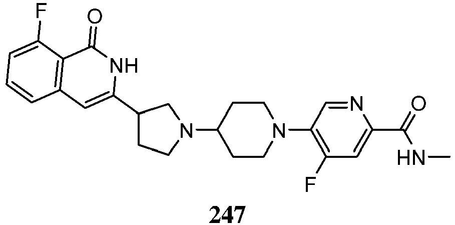

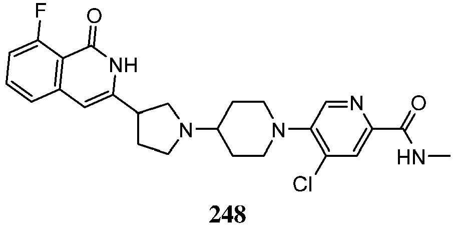

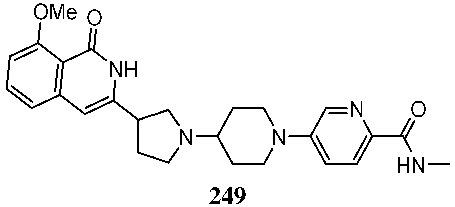

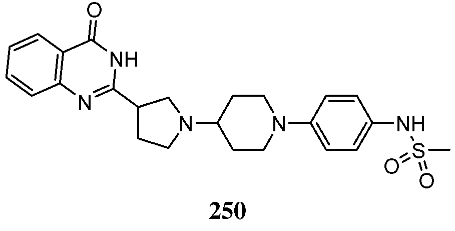

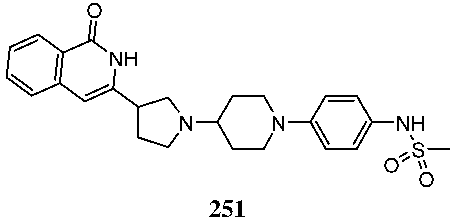

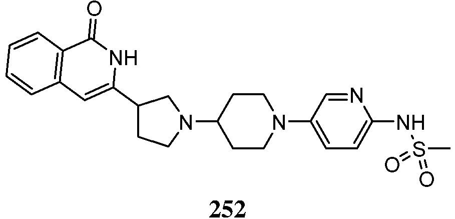

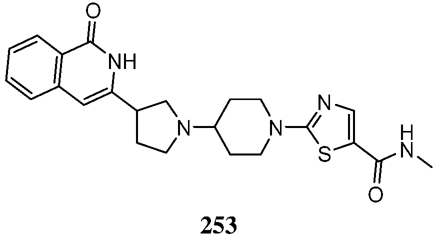

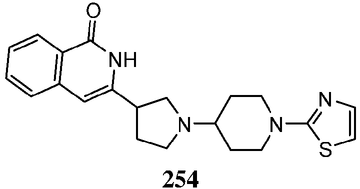

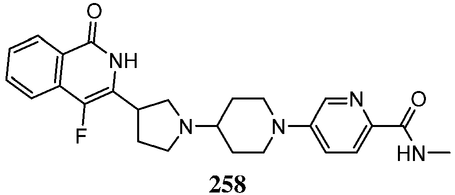

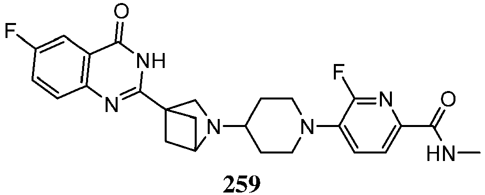

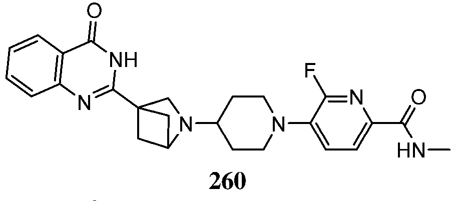

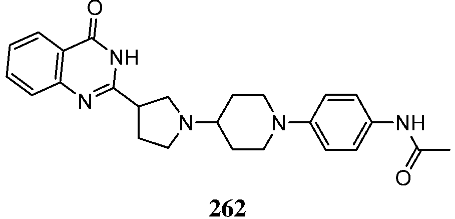

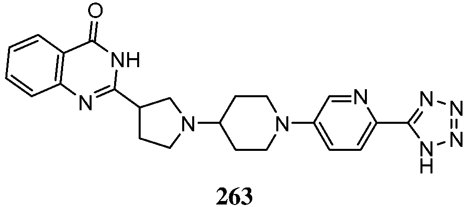

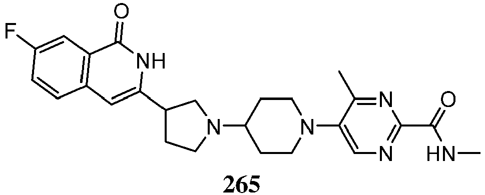

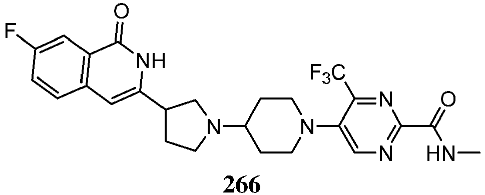

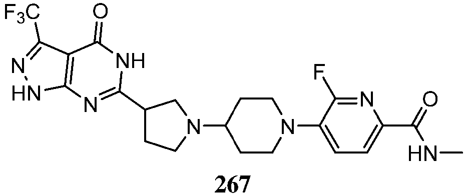

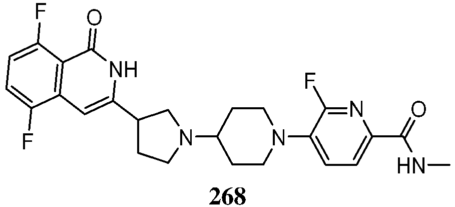

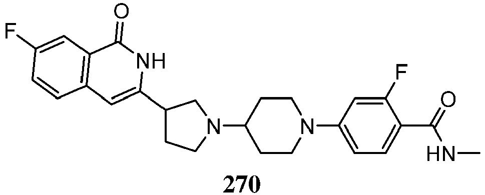

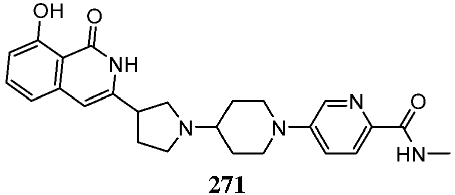

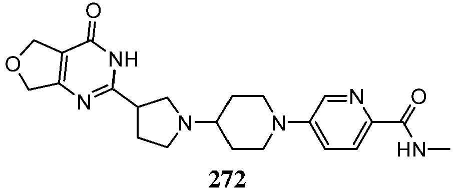

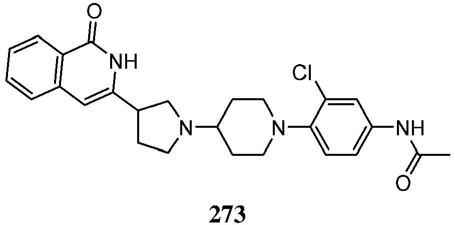

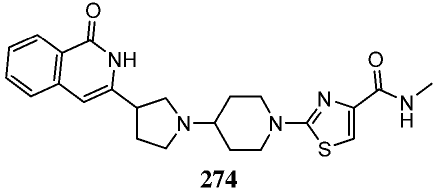

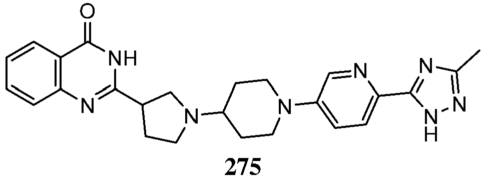

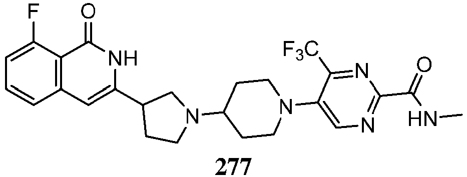

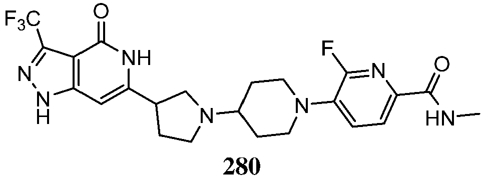

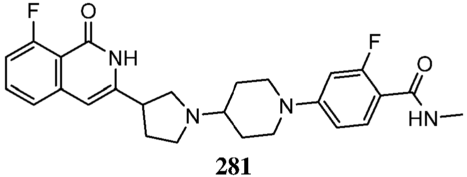

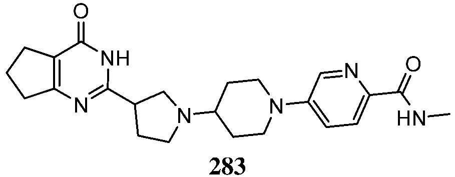

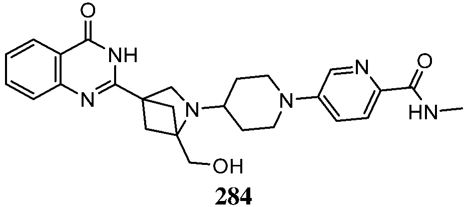

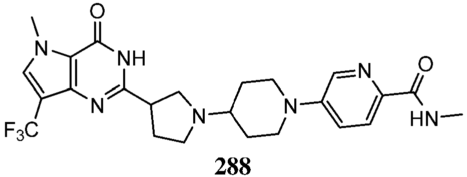

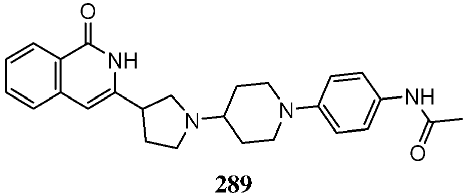

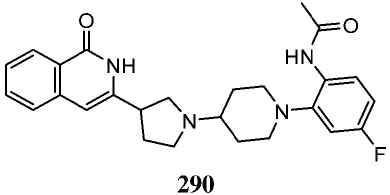

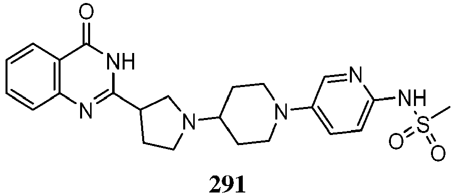

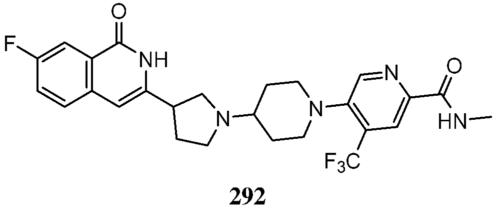

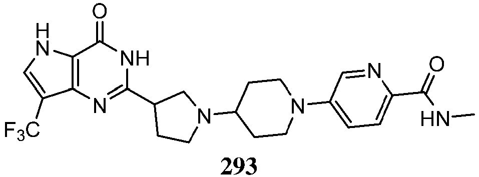

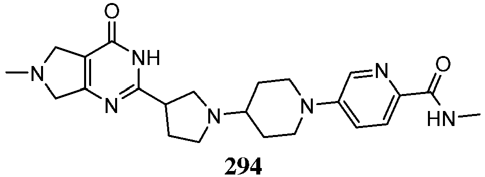

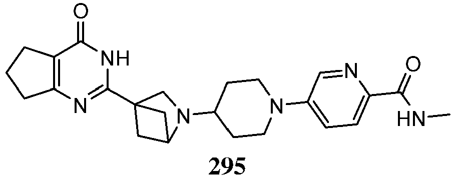

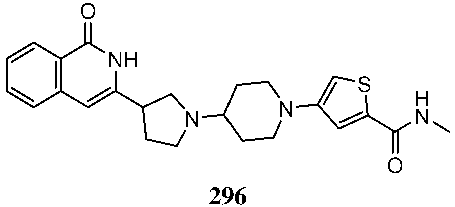

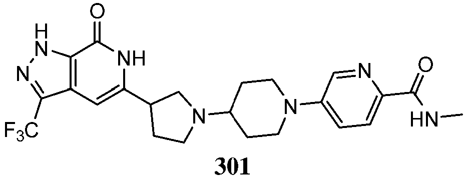

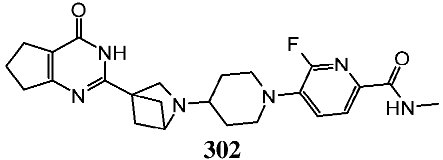

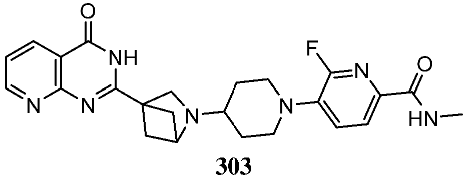

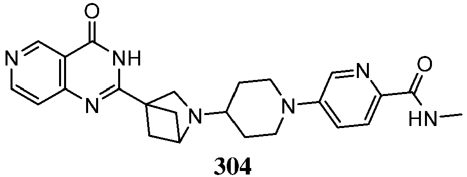

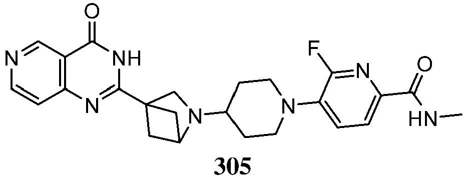

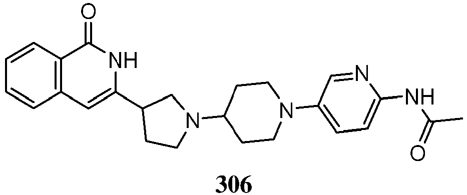

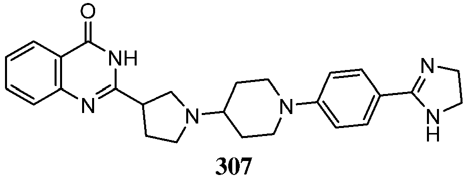

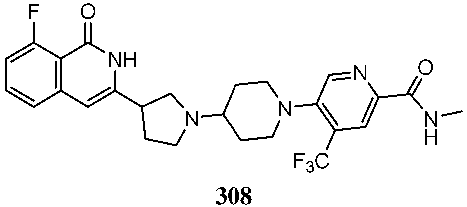

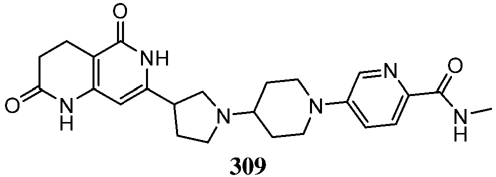

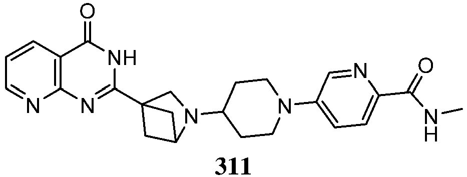

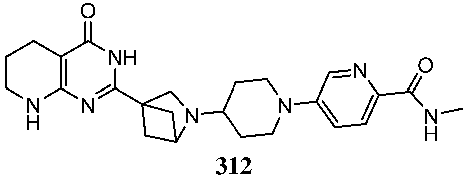

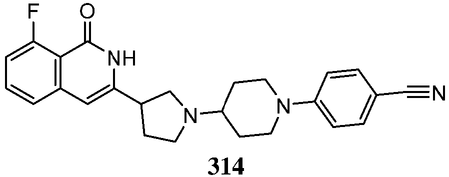

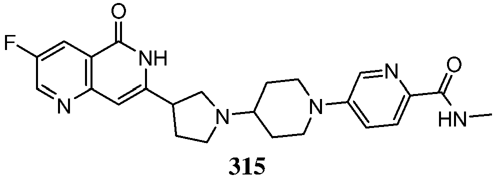

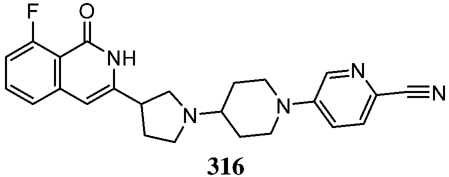

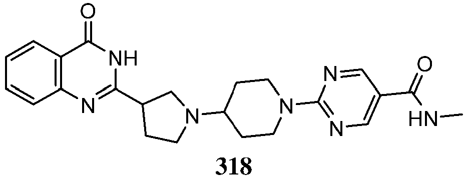

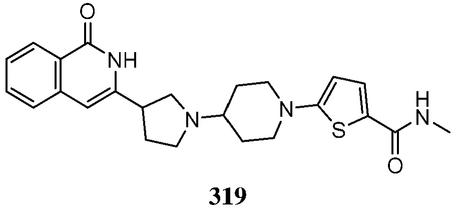

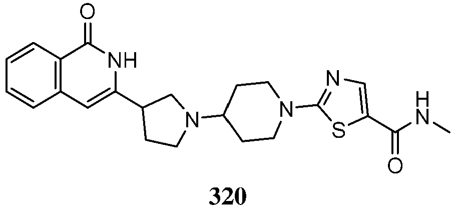

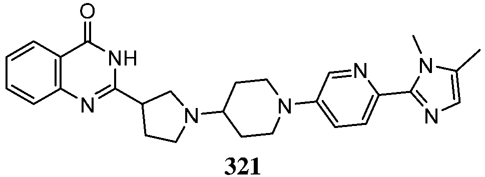

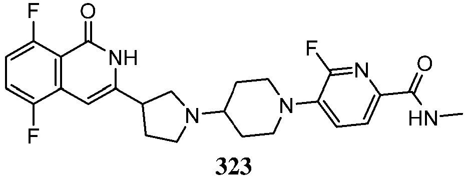

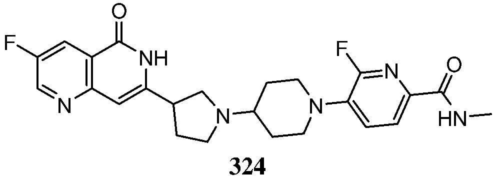

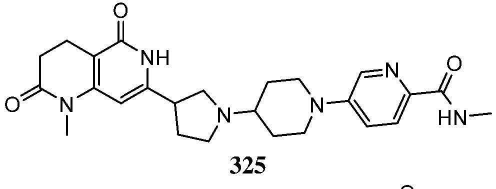

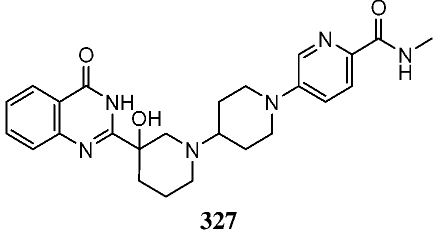

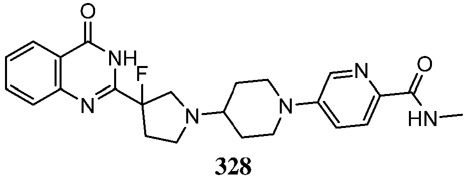

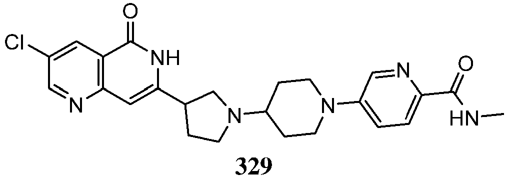

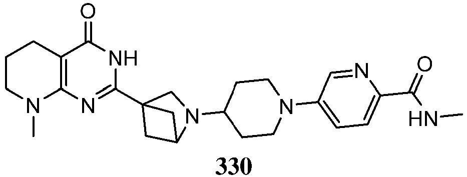

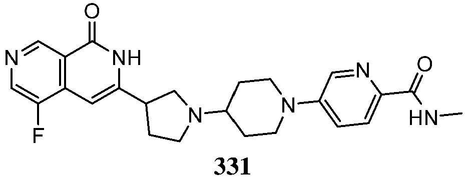

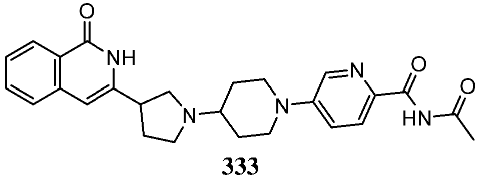

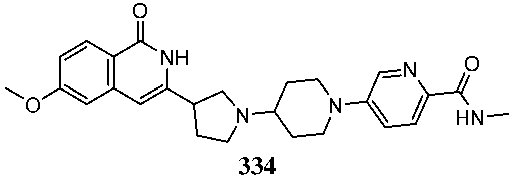

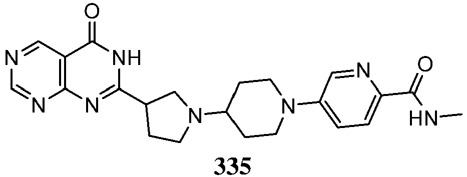

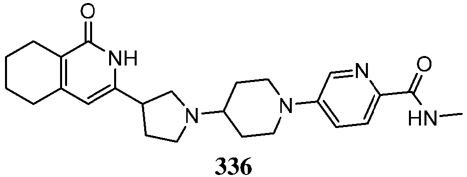

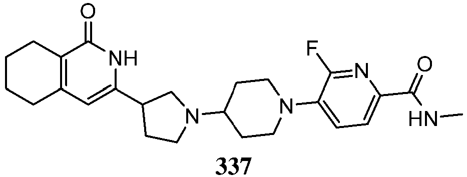

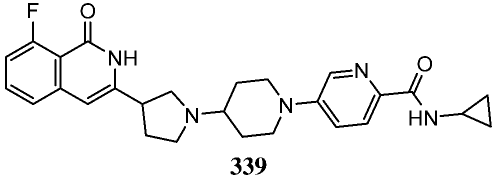

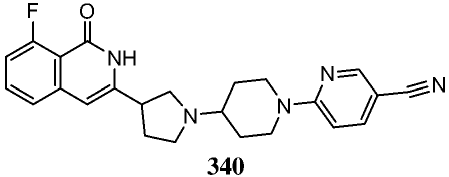

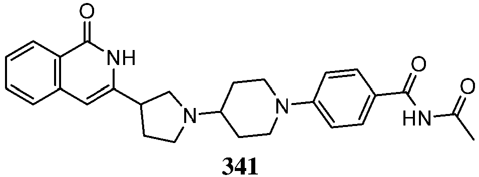

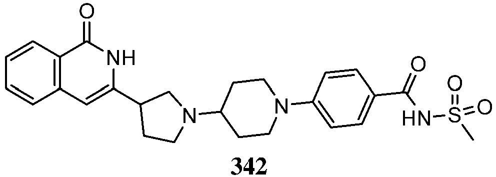

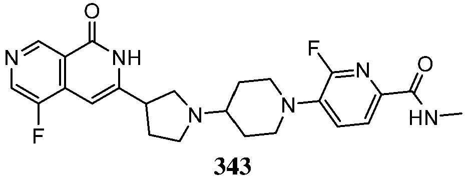

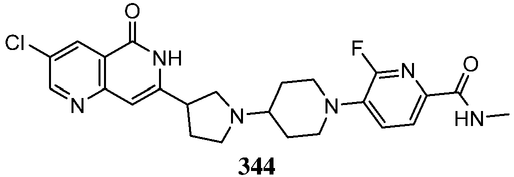

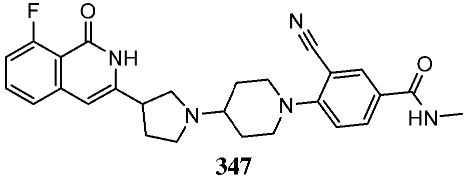

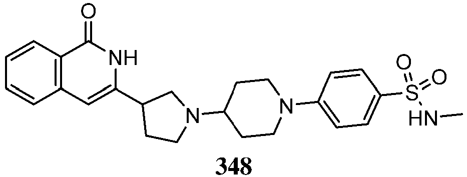

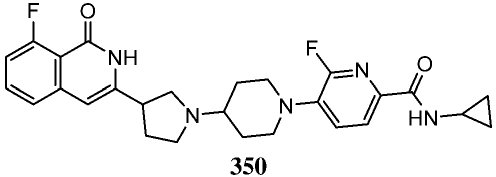

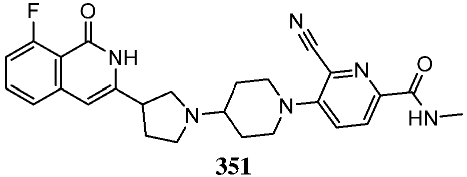

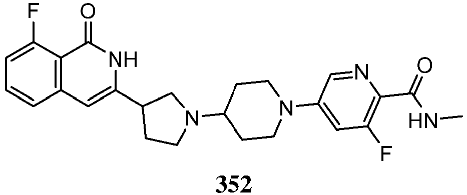

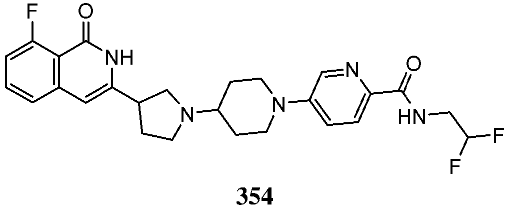

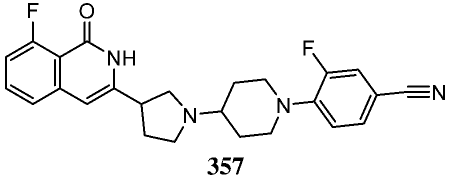

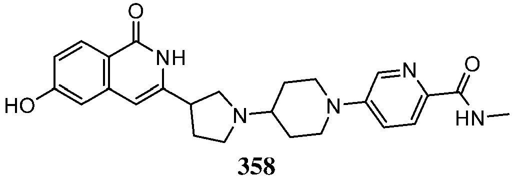

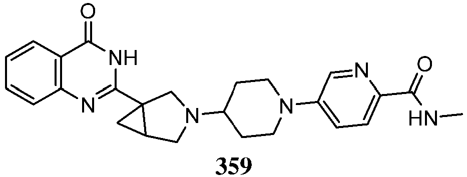

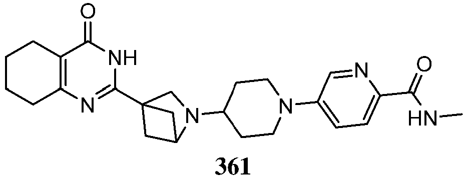

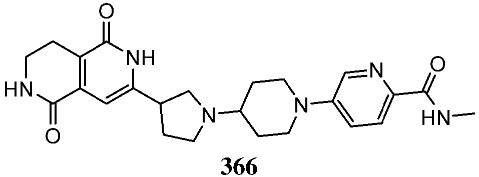

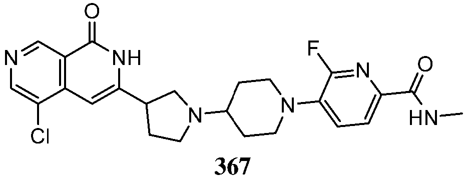

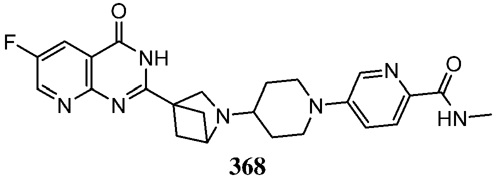

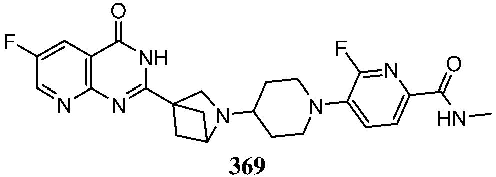

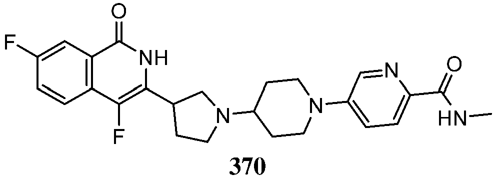

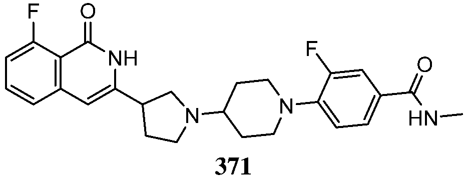

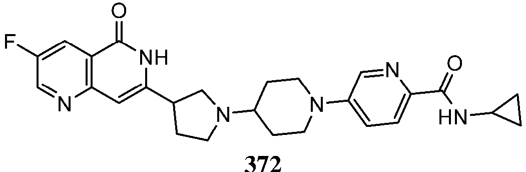

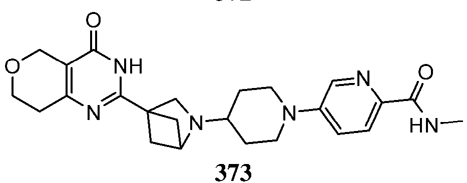

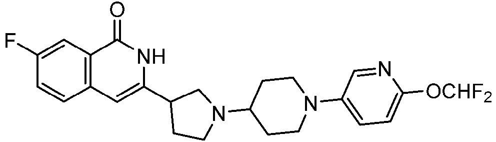

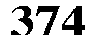

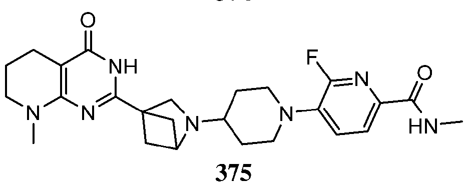

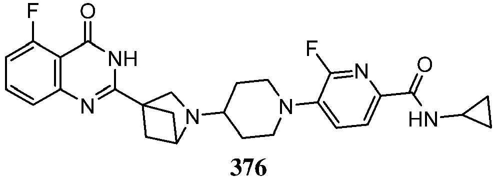

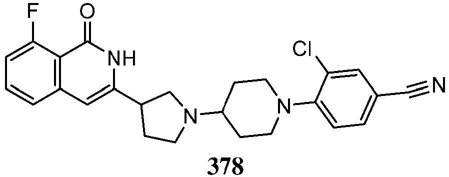

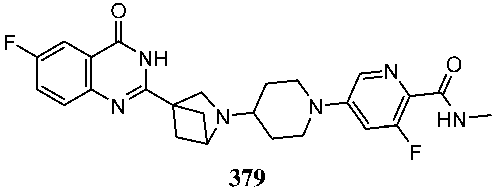

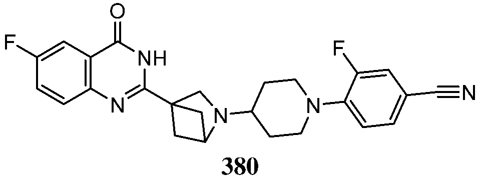

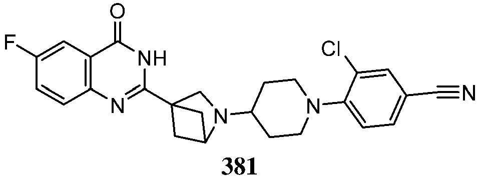

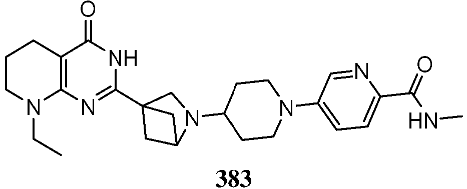

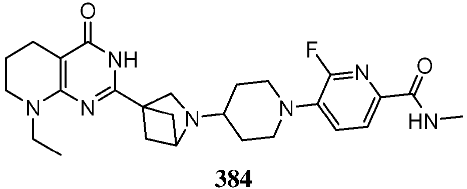

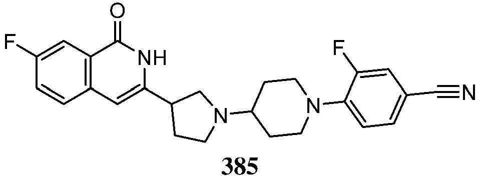

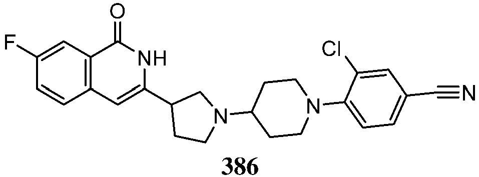

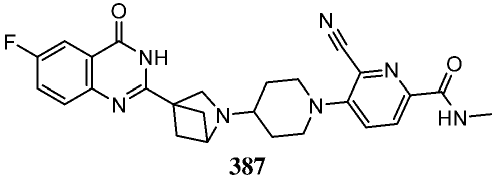

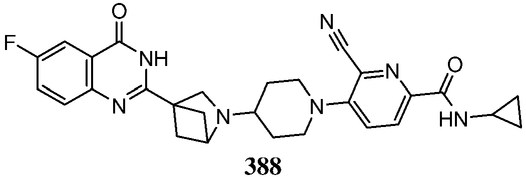

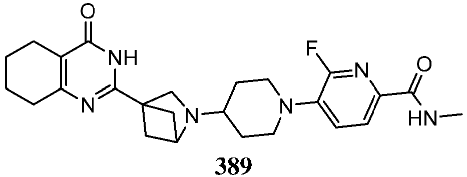

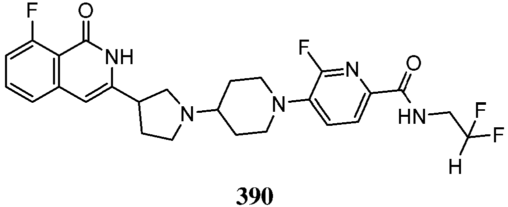

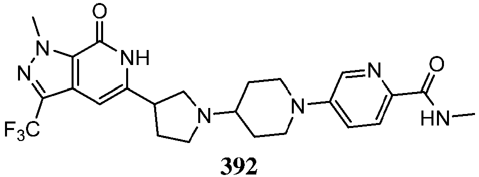

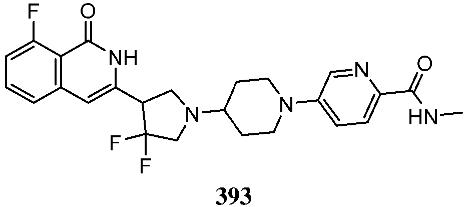

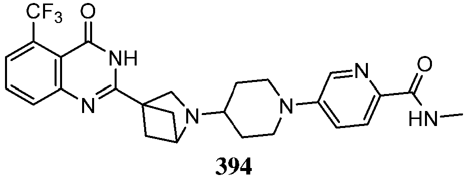

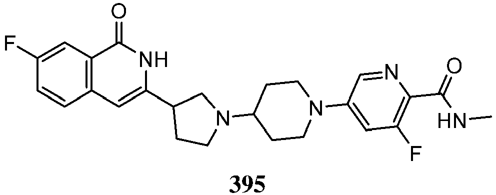

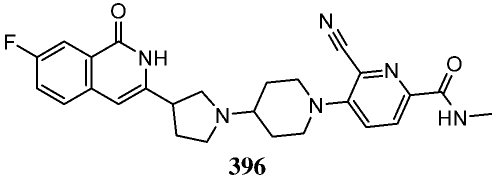

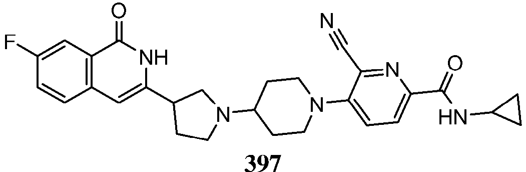

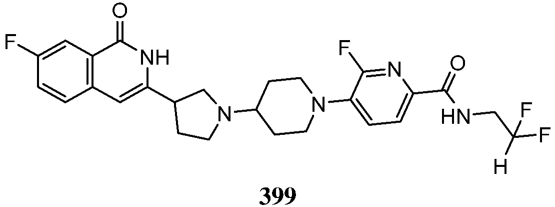

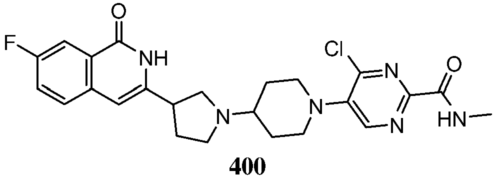

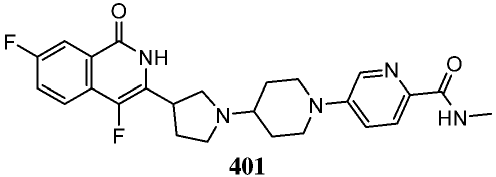

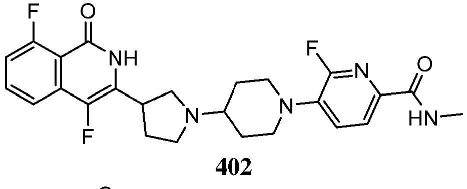

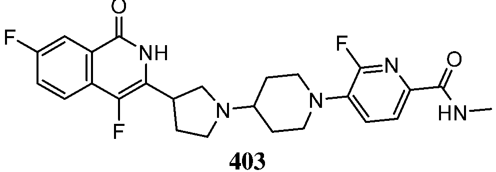

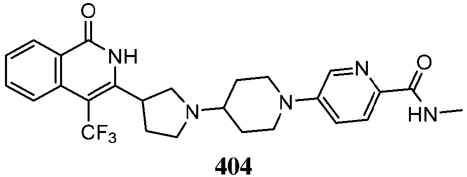

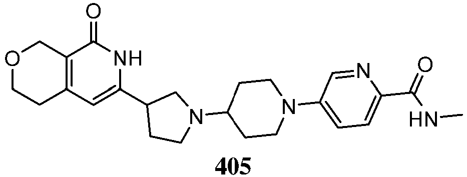

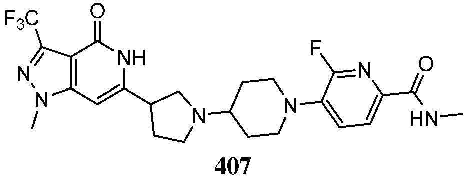

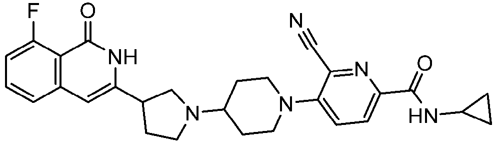

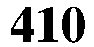

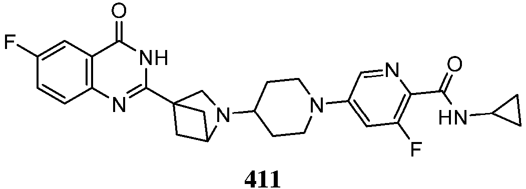

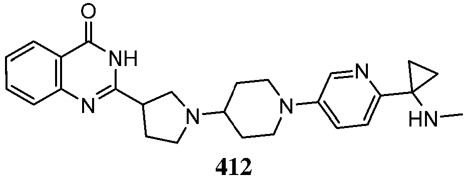

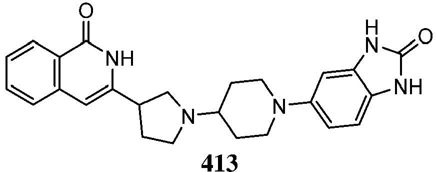

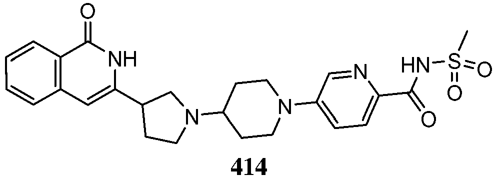

21. Un composé destiné à être utilisé selon l'une quelconque des revendications précédentes, lequel composé comprend :
- un énantiomère isolé, ou
- un mélange de deux ou plus de deux énantiomères, ou
- un mélange de deux ou plus de deux diastéréomères, et/ou épimères, ou
- un mélange racémique, ou
- un tautomère du composé.

22. Un composé destiné à être utilisé selon l'une quelconque des revendications précédentes, lequel est sélectif pour PARP1 par rapport à PARP2.

23. Une composition pharmaceutique comprenant un composé tel que défini dans l'une quelconque des revendications précédentes.

24. Une composition pharmaceutique selon la revendication 23, comprenant en outre un additif et/ou un excipient acceptable pharmaceutiquement, et/ou dans lequel le composé est sous la forme d'un sel, un hydrate, un acide, un ester, ou une autre forme alternative du composé acceptable pharmaceutiquement.

25. Un composé destiné à être utilisé selon l'une quelconque des revendications 1 à 22, ou une composition pharmaceutique selon la revendication 22 ou la revendication 23, qui est destiné à être utilisé dans le traitement du cancer.

26. Un composé ou une composition pharmaceutique destiné à être utilisé selon la revendication 25, dans lequel :
i) le cancer est un cancer sélectionné parmi : une tumeur solide ou liquide incluant un cancer de l'œil, du cerveau, de la moelle épinière, du rein, de la bouche, de la lèvre, de la gorge, de la cavité orale, de la cavité nasale, de l'intestin grêle, de l'intestin, de la glande parathyroïde, de la vésicule biliaire, de la tête et du cou, du sein, des os, du canal biliaire, du col de l'utérus, du cœur, de la glande hypopharyngienne, du poumon, des bronches, du foie, de la peau, de l'uretère, de l'urètre, des testicules, du vagin, de l'anus, de la glande laryngienne, de l'ovaire, de la thyroïde, de l'œsophage, de la glande nasopharyngienne, de l'hypophyse, de la glande salivaire, de la prostate, du pancréas, des glandes surrénales ; un cancer endométrial, un cancer oral, un mélanome, un neuroblastome, un cancer gastrique, une angiomatose, un hémangioblastome, un phéochromocytome, un kyste pancréatique, un carcinome rénal, une tumeur de Wilms, un carcinome épidermoïde, un sarcome, un ostéosarcome, un sarcome de Kaposi, un rhabdomyosarcome, un carcinome hépatocellulaire, des syndromes des hamartomes tumoraux liés au gène PTEN, des leucémies et des lymphomes, des cancers nasopharyngiens et gastrointestinaux ;
et/ou
ii) le cancer est déficient en voie de réparation en réponse aux dommages de l'ADN, telle qu'une activité de réparation de l'ADN de cassure double brin d'ADN dépendante de la recombinaison homologue ; et/ou
iii) le cancer est déficient en fonction BRCA1 et/ou BRCA2.

27. Une composition pharmaceutique destinée à être utilisée selon la revendication 25 ou la revendication 26, comprenant en outre un autre agent destiné au traitement du cancer ;
en option, dans lequel l'autre agent destiné au traitement du cancer est sélectionné parmi des agents anti-microtubule, des complexes de coordination de platine, des agents alkylants, des agents antibiotiques, des inhibiteurs de topoisomérase II, antimétabolites, des inhibiteurs de topoisomérase I, des agents hémolytiques, des hormones et des analogues d'hormone, des inhibiteurs de la voie de transduction de signaux, une autre voie de réparation en réponse aux dommages des inhibiteurs de l'ADN, des inhibiteurs de l'angiogenèse par tyrosine kinase non réceptrice, des conjugués anticorps-médicament, immunothérapeutiques, la thérapie antihormonale, des agents proapoptotiques et des inhibiteurs de la signalisation du cycle cellulaire ;
en option en outre, dans lequel l'autre agent est un agent immunothérapeutique sélectionné parmi un vaccin anti-tumeur, un virus oncolytique, un anticorps immunostimulateur tel que anti-CTLA4, anti-PD1, anti-PDL-1, anti-OX40, anti-41BB, anti-CD27, anti-CD40, anti-LAG3, anti-TIM3 et anti-GITR, un agoniste des récepteurs de reconnaissance des motifs tel qu'un agoniste STING, TLR-9 ou RIG-I Hélicase, un inhibiteur IDO ou TDO, un nouvel adjuvant, un peptide, une cytokine, une thérapie par lymphocytes T récepteurs d'antigènes chimériques, un modulateur immunitaire à petites molécules, des modulateurs du microenvironnement tumoral et des agents anti-angiogéniques.

28. Un kit pharmaceutique destiné au traitement un cancer, lequel kit pharmaceutique comprend :
a) un composé tel que défini dans l'une quelconque des revendications 1 à 22 ; et
b) un autre agent destiné au traitement du cancer ;
dans lequel le composé et l'autre agent conviennent pour l'administration simultanément, séquentiellement ou séparément ; et
de préférence, dans lequel l'autre agent destiné au traitement du cancer est sélectionné parmi des agents anti-microtubule, des complexes de coordination de platine, des agents alkylants, des agents antibiotiques, des inhibiteurs de topoisomérase II, des antimétabolites, des inhibiteurs de topoisomérase I, des agents sénolytiques, des hormones et des analogues d'hormone, des inhibiteurs de la voie de transduction de signaux, une autre voie de réparation en réponse aux dommages des inhibiteurs de l'ADN, des inhibiteurs de l'angiogenèse par tyrosine kinase non réceptrice, des conjugués anticorps-médicament, la thérapie antihormonale, des agents immunothérapeutiques, des agents proapoptotiques et des inhibiteurs de la signalisation du cycle cellulaire.

29. Un composé, qui est sélectionné parmi l'un quelconque des composés définis selon la revendication 20,
en option, dans lequel le composé comprend :
- un énantiomère isolé, ou
- un mélange de deux ou plus de deux énantiomères, ou
- un mélange de deux ou plus de deux diastéréomères, et/ou épimères, ou
- un mélange racémique, ou
- un tautomère du composé.

30. Un procédé de synthèse d'un composé inhibiteur de PARP1 tel que défini dans l'une quelconque des revendications 1 à 22, lequel procédé comprend la réalisation d'une réaction entre i) un premier réactif comprenant un cycle E portant une partie du groupe substituant L et ii) un deuxième réactif comprenant le reste du groupe substituant L, de façon à former le composé inhibiteur de PARP1.

31. - Un procédé selon la revendication 30, dans lequel la réaction comprend une alkylation, une amination réductrice ou une formation d'amide de façon à former le groupe L.
